(19) Europäisches Patentamt
European Patent Office
Office européen des brevets

(11) EP 4 269 400 B1

(12) **EUROPEAN PATENT SPECIFICATION**

(45) Date of publication and mention of the grant of the patent:
**09.07.2025 Bulletin 2025/28**

(21) Application number: **23170807.4**

(22) Date of filing: **28.04.2023**

(51) International Patent Classification (IPC):
***C07D 401/10*** (2006.01) ***C07D 413/10*** (2006.01)
***C07D 413/14*** (2006.01) ***C07D 417/10*** (2006.01)
***C07D 417/14*** (2006.01) ***C07D 495/04*** (2006.01)
***C07D 498/04*** (2006.01) ***C07H 15/26*** (2006.01)
***A61K 31/5377*** (2006.01) ***A61K 31/7012*** (2006.01)
***C07D 401/08*** (2006.01) ***C07D 413/08*** (2006.01)
***C07D 417/08*** (2006.01)

(52) Cooperative Patent Classification (CPC):
**C07D 401/08; C07D 413/08; C07D 413/14; C07D 417/08; C07D 417/14; C07D 495/04; C07D 498/04; C07H 15/26**

(54) **YKL-40 INHIBITORS AND THEIR THERAPEUTIC APPLICATIONS**

YKL-40-INHIBITOREN UND IHRE THERAPEUTISCHEN ANWENDUNGEN

INHIBITEURS DE YKL-40 ET LEURS APPLICATIONS THÉRAPEUTIQUES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.04.2022 PL 44106622**
**29.04.2022 US 202263336426 P**

(43) Date of publication of application:
**01.11.2023 Bulletin 2023/44**

(73) Proprietor: **Molecure SA**
**02-089 Warszawa (PL)**

(72) Inventors:
- **CZESTKOWSKI, Wojciech**
  **95-200 Pabianice (PL)**
- **MAZUR, Marzena**
  **91-158 Lodz (PL)**
- **WANAT, Przemyslaw**
  **04-131 Warszawa (PL)**
- **ANDRYIANAU, Gleb**
  **Irvine, 92602 (US)**
- **NIEDZIEJKO, Piotr**
  **00-168 Warszawa (PL)**
- **KOWALSKI, Michal**
  **02-661 Warszawa (PL)**
- **GRUZA, Mariusz Marek**
  **04-535 Warszawa (PL)**
- **LISIECKI, Kamil**
  **06-100 Pultusk (PL)**
- **POMARANSKI, Piotr**
  **05-088 Brochow (PL)**
- **KUSMIREK, Damian**
  **97-216 Czerniewice (PL)**
- **PAPIERNIK, Diana**
  **02-495 Warszawa (PL)**
- **DRZEWICKA, Katarzyna**
  **01-452 Warszawa (PL)**
- **PIWOWAR, Katarzyna**
  **02-367 Warszawa (PL)**
- **KRYSZTOFIAK, Katarzyna**
  **09-410 Plock (PL)**
- **OLCZAK, Jacek**
  **92-701 Lodz (PL)**
- **GOLEBIOWSKI, Adam**
  **Madison, 06443 (US)**
- **KRZEMINSKI, Lukasz**
  **03-287 Warszawa (PL)**
- **BARTOSZEWICZ, Agnieszka**
  **02-384 Warszawa (PL)**

(74) Representative: **Patpol Kancelaria Patentowa Sp. z o.o.**
**Nowoursynowska 162J**
**02-776 Warszawa (PL)**

(56) References cited:
**WO-A1-2015/095701** **WO-A1-2017/037670**
**WO-A1-2021/009209**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

CROSS REFERENCE TO RELATED APPLICATIONS

This application claims the benefit of priority to Polish Patent Application number P.441066, filed April 29, 2022; and United States Provisional Patent Application number 63/336,426, filed April 29, 2022.

FIELD OF THE INVENTION

This invention is related to novel inhibitors of chitinase-like protein YKL-40, and their therapeutic applications.

BACKGROUND OF THE INVENTION

The glycosyl hydrolase family 18 (GH 18) is an ancient gene family that is widely expressed in species as diverse as plants, insects and mammals. GH 18 protein family in humans, consists of two enzymatically active chitinases, CHIT1 and AMCase, and the non-active chitinase-like proteins (CLPs): YKL-40, YKL-39, oviductin and stabilin-interacting protein that lack enzymatic activity due to amino acid substitutions in the active site. Although chitinases and CLPs are evolutionary highly conserved, their physiological role in mammals has not been fully elucidated. In most mammals, *e.g.*, rodents, chitinases protect against chitin-containing organisms which are either inhaled or ingested. Mammals are not known to synthesize chitin or metabolize it as a nutrient, yet the human genome encodes eight GH18 family members (Funkhouser et al., Chitinase family GH18: evolutionary insights from the genomic history of a diverse protein family. BMC Evol Biol. Jun 26:7-96, 2007). In humans, chitinases and CLPs are thought to have evolved to perform different functions, often associated with inflammatory and fibrotic pathologies as well as tissue remodeling and cancer progression (Lee et al., Role of chitin and chitinase/chitinase-like proteins in inflammation, tissue remodeling, and injury. Annu Rev Physiol. 73:479-501, 2011).

Chitinase-3-like protein 1 (CHI3L1), also known as YKL-40 is a secreted glycoprotein that was first identified in culture media of a human osteosarcoma cell line MG63 (Johansen et al., Identification of proteins secreted by human osteoblastic cells in culture. Journal of Bone & Mineral Research 7:501-512, 1992). YKL-40 protein is approximately 40 kDa in size that in humans is encoded by the CHI3L1 gene, located on chromosome 1q31-1q32 and consists of 10 exons and spans about 8 kilobases of genomic DNA (Rehli et al., Molecular characterization of the gene for human cartilage gp-39 (CHI3L1), a member of the chitinase protein family and marker for late stages of macrophage differentiation. Genomics. 43(2):221-5, 1997). The name YKL-40 derives from the three N-terminal amino acids present on the secreted form and its molecular mass in kilodaltons. YKL-40 lacks chitinase activity due to mutations of one of the three amino acids (Asp, Glu and Asp) essential for the chitinase-like catalytic activity, namely Glu -> Leu, which completely deprives chitinase activity (Renkema et al., Chitotriosidase, a chitinase, and the 39-kDa human cartilage glycoprotein, a chitin-binding lectin, are homologues of family 18 glycosyl hydrolases secreted by human macrophages. Eur J Biochem. 251: 504-509, 1998). YKL-40 has been shown to bind chitin oligomers, chitin-like oligosaccharides, hyalurorian, heparin as well as heparan sulfate. However, the biological role of YKL-40 still remains unclear. YKL-40 is not even known to have a specific receptor, although it has been proposed to act through IL13R$\alpha$2 (Chuanet at al., Chitinase 3-like 1 Regulates Cellular and Tissue Responses via IL-13 Receptor $\alpha$2. Cell Rep. 4(4): 830-841, 2013) and Syndecan-1 (Shao et al., YKL-40, a secreted glycoprotein, promotes tumor angiogenesis, Oncogene. 28:4456-4468, 2009), CD44 (Geng et al. Chitinase 3-like 1-CD44 interaction promotes metastasis and epithelial-to-mesenchymal transition through $\beta$-catenin/Erk/Akt signaling in gastric cancer, J Exp Clin Cancer Res 37:208, 2018, RAGE (Low et al., Chitinase 3-like 1 induces survival and proliferation of intestinal epithelial cells during chronic inflammation and colitis-associated cancer by regulating S100A9. Oncotarget.;6:36535-50 2015) receptors and other proteins as lectin - Galectin3 (Bouvet et al., Peripheral blood mononuclear cell response to YKL-40 and Galectin-3 in cystic fibrosis. Cytokine. 146:155635, 2021) via respectively chitin-binding and heparin-binding domains but its detailed biological functions are still poorly understood.

Despite the fact that the exact function of YKL-40 is still unknown, the pattern of YKL-40 expression is associated with pathogenic processes related to inflammation (including CNS), extracellular tissue remodeling, fibrosis oncogenesis. YKL-40 is expressed and secreted by various cell-types including macrophages, chondrocytes, fibroblast-like synovial cells, vascular smooth muscle cells, hepatic stellate cells as well as cancer cells. In accordance to these observations, elevated levels of YKL-40 protein have been detected in a wide range of diseases, comprising of infections, arthritis, inflammatory bowel disease, chronic obstructive lung disease, idiopathic pulmonary fibrosis, asthma, nonalcoholic steatohepatitis, hepatitis, diabetes, atherosclerosis, giant cell arteritis (Lee et al., Chitin, chitinases and chitinase-like proteins in allergic inflammation and tissue remodeling. Yonsei Med J 50:22-30, 2009; Lee et al., Role of chitin and chitinase/chitinase-like proteins in inflammation, tissue remodeling and injury. Annu Rev Physiol 73:479-501, 2011; Lee et al., Role of breast regression protein-39/YKL-40 in asthma and allergic responses. Allergy Asthma Immunol Res 2:20-7, 2010; 7. Lee et al. Role of breast regression protein 39 (BRP-39)/chitinase 3-like-1 in Th2 and IL-13-induced tissue

responses and apoptosis. J Exp Med 206:1149-66, 2009; Dela Cruz et al., Chitinase 3-like-1 (Chi3l1) Regulation of Streptococcus pneumoniae Lung Infection. Cell Host Microbe 12:34-4, 2012; Johansen et al., A new biochemical marker for joint injury. Analysis of YKL-40 in serum and synovial fluid. Br J Rheumatol 32:949-55, 1993), neurodegenerative diseases like Alzheimer disease (Muszyński et al., YKL-40 as a Potential Biomarker and a Possible Target in Therapeutic Strategies of Alzheimer's Disease. Curr Neuropharmacol. 15(6):906-917, 2017), cancer and many others.

Cancer is a group of diseases caused by abnormal and uncontrolled cell proliferation with the potential to invade or spread to other organs of the body. YKL-40 is produced and secreted by several types of human cancer cells and tumor-associated macrophages as well as cancer-associated fibroblasts. It has been postulated that it may play a role in cell proliferation, differentiation, cell survival, invasiveness, metastasis, in angiogenesis, inflammatory response to cancer and tissue remodeling. YKL-40 serum protein level is considered as prognostic biomarker in a variety of different cancer types (breast, colon, lung, kidney, ovarian, prostate, uterine, osteosarcoma, oligodendroglioma, glioblastoma and germ cell tumors), elevated serum YKL-40 level has been correlated with poor prognosis and shorter survival of patients with cancer (Johansen et al., Is YKL-40 a new therapeutic target in cancer? Expert Opin Ther Targets. 11(2):219-34, 2017). Studies in animal models have demonstrated that YKL-40 overexpression enhances breast (MDA-MB-231 cells) and colon (HCT116 cells) tumor growth and angiogenesis in vivo, on the other hand, U87 glioblastoma cancer cells expressing YKL-40 siRNA abrogated tumor angiogenesis and tumor growth in vivo (Shao et al., YKL-40, a secreted glycoprotein, promotes tumor angiogenesis. Oncogene 28(50): 4456-4468, 2009). It has been also reported that YKL-40 neutralizing antibodies are effective in blocking tumor angiogenesis and tumor progression (Faibish et al., A YKL-40-neutralizing antibody blocks tumor angiogenesis and progression: a potential therapeutic agent in cancers. Mol Cancer Ther. 10(5):742-51, 2011). Also several small molecules targeting YKL-40 were shown to suppress tumor growth and metastasis in lung, osteosarcoma, melanoma and breast cancer models (Park, et al. G721-0282 inhibits cell growth and induces apoptosis in human osteosarcoma through down-regulation of the STAT3 pathway. International journal of biological sciences 16, 2, 330-341, 2020; Lee et al., A small molecule targeting CHI3L1 inhibits lung metastasis by blocking IL-13R$\alpha$2-mediated JNK-AP-1 signals. Mol Oncol. 16(2):508-526, 2022).

Chitinase and chitinase-like proteins are over expressed in many cancers including brain tumors such as glioblastoma (Francescone et al. J Biol Chem 2011;286:15332-43; Ku et al. Int J Cancer 2011;128:1316-26) or astrocytoma (Zhang et al. Cancer. 2010;116:2688), breast cancer (Johansen et al. Breast Cancer Res Treat 2003;80:15-21), colon cancer (Nutt *et al.*, 2005, Pelloski *et al.*, 2005; Fijneman et al. Clin Cancer Res. 2012;18:2613; Chen et al. Am J Pathol. 2011;179:1494), primary and metastatic lung cancer (Wang et al. Tumour Biol 2015;36:901-7; Johansen et al. Lung Cancer 2004;46:333-40), mesothelioma (Corradi et al. Anticancer Res. 2013 Dec;33(12):5517), osteosarcoma, malignant melanoma (Ma et al. Cancer Res 2015;75:487-96), ovarian cancer (Hogdall et al. BMC Cancer 2009;9:8; Dupont et al. J Clin Oncol. 2004;22:3330), cervical cancer (Ngernyuang et al. Int J Biochem Cell Biol 2014;51:45-52.), prostate cancer (Jeet et al. Endocr Relat Cancer. 2014;21:723), liver cancer (Pan et al. J Cancer Res Clin Oncol 2013;139:1043-54), gastric cancer (Li et al. Chin Med J 2012;125:1777), metastatic renal cancer (Zhangg et al. Tumour Biol 2014;35:12131-7), hematologic malignancies such as leukemia or lymphoma (Mactier et al. J Proteome Res. 2011;10:1030; Marchesi et al. Vet Pathol. 2006;43:773-6; Marchesi et al. J Vet Med A Physiol Pathol Clin Med. 2003;50:103) and other types of cancers with inflammatory background (Quershi et al. Genes Cancer. 2011;2:74; Eurich et al. World J Gastroenterol. 2009;15:5249; Roslind and Johansen, Methods of Mol Biol. 2009;511:159). In fact, higher plasma levels indicate poor prognosis and increased metastatic potential for wide range of cancers (Johansen et al., Cancer Epidemiol. Biomarkers Prev., 15(2):194-202, 2006). Inhibition of chitinase and chitinase-like protein biological function with one or more compounds described in this invention is anticipated to have therapeutic utility in subjects with cancer.

Interstitial lung diseases (ILDs) is a group of over 300 lung disorders which affect lung interstitium: the most common are sarcoidosis and idiopathic pulmonary fibrosis (IPF). These diseases, many with unknown etiology, are characterized by the alveolar damage which often leads to a chronic inflammation and fibrosis resulting in diminished lung functions.

Sarcoidosis is a multiorgan systemic disease characterized by a formation of granulomas that can develop in various organs, with most patients developing pulmonary presentation. Spontaneous remission occurs in a majority of cases, but up to one-third of patients develop a chronic, progressive or relapsing disease with a concomitant interstitial fibrosis and decline in lung functions (Valeyre et al., Sarcoidosis. Lancet 383(9923):1155-67, 2014). The clinical studies demonstrated highly elevated YKL-40 serum levels in sarcoidosis patients compared to controls (Long et al., Serum YKL-40 as predictor of outcome in hypersensitivity pneumonitis. Eur Respir J. 23:49-51, 2017) and YKL-40 serum protein level has been postulated as new sarcoidosis marker (Johansen et al., Increased serum YKL-40 in patients with pulmonary sarcoidosis-a potential marker of disease activity? Respir Med. 99(4):396-402, 2005).

IPF is a progressive fibroproliferative disorder with no curative therapies with a median survival of only 3-5 years following diagnosis. IPF is a devastating disease characterized by excessive matrix deposition that disrupts the normal architecture of the lung parenchyma and impairs lung functions. The key pathological features of IPF include fibroblastic foci, areas of epithelial cysts associated with the honeycombing appearance of the lung, and mild lymphoplasmacytic

interstitial inflammation that is associated with areas of type II cell hyperplasia (Richeldi et al., Idiopathic pulmonary fibrosis. Lancet 389(10082):1941-1952, 2017). A series of clinical studies have demonstrated an increased YKL-40 protein levels in serum and bronchoalveolar lavage fluid (BALF) of patients with idiopathic pulmonary fibrosis (Furuhashi et al., Increased expression of YKL-40, a chitinase-like protein, in serum and lung of patients with idiopathic pulmonary fibrosis. Respir Med. 104(8):1204-10, 2010; Vij et al., Peripheral blood biomarkers in idiopathic pulmonary fibrosis. Transl Res. 159(4):218-27, 2012) suggesting that YKL-40 protein might be involved in remodeling and tissue damage seen in lungs from IPF patients. Furthermore, elevated YKL-40 serum and BALF level are considered as predictors of survival in idiopathic pulmonary fibrosis (Korthagen et al., Serum and BALF YKL-40 levels are predictors of survival in idiopathic pulmonary fibrosis. Respir Med. 2011 Jan;105(1):106-13).

Obstructive lung diseases (among them asthma and chronic obstructive lung disease COPD) are chronic inflammatory disorders that involve the small airways and cause airflow limitation. Asthma is characterized by recurrent episodes of reversible airway obstruction and airway hyper responsiveness. Typical clinical manifestations include shortness of breath, wheezing, coughing and chest tightness that can become life threatening or fatal. While existing therapies focus on reducing the symptomatic bronchospasm and pulmonary inflammation, there is a growing recognition of the role of long-term airway remodeling in the accelerated lung deterioration in asthmatics. (Papi et al. Asthma. Lancet 391(10122):783-800, 2018). COPD is a disease characterized by a progressive, irreversible limitation of airflow due to emphysema and remodeling of small airways. Currently, no drugs reducing COPD progression are available with smoking cessation the only intervention demonstrated to slow the rate of decline in lung function. (Rabe et al. Chronic obstructive pulmonary disease. Lancet 389(10082):1931-1940, 2017). Multiple studies have demonstrated that YKL-40 may be implicated in bronchial inflammation and remodeling in COPD and its elevated level may be considered as a useful biomarker for COPD diagnosis and monitoring (Xiang et al., The YKL-40 protein is a potential biomarker for COPD: a meta-analysis and systematic review. Int J Chron Obstruct Pulmon Dis. 13: 409-418, 2018). Another meta-analysis suggested that YKL-40 protein may play an important role in the pathogenesis of asthma and its serum levels may correlate with exacerbation attacks and severity as well (Xiang et al., The serum YKL-40 is a useful biomarker for asthma. European Respiratory Journal 50: PA3566, 2017).

There is a need for novel active compounds interfering with interactions of YKL-40 and other biomolecules such as chitin oligomers and heparins. Such active compounds could act as YKL-40 inhibitors, and be of use as therapeutic agents for the treatment a variety of diseases and disorders, including cancers, inflammation, and pulmonary diseases. Alternative way of targeting YKL-40 is to use bispecific small molecule degraders that bind strongly to YKL-40 and simultaneously to one of proteins from protein degradation machinery. The mechanism of action of such degraders is to bring into close proximity these two proteins and trigger degradation of YKL-40.

SUMMARY OF THE INVENTION

Any references hereinafter to methods of treatment refer to the compounds, pharmaceutical compositions and medicaments of the present invention for use in a method for treatment of the human (or animal) body by therapy (or for diagnosis).

In one aspect, this invention concerns a novel compound represented by structural formula (I),

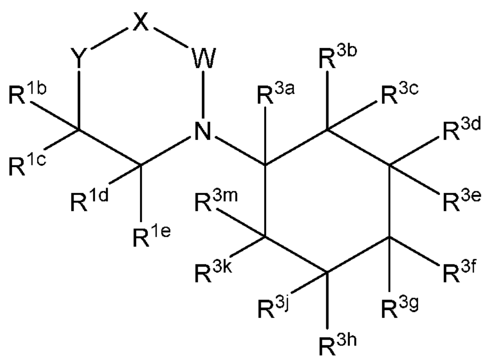

(I)

wherein:

W represents $-C(-R^{1f})(-R^{1g})-$;
X represents $-C(-R^{1h})(-R^{1j})-$;
Y represents $-C(-R^{1k})(-R^{1m})-$, $-N(-R^{1k})-$, or -O-;
$R^{1b}$ represents -H;

$R^{1c}$ represents -H;
$R^{1d}$ represents 4-$R^{10}$-benzyl;
$R^{1e}$ represents -H;
$R^{1f}$ represents -H;
$R^{1g}$ represents -H;
$R^{1h}$ is selected from the group consisting of $C_1$-$C_6$ alkyl, $C_2$-$C_6$ alkenyl, $C_2$-$C_6$ alkynyl, hydroxy-$C_1$-$C_6$ alkyl, azido-$C_1$-$C_6$ alkyl, hydroxycarbonyl-$C_1$-$C_3$ alkoxy-$C_1$-$C_6$ alkyl, $C_3$-$C_6$ alkynyloxy-$C_1$-$C_6$ alkyl, halo-$C_1$-$C_6$ alkyl, $C_1$-$C_3$ alkylthio-$C_1$-$C_6$ alkyl, $C_1$-$C_3$ alkylsulfinyl-$C_1$-$C_6$ alkyl, $C_1$-$C_3$ alkylsulfonyl-$C_1$-$C_6$ alkyl, hydroxycarbonyl-$C_1$-$C_3$ alkylsulfonyl-$C_1$-$C_6$ alkyl, hydroxycarbonyl-$C_1$-$C_6$ alkyl, $C_1$-$C_3$ alkylcarbonyl-$C_1$-$C_6$ alkyl, $C_1$-$C_3$ alkylaminocarbonyl-$C_1$-$C_6$ alkyl, di($C_1$-$C_3$ alkyl)aminocarbonyl-$C_1$-$C_6$ alkyl, $C_1$-$C_3$ alkylsulfonylamino-$C_1$-$C_6$ alkyl, halo-$C_1$-$C_3$ alkylsulfonylamino-$C_1$-$C_6$ alkyl, 5- or 6-membered monocyclic heteroaryl-carbonylamino-$C_1$-$C_6$ alkyl, 5- or 6-membered monocyclic heteroaryl-$C_1$-$C_3$ alkoxy-$C_1$-$C_6$ alkyl, 5- or 6-membered monocyclic heteroaryl-$C_1$-$C_6$ alkyl, hydroxycarbonyl, $C_1$-$C_3$ alkoxycarbonyl, aminocarbonyl, $C_1$-$C_3$ alkylaminocarbonyl, di($C_1$-$C_3$ alkyl)aminocarbonyl, $C_3$-$C_6$ alkynylaminocarbonyl, hydroxycarbonyl-$C_1$-$C_3$ alkyl($C_1$-$C_3$ alkyl)aminocarbonyl, (biotinyl-$PEG_{11}$)aminocarbonyl, (biotinyl-$PEG_{11}$)aminocarbonyl-$C_1$-$C_3$ alkylsulfonyl-$C_1$-$C_6$ alkyl, hydroxylaminocarbonyl, *N*$^2$-acetylhydrazinecarbonyl, (5- or 6-membered monocyclic heteroaryl)-$C_1$-$C_3$ alkylaminocarbonyl, (5- or 6-membered monocyclic heteroaryl) aminocarbonyl, $C_1$-$C_3$ alkylsulfonyl, 5- or 6-membered monocyclic heteroaryl, (3-azabicyclo[3.1.0]hexan-3-yl)-$C_1$-$C_3$ alkyl, (4-, 5-, or 6-membered heterocyclyl)amino-$C_1$-$C_3$ alkyl, hydroxycarbonyl-$C_1$-$C_6$ alkyl-(5- or 6-membered monocyclic heteroaryl), $R^{20}$, and 5-(2-(2,6-dioxopiperidin-3-yl)-3-oxoisoindolin-4-yl)pentyl,
where any available position of any heteroaryl ring in $R^{1h}$ can be substituted with a substituent selected from the group consisting of $C_1$-$C_6$ alkyl, $C_3$-$C_6$ alkynyl, halo, trifluoromethyl, 2,2,2-trifluoroethyl, biotinyl-$PEG_{11}$, and any available position of any carbon chain in $R^{1h}$ excluding heteroaryl rings can be substituted with a substituent selected from halogen, amino, hydroxy, and oxo;
$R^{1j}$ represents -H;
$R^{1k}$ represents -H;
$R^{1m}$ represents -H;
or $R^{1k}$ and $R^{1h}$ taken along with nitrogen and carbon atoms bearing them, respectively, represent a 6-membered heterocyclic ring of structural formula (II):

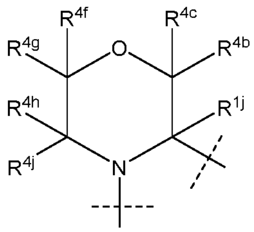
(II)

$R^{3a}$ represents -H;
$R^{3b}$ represents -H;
$R^{3c}$ represents -H;
$R^{3d}$ represents -H;
$R^{3e}$ represents -H;
$R^{3f}$ is selected from the group consisting of 1-methyl-1*H*-imidazol-4-yl, 1,2-dimethyl-1*H*-imidazol-4-yl, 5-methyloxazol-2-yl, 4,5-dimethyloxazol-2-yl, 1*H*-pyrazol-1-yl, 1-methyl-1*H*-pyrazol-3-yl, 4-methyl-1*H*-pyrazol-1-yl, 4-fluoro-1*H*-pyrazol-1-yl, 1-isopropyl-1*H*-pyrazol-3-yl, 5-methyl-1*H*-pyrazol-3-yl, 1,5-dimethyl-1*H*-pyrazol-3-yl, 1-ethyl-5-methyl-1*H*-pyrazol-3-yl, 1,5-dimethyl-1*H*-pyrazol-3-yl, 1,5-dimethyl-1*H*-pyrazol-4-yl, 1-methyl-5-(trifluoromethyl)-1*H*-pyrazol-3-yl, 1-methyl-2-cyano-1*H*-pyrrol-4-yl, 1*H*-tetrazol-5-yl, 2-methyl-2*H*-tetrazol-5-yl, 5-methyl-1,3,4-thiadiazol-2-yl, 5-methylthiazol-2-yl, 4,5-dimethylthiophen-2-yl, 1*H*-1,2,4-triazol-1-yl, 1-methyl-1*H*-1,2,3-triazol-4-yl, 1-methyl-1*H*-1,2,4-triazol-3-yl, 2-methyl-2*H*-1,2,3-triazol-4-yl, 1,5-dimethyl-1*H*-1,2,4-triazol-3-yl, 5-hydroxy-1-methyl-1*H*-1,2,4-triazol-3-yl, and 5-methoxy-1-methyl-1*H*-1,2,4-triazol-3-yl;
$R^{3g}$ represents -H;
$R^{3h}$ represents -H;
$R^{3j}$ represents -H;
$R^{3k}$ represents -H;
$R^{3m}$ represents -H;
or $R^{3d}$ and $R^{3g}$ taken along with the single bond linking two carbon atoms bearing them represent a double bond between these two carbon atoms;

$R^{4b}$ represents -H;
$R^{4c}$ represents -H;
$R^{4f}$ represents -H;
$R^{4g}$ represents -H;
$R^{4h}$ represents -H;
$R^{4j}$ represents -H;
$R^{10}$ represents halogen or trifluoromethyl;
and $R^{20}$ represents

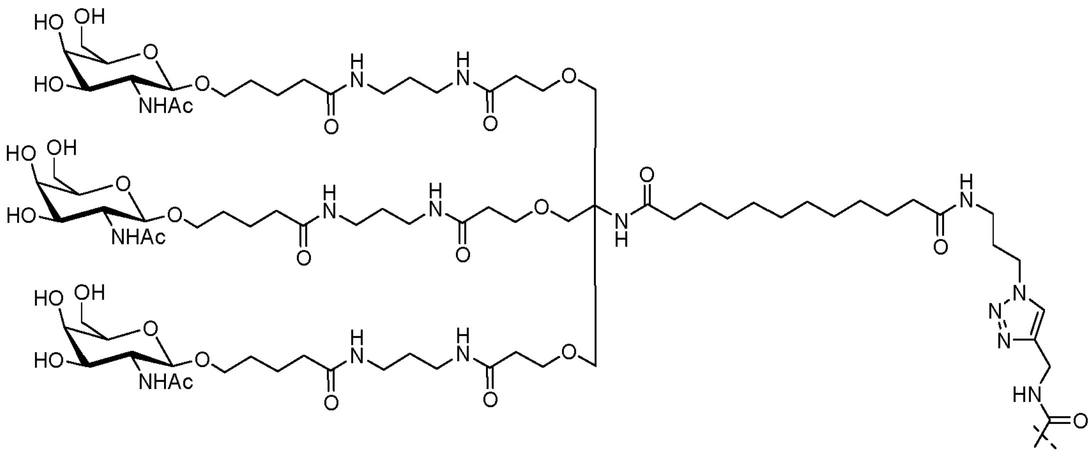
;

or a tautomer, stereoisomer, N-oxide, methiodide, pharmaceutically acceptable salt, solvate, or polymorph thereof.

Another aspect of the invention is directed to a pharmaceutical composition comprising a therapeutically effective amount of at least one compound according to the invention and a pharmaceutically acceptable carrier therefor.

Yet another aspect of the invention concerns a method for inhibiting the chitinase-like protein YKL-40 in a cell or a tissue, comprising contacting the cell or the tissue with at least one compound according to the invention.

Yet another aspect of the invention concerns a method for degrading the chitinase-like protein YKL-40 in a cell or a tissue, comprising contacting the cell or the tissue with at least one compound according to the invention.

The invention provides also a method for the treatment or prevention of a disease, disorder, or condition associated with aberrant expression or activity of the chitinase-like protein YKL-40, comprising administering to a subject in need thereof a therapeutically effective amount of at least one compound according to the invention, or a pharmaceutical composition according to the invention.

DETAILED DESCRIPTION OF THE INVENTION

*Definitions*

The articles "a" and "an" are used herein to refer to one or to more than one (*i.e.,* to at least one) of the grammatical object of the article. By way of example, "an element" means one element or more than one element.

The terms "substituent," "group," and "radical" are used interchangeably herein and denote a portion of a molecule in question which is bound to the rest of the molecule by a covalent bond (or bonds, as it results from the previous paragraph).

The terms used herein may be preceded and/or followed by a single dash "-", or a double dash "=", to indicate the bond order of the bond between the named substituent and its parent moiety; a single dash indicates a single bond and a double dash indicates a double bond. In the absence of a single or double dash, it is understood that a single bond is formed between the substituent and its parent moiety; further, substituents are intended to be read "from left to right," unless a dash indicates otherwise. For example, $C_1$-$C_6$ alkylcarboxy and -OC(O)-$C_1$-$C_6$ alkyl indicate the same functionality; similarly, arylalkyl and -alkylaryl indicate the same functionality.

The term "single bond" as used herein, denotes a single covalent bond between two atoms, such as C-C, C-H, or C-O.

The term "alkyl" as used herein is a term of art and refers to saturated aliphatic groups, including straight-chain alkyl groups and branched-chain alkyl groups. The subscripts following C indicate the number (or range of numbers) of carbon atoms in the straight-chain or branched-chain alkyl. From the practical reasons, the number of carbon atoms in a

straight-chain alkyl is limited to the range of 1-8, inclusive, and the number of carbon atoms in a branched-chain alkyl is in the range of 3-8, inclusive. If there is no subscript specified defining the number of carbon atoms in an alkyl group, then this number is not greater than 8. Representative examples of $C_1$-$C_6$ alkyl include, but are not limited to, methyl, ethyl, *n*-propyl, isopropyl, *n*-butyl, *sec*-butyl, isobutyl, *tert*-butyl, *n*-pentyl, isopentyl, neopentyl, and *n*-hexyl. Examples of $C_1$-$C_3$ alkyl include methyl, ethyl, *n*-propyl, and isopropyl. Alkyl may represent a group, as already defined, or a portion of a larger moiety, such as $C_1$-$C_3$ alkoxy-$C_1$-$C_3$ alkyl. A $C_1$-$C_3$ alkoxy-$C_1$-$C_3$ alkyl is bound to the rest of the molecule through the $C_1$-$C_3$ alkyl moiety.

If the name of particular alkyl group appears in the name of a chemical compound, the prefixes such as *iso*-, *sec*-, *tert*-, or *neo*-, indicate an appropriately branched alkyl, as understood by the person skilled in the art; the prefix *n*- or lack of any prefix means that the alkyl group in question is a straight-chain alkyl.

The term "cycloalkyl" means a radical derived from mono- or bicyclic or bridged saturated or partially saturated carbocyclic rings, each having from 3 to 12 carbon atoms. Certain cycloalkyls have from 3-8, or from 3-6 carbon atoms in their ring structure. Certain cycloalkyls have from 5-12 carbon atoms in their ring structure, and may have 6-10 carbons in the ring structure. Preferably, cycloalkyl is $C_3$-$C_7$ cycloalkyl, which represents a monocyclic saturated carbocyclic ring, having from 3 to 7 carbon atoms. Examples of monocyclic cycloalkyls include cyclopropyl, cyclobutyl, cyclopentyl, cyclopentenyl, cyclohexyl, cyclohexenyl, cycloheptyl, and cyclooctyl. Bicyclic cycloalkyl ring systems include bridged monocyclic rings and fused bicyclic rings. Bridged monocyclic rings contain a monocyclic cycloalkyl ring where two non-adjacent carbon atoms of the monocyclic ring are linked by an alkylene bridge of between one and three additional carbon atoms (*i.e.,* a bridging group of the form $-(CH_2)_w-$, where *w* is 1, 2, or 3). Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. Fused bicyclic cycloalkyl ring systems contain a monocyclic cycloalkyl ring fused to either a phenyl, a monocyclic cycloalkyl, a monocyclic cycloalkenyl, a monocyclic heterocyclyl, or a monocyclic heteroaryl. The bridged or fused bicyclic cycloalkyl is attached to the parent molecular moiety through any carbon atom contained within the monocyclic cycloalkyl ring. Cycloalkyl groups are optionally substituted. In certain embodiments, the fused bicyclic cycloalkyl is a 5 or 6 membered monocyclic cycloalkyl ring fused to either a phenyl ring, a 5 or 6 membered monocyclic cycloalkyl, a 5 or 6 membered monocyclic cycloalkenyl, a 5 or 6 membered monocyclic heterocyclyl, or a 5 or 6 membered monocyclic heteroaryl, wherein the fused bicyclic cycloalkyl is optionally substituted.

The term "cycloalkylalkyl" as used herein refers to an alkyl group substituted with one or more cycloalkyl groups. An example of cycloalkylalkyl is cyclohexylmethyl group.

The terms "heterocyclyl" and "heterocycloalkyl" as used herein refers to a radical of a nonaromatic ring system, including, but not limited to, monocyclic, bicyclic, and tricyclic rings, which can be completely saturated or which can contain one or more units of unsaturation, for the avoidance of doubt, the degree of unsaturation does not result in an aromatic ring system, and having 3 to 14, or 3 to 12 atoms other than hydrogen, including at least one heteroatom, such as nitrogen, oxygen, or sulfur. More preferred heterocycloalkyl groups have from 5-10 ring members where from 1-4 of the ring members are hetero atoms selected from the group consisting of O, N, and S, the remaining ring atoms being C. For purposes of exemplification, which should not be construed as limiting the scope of this invention, the following are examples of heterocycloalkyl: aziridinyl, azirinyl, oxiranyl, thiiranyl, thiirenyl, dioxiranyl, diazirinyl, diazepanyl, 1,3-dioxanyl, 1,3-dioxolanyl, 1,3-dithiolanyl, 1,3-dithianyl, imidazolidinyl, isothiazolinyl, isothiazolidinyl, isoxazolinyl, isoxazolidinyl, azetyl, oxetanyl, oxetyl, thietanyl, thietyl, diazetidinyl, dioxetanyl, dioxetenyl, dithietanyl, dithietyl, dioxalanyl, oxazolyl, thiazolyl, triazinyl, isothiazolyl, isoxazolyl, azepines, azetidinyl, morpholinyl, oxadiazolinyl, oxadiazolidinyl, oxazolinyl, oxazolidinyl, oxopiperidinyl, oxopyrrolidinyl, piperazinyl, piperidinyl, pyranyl, pyrazolinyl, pyrazolidinyl, pyrrolinyl, pyrrolidinyl, quinuclidinyl, thiomorpholinyl, tetrahydropyranyl, tetrahydrofuranyl, tetrahydrothienyl, thiadiazolinyl, thiadiazolidinyl, thiazolinyl, thiazolidinyl, thiomorpholinyl, 1,1-dioxidothiomorpholinyl (thiomorpholine sulfone), thiopyranyl, and trithianyl. A heterocycloalkyl group is optionally substituted by one or more substituents as described below.

As used herein, the terms "heterocyclylene" and "heterocycloalkylene" refers to a bivalent heterocyclyl (heterocycloalkyl) group, *i.e.,* a cyclic alkylene group, having from 3-10 members and from 1-4 hetero atoms selected from S, O, and N. An example is piperidine-2,3-dicarboxylic acid, *i.e.,* in that compound, the piperidine ring is a heterocyclylene group.

The term "heteroatom" is art-recognized, and includes an atom of any element other than carbon or hydrogen. Illustrative heteroatoms include boron, nitrogen, oxygen, phosphorus, sulfur and selenium, and alternatively oxygen, nitrogen or sulfur.

The term "heterocycloalkylalkyl" as used herein refers to an alkyl group substituted with one or more heterocycloalkyl (*i.e.,* heterocyclyl) groups.

The term "alkenyl" as used herein means a straight or branched chain hydrocarbon radical containing from 2 to 10 carbons and containing at least one carbon-carbon double bond formed by the removal of two hydrogens. Preferably, alkenyl contains from 2 to 6 carbons. Representative examples of alkenyl include, but are not limited to, ethenyl, 2-propenyl, 2-methyl-2-propenyl, 3-butenyl, 4-pentenyl, and 5-hexenyl. The unsaturated bond(s) of the alkenyl group can be located anywhere in the moiety and can have either the (*Z*) or the (*E*) configuration about the double bond(s). The molecules differing only in their configuration about the double bond are called geometrical isomers.

The term "alkynyl" as used herein means a straight or branched chain hydrocarbon radical containing from 2 to 10 carbon atoms and containing at least one carbon-carbon triple bond. "$C_3$-$C_6$ alkynyl" means alkynyl containing from 3 to 6 carbon atoms. Representative examples of alkynyl include, but are not limited, to acetylenyl (*i.e.*, ethynyl), 1-propynyl, 2-propynyl, 3-butynyl, 2-pentynyl, and 1-butynyl. This group can form a portion of another substituent, such as $C_3$-$C_6$ alkynyloxy-$C_1$-$C_6$ alkyl.

The term "alkylene" is art-recognized, and as used herein pertains to a diradical obtained by removing two hydrogen atoms of an alkyl group, as defined above. In one embodiment an alkylene refers to a disubstituted alkane, *i.e.*, an alkane substituted at two positions with substituents such as halogen, azide, alkyl, aralkyl, alkenyl, alkynyl, cycloalkyl, hydroxyl, alkoxyl, amino, nitro, sulfhydryl, imino, carboxamido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, fluoroalkyl (such as trifluromethyl), cyano, or the like. That is, in one embodiment, a "substituted alkyl" is an "alkylene".

The term "amino" is a term of art and as used herein refers to both unsubstituted and substituted amines, *e.g.*, a moiety that may be represented by the general formulas:

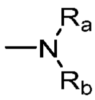 and 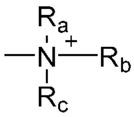 ,

wherein $R_a$, $R_b$, and $R_c$ each independently represent a hydrogen, an alkyl, an alkenyl, $-(CH_2)_x$-$R_d$, or $R_a$ and $R_b$, taken together with the N atom to which they are attached complete a heterocycle having from 4 to 8 atoms in the ring structure; $R_d$ represents an aryl, a cycloalkyl, a cycloalkenyl, a heterocyclyl or a polycyclyl; and x is zero or an integer in the range of 1 to 8. In certain embodiments, only one of $R_a$ or $R_b$ may be a carbonyl, *e.g.*, $R_a$, $R_b$, and the nitrogen together do not form an imide. In other embodiments, $R_a$ and $R_b$ (and optionally $R_c$) each independently represent a hydrogen, an alkyl, an alkenyl, or $-(CH_2)_x$-$R_d$. In certain embodiments, the term "amino" refers to $-NH_2$.

The term "aminocarbonyl" refers to $H_2NC(=O)$-, and one or both hydrogen atoms attached to the N atom can be substituted with the substituents $R_a$, or $R_a$ and $R_b$, as defined above. Additionally, $R_a$ can also represent $C_3$-$C_6$ alkynyl, hydroxyl, or (biotynyl-$PEG_n$), wherein n ranges from 9 to 15.

The term "carboxamido", as used herein, means -NHC(=O)-, wherein the carboxamido group is bound to the parent molecular moiety through the nitrogen. Examples of carboxamido include alkylcarboxamido such as $CH_3C(=O)N(H)$- and $CH_3CH_2C(=O)N(H)$-.

The term "acyl" is a term of art and as used herein refers to any group or radical of the form RCO- where R is any organic group, *e.g.*, alkyl, aryl, heteroaryl, aralkyl, and heteroaralkyl. Representative acyl groups include acetyl, benzoyl, and malonyl.

The term "$N^2$-acetylhydrazinecarbonyl" as used herein refers to a group of the formula: Ac-NH-NH-C(=O)- that occurs, *e.g.*, in the molecular formula of Example 23 below, where Ac represents acetyl.

The term "aminoalkyl" as used herein refers to an alkyl group substituted with one or more one amino groups. In one embodiment, the term "aminoalkyl" refers to an aminomethyl group.

The term "aminoacyl" is a term of art and as used herein refers to an acyl group substituted with one or more amino groups.

The term "aminothionyl" as used herein refers to an analog of an aminoacyl in which the O of RC(=O)- has been replaced by sulfur, hence is of the form RC(=S)-.

The term "azide" or "azido", as used herein, means an $-N_3$ group.

The term "carbonyl" as used herein refers to -C(=O)-.

The term "thiocarbonyl" as used herein refers to -C(=S)-.

The term "alkylthio" as used herein refers to alkyl-S-. Representative examples of $C_1$-$C_6$ alkylthio include methylthio, ethylthio, *n*-propylthio, and *tert*-butylthio. Representative examples of $C_1$-$C_3$ alkylthio include methylthio, ethylthio, and *n*-propylthio.

The term "alkylsulfinyl" as used herein refers to alkyl-S(=O)-. Here, the sulfur atom may have three different substituents and a lone pair of electrons, thus making sulfur a center of chirality and giving rise to a pair of optically stable epimers. Representative examples of $C_1$-$C_3$ alkylsulfinyl include methylsulfinyl, ethylsulfinyl, and n-propylsulfinyl, in the form of either one or both of their epimers.

The term "alkylsulfonyl" as used herein refers to alkyl-$S(=O)_2$-. Representative examples of $C_1$-$C_3$ alkylsulfonyl include methylsulfonyl (also termed methanesulfonyl), ethylsulfonyl, and *n*-propylsulfonyl.

The term "mercaptoalkyl" as used herein refers to an alkyl group substituted by an -SH moiety. Representative examples of $C_1$-$C_6$ mercaptoalkyl include mercaptomethyl, mercaptoethyl, and mercapto-*n*-propyl.

The term "carboxy" as used herein refers to a -C(=O)-O- moiety, forming a portion of another substituents, such as alkylaminocarboxymethyl, or a portion of carboxylic acid esters. There is an exception to this convention in the case of

functionalized cellulose derivatives, such as carboxymethyl cellulose, wherein "carboxymethyl" denotes $HO_2C-CH_2-$.

The terms "carboxyl", and "hydroxycarbonyl", as used herein, refer to a $-CO_2H$ group. This group can form a portion of another substituent, such as hydroxycarbonylmethyl, *i.e.*, $HO_2C-CH_2-$.

The term "aryl" is a term of art and as used herein refers to include monocyclic, bicyclic, and polycyclic aromatic hydrocarbon groups, for example, benzene, naphthalene, 1,2,3,4-tetrahydronaphthalene, indene, 2,3-dihydroindene, and pyrene. The aromatic ring may be substituted at one or more ring positions with one or more substituents, such as halogen, azide, alkyl, aralkyl, alkenyl, alkynyl, cycloalkyl, cycloalkylalkyl, (cycloalkyl)alkoxyl, hydroxyl, alkoxyl, amino, nitro, sulfhydryl, imino, carboxamido, phosphonate, phosphinate, carbonyl, carboxyl, silyl, ether, alkylthio, sulfonyl, aminosulfonyl, sulfonamido, ketone, aldehyde, ester, heterocyclyl, heterocyclylalkyl, aromatic or heteroaromatic moieties, aminoalkyl, haloalkyl, fluoroalkyl (such as trifluoromethyl), haloalkoxyl, cyano, or the like. The term "aryl" also includes polycyclic ring systems having two or more cyclic rings in which two or more carbons are common to two adjoining rings (the rings are "fused rings") wherein at least one of the rings is an aromatic hydrocarbon, *e.g.*, the other cyclic rings may be cycloalkyls, cycloalkenyls, cycloalkynyls, aryls, heteroaryls, and/or heterocycloalkyls. Representative examples of the polycyclic aryl ring systems include, but are not limited to, azulenyl, naphthyl, dihydroinden-1-yl, dihydroinden-2-yl, dihydroinden-3-yl, dihydroinden-4-yl, 2,3-dihydroindol-4-yl, 2,3-dihydroindol-5-yl, 2,3-dihydroindol-6-yl, 2,3-dihydroindol-7-yl, inden-1-yl, inden-2-yl, inden-3-yl, inden-4-yl, dihydronaphthalen-2-yl, dihydronaphthalen-3-yl, dihydronaphthalen-4-yl, dihydronaphthalen-1-yl, 5,6,7,8-tetrahydronaphthalen-1-yl, 5,6,7,8-tetrahydronaphthalen-2-yl, 2,3-dihydrobenzofuran-4-yl, 2,3-dihydrobenzofuran-5-yl, 2,3-dihydrobenzofuran-6-yl, 2,3-dihydrobenzofuran-7-yl, benzo[*d*][1,3]dioxol-4-yl, benzo[*d*][1,3]dioxol-5-yl, 2*H*-chromen-2-on-5-yl, 2*H*-chromen-2-on-6-yl, 2*H*-chromen-2-on-7-yl, 2*H*-chromen-2-on-8-yl, isoindoline-1,3-dion-4-yl, isoindoline-1,3-dion-5-yl, inden-1-on-4-yl, inden-1-on-5-yl, inden-1-on-6-yl, inden-1-on-6-yl, 2,3-dihydrobenzo[*b*][1,4]dioxan-5-yl, 2,3-dihydrobenzo[*b*][1,4]dioxan-6-yl, 2*H*-benzo[*b*][1,4]-oxazin3(4*H*)-on-5-yl, 2*H*-benzo[*b*][1,4]oxazin3(4*H*)-on-6-yl, 2*H*-benzo[*b*][1,4]oxazin3(4*H*)-on-7-yl, 2H-benzo[*b*][1,4]oxazin3(4*H*)-on-8-yl, benzo[*d*]oxazin-2(3*H*)-on-5-yl, benzo[*d*]oxazin-2(3*H*)-on-6-yl, benzo[*d*]oxazin-2(3*H*)-on-7-yl, benzo[*d*]oxazin-2(3*H*)-on-8-yl, quinazolin-4(3*H*)-on-5-yl, quinazolin-4(3*H*)-on-6-yl, quinazolin-4(3*H*)-on-7-yl, quinazolin-4(3*H*)-on-8-yl, quinoxalin-2(1*H*)-on-5-yl, quinoxalin-2(1*H*)-on-6-yl, quinoxalin-2(1*H*)-on-7-yl, quinoxalin-2(1*H*)-on-8-yl, benzo[*d*]thiazol-2(3*H*)-on-4-yl, benzo[*d*]thiazol-2(3*H*)-on-5-yl, benzo[*d*]thiazol-2(3*H*)-on-6-yl, and, benzo[*d*]thiazol-2(3*H*)-on-7-yl. In certain embodiments, the bicyclic aryl is (i) naphthyl, or (ii) a phenyl ring fused to either a 5 or 6 membered monocyclic cycloalkyl, a 5 or 6 membered monocyclic cycloalkenyl, or a 5 or 6 membered monocyclic heterocyclyl, wherein the fused cycloalkyl, cycloalkenyl, and heterocyclyl groups are optionally substituted. In certain embodiments, the term "aryl" refers to $C_6$-$C_{10}$ aryl. In certain embodiments, the term "aryl" refers to a phenyl group or a naphthyl group.

The term "heteroaryl" is a term of art and as used herein refers to a monocyclic or bicyclic aromatic group having 3 to 14, 5 to 14, 3 to 12, or 3 to 10 total atoms (other than hydrogen) including one or more heteroatoms such as nitrogen, oxygen, or sulfur in the ring structure. More preferred heteroaryl groups have from 5-10 ring members where from 1-4 of the ring members are heteroatoms selected from the group consisting of O, N, and S. Exemplary heteroaryl groups include, for example, azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazo-pyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-*d*]pyrimidinyl, pyrazolo[3,4-*d*]pyrimidinyl, quinolinyl, quinazolinyl, triazolyl, thiazolyl, thiophenyl, tetrahydroindolyl, tetrazolyl, thiadiazolyl, thienyl, thiomorpholinyl, triazolyl or tropanyl, and the like.

Exemplary "5- or 6-membered monocyclic heteroaryl" groups include, for example, pyrrolyl, furyl, thiophenyl, imidazolyl, oxazolyl, isoxazolyl, thiazolyl, isothiazolyl, triazolyl, dithiazolyl, oxadiazolyl, tetrazolyl, pyridyl, pyranyl, diazinyl, oxazinyl, thiazinyl, triazinyl, tetrazinyl, where the pattern of heteroatoms within the heteroaryl ring is any allowable (such as in 1,2,3-triazine, 1,2,4-triazine, or 1,3,5-triazine), and the attachment point of the heteroaryl group to the rest of the molecule is at any allowable carbon atom being the member of said heteroaryl group (such as in 2-thiophenyl or 3-thiophenyl).

Any heteroaryl can be optionally substituted at one or more ring positions with one or more substituents such as halogen, azide, alkyl, aralkyl, alkenyl, alkynyl, cycloalkyl, hydroxyl, alkoxyl, amino, nitro, sulfhydryl, imino, carboxamido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, fluoroalkyl (such as trifluromethyl), cyano, or the like. The term "heteroaryl" also includes polycyclic ring systems having two or more cyclic rings in which two or more carbons are common to two adjoining rings (the rings are "fused rings") wherein at least one of the rings is an aromatic group having one or more heteroatoms in the ring structure, *e.g.*, the other cyclic rings may be cycloalkyls, cycloalkenyls, cycloalkynyls, aryls, heteroaryls, and/or heterocyclyls. Representative examples of bicyclic heteroaryl include, but are not limited to, benzimidazolyl, benzofuranyl, benzothienyl, benzoxadiazolyl, benzoxathiadiazolyl, benzothiazolyl, cinnolinyl, 5,6-dihydroquinolin-2-yl, 5,6-dihydroisoquinolin-1-yl, furopyridinyl, indazolyl, indolyl, isoquinolinyl, naphthyridinyl, quinolinyl, purinyl, 5,6,7,8-tetrahydroquinolin-2-yl, 5,6,7,8-tetrahydroquinolin-3-yl, 5,6,7,8-tetrahydroquinolin-4-yl, 5,6,7,8-tetrahydroiso-quinolin-1-yl, thienopyridinyl, 4,5,6,7-tetrahydrobenzo[*c*][1,2,5]oxadiazolyl, and 6,7-dihydro-benzo[*c*][1,2,5]oxadiazol-4(5*H*)-onyl. Any heteroaryl or bicyclic heteroaryl can be optionally substituted as detailed below.

The term "aralkyl", or "arylalkyl", for example "aryl-$C_1$-$C_6$ alkyl", is a term of art and as used herein refers to an alkyl group, for example a $C_1$-$C_6$ alkyl group, substituted with an aryl group, wherein the moiety is appended to the parent molecule through the alkyl group. An example of aralkyl is the benzyl group, *i.e.,* the phenyl-methyl- group.

The term "arylene" is art-recognized, and as used herein pertains to a diradical obtained by removing two hydrogen atoms of an aryl group, as defined above. An exemplary arylene group is 1,4-phenylene. Another exemplary arylene group is benzo, or 1,2-phenylene, of the following structural formula:

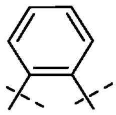.

The term "heteroaralkyl", "heteroarylalkyl", for example, "heteroaryl-$C_1$-$C_6$ alkyl" is a term of art and as used herein refers to an alkyl group, for example a $C_1$-$C_6$ alkyl group, substituted with a heteroaryl group, appended to the parent molecular moiety through the alkyl group.

The term "alkoxy" or "alkoxyl" as used herein means an alkyl group, as defined herein, appended to the parent molecular moiety through an oxygen atom. Preferably, the alkoxy group is $C_1$-$C_6$ alkoxy. Representative examples include, but are not limited to, methoxy, ethoxy, propoxy, 2-propoxy, butoxy, *tert*-butoxy, pentyloxy, and hexyloxy. Representative examples of $C_1$-$C_3$ alkoxy include methoxy, ethoxy, and propoxy.

The term "alkoxycarbonyl" means an alkoxy group, as defined herein, appended to the parent molecular moiety through a carbonyl group, represented by -C(=O)-, as defined herein. Representative examples of alkoxycarbonyl include, but are not limited to, methoxycarbonyl, ethoxycarbonyl, and *tert*-butoxycarbonyl. Alkoxycarbonyl can form a portion of another moiety, *e.g.*, methoxycarbonylmethyl.

The term "alkylcarbonyl", as used herein, means an alkyl group, as defined herein, appended to the parent molecular moiety through a carbonyl group, as defined herein. Representative examples of alkylcarbonyl include, but are not limited to, acetyl, 1-oxopropyl, 2,2-dimethyl-1-oxopropyl, 1-oxobutyl, and 1-oxopentyl.

The term "arylcarbonyl", as used herein, means an aryl group, as defined herein, appended to the parent molecular moiety through a carbonyl group, as defined herein. Representative examples of arylcarbonyl include, but are not limited to, benzoyl and (2-naphthyl)carbonyl.

The term "alkylcarbonyloxy" and "arylcarbonyloxy", as used herein, means an alkylcarbonyl or arylcarbonyl group, as defined herein, appended to the parent molecular moiety through an oxygen atom. Representative examples of alkylcarbonyloxy include, but are not limited to, acetyloxy, ethylcarbonyloxy, and *tert*-butylcarbonyloxy. Representative examples of arylcarbonyloxy include, but are not limited to phenylcarbonyloxy.

The term "alkenoxy" or "alkenoxyl" means an alkenyl group, as defined herein, appended to the parent molecular moiety through an oxygen atom. Representative examples of alkenoxy include, but are not limited to, 2-propen-1-oxy (*i.e.*, $CH_2$=CH-$CH_2$-O-) and vinyloxy (*i.e.*, $CH_2$=CH-O-).

The term "aryloxy" as used herein means an aryl group, as defined herein, appended to the parent molecular moiety through an oxygen atom.

The term "heteroaryloxy" as used herein means a heteroaryl group, as defined herein, appended to the parent molecular moiety through an oxygen atom.

The terms "cyano" and "nitrile" are terms of art and as used herein refers to -CN.

The term "nitro", as used herein, means $-NO_2$.

The term "halo" or "halogen" is a term of art and as used herein refers to -F, -Cl, -Br, or -I.

The term "haloalkyl" as used herein refers to an alkyl group, as defined herein, wherein one or more or all of the hydrogens are replaced with halogen atoms. The term "haloalkoxy" refers to an alkoxy group, as defined herein, wherein one or more or all of the hydrogens are replaced with halogen atoms. An exemplary $C_1$-$C_6$ haloalkyl group is trifluoromethyl.

The term "hydroxy" or "hydroxyl" is a term of art and as used herein refers to -OH.

The term "hydroxyalkyl", as used herein, means at least one hydroxy group, as defined herein, is appended to the parent molecular moiety through an alkyl group, as defined herein. Representative examples of $C_1$-$C_6$ hydroxyalkyl include, but are not limited to, hydroxymethyl, 2-hydroxyethyl, 3-hydroxypropyl, and 2,3-dihydroxypentyl.

The term "sulfonyl", as used herein, refers to the group $-S(=O)_2-$ that may form a portion of larger moieties, such as methanesulfonyl or *p*-toluenesulfonyl.

The term "silyl", as used herein, includes hydrocarbyl derivatives of the silyl ($H_3Si$-) group (*i.e.*, $(hydrocarbyl)_3Si$-), wherein a hydrocarbon radicals are univalent groups formed by removing a hydrogen atom from a hydrocarbon, *e.g.*, ethyl, phenyl. The hydrocarbon radicals can be combinations of differing groups which can be varied in order to provide a number of silyl groups, such as trimethylsilyl (TMS), *tert*-butyldiphenylsilyl (TBDPS), *tert*-butyldimethylsilyl (TBS/TBDMS), triisopropylsilyl (TIPS), and [2-(trimethylsilyl)ethoxy]methyl (SEM).

The term "silyloxy", as used herein, means a silyl group, as defined herein, is appended to the parent molecule through an oxygen atom.

The term "PEG", is a term of art and, as used herein, refers to a poly(ethylene glycol) group. The number appearing after PEG denotes the number of repeating ethylene glycol units. PEG may appear as a part of a substituent attached to the molecule in question. For example, a full structural formula of the biotin-attaching substituent, such as:

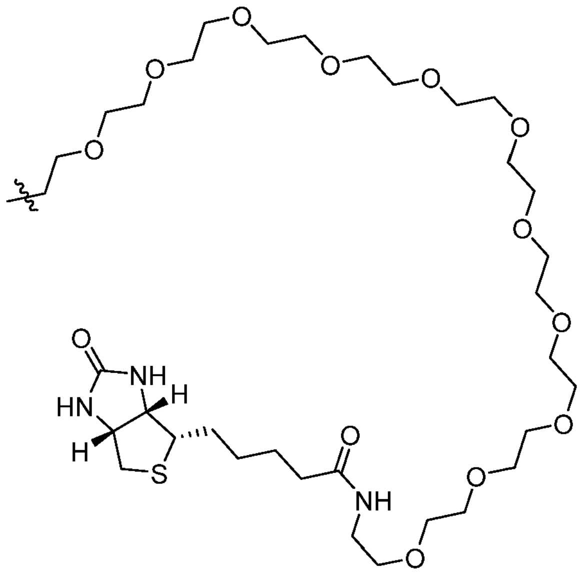

may be drawn as:

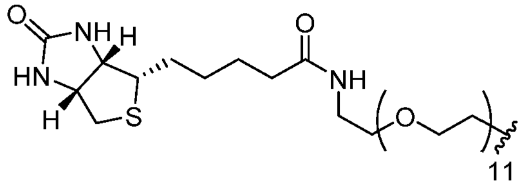

or even written as biotinyl-$PEG_{11}$- for brevity.

The term "asialofetuin", as used herein, means a glycoprotein with three asparagine-linked triantennary complex carbohydrate chains and terminal *N*-acetylgalactosamine residues. Asialofetuin is known to bind selectively to ASGP-R (asialoglycoprotein receptor) and some galectins.

The term "small molecule protein degrader", as used herein, means a bifunctional molecule comprised of three components: i) a ligand for a target protein of interest, ii) a ligand that binds to protein involved in protein degradation system (*i.e.*, an E3 ubiquitin ligase ligand or ligand of asialoglycoprotein receptors), and iii) a linker. Formation of a complex between the target protein and the protein involved in proteasome machinery, aided by a small molecule degrader, induces degradation of the target protein and down regulates its level.

Certain compounds contained in compositions of the present invention may exist in particular geometric or stereoisomeric forms. In addition, compounds of the present invention may also be optically active. The present invention contemplates all such compounds, including *cis*- and *trans*-isomers, (*R*)- and (*S*)-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

The term "racemic mixture" refers to a mixture containing equal proportions of the first enantiomer of the molecule and of the second enantiomer of this molecule, wherein the second enantiomer is the mirror image of the first one.

The term "scalemic mixture" refers to any non-racemic mixture of stereoisomers of the molecule.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

Organic compounds frequently occur in more than one crystalline form, that can differ in their physical and biological properties, such as melting point, stability, solubility, bioavailability. Such crystalline forms are termed polymorphs. All polymorphs of the inventive compounds of formula (I) and of their salts are intended to be within the scope of this invention.

Since many chemical elements can occur as isotopes, their abundance in the molecule of the inventive compound of formula (I) may be identical as in the nature or altered. Some isotopes exhibit different spectral or biological properties, and this phenomenon may be used for analysis of distribution and metabolism of drugs in the body of the recipient. All forms of the compounds of formula (I), both having a natural or unnatural abundance of isotopes of any of their constituent elements are intended to be within the scope of this invention.

It will be understood that "substitution" or "substituted with" includes the implicit proviso that such substitution is in accordance with permitted valence of the substituted atom and the substituent, and that the substitution results in a stable compound, *e.g.*, which does not spontaneously undergo transformation such as by rearrangement, fragmentation, decomposition, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, halogen, azide, alkyl, aralkyl, alkenyl, alkynyl, cycloalkyl, cycloalkylalkyl, (cycloalkyl)alkoxyl, hydroxyl, alkoxyl, amino, nitro, sulfhydryl, imino, carboxamido, phosphonate, phosphinate, carbonyl, carboxyl, silyl, ether, alkylthio, sulfonyl, aminosulfonyl, sulfonamido, ketone, aldehyde, ester, heterocycloalkyl, heterocycloalkylalkyl, (heterocycloalkyl)alkoxyl, aromatic or heteroaromatic moieties, aminoalkyl, haloalkyl, fluoroalkyl (such as trifluoromethyl), haloalkoxyl, cyano, or other substituents described above. The permissible substituents may be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

The phrase "protecting group," as used herein, means temporary substituents which protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P.G.M. Protective Groups in Organic Synthesis, 2nd ed.; Wiley: New York, 1991). Protected forms of the inventive compounds are included within the scope of this invention.

A "saturated" or "fully saturated" compound means that the referenced chemical structure does not contain any multiple carbon-carbon bonds. For example, a saturated cycloalkyl group as defined herein includes cyclohexyl, cyclopropyl, and the like.

A "unsaturated" or "partially saturated" compound means that the referenced chemical structure may contain one or more multiple carbon-carbon bonds, but is not aromatic. For example, a unsaturated cycloalkyl group as defined herein includes cyclohexenyl, cyclopentenyl, cyclohexadienyl, and the like.

For purposes of the invention, the chemical elements are identified in accordance with the Periodic Chart of the Elements, IUPAC version, The Merck Index, Twelfth Edition, 1996, inside cover.

Other chemistry terms herein are used according to conventional usage in the art, as exemplified by The McGraw-Hill Dictionary of Chemical Terms (ed. Parker, S., 1985), McGraw-Hill, San Francisco). Unless otherwise defined, all technical and scientific terms used herein have the same meaning as commonly understood by one of ordinary skill in the art to which this invention pertains.

It will be apparent to one skilled in the art that certain compounds of this disclosure may exist in tautomeric forms, all such tautomeric forms of the compounds being within the scope of the disclosure.

Unless otherwise stated, structures depicted herein include all stereochemical configurations consistent with the depicted structure. For example, (i) a structure with a single stereocenter encompasses both the *R* and *S* configurations at the stereocenter and mixtures thereof, including racemic mixtures, and (ii) in a structure with two or more stereocenters, any wedged and dashed bonds show relative stereochemistry unless otherwise noted, such that the structure encompasses the individual enantiomers of the depicted compound and mixtures thereof, including racemic mixtures. Therefore, single stereochemical isomers as well as enantiomeric and diastereomeric mixtures of the present compounds are within the scope of the disclosure. Both the R and the S stereochemical isomers, as well as all mixtures thereof, are included within the scope of the disclosure. Similarly, indications of stereochemistry in chemical structures with two or more chiral centers convey relative stereochemistry unless otherwise defined.

The chemical structures of examples that are a mixture of diastereoisomers or a single diastereoisomer but with unknown relative configuration are drawn and named without specifying defined stereochemical configuration.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or

complication, commensurate with a reasonable benefit/risk ratio.

The term "pharmaceutically acceptable salt" as used herein includes salts derived from inorganic or organic acids including, for example, hydrochloric, hydrobromic, sulfuric, nitric, perchloric, phosphoric, formic, acetic, lactic, maleic, fumaric, succinic, tartaric, glycolic, salicylic, citric, methanesulfonic, benzenesulfonic, benzoic, malonic, trifluoroacetic, trichloroacetic, naphthalene-2-sulfonic, and other acids. Pharmaceutically acceptable salt forms can include forms wherein the ratio of molecules comprising the salt is not 1:1. For example, the salt may comprise more than one inorganic or organic acid molecule per molecule of base, such as two hydrochloric acid molecules per molecule of compound of Formula I. As another example, the salt may comprise less than one inorganic or organic acid molecule per molecule of base, such as two molecules of compound of Formula I per molecule of tartaric acid.

As used herein, a protic solvent is a solvent that has a hydrogen atom bound to an oxygen (as in a hydroxyl group) or a nitrogen (as in an amine group). In general terms, any solvent that contains labile $H^+$ is called a protic solvent. The molecules of such solvents readily donate protons ($H^+$) to reagents. In contrast, an aprotic solvent is a solvent that does not have a hydrogen atom bound to an oxygen (as in a hydroxyl group) or a nitrogen (as in an amine group), and it cannot donate hydrogen.

As used herein, a polar protic solvent is a protic solvent that will dissolve many salts. In general, these solvents have high dielectric constants and high polarity. Non-limiting examples of polar protic solvents include acetic acid, ammonia, ethanol, formic acid, isopropanol, methanol, *n*-butanol, nitromethane, *n*-propanol, *tert*-butanol, and water.

As used herein, a polar aprotic solvent is a solvent that will dissolve many salts, but lacks an acidic hydrogen; these solvents generally have intermediate to high dielectric constants and polarity. Non-limiting examples of polar aprotic solvents include acetone, acetonitrile, dichloromethane (DCM), dimethyl sulfoxide (DMSO), ethyl acetate, hexamethylphosphoric triamide (HMPT), *N,N*-dimethylformamide (DMF), and tetrahydrofuran (THF).

As used herein, a nonpolar aprotic solvent is a solvent that will dissolve many salts, but lacks an acidic hydrogen; these solvents generally have low dielectric constants and polarity. Non-limiting examples of nonpolar aprotic solvents include benzene, chloroform, cyclohexane, diethyl ether, hexane, pentane, and toluene.

Many organic compounds form stable crystals that incorporate solvent molecules into the crystalline structure, without chemical alteration of the organic molecule. Such solvated compounds are called solvates in general, and they are called hydrates when the solvent is water.

A physician or veterinarian having ordinary skill in the art can readily determine and prescribe the therapeutically effective amount of the pharmaceutical composition required. For example, the physician or veterinarian could start doses of the pharmaceutical composition or compound at levels lower than that required in order to achieve the desired therapeutic effect and gradually increase the dosage until the desired effect is achieved. By "therapeutically effective amount" is meant the concentration of a compound that is sufficient to elicit the desired therapeutic effect. It is generally understood that the effective amount of the compound will vary according to the weight, sex, age, and medical history of the subject. Other factors which influence the effective amount may include, but are not limited to, the severity of the patient's condition, the disorder being treated, the stability of the compound, the mode of administration, the bioavailability of the particular compound, and, if desired, another type of therapeutic agent being administered with the compound of the invention. A larger total dose can be delivered by multiple administrations of the agent. Methods to determine efficacy and dosage are known to those skilled in the art (Isselbacher et al. (1996) Harrison's Principles of Internal Medicine 13 ed., 1814-1882)

"Modulating" or "modulate" refers to the treating, prevention, suppression, enhancement or induction of a function, condition or disorder.

The term "treating" includes prophylactic and/or therapeutic treatments. The term "prophylactic or therapeutic" treatment is art-recognized and includes administration to the host of one or more of the subject compositions. If it is administered prior to clinical manifestation of the unwanted condition (*e.g.*, disease or other unwanted state of the host animal) then the treatment is prophylactic (*i.e.*, it protects the host against developing the unwanted condition), whereas if it is administered after manifestation of the unwanted condition, the treatment is therapeutic, (*i.e.*, it is intended to diminish, ameliorate, or stabilize the existing unwanted condition or side effects thereof). In certain embodiments, the methods of the invention are for therapeutically treating.

As used herein, "subject" refers to a warm blooded animal such as a mammal, preferably a human, or a human child, which is afflicted with, or has the potential to be afflicted with one or more diseases and disorders described herein.

"$EC_{50}$" refers to a dosage, concentration or amount of a particular test compound that elicits a dose-dependent response at 50% of maximal expression of a particular response that is induced, provoked or potentiated by the particular test compound.

"$IC_{50}$" refers to an amount, concentration or dosage of a particular test compound that achieves a 50% inhibition of a maximal response in an assay that measures such response.

"Kd", expressed in molar units (M) refers to the concentration of analyzed compound/probe at which 50% of ligand binding sites of protein are occupied at equilibrium, or the concentration at which the number of ligand molecules bound to target protein equals the number of ligand molecules that is not bound.

*Compounds of the Invention*

In one aspect, this invention concerns a novel compound represented by structural formula (I),

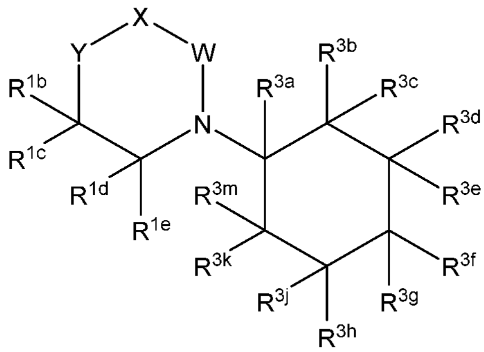

(I)

wherein:

W represents $-C(-R^{1f})(-R^{1g})-$;
X represents $-C(-R^{1h})(-R^{1j})-$;
Y represents $-C(-R^{1k})(-R^{1m})-$, $-N(-R^{1k})-$, or -O-;
$R^{1b}$ represents -H;
$R^{1c}$ represents -H;
$R^{1d}$ represents 4-$R^{10}$-benzyl;
$R^{1e}$ represents -H;
$R^{1f}$ represents -H;
$R^{1g}$ represents -H;
$R^{1h}$ is selected from the group consisting of $C_1$-$C_6$ alkyl, $C_2$-$C_6$ alkenyl, $C_2$-$C_6$ alkynyl, hydroxy-$C_1$-$C_6$ alkyl, azido-$C_1$-$C_6$ alkyl, hydroxycarbonyl-$C_1$-$C_3$ alkoxy-$C_1$-$C_6$ alkyl, $C_3$-$C_6$ alkynyloxy-$C_1$-$C_6$ alkyl, halo-$C_1$-$C_6$ alkyl, $C_1$-$C_3$ alkylthio-$C_1$-$C_6$ alkyl, $C_1$-$C_3$ alkylsulfinyl-$C_1$-$C_6$ alkyl, $C_1$-$C_3$ alkylsulfonyl-$C_1$-$C_6$ alkyl, hydroxycarbonyl-$C_1$-$C_3$ alkylsulfonyl-$C_1$-$C_6$ alkyl, hydroxycarbonyl-$C_1$-$C_6$ alkyl, $C_1$-$C_3$ alkylcarbonyl-$C_1$-$C_6$ alkyl, $C_1$-$C_3$ alkylaminocarbonyl-$C_1$-$C_6$ alkyl, di($C_1$-$C_3$ alkyl)aminocarbonyl-$C_1$-$C_6$ alkyl, $C_1$-$C_3$ alkylsulfonylamino-$C_1$-$C_6$ alkyl, halo-$C_1$-$C_3$ alkylsulfonylamino-$C_1$-$C_6$ alkyl, 5- or 6-membered monocyclic heteroaryl-carbonylamino-$C_1$-$C_6$ alkyl, 5- or 6-membered monocyclic heteroaryl-$C_1$-$C_3$ alkoxy-$C_1$-$C_6$ alkyl, 5- or 6-membered monocyclic heteroaryl-$C_1$-$C_6$ alkyl, hydroxycarbonyl, $C_1$-$C_3$ alkoxycarbonyl, aminocarbonyl, $C_1$-$C_3$ alkylaminocarbonyl, di($C_1$-$C_3$ alkyl)aminocarbonyl, $C_3$-$C_6$ alkynylaminocarbonyl, hydroxycarbonyl-$C_1$-$C_3$ alkyl($C_1$-$C_3$ alkyl)aminocarbonyl, (biotinyl-$PEG_{11}$)aminocarbonyl, (biotinyl-$PEG_{11}$)aminocarbonyl-$C_1$-$C_3$ alkylsulfonyl-$C_1$-$C_6$ alkyl, hydroxylaminocarbonyl, $N^2$-acetylhydrazinecarbonyl, (5- or 6-membered monocyclic heteroaryl)-$C_1$-$C_3$ alkylaminocarbonyl, (5- or 6-membered monocyclic heteroaryl) aminocarbonyl, $C_1$-$C_3$ alkylsulfonyl, 5- or 6-membered monocyclic heteroaryl, (3-azabicyclo[3.1.0]hexan-3-yl)-$C_1$-$C_3$ alkyl, (4-, 5-, or 6-membered heterocyclyl)amino-$C_1$-$C_3$ alkyl, hydroxycarbonyl-$C_1$-$C_6$ alkyl-(5- or 6-membered monocyclic heteroaryl), $R^{20}$, and 5-(2-(2,6-dioxopiperidin-3-yl)-3-oxoisoindolin-4-yl)pentyl,
where any available position of any heteroaryl ring in $R^{1h}$ can be substituted with a substituent selected from the group consisting of $C_1$-$C_6$ alkyl, $C_3$-$C_6$ alkynyl, halo, trifluoromethyl, 2,2,2-trifluoroethyl, biotinyl-$PEG_{11}$, and any available position of any carbon chain in $R^{1h}$ excluding heteroaryl rings can be substituted with a substituent selected from halogen, amino, hydroxy, and oxo;
$R^{1j}$ represents -H;
$R^{1k}$ represents -H;
$R^{1m}$ represents -H;
or $R^{1k}$ and $R^{1h}$ taken along with nitrogen and carbon atoms bearing them, respectively, represent a 6-membered heterocyclic ring of structural formula (II):

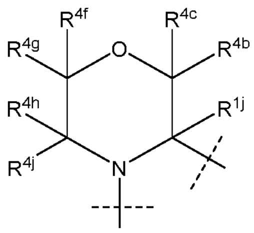

(II)

$R^{3a}$ represents -H;
$R^{3b}$ represents -H;
$R^{3c}$ represents -H;
$R^{3d}$ represents -H;
$R^{3e}$ represents -H;
$R^{3f}$ is selected from the group consisting of 1-methyl-1*H*-imidazol-4-yl, 1,2-dimethyl-1*H*-imidazol-4-yl, 5-methyloxazol-2-yl, 4,5-dimethyloxazol-2-yl, 1*H*-pyrazol-1-yl, 1-methyl-1*H*-pyrazol-3-yl, 4-methyl-1*H*-pyrazol-1-yl, 4-fluoro-1*H*-pyrazol-1-yl, 1-isopropyl-1*H*-pyrazol-3-yl, 5-methyl-1*H*-pyrazol-3-yl, 1,5-dimethyl-1*H*-pyrazol-3-yl, 1-ethyl-5-methyl-1*H*-pyrazol-3-yl, 1,5-dimethyl-1*H*-pyrazol-3-yl, 1,5-dimethyl-1*H*-pyrazol-4-yl, 1-methyl-5-(trifluoromethyl)-1*H*-pyrazol-3-yl, 1-methyl-2-cyano-1*H*-pyrrol-4-yl, 1*H*-tetrazol-5-yl, 2-methyl-2*H*-tetrazol-5-yl, 5-methyl-1,3,4-thiadiazol-2-yl, 5-methylthiazol-2-yl, 4,5-dimethylthiophen-2-yl, 1*H*-1,2,4-triazol-1-yl, 1-methyl-1*H*-1,2,3-triazol-4-yl, 1-methyl-1*H*-1,2,4-triazol-3-yl, 2-methyl-2*H*-1,2,3-triazol-4-yl, 1,5-dimethyl-1*H*-1,2,4-triazol-3-yl, 5-hydroxy-1-methyl-1*H*-1,2,4-triazol-3-yl, and 5-methoxy-1-methyl-1*H*-1,2,4-triazol-3-yl;
$R^{3g}$ represents -H;
$R^{3h}$ represents -H;
$R^{3j}$ represents -H;
$R^{3k}$ represents -H;
$R^{3m}$ represents -H;
or $R^{3d}$ and $R^{3g}$ taken along with the single bond linking two carbon atoms bearing them represent a double bond between these two carbon atoms;
$R^{4b}$ represents -H;
$R^{4c}$ represents -H;
$R^{4f}$ represents -H;
$R^{4g}$ represents -H;
$R^{4h}$ represents -H;
$R^{4j}$ represents -H;
$R^{10}$ represents halogen or trifluoromethyl;
and $R^{20}$ represents

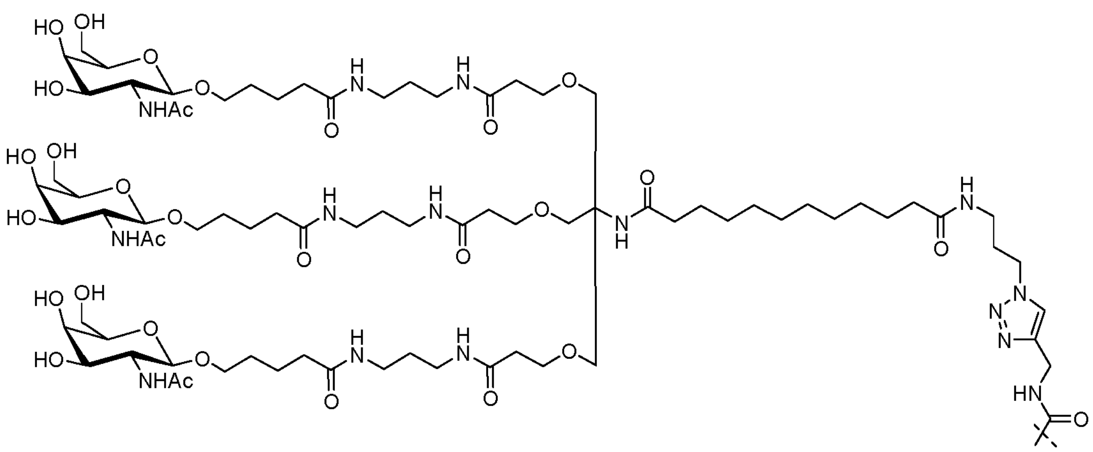

;

or a tautomer, stereoisomer, *N*-oxide, methiodide, pharmaceutically acceptable salt, solvate, or polymorph thereof.

The $R^{20}$ substituent is an asialoglycoprotein receptor ligand containing a linker with amide and triazole groups.
Preferably, this invention concerns a novel compound of structural formula (I),

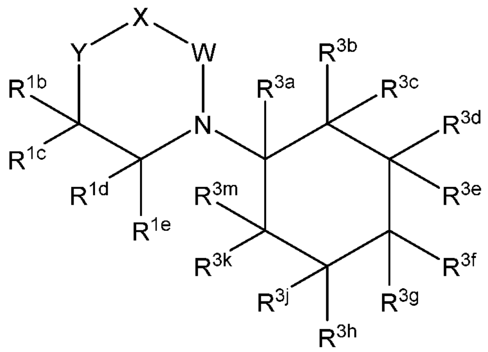

(I)

wherein:

W represents -C(-$R^{1f}$)(-$R^{1g}$)-;
X represents -C(-$R^{1h}$)(-$R^{1j}$)-;
Y represents -C(-$R^{1k}$)(-$R^{1m}$)-, -N(-$R^{1k}$)-, or -O-;
$R^{1b}$ represents -H;
$R^{1c}$ represents -H;
$R^{1d}$ represents 4-$R^{10}$-benzyl;
$R^{1e}$ represents -H;
$R^{1f}$ represents -H;
$R^{1g}$ represents -H;
$R^{1h}$ is selected from the group consisting of $C_1$-$C_6$ alkyl, $C_2$-$C_6$ alkenyl, $C_2$-$C_6$ alkynyl, hydroxy-$C_1$-$C_6$ alkyl, azido-$C_1$-$C_6$ alkyl, hydroxycarbonyl-$C_1$-$C_3$ alkoxy-$C_1$-$C_6$ alkyl, $C_3$-$C_6$ alkynyloxy-$C_1$-$C_6$ alkyl, halo-$C_1$-$C_6$ alkyl, $C_1$-$C_3$ alkylthio-$C_1$-$C_6$ alkyl, $C_1$-$C_3$ alkylsulfinyl-$C_1$-$C_6$ alkyl, $C_1$-$C_3$ alkylsulfonyl-$C_1$-$C_6$ alkyl, hydroxycarbonyl-$C_1$-$C_3$ alkylsulfonyl-$C_1$-$C_6$ alkyl, hydroxycarbonyl-$C_1$-$C_6$ alkyl, $C_1$-$C_3$ alkylcarbonyl-$C_1$-$C_6$ alkyl, $C_1$-$C_3$ alkylaminocarbonyl-$C_1$-$C_6$ alkyl, di($C_1$-$C_3$ alkyl)aminocarbonyl-$C_1$-$C_6$ alkyl, $C_1$-$C_3$ alkylsulfonylamino-$C_1$-$C_6$ alkyl, halo-$C_1$-$C_3$ alkylsulfonylamino-$C_1$-$C_6$ alkyl, 5- or 6-membered monocyclic heteroaryl-carbonylamino-$C_1$-$C_6$ alkyl, 5- or 6-membered monocyclic heteroaryl-$C_1$-$C_3$ alkoxy-$C_1$-$C_6$ alkyl, 5- or 6-membered monocyclic heteroaryl-$C_1$-$C_6$ alkyl, hydroxycarbonyl, $C_1$-$C_3$ alkoxycarbonyl, aminocarbonyl, $C_1$-$C_3$ alkylaminocarbonyl, di($C_1$-$C_3$ alkyl)aminocarbonyl, $C_3$-$C_6$ alkynylaminocarbonyl, hydroxycarbonyl-$C_1$-$C_3$ alkyl($C_1$-$C_3$ alkyl)aminocarbonyl, (biotinyl-$PEG_{11}$)aminocarbonyl, hydroxylaminocarbonyl, *N*2-acetylhydrazinecarbonyl, (5- or 6-membered monocyclic heteroaryl)-$C_1$-$C_3$ alkylaminocarbonyl, (5- or 6-membered monocyclic heteroaryl)aminocarbonyl, $C_1$-$C_3$ alkylsulfonyl, 5- or 6-membered monocyclic heteroaryl, (3-azabicyclo[3.1.0]hexan-3-yl)-$C_1$-$C_3$ alkyl, (4-, 5-, or 6-membered heterocyclyl)amino-$C_1$-$C_3$ alkyl, and hydroxycarbonyl-$C_1$-$C_6$ alkyl-(5- or 6-membered monocyclic heteroaryl), where any available position of any heteroaryl ring in $R^{1h}$ can be substituted with a substituent selected from the group consisting of $C_1$-$C_6$ alkyl, $C_3$-$C_6$ alkynyl, halo, trifluoromethyl, 2,2,2-trifluoroethyl, biotinyl-$PEG_{11}$, and any available position of any carbon chain in $R^{1h}$ excluding heteroaryl rings can be substituted with a substituent selected from halogen, amino, hydroxy, and oxo;
$R^{1j}$ represents -H;
$R^{1k}$ represents -H;
$R^{1m}$ represents -H;
or $R^{1k}$ and $R^{1h}$ taken along with nitrogen and carbon atoms bearing them, respectively, represent a 6-membered heterocyclic ring of structural formula (II):

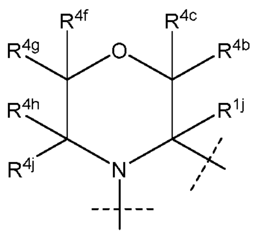

(II)

$R^{3a}$ represents -H;
$R^{3b}$ represents -H;
$R^{3c}$ represents -H;

$R^{3d}$ represents -H;
$R^{3e}$ represents -H;
$R^{3f}$ is selected from the group consisting of 5-methyloxazol-2-yl, 1,5-dimethyl-1*H*-pyrazol-3-yl, 1,5-dimethyl-1*H*-1,2,4-triazol-3-yl, 4,5-dimethyloxazol-2-yl, 5-fluoro-1-methyl-1*H*-pyrazol-3-yl, 4,5-dimethylthiazol-2-yl, and 5-methylthiazol-2-yl;
$R^{3g}$ represents -H;
$R^{3h}$ represents -H;
$R^{3j}$ represents -H;
$R^{3k}$ represents -H;
$R^{3m}$ represents -H;
or $R^{3d}$ and $R^{3g}$ taken along with the single bond linking two carbon atoms bearing them represent a double bond between these two carbon atoms;
$R^{4b}$ represents -H;
$R^{4c}$ represents -H;
$R^{4f}$ represents -H;
$R^{4g}$ represents -H;
$R^{4h}$ represents -H;
$R^{4j}$ represents -H;
and $R^{10}$ represents halogen or trifluoromethyl;
or a tautomer, stereoisomer, *N*-oxide, methiodide, pharmaceutically acceptable salt, solvate, or polymorph thereof.

The above-defined general formula (I) covers certain compounds of the invention, which can be described in more detail as follows.

In some embodiments, the compound of the invention is of structural formula (Ia)

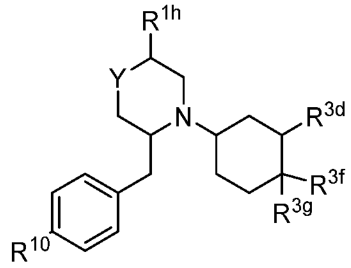

(Ia)

wherein:

Y represents $-CH_2-$, $-N(-R^{1k})-$, or -O-;
$R^{1h}$ is selected from the group consisting of methyl, ethynyl, 2-hydroxyprop-2-yl, azidomethyl, hydroxymethyl, hydroxycarbonylmethoxymethyl, (prop-2-yn-1-yloxy)methyl, fluoromethyl, methylthiomethyl, methylsulfinylmethyl, methylsulfonylmethyl, ethylsulfonylmethyl, 2-(hydroxycarbonyl)ethylsulfonylmethyl, hydroxycarbonylmethyl, methoxycarbonylmethyl, ethylaminocarbonylmethyl, methylsulfonylaminomethyl, trifluoromethylsulfonylaminomethyl, (1*H*-pyrazol-3-yl)carbonylaminomethyl, 1-(trifluoromethyl)-1*H*-1,2,3-triazol-4-yl, (1-(biotinyl-$PEG_{11}$)-1*H*-1,2,3-triazol-4-yl)methoxymethyl, (2*H*-1,2,3-triazol-4-yl)carbonylaminomethyl, (1*H*-1,2,3-triazol-5-yl)carbonylaminomethyl, (1-methyl-1*H*-imidazol-4-yl)carbonylaminomethyl, (1*H*-imidazol-4-yl)carbonylaminomethyl, (1,5-dimethyl-1*H*-pyrazol-3-yl)carbonylaminomethyl, (2,5-dioxopyrrolidin-1-yl)methyl, (2,4-dioxoimidazolidin-3-yl)methyl, (1-methyl-2,4-dioxoimidazolidin-3-yl)methyl, hydroxycarbonyl, methoxycarbonyl, aminocarbonyl, methylaminocarbonyl, dimethylaminocarbonyl, ethylaminocarbonyl, isopropylaminocarbonyl, (prop-2-yn-1-yl)aminocarbonyl, hydroxycarbonylmethyl(methyl)aminocarbonyl, (biotinyl-$PEG_{11}$)aminocarbonyl, hydroxylaminocarbonyl, $N^2$-acetylhydrazinecarbonyl, (1-(biotinyl-$PEG_{11}$)-1*H*-1,2,3-triazol-4-yl)methylaminocarbonyl, (1-methyl-1*H*-imidazol-4-yl)aminocarbonyl, (1,5-dimethyl-1*H*-pyrazol-3-yl)aminocarbonyl, (2-(3-(but-3-yn-1-yl)-3*H*-diazirin-3-yl)ethyl)aminocarbonyl, methylsulfonyl, 3-methylisoxazol-5-yl, 1-(2,2,2-trifluoroethyl)-1*H*-1,2,3-triazol-4-yl, 5-methyl-1,3,4-oxadiazol-2-yl, (6,6-dimethyl-2,4-dioxo-3-azabicyclo[3.1.0]hexan-3-yl)methyl, (1,2-dioxo-3-hydroxy-cyclobut-3-en-4-yl)aminomethyl, (1,2-dioxo-3-amino-cyclobut-3-en-4-yl)aminomethyl, 1-(biotinyl-$PEG_{11}$)-1*H*-1,2,3-triazol-4-yl, 1-(hydroxycarbonylmethyl)-1*H*-1,2,3-triazol-4-yl, 1-(2-(hydroxycarbonyl)ethyl)-1*H*-1,2,3-triazol-4-yl, 1-(3-(hydroxycarbonyl)prop-1-yl)-1*H*-1,2,3-triazol-4-yl, 1-(4-(hydroxycarbonyl)but-1-yl)-1*H*-1,2,3-triazol-4-yl, and 5-methyl-1*H*-pyrazol-3-yl;
or $R^{1k}$ and $R^{1h}$ taken along with nitrogen and carbon atoms bearing them, respectively, represent a 6-membered heterocyclic ring of structural formula (IIa):

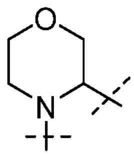

(IIa)

$R^{3d}$ represents H;
$R^{3g}$ represents H;
or $R^{3d}$ and $R^{3g}$ taken along with the single bond linking two carbon atoms bearing them represent a double bond between these two carbon atoms;
$R^{3f}$ is selected from the group consisting of 5-methyloxazol-2-yl, 1,5-dimethyl-1*H*-pyrazol-3-yl, 1,5-dimethyl-1*H*-1,2,4-triazol-3-yl, 4,5-dimethyloxazol-2-yl, 5-fluoro-1-methyl-1*H*-pyrazol-3-yl, 4,5-dimethylthiazol-2-yl, and 5-methylthiazol-2-yl;
and $R^{10}$ represents chloro, bromo, or trifluoromethyl;
or a tautomer, stereoisomer, pharmaceutically acceptable salt, solvate, or polymorph thereof.

In a preferred embodiment, the compound according to the invention is as defined above, and:

X represents -CH(-$R^{1h}$)-;
Y represents -$CH_2$-; and
$R^{1h}$ represents methanesulfonyl.

In a more preferred embodiment, the compound according to the invention is as defined above, and:

X represents -CH(-$R^{1h}$)-;
Y represents -N(-$R^{1k}$)-.
and $R^{1k}$ and $R^{1h}$ taken along with nitrogen and carbon atoms bearing them, respectively, represent a 6-membered heterocyclic ring of structural formula (IIb):

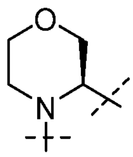

(IIb).

In another embodiment, the compound according to the invention is represented by structural formula (I) or (Ia), and Y represents -O-.

More preferably, the compound of the invention is as defined above, and $R^{1d}$ and $R^{1h}$ are in *cis* relationship.

Preferably, the compound of the invention is as defined above, and the absolute stereochemistry at the carbon atom bearing $R^{1d}$ is S.

In some embodiments, the compound of the invention is of structural formula (Ib)

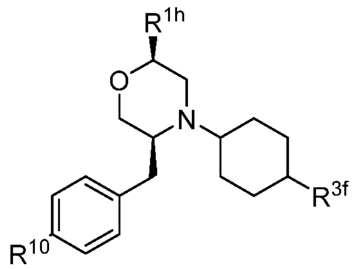

(Ib)

wherein:

$R^{1h}$ is selected from the group consisting of methyl, ethynyl, 2-hydroxyprop-2-yl, azidomethyl, hydroxymethyl, hydroxycarbonylmethoxymethyl, (prop-2-yn-1-yloxy)methyl, fluoromethyl, methylthiomethyl, methylsulfinylmethyl, methylsulfonylmethyl, ethylsulfonylmethyl, 2-(hydroxycarbonyl)ethylsulfonylmethyl, hydroxycarbonylmethyl, methoxycarbonylmethyl, ethylaminocarbonylmethyl, methylsulfonylaminomethyl, trifluoromethylsulfonylaminomethyl, (1*H*-pyrazol-3-yl)carbonylaminomethyl, 1-(trifluoromethyl)-1*H*-1,2,3-triazol-4-yl, (1-(biotinyl-$PEG_{11}$)-1*H*-1,2,3-triazol-4-yl)methoxymethyl, (2*H*-1,2,3-triazol-4-yl)carbonylaminomethyl, (1*H*-1,2,3-triazol-5-yl)carbonylaminomethyl, (1-methyl-1*H*-imidazol-4-yl)carbonylaminomethyl, (1*H*-imidazol-4-yl)carbonylaminomethyl, (1,5-dimethyl-1*H*-pyrazol-3-yl)carbonylaminomethyl, (2,5-dioxopyrrolidin-1-yl)methyl, (2,4-dioxoimidazolidin-3-yl)methyl, (1-methyl-2,4-dioxoimidazolidin-3-yl)methyl, hydroxycarbonyl, methoxycarbonyl, aminocarbonyl, methylaminocarbonyl, dimethylaminocarbonyl, ethylaminocarbonyl, isopropylaminocarbonyl, (prop-2-yn-1-yl)aminocarbonyl, (biotinyl-$PEG_{11}$)aminocarbonyl, hydroxylaminocarbonyl, $N^2$-acetylhydrazinecarbonyl, (1-(biotinyl-$PEG_{11}$)-1*H*-1,2,3-triazol-4-yl)methylaminocarbonyl, (1-methyl-1*H*-imidazol-4-yl)aminocarbonyl, (1,5-dimethyl-1H-pyrazol-3-yl)aminocarbonyl, (2-(3-(but-3-yn-1-yl)-3*H*-diazirin-3-yl)ethyl)aminocarbonyl, 3-methylisoxazol-5-yl, 1-(2,2,2-trifluoroethyl)-1*H*-1,2,3-triazol-4-yl, 5-methyl-1,3,4-oxadiazol-2-yl, (6,6-dimethyl-2,4-dioxo-3-azabicyclo[3.1.0]hexan-3-yl)methyl, (1,2-dioxo-3-hydroxy-cyclobut-3-en-4-yl)aminomethyl, (1,2-dioxo-3-amino-cyclobut-3-en-4-yl)aminomethyl, 1-(biotinyl-$PEG_{11}$)-1*H*-1,2,3-triazol-4-yl, 1-(hydroxycarbonylmethyl)-1*H*-1,2,3-triazol-4-yl, 1-(2-(hydroxycarbonyl)ethyl)-1*H*-1,2,3-triazol-4-yl, 1-(3-(hydroxycarbonyl)prop-1-yl)-1*H*-1,2,3-triazol-4-yl, 1-(4-(hydroxycarbonyl)but-1-yl)-1*H*-1,2,3-triazol-4-yl, and 5-methyl-1*H*-pyrazol-3-yl; and

$R^{3f}$ is selected from the group consisting of 5-methyloxazol-2-yl, 1,5-dimethyl-1*H*-pyrazol-3-yl, 1,5-dimethyl-1*H*-1,2,4-triazol-3-yl, 4,5-dimethyloxazol-2-yl, 5-fluoro-1-methyl-1*H*-pyrazol-3-yl, 4,5-dimethylthiazol-2-yl, and 5-methylthiazol-2-yl;

or a tautomer, stereoisomer, pharmaceutically acceptable salt, solvate, or polymorph thereof.

More preferably, the compound of the invention is as defined above, and R10 is bromo.

Alternatively, the compound of the invention is as defined above, and R10 is chloro.

In some embodiments, $R^{3f}$ is 5-methyloxazol-2-yl.

In some embodiments, $R^{3f}$ is 1,5-dimethyl-1*H*-pyrazol-3-yl.

In some embodiments, $R^{3f}$ is 1,5-dimethyl-1*H*-1,2,4-triazol-3-yl.

In some embodiments, $R^{3f}$ is 4,5-dimethyloxazol-2-yl.

In some embodiments, $R^{3f}$ is 5-fluoro-1-methyl-1*H*-pyrazol-3-yl.

In some embodiments, $R^{3f}$ is 4,5-dimethylthiazol-2-yl.

In some embodiments, $R^{3f}$ is 5-methylthiazol-2-yl.

More preferably, the compound of the invention is as defined above, and:

$R^{3a}$ represents H;

$R^{3g}$ represents H; and

the $R^{3f}$ substituent and the heterocyclic ring attached by its nitrogen ring member atom to the cyclohexane ring in 1,4 substitution pattern are in *trans* relationship.

Preferably, the invention concerns the compound of structural formula (I), that is selected from the group consisting of the following compounds:

(2*S*,5*S*)-5-(4-chlorobenzyl)-2-methyl-4-(4-(5-methyloxazol-2-yl)cyclohexyl)morpholine;
(2*S*,5*S*)-5-(4-chlorobenzyl)-4-(4-(1,5-dimethyl-1*H*-pyrazol-3-yl)cyclohexyl)-2-methylmorpholine;
(2*R*,5*S*)-5-(4-chlorobenzyl)-4-(4-(5-methyloxazol-2-yl)cyclohexyl)-2-((methylsulfonyl)methyl)morpholine;
(2*R*,5*S*)-5-(4-chlorobenzyl)-4-((1*r*,4*S*)-4-(1,5-dimethyl-1*H*-pyrazol-3-yl)cyclohexyl)-2-((methylsulfonyl)methyl)morpholine;
(2*R*,5*S*)-5-(4-chlorobenzyl)-4-(4-(1,5-dimethyl-1*H*-pyrazol-3-yl)cyclohexyl)-2-(3-methylisoxazol-5-yl)morpholine;
(7*S*,9a*R*)-7-(4-chlorobenzyl)-8-(4-(5-methyloxazol-2-yl)cyclohexyl)octahydropyrazino[2,1-*c*][1,4]oxazine;
(7*S*,9a*R*)-7-(4-chlorobenzyl)-8-(4-(1,5-dimethyl-1*H*-pyrazol-3-yl)cyclohexyl)octahydropyrazino[2,1-*c*][1,4]oxazine;
(2*R*,5*S*)-5-(4-chlorobenzyl)-4-(4-(5-methyloxazol-2-yl)cyclohexyl)morpholine-2-carboxylic acid;
methyl (2*R*,5*S*)-5-(4-chlorobenzyl)-4-(4-(1,5-dimethyl-1*H*-pyrazol-3-yl)cyclohexyl)morpholine-2-carboxylate;
(2*R*,5*S*)-5-(4-chlorobenzyl)-4-(4-(1,5-dimethyl-1*H*-pyrazol-3-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholine;
(2*R*,5*S*)-5-(4-chlorobenzyl)-4-(4-(5-methyloxazol-2-yl)cyclohexyl)-2-(1-(2,2,2-trifluoroethyl)-1*H*-1,2,3-triazol-4-yl)morpholine;
(2*R*,5*S*)-5-(4-chlorobenzyl)-4-(4-(1,5-dimethyl-1*H*-pyrazol-3-yl)cyclohexyl)-*N*-(37-oxo-41-((3a*S*,4*S*,6a*R*)-2-oxo-hexahydro-1*H*-thieno[3,4-*d*]imidazol-4-yl)-3,6,9,12,15,18,21,24,27,30,33-undecaoxa-36-azahentetracontyl)morpholine-2-carboxamide;
(2*R*,5*S*)-5-(4-chlorobenzyl)-4-(4-(1,5-dimethyl-1*H*-1,2,4-triazol-3-yl)cyclohexyl)-2-((methylsulfonyl)methyl)morpholine;
(2*R*,5*S*)-5-(4-chlorobenzyl)-4-(4-(1,5-dimethyl-1*H*-pyrazol-3-yl)cyclohexyl)-2-(fluoromethyl)morpholine;
((2*R*,5*S*)-5-(4-chlorobenzyl)-4-(4-(1,5-dimethyl-1*H*-pyrazol-3-yl)cyclohexyl)morpholin-2-yl)methanol;
(2*R*,5*S*)-5-(4-chlorobenzyl)-*N*-ethyl-4-(4-(5-methyloxazol-2-yl)cyclohexyl)morpholine-2-carboxamide;
(2*R*,5*S*)-5-(4-chlorobenzyl)-4-(4-(1,5-dimethyl-1*H*-pyrazol-3-yl)cyclohexyl)-*N*-ethylmorpholine-2-carboxamide;
(2*R*,5*S*)-5-(4-chlorobenzyl)-4-(4-(1,5-dimethyl-1*H*-pyrazol-3-yl)cyclohexyl)-*N*,*N*-dimethylmorpholine-2-carboxamide;
(2*R*,5*S*)-5-(4-chlorobenzyl)-*N,N*-dimethyl-4-(4-(5-methyloxazol-2-yl)cyclohexyl)morpholine-2-carboxamide;
(2*R*,5*S*)-5-(4-chlorobenzyl)-4-(4-(1,5-dimethyl-1*H*-pyrazol-3-yl)cyclohexyl)-2-(5-methyl-1,3,4-oxadiazol-2-yl)morpholine;
(2*R*,5*S*)-*N*'-acetyl-5-(4-chlorobenzyl)-4-(4-(1,5-dimethyl-1*H*-pyrazol-3-yl)cyclohexyl)morpholine-2-carbohydrazide;
(2*R*,5*S*)-5-(4-chlorobenzyl)-2-(5-methyl-1,3,4-oxadiazol-2-yl)-4-(4-(5-methyloxazol-2-yl)cyclohexyl)morpholine;
(2*R*,5*S*)-*N'*-acetyl-5-(4-chlorobenzyl)-4-(4-(5-methyloxazol-2-yl)cyclohexyl)morpholine-2-carbohydrazide;
(2*R*,5*S*)-5-(4-chlorobenzyl)-4-(4-(4,5-dimethyloxazol-2-yl)cyclohexyl)-2-((methylsulfonyl)methyl)morpholine;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1,5-dimethyl-1*H*-pyrazole-3-carboxamide;
(2*S*,5*S*)-5-(4-chlorobenzyl)-4-(4-(1,5-dimethyl-1*H*-pyrazol-3-yl)cyclohexyl)-2-ethynylmorpholine;
*N*-(35-(4-((2*R*,5*S*)-5-(4-chlorobenzyl)-4-(4-(1,5-dimethyl-1*H*-pyrazol-3-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
(2*R*,5*S*)-5-(4-chlorobenzyl)-4-(4-(1,5-dimethyl-1*H*-pyrazol-3-yl)cyclohexyl)-*N*-isopropylmorpholine-2-carboxamide;
(2*R*,5*S*)-5-(4-chlorobenzyl)-4-(4-(1,5-dimethyl-1*H*-pyrazol-3-yl)cyclohexyl)-*N*-methylmorpholine-2-carboxamide;
(2*R*,5*S*)-5-(4-chlorobenzyl)-4-(4-(4,5-dimethyloxazol-2-yl)cyclohexyl)-*N*,*N*-dimethylmorpholine-2-carboxamide;
(2*R*,5*S*)-5-(4-chlorobenzyl)-4-(4-(4,5-dimethyloxazol-2-yl)cyclohexyl)-*N*-isopropylmorpholine-2-carboxamide;
(2*R*,5*S*)-5-(4-chlorobenzyl)-*N*-methyl-4-(4-(5-methyloxazol-2-yl)cyclohexyl)morpholine-2-carboxamide;
(2*R*,5*S*)-5-(4-chlorobenzyl)-4-(4-(4,5-dimethyloxazol-2-yl)cyclohexyl)-*N*-ethylmorpholine-2-carboxamide;
(2*R*,5*S*)-5-(4-chlorobenzyl)-4-(4-(5-fluoro-1-methyl-1*H*-pyrazol-3-yl)cyclohexyl)-2-((methylsulfonyl)methyl)morpholine;
(2*R*,5*S*)-5-(4-chlorobenzyl)-4-(4-(4,5-dimethylthiazol-2-yl)cyclohexyl)-2-((methylsulfonyl)methyl)morpholine;
(2*R*,5*S*)-5-(4-chlorobenzyl)-2-((methylsulfonyl)methyl)-4-(4-(5-methylthiazol-2-yl)cyclohexyl)morpholine;
(2*R*,5*S*)-5-(4-chlorobenzyl)-4-(4-(4,5-dimethyloxazol-2-yl)cyclohexyl)morpholine-2-carboxylic acid;
(2*R*,5*S*)-5-(4-chlorobenzyl)-4-(4-(4,5-dimethylthiazol-2-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)-2-((prop-2-yn-1-yloxy)methyl)morpholine;
(2*R*,5*S*)-5-(4-chlorobenzyl)-4-(4-(4,5-dimethylthiazol-2-yl)cyclohexyl)-*N*-ethylmorpholine-2-carboxamide;
*N*-(35-(4-((((2*R*,5*S*)-5-(4-chlorobenzyl)-4-(4-(1,5-dimethyl-1*H*-pyrazol-3-yl)cyclohexyl)morpholin-2-yl)methoxy)methyl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
(2*R*,5*S*)-2-(azidomethyl)-5-(4-chlorobenzyl)-4-(4-(1,5-dimethyl-1*H*-pyrazol-3-yl)cyclohexyl)morpholine;
(2*R*,5*S*)-5-(4-chlorobenzyl)-4-(4-(1,5-dimethyl-1*H*-pyrazol-3-yl)cyclohexyl)-*N*-(prop-2-yn-1-yl)morpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-pyrazole-3-carboxamide;
(2*R*,5*S*)-5-(4-chlorobenzyl)-4-(4-(1,5-dimethyl-1*H*-pyrazol-3-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-2*H*-1,2,3-triazole-4-carboxamide;
3-(((2*R*,5*S*)-5-(4-chlorobenzyl)-4-(4-(1,5-dimethyl-1*H*-pyrazol-3-yl)cyclohexyl)morpholin-2-yl)methyl)imidazolidine-2,4-dione;
(2*R*,5*S*)-5-(4-chlorobenzyl)-4-(4-(1,5-dimethyl-1*H*-1,2,4-triazol-3-yl)cyclohexyl)-*N*-ethylmorpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-imidazole-4-carboxamide;
(2*R*,5*S*)-5-(4-chlorobenzyl)-4-(4-(4,5-dimethylthiazol-2-yl)cyclohex-3-en-1-yl)-2-((methylsulfonyl)methyl)morpholine;
(2*R*,5*S*)-5-(4-chlorobenzyl)-4-(4-(4,5-dimethylthiazol-2-yl)cyclohex-3-en-1-yl)-*N*-ethylmorpholine-2-carboxamide;
(2*R*,5*S*)-5-(4-chlorobenzyl)-4-(4-(4,5-dimethylthiazol-2-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)morpholine-2-carboxamide;
(2*R*,5*S*)-2-(azidomethyl)-5-(4-chlorobenzyl)-4-(4-(4,5-dimethylthiazol-2-yl)cyclohexyl)morpholine;
(2*R*,5*S*)-5-(4-chlorobenzyl)-4-(4-(4,5-dimethylthiazol-2-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide;
*N*-(((2*S*,5*S*)-5-(4-chlorobenzyl)-4-(4-(4,5-dimethylthiazol-2-yl)cyclohexyl)morpholin-2-yl)methyl)-1*H*-1,2,3-triazole-5-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1-methyl-1*H*-imidazole-4-carboxamide;
methyl 2-((2*S*,5*S*)-5-(4-chlorobenzyl)-4-(4-(1,5-dimethyl-1*H*-pyrazol-3-yl)cyclohexyl)morpholin-2-yl)acetate;
2-((2*S*,5*S*)-5-(4-chlorobenzyl)-4-(4-(1,5-dimethyl-1*H*-pyrazol-3-yl)cyclohexyl)morpholin-2-yl)acetic acid;
2-((2*S*,5*S*)-5-(4-chlorobenzyl)-4-(4-(1,5-dimethyl-1*H*-pyrazol-3-yl)cyclohexyl)morpholin-2-yl)-*N*-ethylacetamide;
(2*R*,5*S*)-5-(4-chlorobenzyl)-4-(4-(1,5-dimethyl-1*H*-1,2,4-triazol-3-yl)cyclohexyl)morpholine-2-carboxylic acid;
(2*R*,5*S*)-5-(4-bromobenzyl)-4-(4-(1,5-dimethyl-1*H*-pyrazol-3-yl)cyclohexyl)-2-((methylsulfonyl)methyl)morpholine;
*N*-(((2*R*,5*S*)-5-(4-chlorobenzyl)-4-(4-(1,5-dimethyl-1*H*-pyrazol-3-yl)cyclohexyl)morpholin-2-yl)methyl)methanesulfonamide;
(2*R*,5*S*)-5-(4-bromobenzyl)-4-(4-(1,5-dimethyl-1*H*-pyrazol-3-yl)cyclohexyl)morpholine-2-carboxylic acid;
(2*R*,5*S*)-5-(4-bromobenzyl)-4-(4-(5-methyloxazol-2-yl)cyclohexyl)morpholine-2-carboxylic acid;
(2*R*,5*S*)-5-(4-bromobenzyl)-4-(4-(5-methyloxazol-2-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)morpholine-2-carboxylic acid;
*N*-(((2*R*,5*S*)-5-(4-chlorobenzyl)-4-(4-(1,5-dimethyl-1*H*-pyrazol-3-yl)cyclohexyl)morpholin-2-yl)methyl)-1,1,1-trifluoromethanesulfonamide;
(2*R*,5*S*)-5-(4-bromobenzyl)-4-(4-(1,5-dimethyl-1*H*-pyrazol-3-yl)cyclohexyl)-*N*-ethylmorpholine-2-carboxamide;
(2*R*,5S)-*N*-(2-(3-(but-3-yn-1-yl)-3*H*-diazirin-3-yl)ethyl)-5-(4-chlorobenzyl)-4-(4-(1,5-dimethyl-1*H*-pyrazol-3-yl)cyclohexyl)morpholine-2-carboxamide;
2-(((2*R*,5*S*)-5-(4-chlorobenzyl)-4-(4-(4,5-dimethylthiazol-2-yl)cyclohexyl)morpholin-2-yl)methoxy)acetic acid;
2-((2*R*,5*S*)-5-(4-chlorobenzyl)-4-(4-(1,5-dimethyl-1*H*-pyrazol-3-yl)cyclohexyl)morpholin-2-yl)propan-2-ol;
(2*R*,5*S*)-5-(4-chlorobenzyl)-4-(4-(1,5-dimethyl-1*H*-pyrazol-3-yl)cyclohexyl)-2-(5-methyl-1*H*-pyrazol-3-yl)morpholine;
(2*R*,5*R*)-2-(4-chlorobenzyl)-1-(4-(1,5-dimethyl-1*H*-pyrazol-3-yl)cyclohexyl)-5-(methylsulfonyl)piperidine;
(2*R*,5*S*)-5-(4-chlorobenzyl)-4-(4-(1,5-dimethyl-1*H*-pyrazol-3-yl)cyclohexyl)morpholine-2-carboxamide;
2-(4-((2*R*,5*R*)-2-(4-chlorobenzyl)-5-(methylsulfonyl)piperidin-1-yl)cyclohexyl)-5-methyloxazole;
(2*R*,5*S*)-5-(4-chlorobenzyl)-4-(4-(1,5-dimethyl-1*H*-pyrazol-3-yl)cyclohexyl)-2-((methylsulfinyl)methyl)morpholine;
(2*R*,5*S*)-5-(4-chlorobenzyl)-4-(4-(1,5-dimethyl-1*H*-pyrazol-3-yl)cyclohexyl)-2-((methylthio)methyl)morpholine;
1-(((2*R*,5*S*)-5-(4-chlorobenzyl)-4-(4-(1,5-dimethyl-1*H*-pyrazol-3-yl)cyclohexyl)morpholin-2-yl)methyl)pyrrolidine-2,5-dione;
(2*S*,5*S*)-5-(4-chlorobenzyl)-4-(4-(4,5-dimethylthiazol-2-yl)cyclohexyl)-2-ethynylmorpholine;
2-(4-((2*R*,5*S*)-5-(4-chlorobenzyl)-4-(4-(4,5-dimethylthiazol-2-yl)cyclohexyl)morpholin-2-yl)-1H-1,2,3-triazol-1-yl) acetic acid;
3-(4-((2*R*,5*S*)-5-(4-chlorobenzyl)-4-(4-(4,5-dimethylthiazol-2-yl)cyclohexyl)morpholin-2-yl)-1H-1,2,3-triazol-1-yl) propanoic acid;
(2*R*,5*S*)-5-(4-chlorobenzyl)-4-(4-(1,5-dimethyl-1*H*-pyrazol-3-yl)cyclohex-3-en-1-yl)-2-((methylsulfonyl)methyl)morpholine;
*N*-(35-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*] imidazol-4-yl)pentanamide;
2-((2*S*,5*S*)-5-(4-chlorobenzyl)-4-(4-(4,5-dimethylthiazol-2-yl)cyclohexyl)morpholin-2-yl)acetic acid;
3-((((2*R*,5*S*)-5-(4-chlorobenzyl)-4-(4-(4,5-dimethylthiazol-2-yl)cyclohexyl)morpholin-2-yl)methyl)sulfonyl)propanoic acid;
3-(((2*R*,5*S*)-5-(4-chlorobenzyl)-4-(4-(1,5-dimethyl-1*H*-pyrazol-3-yl)cyclohexyl)morpholin-2-yl)methyl)-6,6-dimethyl-3-azabicyclo[3.1.0]hexane-2,4-dione;
3-(((2*R*,5*S*)-5-(4-chlorobenzyl)-4-(4-(4,5-dimethylthiazol-2-yl)cyclohexyl)morpholin-2-yl)methyl)-6,6-dimethyl-3-azabicyclo[3.1.0]hexane-2,4-dione;
3-((((2*S*,5*S*)-5-(4-chlorobenzyl)-4-(4-(1,5-dimethyl-1*H*-pyrazol-3-yl)cyclohexyl)morpholin-2-yl)methyl)amino)-4-hydroxycyclobut-3-ene-1,2-dione;

3-amino-4-((((2*S*,5*S*)-5-(4-chlorobenzyl)-4-(4-(1,5-dimethyl-1*H*-pyrazol-3-yl)cyclohexyl)morpholin-2-yl)methyl) amino)cyclobut-3-ene-1,2-dione;
(2*R*,5*S*)-5-(4-chlorobenzyl)-4-(4-(4,5-dimethylthiazol-2-yl)cyclohexyl)-2-((ethylsulfonyl)methyl)morpholine;
(2*R*,5*S*)-5-(4-chlorobenzyl)-4-(4-(1,5-dimethyl-1*H*-pyrazol-3-yl)cyclohexyl)-2-((ethylsulfonyl)methyl)morpholine;
3-(((2*R*,5*S*)-5-(4-chlorobenzyl)-4-(4-(1,5-dimethyl-1*H*-pyrazol-3-yl)cyclohexyl)morpholin-2-yl)methyl)-1-methylimidazolidine-2,4-dione;
(2*R*,5*S*)-5-(4-chlorobenzyl)-4-(4-(4,5-dimethylthiazol-2-yl)cyclohexyl)-*N*-(1-methyl-1*H*-imidazol-4-yl)morpholine-2-carboxamide;
4-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl) butanoic acid;
(2*R*,5*S*)-5-(4-chlorobenzyl)-4-(4-(4,5-dimethylthiazol-2-yl)cyclohexyl)-*N*-hydroxymorpholine-2-carboxamide;
(2*R*,5*S*)-5-(4-chlorobenzyl)-N-(1,5-dimethyl-1*H*-pyrazol-3-yl)-4-(4-(4,5-dimethylthiazol-2-yl)cyclohexyl)morpholine-2-carboxamide;
5-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl) pentanoic acid;
(2*R*,5*S*)-5-(4-chlorobenzyl)-*N*-(1,5-dimethyl-1*H*-pyrazol-3-yl)-4-(4-(1,5-dimethyl-1*H*-pyrazol-3-yl)cyclohexyl)morpholine-2-carboxamide;
*N*-((2*R*,5*S*)-5-(4-chlorobenzyl)-4-(4-(4,5-dimethylthiazol-2-yl)cyclohexyl)morpholine-2-carbonyl)-*N*-methylglycine;
(2*R*,5*S*)-4-(4-(1-methyl-1*H*-1,2,4-triazol-3-yl)cyclohexyl)-2-((methylsulfonyl)methyl)-5-(4-(trifluoromethyl)benzyl) morpholine;
(2*R*,5*S*)-5-(4-chlorobenzyl)-4-(4-(1-methyl-1*H*-imidazol-4-yl)cyclohexyl)-2-((methylsulfonyl)methyl)morpholine;
(2*R*,5*S*)-4-(4-(1-methyl-1*H*-1,2,4-triazol-3-yl)cyclohexyl)-5-(4-(trifluoromethyl)benzyl)-morpholine-2-carboxylic acid;
(2*S*,5*S*)-5-(4-chlorobenzyl)-2-methyl-4-(4-(5-methyl-1*H*-pyrazol-3-yl)cyclohexyl)morpholine;
(2*R*,5*S*)-5-(4-chlorobenzyl)-4-(4-(1-methyl-1*H*-1,2,4-triazol-3-yl)cyclohexyl)-2-((methylsulfonyl)methyl)morpholine;
(2*R*,5*S*)-5-(4-chlorobenzyl)-4-(4-(1-methyl-1*H*-1,2,4-triazol-3-yl)cyclohexyl)morpholine-2-carboxylic acid;
(2*R*,5*S*)-5-(4-chlorobenzyl)-4-(4-(1-methyl-1*H*-1,2,3-triazol-4-yl)cyclohexyl)-2-((methylsulfonyl)methyl)morpholine;
methyl (2*R*,5*S*)-5-(4-chlorobenzyl)-4-(4-(1-methyl-1*H*-1,2,3-triazol-4-yl)cyclohexyl)morpholine-2-carboxylate;
(2*R*,5*S*)-5-(4-chlorobenzyl)-4-(4-(1-methyl-1*H*-1,2,3-triazol-4-yl)cyclohexyl)morpholine-2-carboxylic acid;
(2*R*,5*S*)-5-(4-chlorobenzyl)-4-(4-(1,5-dimethyl-1*H*-pyrazol-4-yl)cyclohexyl)-2-((methylsulfonyl)methyl)morpholine;
(2*R*,5*S*)-5-(4-chlorobenzyl)-4-(4-(1-methyl-1*H*-pyrazol-3-yl)cyclohexyl)-2-((methylsulfonyl)methyl)morpholine;
(2*R*,5*S*)-5-(4-chlorobenzyl)-4-(4-(2-methyl-2H-1,2,3-triazol-4-yl)cyclohexyl)-2-((methylsulfonyl)methyl)morpholine;
(2*R*,5*S*)-5-(4-chlorobenzyl)-4-(4-(2-methyl-2*H*-tetrazol-5-yl)cyclohexyl)-2-((methylsulfonyl)methyl)morpholine;
(2*R*,5*S*)-5-(4-chlorobenzyl)-*N*,*N*-dimethyl-4-(4-(5-methyl-1,3,4-thiadiazol-2-yl)cyclohexyl)morpholine-2-carboxamide;
(2*R*,5*S*)-5-(4-chlorobenzyl)-4-(4-(5-methyl-1,3,4-thiadiazol-2-yl)cyclohexyl)-2-((methylsulfonyl)methyl)morpholine;
(2*R*,5*S*)-5-(4-chlorobenzyl)-4-(4-(5-methoxy-1-methyl-1*H*-1,2,4-triazol-3-yl)cyclohexyl)-2-((methylsulfonyl)methyl) morpholine;
3-(4-((2*R*,5*S*)-5-(4-chlorobenzyl)-2-((methylsulfonyl)methyl)morpholino)cyclohexyl)-1-methyl-1*H*-1,2,4-triazol-5-ol;
(2*R*,5*S*)-5-(4-chlorobenzyl)-4-(4-(1-isopropyl-1*H*-pyrazol-3-yl)cyclohexyl)-2-((methylsulfonyl)methyl)morpholine;
(2*R*,5*S*)-5-(4-chlorobenzyl)-4-(4-(5-methoxy-1-methyl-1*H*-1,2,4-triazol-3-yl)cyclohexyl)-*N*,*N*-dimethylmorpholine-2-carboxamide;
(2*R*,5*S*)-5-(4-chlorobenzyl)-4-(4-(5-hydroxy-1-methyl-1*H*-1,2,4-triazol-3-yl)cyclohexyl)-*N*,*N*-dimethylmorpholine-2-carboxamide;
(2*R*,5*S*)-5-(4-chlorobenzyl)-4-(4-(1,2-dimethyl-1*H*-imidazol-4-yl)cyclohexyl)-2-((methylsulfonyl)methyl)morpholine;
(2*R*,5*S*)-5-(4-chlorobenzyl)-4-(4-(1-methyl-5-(trifluoromethyl)-1*H*-pyrazol-3-yl)cyclohexyl)-2-((methylsulfonyl) methyl)morpholine;
(2*R*,5*S*)-5-(4-chlorobenzyl)-4-(4-(4,5-dimethylthiophen-2-yl)cyclohexyl)-2-((methylsulfonyl)methyl)morpholine;
4-(4-((2*R*,5*S*)-5-(4-chlorobenzyl)-2-((methylsulfonyl)methyl)morpholino)cyclohexyl)-1-methyl-1*H*-pyrrole-2-carbonitrile;
(2*R*,5*S*)-5-(4-chlorobenzyl)-4-(4-(5-cyano-1-methyl-1*H*-pyrrol-3-yl)cyclohexyl)morpholine-2-carboxylic acid;
(2*R*,5*S*)-5-(4-chlorobenzyl)-4-(4-(1-ethyl-5-methyl-1*H*-pyrazol-3-yl)cyclohexyl)-2-((methylsulfonyl)methyl)morpholine;
(2*R*,5*S*)-5-(4-chlorobenzyl)-4-(4-(1-ethyl-5-methyl-1*H*-pyrazol-3-yl)cyclohexyl)morpholine-2-carboxylic acid;
(2*R*,5*S*)-5-(4-chlorobenzyl)-*N*-ethyl-4-(4-(1-ethyl-5-methyl-1*H*-pyrazol-3-yl)cyclohexyl)morpholine-2-carboxamide;
(2*R*,5*S*)-5-(4-chlorobenzyl)-4-(4-(4-methyl-1*H*-pyrazol-1-yl)cyclohexyl)-2-((methylsulfonyl)methyl)morpholine;
(2*R*,5*S*)-5-(4-chlorobenzyl)-4-(4-(4-methyl-1*H*-pyrazol-1-yl)cyclohexyl)morpholine-2-carboxylic acid;
*N*-(39-((((2*R*,5*S*)-5-(4-chlorobenzyl)-4-(4-(4,5-dimethylthiazol-2-yl)cyclohexyl)morpholin-2-yl)methyl)sulfonyl)-37-oxo-3,6,9,12,15,18,21,24,27,30,33-undecaoxa-36-azanonatriacontyl)-5-((3a*S*,4*S*,6a*R*)-2-oxohexahydro-1*H*-thieno[3,4-*d*]imidazol-4-yl)pentanamide;
(2*R*,5*S*)-5-(4-chlorobenzyl)-4-(4-(1,5-dimethyl-1*H*-pyrazol-3-yl)cyclohexyl)-*N*-(1*H*-tetrazol-5-yl)morpholine-2-carboxamide;
(2*R*,5*S*)-4-(4-(1*H*-tetrazol-5-yl)cyclohexyl)-5-(4-chlorobenzyl)-2-((methylsulfonyl)methyl)-morpholine;
(2*R*,5*S*)-4-(4-(1*H*-1,2,4-triazol-1-yl)cyclohexyl)-5-(4-chlorobenzyl)-2-((methylsulfonyl)methyl)-morpholine;
(2*R*,5*S*)-4-(4-(1*H*-pyrazol-1-yl)cyclohexyl)-5-(4-chlorobenzyl)-2-((methylsulfonyl)methyl)-morpholine;
(2*R*,5*S*)-5-(4-chlorobenzyl)-4-(4-(4-fluoro-1*H*-pyrazol-1-yl)cyclohexyl)-2-((methylsulfonyl)methyl)morpholine;
(2*R*,5*S*)-5-(4-chlorobenzyl)-4-(4-(5-fluoro-1-methyl-1*H*-pyrazol-3-yl)cyclohexyl)morpholine-2-carboxylic acid;
*N*[1]-(1,31-bis(((2*R*,3*R*,4*R*,5*R*,6*R*)-3-acetamido-4,5-dihydroxy-6-(hydroxymethyl)tetrahydro-2*H*-pyran-2-yl)oxy)-16-((3-((3-(5-(((2*R*,3*R*,4*R*,5*R*,6*R*)-3-acetamido-4,5-dihydroxy-6-(hydroxymethyl)tetrahydro-2*H*-pyran-2-yl)oxy)pentanamido)propyl)amino)-3-oxopropoxy)methyl)-5,11,21,27-tetraoxo-14,18-dioxa-6,10,22,26-tetraazahentriacontan-16-yl)-*N*[12]-(3-(4-(((2*R*,5*S*)-5-(4-chlorobenzyl)-4-(4-(1,5-dimethyl-1*H*-pyrazol-3-yl)cyclohexyl)morpholine-2-carboxamido)methyl)-1*H*-1,2,3-triazol-1-yl)propyl)dodecanediamide; and
(2*R*,5*S*)-5-(4-chlorobenzyl)-4-(4-(1,5-dimethyl-1*H*-pyrazol-3-yl)cyclohexyl)-*N*-(5-(2-(2,6-dioxopiperidin-3-yl)-3-oxoisoindolin-4-yl)pentyl)morpholine-2-carboxamide;
or is a tautomer, stereoisomer, pharmaceutically acceptable salt, solvate, or polymorph thereof.

More preferably, the invention concerns the compound of structural formula (I), that is selected from the group consisting of the following compounds:

(2*S*,5*S*)-5-(4-chlorobenzyl)-2-methyl-4-(4-(5-methyloxazol-2-yl)cyclohexyl)morpholine;
(2*S*,5*S*)-5-(4-chlorobenzyl)-4-(4-(1,5-dimethyl-1*H*-pyrazol-3-yl)cyclohexyl)-2-methylmorpholine;
(2*R*,5*S*)-5-(4-chlorobenzyl)-4-(4-(5-methyloxazol-2-yl)cyclohexyl)-2-((methylsulfonyl)methyl)morpholine;
(2*R*,5*S*)-5-(4-chlorobenzyl)-4-((1*r*,4*S*)-4-(1,5-dimethyl-1*H*-pyrazol-3-yl)cyclohexyl)-2-((methylsulfonyl)methyl)morpholine;
(2*R*,5*S*)-5-(4-chlorobenzyl)-4-(4-(1,5-dimethyl-1*H*-pyrazol-3-yl)cyclohexyl)-2-(3-methylisoxazol-5-yl)morpholine;
(7*S*,9a*R*)-7-(4-chlorobenzyl)-8-(4-(5-methyloxazol-2-yl)cyclohexyl)octahydropyrazino[2,1-*c*][1,4]oxazine;
(7*S*,9a*R*)-7-(4-chlorobenzyl)-8-(4-(1,5-dimethyl-1*H*-pyrazol-3-yl)cyclohexyl)octahydropyrazino[2,1-*c*][1,4]oxazine;
(2*R*,5*S*)-5-(4-chlorobenzyl)-4-(4-(5-methyloxazol-2-yl)cyclohexyl)morpholine-2-carboxylic acid;
methyl (2*R*,5*S*)-5-(4-chlorobenzyl)-4-(4-(1,5-dimethyl-1*H*-pyrazol-3-yl)cyclohexyl)morpholine-2-carboxylate;
(2*R*,5*S*)-5-(4-chlorobenzyl)-4-(4-(1,5-dimethyl-1*H*-pyrazol-3-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholine;
(2*R*,5*S*)-5-(4-chlorobenzyl)-4-(4-(5-methyloxazol-2-yl)cyclohexyl)-2-(1-(2,2,2-trifluoroethyl)-1*H*-1,2,3-triazol-4-yl)morpholine;
(2*R*,5*S*)-5-(4-chlorobenzyl)-4-(4-(1,5-dimethyl-1*H*-pyrazol-3-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)morpholine-2-carboxamide;
(2*R*,5*S*)-5-(4-chlorobenzyl)-4-(4-(1,5-dimethyl-1*H*-1,2,4-triazol-3-yl)cyclohexyl)-2-((methylsulfonyl)methyl)morpholine;
(2*R*,5*S*)-5-(4-chlorobenzyl)-4-(4-(1,5-dimethyl-1*H*-pyrazol-3-yl)cyclohexyl)-2-(fluoromethyl)morpholine;
((2*R*,5*S*)-5-(4-chlorobenzyl)-4-(4-(1,5-dimethyl-1*H*-pyrazol-3-yl)cyclohexyl)morpholin-2-yl)methanol;
(2*R*,5*S*)-5-(4-chlorobenzyl)-*N*-ethyl-4-(4-(5-methyloxazol-2-yl)cyclohexyl)morpholine-2-carboxamide;
(2*R*,5*S*)-5-(4-chlorobenzyl)-4-(4-(1,5-dimethyl-1*H*-pyrazol-3-yl)cyclohexyl)-*N*-ethylmorpholine-2-carboxamide;
(2*R*,5*S*)-5-(4-chlorobenzyl)-4-(4-(1,5-dimethyl-1*H*-pyrazol-3-yl)cyclohexyl)-*N*,*N*-dimethylmorpholine-2-carboxamide;
(2*R*,5*S*)-5-(4-chlorobenzyl)-*N*,*N*-dimethyl-4-(4-(5-methyloxazol-2-yl)cyclohexyl)morpholine-2-carboxamide;
(2*R*,5*S*)-5-(4-chlorobenzyl)-4-(4-(1,5-dimethyl-1*H*-pyrazol-3-yl)cyclohexyl)-2-(5-methyl-1,3,4-oxadiazol-2-yl)morpholine;
(2*R*,5*S*)-*N*'-acetyl-5-(4-chlorobenzyl)-4-(4-(1,5-dimethyl-1*H*-pyrazol-3-yl)cyclohexyl)morpholine-2-carbohydrazide;
(2*R*,5*S*)-5-(4-chlorobenzyl)-2-(5-methyl-1,3,4-oxadiazol-2-yl)-4-(4-(5-methyloxazol-2-yl)cyclohexyl)morpholine;
(2*R*,5*S*)-*N*'-acetyl-5-(4-chlorobenzyl)-4-(4-(5-methyloxazol-2-yl)cyclohexyl)morpholine-2-carbohydrazide;
(2*R*,5*S*)-5-(4-chlorobenzyl)-4-(4-(4,5-dimethyloxazol-2-yl)cyclohexyl)-2-((methylsulfonyl)methyl)morpholine;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1,5-dimethyl-1*H*-pyrazole-3-carboxamide;
(2*S*,5*S*)-5-(4-chlorobenzyl)-4-(4-(1,5-dimethyl-1*H*-pyrazol-3-yl)cyclohexyl)-2-ethynylmorpholine;
*N*-(35-(4-((2*R*,5*S*)-5-(4-chlorobenzyl)-4-(4-(1,5-dimethyl-1*H*-pyrazol-3-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
(2*R*,5*S*)-5-(4-chlorobenzyl)-4-(4-(1,5-dimethyl-1*H*-pyrazol-3-yl)cyclohexyl)-*N*-isopropylmorpholine-2-carboxamide;
(2*R*,5*S*)-5-(4-chlorobenzyl)-4-(4-(1,5-dimethyl-1*H*-pyrazol-3-yl)cyclohexyl)-*N*-methylmorpholine-2-carboxamide;
(2*R*,5*S*)-5-(4-chlorobenzyl)-4-(4-(4,5-dimethyloxazol-2-yl)cyclohexyl)-*N*,*N*-dimethylmorpholine-2-carboxamide;
(2*R*,5*S*)-5-(4-chlorobenzyl)-4-(4-(4,5-dimethyloxazol-2-yl)cyclohexyl)-*N*-isopropylmorpholine-2-carboxamide;
(2*R*,5*S*)-5-(4-chlorobenzyl)-*N*-methyl-4-(4-(5-methyloxazol-2-yl)cyclohexyl)morpholine-2-carboxamide;
(2*R*,5*S*)-5-(4-chlorobenzyl)-4-(4-(4,5-dimethyloxazol-2-yl)cyclohexyl)-*N*-ethylmorpholine-2-carboxamide;
(2*R*,5*S*)-5-(4-chlorobenzyl)-4-(4-(5-fluoro-1-methyl-1*H*-pyrazol-3-yl)cyclohexyl)-2-((methylsulfonyl)methyl)morpholine;
(2*R*,5*S*)-5-(4-chlorobenzyl)-4-(4-(4,5-dimethylthiazol-2-yl)cyclohexyl)-2-((methylsulfonyl)methyl)morpholine;
(2*R*,5*S*)-5-(4-chlorobenzyl)-2-((methylsulfonyl)methyl)-4-(4-(5-methylthiazol-2-yl)cyclohexyl)morpholine;
(2*R*,5*S*)-5-(4-chlorobenzyl)-4-(4-(4,5-dimethyloxazol-2-yl)cyclohexyl)morpholine-2-carboxylic acid;
(2*R*,5*S*)-5-(4-chlorobenzyl)-4-(4-(4,5-dimethylthiazol-2-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)-2-((prop-2-yn-1-yloxy)methyl)morpholine;
(2*R*,5*S*)-5-(4-chlorobenzyl)-4-(4-(4,5-dimethylthiazol-2-yl)cyclohexyl)-*N*-ethylmorpholine-2-carboxamide;
*N*-(35-(4-((((2*R*,5*S*)-5-(4-chlorobenzyl)-4-(4-(1,5-dimethyl-1*H*-pyrazol-3-yl)cyclohexyl)morpholin-2-yl)methoxy)methyl)-1H-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
(2*R*,5*S*)-2-(azidomethyl)-5-(4-chlorobenzyl)-4-(4-(1,5-dimethyl-1*H*-pyrazol-3-yl)cyclohexyl)morpholine;
(2*R*,5*S*)-5-(4-chlorobenzyl)-4-(4-(1,5-dimethyl-1*H*-pyrazol-3-yl)cyclohexyl)-*N*-(prop-2-yn-1-yl)morpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-pyrazole-3-carboxamide;
(2*R*,5*S*)-5-(4-chlorobenzyl)-4-(4-(1,5-dimethyl-1*H*-pyrazol-3-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-2*H*-1,2,3-triazole-4-carboxamide;
3-(((2*R*,5*S*)-5-(4-chlorobenzyl)-4-(4-(1,5-dimethyl-1*H*-pyrazol-3-yl)cyclohexyl)morpholin-2-yl)methyl)imidazolidine-2,4-dione;
(2*R*,5*S*)-5-(4-chlorobenzyl)-4-(4-(1,5-dimethyl-1*H*-1,2,4-triazol-3-yl)cyclohexyl)-*N*-ethylmorpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-imidazole-4-carboxamide;
(2*R*,5*S*)-5-(4-chlorobenzyl)-4-(4-(4,5-dimethylthiazol-2-yl)cyclohex-3-en-1-yl)-2-((methylsulfonyl)methyl)morpholine;
(2*R*,5*S*)-5-(4-chlorobenzyl)-4-(4-(4,5-dimethylthiazol-2-yl)cyclohex-3-en-1-yl)-*N*-ethylmorpholine-2-carboxamide;
(2*R*,5*S*)-5-(4-chlorobenzyl)-4-(4-(4,5-dimethylthiazol-2-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)morpholine-2-carboxamide;
(2*R*,5*S*)-2-(azidomethyl)-5-(4-chlorobenzyl)-4-(4-(4,5-dimethylthiazol-2-yl)cyclohexyl)morpholine;
(2*R*,5*S*)-5-(4-chlorobenzyl)-4-(4-(4,5-dimethylthiazol-2-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide;
*N*-(((2*S*,5*S*)-5-(4-chlorobenzyl)-4-(4-(4,5-dimethylthiazol-2-yl)cyclohexyl)morpholin-2-yl)methyl)-1*H*-1,2,3-triazole-5-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1-methyl-1*H*-imidazole-4-carboxamide;
methyl 2-((2*S*,5*S*)-5-(4-chlorobenzyl)-4-(4-(1,5-dimethyl-1*H*-pyrazol-3-yl)cyclohexyl)morpholin-2-yl)acetate;
2-((2*S*,5*S*)-5-(4-chlorobenzyl)-4-(4-(1,5-dimethyl-1*H*-pyrazol-3-yl)cyclohexyl)morpholin-2-yl)acetic acid;
2-((2*S*,5*S*)-5-(4-chlorobenzyl)-4-(4-(1,5-dimethyl-1*H*-pyrazol-3-yl)cyclohexyl)morpholin-2-yl)-*N*-ethylacetamide;
(2*R*,5*S*)-5-(4-chlorobenzyl)-4-(4-(1,5-dimethyl-1*H*-1,2,4-triazol-3-yl)cyclohexyl)morpholine-2-carboxylic acid;
(2*R*,5*S*)-5-(4-bromobenzyl)-4-(4-(1,5-dimethyl-1*H*-pyrazol-3-yl)cyclohexyl)-2-((methylsulfonyl)methyl)morpholine;
*N*-(((2*R*,5*S*)-5-(4-chlorobenzyl)-4-(4-(1,5-dimethyl-1*H*-pyrazol-3-yl)cyclohexyl)morpholin-2-yl)methyl)methanesulfonamide;
(2*R*,5*S*)-5-(4-bromobenzyl)-4-(4-(1,5-dimethyl-1*H*-pyrazol-3-yl)cyclohexyl)morpholine-2-carboxylic acid;

(2*R*,5*S*)-5-(4-bromobenzyl)-4-(4-(5-methyloxazol-2-yl)cyclohexyl)morpholine-2-carboxylic acid;
(2*R*,5*S*)-5-(4-bromobenzyl)-4-(4-(5-methyloxazol-2-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)morpholine-2-carboxylic acid;
*N*-(((2*R*,5*S*)-5-(4-chlorobenzyl)-4-(4-(1,5-dimethyl-1*H*-pyrazol-3-yl)cyclohexyl)morpholin-2-yl)methyl)-1,1,1-trifluoromethanesulfonamide;
(2*R*,5*S*)-5-(4-bromobenzyl)-4-(4-(1,5-dimethyl-1*H*-pyrazol-3-yl)cyclohexyl)-*N*-ethylmorpholine-2-carboxamide;
(2*R*,5*S*)-*N*-(2-(3-(but-3-yn-1-yl)-3*H*-diazirin-3-yl)ethyl)-5-(4-chlorobenzyl)-4-(4-(1,5-dimethyl-1*H*-pyrazol-3-yl)cyclohexyl)morpholine-2-carboxamide;
2-(((2*R*,5*S*)-5-(4-chlorobenzyl)-4-(4-(4,5-dimethylthiazol-2-yl)cyclohexyl)morpholin-2-yl)methoxy)acetic acid;
2-((2*R*,5*S*)-5-(4-chlorobenzyl)-4-(4-(1,5-dimethyl-1*H*-pyrazol-3-yl)cyclohexyl)morpholin-2-yl)propan-2-ol;
(2*R*,5*S*)-5-(4-chlorobenzyl)-4-(4-(1,5-dimethyl-1*H*-pyrazol-3-yl)cyclohexyl)-2-(5-methyl-1*H*-pyrazol-3-yl)morpholine;
(2*R*,5*R*)-2-(4-chlorobenzyl)-1-(4-(1,5-dimethyl-1*H*-pyrazol-3-yl)cyclohexyl)-5-(methylsulfonyl)piperidine;
(2*R*,5*S*)-5-(4-chlorobenzyl)-4-(4-(1,5-dimethyl-1*H*-pyrazol-3-yl)cyclohexyl)morpholine-2-carboxamide;
2-(4-((2*R*,5*R*)-2-(4-chlorobenzyl)-5-(methylsulfonyl)piperidin-1-yl)cyclohexyl)-5-methyloxazole;
(2*R*,5*S*)-5-(4-chlorobenzyl)-4-(4-(1,5-dimethyl-1*H*-pyrazol-3-yl)cyclohexyl)-2-((methylsulfinyl)methyl)morpholine;
(2*R*,5*S*)-5-(4-chlorobenzyl)-4-(4-(1,5-dimethyl-1*H*-pyrazol-3-yl)cyclohexyl)-2-((methylthio)methyl)morpholine;
1-(((2*R*,5*S*)-5-(4-chlorobenzyl)-4-(4-(1,5-dimethyl-1*H*-pyrazol-3-yl)cyclohexyl)morpholin-2-yl)methyl)pyrrolidine-2,5-dione;
(2*S*,5*S*)-5-(4-chlorobenzyl)-4-(4-(4,5-dimethylthiazol-2-yl)cyclohexyl)-2-ethynylmorpholine;
2-(4-((2*R*,5*S*)-5-(4-chlorobenzyl)-4-(4-(4,5-dimethylthiazol-2-yl)cyclohexyl)morpholin-2-yl)-1H-1,2,3-triazol-1-yl) acetic acid;
3-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl) propanoic acid;
(2*R*,5*S*)-5-(4-chlorobenzyl)-4-(4-(1,5-dimethyl-1*H*-pyrazol-3-yl)cyclohex-3-en-1-yl)-2-((methylsulfonyl)methyl)morpholine;
*N*-(35-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*] imidazol-4-yl)pentanamide;
2-((2*S*,5*S*)-5-(4-chlorobenzyl)-4-(4-(4,5-dimethylthiazol-2-yl)cyclohexyl)morpholin-2-yl)acetic acid;
3-((((2*R*,5*S*)-5-(4-chlorobenzyl)-4-(4-(4,5-dimethylthiazol-2-yl)cyclohexyl)morpholin-2-yl)methyl)sulfonyl)propanoic acid;
3-(((2*R*,5*S*)-5-(4-chlorobenzyl)-4-(4-(1,5-dimethyl-1*H*-pyrazol-3-yl)cyclohexyl)morpholin-2-yl)methyl)-6,6-dimethyl-3-azabicyclo[3.1.0]hexane-2,4-dione;
3-(((2*R*,5*S*)-5-(4-chlorobenzyl)-4-(4-(4,5-dimethylthiazol-2-yl)cyclohexyl)morpholin-2-yl)methyl)-6,6-dimethyl-3-azabicyclo[3.1.0]hexane-2,4-dione;
3-((((2*S*,5*S*)-5-(4-chlorobenzyl)-4-(4-(1,5-dimethyl-1*H*-pyrazol-3-yl)cyclohexyl)morpholin-2-yl)methyl)amino)-4-hydroxycyclobut-3-ene-1,2-dione;
3-amino-4-((((2*S*,5*S*)-5-(4-chlorobenzyl)-4-(4-(1,5-dimethyl-1*H*-pyrazol-3-yl)cyclohexyl)morpholin-2-yl)methyl) amino)cyclobut-3-ene-1,2-dione;
(2*R*,5*S*)-5-(4-chlorobenzyl)-4-(4-(4,5-dimethylthiazol-2-yl)cyclohexyl)-2-((ethylsulfonyl)methyl)morpholine;
(2*R*,5*S*)-5-(4-chlorobenzyl)-4-(4-(1,5-dimethyl-1*H*-pyrazol-3-yl)cyclohexyl)-2-((ethylsulfonyl)methyl)morpholine;
3-(((2*R*,5*S*)-5-(4-chlorobenzyl)-4-(4-(1,5-dimethyl-1*H*-pyrazol-3-yl)cyclohexyl)morpholin-2-yl)methyl)-1-methylimidazolidine-2,4-dione;
(2*R*,5*S*)-5-(4-chlorobenzyl)-4-(4-(4,5-dimethylthiazol-2-yl)cyclohexyl)-*N*-(1-methyl-1*H*-imidazol-4-yl)morpholine-2-carboxamide;
4-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl) butanoic acid;
(2*R*,5*S*)-5-(4-chlorobenzyl)-4-(4-(4,5-dimethylthiazol-2-yl)cyclohexyl)-*N*-hydroxymorpholine-2-carboxamide;
(2*R*,5*S*)-5-(4-chlorobenzyl)-*N*-(1,5-dimethyl-1*H*-pyrazol-3-yl)-4-(4-(4,5-dimethylthiazol-2-yl)cyclohexyl)morpholine-2-carboxamide;
5-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl) pentanoic acid;
(2*R*,5*S*)-5-(4-chlorobenzyl)-*N*-(1,5-dimethyl-1*H*-pyrazol-3-yl)-4-(4-(1,5-dimethyl-1*H*-pyrazol-3-yl)cyclohexyl)morpholine-2-carboxamide; and
*N*-((2*R*,5*S*)-5-(4-chlorobenzyl)-4-(4-(4,5-dimethylthiazol-2-yl)cyclohexyl)morpholine-2-carbonyl)-*N*-methylglycine;

or is a tautomer, stereoisomer, pharmaceutically acceptable salt, solvate, or polymorph thereof.

Still more preferably, the inventive compound as defined above is selected from the group consisting of the following compounds:

(2*S*,5*S*)-5-(4-chlorobenzyl)-2-methyl-4-(4-(5-methyloxazol-2-yl)cyclohexyl)morpholine;
(2*S*,5*S*)-5-(4-chlorobenzyl)-4-(4-(1,5-dimethyl-1*H*-pyrazol-3-yl)cyclohexyl)-2-methylmorpholine;
(2*R*,5*S*)-5-(4-chlorobenzyl)-4-(4-(5-methyloxazol-2-yl)cyclohexyl)-2-((methylsulfonyl)methyl)morpholine;
(2*R*,5*S*)-5-(4-chlorobenzyl)-4-((1*r*,4*S*)-4-(1,5-dimethyl-1*H*-pyrazol-3-yl)cyclohexyl)-2-((methylsulfonyl)methyl)morpholine;
(2*R*,5*S*)-5-(4-chlorobenzyl)-4-(4-(1,5-dimethyl-1*H*-pyrazol-3-yl)cyclohexyl)-2-(3-methylisoxazol-5-yl)morpholine;
(2*R*,5*S*)-5-(4-chlorobenzyl)-4-(4-(5-methyloxazol-2-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)morpholine-2-carboxylic acid;
(2*R*,5*S*)-5-(4-chlorobenzyl)-4-(4-(1,5-dimethyl-1*H*-1,2,4-triazol-3-yl)cyclohexyl)-2-((methylsulfonyl)methyl)morpholine;
(2*R*,5*S*)-5-(4-chlorobenzyl)-4-(4-(1,5-dimethyl-1*H*-pyrazol-3-yl)cyclohexyl)-2-(fluoromethyl)morpholine;
((2*R*,5*S*)-5-(4-chlorobenzyl)-4-(4-(1,5-dimethyl-1*H*-pyrazol-3-yl)cyclohexyl)morpholin-2-yl)methanol;
(2*R*,5*S*)-5-(4-chlorobenzyl)-4-(4-(1,5-dimethyl-1*H*-pyrazol-3-yl)cyclohexyl)-*N*,*N*-dimethylmorpholine-2-carboxamide;
(2*R*,5*S*)-5-(4-chlorobenzyl)-4-(4-(4,5-dimethyloxazol-2-yl)cyclohexyl)-2-((methylsulfonyl)methyl)morpholine;
(2*R*,5*S*)-5-(4-chlorobenzyl)-4-(4-(1,5-dimethyl-1*H*-pyrazol-3-yl)cyclohexyl)-*N*-methylmorpholine-2-carboxamide;
(2*R*,5*S*)-5-(4-chlorobenzyl)-4-(4-(5-fluoro-1-methyl-1*H*-pyrazol-3-yl)cyclohexyl)-2-((methylsulfonyl)methyl)morpholine;
(2*R*,5*S*)-5-(4-chlorobenzyl)-4-(4-(4,5-dimethylthiazol-2-yl)cyclohexyl)-2-((methylsulfonyl)methyl)morpholine;
(2*R*,5*S*)-5-(4-chlorobenzyl)-4-(4-(4,5-dimethyloxazol-2-yl)cyclohexyl)morpholine-2-carboxylic acid;
(2*R*,5*S*)-5-(4-chlorobenzyl)-4-(4-(4,5-dimethylthiazol-2-yl)cyclohexyl)morpholine-2-carboxylic acid;
(2*R*,5*S*)-5-(4-chlorobenzyl)-4-(4-(4,5-dimethylthiazol-2-yl)cyclohex-3-en-1-yl)-2-((methylsulfonyl)methyl)morpholine;
2-((2*S*,5*S*)-5-(4-chlorobenzyl)-4-(4-(1,5-dimethyl-1*H*-pyrazol-3-yl)cyclohexyl)morpholin-2-yl)acetic acid;
(2*R*,5*S*)-5-(4-chlorobenzyl)-4-(4-(1,5-dimethyl-1*H*-1,2,4-triazol-3-yl)cyclohexyl)morpholine-2-carboxylic acid;
(2*R*,5*S*)-5-(4-bromobenzyl)-4-(4-(1,5-dimethyl-1*H*-pyrazol-3-yl)cyclohexyl)-2-((methylsulfonyl)methyl)morpholine;
*N*-(((2*R*,5*S*)-5-(4-chlorobenzyl)-4-(4-(1,5-dimethyl-1*H*-pyrazol-3-yl)cyclohexyl)morpholin-2-yl)methyl)methanesulfonamide;
(2*R*,5*S*)-5-(4-bromobenzyl)-4-(4-(1,5-dimethyl-1*H*-pyrazol-3-yl)cyclohexyl)morpholine-2-carboxylic acid;
(2*R*,5*S*)-5-(4-bromobenzyl)-4-(4-(5-methyloxazol-2-yl)cyclohexyl)morpholine-2-carboxylic acid;
(2*R*,5*S*)-5-(4-bromobenzyl)-4-(4-(5-methyloxazol-2-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)morpholine-2-carboxylic acid;
(2*R*,5*R*)-2-(4-chlorobenzyl)-1-(4-(1,5-dimethyl-1*H*-pyrazol-3-yl)cyclohexyl)-5-(methylsulfonyl)piperidine;
(2*R*,5*S*)-5-(4-chlorobenzyl)-4-(4-(1,5-dimethyl-1*H*-pyrazol-3-yl)cyclohexyl)morpholine-2-carboxamide;
2-(4-((2*R*,5*R*)-2-(4-chlorobenzyl)-5-(methylsulfonyl)piperidin- 1-yl)cyclohexyl)-5-methyloxazole;
(2*R*,5*S*)-5-(4-chlorobenzyl)-4-(4-(1,5-dimethyl-1*H*-pyrazol-3-yl)cyclohexyl)-2-((methylsulfinyl)methyl)morpholine;
(2*R*,5*S*)-5-(4-chlorobenzyl)-4-(4-(1,5-dimethyl-1*H*-pyrazol-3-yl)cyclohexyl)-2-((methylthio)methyl)morpholine;
(2*R*,5*S*)-5-(4-chlorobenzyl)-4-(4-(1,5-dimethyl-1*H*-pyrazol-3-yl)cyclohex-3-en-1-yl)-2-((methylsulfonyl)methyl)morpholine;
2-((2*S*,5*S*)-5-(4-chlorobenzyl)-4-(4-(4,5-dimethylthiazol-2-yl)cyclohexyl)morpholin-2-yl)acetic acid; and
(2*R*,5*S*)-5-(4-chlorobenzyl)-4-(4-(1,5-dimethyl-1*H*-pyrazol-3-yl)cyclohexyl)-2-((ethylsulfonyl)methyl)morpholine;
or is a tautomer, stereoisomer, pharmaceutically acceptable salt, solvate, or polymorph thereof.

*Pharmaceutical Compositions of the Invention*

Another aspect of the invention provides a pharmaceutical composition comprising a therapeutically effective amount of at least one compound according to the invention, and a pharmaceutically acceptable carrier therefor.

The exact nature of the carrier, or, for example excipient or diluent, will depend upon the desired use for the composition, and may be suitable or acceptable for veterinary use and/or suitable or acceptable for human use. The composition may optionally include one or more additional compounds, including one or more additional therapeutic agents.

Compounds of the invention can be combined with other therapeutic agents. The compound of the invention and other therapeutic agent may be administered simultaneously or sequentially. When the other therapeutic agents are administered simultaneously, they can be administered in the same or separate formulations, but they are administered substantially at the same time. The other therapeutic agents are administered sequentially with one another and with compound of the invention, when the administration of the other therapeutic agents and the compound of the invention is temporally separated. The separation in time between the administration of these compounds may be a matter of minutes or it may be longer.

Examples of other therapeutic agents that may be administered with the compounds of the invention include steroids, membrane stabilizers, 5LO inhibitors, leukotriene synthesis and receptor inhibitors, inhibitors of IgE isotype switching or IgE synthesis, inhibitors of IgG isotype switching or IgG synthesis, β-agonists, tryptase inhibitors, acetylsalicylic acid, COX inhibitors, methotrexate, anti-TNF drugs, rituxin, PD4 inhibitors, p38 inhibitors, PDE4 inhibitors, and antihistamines.

Thus, another embodiment of the invention provides a pharmaceutical composition as defined above, that further comprises a therapeutically effective amount of a therapeutic agent selected from the group consisting of steroids, membrane stabilizers, 5LO inhibitors, leukotriene synthesis and receptor inhibitors, inhibitors of IgE isotype switching or IgE synthesis, IgG isotype switching or IgG synthesis, β-agonists, tryptase inhibitors, acetylsalicylic acid, COX inhibitors, methotrexate, anti-TNF drugs, rituxin and other B-cell targeting agents, THF-targeting agents, PD4 inhibitors, p38 inhibitors, PDE4 inhibitors, and antihistamines.

As stated above, an "effective amount" refers to any amount that is sufficient to achieve a desired biological effect. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial unwanted toxicity and yet is effective to treat the particular subject. The effective amount for any particular application can vary depending on such factors as the disease or condition being treated, the particular compound of the invention being administered, the size of the subject, or the severity of the disease or condition. One of ordinary skill in the art can empirically determine the effective amount of a particular compound of the invention and/or other therapeutic agent without necessitating undue experimentation. It is preferred generally that a maximum dose be used, that is, the highest safe dose according to some medical judgment. Multiple doses per day may be contemplated to achieve appropriate systemic levels of compounds. Appropriate systemic levels can be determined by, for example, measurement of the patient's peak or sustained plasma level of the drug. "Dose" and "dosage" are used interchangeably herein.

Generally, daily oral doses of active compounds will be, for human subjects, from about 0.0001 milligrams/kg per day, 0.001 milligrams/kg per day, or 0.01 milligrams/kg per day to about 100 milligrams/kg per day or 1000 milligrams/kg per day. It is expected that oral doses in the range of 0.5 to 50 milligrams/kg, in one or several administrations per day, will yield the desired results. Dosage may be adjusted appropriately to achieve desired drug levels sufficient to achieve or maintain a desired therapeutic effect, local or systemic, depending upon the mode of administration. For example, it is expected that intravenous administration would be from one order to several orders of magnitude lower dose per day. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of compounds. The compounds may be administered once per week, several times per week (*e.g.*, every other day), once per day or multiple times per day, depending upon, among other things, the mode of administration, the specific indication being treated and the judgment of the prescribing physician.

In one embodiment, intravenous administration of a compound of the invention may typically be from 0.1 mg/kg/day to 20 mg/kg/day.

Determination of an effective dosage of a compound for a particular use and mode of administration is well within the capabilities of those skilled in the art. Effective dosages may be estimated initially from *in vitro* activity and metabolism assays. For example, an initial dosage of compound for use in animals may be formulated to achieve a circulating blood or serum concentration of the metabolite active compound that is at or above an $IC_{50}$ of the particular compound as measured in as *in vitro* assay. Calculating dosages to achieve such circulating blood or serum concentrations taking into account the bioavailability of the particular compound *via* the desired route of administration is well within the capabilities of skilled artisans. Initial dosages of compound can also be estimated from *in vivo* data, such as animal models. For any compound described herein the therapeutically effective amount can be initially determined from animal models. A therapeutically effective dose can also be determined from human data for compounds of the invention which have been tested in humans and for compounds which are known to exhibit similar pharmacological activities, such as other related active agents. Higher doses may be required for parenteral administration. The applied dose can be adjusted based on the relative bioavailability and potency of the administered compound. Adjusting the dose to achieve maximal efficacy based on the methods described above and other methods as are well-known in the art is well within the capabilities of the ordinarily skilled artisan.

The formulations of the invention can be administered in pharmaceutically acceptable solutions, which may

routinely contain pharmaceutically acceptable concentrations of salt, buffering agents, preservatives, compatible carriers, adjuvants, and optionally other therapeutic ingredients.

Pharmaceutical compositions comprising the compound of the invention may be manufactured by means of conventional mixing, dissolving, granulating, dragée-making, levigating, emulsifying, encapsulating, entrapping or lyophilization processes. The compositions may be formulated in conventional manner using one or more physiologically acceptable carriers, diluents, excipients or auxiliaries which facilitate processing of the compounds into preparations which can be used pharmaceutically.

For use in therapy, an effective amount of the compound of the invention can be administered to a subject by any mode that delivers the compound of the invention to the desired surface. Administering the pharmaceutical composition of the present invention may be accomplished by any means known to the skilled artisan. Routes of administration include but are not limited to oral, buccal, nasal, rectal, vaginal, ocular, topical, intravenous, intramuscular, intraperitoneal, subcutaneous, transdermal, intrathecal, direct injection (for example, into an abscess), mucosal, inhalation, and insufflation.

For oral administration, the compounds (*i.e.,* compounds of the invention, and other therapeutic agents) can be formulated readily by combining the active compound(s) with pharmaceutically acceptable carriers well known in the art. Such carriers enable the compounds of the invention to be formulated as tablets, pills, dragées, lozenges, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragée cores. Suitable excipients are, in particular, binding agents, fillers, lubricants, disintegrants, and wetting agents. Suitable fillers include sugars, including lactose, sucrose, mannitol, or sorbitol; cellulose preparations such as, for example, maize starch, wheat starch, rice starch, potato starch, gelatin, gum tragacanth, methyl cellulose, hydroxypropyl methyl cellulose, sodium carboxymethyl cellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers, *e.g.*, EDTA for neutralizing internal acid conditions or may be administered without any carriers.

Also specifically contemplated are oral dosage forms of the above component or components. The component or components may be chemically modified so that oral delivery of the derivative is efficacious. Generally, the chemical modification contemplated is the attachment of at least one moiety to the component molecule itself, where said moiety permits (a) inhibition of acid hydrolysis; and (b) uptake into the blood stream from the stomach or intestine. Also desired is the increase in overall stability of the component or components and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis, "Soluble Polymer-Enzyme Adducts", In: Enzymes as Drugs, Hocenberg and Roberts, eds., Wiley-Interscience, New York, N.Y., pp. 367-383 (1981); Newmark et al., J Appl Biochem 4:185-9 (1982). Other polymers that could be used are poly-1,3-dioxolane and poly-1,3,6-tioxocane. Preferred for pharmaceutical usage, as indicated above, are polyethylene glycol moieties.

For the component (or derivative) the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the compound of the invention (or derivative) or by release of the biologically active material beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropyl methyl cellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic (*e.g.*, powder); for liquid forms, a soft gelatin shell may be used. The shell material of cachets could be thick starch or other edible paper. For pills, lozenges, molded tablets or tablet triturates, moist massing techniques can be used.

The therapeutic can be included in the formulation as fine multi-particulates in the form of granules or pellets of particle size about 1 mm. The formulation of the material for capsule administration could also be as a powder, lightly compressed plugs or even as tablets. The therapeutic could be prepared by compression.

Colorants and flavoring agents may all be included. For example, the compound of the invention (or derivative) may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, $\alpha$-lactose, anhydrous lactose, cellulose, sucrose, modified dextrans and starch.

Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrates include but are not limited to starch, including the commercial disintegrant based on starch, Explotab. Sodium starch glycolate, Amberlite, sodium carboxymethyl cellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropyl methyl cellulose (HPMC) could both be used in alcoholic solutions to granulate the therapeutic.

An anti-frictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents which can be used and can include benzalkonium chloride and benzethonium chloride. Potential non-ionic detergents that could be included in the formulation as surfactants include lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the compound of the invention or derivative either alone or as a mixture in different ratios.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

Liquid preparations for oral administration may take the form of, for example, elixirs, solutions, syrups or suspensions, or they may be presented as a dry product for constitution with water or other suitable vehicle before use. Such liquid preparations may be prepared by conventional means with pharmaceutically acceptable additives such as suspending agents (*e.g.*, sorbitol syrup, cellulose derivatives or hydrogenated edible fats); emulsifying agents (*e.g.*, lecithin or acacia); non-aqueous vehicles (*e.g.*, almond oil, oily esters, ethyl alcohol, or fractionated vegetable oils); and preservatives (*e.g.*, methyl or propyl-*p*-hydroxybenzoates or sorbic acid). The preparations may also contain buffer salts, preservatives, flavoring, coloring and sweetening agents as appropriate.

The pharmaceutical compositions may, if desired, be presented in a pack or dispenser device which may contain one or more unit dosage forms containing the compound(s). The pack may, for example, comprise metal or plastic foil, such as a blister pack. The pack or dispenser device may be accompanied by instructions for administration.

For buccal administration, the compositions may take the form of tablets or lozenges formulated in conventional manner.

For topical administration, the compound may be formulated as solutions, gels, ointments, creams, suspensions, etc. as are well-known in the art. Systemic formulations include those designed for administration by injection, *e.g.*, subcutaneous, intravenous, intramuscular, intrathecal or intraperitoneal injection, as well as those designed for transdermal, transmucosal oral or pulmonary administration.

For administration by inhalation, the compounds for use according to the present invention may be conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.,* gelatin for use in an inhaler or insufflator may be formulated containing a powder mix of the compound and a suitable powder base such as lactose or starch.

Also contemplated herein is pulmonary delivery of the compounds of the invention (or derivatives thereof). The compound of the invention (or derivative) is delivered to the lungs of a mammal while inhaling and traverses across the lung epithelial lining to the blood stream. Other reports of inhaled molecules include Adjei et al., Pharm Res 7:565-569 (1990);

Adjei et al., Int J Pharmaceutics 63:135-144 (1990) (leuprolide acetate); Braquet et al., J Cardiovasc Pharmacol 13(suppl. 5):143-146 (1989) (endothelin-1); Hubbard et al., Annal Int Med 3:206-212 (1989) ($\alpha$1-antitrypsin); Smith et al., 1989, J Clin Invest 84:1145-1146 (a-1-proteinase); Oswein et al., 1990, "Aerosolization of Proteins", Proceedings of Symposium on Respiratory Drug Delivery II, Keystone, Colorado, March, (recombinant human growth hormone); Debs et al., 1988, J Immunol 140:3482-3488 (interferon-gamma and tumor necrosis factor alpha) and Platz et al., U.S. Pat. No. 5,284,656 (granulocyte colony stimulating factor). A method and composition for pulmonary delivery of drugs for systemic effect is described in U.S. Pat. No. 5,451,569, issued Sep. 19, 1995 to Wong *et al.*

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art.

Some specific examples of commercially available devices suitable for the practice of this invention are the Ultravent nebulizer, manufactured by Mallinckrodt, Inc., St. Louis, Mo.; the Acorn II nebulizer, manufactured by Marquest Medical Products, Englewood, Colo.; the Ventolin metered dose inhaler, manufactured by Glaxo Inc., Research Triangle Park, North Carolina; the Spinhaler powder inhaler, manufactured by Fisons Corp., Bedford, Mass.; and the Respimat Soft Mist Inhaler, manufactured by Boehringer Ingelheim, Germany. Other hand-driven or human-powered inhaler devices are also applicable.

All such devices require the use of formulations suitable for the dispensing of compound of the invention (or derivative). Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated. Chemically modified compound of the invention may also be prepared in different formulations depending on the type of chemical modification or the type of device employed.

Formulations suitable for use with a nebulizer, either jet, ultrasonic, or soft mist type, will typically comprise compound of the invention (or derivative) dissolved in water at a concentration of about 0.1 to 25 mg of biologically active compound of the invention per mL of solution. The formulation may also include a buffer and a simple sugar (*e.g.,* for compound of the invention stabilization and regulation of osmotic pressure). The nebulizer formulation may also contain a surfactant, to reduce or prevent surface induced aggregation of the compound of the invention caused by atomization of the solution in forming the aerosol.

Formulations for use with a metered-dose inhaler device will generally comprise a finely divided powder containing the compound of the invention (or derivative) suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoro-ethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing compound of the invention (or derivative) and may also include a bulking agent, such as lactose, sorbitol, sucrose, or mannitol in amounts which facilitate dispersal of the powder from the device, *e.g.,* 50 to 90% by weight of the formulation. The compound of the invention (or derivative) should advantageously be prepared in particulate form with an average particle size of less than 10 micrometers ($\mu$m), most preferably 0.5 to 5 $\mu$m, for most effective delivery to the deep lung.

Nasal delivery of a pharmaceutical composition of the present invention is also contemplated. Nasal delivery allows the passage of a pharmaceutical composition of the present invention to the blood stream directly after administering the therapeutic product to the nose, without the necessity for deposition of the product in the lung. Formulations for nasal delivery include those with dextran or cyclodextran.

For nasal administration, a useful device is a small, hard bottle to which a metered dose sprayer is attached. In one embodiment, the metered dose is delivered by drawing the pharmaceutical composition of the present invention solution into a chamber of defined volume, which chamber has an aperture dimensioned to aerosolize and aerosol formulation by forming a spray when a liquid in the chamber is compressed. The chamber is compressed to administer the pharmaceutical composition of the present invention. In a specific embodiment, the chamber is a piston arrangement. Such devices are commercially available.

Alternatively, a plastic squeeze bottle with an aperture or opening dimensioned to aerosolize an aerosol formulation by forming a spray when squeezed is used. The opening is usually found in the top of the bottle, and the top is generally tapered to partially fit in the nasal passages for efficient administration of the aerosol formulation. Preferably, the nasal inhaler will provide a metered amount of the aerosol formulation, for administration of a measured dose of the drug.

The compounds, when it is desirable to deliver them systemically, may be formulated for parenteral administration by injection, *e.g.*, by bolus injection or continuous infusion. Formulations for injection may be presented in unit dosage form, *e.g.,* in ampoules or in multidose containers, with an added preservative. The compositions may take such forms as sterile suspensions, solutions, or emulsions in oily or aqueous vehicles, and may contain formulatory agents such

as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, *e.g.*, sterile pyrogen-free water, buffer, dextrose solution, before use. To this end, the active compound may be dried by any art-known technique, such as lyophilization, and reconstituted prior to use.

The compounds may also be formulated in rectal or vaginal compositions such as suppositories or retention enemas, *e.g.*, containing conventional suppository bases such as cocoa butter or other glycerides.

For transmucosal administration, penetrants appropriate to the barrier to be permeated are used in the formulation. Such penetrants are known in the art.

For ocular administration, the compound(s) may be formulated as a solution, emulsion, suspension, etc. suitable for administration to the eye. A variety of vehicles suitable for administering compounds to the eye are known in the art.

In addition to the formulations described above, for prolonged delivery, the compounds may also be formulated as a depot preparation for administration by, for example, implantation or intramuscular injection. Such long acting formulations may be formulated with suitable polymeric or hydrophobic materials (for example as an emulsion in an acceptable oil) or ion exchange resins, or as sparingly soluble derivatives, for example, as a sparingly soluble salt. Alternatively, transdermal delivery systems manufactured as an adhesive disc or patch which slowly releases the compound for percutaneous absorption may be used. To this end, permeation enhancers may be used to facilitate transdermal penetration of the compound.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer R, Science 249:1527-33 (1990).

The compounds of the invention and optionally other therapeutics may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulfuric, nitric, phosphoric, maleic, acetic, salicylic, *p*-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group. Typically, such salts are more soluble in aqueous solutions than the corresponding free acids and bases, but salts having lower solubility than the corresponding free acids and bases may also be formed.

The compounds may alternatively be formulated in the pharmaceutical composition per se, or in the form of a hydrate, solvate, or N-oxide.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

Pharmaceutical compositions of the invention contain an effective amount of a compound of the invention and optionally therapeutic agents included in a pharmaceutically acceptable carrier. The term "pharmaceutically acceptable carrier" means one or more compatible solid or liquid filler, diluents or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The therapeutic agent(s), including specifically but not limited to the compound of the invention, may be provided

in particles. Particles as used herein means nanoparticles or microparticles (or in some instances larger particles) which can consist in whole or in part of the compound of the invention or the other therapeutic agent(s) as described herein. The particles may contain the therapeutic agent(s) in a core surrounded by a coating, including, but not limited to, an enteric coating. The therapeutic agent(s) also may be dispersed throughout the particles. The therapeutic agent(s) also may be adsorbed into the particles. The particles may be of any order release kinetics, including zero-order release, first-order release, second-order release, delayed release, sustained release, immediate release, and any combination thereof, etc. The particle may include, in addition to the therapeutic agent(s), any of those materials routinely used in the art of pharmacy and medicine, including, but not limited to, erodible, nonerodible, biodegradable, or nonbiodegradable material or combinations thereof. The particles may be microcapsules which contain the compound of the invention in a solution or in a semi-solid state. The particles may be of virtually any shape.

Both non-biodegradable and biodegradable polymeric materials can be used in the manufacture of particles for delivering the therapeutic agent(s). Such polymers may be natural or synthetic polymers. The polymer is selected based on the period of time over which release is desired. Bioadhesive polymers of particular interest include bioerodible hydrogels described in Sawhney H S et al. (1993) Macromolecules 26:581-7. These include polyhyaluronic acids, casein, gelatin, glutin, polyanhydrides, polyacrylic acid, alginate, chitosan, poly(methyl methacrylates), poly (ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

The therapeutic agent(s) may be contained in controlled release systems. The term "controlled release" is intended to refer to any drug-containing formulation in which the manner and profile of drug release from the formulation are controlled. This refers to immediate as well as non-immediate release formulations, with non-immediate release formulations including but not limited to sustained release and delayed release formulations. The term "sustained release" (also referred to as "extended release") is used in its conventional sense to refer to a drug formulation that provides for gradual release of a drug over an extended period of time, and that preferably, although not necessarily, results in substantially constant blood levels of a drug over an extended time period. The term "delayed release" is used in its conventional sense to refer to a drug formulation in which there is a time delay between administration of the formulation and the release of the drug there from. "Delayed release" may or may not involve gradual release of drug over an extended period of time, and thus may or may not be "sustained release."

Use of a long-term sustained release implant may be particularly suitable for treatment of chronic conditions. "Long-term" release, as used herein, means that the implant is constructed and arranged to deliver therapeutic levels of the active ingredient for at least 7 days, and preferably 30-60 days. Long-term sustained release implants are well-known to those of ordinary skill in the art and include some of the release systems described above.

It will be understood by one of ordinary skill in the relevant arts that other suitable modifications and adaptations to the compositions and methods described herein are readily apparent from the description of the invention contained herein in view of information known to the ordinarily skilled artisan, and may be made without departing from the scope of the invention or any embodiment thereof.

*Methods and Uses*

As shown herein, the compounds of the invention are useful for inhibiting binding activity of the chitinase-like protein YKL-40.

Accordingly, the invention provides methods for inhibiting the chitinase-like protein YKL-40 in a cell or a tissue, comprising contacting the cell or the tissue with at least one compound according to the invention, or with a pharmaceutical composition according to the invention.

In other aspects, the invention provides methods for the treatment or prevention of a disease, disorder, or condition associated with aberrant expression or activity of the chitinase-like protein YKL-40, comprising administering to a subject in need thereof a therapeutically effective amount of at least one compound according to the invention, or of a pharmaceutical composition according to the invention.

Preferably, the disease, disorder, or condition is selected from the group consisting of cancer, allergic diseases, acute and chronic inflammatory diseases, autoimmune diseases, dental diseases, neurologic diseases, metabolic diseases, liver diseases, kidney diseases, polycystic ovary syndrome, endometriosis, asthma, and fibrotic disorders.

In one particular aspect, the disease is cancer selected from the group consisting of glioblastoma, breast cancer, colon cancer, kidney cancer, uterine cancer, primary and metastatic lung cancer, mesothelioma, osteosarcoma, malignant melanoma, ovarian cancer, cervical cancer, prostate cancer, liver cancer, gastric cancer, metastatic renal cancer, leukemia, lymphoma, oligodendroglioma, glioblastoma, and germ cell tumors.

In another particular aspect, the disease, disorder, or condition is an allergic disease selected from the group consisting of asthma, allergic rhinitis, seasonal allergic rhinitis, chronic rhinosinusitis with or without nasal polyps, conjunctivitis, keratoconjunctivitis, seasonal allergic conjunctivitis, dry eye syndrome, eosinophilic esophagitis, celiac

disease, food allergy, irritable bowel syndrome, irritable bowel disease, atopic eczema, atopic dermatitis, allergic contact dermatitis, eosinophilic otitis media, eosinophilic pneumonia, and IgG4 mediated diseases.

In a further particular aspect, disease, disorder, or condition is an acute or chronic inflammatory disease selected from the group consisting of fungal diseases, parasitic infections, celiac disease, microscopic colitis, chronic obstructive pulmonary disease (COPD), idiopathic pulmonary fibrosis, interstitial lung diseases, cystic fibrosis, Hermansky-Pudlak syndrome, and Alzheimer's disease.

In another aspect, the disease, disorder, or condition is an autoimmune disorder selected from the group consisting of inflammatory bowel disease, ulcerative colitis, Crohn's disease, rheumatoid arthritis, osteoarthritis, psoriasis, scleroderma, multiple sclerosis, Sjögren's syndrome, atherosclerosis, and sarcoidosis.

In one particular aspect, the disease, disorder, or condition is periodontitis.

In another aspect, the disease, disorder, or condition is a metabolic disease selected from the group consisting of insulin-dependent diabetes mellitus and non-insulin-dependent diabetes mellitus.

In a further aspect, the disease, disorder, or condition is a liver disease selected from the group consisting of non-alcoholic fatty liver disease, non-alcoholic steatohepatitis, hepatitis-C virus-induced fibrosis and cirrhosis, and alcoholic fibrosis.

In another aspect, the disease is a kidney disease selected from the group consisting of nephropathy (*e.g.*, diabetic nephropathy), focal segmental glomerulosclerosis, tubulointerstitial fibrosis, postransplant fibrosis, and retroperitoneal fibrosis (Ormond's disease).

In one particular aspect, the disease, disorder, or condition is a fibrotic disorder.

Preferably, the fibrotic disorder is idiopathic pulmonary fibrosis (IPF).

In another aspect, the disease is a storage disease selected from the group consisting of Gaucher disease, Fabry disease, lysosomal storage disorders, Niemann-Pick disease, nephropathic cystinosis, and X-linked globotriaosylceramidosis.

Moreover, the invention provides methods of treating diseases caused by infectious agents, such as fungi, worms, and parasites, the method comprising administering to a subject in need of such treatment an effective amount of one or more compounds of the invention.

In one embodiment, the invention provides methods of treating allergies, comprising administering to a subject in need of such treatment an effective amount of one or more compounds of the invention. In certain embodiments, such allergies are caused by any of a variety of antigens including biological sources such as dust mites, mold, cockroaches and other insects, dander from pets or other mammals, pollens, spores, mold, other fungal sources, and other plant antigens, or non-biological sources such as chemicals (*e.g.*, isocyanates).

In other embodiments, the invention provides a method of screening for therapeutic agents useful for treating asthma in a mammal, comprising: (a) contacting the chitinase-like protein YKL-40 with a compound (*e.g.*, a compound of the invention) and a substrate of said chitinase-like protein; and (b) determining if the compound inhibits the binding activity of the chitinase-like protein; wherein if the compound inhibits the binding activity of the chitinase-like protein, then the compound is a therapeutic agent useful for treating asthma.

In other aspects, the invention provides methods for monitoring the efficacy of a treatment for asthma, comprising (a) administering a compound of the invention to a mammal, and (b) monitoring the expression of the chitinase-like protein YKL-40 in the mammal after administration of the compound, wherein a decrease in the expression of the chitinase-like protein YKL-40 indicates that the compound is useful in treating asthma, allergic diseases such as hay fever, allergic rhinitis, atopic dermatitis or other Th-2 mediated or associated diseases.

In other aspects, the invention provides methods for monitoring the efficacy of a treatment for asthma and other allergic diseases, comprising (a) administering a compound of the invention to a mammal, and (b) monitoring the expression of inflammatory mediators such as IL-13, IL-5, IL-4, eotaxin, or IgE or inflammatory cells such as eosinophils, neutrophils, or lymphocytes in broncho-alveolar washings, sputum, or tissues obtained from the mammal after administration of the compound; wherein a decrease in expression indicates that the compound is useful in treating asthma or allergic diseases such as hay fever, allergic rhinitis, atopic dermatitis or other Th-2 mediated or associated diseases.

In another aspect, the invention provides methods for assessing the efficacy of an agent for treating asthma in a subject, comprising the steps of:

a) detecting in a subject sample collected at a first point in time the expression level of the chitinase-like protein YKL-40;
b) repeating step a) at one or more subsequent points in time after administration of the agent; and
c) comparing expression level of the chitinase-like protein YKL-40 detected in step a) with the expression level(s) detected in step(s) b),

wherein a higher expression level of the chitinase-like protein YKL-40 at the first point in time relative to at least one subsequent point in time indicates that the agent is efficacious in treating asthma.

In certain embodiments, an agent identified by such a method is efficacious in treating asthma, hay fever, allergic rhinitis, atopic dermatitis, allergic reactions, or a disorder associated with Th-2.

Alternatively, the efficacy of an agent for treating asthma or an allergic reaction can be assessed *via* measuring the expression level of an inflammatory mediator such as IL-13, IL-5, IL-4, eotaxin, IgE, or measuring the amount of inflammatory cells such as eosinophils, neutrophils, or lymphocytes in broncho-alveolar washings, sputum, or tissues obtained from a mammal. In certain such embodiments, the expression level can be measured prior to and after administration of an agent. When the expression level of the inflammatory mediator or the level of inflammatory cells decreases after administration of an agent, such an agent is efficacious in treating asthma, hay fever, allergic rhinitis, atopic dermatitis, allergic reactions, or a disorder associated with Th-2.

Another aspect of the invention provides methods of identifying an agent for treating asthma, comprising:

a) contacting a sample comprising the chitinase-like protein YKL-40 with the agent; and
b) determining the ability of the agent to inhibit binding activity of the chitinase-like protein YKL-40, wherein decreased activity of the chitinase-like protein YKL-40 identifies an agent for treating asthma.

In certain embodiments, the binding activity of the chitinase-like protein YKL-40 is assessed by MicroScale Thermophoresis direct binding assay and AlphaScreen indirect binding assays using fluorescently labelled YKL-40 protein and biotinylated small molecule compound binding YKL-40, respectively.

Another aspect of the invention provides a method of identifying compounds that interfere with the chitinase-like protein YKL-40 and asialofetuin interaction by:

(1) in a binding competition assay, subjecting (i) a compound according to the invention, and (ii) biotinylated asialofetuin to YKL-40 or derivative of YKL-40 comprising a purification tag; and
(2) assessing the resulting AlphaScreen luminescence signal; wherein an emission signal decline from the high level at lowest inhibitor concentration to the lower level (diminished by the value of at least 3x standard deviation of the noise displayed at plateau values at highest concentrations of inhibitor in the response plots for IC50 determinations) at highest inhibitor concentration indicates that the compound interferes with the chitinase-like protein YKL-40 and asialofetuin interaction.

*Therapeutic Applications*

The inventive compounds are useful for inhibiting the biological activity of the chitinase-like protein YKL-40. The expression of chitinase-like protein YKL-40 has been linked to interstitial lung diseases (ILDs) including idiopathic pulmonary fibrosis (IPF) and chronic obstructive pulmonary disease (COPD).

In some embodiments, the invention provides the inventive compounds for the treatment or prevention of a disease or condition associated with expression or biological activity of YKL-40 in a subject in need thereof. For instance, the disease, disorder, or condition is selected from the group consisting of cancer, allergic diseases, acute and chronic inflammatory diseases, autoimmune diseases, dental diseases, neurologic diseases, metabolic diseases, liver diseases, polycystic ovary syndrome, endometriosis, asthma, and fibrotic disorders.

According to certain embodiments, the invention provides the inventive compounds for the treatment of allergic diseases, such as asthma, allergic rhinitis, seasonal allergic rhinitis, chronic rhinosinusitis with or without nasal polyps, conjunctivitis, keratoconjunctivitis, seasonal allergic conjunctivitis, dry eye syndrome, eosinophilic esophagitis, celiac disease, food allergies, irritable bowel syndrome, irritable bowel disease, atopic eczema, atopic dermatitis, allergic contact dermatitis, eosinophilic otitis media, eosinophilic pneumonia, and IgG4 mediated disease.

In certain embodiments, the reaction caused by an allergen is allergic rhinitis or atopic dermatitis.

In certain embodiments, the reaction caused by an allergen is characterized by the occurrence of one or more symptoms, which can include red eyes, itchiness, runny nose, eczema, impaired hearing, hives, an asthma attack, increased mucus production in the lungs, coughing, wheezing, and shortness of breath.

Exemplary acute and chronic inflammatory disorders that can be treated using the inventive compounds include fungal diseases, parasitic infection, celiac disease, microscopic colitis, chronic obstructive pulmonary disease (COPD), idiopathic pulmonary fibrosis, interstitial lung diseases, cystic fibrosis (CF), Hermansky-Pudlak and Alzheimer’s disease (AD).

In certain embodiments, the disease or condition to be treated is an autoimmune disorder selected from the group consisting of inflammatory bowel disease, ulcerative colitis (UC), Crohn’s disease (CD), rheumatoid arthritis (RA), osteoarthritis, psoriasis, scleroderma, multiple sclerosis (MS), Sjögren’s syndrome, atherosclerosis, and sarcoidosis.

The inventive compounds are also useful for treating dental diseases such as periodontitis.

The compounds of the invention are also useful for treating metabolic diseases such as insulin-dependent diabetes mellitus (IDDM) and non-insulin-dependent diabetes mellitus (NIDDM).

In certain embodiments, the invention provides the inventive compounds for the treatment of a liver disease selected from group consisting of non-alcoholic fatty liver disease, non-alcoholic steatohepatitis, hepatitis-C virus-induced fibrosis and cirrhosis, and alcoholic fibrosis.

In some embodiments, the invention provides the inventive compounds for the treatment of cancer, wherein the cancer is selected from the group consisting of glioblastoma, breast cancer, colon cancer, kidney cancer, uterine cancer, primary and metastatic lung cancer, mesothelioma, osteosarcoma, malignant melanoma, ovarian cancer, cervical cancer, prostate cancer, liver cancer, gastric cancer, metastatic renal cancer, leukemia, lymphoma, oligodendroglioma, glioblastoma and germ cell tumors.

In certain embodiments, the disease or condition to be treated is a kidney disease selected from the group consisting of nephropathy (*e.g.*, diabetic nephropathy), focal segmental glomerulosclerosis, tubulointerstitial fibrosis, postransplant fibrosis, and retroperitoneal fibrosis (Ormond's disease).

In certain embodiments, the disease or condition to be treated is a storage disease selected from the group consisting of Gaucher disease, Fabry disease, lysosomal storage disorders, Niemann-Pick disease, nephropathic cystinosis, and X-linked globotriaosylceramidosis.

In some embodiments, the subject receiving treatment is a mammal. For instance, the methods and uses described herein are suitable for medical use in humans. Alternatively, the methods and uses are also suitable in a veterinary context, wherein the invention may be administered to warm-blooded animals, birds and reptiles. Warm-blooded animals include, for example, all non-human primates (*e.g.,* chimpanzee and ape), ruminants (*e.g.,* cow, sheep and goat), porcines (*e.g.,* pig), equines (*e.g.,* horse, mule and donkey), camelines (*e.g.,* camel and dromedary), canines (*e.g.,* dog), felines (*e.g.,* cat), leporine (*e.g.,* rabbit), murines (*e.g.,* mouse and rat) cavines (*e.g.,* guinea pig), gerbiline (*e.g.,* gerbil), cricetine (*e.g.,* hamster), mustelines (*e.g.,* ferret and weasel) and chinchilines (*e.g.*, chinchilla). Birds include animals of the avian class, for example, all phasianines (*e.g.,* chicken and quail), anserines (*e.g.,* goose), anatines (*e.g.,* ducks), meleagridines (*e.g.,* turkey), daruduelines (*e.g.,* canary), psittacines (*e.g.,* parrot, macaw, parakeet and lovebird), cacatuines (*e.g.,* cockatoo) and columbines (*e.g.,* pigeon and turtle dove).

In certain embodiments, the inventive compound or pharmaceutical composition thereof is preferably administered to domesticated companion animals and to productive and breeding animals.

In certain embodiments, the treatment further comprises administering a second therapeutic agent. Exemplary second therapeutic agents include steroids, membrane stabilizers, 5LO inhibitors, leukotriene synthesis and receptor inhibitors, inhibitors of IgE isotype switching or IgE synthesis, inhibitors of IgG isotype switching or IgG synthesis, β-agonists, tryptase inhibitors, acetylsalicylic acid, COX inhibitors, methotrexate, anti-TNF drugs, rituxin and other B-cell targeting agents, THF-targeting agents, PD4 inhibitors, p38 inhibitors, PDE4 inhibitors, and antihistamines.

EXAMPLES

The present invention is further illustrated by the following examples, which in no way should be construed as limiting the scope of the claimed invention.

**Materials and Methods of Preparation and Characterization**

The compounds of the present disclosure may be prepared by use of known chemical reactions and procedures. Representative methods for synthesizing compounds of the disclosure are presented below. It is understood that the nature of the substituents required for the desired target compound often determines the preferred method of synthesis. All variable groups of these methods are as described in the generic description if they are not specifically defined below. Substituents carry the same meaning as defined above, unless otherwise noted.

Those having skill in the art will recognize that the starting materials and reaction conditions may be varied, the sequence of the reactions altered, and additional steps employed to produce compounds encompassed by the present disclosure, as demonstrated by the following examples. Many general references providing commonly known chemical synthetic schemes and conditions useful for synthesizing the disclosed compounds are available (see, *e.g.,* Smith and March, March's Advanced Organic Chemistry: Reactions, Mechanisms, and Structure, Fifth Edition, Wiley-Interscience, 2001; or Vogel, A Textbook of Practical Organic Chemistry, Including Qualitative Organic Analysis, Fourth Edition, New York: Longman, 1978).

The reactions are performed in a solvent appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the disclosure.

In some cases, protection of certain reactive functionalities may be necessary to achieve some of the above transformations. In general, the need for such protecting groups as well as the conditions necessary to attach and remove

such groups will be apparent to those skilled in the art of organic synthesis. An authoritative account describing the many alternatives to the trained practitioner are J. F. W. McOmie, "Protective Groups in Organic Chemistry," Plenum Press, London and New York 1973, in T. W. Greene and P. G. M. Wuts, "Protective Groups in Organic Synthesis," Third edition, Wiley, New York 1999, in "The Peptides;" Volume 3 (editors: E. Gross and J. Meienhofer), Academic Press, London and New York 1981, in "Methoden der organischen Chemie," Houben-Weyl, 4th edition, Vol. 15/1, Georg Thieme Verlag, Stuttgart 1974, in H.-D. Jakubke and H. Jescheit, "Aminosauren, Peptide, Proteine," Verlag Chemie, Weinheim, Deerfield Beach, and Basel 1982, and/or in Jochen Lehmann, "Chemie der Kohlenhydrate: Monosaccharide und Derivate," Georg Thieme Verlag, Stuttgart 1974. The protecting groups may be removed at a convenient subsequent stage using methods known from the art.

Starting materials can be obtained from commercial sources or prepared by well-established literature methods known to those skilled in the art.

All solvents, substrates and reagents that were commercially available were used without further purification. TLC analysis was performed using pre-coated glass plates (0.2 ±0.03 mm thickness, GF-254, particle size 0.01-0.04 mm) from Fluorochem Ltd, UK or using pre-coated glass plates (TLC silica gel 60 $F_{254}$) or using pre-coated aluminium plates (TLC silica gel 60 $F_{254}$) fromMerck. TLC analysis was performed for compounds before their transferring in the appropriate salts (hydrochloride or TFA salt).
The column chromatography was performed using high-purity grade silica gel (pore size 60 Å, 220-440 mesh particle size, 35-75 μm particle size) from Fluka or using high-purity grade silica gel (pore size 60 Å, 230-400 mesh particle size, 40-63 μm particle size) from Merck.

Column chromatography (LCC) was performed using high-purity grade silica gel (pore size 60 Å, 220-440 mesh particle size, 35-75 μm particle size) purchased from Fluka.

Flash column chromatography (FLCC) was performed using Buchi Reverelis® X2-UV equipped in evaporative light scattering detector (ELSD), with UV detection in range UV 200-500 nm, purchased from BÜCHI Labortechnik AG, using puriFlash® pre-packed cartridges with amorphous or spherical virgin silica gel, 50 μm, from InterChim®.

Preparative HPLC were performed on preparative Shimadzu HPLC system containing two pumps LC-20AP, CBM-20A communication module and ELSD-LTII detector with the use of:

- Phenomenex Luna 21.2/250 mm, 5 μm C-18(2) 100 Å column;
- Thermo Scientific Hypersil GOLD 21.2/250 mm, 5 μm C-18 column;
- Phenomenex Luna® 250 × 30 mm, 5 μm Phenyl-Hexyl 100 Å column;
- Phenomenex Luna 250 × 21.2 mm, 5 μm Phenyl-Hexyl 100 Å column;
- Phenomenex Lux® 250 × 21.2 mm Cellulose-4 column;

or on preparative Shimadzu HPLC system containing LC-20AP pumps, CBM-20A communication module and SPD-20A UV/VIS detector and FRC-20 fraction collector with the use of:

- Phenomenex Luna 250 × 21.2 mm, 5 μm Phenyl-Hexyl 100 Å column;
- Phenomenex Luna® 250 × 30 mm, 5 μm Phenyl-Hexyl 100 Å column.

The target compounds, when subjected to reversed-phase chromatographic purification in the presence of TFA, were usually obtained in the form of TFA salts.

$^1$H NMR spectra were recorded on an Agilent 400-MR DD2 400 MHz spectrometer and on Bruker AVANCE DRX500, AVANCE DRX600 and on Bruker AVANCE II PLUS (respectively at 500, 600, or 700 MHz) NMR spectrometers.

All spectra were recorded in appropriate deuterated solvents ($CDCl_3$, DMSO-*d*$_6$, $D_2O$, Methanol-*d*$_4$, *etc.*) that were commercially available.

Resonances are given in parts per million (ppm) relative to tetramethylsilane internal standard. Data are reported as follows: chemical shift (δ), multiplicity (s = singlet, d = doublet, t = triplet, m = multiplet, bs = broad singlet), coupling constants (*J* in Hz) and integration.

ESI-MS spectra were obtained on:

A) Waters Alliance 2695 separation module with a PDA 1996 UV detector and Waters Micromass ZQ 2000 mass detector equipped with Phenomenex Kinetex 2.1/50 mm, 2.6 μm C-18 column eluted with 0.3 mL/min flow of 3-100% gradient (over 6 min) of acetonitrile in water,
B) Shimadzu LCMS system containing LCMS-2020 and SPD-M20A detectors, SIL-20AHT autosampler, LC-20AD pump with LPG module, DGU-20A3R degasser and CMB-20A communication module equipped with:

- Phenomenex Kinetex® 2.1/50 mm, 2.6 μm C-18 column eluted with 0.5 mL/min flow of 10-90% gradient (over 6 or 8 min) of acetonitrile in water;

- Phenomenex Kinetex® 2.6 mm XB C-18 100 Å LC Column 50 × 2.1 mm eluted with 0.5 mL/min flow of 10-90% gradient (over 10 or 16 min) of acetonitrile in water;
- Phenomenex Luna® 3 mm Phenyl-Hexyl 100 Å LC Column 100 × 3 mm eluted with 0.5 mL/min flow of 10-90% gradient (over 10 or 16 or 25 min) of acetonitrile in water.

C) Shimadzu LCMS system containing LCMS-2020 and SPD-M20A detectors, SIL-30ACMP autosampler, two LC-30AD pumps, DGU-20A5R degasser and CTO-20AC column oven equipped with:

- Phenomenex Luna, C18, 2 μm, 100A, 150x3 mm column eluted with 0.5 mL/min flow of 15-90% gradient (over 13 min) of acetonitrile in water or:
  - Phenomenex Kinetex 2.1/30 mm, 1.7 μm XB-C18 100 Å LC column eluted with 1 mL/min flow of 10-90% gradient (over 3 min) of acetonitrile in water.

ESI-MS spectra were obtained in one of the following Methods:

| | |
|---|---|
| **Method A** | UPLC, Solid phase: Kinetex® 1.7μm, XB-C18, LC column 30 × 2.1 mm;<br>Mobile phase: Acetonitrile in water, 1.0 mL/min, gradient<br>$10\% \xrightarrow{2.0\ min} 90\% \xrightarrow{1.0\ min} 90\%$ |
| **Method B** | UPLC, Solid phase: Kinetex® 1.7μm, XB-C18, LC column 30 × 2.1 mm;<br>Mobile phase: Acetonitrile in water, 1.0 mL/min, gradient<br>$10\% \xrightarrow{2.0\ min} 70\% \xrightarrow{0\ min} 90\% \xrightarrow{1.0\ min} 90\%$ |
| **Method C** | HPLC, Solid phase: Luna® 3 μm, Phenyl-Hexyl 100 Å, LC column 100 × 3 mm;<br>Mobile Phase: Acetonitrile in water, 1.5 mL/min, gradient<br>$10\% \xrightarrow{4,5\ min} 70\% \xrightarrow{0\ min} 90\% \xrightarrow{2,5\ min} 90\% \xrightarrow{0\ min} 10\% \xrightarrow{3.0\ min} 10\%$ |
| **Method D** | UPLC, Solid phase: Kinetex® 1.7μm, XB-C18, LC column 30 × 2.1 mm;<br>Mobile phase: Acetonitrile in water, 1.5 mL/min, gradient<br>$10\% \xrightarrow{5.0\ min} 90\% \xrightarrow{1.0\ min} 90\% \xrightarrow{0\ min} 10\% \xrightarrow{2.0\ min} 10\%$ |
| **Method E** | UPLC, Solid phase: Kinetex® 1.7μm, XB-C18, LC column 30 × 2.1 mm;<br>Mobile phase: Acetonitrile in water, 1.0 mL/min, gradient<br>$0\% \xrightarrow{2.0\ min} 50\% \xrightarrow{1.0\ min} 50\%$ |
| **Method F** | UPLC, Solid phase: SecurityGuard™ ULTRA Cartridges UHPLC C18 2.1mm ID Columns; Kinetex® 1.7μm, XB-C18, LC column 30 × 2.1 mm, 100 Å;<br>Mobile phase: Acetonitrile in water, 0.5 mL/min, gradient<br>$10\% \xrightarrow{5.0\ min} 90\% \xrightarrow{1.0\ min} 90\% \xrightarrow{0\ min} 10\% \xrightarrow{2.0\ min} 10\%$ |

Formic acid was used at a concentration of 0.5 weight% (1.1 mL of HCOOH per 2.5 L of the solvent) in all HPLC systems.

Abbreviations used are those conventional in the art or the following:

Ac = acetyl,
AcCl = acetyl chloride,
AcOH = acetic acid,
Alloc = allyloxycarbonyl,
aq = aqueous,
BINAP = 2,2'-bis(diphenylphosphino)-1,1'-binaphthyl,
Bn = benzyl,

Boc = *tert*-butoxycarbonyl,
*t*Bu = *tert*-butyl,
°C = degree Celsius,
CDI = carbonyldiimidazole,
COD = 1,5-cyclooctadiene,
DAST = diethylaminosulfur trifluoride,
dba = dibenzylideneacetone,
DCE = 1,2-dichloroethane,
DCM = dichloromethane,
DIAD = diisopropyl azodicarboxylate,
DIPEA = *N*,*N*-diisopropylethylamine,
DMAP = 4-dimethylaminopyridine,
DMF = *N*,*N*-dimethylformamide,
DMS = dimethyl sulfide,
DMSO = dimethyl sulfoxide,
dppf = 1,1'-ferrocenediyl-bis(diphenylphosphine),
EDCI = 1-ethyl-3-(3-dimethylaminopropyl)carbodiimide,
ELSD = evaporative light scattering detector,
ESI-MS = electrospray ionization mass spectrometry,
$Et_3N$ = triethylamine,
EtOAc or AcOEt = ethyl acetate,
EtOH = ethanol,
FLCC = flash column chromatography,
g = gram,
h = hour(s),
HATU = 1-[bis(dimethylamino)methylene]-1*H*-1,2,3-triazolo[4,5-*b*]pyridinium 3-oxide hexafluorophosphate,
HPLC = high pressure liquid chromatography,
*i*PrOH = isopropyl alcohol,
K = kelvin,
L = liter,
LCC = liquid column chromatography,
LC-MS = liquid chromatography and mass spectrometry,
MeCN = acetonitrile,
MeOH = methanol,
min = minutes,
mL = milliliter(s),
M = molar,
mmol = millimoles,
MOMCl = chloromethyl methyl ether,
Ms = mesyl (methanesulfonyl),
MTBE = methyl *tert*-butyl ether,
m/z = mass to charge ratio,
μg = microgram,
μL = microliter,
μM = micromolar,
nM = nanomolar,
NMM = *N*-methylmorpholine,
NMP = *N*-methyl-2-pyrrolidone,
NMR = nuclear magnetic resonance,
N = normal,
$PEG_{11}$ = polyoxyethylene glycol,
RT or rt = room temperature,
$R_t$ = retention time,
TBDMSCl = *tert*-butyldimethylsilyl chloride,
TBTU = 2-(1*H*-benzotriazole-1-yl)-1,1,3,3-tetramethylaminium tetrafluoroborate,
TFA = trifluoroacetic acid,
TfCl = trifluoromethanesulfonyl chloride,
THF = tetrahydrofuran,

TLC = thin layer chromatography,
TMSCl = chlorotrimethylsilane.

If not otherwise defined, purity of a solid substance is expressed as a ratio of the weight of the component in question to the total weight, multiplied by 100 (weight %); purity of a liquid is expressed as a ratio of the volume of the component in question to the total volume, multiplied by 100 (volume %); concentration of a solution is expressed as a ratio of the weight of the solute (in grams) to the total volume (in mL) of the solution, multiplied by 100 (w/v %). Yield of a reaction is expressed as a ratio of the weight of the product in question to the theoretical yield of this product, multiplied by 100 (%). Composition of a mixed solvent is expressed as a proportion of volume parts of the component solvents (*e.g.*, 3:1).

**YKL-40 Assay**

Full details of the assay were described in the Polish patent application No. P.440558, filed on March 4, 2022, and in the US provisional patent application No. 63316477, filed on March 4, 2022. The specific biotinylated compound used in the above-mentioned patent applications for this assay, "Example 4", was ((2*R*,5*S*)-5-(4-chlorobenzyl)-4-(1-(pyridin-2-yl)piperidin-4-yl)morpholin-2-yl)methyl 41-oxo-45-((3aS,4S,6aR)-2-oxohexahydro-1*H*-thieno[3,4-d]imidazol-4-yl)-4,7,10,13,16,19,22,25,28,31,34,37-dodecaoxa-40-azapentatetracontanoate. Apart from this state-of-the-art compound "Example 4", the biotinylated compound Example 14 from this patent application was also alternatively used as an assay probe.

The standard experimental protocol for YKL-40 indirect binding AlphaScreen assay comprised of 5 step additions to wells of 96 well plate: (i) in the 1st step, 8 μL of the biotinylated compound ("Example 4") was added at 5 x 15 nM concentration in MST buffer containing 1% DMSO; (ii) in the 2nd step, 8 μL of a small molecule compound inhibitor was added at 5 x 1 μM or 5 x 100 μM concentration in MST buffer containing 2% DMSO; (iii) in the 3rd step, 8 μL of YKL-40-histag was added at concentration 5 x 5 nM in MST buffer containing 0% DMSO, after which the plate was spun down for 2 minutes at 1500 g and incubated 1 h at 37 °C; (iv) in the 4th step, 8 μL of Nickel Chelate Acceptor beads were added at concentration 5 x 10 μg/mL in MST buffer containing 1% DMSO, after which the plate was spun down for 1 minute at 250 g and incubated in dark for 1 hour at RT; (v) in the 5th step, 8 μL of Streptavidin Donor beads were added at concentration 5 x 10 μg/mL in MST buffer containing 1% DMSO, after which the plate was spun down for 1 minute at 250 g and incubated in dark for 1 hour at RT. Positive and negative control wells received just 8 μL of MST buffer containing 2% DMSO without inhibitor in the 2nd step (ii). In addition, negative control received just 8 μL of MST buffer containing 0% DMSO without YKL-40-histag in the 3rd step (iii). Finally, the plate luminescence was excited at 680 nm and the emission read at 520-620 nm using the AlphaScreen module in the Spark™ 10 M multimode microplate reader (Tecan Trading AG, Männedorf, Switzerland). Inhibition percentage and $IC_{50}$ values were determined using GraphPad Prism 7.0 software (GraphPad Software, San Diego, CA, USA).

**Orthogonal YKL-40 AlphaScreen assay using biotinylated asialofetuin (AF-biot) in place of Example 14.**

During our studies we found out that YKL-40, being a lectin, can bind oligosaccharides other than oligomers of chitin. Specifically the screening method based on interaction of YKL-40 with heparan sulfate and influence of small molecule binders of YKL-40 on this interaction was described in the Polish patent application No. P.440558, filed on March 4, 2022, in the US provisional patent application No. 63316477, filed on March 4, 2022, and in the US patent application No. 18117193, filed on March 3, 2023. During more extensive search for other oligosaccharides we found out that immobilized YKL40-his on NiNTA BLITz sensors specifically interacts with biotinylated asialofetuin AF-biot (branched triantennary LacNac) with Kd = 2.6 nM in PBS buffer containing 1 mg/ml BSA and 0.05% Tween20, while AF-biot does not interact with bare NiNTA BLITz sensor or same sensor covered with non-YKL40 his-tagged protein. Based on these findings, an alternative AlphaScreen assay using same biotinylated asialofetuin (AF-biot) in place of Example 14 was established.

The standard experimental protocol for YKL-40 indirect binding AlphaScreen assay comprised of 5 step additions to wells of 96 well plate: (i) in the 1st step, the biotinylated asialofetuin was added at 5 x 1.6 nM concentration in assay buffer, i.e. PBS containing 1 mg/ml BSA and 0.05% Tween20; (ii) in the 2nd step, 8 μL of a small molecule compound inhibitor was added at starting dose 5 x 1 μM, 3x serial dilution, 10 data points in the assay buffer containing 2% DMSO; (iii) in the 3rd step, 8 μL of YKL-40-histag was added at concentration 5 x 20 nM in the assay buffer containing 0% DMSO, after which the plate was spun down for 2 minutes at 500 g and incubated 1 h at 37 °C; (iv) in the 4th step, 8 μL of Nickel Chelate Acceptor beads were added at concentration 5 x 10 μg/mL in the assay buffer containing 0% DMSO, after which the plate was spun down for 1 minute at 250 g and incubated in dark for 1 hour at RT; (v) in the 5th step, 8 μL of Streptavidin Donor beads were added at concentration 5 x 10 μg/mL in the assay buffer containing 0% DMSO, after which the plate was spun down for 1 minute at 250 g and incubated in dark for 1 hour at RT. Positive and negative control wells received just 8 μL of the assay buffer containing 2% DMSO without inhibitor in the 2nd step (ii). In addition, negative control received just 8 μL of the assay buffer containing 0% DMSO without YKL-40-histag in the 3nd step (iii). Finally, the plate

luminescence was excited at 680 nm and the emission read at 520-620 nm using the AlphaScreen module in the Spark™ 10 M multimode microplate reader (Tecan Trading AG, Männedorf, Switzerland). Inhibition percentage and $IC_{50}$ values were determined using GraphPad Prism 7.0 software (GraphPad Software, San Diego, CA, USA). Compounds from Examples 4 and 11 (both of this patent application) can substantially interfere with YKL-40 and asialofetuin (triantennary LacNac) interaction - 62% and 64%, with $IC_{50}$ values 22 nM and 20 nM, respectively.

**BLITz system (Sartorius)** that used Bio-layer interferometry technology was applied to assess Kd of biotinylated compounds. 10 min before Kd measurement, streptavidin biosensors were dehydrated in the MST buffer. During dehydration process the 10 mM stock of biotinylated compound in DMSO was diluted in MST buffer to final concentration 0.5 μM. In addition, protein stock of recombinant YKL40 (un-tagged, purified in-house) was also prepared in MST buffer at two concentrations: 200 μM and 400 μM. The dehydrated biosensor was applied on the BLITz system, followed by 5-step procedure: 1) initial baseline for 30 s with MST buffer in tube, 2) loading of 4 μl of biotinylated compound on drop, 3) baseline for 30 s with MST buffer in tube, 4) association of 4 μl of protein stock at one concentration or MST buffer (negative control), 5) dissociation with MST buffer in tube. The same multi-step measurement was done twice for each protein stock concentration and once for negative control. Kd was measured by the BLITz software based on global fitting using the negative control as a reference.

The compounds disclosed in Table 1 below have the $IC_{50}$ values towards YKL-40 generally ranging from about 0.001 μM to about 100 μM. Their ranges of activity have been assigned as follows:

A:

$$< 0.1\ \mu M;$$

B:

$$0.1\text{-}1\ \mu M;$$

C:

$$1\text{-}10\ \mu M;$$

D:

$$10\text{-}100\ \mu M;$$

and

E:

$$> 100\ \mu M.$$

In Table 1, whenever the symbol of range of activity is preceded by asterisk (*), this symbol represents the Kd range rather than the $IC_{50}$ value range. The ranges of activity have been assigned as follows:

*A:

$$< 0.1\ \mu M;$$

*B:

$$0.1\text{-}1\ \mu M;$$

*C:

1-10 μM;

*D:

10-100 μM;

and

*E:

> 100 μM.

Table 1

| Ex.# | Structure | YKL-40, $IC_{50}$ / Kd | IUPAC Name |
|---|---|---|---|
| 1. | 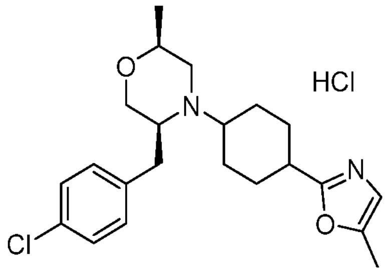 *A single isomer - cis-trans relationship unknown* | A | (2*S*,5*S*)-5-(4-chlorobenzyl)-2-methyl-4-(4-(5-methyloxazol-2-yl)cyclohexyl)morpholine hydro-chloride |
| 2. | 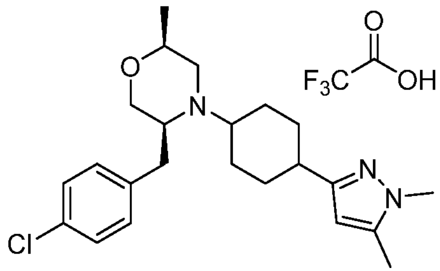 *A single isomer - cis-trans relationship unknown* | A | (2*S*,5*S*)-5-(4-chlorobenzyl)-4-(4-(1,5-dimethyl-1*H*-pyrazol-3-yl)cyclohexyl)-2-methylmorpholine 2,2,2-trifluoroacetate |
| 3. | 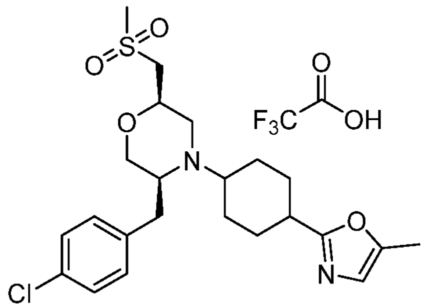 *A single isomer - cis-trans relationship unknown* | A | (2*R*,5*S*)-5-(4-chlorobenzyl)-4-(4-(5-methyloxazol-2-yl)-cyclohexyl)-2-((methylsulfonyl) methyl)-morpholine 2,2,2-trifluoroacetate |

(continued)

| Ex.# | Structure | YKL-40, $IC_{50}$ / Kd | IUPAC Name |
|---|---|---|---|
| 4. | 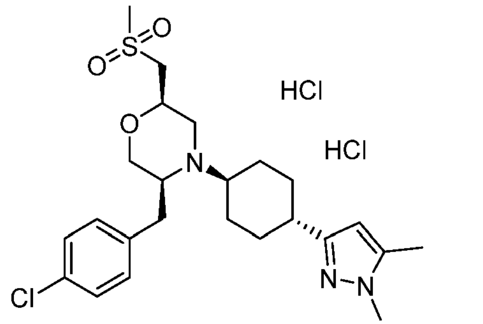 *A single isomer - trans isomer according to X-Ray* | A | (2*R*,5*S*)-5-(4-chlorobenzyl)-4-((1*r*,4*S*)-4-(1,5-dimethyl-1*H*-pyrazol-3-yl)cyclohexyl)-2-((methylsulfonyl)methyl)-morpholine dihydrochloride |
| 5. | 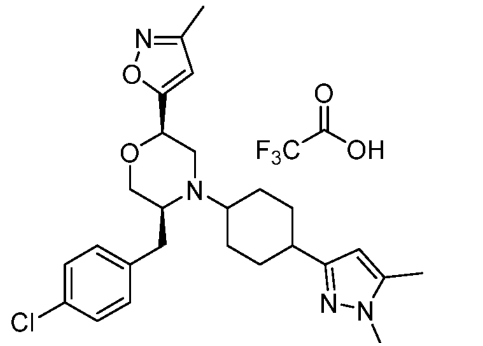 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-2-(3-methylisoxazol-5-yl)morpholine 2,2,2-trifluoroacetate |
| 6. | 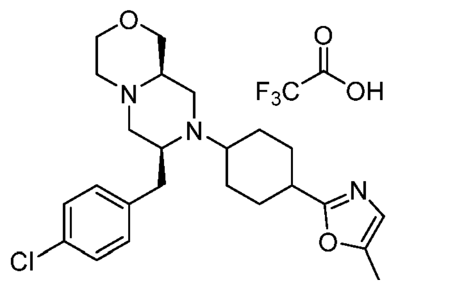 *A single isomer - cis-trans relationship unknown* | A | (7*S*,9*aR*)-7-(4-chlorobenzyl)-8-(4-(5-methyloxazol-2-yl)-cyclohexyl)octahydro-pyrazino[2,1-*c*][1,4]oxazine 2,2,2-trifluoroacetate |

(continued)

| Ex.# | Structure | YKL-40, $IC_{50}$ / Kd | IUPAC Name |
|---|---|---|---|
| 7. | 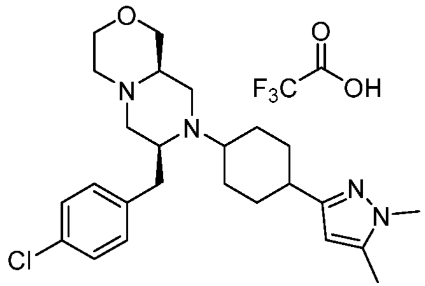 *A single isomer - different to Ex. 8; cis-trans relationship unknown* | A | (7*S*,9a*R*)-7-(4-chlorobenzyl)-8-(4-(1,5-dimethyl-1*H*-pyrazol-3-yl)-cyclohexyl)octahydro-pyrazino [2,1-*c*][1,4]oxazine 2,2,2-trifluoroacetate |
| 8. | 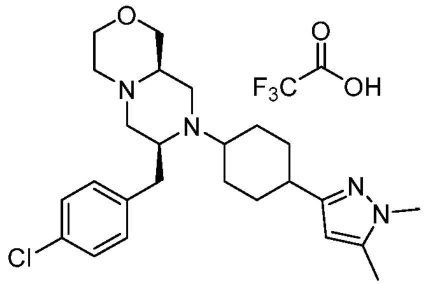 *A single isomer - different to Ex. 7; cis-trans relationship unknown* | A | (7*S*,9a*R*)-7-(4-chlorobenzyl)-8-(4-(1,5-dimethyl-1*H*-pyrazol-3-yl)cyclohexyl)octahydro-pyrazino [2,1-*c*][1,4]oxazine 2,2,2-trifluoroacetate |
| 9. | 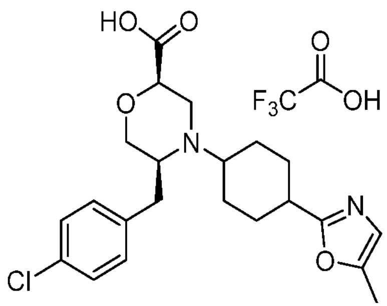 *A single isomer - cis-trans relationship unknown* | A | (2*R*,5*S*)-5-(4-chlorobenzyl)-4-(4-(5-methyloxazol-2-yl)-cyclohexyl)morpholine-2-carboxylic acid 2,2,2-trifluoroacetate |

(continued)

| Ex.# | Structure | YKL-40, $IC_{50}$ / Kd | IUPAC Name |
|---|---|---|---|
| 10. | 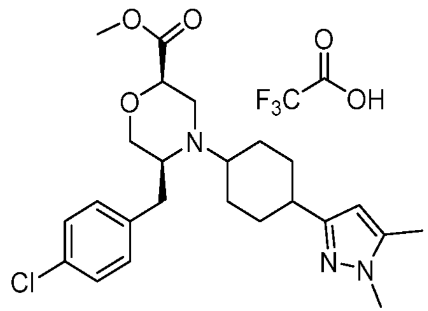 *A single isomer - cis-trans relationship unknown* | A | methyl (2*R*,5*S*)-5-(4-chlorobenzyl)-4-(4-(1,5-dimethyl-1H-pyrazol-3-yl)-cyclohexyl)morpholine-2-carboxylate 2,2,2-trifluoroacetate |
| 11. | 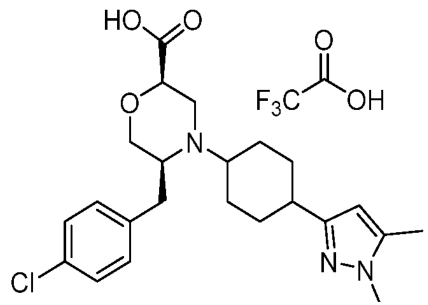 *A single isomer - cis-trans relationship unknown* | A | (2*R*,5*S*)-5-(4-chlorobenzyl)-4-(4-(1,5-dimethyl-1H-pyrazol-3-yl)cyclohexyl)morpholine-2-carboxylic acid 2,2,2-trifluoroacetate |

(continued)

| Ex.# | Structure | YKL-40, $IC_{50}$ / Kd | IUPAC Name |
|---|---|---|---|
| 12. | 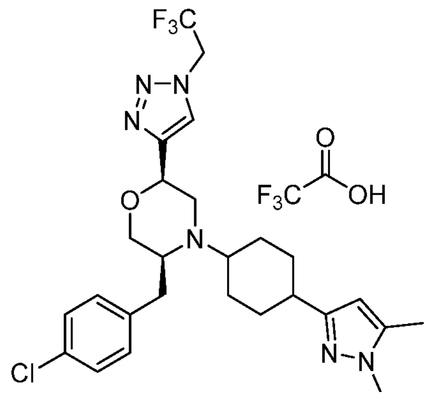 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholine 2,2,2-trifluoroacetate |
| 13. | 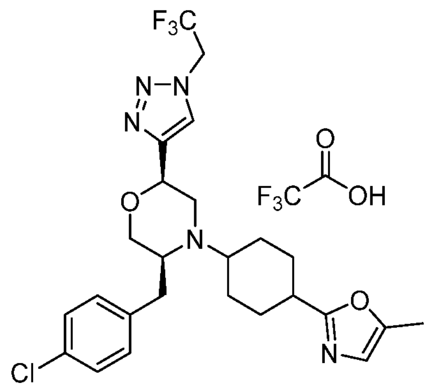 *A single isomer - cis-trans relationship unknown* | A | (2*R*,5*S*)-5-(4-chlorobenzyl)-4-(4-(5-methyloxazol-2-yl)-cyclohexyl)-2-(1-(2,2,2-trifluoroethyl)-1*H*-1,2,3-triazol-4-yl)morpholine 2,2,2-trifluoroacetate |

(continued)

| Ex.# | Structure | YKL-40, $IC_{50}$ / Kd | IUPAC Name |
|---|---|---|---|
| 14. | 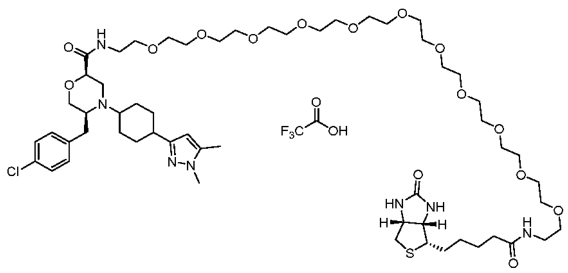 *A single isomer - cis-trans relationship unknown* | *A | (2*R*,5*S*)-5-(4-chlorobenzyl)-4-(4-(1,5-dimethyl-1*H*-pyrazol-3-yl)cyclohexyl)-*N*-(37-oxo-41-((3*aS*,4*S*,6*aR*)-2-oxohexahydro-1*H*-thieno[3,4-d]imidazol-4-yl)-3,6,9,12,15,18,21,24,27,30,33 -undecaoxa-36-azahentetracontyl)morpholine -2-carboxamide 2,2,2-trifluoroacetate |
| 15. | 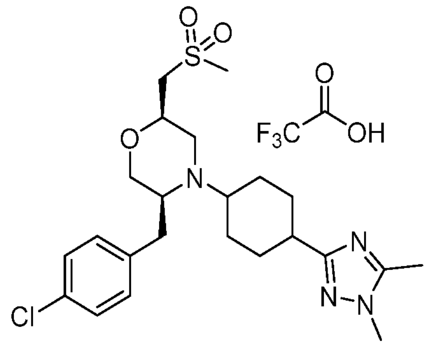 *A single isomer - cis-trans relationship unknown* | A | (2*R*,5*S*)-5-(4-chlorobenzyl)-4-(4-(1,5-dimethyl-1*H*-1,2,4-triazol-3-yl)cyclohexyl)-2-((methylsulfonyl)methyl)-morpholine 2,2,2-trifluoroacetate |
| 16. | 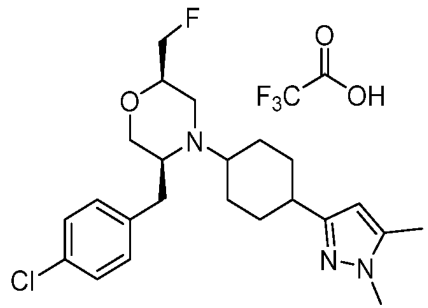 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-2-(fluoromethyl)morpholine 2,2,2-trifluoroacetate |

(continued)

| Ex.# | Structure | YKL-40, $IC_{50}$ / Kd | IUPAC Name |
|---|---|---|---|
| 17. | 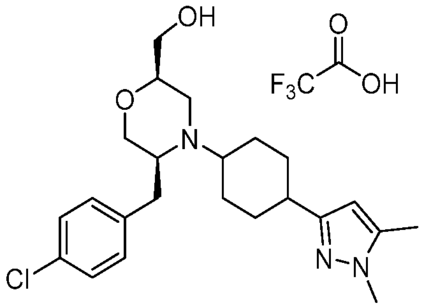 *A single isomer - cis-trans relationship unknown* | A | ((2*R*,5*S*)-5-(4-chlorobenzyl)-4-(4-(1,5-dimethyl-1*H*-pyrazol-3-yl)cyclohexyl)morpholin-2-yl) methanol 2,2,2-trifluoroacetate |
| 18. | 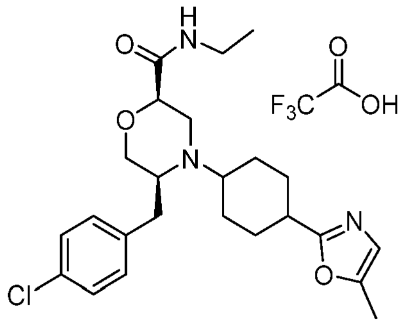 *A single isomer - cis-trans relationship unknown* | A | (2*R*,5*S*)-5-(4-chlorobenzyl)-*N*-ethyl-4-(4-(5-methyloxazol-2-yl)cyclohexyl)morpholine-2-carboxamide 2,2,2-trifluoroacetate |
| 19. | 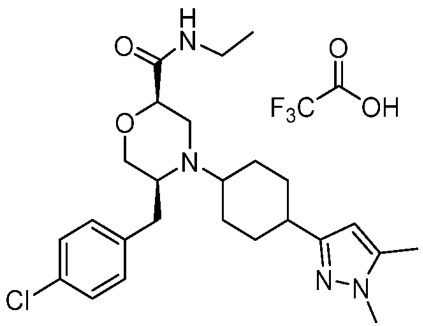 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-*N*-ethylmorpholine-2-carboxamide 2,2,2-trifluoroacetate |

(continued)

| Ex.# | Structure | YKL-40, $IC_{50}$ / Kd | IUPAC Name |
|---|---|---|---|
| 20. | 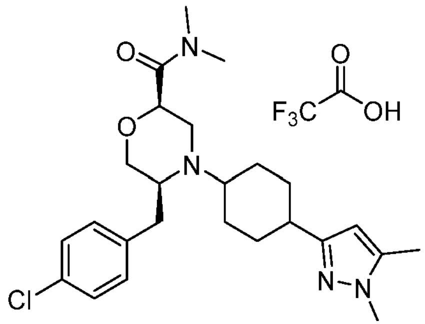 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-*N,N*-dimethylmorpholine-2-carboxamide 2,2,2-trifluoroacetate |
| 21. | 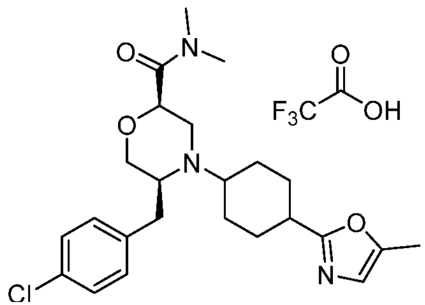 *A single isomer - cis-trans relationship unknown* | A | (2*R*,5*S*)-5-(4-chlorobenzyl)-*N*,*N*-dimethyl-4-(4-(5-methyloxazol-2-yl)cyclohexyl)morpholine-2-carboxamide 2,2,2-trifluoroacetate |
| 22. | 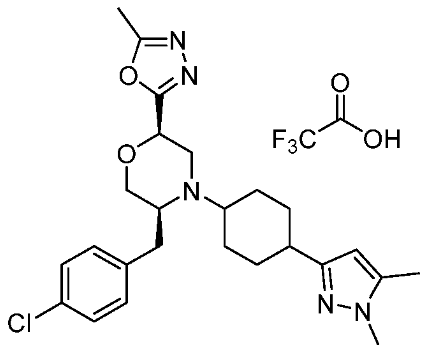 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-2-(5-methyl-1,3,4-oxadiazol-2-yl)morpholine 2,2,2-trifluoroacetate |

(continued)

| Ex.# | Structure | YKL-40, $IC_{50}$ / Kd | IUPAC Name |
|---|---|---|---|
| 23. | 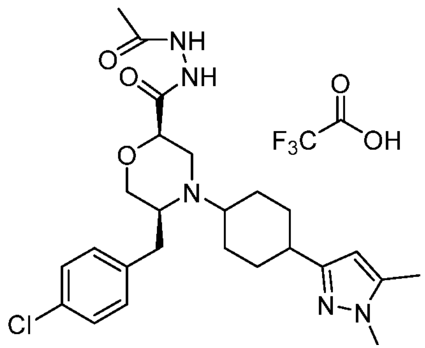 *A single isomer - cis-trans relationship unknown* | A | (2*R*,5*S*)-*N'*-acetyl-5-(4-chlorobenzyl)-4-(4-(1,5-dimethyl-1*H*-pyrazol-3-yl)cyclohexyl)morpholine-2-carbohydrazide 2,2,2-trifluoroacetate |
| 24. | 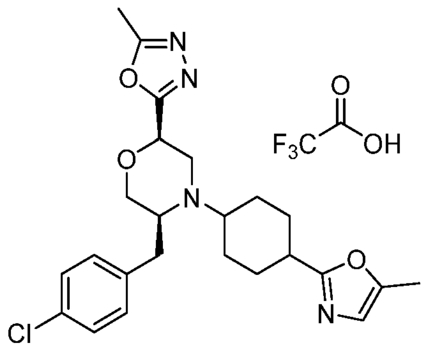 *A single isomer - cis-trans relationship unknown* | A | (2*R*,5*S*)-5-(4-chlorobenzyl)-2-(5-methyl-1,3,4-oxadiazol-2-yl)-4-(4-(5-methyloxazol-2-yl)cyclohexyl)morpholine 2,2,2-trifluoroacetate |

(continued)

| Ex.# | Structure | YKL-40, $IC_{50}$ / Kd | IUPAC Name |
|---|---|---|---|
| 25. | 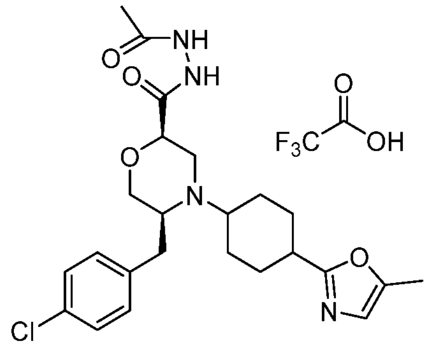 *A single isomer - cis-trans relationship unknown* | A | (2*R*,5*S*)-*N'*-acetyl-5-(4-chlorobenzyl)-4-(4-(5-methyloxazol-2-yl)cyclohexyl)morpholine-2-carbohydrazide 2,2,2-trifluoroacetate |
| 26. | 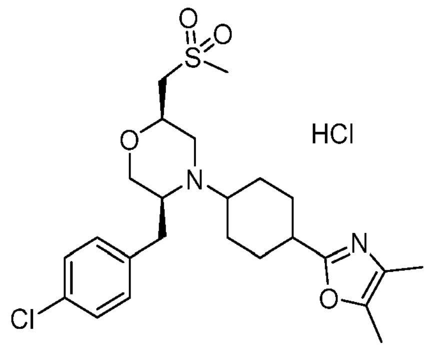 *A single isomer - cis-trans relationship unknown* | A | (2*R*,5*S*)-5-(4-chlorobenzyl)-4-(4-(4,5-dimethyloxazol-2-yl)cyclohexyl)-2-((methylsulfonyl)methyl)-morpholine hydrochloride |

(continued)

| Ex.# | Structure | YKL-40, $IC_{50}$ / Kd | IUPAC Name |
|---|---|---|---|
| 27. | 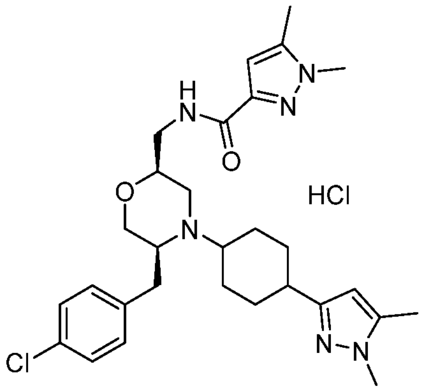<br>*A single isomer - cis-trans relationship unknown* | A | *N*-(((2*S*,5*S*)-5-(4-chlorobenzyl)-4-(4-(1,5-dimethyl-1*H*-pyrazol-3-yl)cyclohexyl)morpholin-2-yl) methyl)-1,5-dimethyl-1*H*-pyrazole-3-carboxamide hydrochloride |
| 28. | 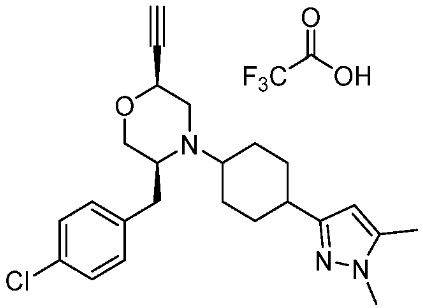<br>*A single isomer - cis-trans relationship unknown* | A | (2*S*,5*S*)-5-(4-chlorobenzyl)-4-(4-(1,5-dimethyl-1*H*-pyrazol-3-yl)cyclohexyl)-2-ethynylmorpholine 2,2,2-trifluoroacetate |
| 29. | 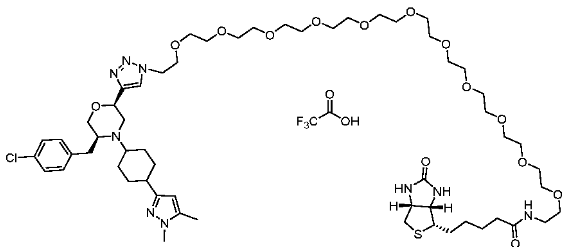<br>*A single isomer - cis-trans relationship unknown* | *A | *N*-(35-(4-((2*R*,5*S*)-5-(4-chlorobenzyl)-4-(4-(1,5-dimethyl-1*H*-pyrazol-3-yl)cyclohexyl)morpholin-2-yl)-1*H*-1,2,3-triazol-1-yl)-3,6,9,12,15,18,21,24,27,30,33 -undecaoxapentatriacontyl)-5-((3*aS*,4*S*,6*aR*)-2-oxo-hexahydro-1*H*-thieno[3,4-d]imidazol-4-yl)pentanamide 2,2,2-trifluoroacetate |

(continued)

| Ex.# | Structure | YKL-40, $IC_{50}$ / Kd | IUPAC Name |
|---|---|---|---|
| 30. | 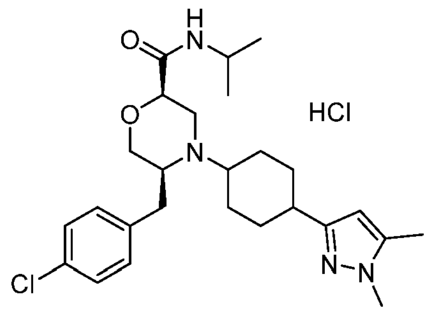 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-*N*-isopropylmorpholine-2-carboxamide hydrochloride |
| 31. | 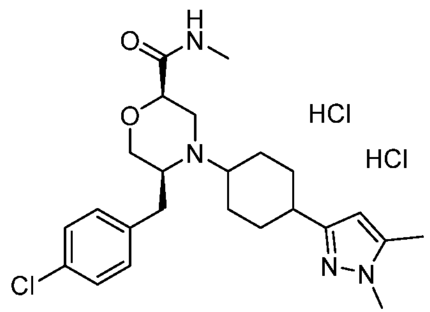 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-*N*-methylmorpholine-2-carboxamide dihydrochloride |
| 32. | 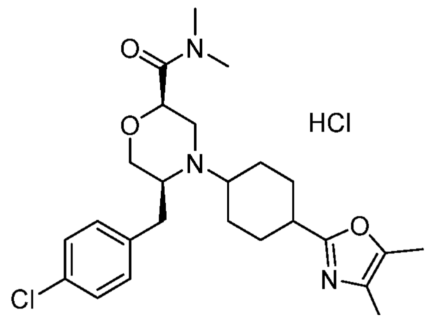 *A single isomer - cis-trans relationship unknown* | A | (2*R*,5*S*)-5-(4-chlorobenzyl)-4-(4-(4,5-dimethyloxazol-2-yl)cyclohexyl)-*N*,*N*-dimethylmorpholine-2-carboxamide hydrochloride |

(continued)

| Ex.# | Structure | YKL-40, $IC_{50}$ / Kd | IUPAC Name |
|---|---|---|---|
| 33. | 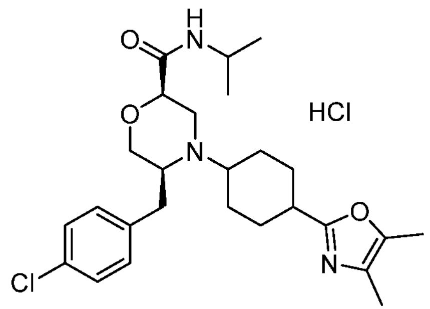 *A single isomer - cis-trans relationship unknown* | A | (2*R*,5*S*)-5-(4-chlorobenzyl)-4-(4-(4,5-dimethyloxazol-2-yl)cyclohexyl)-*N*-isopropylmorpholine-2-carboxamide hydrochloride |
| 34. | 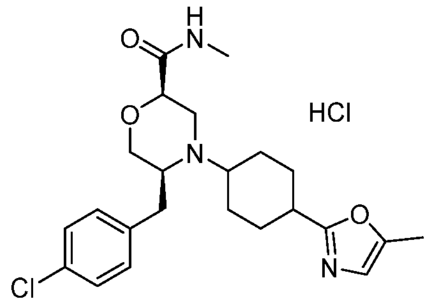 *A single isomer - cis-trans relationship unknown* | A | (2*R*,5*S*)-5-(4-chlorobenzyl)-*N*-methyl-4-(4-(5-methyloxazol-2-yl)cyclohexyl)morpholine-2-carboxamide hydrochloride |
| 35. | 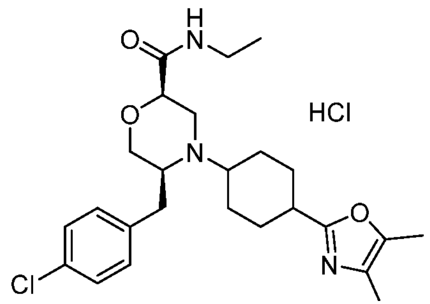 *A single isomer - cis-trans relationship unknown* | A | (2*R*,5*S*)-5-(4-chlorobenzyl)-4-(4-(4,5-dimethyloxazol-2-yl)cyclohexyl)-*N*-ethylmorpholine-2-carboxamide hydrochloride |

(continued)

| Ex.# | Structure | YKL-40, $IC_{50}$ / Kd | IUPAC Name |
|---|---|---|---|
| 36. | 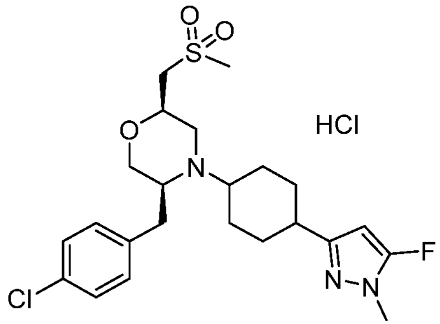 *A single isomer - cis-trans relationship unknown* | A | (2*R*,5*S*)-5-(4-chlorobenzyl)-4-(4-(5-fluoro-1-methyl-1*H*-pyrazol-3-yl)cyclohexyl)-2-((methylsulfonyl)methyl)-morpholine hydrochloride |
| 37. | 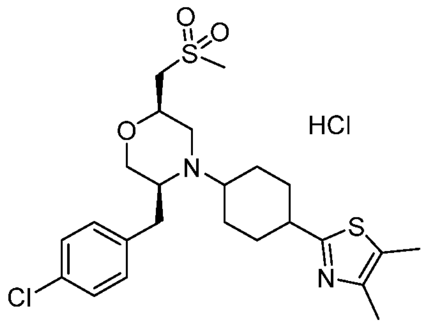 *A single isomer - cis-trans relationship unknown* | A | (2*R*,5*S*)-5-(4-chlorobenzyl)-4-(4-(4,5-dimethylthiazol-2-yl)cyclohexyl)-2-((methylsulfonyl) methyl)-morpholine hydrochloride |
| 38. | 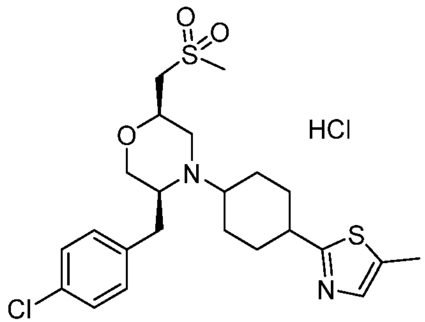 *A single isomer - cis-trans relationship unknown* | A | (2*R*,5*S*)-5-(4-chlorobenzyl)-2-((methylsulfonyl)methyl)-4-(4-(5-methylthiazol-2-yl)-cyclohexyl) morpholine hydrochloride |

(continued)

| Ex.# | Structure | YKL-40, $IC_{50}$ / Kd | IUPAC Name |
|---|---|---|---|
| 39. | 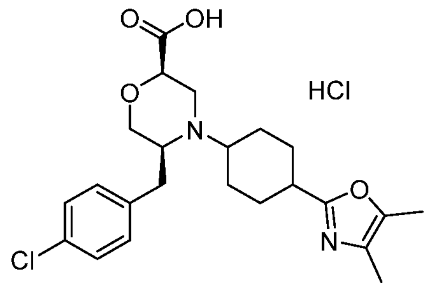 *A single isomer - cis-trans relationship unknown* | A | (2*R*,5*S*)-5-(4-chlorobenzyl)-4-(4-(4,5-dimethyloxazol-2-yl)-cyclohexyl)morpholine-2-carboxylic acid hydrochloride |
| 40. | 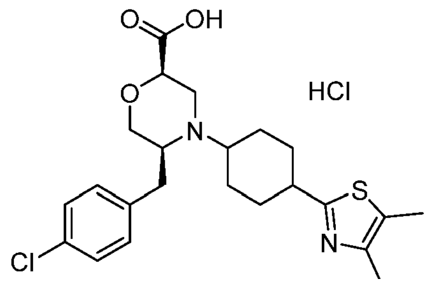 *A single isomer - cis-trans relationship unknown* | A | (2*R*,5*S*)-5-(4-chlorobenzyl)-4-(4-(4,5-dimethylthiazol-2-yl)cyclohexyl)morpholine-2-carboxylic acid hydrochloride |
| 41. | 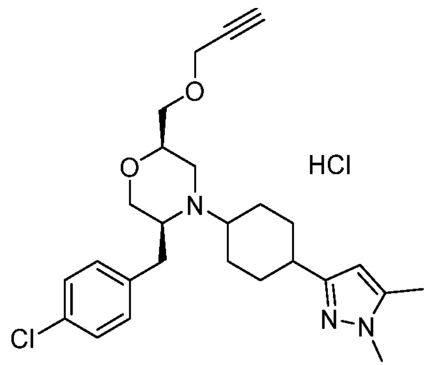 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-2-((prop-2-yn-1-yloxy)methyl)morpholine hydrochloride |

(continued)

| Ex.# | Structure | YKL-40, $IC_{50}$ / Kd | IUPAC Name |
|---|---|---|---|
| 42. | 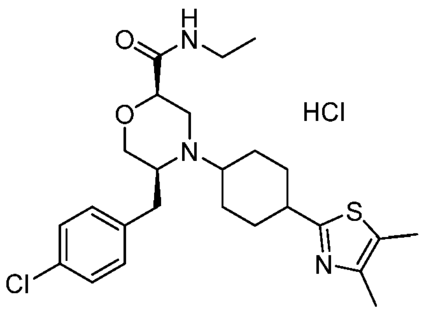 *A single isomer - cis-trans relationship unknown* | A | (2*R*,5*S*)-5-(4-chlorobenzyl)-4-(4-(4,5-dimethylthiazol-2-yl)cyclohexyl)-*N*-ethylmorpholine-2-carboxamide hydrochloride |
| 43. | 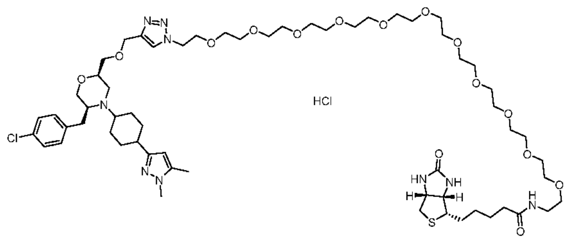 *A single isomer - cis-trans relationship unknown* | *A | *N*-(35-(4-((((2*R*,5*S*)-5-(4-chlorobenzyl)-4-(4-(1,5-dimethyl-1*H*-pyrazol-3-yl)-cyclohexyl)morpholin-2-yl)methoxy)methyl)-1*H*-1,2,3-triazol-1-yl)-3,6,9,12,15,18,21,24,27,30,33 -undecaoxapentatriacontyl)-5-((3*aS*,4*S*,6*aR*)-2-oxo-hexahydro-1*H*-thieno[3,4-d]imidazol-4-yl)pentanamide hydrochloride |
| 44. | 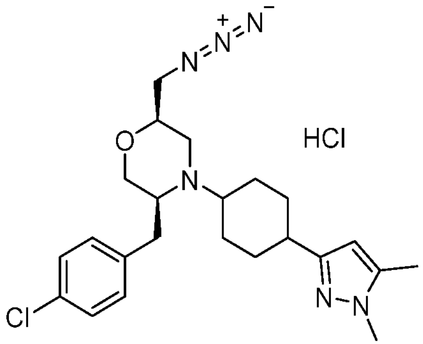 *A single isomer - cis-trans relationship unknown* | A | (2*R*,5*S*)-2-(azidomethyl)-5-(4-chlorobenzyl)-4-(4-(1,5-dimethyl-1*H*-pyrazol-3-yl)cyclohexyl)morpholine hydrochloride |

(continued)

| Ex.# | Structure | YKL-40, $IC_{50}$ / Kd | IUPAC Name |
|---|---|---|---|
| 45. | 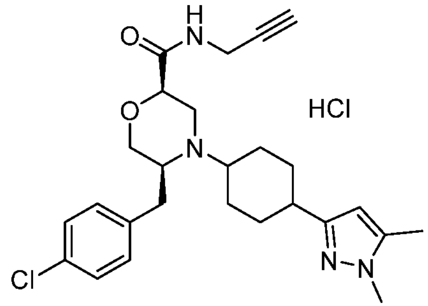 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-*N*-(prop-2-yn-1-yl) morpholine-2-carboxamide hydrochloride |
| 46. | 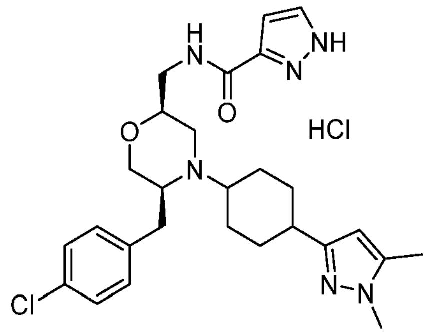 *A single isomer - cis-trans relationship unknown* | A | *N*-(((2*S*,5*S*)-5-(4-chlorobenzyl)-4-(4-(1,5-dimethyl-1*H*-pyrazol-3-yl)cyclohexyl)morpholin-2-yl) methyl)-1*H*-pyrazole-3-carboxamide hydrochloride |
| 47. | 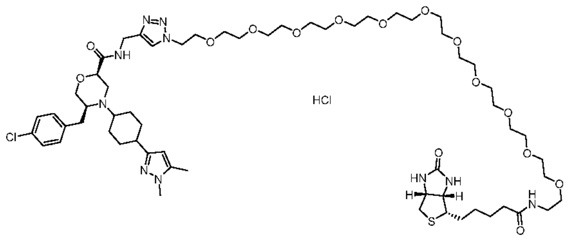 *A single isomer - cis-trans relationship unknown* | *A | (2*R*,5*S*)-5-(4-chlorobenzyl)-4-(4-(1,5-dimethyl-1*H*-pyrazol-3-yl)cyclohexyl)-*N*-((1-(37-oxo-41-((3*aS*,4*S*,6*aR*)-2-oxohexahydro-1*H*-thieno[3,4-*d*]imidazol-4-yl)-3,6,9,12,15,18,21,24,27,30,33 -undecaoxa-36-aza-hentetracontyl)-1*H*-1,2,3-triazol-4-yl) methyl)-morpholine-2-carboxamide hydrochloride |

(continued)

| Ex.# | Structure | YKL-40, $IC_{50}$ / Kd | IUPAC Name |
|---|---|---|---|
| 48. | 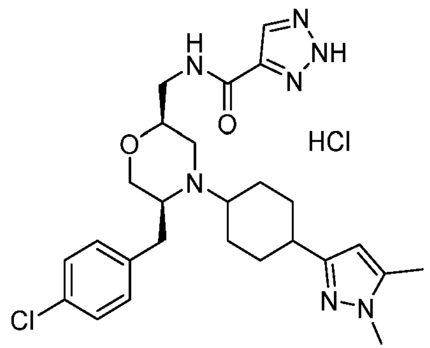 *A single isomer - cis-trans relationship unknown* | A | *N*-(((2*S*,5*S*)-5-(4-chlorobenzyl)-4-(4-(1,5-dimethyl-1*H*-pyrazol-3-yl)cyclohexyl)morpholin-2-yl) methyl)-2*H*-1,2,3-triazole-4-carboxamide hydrochloride |
| 49. | 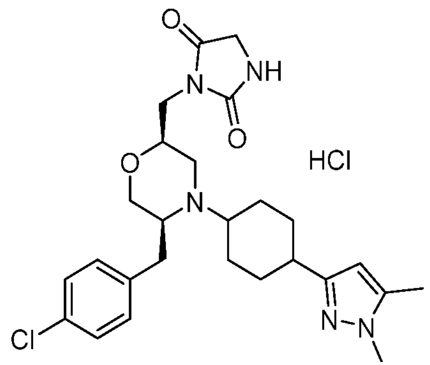 *A single isomer - cis-trans relationship unknown* | A | 3-(((2*R*,5*S*)-5-(4-chlorobenzyl)-4-(4-(1,5-dimethyl-1*H*-pyrazol-3-yl)cyclohexyl)morpholin-2-yl) methyl)imidazolidine-2,4-dione hydrochloride |

(continued)

| Ex.# | Structure | YKL-40, $IC_{50}$ / Kd | IUPAC Name |
|---|---|---|---|
| 50. | 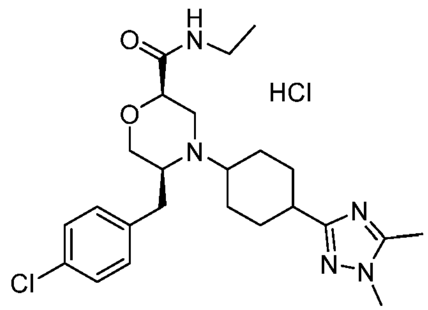 *A single isomer - cis-trans relationship unknown* | B | (2*R*,5*S*)-5-(4-chlorobenzyl)-4-(4-(1,5-dimethyl-1*H*-1,2,4-triazol-3-yl)cyclohexyl)-*N*-ethylmorpholine-2-carboxamide hydrochloride |
| 51. | 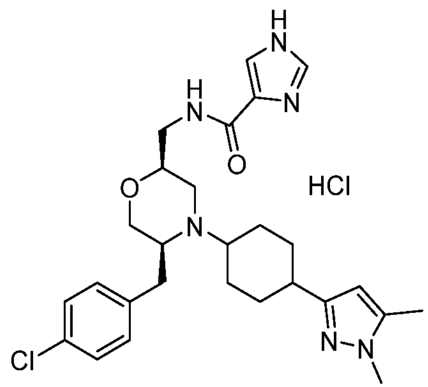 *A single isomer - cis-trans relationship unknown* | A | *N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-imidazole-4-carboxamide hydrochloride |

(continued)

| Ex.# | Structure | YKL-40, $IC_{50}$ / Kd | IUPAC Name |
|---|---|---|---|
| 52. | 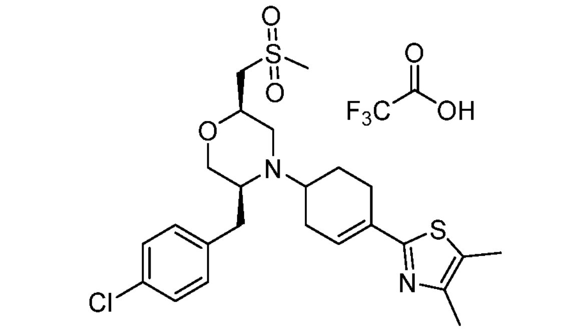 *Probably a mixture of two isomers due to the delicate issue of chirality at cyclohexene ring* | A | (2*R*,5*S*)-5-(4-chlorobenzyl)-4-(4-(4,5-dimethylthiazol-2-yl)cyclohex-3-en-1-yl)-2-((methylsulfonyl)methyl)-morpholine 2,2,2-trifluoroacetate |
| 53. | 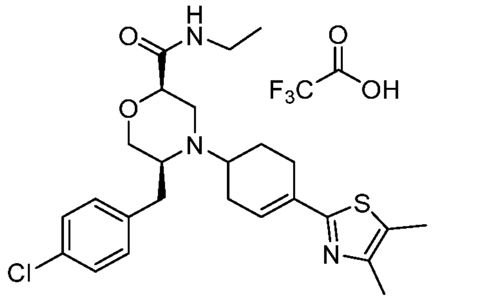 *Probably a mixture of two isomers due to the delicate issue of chirality at cyclohexene ring* | A | (2*R*,5*S*)-5-(4-chlorobenzyl)-4-(4-(4,5-dimethylthiazol-2-yl)cyclohex-3-en-1-yl)-*N*-ethylmorpholine-2-carboxamide 2,2,2-trifluoroacetate |
| 54. | 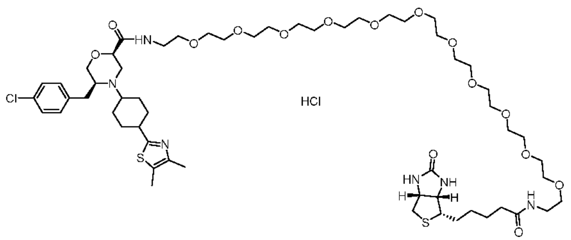 *A single isomer - cis-trans relationship unknown* | *A | (2*R*,5*S*)-5-(4-chlorobenzyl)-4-(4-(4,5-dimethylthiazol-2-yl)cyclohexyl)-*N*-(37-oxo-41-((3*aS*,4*S*,6*aR*)-2-oxo-hexahydro-1*H*-thieno[3,4-d]imidazol-4-yl)-3,6,9,12,15,18,21,24,27,30,33 -undecaoxa-36-aza-hentetracontyl)morpholine-2-carboxamide hydrochloride |

(continued)

| Ex.# | Structure | YKL-40, $IC_{50}$ / Kd | IUPAC Name |
|---|---|---|---|
| 55. | 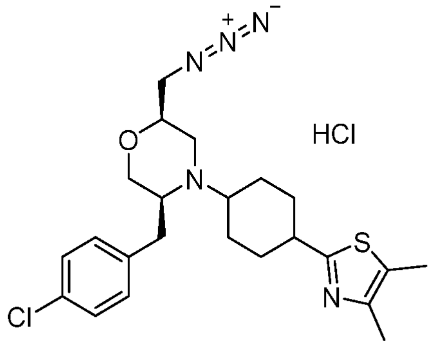 *A single isomer - cis-trans relationship unknown* | A | (2*R*,5*S*)-2-(azidomethyl)-5-(4-chlorobenzyl)-4-(4-(4,5-dimethylthiazol-2-yl)cyclohexyl)morpholine hydrochloride |
| 56. | 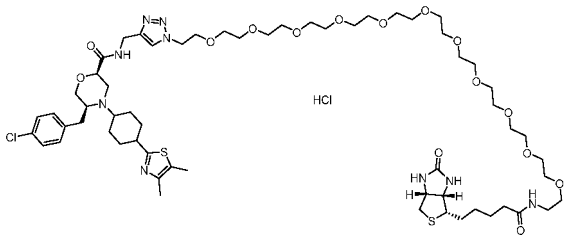 *A single isomer - cis-trans relationship unknown* | *A | (2*R*,5*S*)-5-(4-chlorobenzyl)-4-(4-(4,5-dimethylthiazol-2-yl)cyclohexyl)-*N*-((1-(37-oxo-41-((3*aS*,4*S*,6*aR*)-2-oxo-hexahydro-1*H*-thieno[3,4-*d*]imidazol-4-yl)-3,6,9,12,15,18,21,24,27,30,33 -undecaoxa-36-aza-hentetracontyl)-1*H*-1,2,3-triazol-4-yl) methyl)-morpholine-2-carboxamide hydrochloride |
| 57. | 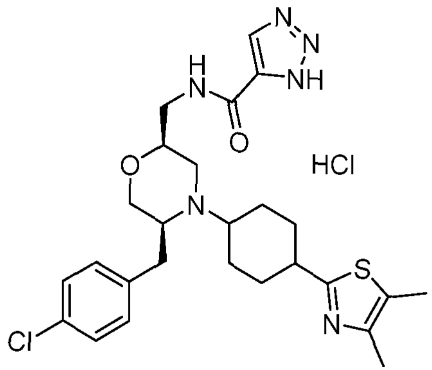 *A single isomer - cis-trans relationship unknown* | A | *N*-(((2*S*,5*S*)-5-(4-chlorobenzyl)-4-(4-(4,5-dimethylthiazol-2-yl)cyclohexyl)morpholin-2-yl) methyl)-1*H*-1,2,3-triazole-5-carboxamide hydrochloride |

(continued)

| Ex.# | Structure | YKL-40, $IC_{50}$ / Kd | IUPAC Name |
|---|---|---|---|
| 58. | 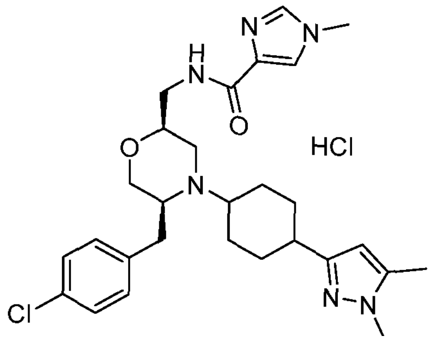 *A single isomer - cis-trans relationship unknown* | A | *N*-(((2*S*,5*S*)-5-(4-chlorobenzyl)-4-(4-(1,5-dimethyl-1*H*-pyrazol-3-yl)cyclohexyl)morpholin-2-yl) methyl)-1-methyl-1*H*-imidazole-4-carboxamide hydrochloride |
| 59. | 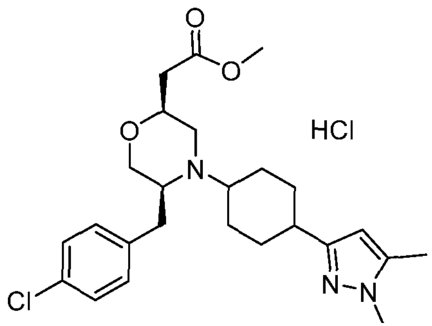 *A single isomer - cis-trans relationship unknown* | A | methyl 2-((2*S*,5*S*)-5-(4-chlorobenzyl)-4-(4-(1,5-dimethyl-1*H*-pyrazol-3-yl)cyclohexyl)morpholin-2-yl)acetate hydrochloride |

(continued)

| Ex.# | Structure | YKL-40, $IC_{50}$ / Kd | IUPAC Name |
|---|---|---|---|
| 60. | 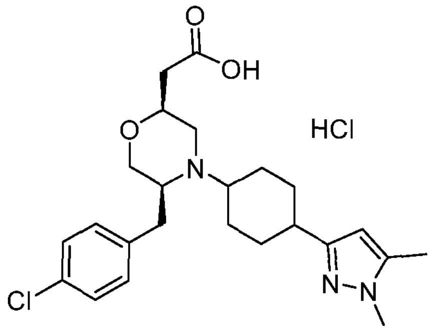 *A single isomer - cis-trans relationship unknown* | A | 2-((2*S*,5*S*)-5-(4-chlorobenzyl)-4-(4-(1,5-dimethyl-1*H*-pyrazol-3-yl)cyclohexyl)morpholin-2-yl) acetic acid hydrochloride |
| 61. | 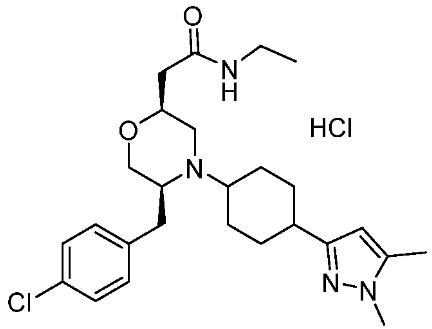 *A single isomer - cis-trans relationship unknown* | A | 2-((2*S*,5*S*)-5-(4-chlorobenzyl)-4-(4-(1,5-dimethyl-1*H*-pyrazol-3-yl)cyclohexyl)morpholin-2-yl)-*N*-ethylacetamide hydrochloride |
| 62. | 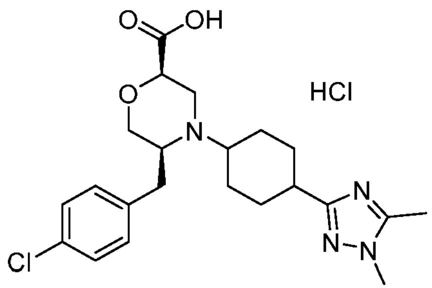 *A single isomer - cis-trans relationship unknown* | B | (2*R*,5*S*)-5-(4-chlorobenzyl)-4-(4-(1,5-dimethyl-1*H*-1,2,4-triazol-3-yl)cyclohexyl)morpholine-2-carboxylic acid hydrochloride |

(continued)

| Ex.# | Structure | YKL-40, $IC_{50}$ / Kd | IUPAC Name |
|---|---|---|---|
| 63. | 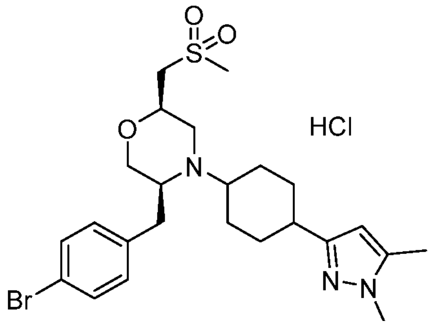 *A single isomer - cis-trans relationship unknown* | A | (2*R*,5*S*)-5-(4-bromobenzyl)-4-(4-(1,5-dimethyl-1*H*-pyrazol-3-yl)cyclohexyl)-2-((methylsulfonyl) methyl)-morpholine hydrochloride |
| 64. | 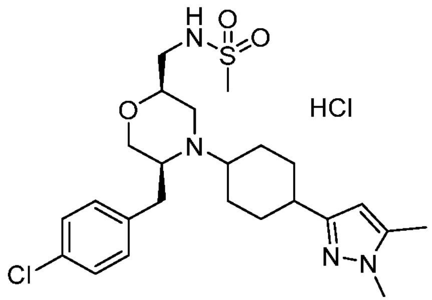 *A single isomer - cis-trans relationship unknown* | A | *N*-(((2*R*,5*S*)-5-(4-chlorobenzyl)-4-(4-(1,5-dimethyl-1*H*-pyrazol-3-yl)cyclohexyl)morpholin-2-yl) methyl)methanesulfonamid e hydrochloride |
| 65. | 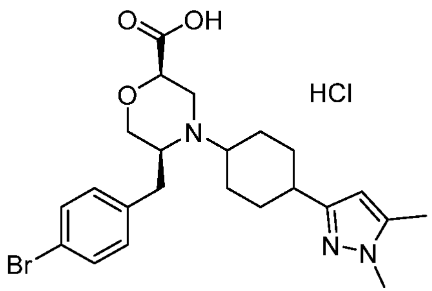 *A single isomer - cis-trans relationship unknown* | A | (2*R*,5*S*)-5-(4-bromobenzyl)-4-(4-(1,5-dimethyl-1*H*-pyrazol-3-yl)cyclohexyl)morpholine-2-car-boxylic acid hydrochloride |

(continued)

| Ex.# | Structure | YKL-40, $IC_{50}$ / Kd | IUPAC Name |
|---|---|---|---|
| 66. | 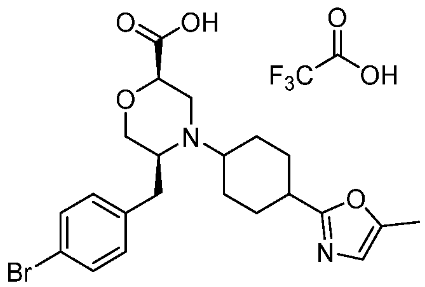 *A single isomer - cis-trans relationship unknown* | A | (2*R*,5*S*)-5-(4-bromobenzyl)-4-(4-(5-methyloxazol-2-yl)cyclohexyl)morpholine-2-carboxylic acid 2,2,2-trifluoroacetate |
| 67. | 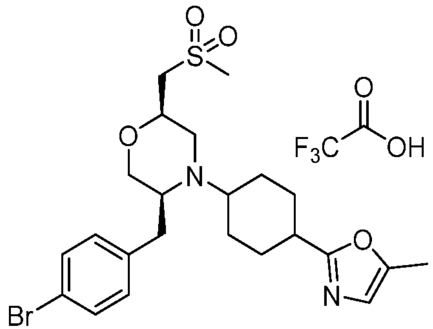 *A single isomer - cis-trans relationship unknown* | A | (2*R*,5*S*)-5-(4-bromobenzyl)-4-(4-(5-methyloxazol-2-yl)cyclohexyl)-2-((methylsulfonyl) methyl)-morpholine 2,2,2-trifluoroacetate |
| 68. | 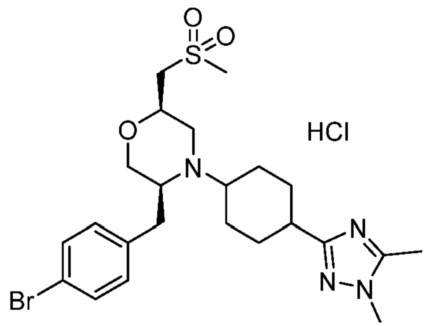 *A single isomer - cis-trans relationship unknown* | A | (2*R*,5*S*)-5-(4-bromobenzyl)-4-(4-(1,5-dimethyl-1*H*-1,2,4-triazol-3-yl)cyclohexyl)-2-((methylsulfo-nyl)methyl)-morpholine hydrochloride |

(continued)

| Ex.# | Structure | YKL-40, $IC_{50}$ / Kd | IUPAC Name |
|---|---|---|---|
| 69. | 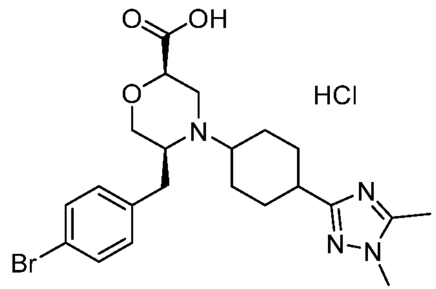 HCl *A single isomer - cis-trans relationship unknown* | A | (2*R*,5*S*)-5-(4-bromobenzyl)-4-(4-(1,5-dimethyl-1*H*-1,2,4-triazol-3-yl)cyclohexyl)-morpholine-2-carboxylic acid hydrochloride |
| 70. | 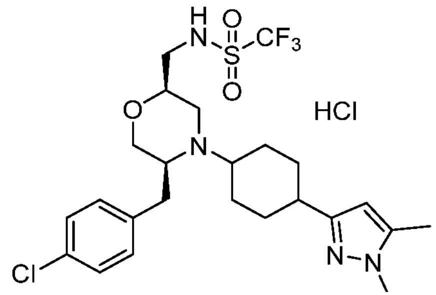 HCl *A single isomer - cis-trans relationship unknown* | A | *N*-(((2*R*,5*S*)-5-(4-chlorobenzyl)-4-(4-(1,5-dimethyl-1*H*-pyrazol-3-yl)cyclohexyl)morpholin-2-yl) methyl)-1,1,1-trifluoromethanesulfonamide hydrochloride |
| 71. | 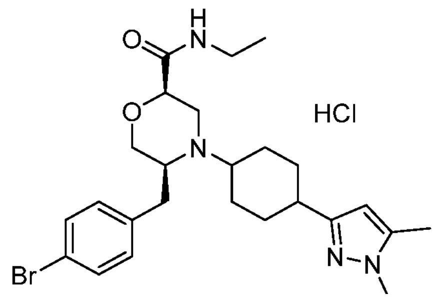 HCl *A single isomer - cis-trans relationship unknown* | A | (2*R*,5*S*)-5-(4-bromobenzyl)-4-(4-(1,5-dimethyl-1*H*-pyrazol-3-yl)cyclohexyl)-*N*-ethylmorpholine-2-carboxamide hydrochloride |

(continued)

| Ex.# | Structure | YKL-40, $IC_{50}$ / Kd | IUPAC Name |
|---|---|---|---|
| 72. | 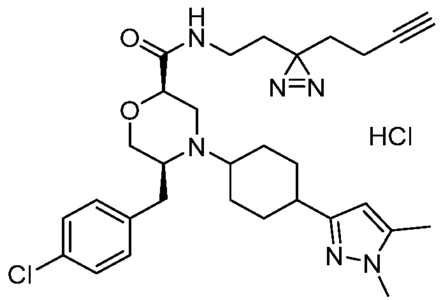 *A single isomer - cis-trans relationship unknown* | B | (2*R*,5*S*)-*N*-(2-(3-(but-3-yn-1-yl)-3*H*-diazirin-3-yl)ethyl)-5-(4-chlorobenzyl)-4-(4-(1,5-dimethyl-1*H*-pyrazol-3-yl)cyclohexyl)morpholine-2-carboxamide hydrochloride |
| 73. | 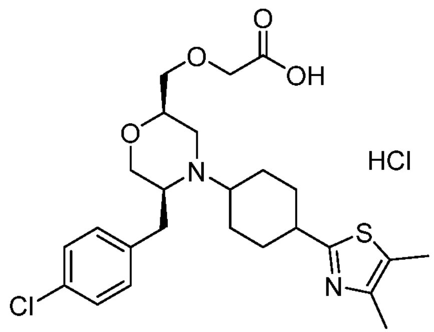 *A single isomer - cis-trans relationship unknown* | A | 2-(((2*R*,5*S*)-5-(4-chlorobenzyl)-4-(4-(4,5-dimethylthiazol-2-yl)cyclohexyl)morpholin-2-yl)methoxy)acetic acid hydrochloride |
| 74. | 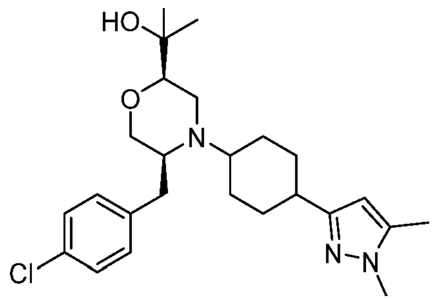 *A single isomer - cis-trans relationship unknown* | A | 2-((2*R*,5*S*)-5-(4-chlorobenzyl)-4-(4-(1,5-dimethyl-1*H*-pyrazol-3-yl)cyclohexyl)morpholin-2-yl)propan-2-ol |

(continued)

| Ex.# | Structure | YKL-40, $IC_{50}$ / Kd | IUPAC Name |
|---|---|---|---|
| 75. | 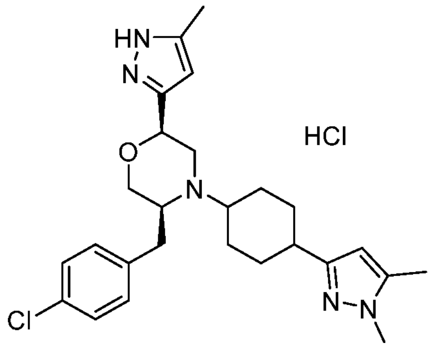 HCl *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-2-(5-methyl-1*H*-pyrazol-3-yl)morpholine hydrochloride |
| 76. | 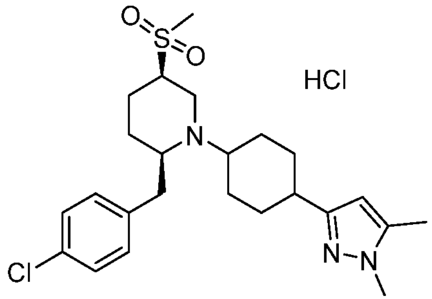 HCl *A single isomer - cis-trans relationship unknown* | A | (2*R*,5*R*)-2-(4-chlorobenzyl)-1-(4-(1,5-dimethyl-1*H*-pyrazol-3-yl)cyclohexyl)-5-(methylsulfonyl)piperidine hydrochloride |
| 77. | 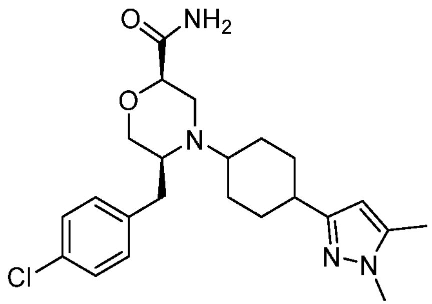 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)morpholine-2-carboxamide |

(continued)

| Ex.# | Structure | YKL-40, $IC_{50}$ / Kd | IUPAC Name |
|---|---|---|---|
| 78. | 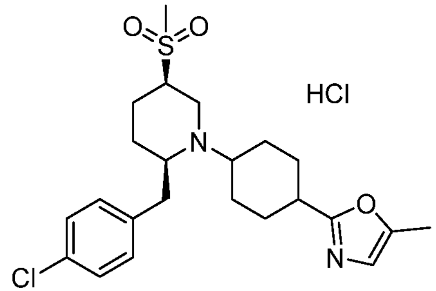 *A single isomer - cis-trans relationship unknown* | A | 2-(4-((2*R*,5*R*)-2-(4-chlorobenzyl)-5-(methylsulfonyl)piperidin-1-yl)-cyclohexyl)-5-methyloxazole hydrochloride |
| 79. | 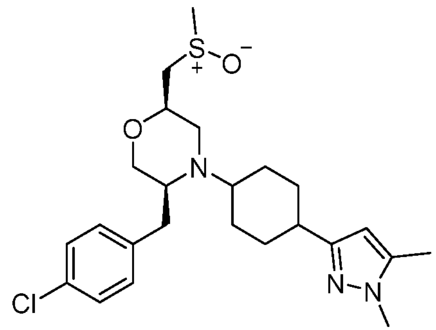 *A single isomer - cis-trans relationship unknown (unknown sulfur chirality)* | A | (2*R*,5*S*)-5-(4-chlorobenzyl)-4-(4-(1,5-dimethyl-1*H*-pyrazol-3-yl)cyclohexyl)-2-((methylsulfinyl) methyl)-morpholine |

(continued)

| Ex.# | Structure | YKL-40, $IC_{50}$ / Kd | IUPAC Name |
|---|---|---|---|
| 80. | 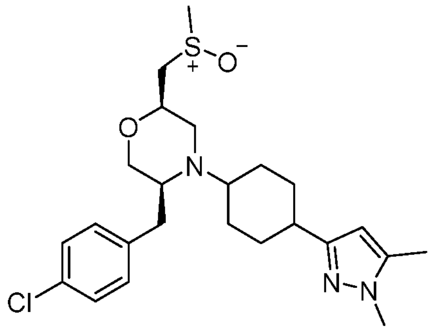 *A single isomer - cis-trans relationship unknown (unknown sulfur chirality)* | A | (2*R*,5*S*)-5-(4-chlorobenzyl)-4-(4-(1,5-dimethyl-1*H*-pyrazol-3-yl)cyclohexyl)-2-((methylsulfinyl) methyl)-morpholine |
| 81. | 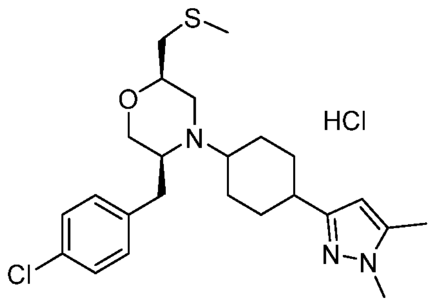 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-2-((methylthio) methyl)-morpholine hydrochloride |

(continued)

| Ex.# | Structure | YKL-40, $IC_{50}$ / Kd | IUPAC Name |
|---|---|---|---|
| 82. | 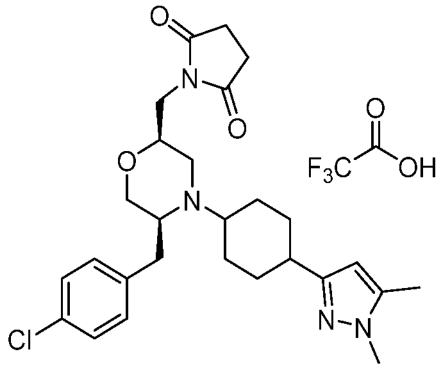 *A single isomer - cis-trans relationship unknown* | A | 1-(((2*R*,5*S*)-5-(4-chlorobenzyl)-4-(4-(1,5-dimethyl-1*H*-pyrazol-3-yl)cyclohexyl)morpholin-2-yl) methyl)pyrrolidine-2,5-dione 2,2,2-trifluoroacetate |
| 83. | 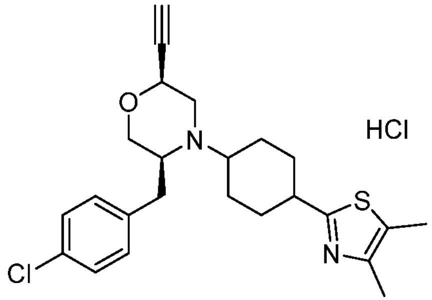 *A single isomer - cis-trans relationship unknown* | A | (2*S*,5*S*)-5-(4-chlorobenzyl)-4-(4-(4,5-dimethylthiazol-2-yl)cyclohexyl)-2-ethynylmorpholine hydrochloride |

(continued)

| Ex.# | Structure | YKL-40, $IC_{50}$ / Kd | IUPAC Name |
|---|---|---|---|
| 84. | 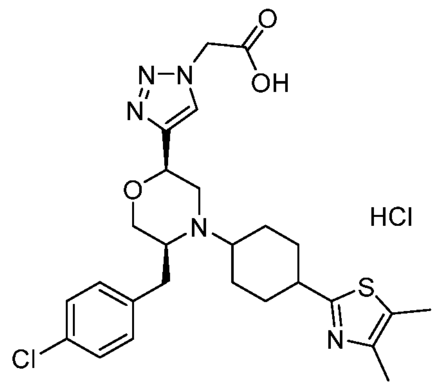 *A single isomer - cis-trans relationship unknown* | A | 2-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)acetic acid hydrochloride |
| 85. | 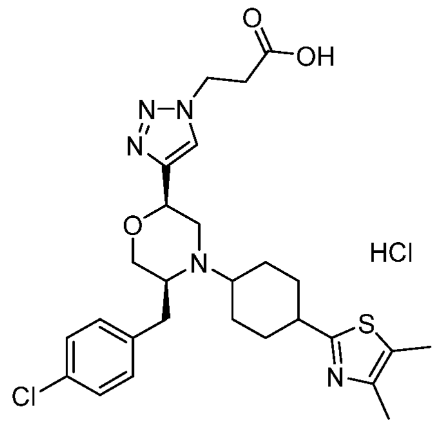 *A single isomer - cis-trans relationship unknown* | A | 3-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)propanoic acid hydrochloride |

(continued)

| Ex.# | Structure | YKL-40, $IC_{50}$ / Kd | IUPAC Name |
|---|---|---|---|
| 86. | 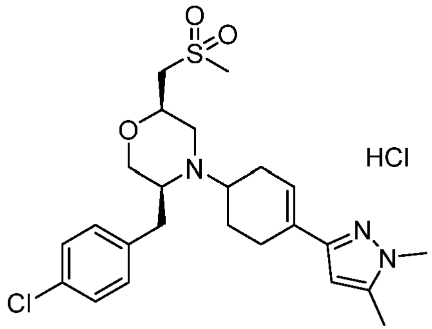 HCl | A | (2*R*,5*S*)-5-(4-chlorobenzyl)-4-(4-(1,5-dimethyl-1*H*-pyrazol-3-yl)cyclohex-3-en-1-yl)-2-((methylsulfonyl)methyl)-morpholine hydrochloride |
| 87. | 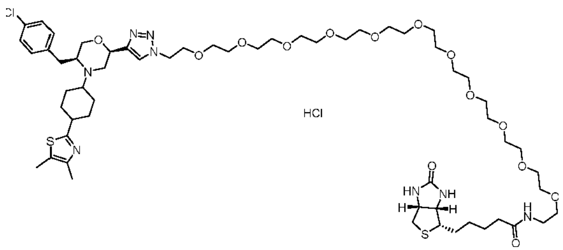 HCl<br>*A single isomer - cis-trans relationship unknown* | *A | *N*-(35-(4-((2*R*,5*S*)-5-(4-chlorobenzyl)-4-(4-(4,5-dimethylthiazol-2-yl)-cyclohexyl)morpholin-2-yl)-1*H*-1,2,3-triazol-1-yl)-3,6,9,12,15,18,21,24,27,30,33 -undecaoxapentatriacontyl)-5-((3*aS*,4*S*,6*aR*)-2-oxo-hexahydro-1*H*-thieno[3,4-*d*]imidazol-4-yl)pentanamide hydrochloride |
| 88. | 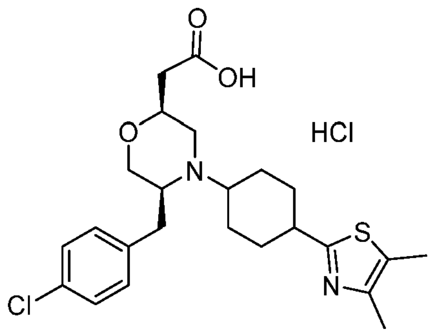 HCl<br>*A single isomer - cis-trans relationship unknown* | A | 2-((2*S*,5*S*)-5-(4-chlorobenzyl)-4-(4-(4,5-dimethylthiazol-2-yl)cyclohexyl)morpholin-2-yl)acetic acid hydrochloride |

(continued)

| Ex.# | Structure | YKL-40, $IC_{50}$ / Kd | IUPAC Name |
|---|---|---|---|
| 89. | 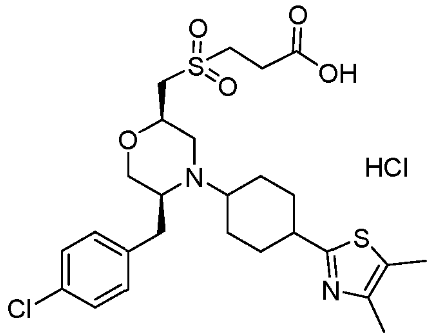 A single isomer - *cis-trans* relationship unknown | A | 3-((((2*R*,5*S*)-5-(4-chlorobenzyl)-4-(4-(4,5-dimethylthiazol-2-yl)cyclohexyl)morpholin-2-yl)methyl) sulfonyl)propanoic acid hydrochloride |
| 90. | 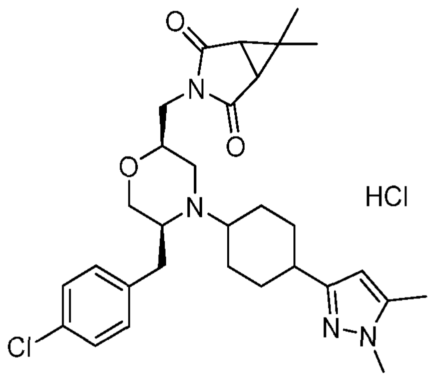 A single isomer - *cis-trans* relationship unknown | A | 3-(((2*R*,5*S*)-5-(4-chlorobenzyl)-4-(4-(1,5-dimethyl-1*H*-pyrazol-3-yl)cyclohexyl)morpholin-2-yl) methyl)-6,6-dimethyl-3-azabicyclo[3.1.0]hexane-2,4-dione hydrochloride |

(continued)

| Ex.# | Structure | YKL-40, $IC_{50}$ / Kd | IUPAC Name |
|---|---|---|---|
| 91. | 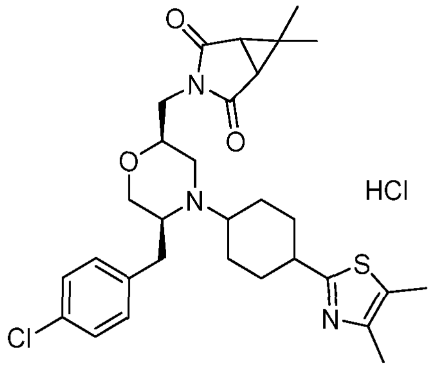 HCl *A single isomer - cis-trans relationship unknown* | A | 3-(((2*R*,5*S*)-5-(4-chlorobenzyl)-4-(4-(4,5-dimethylthiazol-2-yl)cyclohexyl)morpholin-2-yl) methyl)-6,6-dimethyl-3-azabicyclo[3.1.0]hexane-2,4-dione hydrochloride |
| 92. | 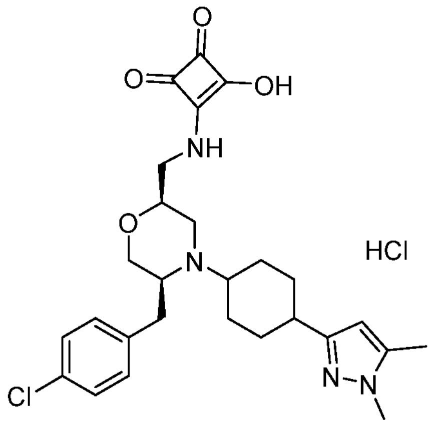 HCl *A single isomer - cis-trans relationship unknown* | A | 3-((((2*S*,5*S*)-5-(4-chlorobenzyl)-4-(4-(1,5-dimethyl-1*H*-pyrazol-3-yl)cyclohexyl)morpholin-2-yl) methyl)amino)-4-hydroxycyclobut-3-ene-1,2-dione hydrochloride |

(continued)

| Ex.# | Structure | YKL-40, $IC_{50}$ / Kd | IUPAC Name |
|---|---|---|---|
| 93. | 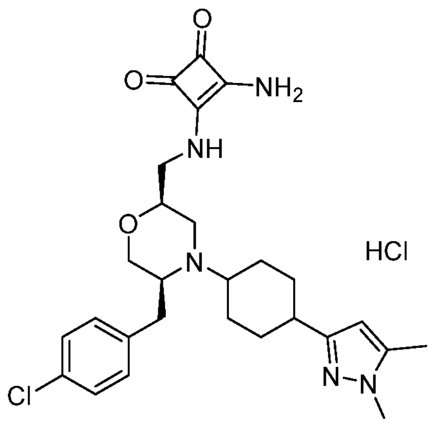 HCl *A single isomer - cis-trans relationship unknown* | A | 3-amino-4-((((2*S*,5*S*)-5-(4-chlorobenzyl)-4-(4-(1,5-dimethyl-1*H*-pyrazol-3-yl)cyclohexyl)morpholin-2-yl)methyl)amino)cyclobut-3-ene-1,2-dione hydrochloride |
| 94. | 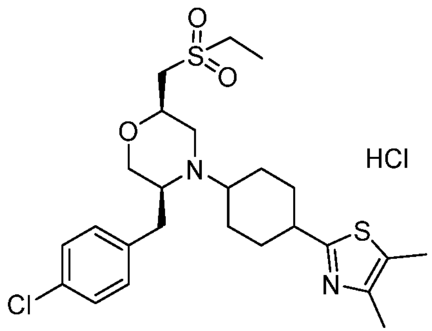 HCl *A single isomer - cis-trans relationship unknown* | A | (2*R*,5*S*)-5-(4-chlorobenzyl)-4-(4-(4,5-dimethylthiazol-2-yl)cyclohexyl)-2-((ethylsulfonyl)methyl)-morpholine hydrochloride |

(continued)

| Ex.# | Structure | YKL-40, $IC_{50}$ / Kd | IUPAC Name |
|---|---|---|---|
| 95. | 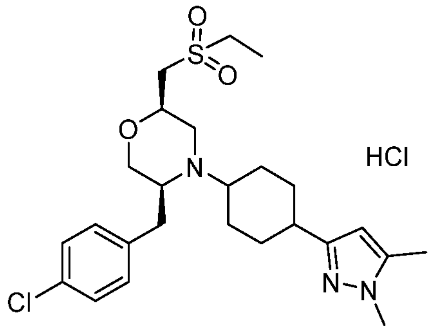 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-2-((ethylsulfonyl) methyl)-morpholine hydrochloride |
| 96. | 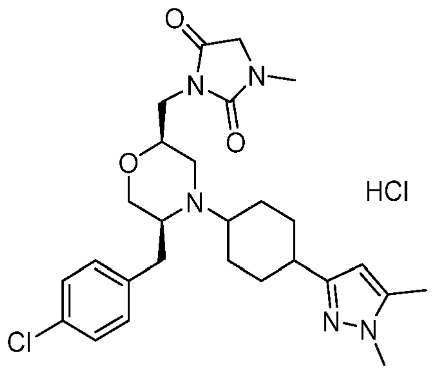 *A single isomer - cis-trans relationship unknown* | A | 3-(((2*R*,5*S*)-5-(4-chlorobenzyl)-4-(4-(1,5-dimethyl-1*H*-pyrazol-3-yl)cyclohexyl)morpholin-2-yl) methyl)-1-methylimidazolidine-2,4-dione hydrochloride |

(continued)

| Ex.# | Structure | YKL-40, $IC_{50}$ / Kd | IUPAC Name |
|---|---|---|---|
| 97. | 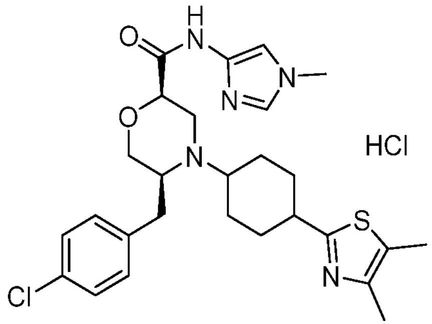 HCl<br>*A single isomer - cis-trans relationship unknown* | A | (2*R*,5*S*)-5-(4-chlorobenzyl)-4-(4-(4,5-dimethylthiazol-2-yl)cyclohexyl)-*N*-(1-methyl-1*H*-imidazol-4-yl)morpholine-2-carboxamide hydrochloride |
| 98. | 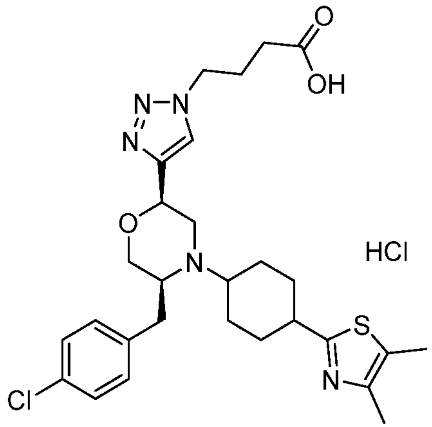 HCl<br>*A single isomer - cis-trans relationship unknown* | A | 4-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)butanoic acid hydrochloride |

(continued)

| Ex.# | Structure | YKL-40, $IC_{50}$ / Kd | IUPAC Name |
|---|---|---|---|
| 99. | 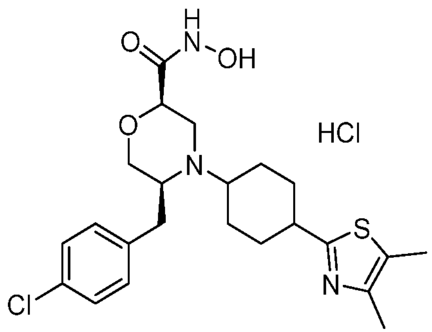 *A single isomer - cis-trans relationship unknown* | A | (2*R*,5*S*)-5-(4-chlorobenzyl)-4-(4-(4,5-dimethylthiazol-2-yl)cyclohexyl)-*N*-hydroxymorpholine-2-carboxamide hydrochloride |
| 100. | 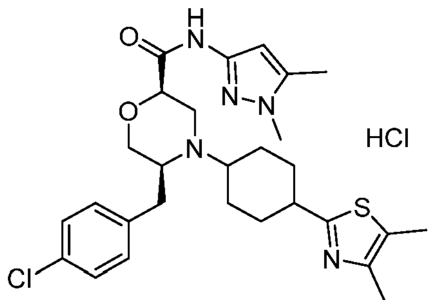 *A single isomer - cis-trans relationship unknown* | A | (2*R*,5*S*)-5-(4-chlorobenzyl)-*N*-(1,5-dimethyl-1*H*-pyrazol-3-yl)-4-(4-(4,5-dimethylthiazol-2-yl)-cyclohexyl)morpholine-2-carboxamide hydrochloride |

(continued)

| Ex.# | Structure | YKL-40, $IC_{50}$ / Kd | IUPAC Name |
|---|---|---|---|
| 101. | 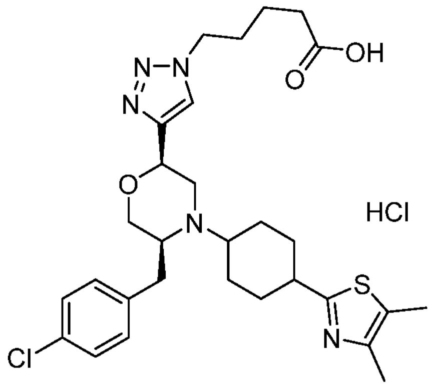 HCl *A single isomer - cis-trans relationship unknown* | A | 5-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)pentanoic acid hydrochloride |
| 102. | 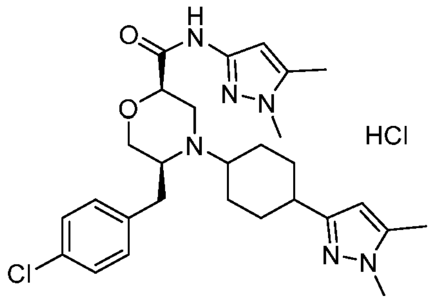 HCl *A single isomer - cis-trans relationship unknown* | A | (2*R*,5*S*)-5-(4-chlorobenzyl)-*N*-(1,5-dimethyl-1*H*-pyrazol-3-yl)-4-(4-(1,5-dimethyl-1*H*-pyrazol-3-yl) cyclohexyl)-morpholine-2-carboxamide hydrochloride |

(continued)

| Ex.# | Structure | YKL-40, $IC_{50}$ / Kd | IUPAC Name |
|---|---|---|---|
| 103. | 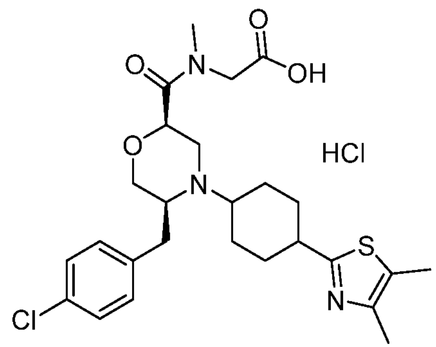 *A single isomer - cis-trans relationship unknown* | A | N-((2*R*,5*S*)-5-(4-chlorobenzyl)-4-(4-(4,5-dimethylthiazol-2-yl)cyclohexyl)morpholine-2-carbonyl)-*N*-methylglycine hydrochloride |
| 104. | 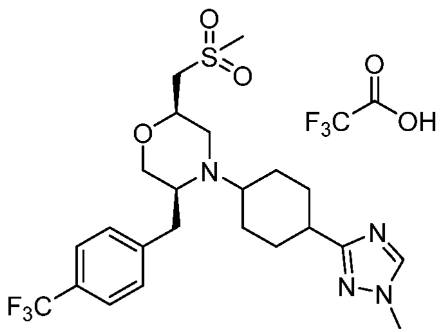 *A single isomer - cis-trans relationship unknown* | B | (2*R*,5*S*)-4-(4-(1-methyl-1*H*-1,2,4-triazol-3-yl)cyclohexyl)-2-((methylsulfonyl)methyl)-5-(4-(trifluoromethyl)benzyl)-morpholine 2,2,2-trifluoroacetate |

(continued)

| Ex.# | Structure | YKL-40, $IC_{50}$ / Kd | IUPAC Name |
|---|---|---|---|
| 105. | 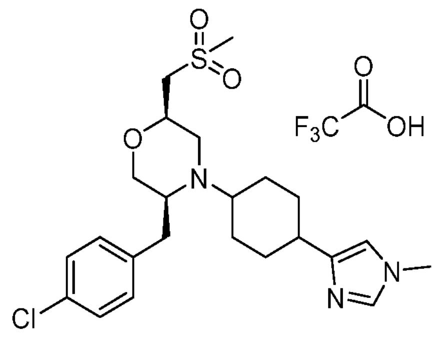 *A single isomer - cis-trans relationship unknown* | A | (2*R*,5*S*)-5-(4-chlorobenzyl)-4-(4-(1-methyl-1*H*-imidazol-4-yl)cyclohexyl)-2-((methylsulfonyl) methyl)-morpholine 2,2,2-trifluoroacetate |
| 106. | 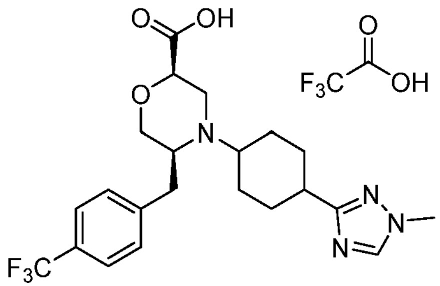 *A single isomer - cis-trans relationship unknown* | B | (2*R*,5*S*)-4-(4-(1-methyl-1*H*-1,2,4-triazol-3-yl)cyclohexyl)-5-(4-(trifluoromethyl)benzyl)-morpholine-2-carboxylic acid 2,2,2-trifluoroacetate |
| 107. | 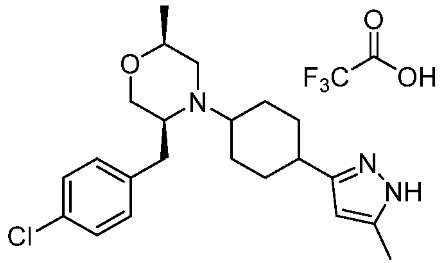 *A single isomer - cis-trans relationship unknown* | B | (2*S*,5*S*)-5-(4-chlorobenzyl)-2-methyl-4-(4-(5-methyl-1*H*-pyrazol-3-yl)cyclohexyl)morpholine 2,2,2-trifluoroacetate |

(continued)

| Ex.# | Structure | YKL-40, $IC_{50}$ / Kd | IUPAC Name |
|---|---|---|---|
| 108. | 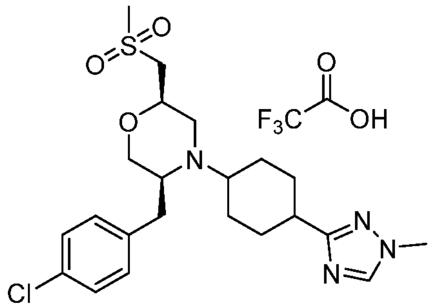 *A single isomer - cis-trans relationship unknown* | A | (2*R*,5*S*)-5-(4-chlorobenzyl)-4-(4-(1-methyl-1*H*-1,2,4-triazol-3-yl)cyclohexyl)-2-((methylsulfonyl) methyl)-morpholine 2,2,2-trifluoroacetate |
| 109. | 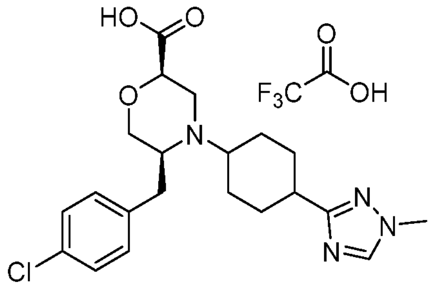 *A single isomer - cis-trans relationship unknown* | A | (2*R*,5*S*)-5-(4-chlorobenzyl)-4-(4-(1-methyl-1*H*-1,2,4-triazol-3-yl)cyclohexyl)morpholine-2-carboxylic acid 2,2,2-trifluoroacetate |
| 110. | 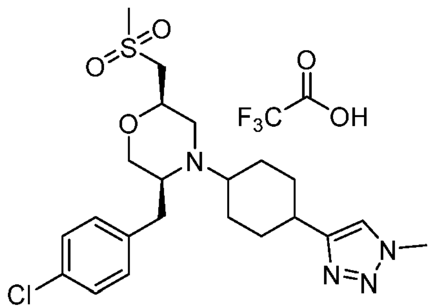 *A single isomer - cis-trans relationship unknown* | C | (2*R*,5*S*)-5-(4-chlorobenzyl)-4-(4-(1-methyl-1*H*-1,2,3-triazol-4-yl)cyclohexyl)-2-((methylsulfonyl) methyl)-morpholine 2,2,2-trifluoroacetate |

(continued)

| Ex.# | Structure | YKL-40, $IC_{50}$ / Kd | IUPAC Name |
|---|---|---|---|
| 111. | 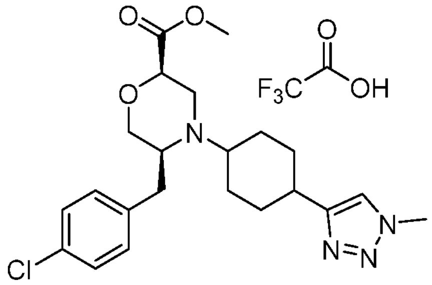 *A single isomer - cis-trans relationship unknown* | C | methyl (2*R*,5*S*)-5-(4-chlorobenzyl)-4-(4-(1-methyl-1*H*-1,2,3-triazol-4-yl)cyclohexyl)morpholine-2-carboxylate 2,2,2-trifluoroacetate |
| 112. | 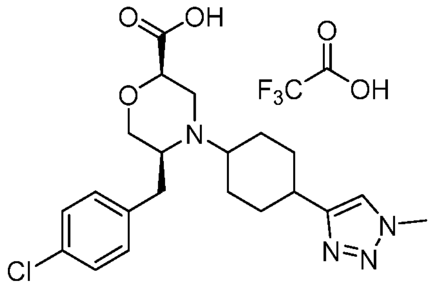 *A single isomer - cis-trans relationship unknown* | C | (2*R*,5*S*)-5-(4-chlorobenzyl)-4-(4-(1-methyl-1*H*-1,2,3-triazol-4-yl)cyclohexyl)morpholine-2-carboxylic acid 2,2,2-trifluoroacetate |
| 113. | 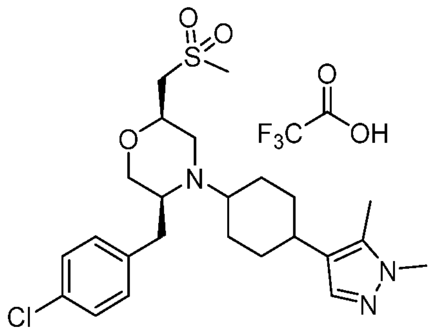 *A single isomer - cis-trans relationship unknown* | E | (2*R*,5*S*)-5-(4-chlorobenzyl)-4-(4-(1,5-dimethyl-1*H*-pyrazol-4-yl)cyclohexyl)-2-((methylsulfonyl)methyl)-morpholine 2,2,2-trifluoroacetate |

(continued)

| Ex.# | Structure | YKL-40, $IC_{50}$ / Kd | IUPAC Name |
|---|---|---|---|
| 114. | 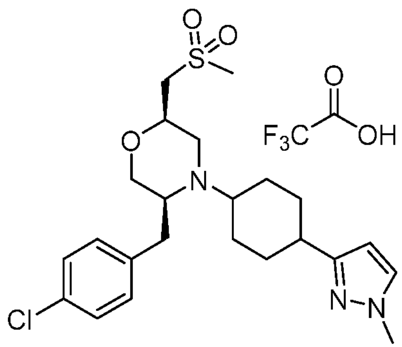 *A single isomer - cis-trans relationship unknown* | B | (2*R*,5*S*)-5-(4-chlorobenzyl)-4-(4-(1-methyl-1*H*-pyrazol-3-yl)cyclohexyl)-2-((methylsulfonyl) methyl)-morpholine 2,2,2-trifluoroacetate |
| 115. | 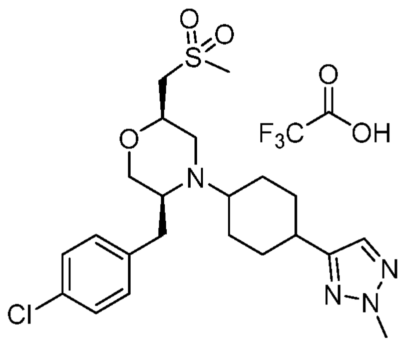 *A single isomer - cis-trans relationship unknown* | B | (2*R*,5*S*)-5-(4-chlorobenzyl)-4-(4-(2-methyl-2*H*-1,2,3-triazol-4-yl)cyclohexyl)-2-((methylsulfonyl) methyl)-morpholine 2,2,2-trifluoroacetate |

(continued)

| Ex.# | Structure | YKL-40, $IC_{50}$ / Kd | IUPAC Name |
|---|---|---|---|
| 116. | 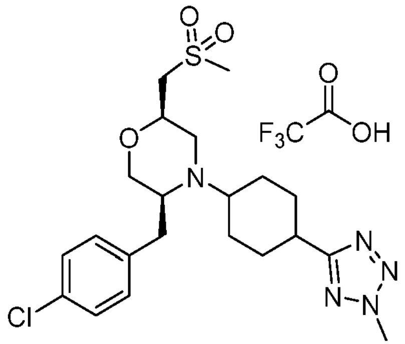 *A single isomer - cis-trans relationship unknown* | B | (2*R*,5*S*)-5-(4-chlorobenzyl)-4-(4-(2-methyl-2*H*-tetrazol-5-yl)cyclohexyl)-2-((methylsulfonyl) methyl)-morpholine 2,2,2-trifluoroacetate |
| 117. | 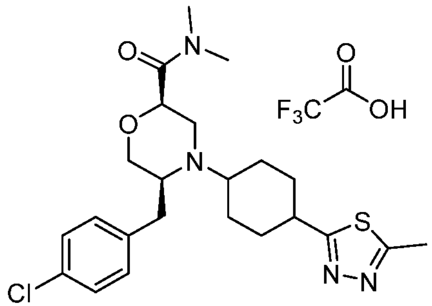 *A single isomer - cis-trans relationship unknown* | C | (2*R*,5*S*)-5-(4-chlorobenzyl)-*N*,*N*-dimethyl-4-(4-(5-methyl-1,3,4-thiadiazol-2-yl)cyclohexyl)morpholine-2-carboxamide 2,2,2-trifluoroacetate |
| 118. | 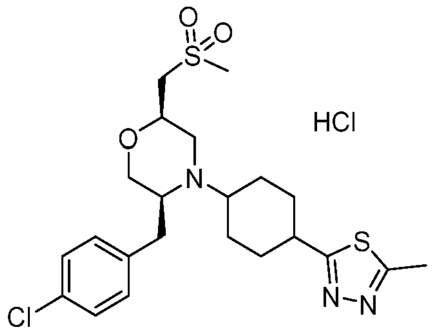 *A single isomer - cis-trans relationship unknown* | C | (2*R*,5*S*)-5-(4-chlorobenzyl)-4-(4-(5-methyl-1,3,4-thiadiazol-2-yl)cyclohexyl)-2-((methylsulfonyl) methyl)-morpholine hydrochloride |

(continued)

| Ex.# | Structure | YKL-40, $IC_{50}$ / Kd | IUPAC Name |
|---|---|---|---|
| 119. | 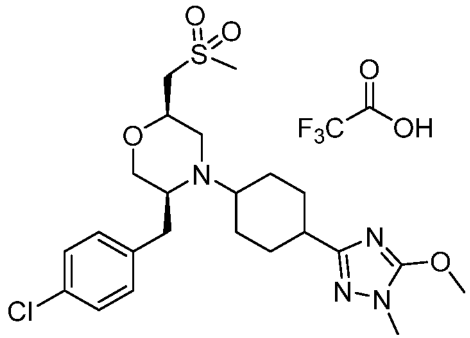 *A single isomer - cis-trans relationship unknown* | C | (2*R*,5*S*)-5-(4-chlorobenzyl)-4-(4-(5-methoxy-1-methyl-1*H*-1,2,4-triazol-3-yl)cyclohexyl)-2-((methylsulfonyl)methyl)-morpholine 2,2,2-trifluoroacetate |
| 120. | 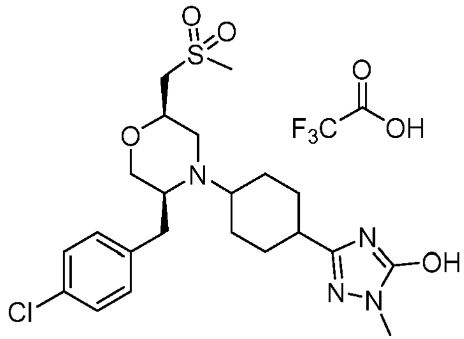 *A single isomer - cis-trans relationship unknown* | C | 3-(4-((2*R*,5*S*)-5-(4-chlorobenzyl)-2-((methylsulfonyl)methyl)-morpholino)cyclohexyl)-1-methyl-1*H*-1,2,4-triazol-5-ol 2,2,2-trifluoroacetate |

(continued)

| Ex.# | Structure | YKL-40, $IC_{50}$ / Kd | IUPAC Name |
|---|---|---|---|
| 121. | 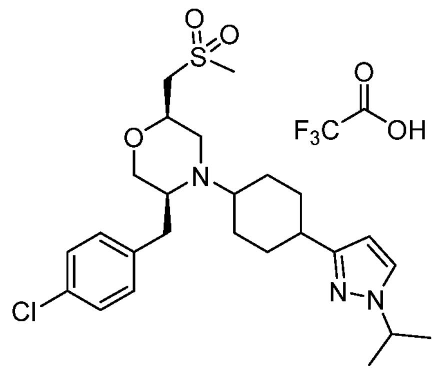 *A single isomer - cis-trans relationship unknown* | D | (2*R*,5*S*)-5-(4-chlorobenzyl)-4-(4-(1-isopropyl-1*H*-pyrazol-3-yl)cyclohexyl)-2-((methylsulfonyl) methyl)-morpholine 2,2,2-trifluoroacetate |
| 122. | 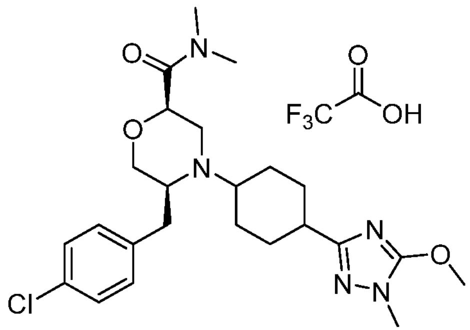 *A single isomer - cis-trans relationship unknown* | C | (2*R*,5*S*)-5-(4-chlorobenzyl)-4-(4-(5-methoxy-1-methyl-1*H*-1,2,4-triazol-3-yl)cyclohexyl)-*N*,*N*-dimethylmorpholine-2-carboxamide 2,2,2-trifluoroacetate |

(continued)

| Ex.# | Structure | YKL-40, $IC_{50}$ / Kd | IUPAC Name |
|---|---|---|---|
| 123. | 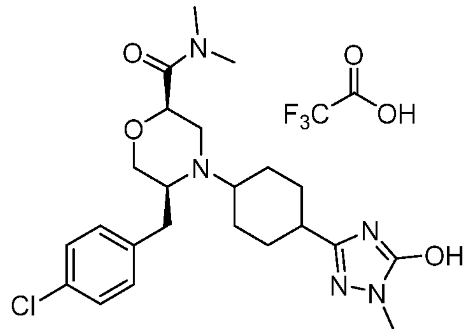 *A single isomer - cis-trans relationship unknown* | - | (2*R*,5*S*)-5-(4-chlorobenzyl)-4-(4-(5-hydroxy-1-methyl-1*H*-1,2,4-triazol-3-yl)cyclohexyl)-*N*,*N*-dimethylmorpholine-2-carboxamide 2,2,2-trifluoroacetate |
| 124. | 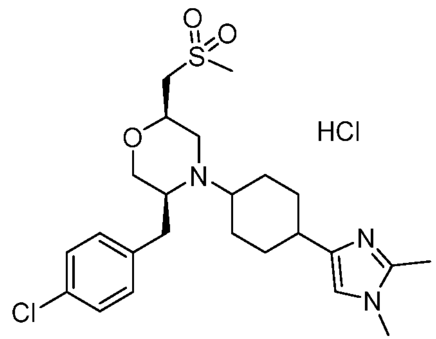 *A single isomer - cis-trans relationship unknown* | B | (2*R*,5*S*)-5-(4-chlorobenzyl)-4-(4-(1,2-dimethyl-1*H*-imidazol-4-yl)cyclohexyl)-2-((methylsulfonyl) methyl)-morpholine hydrochloride |

(continued)

| Ex.# | Structure | YKL-40, $IC_{50}$ / Kd | IUPAC Name |
|---|---|---|---|
| 125. | 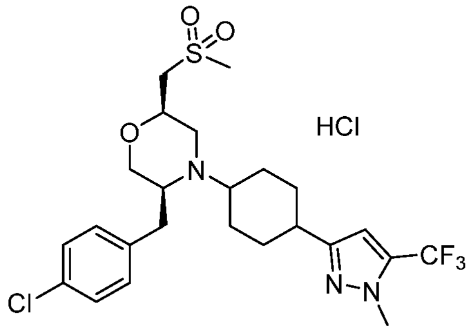 *A single isomer - cis-trans relationship unknown* | B | (2*R*,5*S*)-5-(4-chlorobenzyl)-4-(4-(1-methyl-5-(trifluoromethyl)-1*H*-pyrazol-3-yl)cyclohexyl)-2-((methylsulfonyl)methyl)-morpholine hydrochloride |
| 126. | 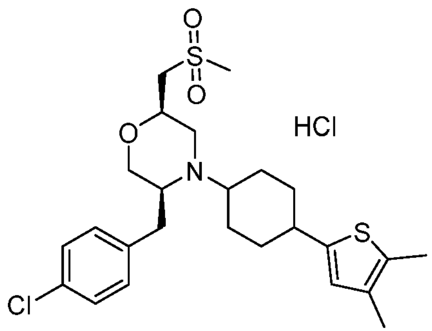 *A single isomer - cis-trans relationship unknown* | C | (2*R*,5*S*)-5-(4-chlorobenzyl)-4-(4-(4,5-dimethylthiophen-2-yl)cyclohexyl)-2-((methylsulfonyl)methyl)-morpholine hydrochloride |

(continued)

| Ex.# | Structure | YKL-40, $IC_{50}$ / Kd | IUPAC Name |
|---|---|---|---|
| 127. | 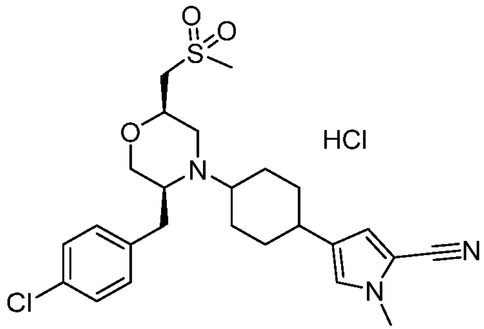 *A single isomer - cis-trans relationship unknown* | E | 4-(4-((2*R*,5*S*)-5-(4-chlorobenzyl)-2-((methylsulfonyl)methyl)-morpholino)cyclohexyl)-1-methyl-1*H*-pyrrole-2-carbonitrile hydrochloride |
| 128. | 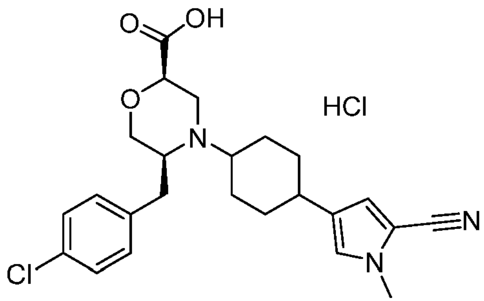 *A single isomer - cis-trans relationship unknown* | E | (2*R*,5*S*)-5-(4-chlorobenzyl)-4-(4-(5-cyano-1-methyl-1*H*-pyrrol-3-yl)cyclohexyl)morpholine-2-carboxylic acid hydrochloride |
| 129. | 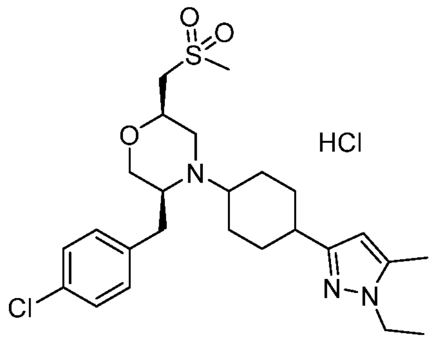 *A single isomer - cis-trans relationship unknown* | B | (2*R*,5*S*)-5-(4-chlorobenzyl)-4-(4-(1-ethyl-5-methyl-1*H*-pyrazol-3-yl)cyclohexyl)-2-((methylsulfonyl)methyl)-morpholine hydrochloride |

(continued)

| Ex.# | Structure | YKL-40, $IC_{50}$ / Kd | IUPAC Name |
|---|---|---|---|
| 130. | 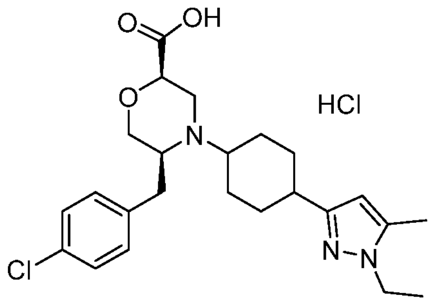 *A single isomer - cis-trans relationship unknown* | B | (2*R*,5*S*)-5-(4-chlorobenzyl)-4-(4-(1-ethyl-5-methyl-1*H*-pyrazol-3-yl)cyclohexyl)morpholine-2-carboxylic acid hydrochloride |
| 131. | 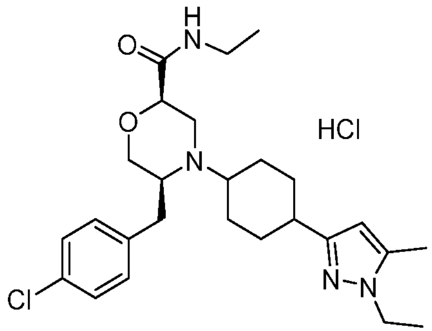 *A single isomer - cis-trans relationship unknown* | C | (2*R*,5*S*)-5-(4-chlorobenzyl)-*N*-ethyl-4-(4-(1-ethyl-5-methyl-1*H*-pyrazol-3-yl)cyclohexyl)morpholine-2-carboxamide hydrochloride |
| 132. | 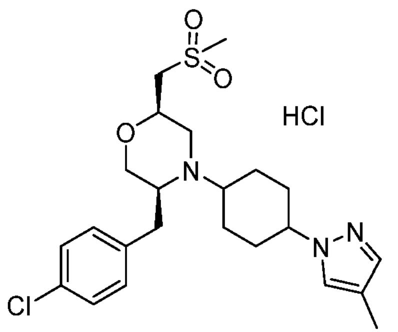 *A single isomer - cis-trans relationship unknown* | A | (2*R*,5*S*)-5-(4-chlorobenzyl)-4-(4-(4-methyl-1*H*-pyrazol-1-yl)cyclohexyl)-2-((methylsulfonyl)methyl)-morpholine hydrochloride |

(continued)

| Ex.# | Structure | YKL-40, $IC_{50}$ / Kd | IUPAC Name |
|---|---|---|---|
| 133. | 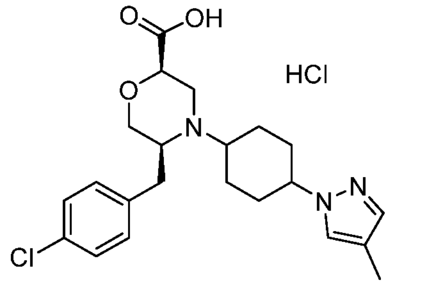 *A single isomer - cis-trans relationship unknown* | A | (2*R*,5*S*)-5-(4-chlorobenzyl)-4-(4-(4-methyl-1*H*-pyrazol-1-yl)cyclohexyl)morpholine-2-carboxylic acid hydrochloride |
| 134. | 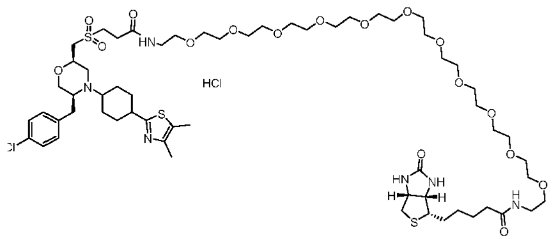 *A single isomer - cis-trans relationship unknown* | *A | *N*-(39-((((2*R*,5*S*)-5-(4-chlorobenzyl)-4-(4-(4,5-dimethylthiazol-2-yl)cyclohexyl)morpholin-2-yl) methyl)sulfonyl)-37-oxo-3,6,9,12,15,18,21,24,27,30,33 -undecaoxa-36-azanonatriacontyl)-5-((3a*S*,4*S*,6a*R*)-2-oxohexahydro-1*H*-thieno[3,4-*d*]imidazol-4-yl)pentanamide hydrochloride |
| 135. | 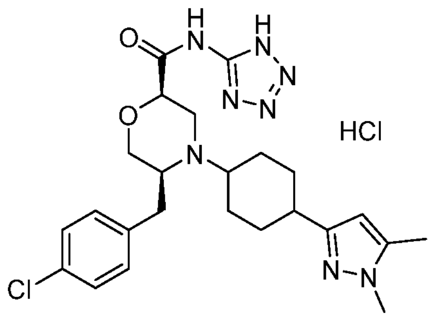 *A single isomer - cis-trans relationship unknown* | A | (2*R*,5*S*)-5-(4-chlorobenzyl)-4-(4-(1,5-dimethyl-1*H*-pyrazol-3-yl)cyclohexyl)-*N*-(1*H*-tetrazol-5-yl) morpholine-2-carboxamide hydrochloride |

(continued)

| Ex.# | Structure | YKL-40, $IC_{50}$ / Kd | IUPAC Name |
| --- | --- | --- | --- |
| 136. | 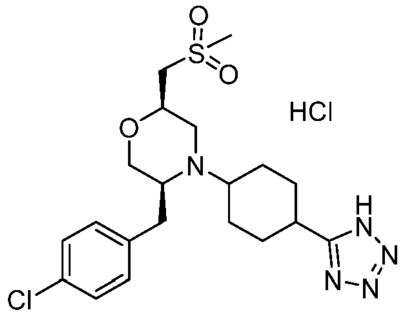 HCl *A single isomer - cis-trans relationship unknown* | - | (2*R*,5*S*)-4-(4-(1*H*-tetrazol-5-yl)cyclohexyl)-5-(4-chlorobenzyl)-2-((methylsulfonyl)methyl)-morpholine hydrochloride |
| 137. | 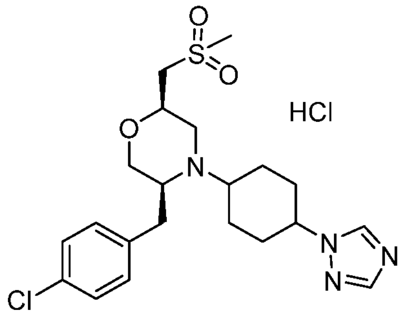 HCl *A single isomer - cis-trans relationship unknown* | E | (2*R*,5*S*)-4-(4-(1*H*-1,2,4-triazol-1-yl)cyclohexyl)-5-(4-chlorobenzyl)-2-((methylsulfonyl)methyl)-morpholine hydrochloride |
| 138. | 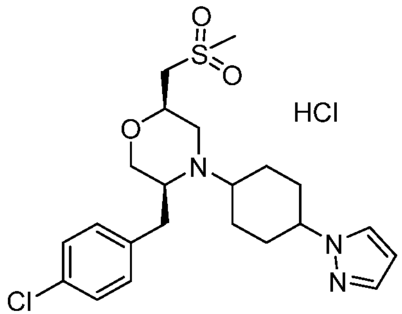 HCl *A single isomer - cis-trans relationship unknown* | C | (2*R*,5*S*)-4-(4-(1*H*-pyrazol-1-yl)cyclohexyl)-5-(4-chlorobenzyl)-2-((methylsulfonyl)methyl)-morpholine hydrochloride |

(continued)

| Ex.# | Structure | YKL-40, $IC_{50}$ / Kd | IUPAC Name |
|---|---|---|---|
| 139. | 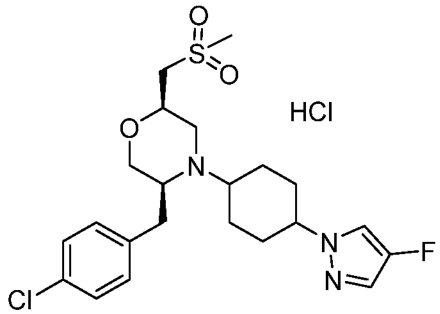 *A single isomer - cis-trans relationship unknown* | B | (2*R*,5*S*)-5-(4-chlorobenzyl)-4-(4-(4-fluoro-1*H*-pyrazol-1-yl)cyclohexyl)-2-((methylsulfonyl) methyl)-morpholine hydrochloride |
| 140. | 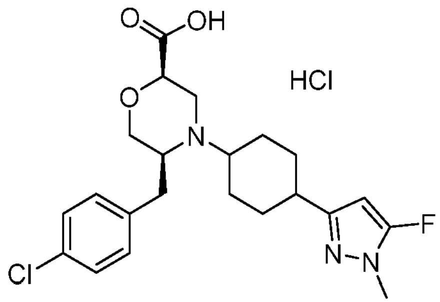 *A single isomer - cis-trans relationship unknown* | A | (2*R*,5*S*)-5-(4-chlorobenzyl)-4-(4-(5-fluoro-1-methyl-1*H*-pyrazol-3-yl)cyclohexyl)morpholine-2-carboxylic acid hydrochloride |
| 141. | 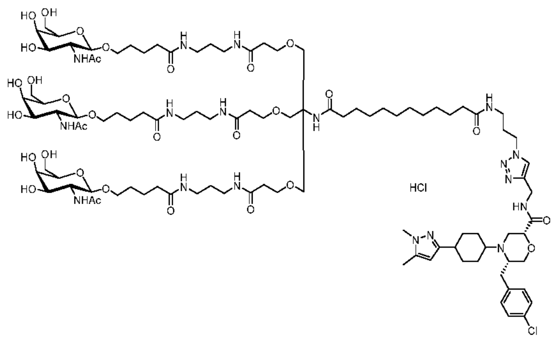 *A single isomer - cis-trans relationship unknown* | A | *N*[1]-(1,31-bis(((2*R*,3*R*,4*R*,5*R*,6*R*)-3-acetamido-4,5-dihydroxy-6-(hydroxymethyl)tetrahydro-2H-pyran-2-yl)oxy)-16-((3-((3-(5-(((2*R*,3*R*,4*R*,5*R*,6*R*)-3-acetamido-4,5-dihydroxy-6-(hydroxymethyl) tetrahydro-2*H*-pyran-2-yl)oxy)pentanamido)propyl)-amino)-3-oxopropoxy)methyl)-5,11,21,27-tetraoxo-14,18-dioxa-6,10,22,26-tetraazahentriacontan-16-yl)-*N*[12]-(3-(4-(((2*R*,5*S*)-5-(4-chlorobenzyl)-4-(4-(1,5-dimethyl-1*H*-pyrazol-3-yl)cyclohexyl)morpholine-2-carboxamido)methyl)-1*H*-1,2,3-triazol-1-yl)propyl)dodecanediamide hydrochloride |

(continued)

| Ex.# | Structure | YKL-40, $IC_{50}$ / Kd | IUPAC Name |
|---|---|---|---|
| 142. | HCl 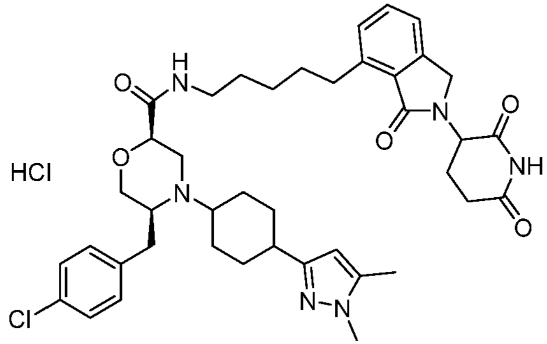 *A single isomer - cis-trans relationship unknown* | B | (2*R*,5*S*)-5-(4-chlorobenzyl)-4-(4-(1,5-dimethyl-1*H*-pyrazol-3-yl)cyclohexyl)-*N*-(5-(2-(2,6-dioxopiperidin-3-yl)-3-oxoisoindolin-4-yl)pentyl)morpholine-2-carboxamide hydrochloride |

Based on NMR spectra, polarity, and activity in AlphaScreen assay, the instant inventors believe that the majority of most active compounds according to the invention have *trans* relationship of the 1,4 substituents of the cyclohexane ring.

## *General Synthetic Procedures*

### General Procedure Ia

Reduction of $\alpha$-amino acid to the corresponding amino alcohol.

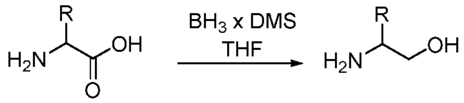


To a suspension of amino acid in anhydrous tetrahydrofuran (THF) (3 mL/mmol) borane-dimethylsulfide complex ($BH_3$ x DMS; 3 equivalents) was added dropwise at 0 °C (Caution: foaming!) The cooling bath was removed and reaction mixture was refluxed overnight, after which time TLC or LC-MS control indicated completion of the reaction. The mixture was cooled to room temperature and 6 M HCl (8 equivalents with respect to the starting material) was carefully added dropwise (Caution: foaming!) and the mixture was again refluxed for 1.5 hours. The mixture was cooled to room temperature and pH was brought up to 10 with suitable addition of 4 M NaOH. Product was extracted several times with ethyl acetate (AcOEt), extracts were combined, dried over anhydrous $MgSO_4$, filtered and concentrated *in vacuo.* The crude product was triturated with ethyl ether ($Et_2O$) and filtered off.

### General Procedure Ib

Reduction of morpholin-3-one to morpholine or amide to amine.

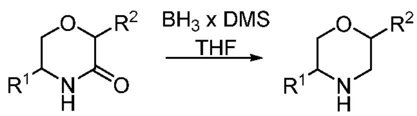


To the solution of either morpholin-3-one or 2-piperazinone or amide in THF (3 mL/mmol) borane-dimethylsulfide complex ($BH_3$ x DMS; 3 equivalents) was added and the reaction mixture was refluxed for 3 hours, after which time the TLC or LC-MS control indicated completed consumption of the starting material. Reaction mixture was cooled to room temperature and 2 M or 6 M HCl was cautiously added (6 equivalents with respect to the starting material). The resulting reaction mixture was refluxed for 2 hours and cooled back to room temperature. The pH of the solution was then adjusted to strongly alkaline (~10) by a dropwise addition of 6 M NaOH. The organic layer was separated and the aqueous layer was additionally extracted with diethyl ether or AcOEt. The combined organic extracts were then dried over anhydrous $MgSO_4$, filtered and the solvents were evaporated. The crude product obtained was, in most cases, sufficiently pure to be used to the next step without any additional purification.

### General Procedure II

Cyclization of $\alpha$-haloamide to morpholin-3-one.

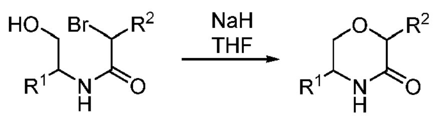


To the solution of the $\alpha$-haloamide (*i.e.*, $\alpha$-chloro- or $\alpha$-bromoamide) in THF (10 mL/mmol) 3 equivalents of sodium

hydride (NaH) was added in one portion (cooling the solution prior to the addition of NaH may was advisable when working on larger scale) and the reaction mixture was allowed to stir at room temperature for 2 hours. The excess of NaH was then carefully quenched by dropwise addition of brine and then additional volume of brine (equal to the initial volume of THF) was added causing phases separation. The organic layer was separated and the aqueous layer was additionally extracted with diethyl ether. The combined organic extracts were then dried over anhydrous $MgSO_4$, filtered and the solvents were evaporated. The crude product was in most cases sufficiently pure to be used to the next step without any additional purification.

**General Procedure III**

Amino-selective acylation of amino alcohol with $\alpha$-bromoacid with the use of an amide-forming reagent.

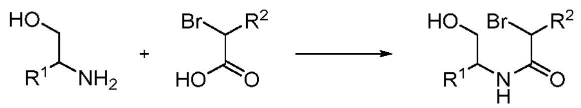

To the solution of $\alpha$-bromoacid in dichloromethane (7 mL/mmol) diisopropylethylamine (DIPEA, 1 equivalent with respect to the starting $\alpha$-bromoacid), coupling reagent (1 equivalent; typically TBTU or HATU, but other commonly used coupling reagents may be used as well) and amino alcohol (1 equivalent) were added sequentially and the reaction mixture was stirred for 1.5 hours or overnight at room temperature. After this time TLC control showed completed consumption of the starting materials and the reaction mixture was transferred to the separatory funnel and washed sequentially with 1 M HCl, 1 M NaOH, and brine. The organic phase was dried over anhydrous $MgSO_4$, filtered and evaporated to give the crude product which was further purified by crystallization or by silica-gel column chromatography.

**General Procedure IVa**

Removal of the *tert*-butoxycarbonyl (Boc-) group from amine with HCl.

The N-Boc protected amine was treated with a 4 M solution of HCl (5 mL/mmol of starting material) in an appropriate organic solvent (*e.g.*, AcOEt, 1,4-dioxane, MeOH, DCM) for the time necessary for complete consumption of the starting material (typically 30 minutes - 2 hours). The volatiles were then removed *in vacuo* providing de-protected amine in the form of its hydrochloride salt. The crude product was usually sufficiently pure to be used in the following step, but additional trituration with diethyl ether may help to remove any colored impurities or the crude product was purified by preparative reversed-phase column chromatography to give the corresponding product.

**General Procedure IVb**

Removal of the *tert*-butoxycarbonyl (Boc-) group from amine with TFA.

The N-Boc protected amine was treated with solution of TFA (6 equivalents) in DCM for the time necessary for complete consumption of the starting material (typically 30 minutes - 2 hours). The volatiles were then removed *in vacuo* providing de-protected amine in the form of its TFA salt. The crude product was usually purified by preparative reversed-phase column chromatography to give the corresponding product.

**General Procedure V**

Activation of carboxylic group by mixed anhydride followed by formation of an amide.

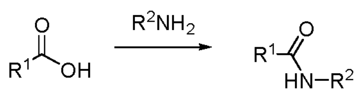


Carboxylic acid was dissolved in dichloromethane (DCM) (3-8 mL/mmol depending on the solubility) and N-methylmorpholine (1.2 equivalent) was added. The solution was cooled to - 15 °C and alkyl (typically methyl, ethyl or

isobutyl) chloroformate (1.2 equivalent) was added and the mixture was stirred for additional 10 minutes at which time the appropriate amine (neat, 1.2 equivalent) was added. The reaction mixture was allowed to warm to room temperature and was typically stirred overnight, though in the cases of reactive amines the coupling was usually completed within minutes. The crude product was isolated by washing of organic phase (DCM) subsequentially with 1 M HCl, 1 M NaOH, and brine. The organic phase was dried over anhydrous $MgSO_4$, filtered and evaporated to give the crude product which was further purified by crystallization or by silica-gel column chromatography or by preparative reversed-phase column chromatography.

**General Procedure VI**

Addition of Grignard reagent to carbonyl group.

An appropriate Grignard reagent (3 equivalents) was added dropwise to a solution of carbonyl compound in $Et_2O$ or THF (6 mL/mmol) at -40 °C. After this reaction was allowed to warm up to room temperature. The reaction progress was monitored by TLC and LC-MS analysis of small aliquots of the crude reaction mixture. When analyses were indicated completion of the reaction, the mixture was poured into saturated solution of $NH_4Cl$. An organic phase was separated, and the aqueous phase was extracted with AcOEt. The combined organic phases were dried over anhydrous $MgSO_4$, concentrated *in vacuo*. The crude product was purified by silica-gel column chromatography or by flash column chromatography on silica or by preparative reversed-phase column chromatography.

**General Procedure VII**

Reductive amination of the cyclic ketone with appropriate amine.

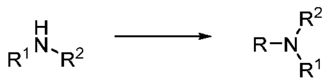

An appropriate amine or an amine hydrochloride (1 equivalent) was dissolved in DCE and acetic acid (AcOH, 2 equivalents) and appropriate ketone (2 equivalents) were added and the mixture was heated at 50 °C for 2 days. The reaction mixture was cooled to room temperature and sodium triacetoxyborohydride ($NaBH(OAc)_3$) (4 equivalents) was then added in one portion and the mixture was stirred overnight at room temperature. After this time a 5% aqueous solution of sodium bicarbonate ($NaHCO_3$) and dichloromethane (DCM) were added and the mixture was stirred for 30 minutes. The layers were separated and the aqueous layer was additionally extracted with dichloromethane. The organic extracts were combined, washed with brine, dried over anhydrous $MgSO_4$, filtered and evaporated to dryness. The crude product was purified by flash column chromatography on silica or by preparative reversed-phase column chromatography.

**General procedure VIII**

Mesylation of appropriate hydroxyl group.

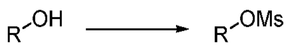

To a cooled to 0 °C solution of alcohol, triethylamine ($Et_3N$, 1.6 equivalent) in DCM (20 mL/mmol) a solution of methanesulfonic anhydride (1.5 equivalent) or methanesulfonic chloride (1.2 equivalent) in DCM (6 mL/mmol) was added dropwise and the mixture was stirred at room temperature for 30 minutes. After this time the reaction was washed with 1 M $K_2CO_3$, brine, dried over anhydrous $MgSO_4$, filtered and the solvents were evaporated *in vacuo.* The crude product was in most cases sufficiently pure to be used to the next step without any additional purification.

**General Procedure IX**

Formation of Weinreb amide from carboxylic acid and *N,O*-dimethylhydroxylamine hydrochloride with the use of the amide forming reagent.

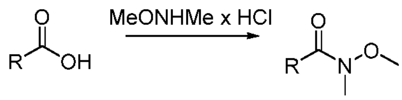


To a solution of carboxylic acid in dichloromethane (2 mL/mmol), DIPEA (only when TBTU was used) (2.1 equivalents) was added, followed by N,O-dimethylhydroxylamine hydrochloride (1.1 equivalent). TBTU (1.1 equivalent) or CDI (1.1 equivalent) was added to the reaction mixture and reaction was stirred at room temperature for the time necessary for the complete consumption of the starting material (usually 16 - 24 hours) as judged by TLC or LC-MS. The reaction mixture was diluted with dichloromethane, then washed with 2 M HCl and brine. An organic layer was dried over anhydrous $MgSO_4$, filtered and concentrated *in vacuo.* Product was purified by silica-gel column chromatography.

**General Procedure X**

Installation of the appropriate R-group on the primary or secondary amine or alcohol

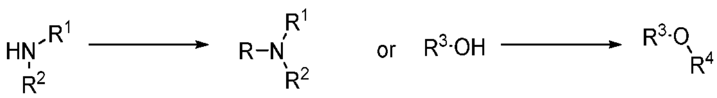

To a solution of the primary or secondary amine or alcohol in DCM (10 mL/mmol) or THF (4 mL/mmol), $Et_3N$ (2 - 4 equivalents) or DIPEA (2 equivalents) followed by DMAP (0.1 equivalent) were added and then *N*-succinimidyl-*N*-methyl carbamate (2.5 equivalents) or appropriate carbamoyl chloride (2.5 equivalents) or appropriate isocyanate (1.1 - 2.5 equivalents) or acid chloride (1 - 2 equivalents) or appropriate anhydride (1.1 - 2 equivalents) or MOMCl (2 equivalents) was added at room temperature. The reaction progress was monitored by TLC and LC-MS. When analyses indicated completion of the reaction, the mixture was quenched by addition of 4 M NaOH or 5% $NaHCO_3$ (for tosylation). Product was extracted with DCM (2 times). The combined organic solutions were dried over anhydrous $Na_2SO_4$, filtered and concentrated *in vacuo* and the crude product was purified by preparative reversed-phase column chromatography or by silica-gel column chromatography.

**General Procedure XI**

Biotynylation (with BiotinPEG$_{11}$NH$_2$) of appropriate carboxylic acid.

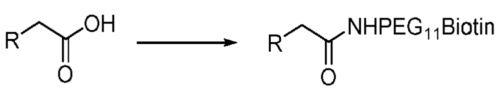

To the solution of appropriate carboxylic acid (1 - 1.2 equivalent) in DMF or DCM (20 mL/mmol), $Et_3N$ (6 equivalents) or DIPEA (6 equivalents) and BiotinPEG$_{11}$NH$_2$ (1 - 2 equivalents) were added followed by addition of HATU (1.1 equivalent) or EDCI hydrochloride (1 equivalent) and the reaction mixture was stirred overnight at room temperature. After this time LC-MS control showed complete consumption of the starting materials and the reaction mixture was evaporated to dryness and the crude product was further purified by preparative reversed-phase column chromatography.

**General Procedure XII**

Fluorination of alcohols and carbonyl compounds

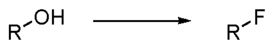

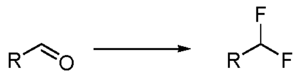

To a cooled to -20 °C or -70 °C solution of alcohol or aldehyde in dry THF or DCM (6 mL/mmol) DAST (2 - 2.5 equivalents) was added dropwise and the reaction was allowed to warm to room temperature and stirring was continued overnight. The reaction was poured into 5% $NaHCO_3$ and the resulting mixture was stirred for 15 minutes. The organic phase was separated and an aqueous one was extracted with DCM, washed with brine, dried over anhydrous $MgSO_4$, filtered and concentrated *in vacuo.* The crude product was purified by silica-gel column chromatography or by flash column chromatography on silica or by preparative reversed-phase column chromatography.

***<u>Exemplary Synthetic Procedures</u>***

**Example 1.**

Synthesis of (2S,5S)-5-(4-chlorobenzyl)-2-methyl-4-(4-(5-methyloxazol-2-yl)cyclohexyl)-morpholine hydrochloride (**1**).

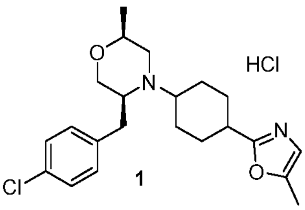

**1**

Step 1.

Synthesis of (2S)-2-amino-3-(4-chlorophenyl)propan-1-ol (**1a**).

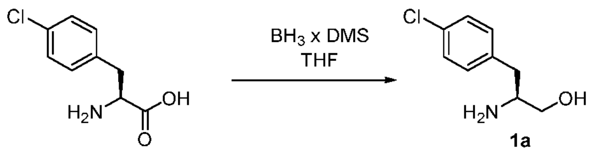


**1a**

The title compound (**1a**) was obtained from optically pure L-p-chlorophenylalanine ((2S)-2-amino-3-(4-chlorophenyl)propanoic acid) (10.67 g; 53.45 mmol) according to the General Procedure **Ia** in 87% yield (8.61 g; 46.50 mmol). ESI-MS m/z for $C_9H_{12}ClNO$ found 185.7/187.7 $[M+H]^+$; Rt = 0.50 min (Method F); $^1H$ NMR (500 MHz, $CDCl_3$) δ 7.28 (d, *J* = 8.3 Hz, 2H), 7.11 (d, *J* = 8.3 Hz, 2H), 3.62 (dd, *J* = 10.6, 3.8 Hz, 1H), 3.37 (dd, *J* = 10.5, 6.9 Hz, 1H), 3.11-3.07 (brs, 1H), 2.76 (dd, *J* = 13.6, 5.4Hz, 1H), 2.50 (dd, *J* = 13.6, 8.6 Hz, 1H).

Step 2.

Synthesis of (R)-2-bromo-N-((S)-1-(4-chlorophenyl)-3-hydroxypropan-2-yl)propanamide (**1b**).

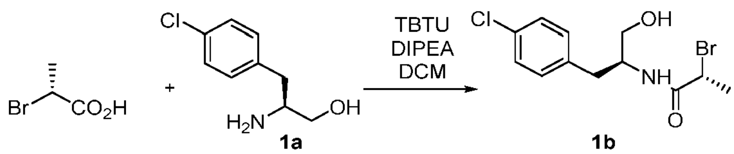


**1a** **1b**

(2S)-2-amino-3-(4-chlorophenyl)propan-1-ol (**1a**) (1 g; 5.38 mmol) was coupled with (R)-2-bromopropanoic acid according to the General Procedure **III** using TBTU as an amide bond forming reagent. The title compound **1b** was

obtained as a white solid in 64% yield (1.1 g; 3.44 mmol).
ESI-MS m/z for $C_{12}H_{16}BrClNO_2$ found 320.7/322.7 $[M+H]^+$; Rt = 2.49 min (Method F); $^1H$ NMR (500 MHz, $CDCl_3$) δ 7.25 (d, *J* = 8.2 Hz, 2H), 7.14 (d, *J* = 8.2 Hz, 2H), 6.58 (d, *J* = 6.7Hz, 1H), 4.33 (dd, *J* = 14.1, 7.1 Hz, 1H), 4.13 - 4.06 (m, 1H), 3.69 - 3.64 (m, 1H), 3.61 - 3.57 (m, 1H), 2.90 - 2.81 (m, 2H), 2.17 (brs, 1H), 1.82 (d, *J* = 6.9 Hz, 3H).

Step 3.

Synthesis of (2*S*,5*S*)-5-(4-chlorobenzyl)-2-methylmorpholin-3-one (**1c**).

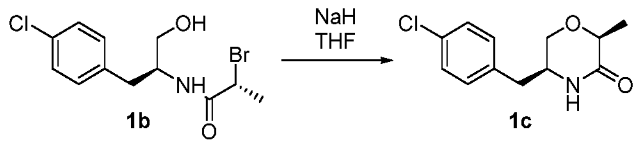


**1b** **1c**

The title compound (**1c**) was obtained from **1b** (1.1 g; 3.44 mmol) according to the General Procedure **II** in 79% yield (650 mg; 2.72 mmol).

ESI-MS m/z for $C_{12}H_{15}ClNO_2$ found 240.1/242.1 $[M+H]^+$; Rt = 2.45 min (Method F); $^1H$ NMR (500 MHz, $CDCl_3$) δ 7.27 (d, *J* = 8.2 Hz, 2H), 7.10 (d. *J* = 8.2 Hz, 2H), 6.13 (brs, 1H), 4.18 (m, 1H), 3.76 (d, *J* = 3.0 Hz, 2H), 3.55 - 3.50 (m, 1H), 2.91 - 2.83 (m, 2H), 1.46 (d, *J* = 6.9 Hz, 3H). Step 4.

Synthesis of (2S,5S)-5-(4-chlorobenzyl)-2-methylmorpholine (**1d**).

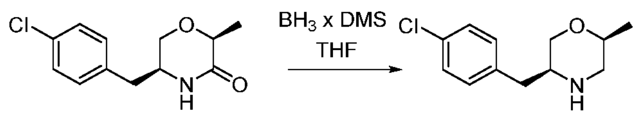


**1c** **1d**

The title compound (**1d**) was obtained from **1c** (1.1 g; 4.6 mmol) according to the General Procedure **Ib** in 90% yield (932 mg; 4.14 mmol).
ESI-MS m/z for $C_{12}H_{17}ClNO$ found 226.4/ 228.4 $[M+H]^+$; Rt = 1.30 min (Method F); $^1H$ NMR (500 MHz, DMSO-$d_6$+ $D_2O$) δ 7.33 (d, *J* = 8.3 Hz, 2H), 7.23 (d, *J* = 8.3 Hz, 2H), 3.50 - 3.48 (m, 1H), 3.49 - 3.47 (m, 2H), 2.87 - 2.81 (m, 1H), 2.80 - 2.76 (m, 2H), 2.65 (dd, *J* = 12.4 Hz, 8.3 Hz, 1H), 2.58 (dd, *J* = 12.4 Hz, 3.0 Hz, 1H), 1.09 (d, *J* = 6.2Hz, 3H).

Step 5.

Synthesis of *N*-(prop-2-yn-1-yl)-1,4-dioxaspiro[4.5]decane-8-carboxamide (**1e**).

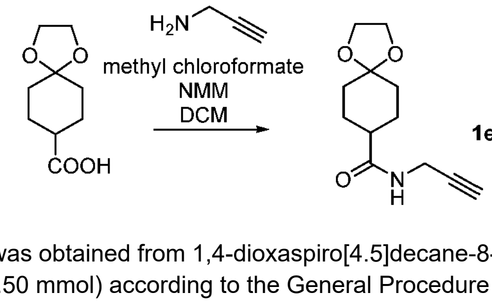


**1e**

The title compound (**1e**) was obtained from 1,4-dioxaspiro[4.5]decane-8-carboxylic acid (560 mg; 3.00 mmol) and prop-2-yn-1-amine (288 μL; 4.50 mmol) according to the General Procedure V in 84% yield (560 mg; 2.51 mmol). ESI-MS m/z for $C_{12}H_{18}NO_3$ found 224.0 $[M+H]^+$; Rt = 0.49 min (Method A); $^1H$ NMR (700 MHz, DMSO-$d_6$ + $D_2O$) δ 3.86 - 3.83 (m, 4H), 3.82 - 3.78 (m, 2H), 2.98 (s, 1H), 2.16 - 2.11 (m, 1H), 1.69 - 1.62 (m, 4H), 1.59 - 1.52 (m, 2H), 1.46 - 1.37 (m, 2H).

Step 6.

Synthesis of 5-methyl-2-(1,4-dioxaspiro[4.5]decan-8-yl)oxazole (**1f**).

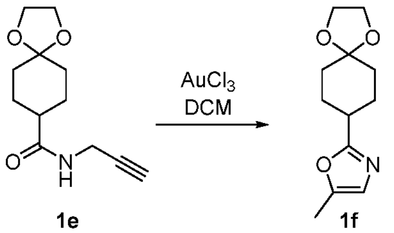


1e 1f

To the solution of **1e** (400 mg; 1.80 mmol) in DCM (8 mL) placed into a reactor $AuCl_3$ (27 mg; 0.09 mmol) was added and whole was stirred at room temperature for 2 days. The reaction progress was monitored by LC-MS analysis. After analytical control indicated completion of the reaction, a whole was concentrated *in vacuo.* The crude product was purified by flash column chromatography on silica (hexane/AcOEt, 10:1 to 2:1, v/v). Compound **1f** was obtained in 87% yield (349 mg; 1.56 mmol).
ESI-MS m/z for $C_{12}H_{18}NO_3$ found 224.0 $[M+H]^+$; Rt = 0.93 min (Method A); $^1$H NMR (700 MHz, DMSO-$d_6$ + $D_2O$) δ 6.66 (q, $J$ = 1.3 Hz, 1H), 3.84 (s, 4H), 2.81 - 2.76 (m, 1H), 2.22 (d, $J$ = 1.3 Hz, 3H), 1.93 - 1.88 (m, 2H), 1.71 - 1.65 (m, 4H), 1.59 - 1.52 (m, 2H).

Step 7.

Synthesis of 4-(5-methyloxazol-2-yl)cyclohexan-1-one (**1g**).

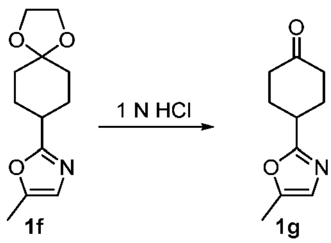


1f 1g

The solution of **1f** (346 mg; 1.55 mmol) in 1 N HCl (10 mL) was stirred at room temperature for 2 hours. The reaction progress was monitored by LC-MS. When analysis indicated completion of the reaction, to this mixture aqueous solution of NaOH (4 M) was added to pH 12 and then extracted with DCM (2 x). The combined organic solutions were dried over anhydrous $MgSO_4$, filtered and concentrated *in vacuo.* The crude product was used to the next step without additional purification. Compound **1g** was obtained in 96% yield (267 mg; 1.49 mmol).
ESI-MS m/z for $C_{10}H_{14}NO_2$ found 180.0 $[M+H]^+$; Rt = 0.71 min (Method A); $^1$H NMR (700 MHz, DMSO-$d_6$ + $H_2O$, 348 K) δ 6.68 (q, $J$ = 1.3 Hz, 1H), 3.26 - 3.21 (m, 1H), 2.49 - 2.43 (m, 2H), 2.36 - 2.29 (m, 2H), 2.27 - 2.21 (m, 5H), 1.98 - 1.91 (m, 2H).

Step 8.

Synthesis of (2*S*,5*S*)-5-(4-chlorobenzyl)-2-methyl-4-(4-(5-methyloxazol-2-yl)cyclohexyl)-morpholine hydrochloride (**1**).

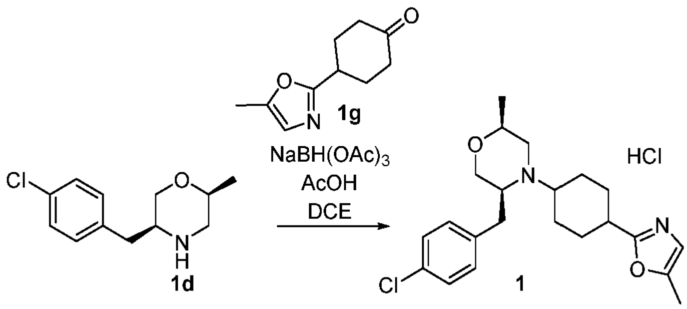


The title compound (**1**) was obtained as a single isomer as a hydrochloride salt from **1d** (270 mg; 1.20 mmol) and ketone **1g** (163 mg; 0.90 mmol) according to the General Procedure **VII** in 14% yield (57 mg; 0.13 mmol). The crude product was purified by flash column chromatography on silica (hexane/AcOEt, 4:1 to 1:2, v/v) and then transformed into hydrochloride salt with using HCl/AcOEt.
ESI-MS m/z for $C_{22}H_{30}ClN_2O_2$ found 389.3/391.3 $[M+H]^+$; Rt = 0.99 min (Method A); $^1H$ NMR (700 MHz, DMSO-$d_6$ + $D_2O$, 348 K) δ 7.38 (d, $J_{AA'}$ = 8.5 Hz, 2H), 7.32 (d, $J_{BB'}$ = 8.5 Hz, 2H), 6.66 (q, $J$ = 1.2 Hz, 1H), 3.90 - 3.89 (m, 1H), 3.79 - 3.77 (m, 1H), 3.72 - 3.70 (m, 1H), 3.59 - 3.57 (m, 1H), 3.47 - 3.42 (m, 2H), 3.16 - 3.10 (m, 2H), 3.01 - 2.99 (m, 1H), 2.73 (tt, $J$ = 11.8 Hz, $J$ = 3.6 Hz, 1H), 2.34 - 2.31 (m, 1H), 2.28 - 2.26 (m, 1H), 2.23 (d, $J$ = 1.2 Hz, 3H), 2.19 - 2.15 (m, 2H), 1.65 - 1.52 (m, 4H), 1.22 (d, $J$ = 6.3 Hz, 3H).

**Example 2.**

Synthesis of (2*S*,5*S*)-5-(4-chlorobenzyl)-4-(4-(1,5-dimethyl-1*H*-pyrazol-3-yl)cyclohexyl)-2-methylmorpholine 2,2,2-trifluoroacetate (**2**).

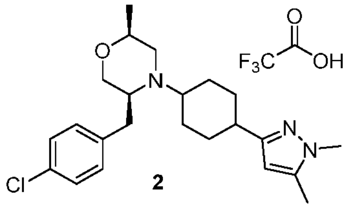

2

Step 1.

Synthesis of *N*-methoxy-*N*-methyl-1,4-dioxaspiro[4.5]decane-8-carboxamide (**2a**).

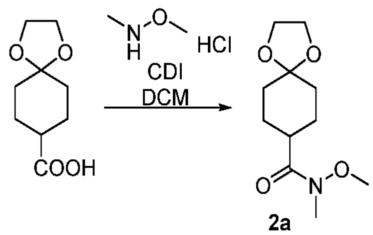


2a

The title compound (**2a**) was obtained from 1,4-dioxaspiro[4.5]decane-8-carboxylic acid (6 g; 32.2 mmol) according to the General Procedure **IX** in 99% yield (7.31 g; 31.9 mmol). ESI-MS m/z for $C_{11}H_{20}NO_4$ found 230.1 $[M+H]^+$; $R_t$ = 0.67 min (Method A)

Step 2.

Synthesis of 1-(1,4-dioxaspiro[4.5]decan-8-yl)but-2-yn-1-one (**2b**).

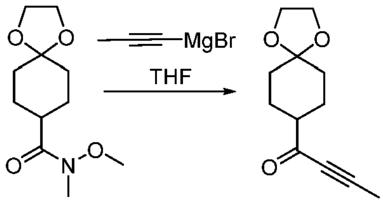


2a 2b

The title compound (**2b**) was obtained from **2a** (690 mg; 3.00 mmol) and from prop-1-yn-1-ylmagnesium bromide (0.5 M; 18 mL; 9.00 mmol) according to the General Procedure **VI** in 99% yield (618 mg; 2.97 mmol).
ESI-MS m/z for $C_{12}H_{17}O_3$ found 209.0 $[M+H]^+$; Rt = 0.98 min (Method A); $^1H$ NMR (700 MHz, DMSO-$d_6$ + $D_2O$, 348 K) δ 3.85 - 3.83 (m, 4H), 2.44 - 2.38 (m, 1H), 2.03 (s, 3H), 1.89 - 1.83 (m, 2H), 1.68 - 1.58 (m, 4H), 1.54 - 1.48 (m, 2H).

Step 3.

Synthesis of 1,5-dimethyl-3-(1,4-dioxaspiro[4.5]decan-8-yl)-1*H*-pyrazole (**2c**).

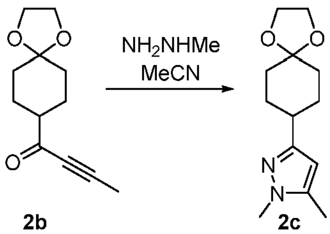


2b 2c

To the suspension of **2b** (208 mg; 1.00 mmol) in MeCN (2 mL) methylhydrazine (105 μL; 2.00 mmol) was added and the reaction mixture was stirred at room temperature for 1 hour. The reaction progress was monitored by LC-MS. When analysis indicated completion of the reaction, the whole was concentrated *in vacuo* and the crude product was used to the next step without additional purification. Compound **2c** was obtained in 99% yield (234 mg; 0.99 mmol).
ESI-MS m/z for $C_{13}H_{21}N_2O_2$ found 237.0 $[M+H]^+$; Rt = 0.81 min (Method A); $^1H$ NMR (700 MHz, DMSO-$d_6$ + $D_2O$, 348 K) δ 5.79 (s, 1H), 3.87 (s, 4H), 3.63 (s, 3H), 2.57 - 2.51 (m, 1H), 2.19 (s, 3H), 1.87 - 1.80 (m, 2H), 1.75 - 1.69 (m, 2H), 1.68 - 1.60 (m, 2H), 1.58 - 1.51 (m, 2H).

Step 4.

Synthesis of 4-(1,5-dimethyl-1*H*-pyrazol-3-yl)cyclohexan-1-one (**2d**).

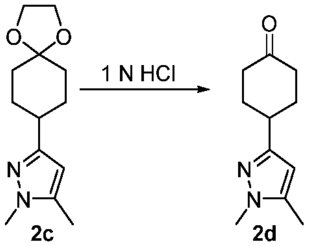


2c 2d

The solution of **2c** (234 mg; 0.99 mmol) in 1 N HCl (10 mL) was stirred at room temperature for 2 hours. The reaction progress was monitored by LC-MS. When analysis indicated completion of the reaction, to this mixture aqueous solution of NaOH (4 M) was added to pH 13 and then extracted with DCM (2 x). The combined organic solutions were dried

over anhydrous $MgSO_4$, filtered and concentrated *in vacuo.* The crude product was used to the next step without additional purification. Compound **2d** was obtained in 99% yield (188 mg; 0.98 mmol).
ESI-MS m/z for $C_{11}H_{17}N_2O$ found 193.1 $[M+H]^+$; Rt = 0.71 min (Method A); $^1H$ NMR (700 MHz, DMSO-$d_6$ + $D_2O$) δ 5.89 (s, 1H), 3.60 (s, 3H), 3.00 - 2.93 (m, 1H), 2.50 - 2.44 (m, 2H), 2.27 - 2.21 (m, 2H), 2.16 (s, 3H), 2.14 - 2.08 (m, 2H), 1.79 - 1.70 (m, 2H).

Step 5.

Synthesis of (2*S*,5*S*)-5-(4-chlorobenzyl)-4-(4-(1,5-dimethyl-1*H*-pyrazol-3-yl)cyclohexyl)-2-methylmorpholine 2,2,2-trifluoroacetate (**2**).

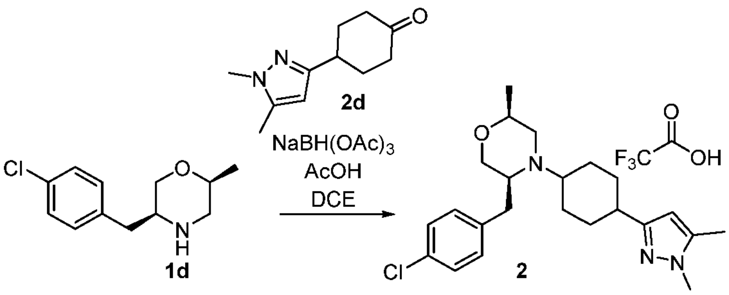


The title compound (**2**) was obtained as a single isomer as a TFA salt from **1d** (228 mg; 1.02 mmol) and from ketone **2d** (177 mg; 0.92 mmol) according to the General Procedure **VII** in 10% yield (47 mg; 0.09 mmol). The crude product was purified by flash column chromatography on silica (hexane/AcOEt, 2:1, *v*/*v*, then AcOEt, 100%, then: AcOEt/MeOH, 20:1, v/v) and then purified by preparative reversed-phase column chromatography (C-18, water/MeCN + 1‰ TFA, 90:10 to 10:90, 30 min, 20 mL/min).
ESI-MS m/z for $C_{23}H_{33}ClN_3O$ found 402.4/404.4 $[M+H]^+$; Rt = 0.94 min (Method A); $^1H$ NMR (700 MHz, DMSO-$d_6$ + $D_2O$, 348 K) δ 7.39 (d, $J_{AA'}$ = 8.5 Hz, 2H), 7.31 (d, $J_{BB'}$ = 8.5 Hz, 2H), 5.84 (s, 1H), 3.86 - 3.84 (m, 1H), 3.74 - 3.70 (m, 2H), 3.61 - 3.59 (m, 4H), 3.49 - 3.39 (m, 3H), 3.18 - 3.14 (m, 1H), 3.09 (dd, *J* = 13.8 Hz, *J* = 4.0 Hz, 1H), 3.04 - 3.00 (m, 1H), 2.31 - 2.28 (m, 1H), 2.26 - 2.23 (m, 1H), 2.17 (s, 3H), 2.06 - 2.02 (m, 2H), 1.55 - 1.48 (m, 2H), 1.47 - 1.40 (m, 2H), 1.22 (d, *J* = 6.2 Hz, 3H).

**Example 3.**

Synthesis of (2R,5S)-5-(4-chlorobenzyl)-4-(4-(5-methyloxazol-2-yl)cyclohexyl)-2-((methylsulfonyl)methyl)morpholine 2,2,2-trifluoroacetate (**3**).

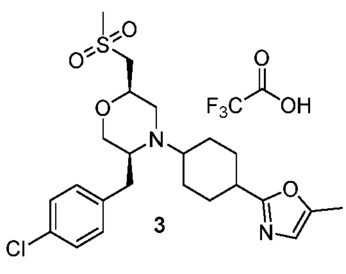

3

Step 1.

Synthesis of (*R*)-2-bromo-3-(*tert*-butoxy)-*N*-((*S*)-1-(4-chlorophenyl)-3-hydroxypropan-2-yl)propanamide (**3a**).

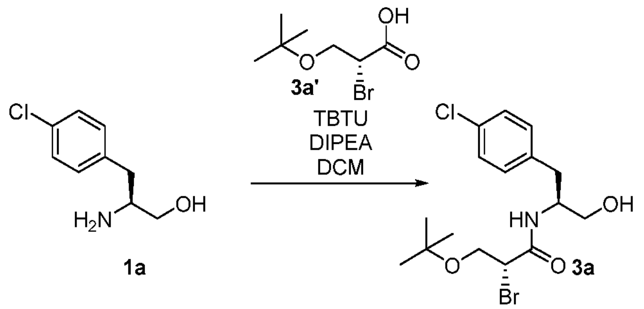


(2*S*)-2-amino-3-(4-chlorophenyl)propan-1-ol (**1a**) (1.89 g; 10.2 mmol) was coupled with (*R*)-2-bromo-3-(*tert*-butoxy)propanoic acid (**3a'**) (2.28 g; 10.2 mmol) according to the General Procedure **III** using TBTU as an amide bond forming reagent. The title compound **3a** was obtained in 70% yield (2.96 g; 7.14 mmol).
ESI-MS m/z for $C_{16}H_{23}BrClNO_3Na$ found 414.3/416.3 $[M+Na]^+$; $R_t$ = 1.43 min (Method A) Step 2.

Synthesis of (*R*)-2-bromo-3-(*tert*-butoxy)propanoic acid (**3a'**).

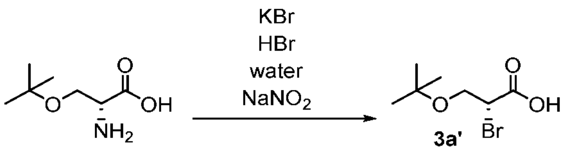


To a solution of *O*-(*tert*-butyl)-*D*-serine (26.8 g; 166 mmol) and KBr (69.2 g; 582 mmol) in water (134 mL) HBr (48%; 45 mL) was added at room temperature. Then the mixture was cooled to -10 °C and a solution of $NaNO_2$ (13.8 g; 200 mmol) in water (20 mL) was added by syringe pump over the period of 2 hours keeping the temperature below -5 °C. Then the mixture was stirred for 2 hours at 0 °C. The yellow foam appeared. To this mixture, a 10% solution of $Na_2SO_3$ in water (50 mL) was added (to remove an excess of bromine). The phases were separated and an aqueous one was extracted with MTBE (4 x 250 mL). The combined organic phases were dried over anhydrous $Na_2SO_4$, filtered, and concentrated *in vacuo* and the crude bromoacid was then dissolved in a solution of 1 M NaOH (340 mL) and extracted with MTBE (100 mL). To the aqueous phase, a solution of 1 M HCl (340 mL) was added (the white precipitate appeared) and the whole was transferred to the separatory funnel and extracted with MTBE (4 x 500 mL). The combined organic phases were dried over anhydrous $Na_2SO_4$, filtered, and concentrated *in vacuo* and the obtained product was used to the next step without additional purification. Compound **3a'** was obtained in 95% yield (35.6 g; 158 mmol).
ESI-MS m/z for $C_7H_{12}BrO_3$ found 223.0/225.0 $[M-H]^-$; Rt = 1.01 min (Method A)

Step 3.

Synthesis of (2*S*,5*S*)-2-(*tert*-butoxymethyl)-5-(4-chlorobenzyl)morpholin-3-one (**3b**).

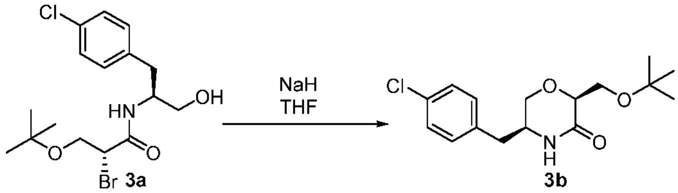


The title compound (**3b**) was obtained from **3a** (1.2 g, 3.05 mmol) according to the General Procedure **II** in 89% yield (0.85 g; 2.71 mmol).
ESI-MS m/z for $C_{16}H_{22}ClNO_3Na$ found 334.1/336.1 $[M+Na]^+$; Rt = 1.39 min (Method A)

Step 4.

Synthesis of ((2*R*,5*S*)-5-(4-chlorobenzyl)morpholin-2-yl)methanol (**3c**).

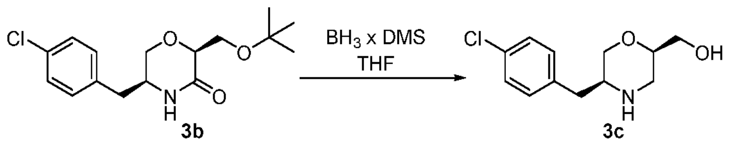


The title compound (**3c**) was obtained from **3b** (0.85 g; 2.71 mmol) according to the General Procedure **Ib** in 74% yield (0.48 g; 2.01 mmol).
ESI-MS m/z for $C_{12}H_{17}ClNO_2$ found 242.2/244.2 $[M+H]^+$; Rt = 0.93 min (Method B)

Step 5.

Synthesis of *tert*-butyl (2*R*,5*S*)-5-(4-chlorobenzyl)-2-(hydroxymethyl)morpholine-4-carboxylate (**3d**).

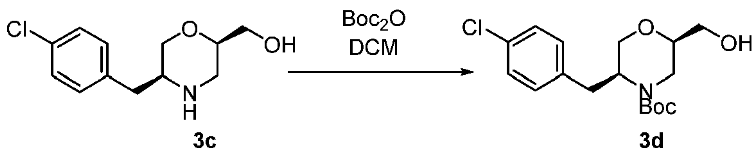


To a solution of amino alcohol **3c** (2.87 g; 11.9 mmol) in dichloromethane (110 mL), di-*tert*-butyl dicarbonate ($Boc_2O$) (2.46 g; 11.3 mmol) was added and the reaction mixture was stirred at room temperature for 2 hours, after which time TLC showed almost complete consumption of the starting material. Volatiles were removed *in vacuo* and the residue was purified by column chromatography (hexane/AcOEt, 1:1 *v*/*v*). Compound **3d** was obtained as a colorless oil in 77% yield (3.14 g; 9.16 mmol).
ESI-MS m/z for $C_{12}H_{17}ClNO_2$ found 242.1/246.1 $[M+H\text{-}Boc]^+$; Rt = 1.41 min (Method A)

Step 6.

Synthesis of *tert*-butyl (2*R*,5*S*)-5-(4-chlorobenzyl)-2-(((methylsulfonyl)oxy)methyl)morpholine-4-carboxylate (**3e**).

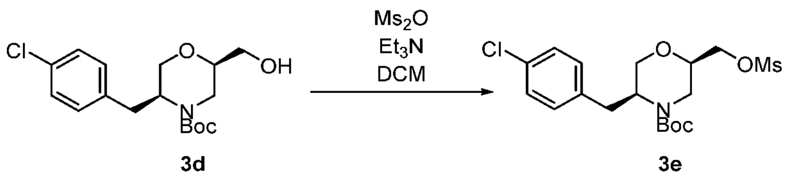


The title compound (**3e**) was obtained from **3d** (16.9 g; 49.44 mmol) according to the General Procedure **VIII** in 99% yield (20.52 g; 48.95 mmol).
ESI-MS m/z for $C_{23}H_{31}ClN_3O_4S$ found 420.1/422.1 $[M+H]^+$; Rt = 1.60 min (Method A)

Step 7.

Synthesis of (2*R*,5*S*)-*tert*-butyl 5-(4-chlorobenzyl)-2-((methylthio)methyl)morpholine-4-carboxylate (**3f**).

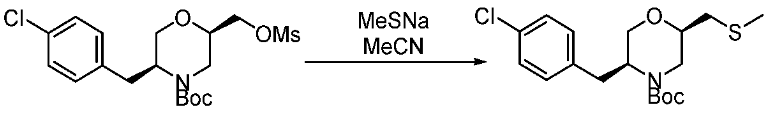


3e 3f

To a solution of **3e** (1.3 g; 3.09 mmol) in acetonitrile (15 mL) MeSNa (550 mg; 7.74 mmol) was added and the reaction mixture was stirred at 80 °C overnight. The reaction progress was monitored by LC-MS analysis. After analytical control indicated completion of the reaction, the mixture was dissolved in DCM and then washed with 2 M NaOH and brine, dried over anhydrous $Na_2SO_4$, filtered and concentrated *in vacuo* and the crude product was used to the next step without additional purification. Compound **3f** was obtained in 79% yield (0.9 g; 2.43 mmol).
ESI-MS m/z for $C_{18}H_{27}ClNO_3S$ found 372.1/374.1 $[M+H]^+$; Rt = 1.89 min (Method A)

Step 8.

Synthesis of (2*R*,5*S*)-*tert*-butyl 5-(4-chlorobenzyl)-2-((methylsulfonyl)methyl)morpholine-4-carboxylate (**3g**).

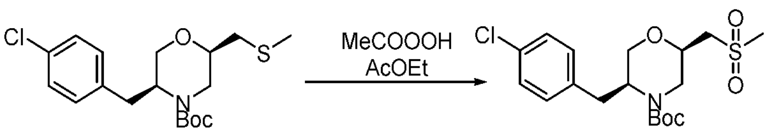


3f 3g

The reaction of **3f** (0.32 g; 0.86 mmol) with peracetic acid (35% in AcOH; 0.33 mL; 1.72 mmol) was stirred at room temperature overnight. Then the reaction was concentrated *in vacuo* and the residue was transferred to AcOEt/1 M NaOH. Phases were separated and the organic one was washed with brine, dried over anhydrous $Na_2SO_4$, filtered and concentrated *in vacuo* and the crude product was used to the next step without additional purification. Compound **3g** was obtained in 99% yield (0.34 g; 0.85 mmol).
ESI-MS m/z for $C_{18}H_{27}ClNO_5S$ found 404.1/406.1 $[M+H]^+$; Rt = 1.55 min (Method A); $^1H$ NMR (700 MHz, DMSO-$d_6$ + $D_2O$, 348 K) δ 7.33 - 7.26 (m, 2H), 7.24 - 7.18 (m, 2H), 4.11 - 4.05 (m, 1H), 3.87 - 3.82 (m, 1H), 3.81 - 3.77 (m, 1H), 3.77 - 3.73 (m, 1H), 3.61 - 3.54 (m, 1H), 3.34 - 3.29 (m, 1H), 3.06 - 2.94 (m, 5H), 2.86 - 2.79 (m, 1H), 1.98 - 1.95 (m, 1H), 1.27 - 1.14 (m, 9H).

Step 9.

Synthesis of (2*R*,5*S*)-5-(4-chlorobenzyl)-2-((methylsulfonyl)methyl)morpholine hydrochloride (**3h**).

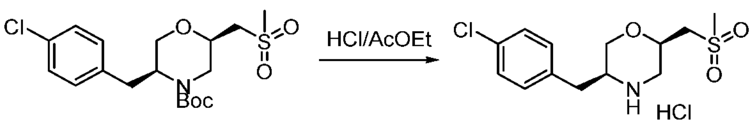


3g 3h

The title compound (**3h**) was obtained as a hydrochloride salt from **3g** (16.5 g; 40.84 mmol) according to the General Procedure **IVa** in 99% yield (13.73 g; 40.43 mmol).
ESI-MS m/z for $C_{13}H_{18}ClNO_3S$ found 303.8/305.8 $[M+H]^+$; Rt = 0.51 min (Method A)

Step 10.

Synthesis of (2*R*,5*S*)-5-(4-chlorobenzyl)-4-(4-(5-methyloxazol-2-yl)cyclohexyl)-2-((methylsulfonyl)methyl)morpholine 2,2,2-trifluoroacetate (**3**).

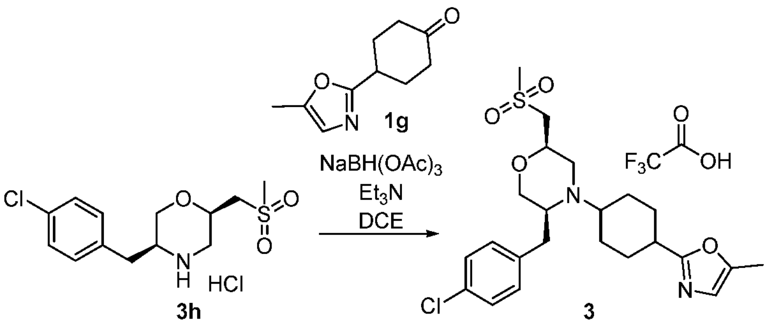


The title compound (**3**) was obtained as a single isomer as a TFA salt from **3h** (170 mg; 0.50 mmol) and ketone **1g** (104 mg; 0.58 mmol) according to the General Procedure **VII** in 24% yield (72 mg; 0.12 mmol). The crude product was purified by flash column chromatography on silica (hexane/AcOEt, 5:1 to 2:1, v/v, then AcOEt, 100%, then: AcOEt/MeOH, 120:1, v/v) and then purified by preparative reversed-phase column chromatography (C-18, water/MeCN + 1‰ TFA, 90:10 to 10:90, 30 min, 20 mL/min).
ESI-MS m/z for $C_{23}H_{32}ClN_2O_4S$ found 467.3/469.3 $[M+H]^+$; Rt = 0.95 min (Method A); $^1H$ NMR (700 MHz, DMSO-$d_6$ + $D_2O$, 348 K) δ 7.38 (d, $J_{AA'}$ = 8.5 Hz, 2H), 7.31 (d, $J_{BB'}$ = 8.5 Hz, 2H), 6.65 (q, *J* = 1.2 Hz, 1H), 4.26 - 4.23 (m, 1H), 3.76 - 3.73 (m, 2H), 3.67 - 3.65 (m, 1H), 3.58 (dd, *J* = 15.0 Hz, *J* = 7.9 Hz, 1H), 3.53 - 3.51 (m, 1H), 3.48 - 3.46 (m, 1H), 3.40 - 3.37 (m, 1H), 3.27 - 3.24 (m, 1H), 3.17 (dd, *J* = 13.8 Hz, *J* = 10.9 Hz, 1H), 3.09 (dd, *J* = 13.6 Hz, *J* = 4.0 Hz, 1H), 3.02 (s, 3H), 2.73 (tt, *J* = 11.9 Hz, *J* = 3.5 Hz, 1H), 2.27 - 2.25 (m, 2H), 2.22 (d, *J* = 1.2 Hz, 3H), 2.18 - 2.15 (m, 2H), 1.60 - 1.49 (m, 4H).

**Example 4.**

Synthesis of (2*R*,5*S*)-5-(4-chlorobenzyl)-4-((1*r*,4*S*)-4-(1,5-dimethyl-1*H*-pyrazol-3-yl)cyclohexyl)-2-((methylsulfonyl)methyl)morpholine dihydrochloride (**4**).

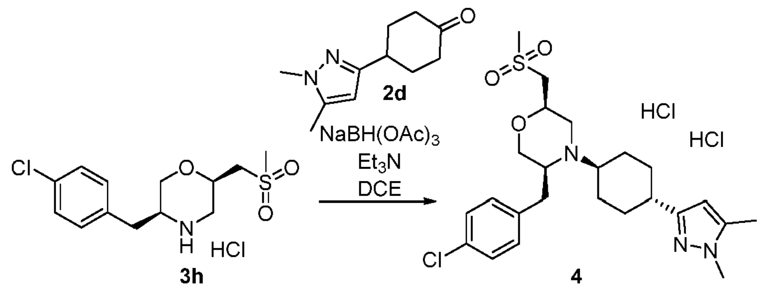


The title compound (**4**) was obtained as a single isomer as a dihydrochloride salt from **3h** (168 mg; 0.49 mmol) and ketone **2d** (90 mg; 0.47 mmol) according to the General Procedure **VII** in 17% yield (44 mg; 0.08 mmol). The crude product was purified by flash column chromatography on silica (hexane/AcOEt, 5:1, *v*/*v*, then AcOEt, 100%, then: AcOEt/MeOH, 20:1, v/v) and then transformed into dihydrochloride salt with using HCl/AcOEt. The obtained solid of the dihydrochloride salt was then lyophilized.
ESI-MS m/z for $C_{24}H_{35}ClN_3O_3S$ found 480.4/482.4 $[M+H]^+$; Rt = 1.00 min (Method A); $^1H$ NMR (400 MHz, $D_2O$) δ 7.30 - 7.25 (m, 2H), 7.20 - 7.14 (m, 2H), 6.19 (s, 1H), 4.32 - 4.19 (m, 1H), 3.85 - 3.70 (m, 5H), 3.70 - 3.54 (m, 3H), 3.51 - 3.35 (m, 2H), 3.33 - 3.23 (m, 1H), 3.19 - 3.00 (m, 5H), 2.76 - 2.63 (m, 1H), 2.38 - 2.27 (m, 2H), 2.21 (s, 3H), 2.13 - 2.05 (m, 2H), 1.61 - 1.37 (m, 4H).

**Example 5.**

Synthesis of (2*R*,5*S*)-5-(4-chlorobenzyl)-4-(4-(1,5-dimethyl-1*H*-pyrazol-3-yl)cyclohexyl)-2-(3-methylisoxazol-5-yl)morpholine 2,2,2-trifluoroacetate (**5**).

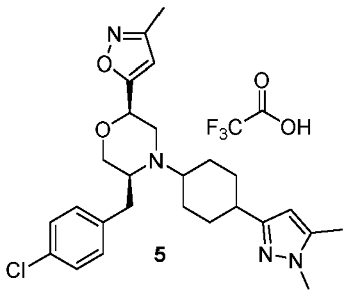

5

Step 1.

Synthesis of (2*R*,5*S*)-4-(*tert*-butoxycarbonyl)-5-(4-chlorobenzyl)morpholine-2-carboxylic acid (**5a**).

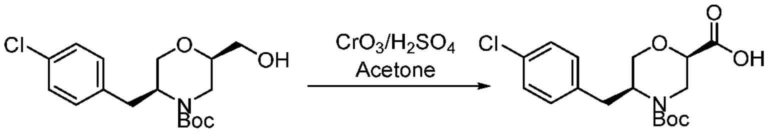


3d 5a

To a solution of alcohol **3d** (1.8 g; 5.26 mmol) in acetone (40 mL) at 0 °C, Jones reagent (12 mL; 2.6 M) was added dropwise. The reaction mixture was stirred at 0 °C for 1 hour, and then isopropanol (iPrOH) (5 mL) was added. After 10 minutes ethyl acetate (150 mL) was added and the mixture was filtered through a pad of Celite. The filtrate was washed with brine, dried over anhydrous $MgSO_4$ and evaporated affording the title compound **5a** as a white foam in 91% yield (1.7 g; 4.79 mmol).

ESI-MS m/z for $C_{17}H_{22}ClNO_5Na$ found 378.3/380.3 $[M+Na]^+$, 256.1/258.1 $[M+H-Boc]^+$; Rt = 1.45 min (Method A)

Step 2.

Synthesis of (2*R*,5*S*)-*tert*-butyl 5-(4-chlorobenzyl)-2-(methoxy(methyl)carbamoyl)morpholine-4-carboxylate (**5b**).

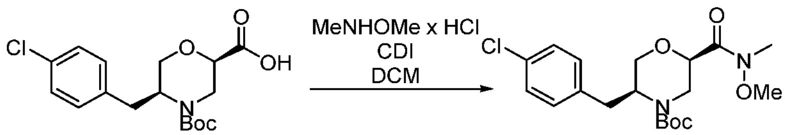


5a 5b

The title compound (**5b**) was obtained as a white crystal from **5a** (0.5 g; 1.4 mmol) according to the General Procedure **IX** in 67% yield (0.37 g; 0.94 mmol).

ESI-MS m/z for $C_{14}H_{20}ClN_2O_3$ found 298.9/300.9 $[M+H-Boc]^+$; Rt = 1.51 min (Method A); $^1H$ NMR (700 MHz, DMSO-$d_6$ + $D_2O$, 348 K) δ 7.33 - 7.25 (m, 2H), 7.25 - 7.15 (m, 2H), 4.26 - 4.21 (m, 1H), 4.13 - 4.04 (m, 1H), 3.91 - 3.83 (m, 1H), 3.78 - 3.74 (m, 1H), 3.69 (s, 3H), 3.62 - 3.57 (m, 1H), 3.18 - 3.10 (m, 4H), 3.01 - 2.91 (m, 1H), 2.88 - 2.81 (m, 1H), 1.21 (s, 9H).

Step 3.

Synthesis of (2*R*,5*S*)-tert-butyl 5-(4-chlorobenzyl)-2-(3-methylisoxazol-5-yl)morpholine-4-carboxylate (**5c**).

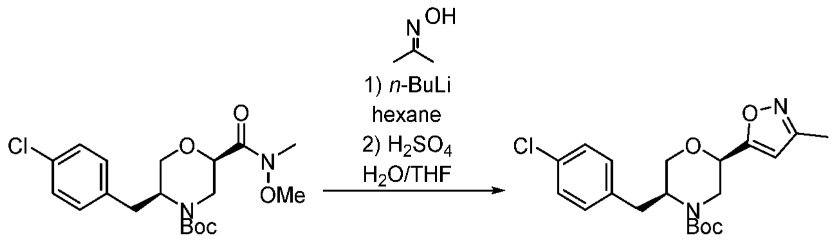


5b 5c

To a cooled to 0 °C solution of an oxime (55 mg; 074 mmol) in THF (1.5 mL) a solution of *n*-butyllithium (2.5 M in hexanes; 0.6 mL; 1.49 mmol) was added dropwise and the mixture was stirred for 30 minutes. Then the solution of **5b** (246 mg; 0.62 mmol) in THF (3 mL) was added dropwise over 15 minutes. After 30 minutes to this mixture a solution of $H_2SO_4$ (0.13 mL) in THF/water (1.5 mL/0.4 mL) was added and the resulting mixture was refluxed for 1 hour. The reaction progress was monitored by LC-MS. When analysis indicated completion of the reaction, the reaction mixture was cooled to 0 °C and neutralized with 5% $NaHCO_3$. Then water was added and the mixture was extracted with $Et_2O$. An organic layer was then dried over anhydrous $MgSO_4$, filtered and concentrated *in vacuo* and the crude product was used to the next step without additional purification. Compound **5c** was obtained in 97% yield (235 mg; 0.6 mmol).
ESI-MS m/z for $C_{20}H_{26}ClN_2O_4$ found 393.2/395.2 $[M+H]^+$; $R_t$ = 1.47 min (Method A)

Step 4.

Synthesis of (2*R*,5*S*)-5-(4-chlorobenzyl)-2-(3-methylisoxazol-5-yl)morpholine hydrochloride (**5d**).

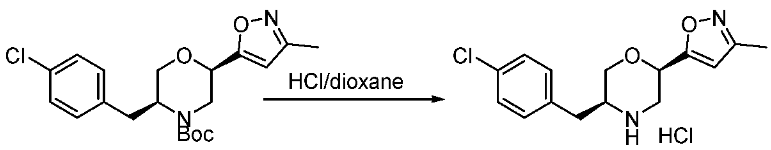


5c 5d

The title compound (**5d**) was obtained as a hydrochloride salt from **5c** (235 mg; 0.6 mmol) according to the General Procedure **IVa** in 99% yield (194 mg; 0.59 mmol).
ESI-MS m/z for $C_{12}H_{16}ClN_4O$ found 293.1/295.1 $[M+H]^+$; Rt = 0.76 min (Method A)

Step 5.

Synthesis of (2*R*,5*S*)-5-(4-chlorobenzyl)-4-(4-(1,5-dimethyl-1*H*-pyrazol-3-yl)cyclohexyl)-2-(3-methylisoxazol-5-yl)morpholine 2,2,2-trifluoroacetate (**5**).

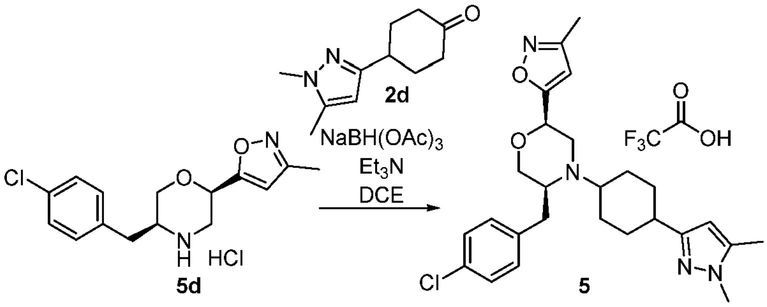


5d 5

The title compound (**5**) was obtained as a single isomer as a TFA salt from **5d** (181 mg; 0.550 mmol) and ketone **2d** (100 mg; 0.520 mmol) according to the General Procedure **VII** in 10% yield (30 mg; 0.055 mmol). The crude product was purified by flash column chromatography on silica (hexane/AcOEt, 10:1, *v*/*v*, then AcOEt, 100%) and then transformed into

TFA salt with using TFA/DCM and then purified by preparative reversed-phase column chromatography (C-18, water/-MeCN + 1‰ TFA, 90:10 to 20:80, 30 min, 20 mL/min).
ESI-MS m/z for $C_{26}H_{34}ClN_4O_2$ found 469.3/471.3 [M+H]$^+$; Rt = 1.10 min (Method A); $^1$H NMR (700 MHz, DMSO-$d_6$ + $D_2O$, 348 K) δ 7.38 (d, $J_{AA'}$ = 8.5 Hz, 2H), 7.31 (d, $J_{BB'}$ = 8.5 Hz, 2H), 6.53 (s, 1H), 5.81 (s, 1H), 5.05 - 5.00 (m, 1H), 3.87 - 3.86 (m, 1H), 3.80 - 3.78 (m, 1H), 3.73 - 3.71 (m, 1H), 3.68 - 3.66 (m, 1H), 3.60 (s, 3H), 3.53 - 3.49 (m, 1H), 3.45 - 3.43 (m, 1H), 3.16 - 3.14 (m, 2H), 2.49 - 2.45 (m, 1H), 2.27 - 2.22 (m, 5H), 2.17 (s, 3H), 2.05 - 2.02 (m, 2H), 1.56 - 1.50 (m, 2H), 1.46 - 1.40 (m, 2H).

**Example 6.**

Synthesis of (7*S*,9*aR*)-7-(4-chlorobenzyl)-8-(4-(5-methyloxazol-2-yl)cyclohexyl)octahydropyrazino[2,1-*c*][1,4]oxazine 2,2,2-trifluoroacetate (**6**).

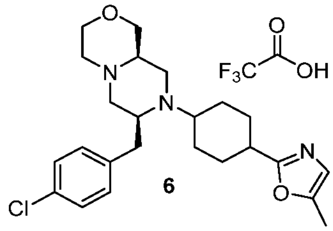

Step 1.

Synthesis of methyl (*S*)-4-((*R*)-2-((*tert*-butoxycarbonyl)amino)-3-(4-chlorophenyl)propanoyl)-morpholine-3-carboxylate (**6a**).

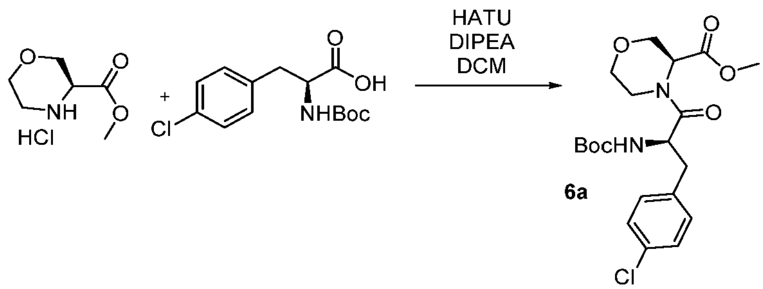


The title compound (**6a**) was obtained from methyl (*S*)-morpholine-3-carboxylate hydrochloride (1.6 g; 8.81 mmol) and Boc-4-chloro-*L*-phenylalanine (2.64 g; 8.81 mmol) according to the General Procedure **III** in 77% yield (2.9 g; 6.81 mmol).
ESI-MS m/z for $C_{20}H_{28}ClN_2O_6$ found 427.2/429.2 [M+H]$^+$; $R_t$ = 1.46 min (Method A); $^1$H NMR (250 MHz, $CDCl_3$) δ 7.29 - 7.22 (m, 2H), 7.20 - 7.02 (m, 2H), 5.32 - 5.22 (m, 1H), 5.12 - 5.07 (m, 1H), 4.94 - 4.83 (m, 1H), 4.49 - 4.37 (m, 1H), 4.25 - 3.99 (m, 1H), 3.90 - 3.81 (m, 1H), 3.81 - 3.71 (m, 3H), 3.72 - 3.48 (m, 2H), 3.20 - 3.06 (m, 1H), 2.93 - 2.77 (m, 1H), 1.39 (s, 9H).

Step 2.

Synthesis of methyl (*S*)-4-((*R*)-2-amino-3-(4-chlorophenyl)propanoyl)morpholine-3-carboxylate hydrochloride (**6b**).

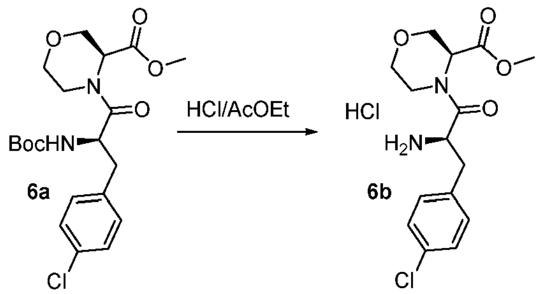


The title compound (**6b**) was obtained as a hydrochloride salt from **6a** (2.9 g; 6.81 mmol) according to the General Procedure **IVa** in 99% yield (2.44 g; 6.74 mmol).
ESI-MS m/z for $C_{15}H_{20}ClN_2O_4$ found 326.8/328.8 $[M+H]^+$; $R_t$ = 0.56 min (Method A)

Step 3.

Synthesis of (7*S*,9a*S*)-7-(4-chlorobenzyl)hexahydropyrazino[2,1-*c*][1,4]oxazine-6,9-dione (**6c**).

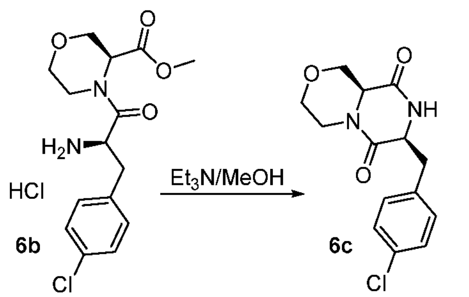


To a solution of crude **6b** (2.44 g; 6.74 mmol) in MeOH (70 mL) $Et_3N$ (4.8 mL; 33.96 mmol) was added and the mixture was stirred at room temperature for 2 hours. LC-MS showed completion of the reaction. The mixture was concentrated *in vacuo* and the yellow oily residue was purified by flash column chromatography on silica (DCM/MeOH, 100:1 to 10:1, *v*/*v*). Compound **6c** was obtained as a light yellow foam in 59% yield (1.18 g; 4.01 mmol).
ESI-MS m/z for $C_{14}H_{16}ClN_2O_3$ found 295.1/297.1 $[M+H]^+$; Rt = 0.84 min (Method A)

Step 4.

Synthesis of (7*S*,9a*R*)-7-(4-chlorobenzyl)octahydropyrazino[2,1-*c*][1,4]oxazine (**6d**).

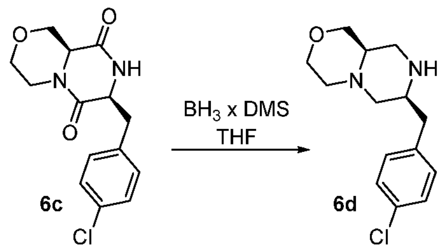


The title compound (**6d**) was obtained as a white solid from **6c** (1.1 g; 3.73 mmol) according to the General Procedure **Ib** in 96% yield (950 mg; 3.57 mmol).
ESI-MS m/z for $C_{14}H_{20}ClN_2O$ found 267.1/269.1 $[M+H]^+$; $R_t$ = 1.25 min (Method E)

Step 5.

Synthesis of (7*S*,9*aR*)-7-(4-chlorobenzyl)-8-(4-(5-methyloxazol-2-yl)cyclohexyl)octahydro-pyrazino[2,1-*c*][1,4]oxazine 2,2,2-trifluoroacetate (**6**).

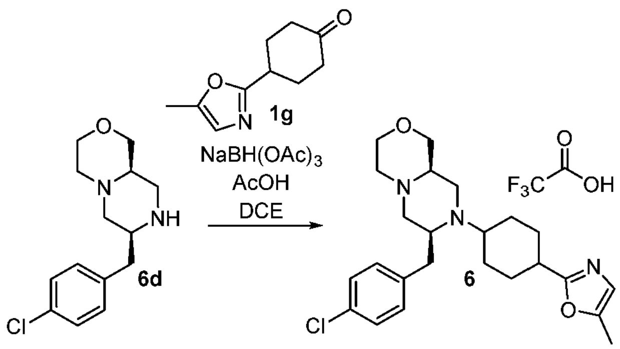


The title compound (**6**) was obtained as a single isomer as a TFA salt from **6d** (150 mg; 0.560 mmol) and ketone **1g** (100 mg; 0.560 mmol) according to the General Procedure **VII** in 14% yield (42 mg; 0.078 mmol). The crude product was purified by flash column chromatography on silica (hexane/AcOEt, 5:1, *v*/*v*, then AcOEt, 100%, then: AcOEt/MeOH, 5:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 95:5 to 20:80, 30 min, 20 mL/min). ESI-MS m/z for $C_{24}H_{33}ClN_3O_2$ found 430.3/432.3 $[M+H]^+$; Rt = 0.98 min (Method A); $^1H$ NMR (700 MHz, DMSO-$d_6$ + $D_2O$) δ 7.38 - 7.37 (m, 2H), 7.31 - 7.30 (m, 2H), 6.67 (q, *J* = 1.2 Hz, 1H), 3.81 - 3.80 (m, 1H), 3.78 - 3.76 (m, 1H), 3.72 (dd, *J* = 11.1 Hz, *J* = 2.9 Hz, 1H), 3.59 - 3.56 (m, 1H), 3.42 - 3.38 (m, 2H), 3.30 - 3.27 (m, 1H), 3.21 (t, *J* = 10.5 Hz, 1H), 3.00 (dd, *J* = 13.2 Hz, *J* = 3.9 Hz, 1H), 2.93 - 2.89 (m, 1H), 2.71 (tt, *J* = 12.1 Hz, *J* = 3.4 Hz, 1H), 2.52 - 2.49 (m, 2H), 2.48 - 2.36 (m, 2H), 2.28 - 2.26 (m, 2H), 2.22 (d, *J* = 1.2 Hz, 3H), 2.20 - 2.17 (m, 1H), 2.14 - 2.12 (m, 2H), 1.58 - 1.43 (m, 4H).

**Example 7.**

Synthesis of (7*S*,9*aR*)-7-(4-chlorobenzyl)-8-(4-(1,5-dimethyl-1*H*-pyrazol-3-yl)cyclohexyl)octahydropyrazino[2,1-*c*][1,4] oxazine 2,2,2-trifluoroacetate (**7**).

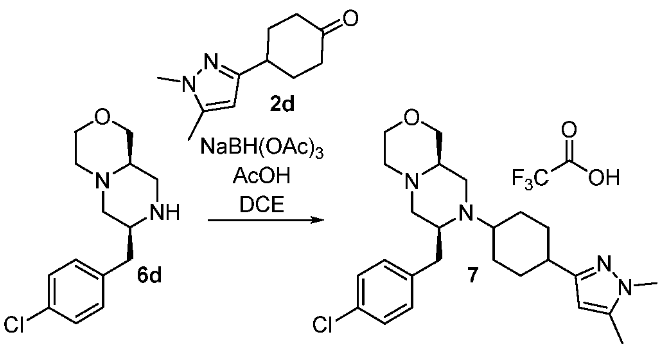


The title compound (**7**) was obtained as a single isomer as a TFA salt from **6d** (126 mg; 0.470 mmol) and ketone **2d** (100 mg; 0.520 mmol) according to the General Procedure **VII** in 29% yield (76 mg; 0.136 mmol). The crude product was purified by flash column chromatography on silica (hexane/AcOEt, 3:1, v/v, then AcOEt, 100%, then: AcOEt/MeOH, 10:1 to 5:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 95:5 to 20:80, 30 min, 20 mL/min).

ESI-MS m/z for $C_{25}H_{36}ClN_4O$ found 443.3/445.3 $[M+H]^+$; Rt = 1.02 min (Method A); $^1H$ NMR (700 MHz, DMSO-$d_6$ + $D_2O$, 348 K) δ 7.36 - 7.35 (m, 2H), 7.29 - 7.27 (m, 2H), 5.90 (s, 1H), 3.78 - 3.71 (m, 3H), 3.62 (s, 3H), 3.60 - 3.57 (m, 1H), 3.38 - 3.35 (m, 1H), 3.31 - 3.27 (m, 2H), 3.21 (t, *J* = 10.6 Hz, 1H), 3.00 (dd, *J* = 13.5 Hz, *J* = 3.8 Hz, 1H), 2.88 - 2.84 (m, 2H), 2.53 - 2.51 (m, 1H), 2.50 - 2.48 (m, 1H), 2.47 - 2.43 (m, 2H), 2.26 - 2.21 (m, 1H), 2.18 (s, 3H), 2.17 - 2.15 (m, 2H), 1.97 - 1.93 (m, 2H), 1.72 - 1.65 (m, 2H), 1.64 - 1.58 (m, 2H).

**Example 8.**

Synthesis of (7*S*,9a*R*)-7-(4-chlorobenzyl)-8-(4-(1,5-dimethyl-1*H*-pyrazol-3-yl)cyclohexyl)octahydropyrazino[2,1-*c*][1,4] oxazine 2,2,2-trifluoroacetate (**8**).

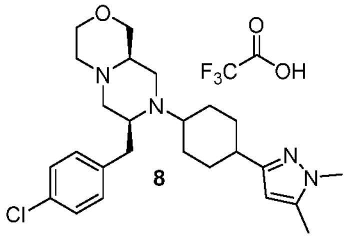

**8**

The title compound (**8**) was obtained during the synthesis of compound **7** as a second single isomer as a TFA salt from **6d** (126 mg; 0.47 mmol) and ketone **2d** (100 mg; 0.52 mmol) according to the General Procedure **VII** in 19% yield (52 mg; 0.09 mmol). The crude product was purified by flash column chromatography on silica (hexane/AcOEt, 3:1, *v*/*v*, then AcOEt, 100%, then: AcOEt/MeOH, 10:1 to 5:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 95:5 to 20:80, 30 min, 20 mL/min).

ESI-MS m/z for $C_{25}H_{36}ClN_4O$ found 443.3/445.3 $[M+H]^+$; Rt = 1.00 min (Method A); $^1H$ NMR (700 MHz, DMSO-$d_6$ + $D_2O$, 348 K) δ 7.37 - 7.36 (m, 2H), 7.31 - 7.30 (m, 2H), 5.81 (s, 1H), 3.82 - 3.77 (m, 2H), 3.74 (dd, *J* = 11.1 Hz, *J* = 3.0 Hz, 1H), 3.62 - 3.58 (m, 4H), 3.39 - 3.31 (m, 3H), 3.24 (t, *J* = 10.5 Hz, 1H), 3.03 (dd, *J* = 13.4 Hz, *J* = 4.2 Hz, 1H), 2.93 (t, *J* = 12.3 Hz, 1H), 2.55 - 2.51 (m, 3H), 2.48 - 2.43 (m, 2H), 2.27 - 2.22 (m, 3H), 2.17 (s, 3H), 2.04 - 2.02 (m, 2H), 1.53 - 1.39 (m, 4H).

**Example 9.**

Synthesis of (2*R*,5*S*)-5-(4-chlorobenzyl)-4-(4-(5-methyloxazol-2-yl)cyclohexyl)morpholine-2-carboxylic acid 2,2,2-trifluoroacetate (**9**).

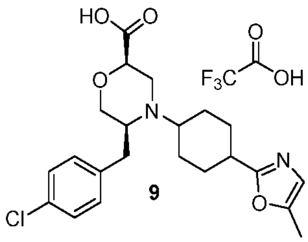

**9**

Step 1.

Synthesis of (2*R*,5*S*)-5-(4-chlorobenzyl)morpholine-2-carboxylic acid hydrochloride (**9a**).

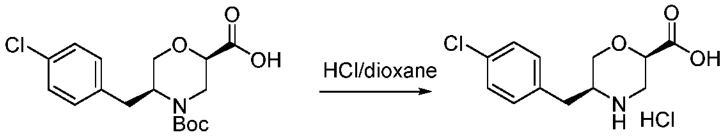


**5a** **9a**

The title compound (**9a**) was obtained as a hydrochloride salt from **5a** (5.16 g; 14.5 mmol) according to the General Procedure **IVa** in 93% yield (3.93 g; 13.49 mmol).

ESI-MS m/z for $C_{12}H_{15}ClNO_3$ found 256.1/258.1 $[M+H]^+$; Rt = 0.39 min (Method A)

Step 2.

Synthesis of methyl (2*R*,5*S*)-5-(4-chlorobenzyl)morpholine-2-carboxylate (**9b**).

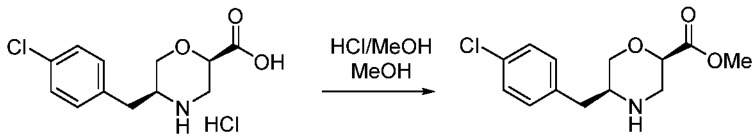


9a 9b

To a cooled to -12 °C (brine/$CO_{2(s)}$) solution of an amino acid **9a** (1.20 g; 4.10 mmol) in MeOH (25 mL) a solution of HCl/MeOH (3.2 M; 6.4 mL) was added dropwise and after 1 hour this reaction mixture was allowed to warm to room temperature and stirred overnight. After reaction was completed as judged by LC-MS, the solvent was evaporated. The residue was taken between 5% $NaHCO_3$ and DCM. An organic layer was dried over anhydrous $Na_2SO_4$, filtered and concentrated *in vacuo*. The crude product was purified by flash column chromatography on silica ($CHCl_3$/MeOH, 200:1 to 180:1, *v*/*v*). Compound **9b** was obtained in 66% yield (726 mg; 2.70 mmol).
ESI-MS m/z for $C_{13}H_{17}ClNO_3$ found 270.0/272.0 $[M+H]^+$; Rt = 0.58 min (Method A); $^1H$ NMR (700 MHz, DMSO-$d_6$ + $D_2O$, 348 K) δ 7.30 - 7.27 (m, 2H), 7.21 - 7.18 (m, 2H), 4.19 - 4.14 (m, 1H), 3.67 (s, 3H), 3.54 - 3.46 (m, 2H), 3.19 - 3.14 (m, 1H), 2.90 - 2.84 (m, 2H), 2.58 - 2.55 (m, 2H).

Step 3.

Synthesis of methyl (2*R*,5*S*)-5-(4-chlorobenzyl)-4-(4-(5-methyloxazol-2-yl)cyclohexyl)-morpholine-2-carboxylate (**9c** and **9c'**).

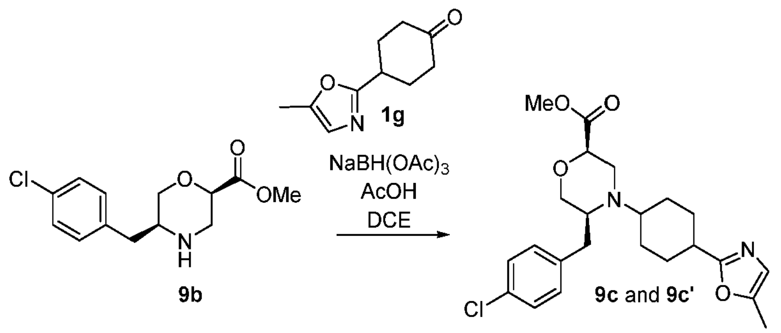


9b 9c and 9c'

The title compound (**9c** and **9c'**) was obtained as a mixture of two isomers from **9b** (150 mg; 0.560 mmol) and ketone **1g** (100 mg; 0.560 mmol) according to the General Procedure **VII** in 61% yield (148 mg; 0.340 mmol). The crude product was purified by flash column chromatography on silica (hexane/AcOEt, 4:1 to 1:2, *v*/*v*) to give two separated isomers **9c** in 30% yield (72 mg; 0.170 mmol) and **9c'** in 31% yield (76 mg; 0.176 mmol).

For **9c:** ESI-MS m/z for $C_{23}H_{30}ClN_2O_4$ found 433.0/435.0 $[M+H]^+$; Rt = 1.08 min (Method A)
For **9c':** ESI-MS m/z for $C_{23}H_{30}ClN_2O_4$ found 433.0/435.0 $[M+H]^+$; $R_t$ = 1.03 min (Method A)

Step 4.

Synthesis of (2*R*,5*S*)-5-(4-chlorobenzyl)-4-(4-(5-methyloxazol-2-yl)cyclohexyl)morpholine-2-carboxylic acid 2,2,2-trifluoroacetate (**9**).

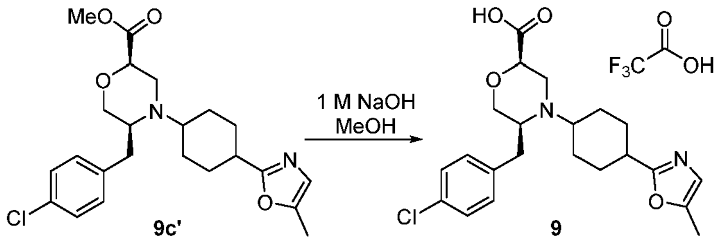

9c' 9

To the solution of **9c'** (76 mg; 0.176 mmol) in MeOH (10 mL) 1 M NaOH (2 mL) was added and whole was stirred at room temperature for 2 hours. The reaction progress was monitored by LC-MS. When analysis indicated completion of the reaction, the solvent was evaporated *in vacuo* and the residue was purified by preparative reversed-phase column chromatography (column: Cosmosil Cholester 20 x 250 mm, water/MeCN + 1‰ TFA, 95:5 to 20:80, 30 min, 20 mL/min). The title compound (**9**) was obtained as a single isomer as a TFA salt in 30% yield (28 mg; 0.053 mmol).
ESI-MS m/z for $C_{22}H_{28}ClN_2O_4$ found 419.2/421.2 $[M+H]^+$; Rt = 0.97 min (Method A); $^1H$ NMR (700 MHz, DMSO-$d_6$ + $D_2O$) δ 7.39 (d, $J_{AA'}$ = 8.4 Hz, 2H), 7.30 (d, $J_{BB'}$ = 8.4 Hz, 2H), 6.67 (q, $J$ = 1.1 Hz, 1H), 4.42 - 4.40 (m, 1H), 3.69 - 3.62 (m, 3H), 3.43 - 3.40 (m, 3H), 3.10 - 3.08 (m, 1H), 2.96 - 2.92 (m, 1H), 2.74 - 2.69 (m, 1H), 2.22 (d, $J$ = 1.2 Hz, 3H), 2.14 - 2.09 (m, 4H), 1.57 - 1.46 (m, 4H).

**Example 10.**

Synthesis of methyl (2*R*,5*S*)-5-(4-chlorobenzyl)-4-(4-(1,5-dimethyl-1*H*-pyrazol-3-yl)cyclohexyl)morpholine-2-carboxylate 2,2,2-trifluoroacetate (**10**).

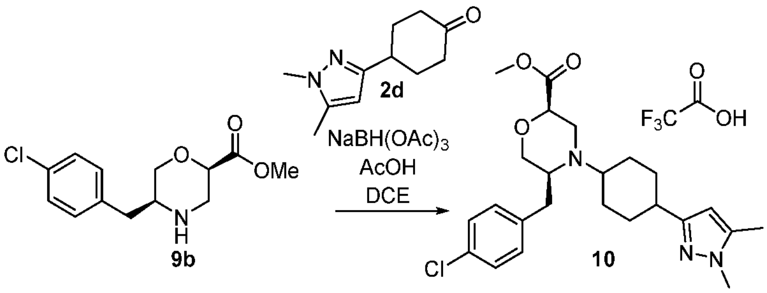

9b 10

The title compound (**10**) was obtained as a single isomer as a TFA salt from **9b** (207 mg; 0.77 mmol) and ketone **2d** (150 mg; 0.78 mmol) according to the General Procedure **VII** in 24% yield (101 mg; 0.18 mmol). The crude product was purified by flash column chromatography on silica (hexane/AcOEt, 2:1, v/v, then AcOEt, 100%, then: AcOEt/MeOH, 16:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 90:10 to 20:80, 30 min, 20 mL/min). ESI-MS m/z for $C_{24}H_{33}ClN_3O_3$ found 446.1/448.1 $[M+H]^+$; Rt = 1.01 min (Method A); $^1H$ NMR (700 MHz, DMSO-$d_6$ + $D_2O$, 348 K) δ 7.37 (d, $J_{AA'}$ = 8.5 Hz, 2H), 7.30 (d, $J_{BB'}$ = 8.5 Hz, 2H), 5.81 (s, 1H), 4.56 (dd, $J$ = 8.6 Hz, $J$ = 3.6 Hz, 1H), 3.76 - 3.70 (m, 6H), 3.60 (s, 3H), 3.55 - 3.53 (m, 1H), 3.50 - 3.44 (m, 2H), 3.13 (dd, $J$ = 14.0 Hz, $J$ = 4.5 Hz, 1H), 3.01 (dd, $J$ = 14.0 Hz, $J$ = 9.9 Hz, 1H), 2.50 - 2.45 (m, 1H), 2.15 - 2.11 (m, 5H), 2.05 - 1.99 (m, 2H), 1.62 - 1.50 (m, 2H), 1.44 (qd, $J$ = 13.2 Hz, $J$ = 3.3 Hz, 1H), 1.37 (qd, $J$ = 13.2 Hz, $J$ = 3.3 Hz, 1H).

**Example 11.**

Synthesis of (2*R*,5*S*)-5-(4-chlorobenzyl)-4-(4-(1,5-dimethyl-1*H*-pyrazol-3-yl)cyclohexyl)morpholine-2-carboxylic acid 2,2,2-trifluoroacetate (**11**).

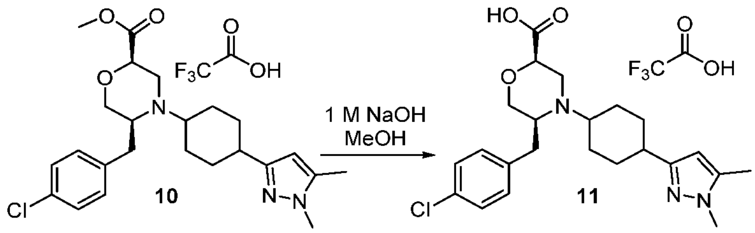


10

11

To the solution of **10** (95 mg; 0.17 mmol) in MeOH (10 mL) 1 M NaOH (3 mL) was added and whole was stirred at room temperature for 2 hours. The reaction progress was monitored by LC-MS. When analysis indicated completion of the reaction, the solvent was evaporated *in vacuo* and the residue was dissolved in water/MeCN with few drops of TFA and then purified by preparative reversed-phase column chromatography (column: Cosmosil Cholester 20 x 250 mm, water/MeCN + 1‰ TFA, 95:5 to 20:80, 30 min, 20 mL/min). The title compound (**11**) was obtained as a single isomer as a TFA salt in 53% yield (48 mg; 0.09 mmol).

ESI-MS m/z for $C_{23}H_{31}ClN_3O_3$ found 432.2/434.2 $[M+H]^+$; Rt = 0.98 min (Method A); $^1$H NMR (700 MHz, DMSO-$d_6$ + $D_2O$, 348 K) δ 7.38 - 7.36 (m, 2H), 7.31 - 7.30 (m, 2H), 5.80 (s, 1H), 4.46 - 4.44 (m, 1H), 3.75 - 3.72 (m, 3H), 3.59 (s, 3H), 3.53 - 3.50 (m, 1H), 3.46 - 3.43 (m, 2H), 3.13 - 3.11 (m, 1H), 3.02 (dd, *J* = 14.1 Hz, *J* = 9.6 Hz, 1H), 2.46 (tt, *J* = 12.2 Hz, *J* = 3.7 Hz, 1H), 2.18 - 2.12 (m, 5H), 2.05 - 1.98 (m, 2H), 1.61 - 1.50 (m, 2H), 1.43 (qd, *J* = 13.0 Hz, *J* = 3.3 Hz, 1H), 1.36 (qd, *J* = 13.0 Hz, *J* = 3.3 Hz, 1H).

**Example 12.**

Synthesis of (2*R*,5*S*)-5-(4-chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholine 2,2,2-trifluoroacetate (**12**).

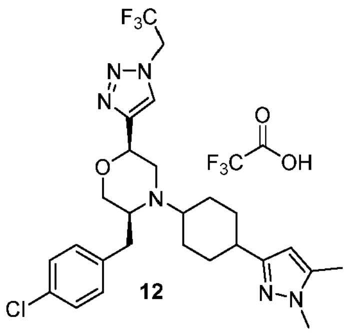

12

Step 1.

Synthesis of (2*R*,5*S*)-*tert*-butyl 5-(4-chlorobenzyl)-2-formylmorpholine-4-carboxylate (**12a**).

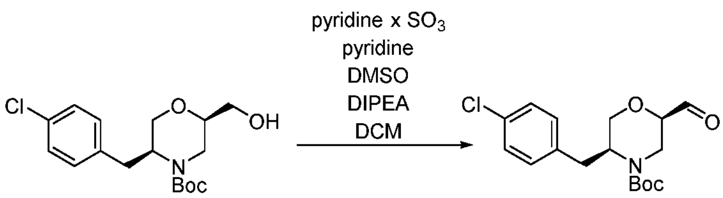


3d

12a

To a sulfur trioxide-pyridine complex (93 mg; 0.59 mmol), pyridine (46 mg; 0.59 mmol) and DMSO (0.11 mL; 1.45 mmol) were added. The resulting suspension was stirred for 15 minutes, then DCM (4 mL) was added. The reaction was cooled to 0 °C and solution of an aminoalcohol **3d** (0.1 g; 0.29 mmol), DIPEA (177 μL; 1 mmol) and DMSO (0.11 mL; 1.45 mmol) were added. The reaction was stirred at 0 °C for 2 hours and after this time it was allowed to warm up to room

temperature. The reaction progress was monitored by TLC and LC-MS analyses of small aliquots of the crude reaction mixture. When analyses indicated completion of the reaction, water (10 mL) was added. The organic phase was separated, washed with brine, dried over anhydrous $MgSO_4$ and concentrated *in vacuo.* After evaporation of solvent, the crude product **12a** (97 mg; 0.29 mmol) was obtained in 99% yield.
ESI-MS m/z for $C_{17}H_{23}ClNO_4$ found 340.1/342.1 $[M+H]^+$; Rt = 1.25 min (Method A)

Step 2.

Synthesis of *tert*-butyl (2*S*,5*S*)-5-(4-chlorobenzyl)-2-ethynylmorpholine-4-carboxylate (**12b**).

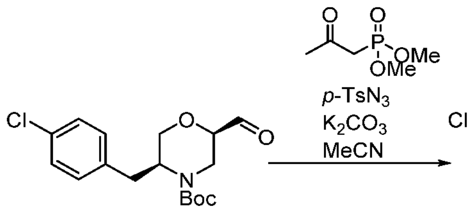


**12a** **12b**

To a suspension of $K_2CO_3$ (726 mg; 5.25 mmol), *p*-toluenesulfonyl azide (415 mg; 2.1 mmol) in MeCN (30 mL) dimethyl-2-oxopropylphosphonate (0.29 mL; 2.1 mmol) was added and this mixture was stirred at room temperature for 2 hours. Then the solution of **12a** (593 mg; 1.75 mmol) in MeOH (6 mL) was added and the resulting mixture was stirred at room temperature overnight. The reaction progress was monitored by LC-MS. When analysis indicated completion of the reaction, the reaction mixture was concentrated *in vacuo* and the residue was suspended in $Et_2O$. After 3 minutes in ultrasound bath an ether was collected by decantation. This manner (action) has been repeated two times. A solid residue was taken between DCM/water followed by an organic layer was concentrated *in vacuo.* The crude product was purified by flash column chromatography on silica (hexane/AcOEt; 30:1 to 5:1, *v*/*v*). Compound **12b** was obtained as a white powder in 63% yield (1.11 g; 3.31 mmol).
ESI-MS m/z for $C_{18}H_{23}ClNO_3$ found 336.1/338.1 $[M+H]^+$; Rt = 1.77 min (Method A); $^1H$ NMR (700 MHz, DMSO-$d_6$ + $D_2O$, 348 K) δ 7.31 - 7.27 (m, 2H), 7.21 - 7.18 (m, 2H), 4.16 - 4.11 (m, 1H), 4.08 - 4.03 (m, 1H), 3.90 - 3.84 (m, 1H), 3.68 (d, *J* = 11.8 Hz, 1H), 3.52 (dd, *J* = 11.8, 3.4 Hz, 1H), 3.36 (d, *J* = 2.2 Hz, 1H), 3.12 - 3.06 (m, 1H), 2.99 - 2.90 (m, 1H), 2.87 - 2.78 (m, 1H), 1.22 (s, 9H).

Step 3.

Synthesis of *tert*-butyl (2*R*,5*S*)-5-(4-chlorobenzyl)-2-(1-(2,2,2-trifluoroethyl)-1*H*-1,2,3-triazol-4-yl)morpholine-4-carboxylate (**12c**).

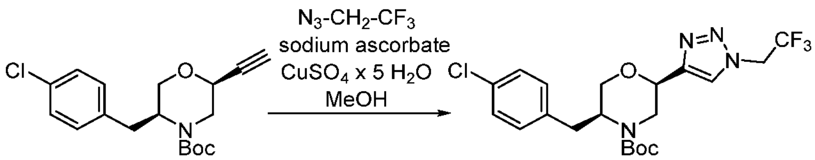


**12b** **12c**

To a solution of **12b** (370 mg; 1.10 mmol) in MeOH (4 mL) $CuSO_4$ x 5 $H_2O$ (137 mg; 0.55 mmol) was added and after 10 minutes sodium ascorbate (218 mg; 1.10 mmol) was added in one portion and the mixture was stirred at room temperature for 20 minutes. Then to this mixture 2-azido-1,1,1-trifluoroethane (2.2 mL; 1.10 mmol) was added and the mixture was stirred at room temperature for 2 hours. The reaction progress was monitored by LC-MS. When analysis indicated completion of the reaction, the green suspension was concentrated *in vacuo* and the residue was taken between water/DCM. An organic layer was washed with brine, dried over anhydrous $MgSO_4$, filtered and concentrated *in vacuo* and the crude product mixture was purified by flash column chromatography on silica ($CHCl_3$/MeOH, 100:1 to 50:1, *v*/*v*). Compound **12c** was obtained as a white foam in 88% yield (445 mg; 0.97 mmol).
ESI-MS m/z for $C_{20}H_{25}ClF_3N_4O_3$ found 461.0/463.0 $[M+H]^+$; Rt = 1.78 min (Method A); $^1H$ NMR (700 MHz, DMSO-$d_6$ +

$D_2O$, 348 K) δ 8.26 (s, 1H), 7.31 - 7.28 (m, 2H), 7.24 - 7.21 (m, 2H), 5.43 - 5.35 (m, 2H), 4.62 - 4.57 (m, 1H), 4.18 - 4.12 (m, 1H), 4.04 - 3.95 (m, 1H), 3.83 - 3.78 (m, 1H), 3.73 - 3.65 (m, 1H), 3.36 - 3.26 (m, 1H), 3.08 - 3.00 (m, 1H), 2.93 - 2.85 (m, 1H), 1.23 (s, 9H); $^{19}F$ NMR (235 MHz, DMSO-$d_6$) δ -70.06 - -70.82 (m).

Step 4.

Synthesis of (2*R*,5*S*)-5-(4-chlorobenzyl)-2-(1-(2,2,2-trifluoroethyl)-1*H*-1,2,3-triazol-4-yl)morpholine (**12d**).

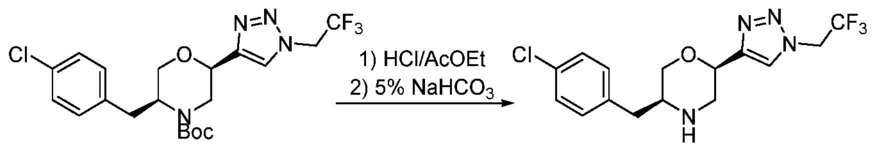


**12c** **12d**

The title compound (**12d**) was obtained as a free amine in 91% yield (317 mg; 0.88 mmol) from **12c** (445 mg; 0.97 mmol) according to the General Procedure **IVa** and then after basic (5% $NaHCO_{3aq}$) extraction with DCM. ESI-MS m/z for $C_{15}H_{17}ClF_3N_4O$ found 360.9/362.9 $[M+H]^+$; Rt = 0.89 min (Method A)

Step 5.

Synthesis of (2*R*,5*S*)-5-(4-chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholine 2,2,2-trifluoroacetate (**12**).

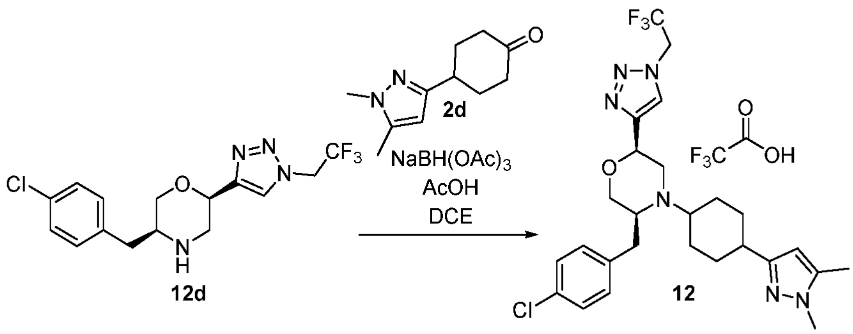


**12d** **12**

The title compound (**12**) was obtained as a single isomer as a TFA salt from **12d** (100 mg; 0.28 mmol) and ketone **2d** (56 mg; 0.29 mmol) according to the General Procedure **VII** in 18% yield (33 mg; 0.05 mmol). The crude product was purified by flash column chromatography on silica (hexane/AcOEt, 1:1, *v*/*v*, then AcOEt, 100%, then: AcOEt/MeOH, 20:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 90:10 to 20:80, 30 min, 18 mL/min). ESI-MS m/z for $C_{26}H_{33}ClF_3N_6O$ found 537.2/539.2 $[M+H]^+$; Rt = 1.10 min (Method A); $^1H$ NMR (700 MHz, DMSO-$d_6$ + $D_2O$, 348 K) δ 8.38 (s, 1H), 7.39 (d, $J_{AA'}$ = 8.5 Hz, 2H), 7.33 (d, $J_{BB'}$ = 8.5 Hz, 2H), 5.82 (s, 1H), 544 (q, $J_{H-F}$ = 9.0 Hz, 2H), 5.10 - 5.08 (m, 1H), 3.93 - 3.91 (m, 1H), 3.88 - 3.85 (m, 1H), 3.75 - 3.72 (m, 2H), 3.65 - 3.63 (m, 1H), 3.60 (s, 3H), 3.55 - 3.51 (m, 1H), 3.25 (dd, *J* = 13.9 Hz, *J* = 10.6 Hz, 1H), 3.20 - 3.17 (m, 1H), 2.49 - 2.46 (m, 1H), 2.32 - 2.26 (m, 2H), 2.17 (d, *J* = 0.6 Hz, 3H), 2.06 - 2.04 (m, 2H), 1.60 - 1.53 (m, 2H), 1.49 - 1.41 (m, 2H); $^{19}F$ NMR (700 MHz, DMSO-$d_6$ + $D_2O$) δ -70.1 (t, $J_{F-H}$ = 8.7 Hz), -74.1 (s, TFA).

**Example 13.**

Synthesis of (2*R*,5*S*)-5-(4-chlorobenzyl)-4-(4-(5-methyloxazol-2-yl)cyclohexyl)-2-(1-(2,2,2-trifluoroethyl)-1*H*-1,2,3-triazol-4-yl)morpholine 2,2,2-trifluoroacetate (**13**).

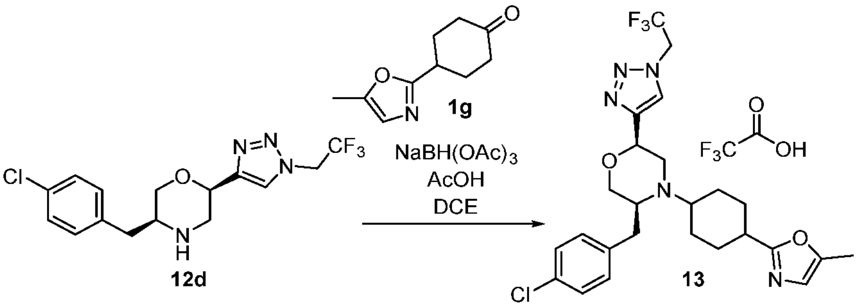


The title compound (**13**) was obtained as a single isomer as a TFA salt from **12d** (110 mg; 0.31 mmol) and ketone **1g** (57 mg; 0.32 mmol) according to the General Procedure **VII** in 6% yield (13 mg; 0.02 mmol). The crude product was purified by flash column chromatography on silica (hexane/AcOEt, 1:1, v/v, then AcOEt, 100%, then: AcOEt/MeOH, 40:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 90:10 to 20:80, 30 min, 20 mL/min). ESI-MS m/z for $C_{25}H_{30}ClF_3N_5O_2$ found 524.2/526.2 $[M+H]^+$; Rt = 1.11 min (Method A); $^1H$ NMR (700 MHz, DMSO-$d_6$ + $D_2O$, 348 K) δ 8.36 (s, 1H), 7.38 (d, $J_{AA'}$ = 8.5 Hz, 2H), 7.32 (d, $J_{BB'}$ = 8.5 Hz, 2H), 6.65 (q, *J* = 1.2 Hz, 1H), 5.42 (q, $J_{H\text{-}F}$ = 9.0 Hz, 2H), 5.08 - 5.06 (m, 1H), 3.91 - 3.89 (m, 1H), 3.83 - 3.81 (m, 1H), 3.73 - 3.69 (m, 2H), 3.62 - 3.58 (m, 1H), 3.53 - 3.51 (m, 1H), 3.23 (dd, *J* = 13.8 Hz, *J* = 10.7 Hz, 1H), 3.17 (dd, *J* = 13.8 Hz, *J* = 4.2 Hz, 1H), 2.76 - 2.72 (m, 1H), 2.34 - 2.29 (m, 2H), 2.22 (d, *J* = 1.2 Hz, 3H), 2.20 - 2.17 (m, 2H), 1.62 - 1.53 (m, 4H); $^{19}F$ NMR (700 MHz, DMSO-$d_6$ + $D_2O$) δ -70.1 (t, $J_{F\text{-}H}$ = 8.7 Hz), -73.9 (s, TFA).

**Example 14.**

Synthesis of (2*R*,5*S*)-5-(4-chlorobenzyl)-4-(4-(1,5-dimethyl-1*H*-pyrazol-3-yl)cyclohexyl)-*N*-(37-oxo-41-((3*aS*,4*S*,6*aR*)-2-oxohexahydro-1*H*-thieno[3,4-*d*]imidazol-4-yl)-3,6,9,12,15,18,21,24,27,30,33-undecaoxa-36-azahentetracontyl)morpholine-2-carboxamide 2,2,2-trifluoroacetate (**14**).

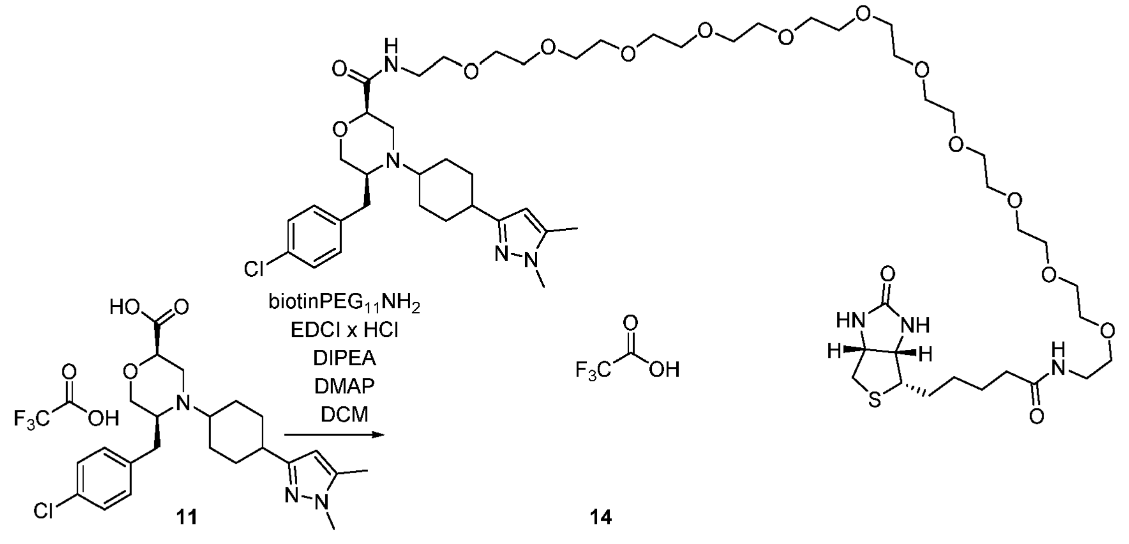


The title compound (**14**) was obtained as a single isomer as a TFA salt from **11** (28 mg; 0.050 mmol) according to the General Procedure **XI** in 18% yield (12 mg; 0.009 mmol). The crude product was purified twice by preparative reversed-phase column chromatography (first: column: Cosmosil Cholester 20 x 250 mm, water/MeCN + 1‰ TFA, 90:10 to 10:90, 30 min, 20 mL/min; second: column: Cosmosil Cholester 20 x 250 mm, water/MeCN + 1‰ TFA, 95:5 to 45:55, 30 min, 20 mL/min).

ESI-MS m/z for $C_{57}H_{95}ClN_7O_{15}S$ found 1184.6/1185.9 $[M+H]^+$; Rt = 1.06 min (Method A)

**Example 15.**

Synthesis of (2*R*,5*S*)-5-(4-chlorobenzyl)-4-(4-(1,5-dimethyl-1*H*-1,2,4-triazol-3-yl)cyclohexyl)-2-((methylsulfonyl) methyl)morpholine 2,2,2-trifluoroacetate (**15**).

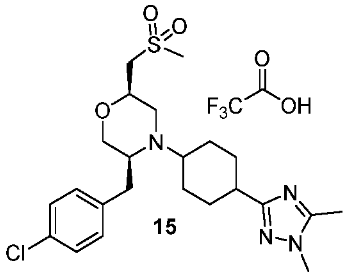

**15**

Step 1.

Synthesis of 1,5-dimethyl-3-(1,4-dioxaspiro[4.5]dec-7-en-8-yl)-1*H*-1,2,4-triazole (**15a**).

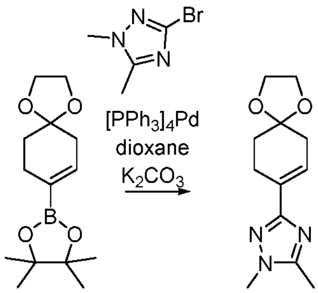


**15a**

The solution of 4,4,5,5-tetramethyl-2-(1,4-dioxaspiro[4.5]dec-7-en-8-yl)-1,3,2-dioxaborolane (560 mg; 2.10 mmol), 3-bromo-1,5-dimethyl-1*H*-1,2,4-triazole (352 mg, 2.00 mmol), $[PPh_3]_4Pd$ (116 mg; 0.10 mmol) in mixture of dioxane and 2 M aqueous solution of $K_2CO_3$ (10 mL; 4:1 v/v) was heated at 100 °C for 24 hours under argon atmosphere. TLC showed complete consumption of the starting material. Then AcOEt (40 mL) was added and this mixture was washed with water (3 x). An organic layer was concentrated *in vacuo* and the residue was purified by flash column chromatography on silica (hexane/AcOEt, 1:3, *v/v*, then AcOEt, 100%, then: AcOEt/MeOH, 14:1, v/v). Compound **15a** was obtained in 28% yield (132 mg; 0.56 mmol). ESI-MS m/z for $C_{12}H_{18}N_3O_2$ found 236.0 $[M+H]^+$; Rt = 0.58 min (Method A); $^1H$ NMR (700 MHz, DMSO-$d_6$ + $D_2O$) δ 6.43 - 6.40 (m, 1H), 3.88 (s, 4H), 3.68 (s, 3H), 2.50 - 2.47 (m, 2H), 2.33 - 2.30 (m, 5H), 1.77 - 1.68 (m, 2H).

Step 2.

Synthesis of 1,5-dimethyl-3-(1,4-dioxaspiro[4.5]decan-8-yl)-1*H*-1,2,4-triazole (**15b**).

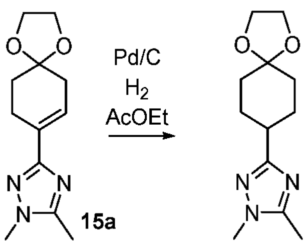


**15a** **15b**

To the solution of **15a** (132 mg; 0.56 mmol) in AcOEt (10 mL) 10% Pd/C (5 mol%; 27 mg, 0.025 mmol) was added and the reaction mixture was conducted under hydrogen atmosphere. The reaction progress was monitored by LC-MS. After 2.5 hours, when analysis indicated completion of the reaction, Pd/C was filtered off through the Celite and the solvent

was concentrated *in vacuo.* The crude product was used to the next step without additional purification. Compound **15b** was obtained in 93% yield (124 mg; 0.52 mmol).
ESI-MS m/z for $C_{12}H_{20}N_3O_2$ found 238.0 $[M+H]^+$; $R_t$ = 0.36 min (Method A)

Step 3.

Synthesis of 4-(1,5-dimethyl-1*H*-1,2,4-triazol-3-yl)cyclohexan-1-one (**15c**).

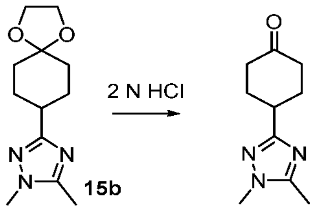


**15b** **15c**

The solution of **15b** (124 mg; 0.52 mmol) in 2 M HCl (10 mL) was stirred at room temperature for 2.5 hours. The reaction progress was monitored by LC-MS. When analysis indicated completion of the reaction, to this mixture aqueous solution of NaOH (4 M) was added to pH 13 and then extracted with DCM (2 x). The combined organic solutions were dried over anhydrous $MgSO_4$, filtered and concentrated *in vacuo.* The crude product was used to the next step without additional purification. Compound **15c** was obtained in 94% yield (95 mg; 0.49 mmol). ESI-MS m/z for $C_{10}H_{16}N_3O$ found 194.0 $[M+H]^+$; $R_t$ = 0.93 min (Method A)

Step 4.

Synthesis of (2*R*,5*S*)-5-(4-chlorobenzyl)-4-(4-(1,5-dimethyl-1*H*-1,2,4-triazol-3-yl)cyclohexyl)-2-((methylsulfonyl) methyl)morpholine 2,2,2-trifluoroacetate (**15**).

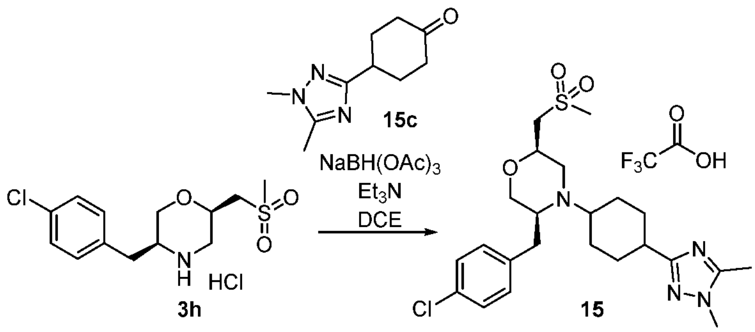


The title compound (**15**) was obtained as a single isomer as a TFA salt from **3h** (184 mg; 0.54 mmol) and ketone **15c** (95 mg; 0.49 mmol) according to the General Procedure **VII** in 18% yield (53 mg; 0.09 mmol). The crude product was purified by flash column chromatography on silica (hexane/AcOEt, 1:1 to 1:4, v/v, then: AcOEt/MeOH, 100:0 to 4:1, v/v) and then transformed into TFA salt with using TFA/DCM and then purified by preparative reversed-phase column chromatography (column: Cosmosil Cholester 20 x 250 mm, water/MeCN + 1‰ TFA, 95:5 to 20:80, 30 min, 20 mL/min). ESI-MS m/z for $C_{23}H_{34}ClN_4O_3S$ found 481.1/483.1 $[M+H]^+$; Rt = 0.80 min (Method A); $^1H$ NMR (700 MHz, DMSO-$d_6$ + $D_2O$, 348 K) $\delta$ 7.38 (d, $J_{AA'}$ = 8.5 Hz, 2H), 7.32 (d, $J_{BB'}$ = 8.5 Hz, 2H), 4.29 - 4.26 (m, 1H), 3.80 - 3.77 (m, 2H), 3.69 (s, 3H), 3.68 - 3.66 (m, 1H), 3.60 - 3.56 (m, 2H), 3.49 - 3.44 (m, 1H), 3.41 - 3.39 (m, 1H), 3.31 - 3.28 (m, 1H), 3.19 (dd, *J* = 13.7 Hz, *J* = 11.0 Hz, 1H), 3.11 (dd, *J* = 13.7 Hz, *J* = 3.9 Hz, 1H), 3.03 (s, 3H), 2.64 - 2.60 (m, 1H), 2.36 (s, 3H), 2.28 - 2.26 (m, 2H), 2.14 - 2.11 (m, 2H), 1.58 - 1.53 (m, 4H).

**Example 16.**

Synthesis of (2*R*,5*S*)-5-(4-chlorobenzyl)-4-(4-(1,5-dimethyl-1*H*-pyrazol-3-yl)cyclohexyl)-2-(fluoromethyl)morpholine 2,2,2-trifluoroacetate (**16**).

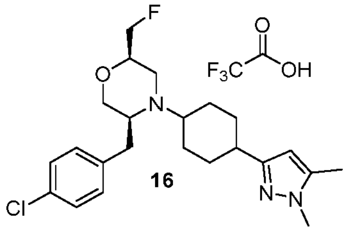

16

Step 1.

Synthesis of *tert*-butyl (2*R*,5*S*)-5-(4-chlorobenzyl)-2-(fluoromethyl)morpholine-4-carboxylate (**16a**).

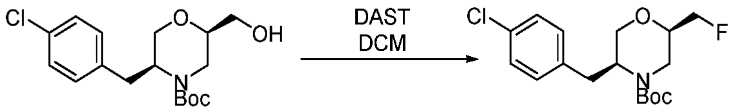


**3d** **16a**

The title compound (**16a**) was obtained from **3d** (160 mg; 0.46 mmol) according to the General Procedure **XII** in 60% yield (95 mg; 0.28 mmol).
ESI-MS $C_{12}H_{16}ClFNO$ found 243.9/245.9 [M+H-Boc]$^+$; $R_t$ = 1.76 min (Method A); $^1H$ NMR (700 MHz, DMSO-$d_6$ + $D_2O$, 348 K) δ 7.31 - 7.24 (m, 2H), 7.23 - 7.12 (m, 2H), 4.54 - 4.48 (m, 1H), 4.46 - 4.40 (m, 1H), 4.08 - 4.02 (m, 1H), 3.79 - 3.68 (m, 2H), 3.63 - 3.57 (m, 1H), 3.54 - 3.50 (m, 1H), 3.01 - 2.90 (m, 2H), 2.84 - 2.79 (m, 1H), 1.31 - 1.13 (m, 9H).

Step 2.

Synthesis of (2*R*,5*S*)-5-(4-chlorobenzyl)-2-(fluoromethyl)morpholine hydrochloride (**3b**).

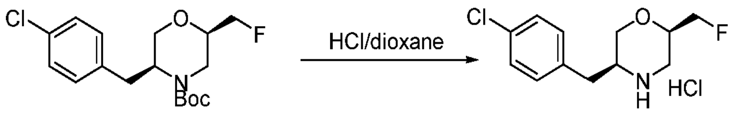


**16a** **16b**

The title compound (**16b**) was obtained as a hydrochloride salt from **16a** (0.55 g; 1.59 mmol) according to the General Procedure **IVa** in 99% yield (439 mg; 1.57 mmol).
ESI-MS $C_{12}H_{16}ClFNO$ found 243.9/245.9 [M+H]$^+$; Rt = 0.63 min (Method A)

Step 3.

Synthesis of (2*R*,5*S*)-5-(4-chlorobenzyl)-4-(4-(1,5-dimethyl-1*H*-pyrazol-3-yl)cyclohexyl)-2-(fluoromethyl)morpholine 2,2,2-trifluoroacetate (**16**).

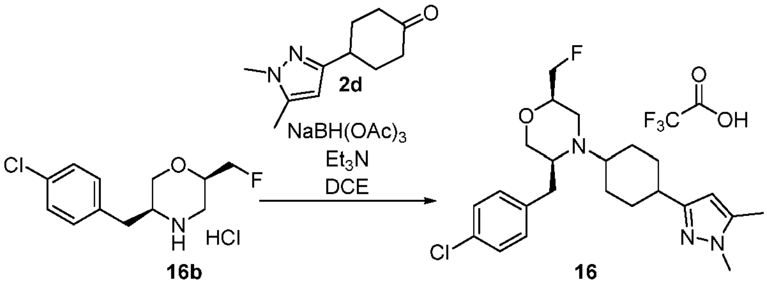


The title compound (**16**) was obtained as a single isomer as a TFA salt from **16b** (61 mg; 0.220 mmol) and ketone **2d** (47 mg; 0.240 mmol) according to the General Procedure **VII** in 8% yield (9 mg; 0.017 mmol). The crude product was purified by flash column chromatography on silica (DCM/MeOH, 100:0 to 9:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 95:5 to 20:80, 30 min, 20 mL/min).
ESI-MS m/z for $C_{23}H_{32}ClFN_3O$ found 420.1/422.1 $[M+H]^+$; Rt = 1.03 min (Method A); $^1H$ NMR (700 MHz, DMSO-$d_6$ + $D_2O$, 348 K) δ 7.38 (d, $J_{AA'}$ = 8.4 Hz, 2H), 7.32 (d, $J_{BB'}$ = 8.4 Hz, 2H), 5.84 (s, 1H), 4.60 (qd, *J* = 10.6 Hz, *J* = 3.6 Hz, 1H), 4.53 (qd, *J* = 10.6 Hz, *J* = 3.6 Hz, 1H), 4.07 - 4.02 (m, 1H), 3.80 - 3.76 (m, 2H), 3.68 - 3.66 (m, 1H), 3.61 (s, 3H), 3.58 - 3.56 (m, 1H), 3.49 - 3.47 (m, 1H), 3.23 - 3.20 (m, 1H), 3.15 - 3.09 (m, 2H), 2.49 - 2.46 (m, 1H), 2.31 - 2.25 (m, 2H), 2.17 (s, 3H), 2.06 - 2.03 (m, 2H), 1.57 - 1.50 (m, 2H), 1.48 - 1.41 (m, 2H); $^{19}F$ NMR (250 MHz, DMSO-$d_6$ + $D_2O$) δ -74.8 (s, TFA), -232.1 (td, $J_{F-H}$ = 47.2 Hz, $J_{F-H}$ = 23.4 Hz).

**Example 17.**

Synthesis of ((2*R*,5*S*-5-(4-chlorobenzyl)-4-(4-(1,5-dimethyl-1*H*-pyrazol-3-yl)cyclohexyl)morpholin-2-yl)methanol 2,2,2-trifluoroacetate (**17**).

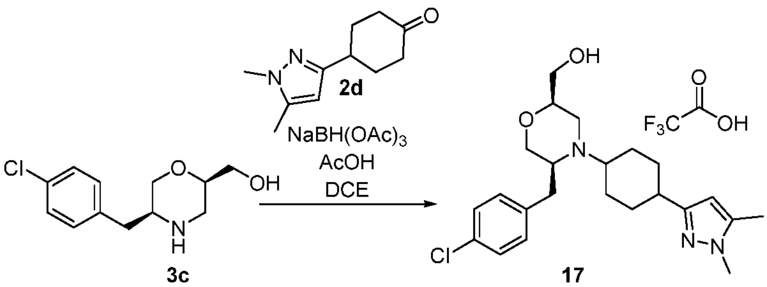


The title compound (**17**) was obtained as a single isomer from **3c** (3.495 g; 14.5 mmol) and ketone **2d** (2.900 g; 15.0 mmol) according to the General Procedure **VII** in 21% yield (1.305 g; 3.1 mmol). The sample of 32 mg of the product was transformed into TFA salt with using TFA/DCM and then purified by preparative reversed-phase column chromatography (column: Cosmosil Cholester 20 x 250 mm, water/MeCN + 1‰ TFA, 95:5 to 30:70, 30 min, 20 mL/min) to give a product as a TFA salt.
ESI-MS m/z for $C_{23}H_{33}ClN_3O_2$ found 418.1/420.1 $[M+H]^+$; Rt = 0.90 min (Method A); $^1H$ NMR (700 MHz, DMSO-$d_6$ + $D_2O$, 348 K) δ 7.40 - 7.37 (m, 2H), 7.34 - 7.31 (m, 2H), 5.83 (d, *J* = 0.9 Hz, 1H), 3.82 - 3.71 (m, 3H), 3.66 - 3.63 (m, 1H), 3.61 (s, 3H), 3.59 - 3.55 (m, 2H), 3.52 - 3.47 (m, 2H), 3.19 - 3.12 (m, 2H), 3.12 - 3.06 (m, 1H), 2.50 - 2.44 (m, 1H), 2.34 - 2.23 (m, 2H), 2.17 (s, 3H), 2.07 - 2.01 (m, 2H), 1.58 - 1.48 (m, 2H), 1.49 - 1.41 (m, 2H).

**Example 18.**

Synthesis of (2*R*,5*S*)-5-(4-chlorobenzyl)-*N*-ethyl-4-(4-(5-methyloxazol-2-yl)cyclohexyl)morpholine-2-carboxamide 2,2,2-trifluoroacetate (**18**).

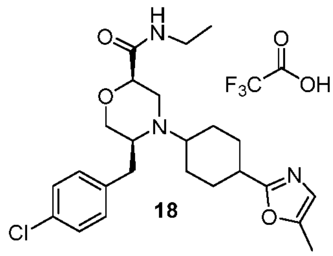

**18**

Step 1.

Synthesis of *tert*-butyl (2*R*,5*S*)-5-(4-chlorobenzyl)-2-(ethylcarbamoyl)morpholine-4-carboxylate (**18a**).

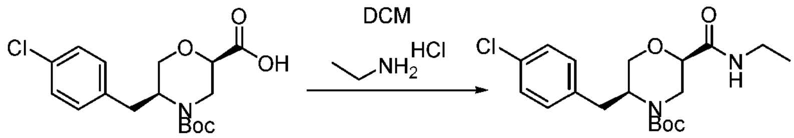


**5a** **18a**

The title compound (**18a**) was obtained from **5a** (712 mg; 2.20 mmol) according to the General Procedure **V** in 39% yield (330 mg; 0.86 mmol).
ESI-MS m/z for $C_{19}H_{28}ClN_2O_4$ found 383.0/385.0 $[M+H]^+$; Rt = 1.54 min (Method A); $^1$H NMR (700 MHz, DMSO-$d_6$ + $D_2O$, 348 K) δ 7.30 - 7.26 (m, 2H), 7.21 - 7.18 (m, 2H), 4.10 - 3.92 (m, 2H), 3.81 - 3.74 (m, 2H), 3.61 - 3.53 (m, 1H), 3.21 - 3.09 (m, 2H), 2.97 - 2.90 (m, 2H), 2.88 - 2.80 (m, 1H), 1.21 (s, 8H), 1.05 (t, *J* = 7.2 Hz, 3H).

Step 2.

Synthesis of (2*R*,5*S*)-5-(4-chlorobenzyl)-*N*-ethylmorpholine-2-carboxamide hydrochloride (**18b**).

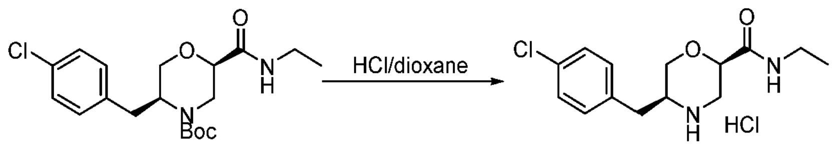


**18a** **18b**

The title compound (**18b**) was obtained as a hydrochloride salt from **18a** (330 mg; 0.86 mmol) according to the General Procedure **IVa** in 99% yield (240 mg; 0.85 mmol).
ESI-MS m/z for $C_{14}H_{20}ClN_2O_2$ found 283.1/285.1 $[M+H]^+$; Rt = 0.61 min (Method A)

Step 3.

Synthesis of (2*R*,5*S*)-5-(4-chlorobenzyl)-*N*-ethyl-4-(4-(5-methyloxazol-2-yl)cyclohexyl)morpholine-2-carboxamide 2,2,2-trifluoroacetate (**18**).

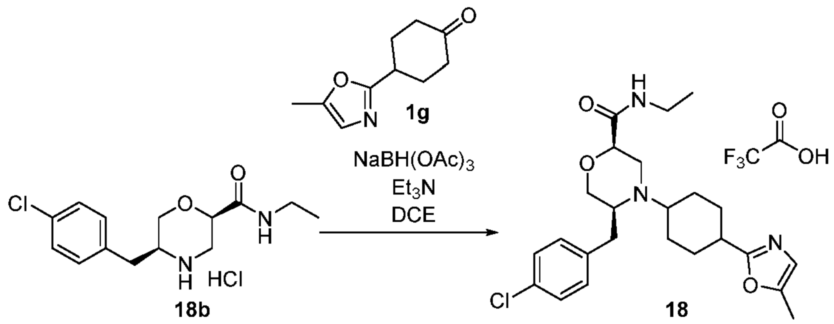


18b

18

The title compound (**18**) was obtained as a single isomer as a TFA salt from **18b** (150 mg; 0.47 mmol) and ketone **1g** (95 mg; 0.52 mmol) according to the General Procedure **VII** in 4% yield (13 mg; 0.02 mmol). The crude product was purified by silica-gel column chromatography (pentane/AcOEt, 1:1 to 1:5, *v*/*v*, then AcOEt, 100%, then: AcOEt/MeOH, 25:1 to 20:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 95:5 to 30:70, 30 min, 20 mL/min).

ESI-MS m/z for $C_{24}H_{33}ClN_3O_3$ found 446.2/448.2 $[M+H]^+$; Rt = 1.03 min (Method A); $^1$H NMR (700 MHz, DMSO-$d_6$ + $D_2O$, 348 K) δ 7.38 (d, $J_{AA'}$ = 8.5 Hz, 2H), 7.35 (d, $J_{BB'}$ = 8.5 Hz, 2H), 6.65 (q, *J* = 1.2 Hz, 1H), 4.31 - 4.29 (m, 1H), 3.78 - 3.76 (m, 2H), 3.73 - 3.70 (m, 1H), 3.56 - 3.54 (m, 1H), 3.51 - 3.48 (m, 1H), 3.35 - 3.32 (m, 1H), 3.23 - 3.07 (m, 4H), 2.76 - 2.71 (m, 1H), 2.26 - 2.25 (m, 1H), 2.23 (d, *J* = 1.2 Hz, 3H), 2.22 - 2.13 (m, 3H), 1.62 - 1.51 (m, 4H), 1.07 (t, *J* = 7.2 Hz, 3H).

**Example 19.**

Synthesis of (2*R*,5*S*)-5-(4-chlorobenzyl)-4-(4-(1,5-dimethyl-1*H*-pyrazol-3-yl)cyclohexyl)-*N*-ethylmorpholine-2-carboxamide 2,2,2-trifluoroacetate (**19**).

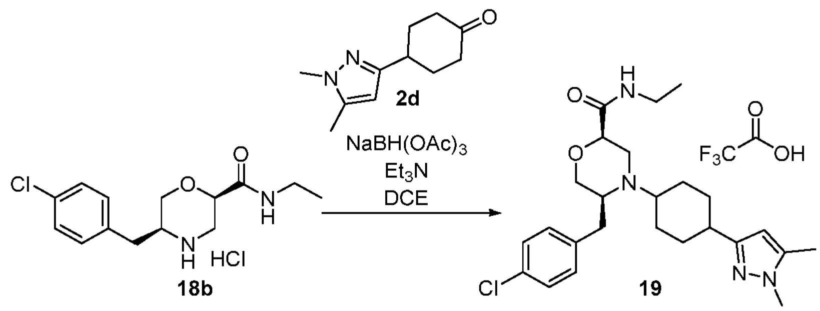


18b

19

The title compound (**19**) was obtained as a single isomer as a TFA salt from **18b** (150 mg; 0.470 mmol) and ketone **2d** (100 mg; 0.520 mmol) according to the General Procedure **VII** in 20% yield (55 mg; 0.096 mmol). The crude product was purified by silica-gel column chromatography (pentane/AcOEt, 2:3 to 1:6, *v*/*v*, then AcOEt, 100%, then: AcOEt/MeOH, 120:1 to 10:1, *v*/*v*) and then transformed into TFA salt with using TFA/DCM and then purified by preparative reversed-phase column chromatography (column: Cosmosil Cholester 20 x 250 mm, water/MeCN + 1‰ TFA, 95:5 to 20:80, 30 min, 20 mL/min).

ESI-MS m/z for $C_{25}H_{36}ClN_4O_2$ found 459.2/461.2 $[M+H]^+$; Rt = 1.00 min (Method A); $^1$H NMR (700 MHz, DMSO-$d_6$ + $D_2O$, 348 K) δ 7.38 (d, $J_{AA'}$ = 8.5 Hz, 2H), 7.34 (d, $J_{BB'}$ = 8.5 Hz, 2H), 5.82 (s, 1H), 4.31 - 4.30 (m, 1H), 3.79 - 3.76 (m, 2H), 3.73 - 3.71 (m, 1H), 3.59 (s, 3H), 3.58 - 3.56 (m, 1H), 3.51 - 3.49 (m, 1H), 3.36 - 3.32 (m, 1H), 3.23 - 3.12 (m, 2H), 3.11 - 3.09 (m, 2H), 2.48 - 2.44 (m, 1H), 2.23 - 2.16 (m, 5H), 2.05 - 1.99 (m, 2H), 1.59 - 1.50 (m, 2H), 1.46 - 1.36 (m, 2H), 1.07 (t, *J* = 7.2 Hz, 3H).

**Example 20.**

Synthesis of (2*R*,5*S*)-5-(4-chlorobenzyl)-4-(4-(1,5-dimethyl-1*H*-pyrazol-3-yl)cyclohexyl)-*N,N*-dimethylmorpholine-2-carboxamide 2,2,2-trifluoroacetate (**20**).

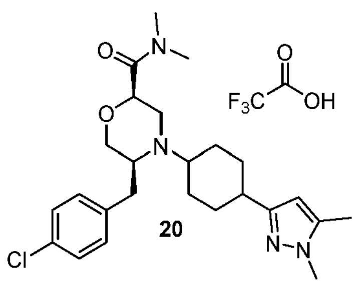

**20**

The title compound (**20**) was obtained as a TFA salt as a single isomer in 15% overall yield in a similar way to Example 18 with the exception that, in the first step of the synthesis, dimethylamine (2 M solution) was used instead of ethylamine hydrochloride and in the third step of the synthesis, the compound **2d** was used instead of the compound **1g.** The crude product was purified by silica-gel column chromatography (pentane/AcOEt, 2:3 to 1:6, v/v, then AcOEt, 100%, then: AcOEt/MeOH, 6:1 to 5:1, *v/v)* and then transformed into TFA salt with using TFA/DCM and then purified by preparative reversed-phase column chromatography (column: Cosmosil Cholester 20 x 250 mm, water/MeCN + 1‰ TFA, 95:5 to 30:70, 30 min, 20 mL/min).

ESI-MS m/z for $C_{25}H_{36}ClN_4O_2$ found 459.2/461.2 $[M+H]^+$; Rt = 0.98 min (Method A); $^1H$ NMR (700 MHz, DMSO-$d_6$ + $D_2O$, 348 K) δ 7.36 (d, $J_{AA'}$ = 8.4 Hz, 2H), 7.29 (d, $J_{BB'}$ = 8.4 Hz, 2H), 5.84 (s, 1H), 4.74 - 4.73 (m, 1H), 3.82 - 3.80 (m, 2H), 3.64 - 3.60 (m, 4H), 3.56 - 3.51 (m, 2H), 3.43 - 3.41 (m, 1H), 3.19 - 3.17 (m, 1H), 3.02 - 3.00 (m, 4H), 2.88 (brs, 3H), 2.49 - 2.45 (m, 1H), 2.20 - 2.16 (m, 4H), 2.12 - 2.09 (m, 1H), 2.05 - 1.98 (m, 2H), 1.61 - 1.51 (m, 2H), 1.44 (qd, *J* = 13.0 Hz, *J* = 3.3 Hz, 1H), 1.35 - 1.32 (m, 1H).

**Example 21.**

Synthesis of (2*R*,5*S*)-5-(4-chlorobenzyl)-*N,N*-dimethyl-4-(4-(5-methyloxazol-2-yl)cyclohexyl)morpholine-2-carboxamide 2,2,2-trifluoroacetate (**21**).

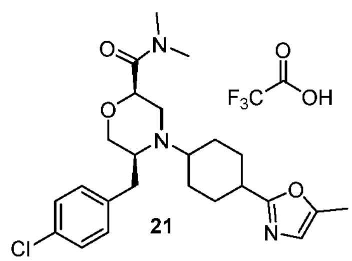

**21**

The title compound (**21**) was obtained as a TFA salt as a single isomer in 3% overall yield in a similar way to Example 18 with the exception that, in the first step of the synthesis, dimethylamine (2 M solution) was used instead of ethylamine hydrochloride. The crude product was purified by silica-gel column chromatography (pentane/AcOEt, 7:3, v/v, then AcOEt, 100%, then: AcOEt/MeOH, 180:1, *v/v*) and then transformed into TFA salt with using TFA/DCM and then purified by preparative reversed-phase column chromatography (column: Cosmosil Cholester 20 x 250 mm, water/MeCN + 1‰ TFA, 95:5 to 30:70, 30 min, 20 mL/min).

ESI-MS m/z for $C_{24}H_{33}ClN_3O_3$ found 446.1/448.1 $[M+H]^+$; Rt = 0.99 min (Method A); $^1H$ NMR (700 MHz, DMSO-$d_6$ + $D_2O$, 348 K) δ 7.37 (d, $J_{AA'}$ = 8.4 Hz, 2H), 7.30 (d, $J_{BB'}$ = 8.4 Hz, 2H), 6.65 - 6.64 (m, 1H), 4.75 - 4.73 (m, 1H), 3.82 - 3.80 (m, 2H), 3.64 - 3.57 (m, 2H), 3.54 - 3.53 (m, 1H), 3.43 - 3.41 (m, 1H), 3.21 - 3.19 (m, 1H), 3.03 - 3.00 (m, 4H), 2.88 (brs, 3H), 2.72 (tt, *J* = 12.1 Hz, *J* = 3.5 Hz, 1H), 2.24 - 2.22 (m, 4H), 2.18 - 2.12 (m, 3H), 1.64 - 1.54 (m, 3H), 1.50 - 1.45 (m, 1H).

**Example 22.**

Synthesis of (2*R*,5*S*)-5-(4-chlorobenzyl)-4-(4-(1,5-dimethyl-1*H*-pyrazol-3-yl)cyclohexyl)-2-(5-methyl-1,3,4-oxadiazol-2-yl)morpholine 2,2,2-trifluoroacetate (**22**).

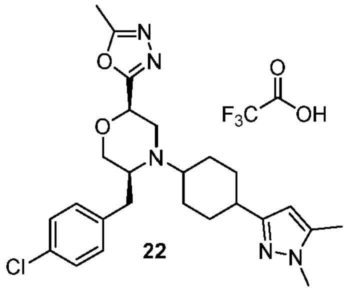

22

Step 1.

Synthesis of 4-(*tert*-butyl) 2-methyl (2*R*,5*S*)-5-(4-chlorobenzyl)morpholine-2,4-dicarboxylate (**22a**).

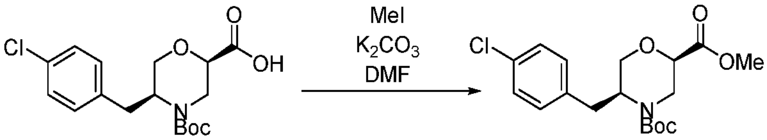


5a 22a

To the solution of compound **5a** (1 g; 2.81 mmol) in DMF (15 mL) $K_2CO_3$ (777 mg; 5.62 mmol) and MeI (0.35 mL; 5.62 mmol) were added and the reaction mixture was stirred at room temperature overnight. The reaction progress was monitored by LC-MS. When analysis indicated completion of the reaction, to this reaction mixture $Et_2O$ (200 mL) was added and a whole was washed with water (2 x). Then dried over anhydrous $MgSO_4$, filtered and concentrated *in vacuo.* The crude product was purified by silica-gel column chromatography (hexane/AcOEt, 20:1 to 5:1, *v*/*v*). Compound **22a** was obtained in 69% yield (717 mg; 1.94 mmol).
ESI-MS m/z for $C_{18}H_{25}ClNO_5$ found 370.2/372.2 $[M+H]^+$; Rt = 1.59 min (Method A); $^1H$ NMR (700 MHz, DMSO-$d_6$ + $D_2O$) δ 7.32 - 7.26 (m, 2H), 7.22 - 7.17 (m, 2H), 4.11 - 3.97 (m, 3H), 3.79 - 3.77 (m, 1H), 3.69 (s, 3H), 3.59 - 3.53 (m, 1H), 3.18 - 3.08 (m, 1H), 3.04 - 2.90 (m, 1H), 2.80 - 2.68 (m, 1H), 1.28 - 1.02 (m, 9H).

Step 2.

Synthesis of *tert*-butyl (2*R*,5*S*)-5-(4-chlorobenzyl)-2-(hydrazinecarbonyl)morpholine-4-carboxylate (**22b**).

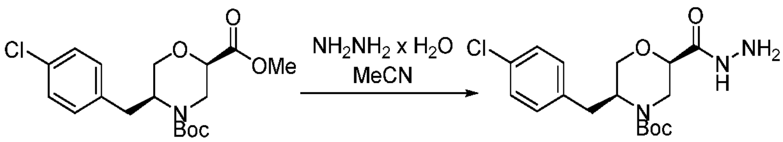


22a 22b

To the suspension of **22a** (715 mg; 1.93 mmol) in MeCN (30 mL) hydrazine monohydrate (1.16 mL; 23.20 mmol) was added and the reaction mixture was stirred under gentle reflux (94 °C) overnight. Then another portion of hydrazine monohydrate (0.5 mL; 10.00 mmol) was added and the reaction mixture was stirred under gentle reflux (94 °C) for 4 hours. The reaction progress was monitored by LC-MS. When analysis indicated completion of the reaction, the whole was concentrated *in vacuo* and the crude product was used to the next step without additional purification. Compound **22b** was obtained in 99% yield (705 mg; 1.91 mmol).
ESI-MS m/z for $C_{17}H_{25}ClN_3O_4$ found 370.0/372.0 $[M+H]^+$; Rt = 1.28 min (Method A); $^1H$ NMR (700 MHz, DMSO-$d_6$ + $D_2O$, 348 K) δ 7.29 - 7.25 (m, 2H), 7.21 - 7.18 (m, 2H), 4.10 - 4.04 (m, 1H), 4.00 - 3.91 (m, 1H), 3.89 - 3.83 (m, 1H), 3.78 - 3.74 (m, 1H), 3.58 - 3.52 (m, 1H), 3.04 - 2.96 (m, 1H), 2.96 - 2.91 (m, 1H), 2.89 - 2.82 (m, 1H), 1.23 (s, 9H).

Step 3.

Synthesis of *tert*-butyl (2*R*,5*S*)-5-(4-chlorobenzyl)-2-(5-methyl-1,3,4-oxadiazol-2-yl)morpholine-4-carboxylate (**22c**).

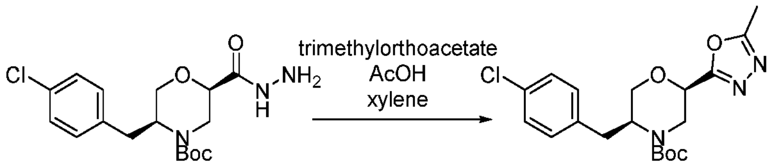


**22b** **22c**

To the suspension of **22b** (705 mg; 1.91 mmol) in xylene (38 mL) trimethylorthoacetate (0.49 mL; 3.86 mmol) and acetic acid (0.14 mL; 2.30 mmol) were added and the reaction mixture was stirred under reflux for 6 hours. The reaction progress was monitored by LC-MS. When analysis indicated completion of the reaction, the reaction mixture was cooled to room temperature and whole was diluted with AcOEt (280 mL) and then washed with 5% $NaHCO_3$ (3 x 70 mL) and brine (70 mL). The organic layer was dried over anhydrous $MgSO_4$, filtered and concentrated *in vacuo.* The crude product was purified by silica-gel column chromatography (cyclohexane/AcOEt, 3:1 to 2:1, v/v). Compound **22c** was obtained in 85% yield (640 mg; 1.63 mmol).
ESI-MS m/z for $C_{19}H_{25}ClN_3O_4$ found 394.0/396.0 $[M+H]^+$; Rt = 1.54 min (Method A); $^1H$ NMR (700 MHz, DMSO-$d_6$ + $D_2O$, 348 K) δ 7.31 - 7.28 (m, 2H), 7.23 - 7.20 (m, 2H), 4.74 - 4.67 (m, 1H), 4.22 - 4.14 (m, 1H), 4.11 - 4.04 (m, 1H), 3.87 - 3.81 (m, 1H), 3.77 - 3.68 (m, 1H), 3.43 - 3.38 (m, 1H), 3.37 (s, 3H), 3.01 - 2.96 (m, 1H), 2.93 - 2.85 (m, 1H), 1.24 (s, 9H).

Step 4.

Synthesis of (2*R*,5*S*)-5-(4-chlorobenzyl)-2-(5-methyl-1,3,4-oxadiazol-2-yl)morpholine (**22d**).

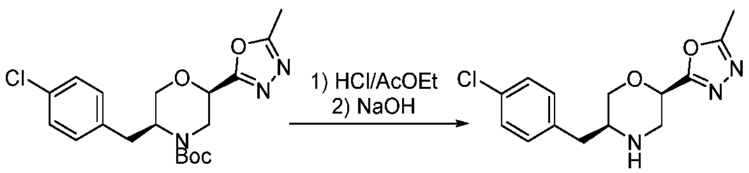


**22c** **22d**

The title compound (**22d**) was obtained as a free amine in 49% yield (236 mg; 0.80 mmol) from **22c** (638 mg; 1.62 mmol) according to the General Procedure **IVa** and then after basic (NaOH) extraction with DCM.
ESI-MS m/z for $C_{14}H_{17}ClN_3O_2$ found 294.0/296.0 $[M+H]^+$; Rt = 0.29 min (Method A)

Step 5.

Synthesis of (2*R*,5*S*)-5-(4-chlorobenzyl)-4-(4-(1,5-dimethyl-1*H*-pyrazol-3-yl)cyclohexyl)-2-(5-methyl-1,3,4-oxadiazol-2-yl)morpholine 2,2,2-trifluoroacetate (**22**).

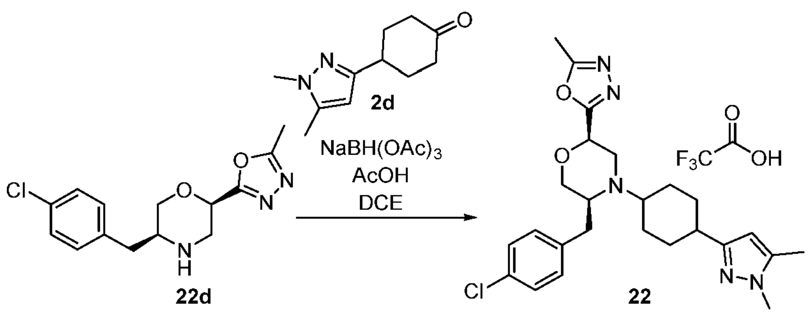


**22d** **22**

The title compound (**22**) was obtained as a single isomer as a TFA salt from **22d** (118 mg; 0.400 mmol) and ketone **2d** (82 mg; 0.420 mmol) according to the General Procedure **VII** in 5% yield (13 mg; 0.022 mmol). The crude product was purified by silica-gel column chromatography (hexane/AcOEt, 1:1, *v*/*v*, then AcOEt, 100%, then: AcOEt/MeOH, 100:1 to 10:1, *v*/*v*) and then transformed into TFA salt with using TFA/DCM and then purified twice by preparative reversed-phase column chromatography (column: Cosmosil Cholester 20 x 250 mm, water/MeCN + 1‰ TFA, 95:5 to 10:90, 30 min, 20 mL/min).
ESI-MS m/z for $C_{25}H_{33}ClN_5O_2$ found 470.2/472.2 $[M+H]^+$; Rt = 1.09 min (Method A); $^1H$ NMR (700 MHz, DMSO-$d_6$ + $D_2O$, 348 K) δ 7.35 (d, $J_{AA'}$ = 8.5 Hz, 2H), 7.28 (d, $J_{BB'}$ = 8.5 Hz, 2H), 5.81 (s, 1H), 5.15 - 5.14 (m, 1H), 3.87 - 3.85 (m, 1H), 3.74 - 3.71 (m, 2H), 3.66 - 3.64 (m, 1H), 3.62 - 3.58 (m, 4H), 3.14 - 3.11 (m, 1H), 3.06 (dd, *J* = 13.9 Hz, *J* = 10.1 Hz, 1H), 2.51 (s, 3H), 2.49 - 2.44 (m, 2H), 2.19 - 2.14 (m, 5H), 2.04 - 1.99 (m, 2H), 1.56 - 1.48 (m, 2H), 1.46 - 1.36 (m, 2H).

**Example 23.**

Synthesis of (2*R*,5*S*)-*N'*-acetyl-5-(4-chlorobenzyl)-4-(4-(1,5-dimethyl-1*H*-pyrazol-3-yl)cyclohexyl)morpholine-2-carbohydrazide 2,2,2-trifluoroacetate (**23**).

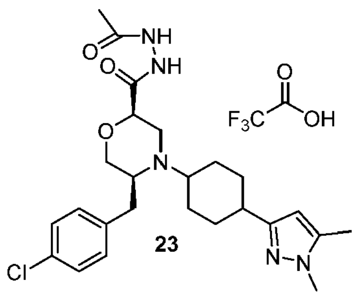

**23**

The title compound (**23**) was obtained during the synthesis of compound 22 as a by-product as a TFA salt as a single isomer from **22d** (118 mg; 0.400 mmol) and ketone **2d** (82 mg; 0.420 mmol) according to the General Procedure **VII** in 5% yield (12 mg; 0.020 mmol). The crude product was purified by silica-gel column chromatography (hexane/AcOEt, 1:1, *v*/*v*, then AcOEt, 100%, then: AcOEt/MeOH, 100:1 to 10:1, *v*/*v*) and then transformed into TFA salt with using TFA/DCM and then purified twice by preparative reversed-phase column chromatography (column: Cosmosil Cholester 20 x 250 mm, water/MeCN + 1‰ TFA, 95:5 to 20:80, 30 min, 20 mL/min).
ESI-MS m/z for $C_{25}H_{35}ClN_5O_3$ found 488.2/490.2 $[M+H]^+$; Rt = 0.93 min (Method A); $^1H$ NMR (700 MHz, DMSO-$d_6$ + $D_2O$, 348 K) δ 7.37 (d, $J_{AA'}$ = 8.4 Hz, 2H), 7.33 (d, $J_{BB'}$ = 8.4 Hz, 2H), 5.80 (s, 1H), 4.46 - 4.45 (m, 1H), 3.79 - 3.75 (m, 3H), 3.59 (s, 3H), 3.54 - 3.52 (m, 1H), 3.45 - 3.42 (m, 1H), 3.37 - 3.35 (m, 1H), 3.12 - 3.11 (m, 2H), 2.49 - 2.45 (m, 1H), 2.21 - 2.16 (m, 5H), 2.07 - 1.99 (m, 2H), 1.90 (s, 3H), 1.59 - 1.50 (m, 2H), 1.46 - 1.36 (m, 2H).

**Example 24.**

Synthesis of (2*R*,5*S*)-5-(4-chlorobenzyl)-2-(5-methyl-1,3,4-oxadiazol-2-yl)-4-(4-(5-methyloxazol-2-yl)cyclohexyl)morpholine 2,2,2-trifluoroacetate (**24**).

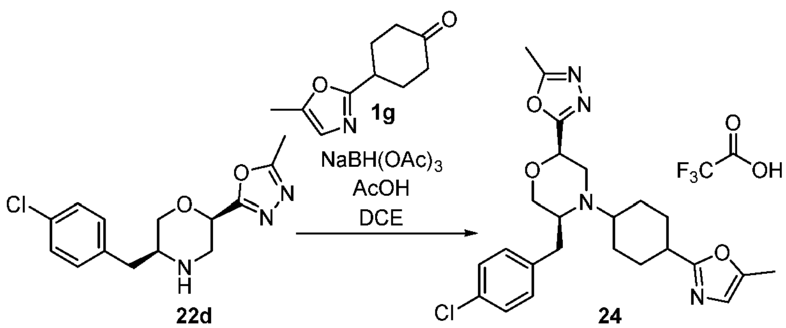

The title compound (**24**) was obtained as a single isomer as a TFA salt from **22d** (118 mg; 0.400 mmol) and ketone **1g** (76 mg; 0.420 mmol) according to the General Procedure **VII** in 4% yield (9 mg; 0.016 mmol). The crude product was purified by silica-gel column chromatography (hexane/AcOEt, 3:4, v/v, then AcOEt, 100%, then: AcOEt/MeOH, 30:1 to 5:1, *v/v)* and then purified twice by preparative reversed-phase column chromatography (column: Cosmosil Cholester 20 x 250 mm, water/MeCN + 1‰ TFA, 95:5 to 20:80, 30 min, 20 mL/min).
ESI-MS m/z for $C_{24}H_{30}ClN_4O_3$ found 457.4/459.4 $[M+H]^+$; Rt = 1.15 min (Method A); $^1H$ NMR (700 MHz, DMSO-$d_6$ + $D_2O$, 348 K) δ 7.39 (d, $J_{AA'}$ = 8.5 Hz, 2H), 7.29 (d, $J_{BB'}$ = 8.5 Hz, 2H), 6.67 (q, *J* = 1.1 Hz, 1H), 5.16 - 5.13 (m, 1H), 3.84 - 3.81 (m, 2H), 3.75 - 3.73 (m, 1H), 3.69 - 3.64 (m, 2H), 3.50 - 3.47 (m, 1H), 3.15 - 3.13 (m, 1H), 3.06 - 3.03 (m, 1H), 3.75 - 3.71 (m, 1H), 2.52 (s, 3H), 2.24 - 2.22 (m, 5H), 2.15 - 2.12 (m, 2H), 1.54 - 1.50 (m, 4H).

**Example 25.**

Synthesis of (2*R*,5*S*)-*N'*-acetyl-5-(4-chlorobenzyl)-4-(4-(5-methyloxazol-2-yl)cyclohexyl)morpholine-2-carbohydrazide 2,2,2-trifluoroacetate (**25**).

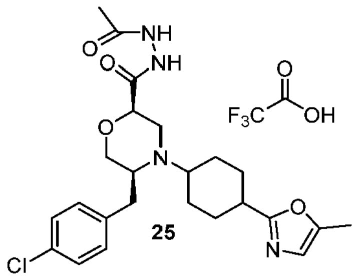

**25**

The title compound (**25**) was obtained during the synthesis of compound 24 as a by-product as a TFA salt as a single isomer from **22d** (118 mg; 0.400 mmol) and ketone **1g** (76 mg; 0.420 mmol) according to the General Procedure **VII** in 2% yield (4 mg; 0.007 mmol). The crude product was purified by silica-gel column chromatography (hexane/AcOEt, 3:4, *v/v*, then AcOEt, 100%, then: AcOEt/MeOH, 30:1 to 5:1, *v/v)* and then purified twice by preparative reversed-phase column chromatography (column: Cosmosil Cholester 20 x 250 mm, water/MeCN + 1‰ TFA, 95:5 to 20:80, 30 min, 20 mL/min).
ESI-MS m/z for $C_{24}H_{32}ClN_4O_4$ found 475.4/477.4 $[M+H]^+$; Rt = 0.93 min (Method A)

**Example 26.**

Synthesis of (2*R*,5*S*)-5-(4-chlorobenzyl)-4-(4-(4,5-dimethyloxazol-2-yl)cyclohexyl)-2-((methylsulfonyl)methyl)morpholine hydrochloride (**26**).

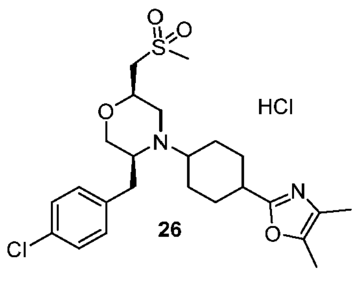

**26**

Step 1.

Synthesis of *N*-(but-3-yn-2-yl)-1,4-dioxaspiro[4.5]decane-8-carboxamide (**26a**).

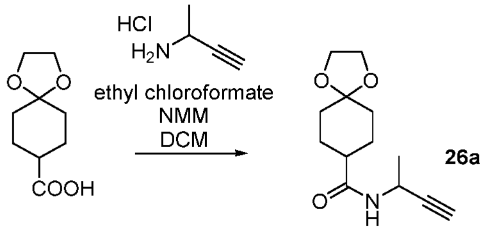


26a

The title compound (**26a**) was obtained from 1,4-dioxaspiro[4.5]decane-8-carboxylic acid (802 mg; 4.30 mmol) and but-3-yn-2-amine hydrochloride (0.50 g; 4.73 mmol) according to the General Procedure **V** in 99% yield (1.01 g; 4.26 mmol).
ESI-MS m/z for $C_{13}H_{20}NO_3$ found 238.2 $[M+H]^+$; $R_t$ = 0.67 min (Method A)

Step 2.

Synthesis of 4,5-dimethyl-2-(1,4-dioxaspiro[4.5]decan-8-yl)oxazole (**26b**).

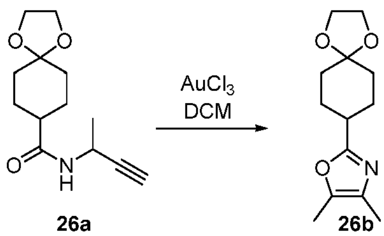


26a 26b

To the solution of **26a** (1.01 g; 4.26 mmol) in DCM (20 mL) placed into a reactor $AuCl_3$ (66 mg; 0.21 mmol) was added and whole was stirred at room temperature overnight. The reaction progress was monitored by LC-MS analysis. After analytical control indicated completion of the reaction, a whole was concentrated *in vacuo*. The crude product was purified by silica-gel column chromatography (hexane/AcOEt, 10:1 to 5:3, v/v). Compound **26b** was obtained in 65% yield (655 mg; 2.76 mmol).
ESI-MS m/z for $C_{13}H_{20}NO_3$ found 238.2 $[M+H]^+$; Rt = 1.04 min (Method A); $^1H$ NMR (700 MHz, DMSO-$d_6$ + $D_2O$) δ 3.84 - 3.83 (m, 4H), 2.76 - 2.69 (m, 1H), 2.14 (s, 3H), 1.93 (s, 3H), 1.91 - 1.86 (m, 2H), 1.70 - 1.63 (m, 4H), 1.57 - 1.51 (m, 2H).

Step 3.

Synthesis of 4-(4,5-dimethyloxazol-2-yl)cyclohexan-1-one (**26c**).

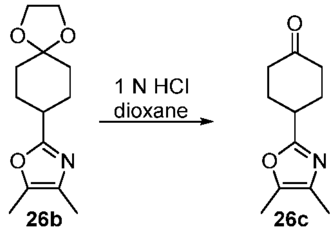


26b 26c

The solution of **26b** (655 mg; 2.76 mmol) and 1 N HCl (15 mL) in dioxane (10 mL) was stirred at room temperature for 3 hours. The reaction progress was monitored by LC-MS. When analysis indicated completion of the reaction, dioxane was evaporated *in vacuo* and to this mixture an aqueous solution of NaOH (4 M) was added to pH 12 and then extracted with DCM (2 x). The combined organic solutions were dried over anhydrous $MgSO_4$, filtered and concentrated *in vacuo*. The crude product was used to the next step without additional purification. Compound **26c** was obtained in 93% yield (495 mg; 2.56 mmol).
ESI-MS m/z for $C_{11}H_{16}NO_2$ found 194.2 $[M+H]^+$; Rt = 0.83 min (Method A); $^1H$ NMR (700 MHz, DMSO-$d_6$ + $H_2O$, 348 K) δ 3.21 - 3.15 (m, 1H), 2.48 - 2.39 (m, 2H), 2.35 - 2.28 (m, 2H), 2.24 - 2.19 (m, 2H), 2.16 (s, 3H), 1.96 (s, 3H), 1.96 - 1.89 (m, 2H).

Step 4.

Synthesis of (2*R*,5*S*)-5-(4-chlorobenzyl)-4-(4-(4,5-dimethyloxazol-2-yl)cyclohexyl)-2-((methylsulfonyl)methyl)morpholine hydrochloride (**26**).

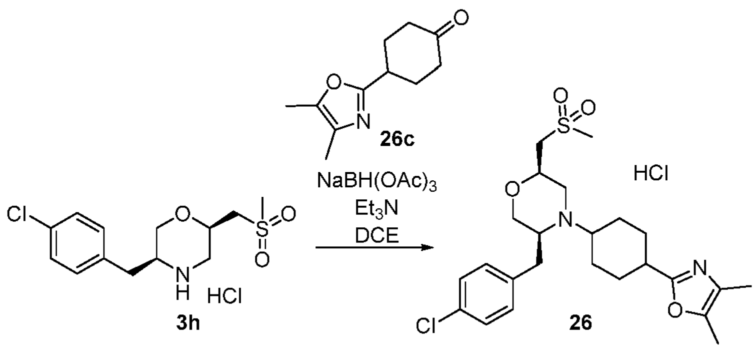


The title compound (**26**) was obtained as a single isomer as a hydrochloride salt from **3h** (173 mg; 0.51 mmol) and ketone **26c** (95 mg; 0.49 mmol) according to the General Procedure **VII** in 14% yield (36 mg; 0.07 mmol). The crude product was purified by silica-gel column chromatography (hexane/AcOEt, 1:1, v/v, then AcOEt, 100%, then: AcOEt/MeOH, 90:1 to 10:1, v/v) and then purified twice by preparative reversed-phase column chromatography (column: Cosmosil Cholester 20 x 250 mm, water + 0.3‰ HCl (36%)/MeCN, 90:10 to 10:90, 30 min, 20 mL/min).
ESI-MS m/z for $C_{24}H_{35}ClN_2O_4S$ found 481.1/483.1 $[M+H]^+$; Rt = 1.03 min (Method A); $^1H$ NMR (700 MHz, DMSO-$d_6$ + $D_2O$) δ 7.37 (d, $J_{AA'}$ = 8.4 Hz, 2H), 7.31 (d, $J_{BB'}$ = 8.4 Hz, 2H), 4.33 - 4.30 (m, 1H), 3.84 - 3.82 (m, 1H), 3.79 - 3.77 (m, 1H), 3.69 - 3.67 (m, 1H), 3.59 - 3.54 (m, 2H), 3.49 - 3.46 (m, 1H), 3.42 - 3.39 (m, 1H), 3.32 - 3.28 (m, 1H), 3.19 (dd, *J* = 13.8 Hz, *J* = 10.8 Hz, 1H), 3.12 (dd, *J* = 13.8 Hz, *J* = 4.0 Hz, 1H), 3.02 (s, 3H), 2.69 (tt, *J* = 10.7 Hz, *J* = 3.1 Hz, 1H), 2.29 - 2.26 (m, 2H), 2.17 - 2.14 (m, 5H), 1.95 (d, *J*' = 0.8 Hz, 3H), 1.62 - 1.52 (m, 4H).

**Example 27.**

Synthesis of *N*-(((2*S*,5*S*)-5-(4-chlorobenzyl)-4-(4-(1,5-dimethyl-1H-pyrazol-3-yl)cyclohexyl)morpholin-2-yl)methyl)-1,5-dimethyl-1*H*-pyrazole-3-carboxamide hydrochloride (**27**).

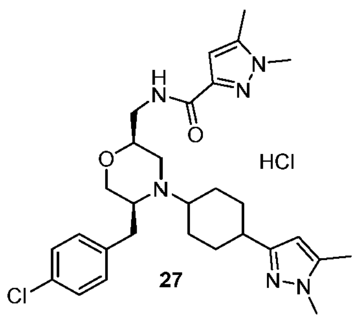

Step 1.

Synthesis of (2*R*,5*S*)-*tert*-butyl 2-(azidomethyl)-5-(4-chlorobenzyl)morpholine-4-carboxylate (**27a**).

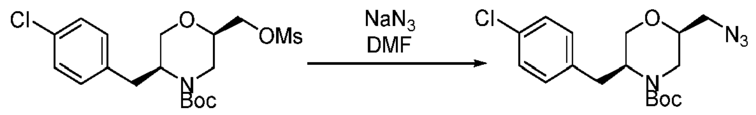


**3e** **27a**

To a solution of **3e** (0.4 g; 0.95 mmol) in DMF (8 mL) $NaN_3$ (0.19 g; 2.86 mmol) was added and the mixture was heated at 50 °C overnight. The reaction progress was monitored by LC-MS. When analysis indicated completion of the reaction, the reaction mixture was taken into water and AcOEt. An organic layer was washed with brine and then dried over anhydrous $MgSO_4$, filtered and concentrated *in vacuo* and the crude product mixture was used to the next step without additional purification. Compound **27a** was obtained in 99% yield (344 mg; 0.94 mmol).
ESI-MS m/z for $C_{17}H_{24}ClN_4O_3$ found 367.2/369.2 $[M+H]^+$; Rt = 6.60 min (Method C)

Step 2.

Synthesis of *tert-butyl* (2*S*,5*S*)-2-(aminomethyl)-5-(4-chlorobenzyl)morpholine-4-carboxylate (**27b**).

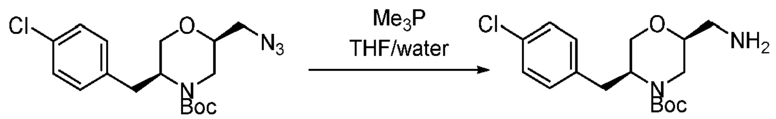


**27a** **27b**

An obtained azide **27a** (225 mg; 0.61 mmol) was dissolved in $THF/H_2O$ (10 mL; 9:1, *v/v*) and then 1 M solution of $Me_3P$ in THF (1 mL; 0.92 mmol) was added and a whole was stirred at room temperature for 2 hours. The reaction progress was monitored by LC-MS. When analysis indicated completion of the reaction, the reaction was quenched with 5% $NaHCO_3$ and a whole was extracted with diethyl ether (2 x). The combined organic solutions were washed with water and then dried over anhydrous $MgSO_4$, filtered and concentrated *in vacuo.* The crude product mixture was used to the next step without additional purification. Compound **27b** was obtained in 90% yield (187 mg; 0.55 mmol).
ESI-MS m/z for $C_{17}H_{26}ClN_2O_3$ found 341.0/343.0 $[M+H]^+$; $R_t$ = 1.06 min (Method A)

Step 3.

Synthesis of *tert*-butyl (2*S*,5*S*)-5-(4-chlorobenzyl)-2-((1,5-dimethyl-1*H*-pyrazole-3-carboxamido)methyl)morpholine-4-carboxylate (**27c**).

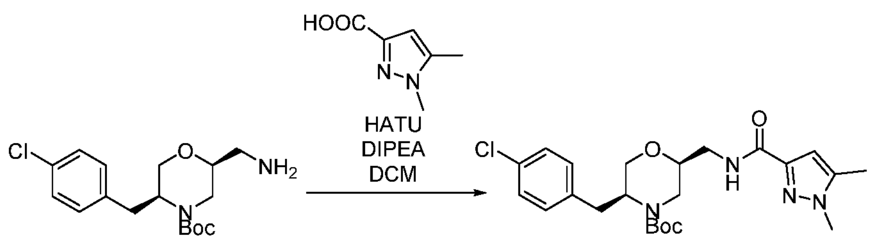


**27b** **27c**

To the solution of **27b** (187 mg; 0.55 mmol) in dry DCM (3 mL) diisopropylethylamine (DIPEA; 0.14 mL; 0.83 mmol), 1,5-dimethyl-1*H*-pyrazole-3-carboxylic acid (84 mg; 0.60 mmol) and HATU (228 mg; 0.60 mmol) were added and the reaction mixture was stirred at room temperature overnight. The reaction progress was monitored by TLC and LC-MS. When analyses indicated completion of the reaction, the mixture was diluted with DCM and washed with an aqueous solution of HCl, and aqueous solution of NaOH, water and brine. The organic phase was dried over anhydrous $MgSO_4$, filtered and concentrated *in vacuo.* The crude product was purified by flash column chromatography on silica (hexane/-MeCN, 100:0 to 2:8, *v/v*). Compound **27c** was obtained in 96% yield (247 mg; 0.53 mmol).
ESI-MS m/z for $C_{23}H_{32}ClN_4O_4$ found 463.2/465.1 $[M+H]^+$; Rt = 1.58 min (Method A); $^1H$ NMR (700 MHz, DMSO-$d_6$ + $D_2O$, 348 K) δ 7.29 - 7.26 (m, 2H), 7.20 - 7.16 (m, 2H), 6.41 (s, 1H), 4.08 - 4.02 (m, 1H), 3.80 - 3.68 (m, 5H), 3.53 - 3.45 (m, 2H),

2.97 - 2.90 (m, 1H), 2.90 - 2.80 (m, 2H), 2.71 - 2.70 (m, 1H), 2.70 - 2.69 (m, 1H), 2.25 (s, 3H), 1.21 (s, 9H).

Step 4.

Synthesis of *N*-(((2*R*,5*S*)-5-(4-chlorobenzyl)morpholin-2-yl)methyl)-1,5-dimethyl-1H-pyrazole-3-carboxamide (**27d**).

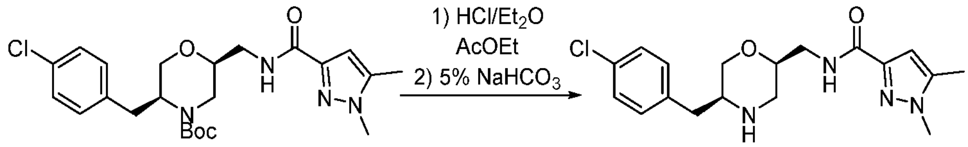


**27c** **27d**

The title compound (**27d**) was obtained as a free amine in 77% yield (147 mg; 0.41 mmol) from **27c** (247 mg; 0.53 mmol) according to the General Procedure **IVa** and then after basic (5% $NaHCO_3$) extraction with DCM.
ESI-MS m/z for $C_{18}H_{24}ClN_4O_2$ found 363.2/365.0 $[M+H]^+$; $R_t$ = 0.83 min (Method A)

Step 5.

Synthesis of *N*-(((2*S*,5*S*)-5-(4-chlorobenzyl)-4-(4-(1,5-dimethyl-1*H*-pyrazol-3-yl)cyclohexyl)morpholin-2-yl)methyl)-1,5-dimethyl-1*H*-pyrazole-3-carboxamide hydrochloride (**27**).

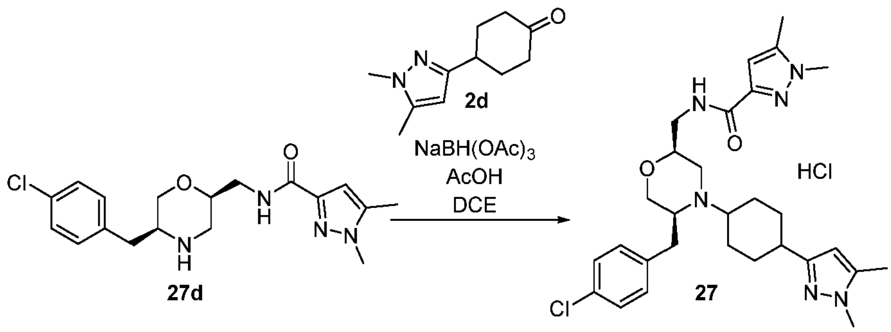


**27d** **27**

The title compound (**27**) was obtained as a single isomer as a hydrochloride salt from **27d** (144 mg; 0.40 mmol) and ketone **2d** (85 mg; 0.44 mmol) according to the General Procedure **VII** in 17% yield (43 mg; 0.07 mmol). The crude product was purified by silica-gel column chromatography (hexane/AcOEt, 1:1, v/v, then AcOEt, 100%, then: AcOEt/-MeOH, 25:1 to 9:1, v/v) and then purified by preparative reversed-phase column chromatography (column: Cosmosil Cholester 20 x 250 mm, water + 0.3‰ HCl (36%)/MeCN, 95:5 to 20:80, 30 min, 20 mL/min). ESI-MS m/z for $C_{29}H_{40}ClN_6O_2$ found 539.0/541.0 $[M+H]^+$; Rt = 1.06 min (Method A); $^1H$ NMR (700 MHz, DMSO-$d_6$ + $D_2O$) δ 7.40 (d, $J_{AA'}$ = 8.5 Hz, 2H), 7.31 (d, $J_{BB'}$ = 8.5 Hz, 2H), 6.44 (s, 1H), 5.86 (s, 1H), 3.90 - 3.87 (m, 1H), 3.80 - 3.78 (m, 1H), 3.73 (s, 3H), 3.68 - 3.66 (m, 1H), 3.60 (s, 3H), 3.59 - 3.53 (m, 2H), 3.47 - 3.38 (m, 3H), 3.11 - 3.03 (m, 3H), 2.47 - 2.43 (m, 1H), 2.26 - 2.23 (m, 5H), 2.16 (m, 3H), 2.02 - 1.99 (m, 2H), 1.53 - 1.37 (m, 4H).

**Example 28.**

Synthesis of (2*S*,5*S*)-5-(4-chlorobenzyl)-4-(4-(1,5-dimethyl-1*H*-pyrazol-3-yl)cyclohexyl)-2-ethynylmorpholine 2,2,2-trifluoroacetate (**28**).

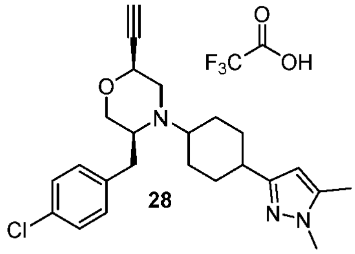

28

Step 1.

Synthesis of (2*S*,5*S*)-5-(4-chlorobenzyl)-2-ethynylmorpholine (**28a**).

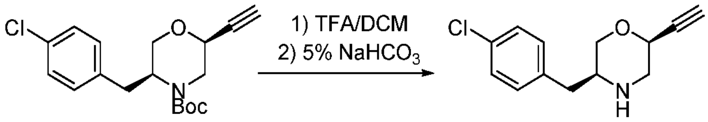


12b 28a

The title compound (**28a**) was obtained as a free amine in 99% yield (313 mg; 1.33 mmol) from **12c** (448 mg; 1.34 mmol) according to the General Procedure **IVb** and then after basic (5% $NaHCO_{3aq}$) extraction with DCM.
ESI-MS m/z for $C_{13}H_{15}ClNO$ found 236.1/238.0 $[M+H]^+$; Rt = 0.66 min (Method A)

Step 2.

Synthesis of (2*S*,5*S*)-5-(4-chlorobenzyl)-4-(4-(1,5-dimethyl-1*H*-pyrazol-3-yl)cyclohexyl)-2-ethynylmorpholine 2,2,2-trifluoroacetate (**28**).

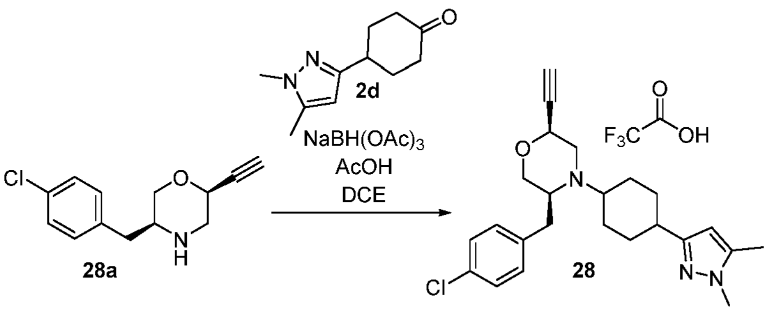


28a 28

The title compound (**28**) was obtained as a single isomer as a TFA salt from **28a** (313 mg; 1.330 mmol) and ketone **2d** (280 mg; 1.450 mmol) according to the General Procedure **VII** in 4% yield (30 mg; 0.057 mmol). The crude product was purified by silica-gel column chromatography (hexane/AcOEt, 6:1 to 1:4, *v*/*v*) and then purified by preparative reversed-phase column chromatography (column: Cosmosil Cholester 20 x 250 mm, water/MeCN + 1‰ TFA, 95:5 to 10:90, 30 min, 20 mL/min).
ESI-MS m/z for $C_{24}H_{31}ClN_3O$ found 412.4/414.3 $[M+H]^+$; Rt = 1.06 min (Method A); $^1H$ NMR (700 MHz, DMSO-$d_6$ + $D_2O$, 348 K) δ 7.37 (d, $J_{AA'}$ = 8.4 Hz, 2H), 7.30 (d, $J_{BB'}$ = 8.4 Hz, 2H), 5.80 (s, 1H), 4.69 - 4.68 (m, 1H), 3.73 - 3.69 (m, 3H), 3.60 (s, 3H), 3.50 - 3.48 (m, 2H), 3.44 - 3.41 (m, 1H), 3.36 - 3.33 (m, 1H), 3.16 - 3.13 (m, 1H), 3.04 (dd, *J* = 14.0 Hz, *J* = 9.5 Hz, 1H), 2.45 (tt, *J* = 12.1 Hz, *J* = 3.7 Hz, 1H), 2.18 - 2.16 (m, 4H), 2.12 - 2.10 (m, 1H), 2.05 - 1.98 (m, 2H), 1.58 - 1.47 (m, 2H), 1.43 (qd, *J* = 13.0 Hz, *J* = 3.3 Hz, 1H), 1.36 (qd, *J* = 13.0 Hz, *J* = 3.3 Hz, 1H).

**Example 29.**

Synthesis of *N*-(35-(4-((2*R*,5*S*)-5-(4-chlorobenzyl)-4-(4-(1,5-dimethyl-1*H*-pyrazol-3-yl)cyclohexyl)morpholin-2-yl)-1*H*-1,2,3-triazol-1-yl)-3,6,9,12,15,18,21,24,27,30,33-undecaoxapentatriacontyl)-5-((3*aS*,4*S*,6*aR*)-2-oxohexahydro-1*H*-thieno[3,4-*d*]imidazol-4-yl)pentanamide 2,2,2-trifluoroacetate (**29**).

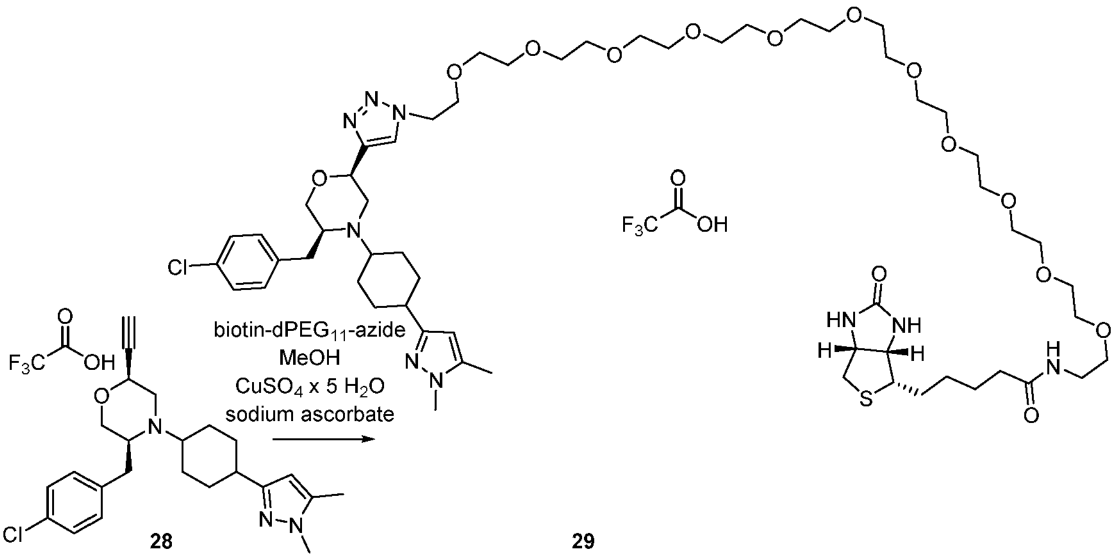


28 29

To the solution of **28** (22 mg; 0.054 mmol) in MeOH (0.5 mL) $CuSO_4$ x 5 $H_2O$ (6.3 mg; 0.025 mmol) was added and after 5 minutes to a light green solution a sodium L-ascorbate (10 mg; 0.050 mmol) was added in one portion. A whole was stirred for 15 minutes at room temperature and then a solution of an azide (39 mg; 0.049 mmol) in MeOH (0.5 mL) was added and the reaction mixture was stirred at room temperature overnight. After analytical control (LC-MS) indicated completion of the reaction, the reaction mixture was concentrated *in vacuo* and the residue was mixed with water/MeCN and TFA and then this mixture was filtered and purified by preparative reversed-phase column chromatography (column: Cosmosil Cholester 20 x 250 mm, water/MeCN + 1‰ TFA, 95:5 to 10:90, 30 min, 20 mL/min). Compound **29** was obtained as a single isomer as a TFA salt in 41% yield (27 mg; 0.020 mmol).
ESI-MS m/z for $C_{58}H_{95}ClN_9O_{14}S$ found 1208.5/1210.0 $[M+H]^+$; Rt = 1.08 min (Method A)

**Example 30.**

Synthesis of (2*R*,5*S*)-5-(4-chlorobenzyl)-4-(4-(1,5-dimethyl-1*H*-pyrazol-3-yl)cyclohexyl)-N-isopropylmorpholine-2-carboxamide hydrochloride (**30**).

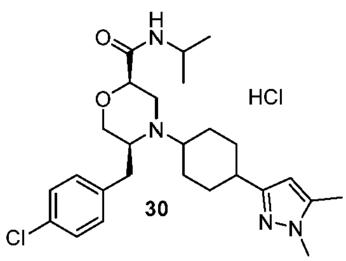

30

Step 1.

Synthesis of *tert*-butyl (2*R*,5*S*)-5-(4-chlorobenzyl)-2-(isopropylcarbamoyl)morpholine-4-carboxylate (**30a**).

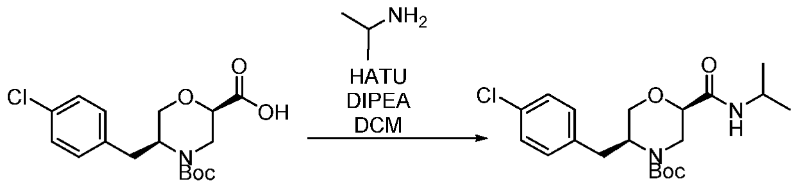

5a
30a

To the solution of **5a** (2.5 g; 7.03 mmol) in dry DCM (30 mL) diisopropylethylamine (DIPEA; 1.8 mL; 10.50 mmol), propan-2-amine (0.72 mL; 8.43 mmol) and HATU (3 g; 7.70 mmol) were added and the reaction mixture was stirred at room temperature overnight. The reaction progress was monitored by TLC and LC-MS. When analyses indicated completion of the reaction, the mixture was diluted with DCM and washed with an aqueous solution of HCl, and aqueous solution of NaOH, water and brine. The organic phase was dried over anhydrous $MgSO_4$, filtered and concentrated *in vacuo.* The crude product was purified by flash column chromatography on silica (hexane/AcOEt, 100:0 to 4:6, v/v). Compound **30a** was obtained in 99% yield (2.76 g; 6.96 mmol).
ESI-MS m/z for $C_{20}H_{30}ClN_2O_4$ found 397.1/399.0 $[M+H]^+$; Rt = 1.69 min (Method A)

Step 2.

Synthesis of (2*R*,5*S*)-5-(4-chlorobenzyl)-*N*-isopropylmorpholine-2-carboxamide (**30b**).

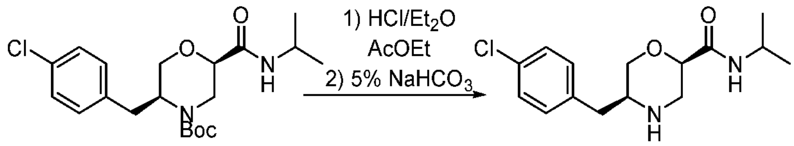

30a
30b

The title compound (**30b**) was obtained as a free amine in 86% yield (1.78 g; 6.01 mmol) from **27c** (2.76 g; 6.96 mmol) according to the General Procedure **IVa** and then after basic (5% $NaHCO_3$) extraction with DCM.
ESI-MS m/z for $C_{15}H_{22}ClN_2O_2$ found 297.2/299.0 $[M+H]^+$; Rt = 0.80 min (Method A); $^1$H NMR (700 MHz, DMSO-$d_6$ + $D_2O$, 348 K) δ 7.30 - 7.27 (m, 2H), 7.23 - 7.20 (m, 2H), 3.93 - 3.87 (m, 1H), 3.85 - 3.81 (m, 1H), 3.61 - 3.55 (m, 1H), 3.52 - 3.47 (m, 1H), 3.06 - 2.99 (m, 1H), 2.89 - 2.83 (m, 1H), 2.83 - 2.78 (m, 1H), 2.74 - 2.69 (m, 2H), 1.12 - 1.06 (m, 6H).

Step 3.

Synthesis of (2*R*,5*S*)-5-(4-chlorobenzyl)-4-(4-(1,5-dimethyl-1*H*-pyrazol-3-yl)cyclohexyl)-*N*-isopropylmorpholine-2-carboxamide hydrochloride (**30**).

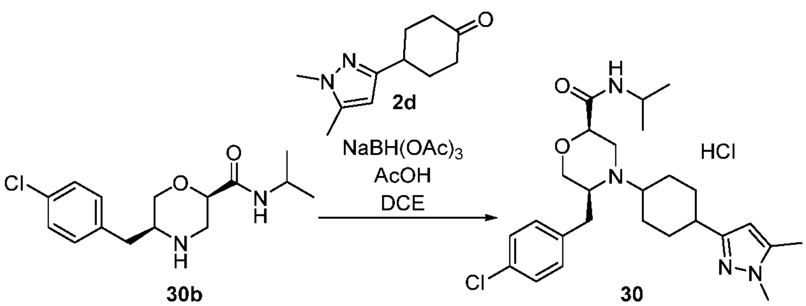

30b
30

The title compound (**30**) was obtained as a single isomer as a hydrochloride salt from **30b** (119 mg; 0.400 mmol) and ketone **2d** (85 mg; 0.440 mmol) according to the General Procedure **VII** in 24% yield (49 mg; 0.096 mmol). The crude product was purified by silica-gel column chromatography (hexane/AcOEt, 1:1, *v*/*v*, then AcOEt, 100%, then: AcOEt/-MeOH, 70:1 to 10:1, v/v) and then purified by preparative reversed-phase column chromatography (column: Cosmosil Cholester 20 x 250 mm, water + 0.3‰ HCl (36%)/MeCN, 95:5 to 20:80, 30 min, 20 mL/min).

ESI-MS m/z for $C_{26}H_{38}ClN_4O_2$ found 473.5/475.5 $[M+H]^+$; Rt = 1.09 min (Method A); $^1H$ NMR (700 MHz, DMSO-$d_6$ + $D_2O$, 348 K) δ 7.38 (d, $J_{AA'}$ = 8.5 Hz, 2H), 7.33 (d, $J_{BB'}$ = 8.5 Hz, 2H), 5.80 (s, 1H), 4.29 - 4.28 (m, 1H), 3.92 (hept, *J* = 6.7 Hz, 1H), 3.80 - 3.79 (m, 1H), 3.74 - 3.72 (m, 2H), 3.60 (s, 3H), 3.53 - 3.51 (m, 1H), 3.45 - 3.41 (m, 1H), 3.35 - 3.33 (m, 1H), 3.13 - 3.07 (m, 2H), 2.48 - 2.44 (m, 2H), 2.22 - 2.19 (m, 1H), 2.16 - 2.13 (m, 4H), 2.06 - 2.00 (m, 2H), 1.61 - 1.51 (m, 2H), 1.46 - 1.36 (m, 2H), 1.14 (d, *J* = 6.6 Hz, 3H), 1.11 (d, *J* = 6.6 Hz, 3H).

**Example 31.**

Synthesis of (2*R*,5*S*)-5-(4-chlorobenzyl)-4-(4-(1,5-dimethyl-1*H*-pyrazol-3-yl)cyclohexyl)-*N*-methylmorpholine-2-carboxamide dihydrochloride (**31**).

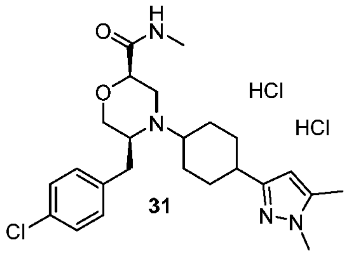

**31**

The title compound (**31**) was obtained as a dihydrochloride salt as a single isomer in 2% overall yield in a similar way to Example 30 with the exception that, in the first step of the synthesis, methylamine was used instead of propan-2-amine. The crude product was purified by silica-gel column chromatography (hexane/AcOEt, 1:1, *v*/*v*, then AcOEt, 100%, then: AcOEt/MeOH, 100:1 to 70:4, *v*/*v*) and then purified by preparative reversed-phase column chromatography (column: Cosmosil Cholester 20 x 250 mm, water + 0.3‰ HCl (36%)/MeCN, 95:5 to 20:80, 30 min, 20 mL/min).
ESI-MS m/z for $C_{24}H_{34}ClN_4O_2$ found 445.4/447.2 $[M+H]^+$; Rt = 0.98 min (Method A); $^1H$ NMR (700 MHz, DMSO-$d_6$ + $D_2O$, 348 K) δ 7.37 (d, $J_{AA'}$ = 8.4 Hz, 2H), 7.34 (d, $J_{BB'}$ = 8.4 Hz, 2H), 5.81 (s, 1H), 4.35 - 4.33 (m, 1H), 3.82 - 3.80 (m, 1H), 3.77 - 3.75 (m, 1H), 3.73 - 3.71 (m, 1H), 3.60 (s, 3H), 3.57 - 3.55 (m, 1H), 3.47 - 3.43 (m, 1H), 3.36 - 3.32 (m, 1H), 3.12 - 3.08 (m, 2H), 2.69 (s, 3H), 2.49 - 2.45 (m, 1H), 2.23 - 2.16 (m, 5H), 2.07 - 2.01 (m, 2H), 1.63 - 1.54 (m, 2H), 1.47 - 1.37 (m, 2H).

**Example 32.**

Synthesis of (2*R*,5*S*)-5-(4-chlorobenzyl)-4-(4-(4,5-dimethyloxazol-2-yl)cyclohexyl)-*N*,*N*-dimethylmorpholine-2-carboxamide hydrochloride (**32**).

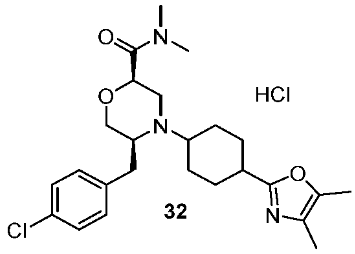

**32**

The title compound (**32**) was obtained as a hydrochloride salt as a single isomer in 8% overall yield in a similar way to Example 30 with the exception that, in the first step of the synthesis, dimethylamine hydrochloride was used instead of propan-2-amine and in the third step of the synthesis, ketone **26c** was used instead of ketone **2d.** The crude product was purified by silica-gel column chromatography (hexane/AcOEt, 3:4, *v*/*v*, then AcOEt, 100%, then: AcOEt/MeOH, 140:1 to 70:4, *v*/*v*) and then purified by preparative reversed-phase column chromatography (column: Cosmosil Cholester 20 x 250 mm, water + 0.3‰ HCl (36%)/MeCN, 95:5 to 20:80, 30 min, 20 mL/min).
ESI-MS m/z for $C_{25}H_{35}ClN_3O_3$ found 460.5/462.3 $[M+H]^+$; Rt = 1.06 min (Method A); $^1H$ NMR (700 MHz, DMSO-$d_6$ + $D_2O$, 348 K) δ 7.37 (d, $J_{AA'}$ = 8.5 Hz, 2H), 7.31 (d, $J_{BB'}$ = 8.5 Hz, 2H), 4.84 - 4.82 (m, 1H), 3.96 - 3.94 (m, 1H), 3.83 - 3.80 (m, 1H), 3.63 - 3.57 (m, 2H), 3.53 - 3.50 (m, 1H), 3.44 - 3.42 (m, 1H), 3.22 - 3.20 (m, 1H), 3.06 - 3.04 (m, 4H), 3.89 (brs, 3H), 2.69 (tt, *J* = 12.2 Hz, *J* = 3.6 Hz, 1H), 2.25 - 2.24 (m, 1H), 2.17 - 2.11 (m, 6H), 1.95 (s, 3H), 1.75 - 1.63 (m, 2H), 1.59 - 1.46 (m, 2H).

**Example 33.**

Synthesis of (2*R*,5*S*)-5-(4-chlorobenzyl)-4-(4-(4,5-dimethyloxazol-2-yl)cyclohexyl)-*N*-isopropylmorpholine-2-carboxamide hydrochloride (**33**).

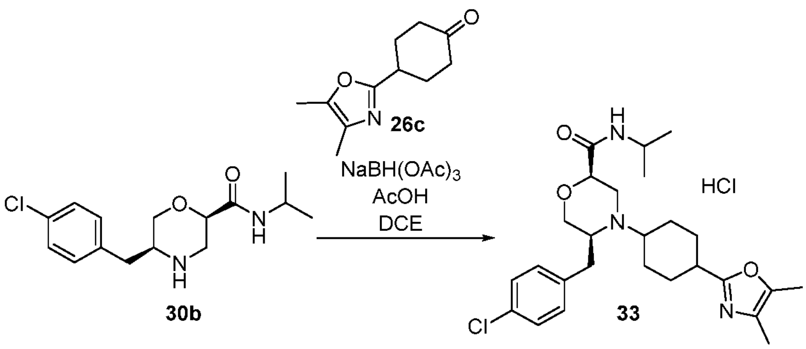


30b

33

The title compound (**33**) was obtained as a single isomer as a hydrochloride salt from **30b** (157 mg; 0.529 mmol) and ketone **26c** (102 mg; 0.527 mmol) according to the General Procedure **VII** in 15% yield (40 mg; 0.078 mmol). The crude product was purified by silica-gel column chromatography (hexane/AcOEt, 3:4, *v*/*v*, then AcOEt, 100%, then: AcOEt/-MeOH, 140:1 to 20:1, v/v) and then purified by preparative reversed-phase column chromatography (column: Cosmosil Cholester 20 x 250 mm, water + 0.3‰ HCl (36%)/MeCN, 95:5 to 20:80, 30 min, 20 mL/min).
ESI-MS m/z for $C_{26}H_{37}ClN_3O_3$ found 474.4/476.4 $[M+H]^+$; Rt = 1.14 min (Method A); $^1$H NMR (700 MHz, DMSO-$d_6$ + $D_2O$, 348 K) δ 7.38 (d, $J_{AA'}$ = 8.5 Hz, 2H), 7.33 (d, $J_{BB'}$ = 8.5 Hz, 2H), 4.32 - 4.30 (m, 1H), 3.92 (hept, *J* = 6.6 Hz, 1H), 3.83 - 3.81 (m, 1H), 3.76 - 3.72 (m, 2H), 3.54 - 3.52 (m, 1H), 3.49 - 3.46 (m, 1H), 3.34 - 3.30 (m, 1H), 3.14 - 3.08 (m, 2H), 2.68 (tt, *J* = 12.0 Hz, *J* = 3.6 Hz, 1H), 2.26 - 2.23 (m, 1H), 2.20 - 2.11 (m, 6H), 1.94 (s, 3H), 1.65 - 1.48 (m, 4H), 1.13 (d, *J* = 6.6 Hz, 3H), 1.11 (d, *J* = 6.6 Hz, 3H).

**Example 34.**

Synthesis of (2*R*,5*S*)-5-(4-chlorobenzyl)-*N*-methyl-4-(4-(5-methyloxazol-2-yl)cyclohexyl)morpholine-2-carboxamide hydrochloride (**34**).

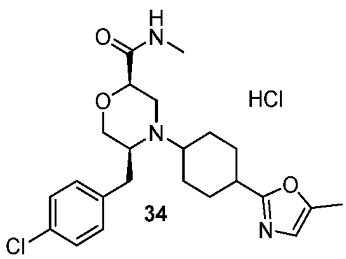


34

The title compound (**34**) was obtained as a hydrochloride salt as a single isomer in 4% overall yield in a similar way to Example 30 with the exception that, in the first step of the synthesis, methylamine was used instead of propan-2-amine and in the third step of the synthesis, ketone **1g** was used instead of ketone **2d.** The crude product was purified by silica-gel column chromatography (AcOEt/MeOH, 100:1 to 10:1, *v*/*v*) and then purified by preparative reversed-phase column chromatography (column: Cosmosil Cholester 20 x 250 mm, water + 0.3‰ HCl (36%)/MeCN, 95:5 to 30:70, 30 min, 20 mL/min).
ESI-MS m/z for $C_{23}H_{31}ClN_3O_3$ found 432.4/434.2 $[M+H]^+$; Rt = 0.97 min (Method A); $^1$H NMR (700 MHz, DMSO-$d_6$ + $D_2O$, 348 K) δ 7.38 (d, $J_{AA'}$ = 8.4 Hz, 2H), 7.35 (d, $J_{BB'}$ = 8.5 Hz, 2H), 6.66 (s, 1H), 4.44 (dd, *J* = 10.4 Hz, *J* = 3.0 Hz, 1H), 3.92 (d, *J* = 13.2 Hz, 1H), 3.81 - 3.79 (m, 1H), 3.72 (dd, *J* = 13.2 Hz, *J* = 1.9 Hz, 1H), 3.60 - 3.58 (m, 1H), 3.54 - 3.51 (m, 1H), 3.35 - 3.33 (m, 1H), 3.17 - 3.10 (m, 2H), 2.74 (tt, *J* = 12.3 Hz, *J* = 3.7 Hz, 1H), 2.69 (s, 3H), 2.28 - 2.23 (m, 5H), 2.20 - 2.15 (m, 2H), 1.76 - 1.66 (m, 2H), 1.59 - 1.52 (m, 2H).

**Example 35.**

Synthesis of (2*R*,5*S*)-5-(4-chlorobenzyl)-4-(4-(4,5-dimethyloxazol-2-yl)cyclohexyl)-*N*-ethylmorpholine-2-carboxamide hydrochloride (**35**).

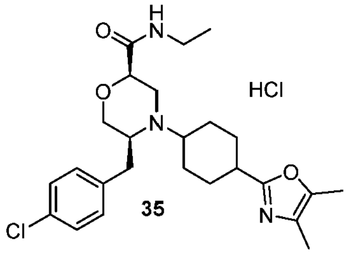

HCl

**35**

The title compound (**35**) was obtained as a hydrochloride salt as a single isomer in 1% overall yield in a similar way to Example 30 with the exception that, in the first step of the synthesis, ethylamine hydrochloride was used instead of propan-2-amine and in the third step of the synthesis, ketone **26c** was used instead of ketone **2d.** The crude product was purified by silica-gel column chromatography (hexane/AcOEt, 1:1, *v*/*v*, then AcOEt, 100%, then: AcOEt/MeOH, 60:1 to 6:1, *v*/*v*) and then purified by preparative reversed-phase column chromatography (column: Cosmosil Cholester 20 x 250 mm, water + 0.3‰ HCl (36%)/MeCN, 95:5 to 30:70, 30 min, 20 mL/min).
ESI-MS m/z for $C_{25}H_{35}ClN_3O_3$ found 460.4/462.4 $[M+H]^+$; Rt = 1.10 min (Method A); $^1$H NMR (700 MHz, DMSO-$d_6$ + $D_2O$, 348 K) δ 7.38 (d, $J_{AA'}$ = 8.5 Hz, 2H), 7.35 (d, $J_{BB'}$ = 8.5 Hz, 2H), 4.39 - 4.37 (m, 1H), 3.89 - 3.87 (m, 1H), 3.80 - 3.78 (m, 1H), 3.74 - 3.72 (m, 1H), 3.59 - 3.57 (m, 1H), 3.54 - 3.50 (m, 1H), 3.35 - 3.32 (m, 1H), 3.23 - 3.10 (m, 4H), 2.69 (tt, *J* = 12.2 Hz, *J* = 3.6 Hz, 1H), 2.28 - 2.21 (m, 2H), 2.18 - 2.13 (m, 5H), 1.95 (brs, 3H), 1.71 - 1.61 (m, 2H), 1.54 (pd, *J* = 13.0 Hz, *J* = 3.2 Hz, 2H), 1.08 (t, *J* = 7.2 Hz, 3H).

**Example 36.**

Synthesis of (2*R*,5*S*)-5-(4-chlorobenzyl)-4-(4-(5-fluoro-1-methyl-1*H*-pyrazol-3-yl)cyclohexyl)-2-((methylsulfonyl) methyl)morpholine hydrochloride (**36**).

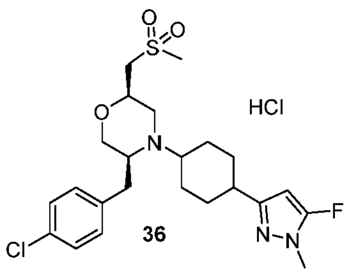

HCl

**36**

The title compound (**36**) was obtained as a hydrochloride salt as a single isomer in 14% overall yield in a similar way to Example 15 with the exception that, in the first step of the synthesis, 3-bromo-5-fluoro-1-methyl-1*H*-pyrazole was used instead of 3-bromo-1,5-dimethyl-1*H*-1,2,4-triazole and Pd(dppf) was used instead of $[PPh_3]_4Pd$. The crude product was purified by silica-gel column chromatography (hexane/AcOEt, 1:1, *v*/*v*, then AcOEt, 100%, then: AcOEt/MeOH, 80:1 to 20:1, *v*/*v*) and then purified by preparative reversed-phase column chromatography (column: Cosmosil Cholester 20 x 250 mm, water + 0.3‰ HCl (36%)/MeCN, 95:5 to 20:80, 30 min, 20 mL/min).
ESI-MS m/z for $C_{23}H_{32}ClFN_3O_3S$ found 484.2/486.2 $[M+H]^+$; $R_t$ = 0.99 min (Method A); $^1$H NMR (700 MHz, DMSO-$d_6$ + $D_2O$, 348 K) δ 7.38 (d, $J_{AA'}$ = 8.4 Hz, 2H), 7.32 (d, $J_{BB'}$ = 8.4 Hz, 2H), 5.71 (d, *J* = 5.7 Hz, 1H), 4.37 - 4.34 (m, 1H), 3.89 (d, *J* = 13.3 Hz, 1H), 3.79 - 3.77 (m, 1H), 3.67 (d, *J* = 13.3 Hz, 1H), 3.60 - 3.55 (m, 5H), 3.47 - 3.41 (m, 2H), 3.32 - 3.30 (m, 1H), 3.20 (dd, *J* = 13.8 Hz, *J* = 10.9 Hz, 1H), 3.14 - 3.11 (m, 1H), 3.02 (s, 3H), 2.49 - 2.45 (m, 1H), 2.27 - 2.24 (m, 2H), 2.07 - 2.04 (m, 2H), 1.65 - 1.59 (m, 2H), 1.48 - 1.41 (m, 2H); $^{19}$F NMR (250 MHz, DMSO-$d_6$ + $D_2O$) δ -135.3 (s).

**Example 37.**

Synthesis of (2*R*,5*S*)-5-(4-chlorobenzyl)-4-(4-(4,5-dimethylthiazol-2-yl)cyclohexyl)-2-((methylsulfonyl)methyl)morpholine hydrochloride (**37**).

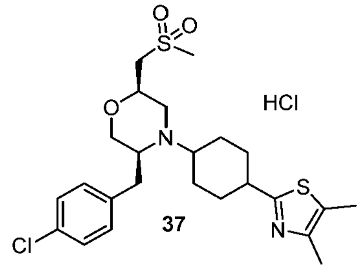

The title compound (**37**) was obtained as a hydrochloride salt as a single isomer in 9% overall yield in a similar way to Example 15 with the exception that, in the first step of the synthesis, 2-bromo-4,5-dimethylthiazole was used instead of 3-bromo-1,5-dimethyl-1*H*-1,2,4-triazole and Pd(dppf) was used instead of $[PPh_3]_4Pd$. The crude product was purified by silica-gel column chromatography (hexane/AcOEt, 1:1, *v*/*v*, then AcOEt, 100%, then: AcOEt/MeOH, 100:1 to 95:5, *v*/*v*) and then purified by preparative reversed-phase column chromatography (column: Cosmosil Cholester 20 x 250 mm, water + 0.3‰ HCl (36%)/MeCN, 95:5 to 10:90, 30 min, 20 mL/min).
ESI-MS m/z for $C_{24}H_{34}ClN_2O_3S_2$ found 497.4/499.4 $[M+H]^+$; $R_t$ = 1.07 min (Method A); $^1H$ NMR (700 MHz, DMSO-$d_6$ + $D_2O$, 348 K) δ 7.39 (d, $J_{AA'}$ = 8.4 Hz, 2H), 7.33 (d, $J_{BB'}$ = 8.4 Hz, 2H), 4.31 - 4.28 (m, 1H), 3.83 - 3.77 (m, 2H), 3.66 (d, *J* = 12.9 Hz, 1H), 3.61 - 3.56 (m, 2H), 3.51 - 3.48 (m, 1H), 3.42 - 3.40 (m, 1H), 3.30 - 3.26 (m, 1H), 3.19 (dd, *J* = 13.5 Hz, *J* = 11.0 Hz, 1H), 3.13 (dd, *J* = 13.8 Hz, *J* = 3.7 Hz, 1H), 3.03 (s, 3H), 2.88 (tt, *J* = 11.9 Hz, *J* = 3.7 Hz, 1H), 2.29 - 2.27 (m, 5H), 2.19 - 2.16 (m, 5H), 1.63 - 1.52 (m, 4H).

**Example 38.**

Synthesis of (2*R*,5*S*)-5-(4-chlorobenzyl)-2-((methylsulfonyl)methyl)-4-(4-(5-methylthiazol-2-yl)cyclohexyl)morpholine hydrochloride (**38**).

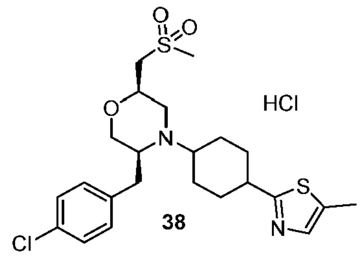

The title compound (**38**) was obtained as a hydrochloride salt as a single isomer in 11% overall yield in a similar way to Example 15 with the exception that, in the first step of the synthesis, 2-bromo-5-methylthiazole was used instead of 3-bromo-1,5-dimethyl-1*H*-1,2,4-triazole and Pd(dppf) was used instead of $[PPh_3]_4Pd$. The crude product was purified by silica-gel column chromatography (hexane/AcOEt, 5:1, *v*/*v*, then AcOEt, 100%) and then purified by preparative reversed-phase column chromatography (column: Cosmosil Cholester 20 x 250 mm, water + 0.3‰ HCl (36%)/MeCN, 90:10 to 20:80, 30 min, 20 mL/min).
ESI-MS m/z for $C_{23}H_{32}ClN_2O_3S_2$ found 483.0/485.0 $[M+H]^+$; Rt = 1.05 min (Method A); $^1H$ NMR (700 MHz, DMSO-$d_6$ + $D_2O$, 348 K) δ 7.38 (d, $J_{AA'}$ = 8.5 Hz, 2H), 7.33 - 7.32 (m, 3H), 4.30 - 4.27 (m, 1H), 3.80 - 3.76 (m, 2H), 3.66 (d, *J* = 12.6 Hz, 1H), 3.59 (dd, *J* = 15.0 Hz, *J* = 8.0 Hz, 1H), 3.56 - 3.54 (m, 1H), 3.49 - 3.46 (m, 1H), 3.40 - 3.39 (m, 1H), 3.29 - 3.25 (m, 1H), 3.18 (dd, *J* = 13.7 Hz, *J* = 10.8 Hz, 1H), 3.12 (dd, *J* = 13.8 Hz, *J* = 3.9 Hz, 1H), 3.03 (s, 3H), 2.94 - 2.90 (m, 1H), 2.38 (d, *J* = 1.2 Hz, 3H), 2.29 - 2.28 (m, 2H), 2.21 - 2.18 (m, 2H), 1.62 - 1.55 (m, 4H).

**Example 39.**

Synthesis of (2*R*,5*S*)-5-(4-chlorobenzyl)-4-(4-(4,5-dimethyloxazol-2-yl)cyclohexyl)morpholine-2-carboxylic acid hydrochloride (**39**).

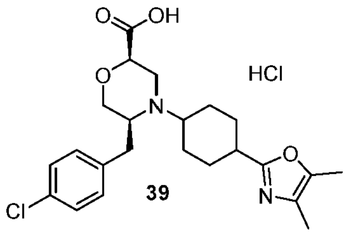

**39**

The title compound (**39**) was obtained as a hydrochloride salt as a single isomer in 2% overall yield in a similar way to Example 9 with the exception that, in the third step of the synthesis, ketone **26c** was used instead of ketone **1g** and only one isomer was obtained. The crude product was purified by preparative reversed-phase column chromatography (column: Cosmosil Cholester 20 x 250 mm, water + 0.3‰ HCl (36%)/MeCN, 95:5 to 20:80, 30 min, 20 mL/min). ESI-MS m/z for $C_{23}H_{30}ClN_2O_4$ found 433.3/435.3 $[M+H]^+$; Rt = 1.04 min (Method A); $^1H$ NMR (700 MHz, DMSO-$d_6$ + $D_2O$, 348 K) δ 7.34 (d, $J_{AA'}$ = 8.5 Hz, 2H), 7.27 (d, $J_{BB'}$ = 8.5 Hz, 2H), 4.31 - 4.29 (m, 1H), 3.64 (dd, *J* = 12.4 Hz, *J* = 4.0 Hz, 1H), 3.59 (dd, *J* = 12.4 Hz, *J* = 2.2 Hz, 1H), 3.42 - 3.40 (m, 1H), 3.23 - 3.20 (m, 2H), 3.16 - 3.13 (m, 1H), 3.00 (dd, *J* = 13.7 Hz, *J* = 4.3 Hz, 1H), 2.88 (dd, *J* = 13.7 Hz, *J* = 9.8 Hz, 1H), 2.65 - 2.61 (m, 1H), 2.14 (brd, *J* = 0.9 Hz, 3H), 2.10 - 2.03 (m, 4H), 1.94 (brd, *J* = 0.9 Hz, 3H), 1.54 - 1.39 (m, 4H).

**Example 40.**

Synthesis of (2*R*,5*S*)-5-(4-chlorobenzyl)-4-(4-(4,5-dimethylthiazol-2-yl)cyclohexyl)morpholine-2-carboxylic acid hydrochloride (**40**).

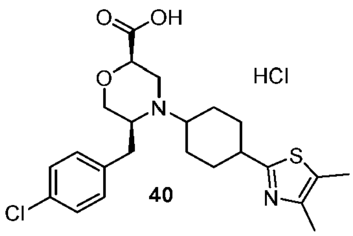

**40**

The title compound (**40**) was obtained as a hydrochloride salt as a single isomer in 23% overall yield in a similar way to Example 9 with the exception that, in the third step of the synthesis, ketone **40c** (synthesis of this ketone is described below) was used instead of ketone **1g** and only one isomer was obtained and in the fourth step of the synthesis, 4 M NaOH was used instead of 1 M NaOH. The crude product was purified by preparative reversed-phase column chromatography (column: Cosmosil Cholester 20 x 250 mm, water + 0.3‰ HCl (36%)/MeCN, 95:5 to 10:90, 30 min, 20 mL/min). ESI-MS m/z for $C_{23}H_{30}ClN_2O_3S$ found 449.4/451.2 $[M+H]^+$; $R_t$ = 1.07 min (Method A); $^1H$ NMR (700 MHz, DMSO-$d_6$ + $D_2O$, 348 K) δ 7.36 (d, $J_{AA'}$ = 8.5 Hz, 2H), 7.29 (d, $J_{BB'}$ = 8.5 Hz, 2H), 4.36 - 4.35 (m, 1H), 3.67 - 3.63 (m, 2H), 3.52 - 3.51 (m, 1H), 3.32 - 3.26 (m, 3H), 3.05 (dd, *J* = 13.9 Hz, *J* = 4.4 Hz, 1H), 2.92 (dd, *J* = 13.8 Hz, *J* = 10.0 Hz, 1H), 2.82 (tt, *J* = 11.6 Hz, *J* = 3.3 Hz, 1H), 2.25 (brd, *J* = 0.6 Hz, 3H), 2.18 (brd, *J* = 0.8 Hz, 3H), 2.15 - 2.09 (m, 4H), 1.60 - 1.42 (m, 4H).

Synthesis of the ketone **40c:**

Step 1.

Synthesis of 4,5-dimethyl-2-(1,4-dioxaspiro[4.5]dec-7-en-8-yl)thiazole (**40a**).

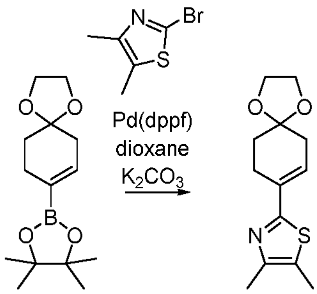

**40a**

The solution of 4,4,5,5-tetramethyl-2-(1,4-dioxaspiro[4.5]dec-7-en-8-yl)-1,3,2-dioxaborolane (560 mg; 2.10 mmol), 2-bromo-4,5-dimethylthiazole (385 mg, 2.00 mmol), Pd(dppf) (163 mg; 0.10 mmol) in mixture of dioxane and 2 M aqueous solution of $K_2CO_3$ (12.5 mL; 4:1 *v/v*) was heated at 100 °C for 2.5 hours under argon atmosphere. TLC showed complete consumption of the starting material. Then a whole was concentrated *in vacuo* and the residue was purified by flash column chromatography on silica (hexane/AcOEt, 100:0 to 4:6, *v/v,* 35 min, 30 mL/min). Compound **40a** was obtained in 81% yield (408 mg; 1.62 mmol).
ESI-MS m/z for $C_{13}H_{18}NO_2S$ found 252.2 $[M+H]^+$; Rt = 1.11 min (Method A); $^1H$ NMR (700 MHz, DMSO-$d_6$ + $D_2O$) δ 6.32 - 6.29 (m, 1H), 3.89 (s, 4H), 2.57 - 2.54 (m, 2H), 2.36 - 2.33 (m, 2H), 2.27 (s, 3H), 2.20 (s, 3H), 1.79 - 1.73 (m, 2H).

Step 2.

Synthesis of 4,5-dimethyl-2-(1,4-dioxaspiro[4.5]decan-8-yl)thiazole (**40b**).

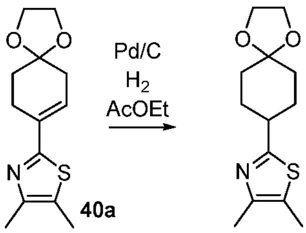

**40a** **40b**

To the solution of **40a** (404 mg; 1.61 mmol) in AcOEt (15 mL) 10% Pd/C (5 mol%; 86 mg, 0.08 mmol) was added and the reaction mixture was conducted under hydrogen atmosphere. The reaction progress was monitored by LC-MS. After 4 hours, when analysis indicated completion of the reaction, Pd/C was filtered off through the Celite and the solvent was concentrated *in vacuo.* The crude product was used to the next step without additional purification. Compound **40b** was obtained in 97% yield (396 mg; 1.56 mmol).
ESI-MS m/z for $C_{13}H_{20}NO_2S$ found 254.2 $[M+H]^+$; Rt = 1.00 min (Method A)

Step 3.

Synthesis of 4-(4,5-dimethylthiazol-2-yl)cyclohexan-1-one (**40c**).

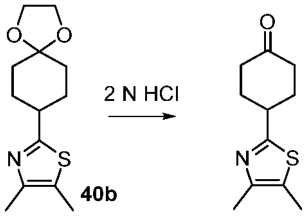

**40b** **40c**

The solution of **40b** (396 mg; 1.56 mmol) in 2 M HCl (15 mL) was stirred at room temperature overnight. The reaction progress was monitored by LC-MS. When analysis indicated completion of the reaction, to this mixture aqueous solution of NaOH (4 M) was added to pH 12 and then extracted with DCM (2 x). The combined organic solutions were dried

over anhydrous $MgSO_4$, filtered and concentrated *in vacuo*. The crude product was used to the next step without additional purification. Compound **40c** was obtained in 95% yield (310 mg; 1.48 mmol).
ESI-MS m/z for $C_{11}H_{16}NOS$ found 210.1 $[M+H]^+$; Rt = 0.84 min (Method A); $^1H$ NMR (700 MHz, DMSO-$d_6$ + $D_2O$, 348 K) δ 2.64 - 2.58 (m, 1H), 2.49 - 2.45 (m, 2H), 2.35 - 2.30 (m, 2H), 2.30 - 2.24 (m, 5H), 2.20 (s, 3H), 1.94 - 1.85 (m, 2H).

**Example 41.**

Synthesis of (2*R*,5*S*)-5-(4-chlorobenzyl)-4-(4-(1,5-dimethyl-1*H*-pyrazol-3-yl)cyclohexyl)-2-((prop-2-yn-1-yloxy)methyl) morpholine hydrochloride (**41**).

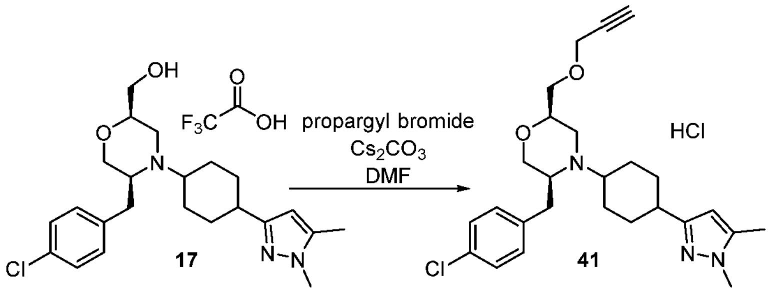

17
41

To the solution of an alcohol **17** (127 mg; 0.24 mmol) in DMF (2 mL) $Cs_2CO_3$ (234 mg; 0.72 mmol) and a solution of a propargyl bromide (80% in toluene; 32 μL; 0.29 mmol) were added and stirred at room temperature overnight, then at 55 °C for 3 hours. Then to this mixture NaH (60% in mineral oil; 6 mg; 0.25 mmol) was added and the reaction mixture was stirred at room temperature for 2 hours. The reaction progress was monitored by LC-MS. Then to this mixture water was added and then whole was diluted with DCM and washed with 10% $Na_2S_2O_3$ and brine. An organic solution was dried over anhydrous $MgSO_4$, filtered and concentrated *in vacuo*. The crude product was purified twice by preparative reversed-phase column chromatography (first: column: Cosmosil Cholester 20 x 250 mm, water + 0.3‰ HCl (36%)/MeCN, 98:2 to 10:90, 30 min, 20 mL/min, second: column: Cosmosil Cholester 20 x 250 mm, water + 0.3‰ HCl (36%)/MeCN, 95:5 to 30:70, 30 min, 20 mL/min). The title compound (**41**) was obtained as a hydrochloride salt as a single isomer in 21% yield (23 mg; 0.05 mmol).
ESI-MS m/z for $C_{26}H_{35}ClN_3O_2$ found 456.4/458.4 $[M+H]^+$; Rt = 1.06 min (Method A); $^1H$ NMR (700 MHz, DMSO-$d_6$ + $D_2O$, 348 K) δ 7.38 (d, $J_{AA'}$ = 8.5 Hz, 2H), 7.31 (d, $J_{BB'}$ = 8.5 Hz, 2H), 5.87 (s, 1H), 4.20 (dd, $J$ = 2.4 Hz, $J$ = 0.7 Hz, 2H), 4.02 - 4.00 (m, 1H), 3.82 - 3.80 (m, 1H), 3.77 - 3.75 (m, 1H), 3.64 - 3.61 (m, 6H), 3.49 - 3.47 (m, 1H), 3.43 - 3.41 (m, 1H), 3.27 (t, $J$ = 2.4 Hz, 1H), 3.17 - 3.12 (m, 3H), 2.50 - 2.47 (m, 1H), 2.29 - 2.23 (m, 2H), 2.18 (s, 3H), 2.07 - 2.02 (m, 2H), 1.63 - 1.57 (m, 2H), 1.48 - 1.40 (m, 2H).

**Example 42.**

Synthesis of (2*R*,5*S*)-5-(4-chlorobenzyl)-4-(4-(4,5-dimethylthiazol-2-yl)cyclohexyl)-*N*-ethylmorpholine-2-carboxamide hydrochloride (**42**).

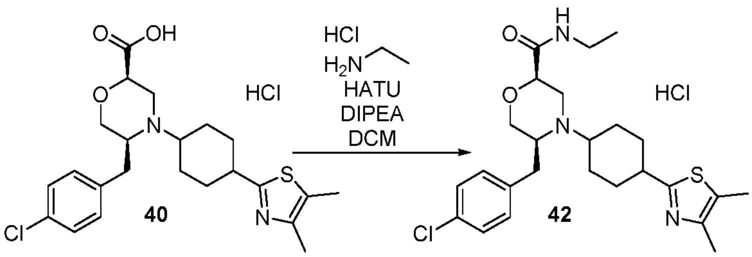

40
42

To the solution of **40** (22 mg; 0.045 mmol) in dry DCM (2 mL) diisopropylethylamine (DIPEA; 20 μL; 0.112 mmol), ethylamine hydrochloride (5 mg; 0.067 mmol) were added and after 10 minutes HATU (21 mg; 0.053 mmol) was added and the reaction mixture was stirred at room temperature for 4 hours. The reaction progress was monitored by TLC and LC-MS.

When analyses indicated completion of the reaction, the mixture was concentrated *in vacuo.* The residue was dissolved in water/MeCN/$HCl_{aq}$, filtered off and taken for preparative reversed-phase column chromatography (column: Cosmosil Cholester 20 x 250 mm, water + 0.3‰ HCl (36%)/MeCN, 95:5 to 20:80, 30 min, 20 mL/min). The title compound (**42**) was obtained as a hydrochloride salt as a single isomer in 64% yield (15 mg; 0.029 mmol).
ESI-MS m/z for $C_{25}H_{35}ClN_3O_2S$ found 476.3/478.3 $[M+H]^+$; Rt = 1.12 min (Method A); $^1H$ NMR (700 MHz, DMSO-$d_6$ + $D_2O$, 348 K) δ 7.39 (d, $J_{AA'}$ = 8.6 Hz, 2H), 7.36 (d, $J_{BB'}$ = 8.6 Hz, 2H), 4.37 - 4.36 (m, 1H), 3.85 (dd, *J* = 13.2 Hz, *J* = 2.1 Hz, 1H), 3.81 - 3.79 (m, 1H), 3.72 - 3.70 (m, 1H), 3.59 - 3.52 (m, 2H), 3.36 - 3.32 (m, 1H), 3.23 - 3.18 (m, 1H), 3.17 - 3.09 (m, 3H), 2.90 (tt, *J* = 12.2 Hz, *J* = 3.6 Hz, 1H), 2.29 - 2.23 (m, 5H), 2.20 - 2.15 (m, 5H), 1.72 - 1.62 (m, 2H), 1.58 - 1.51 (m, 2H), 1.07 (t, *J* = 7.2 Hz, 3H).

**Example 43.**

Synthesis of *N*-(35-(4-((((2*R*,5*S*)-5-(4-chlorobenzyl)-4-(4-(1,5-dimethyl-1*H*-pyrazol-3-yl)cyclohexyl)morpholin-2-yl)methoxy)methyl)-1*H*-1,2,3-triazol-1-yl)-3,6,9,12,15,18,21,24,27,30,33-undecaoxapentatriacontyl)-5-((3*aS*,4*S*,6*aR*)-2-oxohexahydro-1*H*-thieno[3,4-*d*]imidazol-4-yl)pentanamide hydrochloride (**43**).

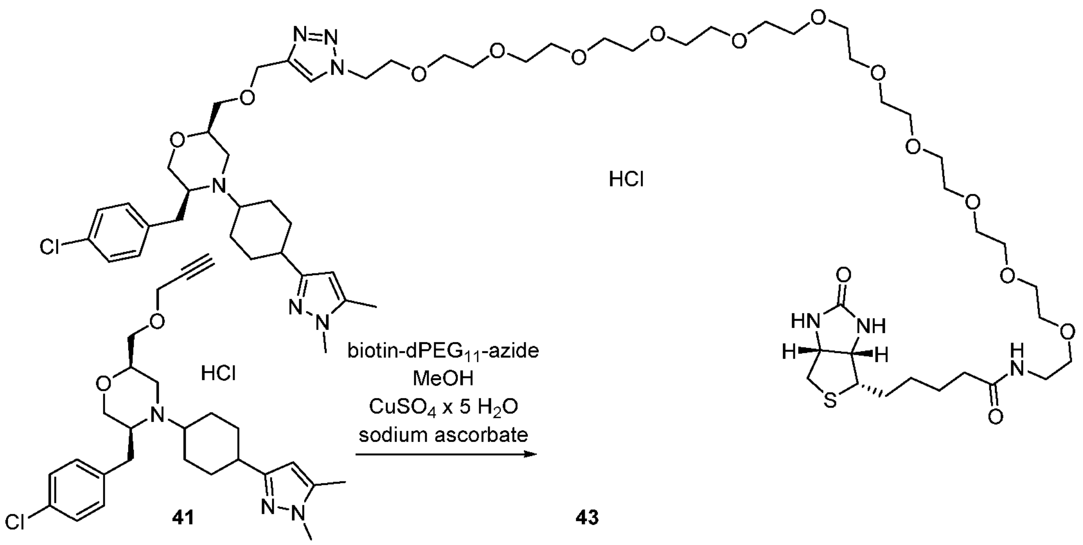


41

43

To the solution of **41** (27 mg; 0.055 mmol) in MeOH (1 mL) $CuSO_4$ x 5 $H_2O$ (7 mg; 0.027 mmol) was added and after 5 minutes to a light green solution sodium L-ascorbate (11 mg; 0.055 mmol) was added in one portion. A whole was stirred for 20 minutes at room temperature and then a solution of an azide (40 mg; 0.050 mmol) in MeOH (1 mL) was added and the reaction mixture was stirred at room temperature for 3 hours. After analytical control (LC-MS) indicated completion of the reaction, the reaction mixture was concentrated *in vacuo* and the residue was dissolved in water/MeCN/$HCl_{aq}$, filtered off and taken for preparative reversed-phase column chromatography (first: column: Cosmosil Cholester 20 x 250 mm, water + 0.3‰ HCl (36%)/MeCN, 95:5 to 10:90, 30 min, 20 mL/min, second: column: Cosmosil Cholester 20 x 250 mm, water + 0.3‰ HCl (36%)/MeCN, 95:5 to 50:70, 30 min, 16 mL/min, third: column: Cosmosil Cholester 20 x 250 mm, water + 0.3‰ HCl (36%)/MeCN, 95:5 to 50:50, 30 min, 16 mL/min). The title compound (**43**) was obtained as a hydrochloride salt as a single isomer in 28% yield (18 mg; 0.014 mmol).
ESI-MS m/z for $C_{60}H_{99}ClN_9O_{15}S$ found 1252.2/1254.2 $[M+H]^+$; Rt = 1.09 min (Method A)

**Example 44.**

Synthesis of (2*R*,5*S*)-2-(azidomethyl)-5-(4-chlorobenzyl)-4-(4-(1,5-dimethyl-1*H*-pyrazol-3-yl)cyclohexyl)morpholine hydrochloride (**44**).

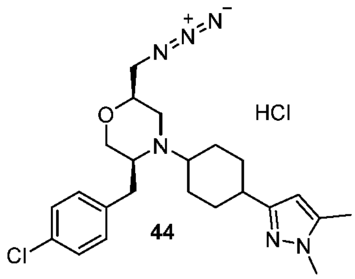

44

Step 1.

Synthesis of (2*R*,5*S*)-2-(azidomethyl)-5-(4-chlorobenzyl)morpholine (**44a**).

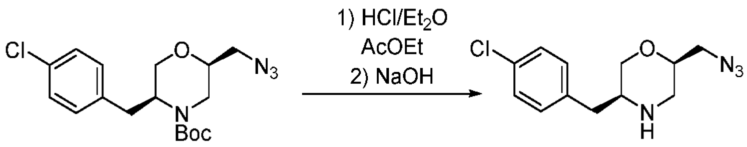


27a 44a

The title compound (**44a**) was obtained as a free amine in 97% yield (1.5 g; 5.64 mmol) from **27a** (2.13 g; 5.81 mmol) according to the General Procedure **IVa** and then after basic (NaOH) extraction with DCM.
ESI-MS m/z for $C_{12}H_{16}ClN_4O$ found 267.0/268.9 $[M+H]^+$; Rt = 0.72 min (Method A)

Step 2.

Synthesis of (2*R*,5*S*)-2-(azidomethyl)-5-(4-chlorobenzyl)-4-(4-(1,5-dimethyl-1*H*-pyrazol-3-yl)cyclohexyl)morpholine hydrochloride (**44**).

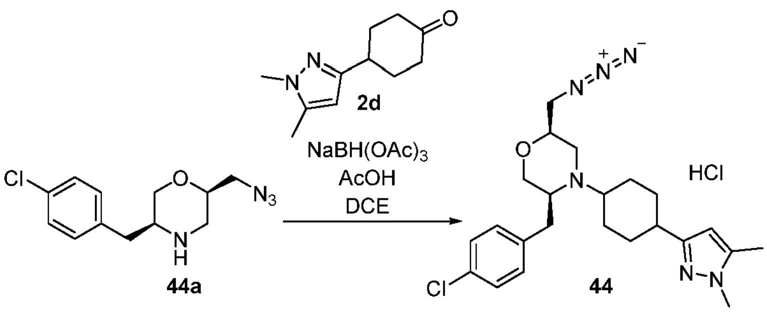


44a 44

The title compound (**44**) was obtained as a single isomer as a free amine from **44a** (800 mg; 3.00 mmol) and ketone **2d** (635 mg; 3.30 mmol) according to the General Procedure **VII** in 46% yield (612 mg; 1.38 mmol). The crude product was purified by silica-gel column chromatography (hexane/AcOEt, 9:1, v/v, then AcOEt, 100%, then: AcOEt/-MeOH, 200:1 to 9:1, v/v). The sample of this product (22 mg) was then purified by preparative reversed-phase column chromatography (column: Cosmosil Cholester 20 x 250 mm, water + 0.3‰ HCl (36%)/MeCN, 95:5 to 20:80, 30 min, 20 mL/min) to give 10 mg of the title product as a hydrochloride salt.
ESI-MS m/z for $C_{23}H_{32}ClN_6O$ found 443.2/445.2 $[M+H]^+$; Rt = 1.12 min (Method A); $^1H$ NMR (700 MHz, DMSO-$d_6$ + $D_2O$, 348 K) δ 7.40 (d, $J_{AA'}$ = 8.5 Hz, 2H), 7.30 (d, $J_{BB'}$ = 8.5 Hz, 2H), 5.88 (s, 1H), 3.97 - 3.94 (m, 1H), 3.79 - 3.77 (m, 1H), 3.74 - 3.72 (m, 1H), 3.62 - 3.54 (m, 6H), 3.43 - 3.36 (m, 2H), 3.21 - 3.17 (m, 1H), 3.11 - 3.10 (m, 2H), 2.49 - 2.44 (m, 1H), 2.27 - 2.25 (m, 2H), 2.17 (s, 3H), 2.02 - 2.00 (m, 2H), 1.52 - 1.38 (m, 4H).

**Example 45.**

Synthesis of (2*R*,5*S*)-5-(4-chlorobenzyl)-4-(4-(1,5-dimethyl-1*H*-pyrazol-3-yl)cyclohexyl)-*N*-(prop-2-yn-1-yl)morpholine-2-carboxamide hydrochloride (**45**).

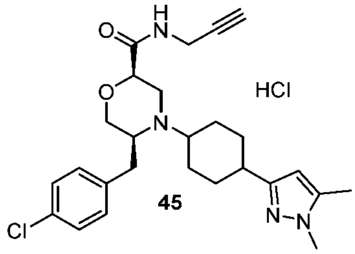

**45**

The title compound (**45**) was obtained as a hydrochloride salt as a single isomer in 2% overall yield in a similar way to Example 30 with the exception that, in the first step of the synthesis, propargylamine was used instead of propan-2-amine. The crude product was purified by silica-gel column chromatography (hexane/AcOEt, 100:0 to 0:100, *v*/*v*, then: AcOEt/MeOH, 10:1, *v*/*v*) and then purified by preparative reversed-phase column chromatography (column: Cosmosil Cholester 20 x 250 mm, water + 0.3‰ HCl (36%)/MeCN, 99:1 to 20:80, 30 min, 20 mL/min).
ESI-MS m/z for $C_{26}H_{34}ClN_4O_2$ found 469.2/471.2 $[M+H]^+$; Rt = 0.96 min (Method A); $^1$H NMR (700 MHz, DMSO-$d_6$ + $D_2O$, 348 K) δ 7.39 (d, $J_{AA'}$ = 8.5 Hz, 2H), 7.35 (d, $J_{BB'}$ = 8.5 Hz, 2H), 5.83 (s, 1H), 4.44 - 4.42 (m, 1H), 3.97 (dd, *J* = 17.4 Hz, *J* = 2.5 Hz, 1H), 3.91 (dd, *J* = 17.4 Hz, *J* = 2.5 Hz, 1H), 3.87 (dd, *J* = 13.2 Hz, *J* = 2.3 Hz, 1H), 3.81 - 3.79 (m, 1H), 3.73 - 3.71 (m, 1H), 3.61 (s, 3H), 3.60 - 3.58 (m, 1H), 3.51 - 3.47 (m, 1H), 3.36 - 3.32 (m, 1H), 3.14 - 3.09 (m, 2H), 2.91 (t, *J* = 2.5 Hz, 1H), 2.50 - 2.46 (m, 1H), 2.24 - 2.17 (m, 5H), 2.06 - 2.01 (m, 2H), 1.67 - 1.57 (m, 2H), 1.46 - 1.38 (m, 2H).

**Example 46.**

Synthesis of *N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-pyrazole-3-carboxamide hydrochloride (**46**).

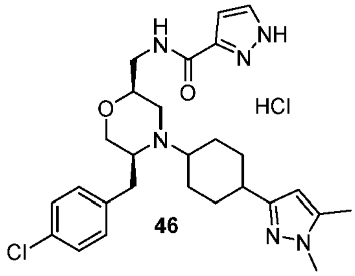

**46**

Step 1.

Synthesis of ((2*S*,5*S*)-5-(4-chlorobenzyl)-4-(4-(1,5-dimethyl-1*H*-pyrazol-3-yl)cyclohexyl)morpholin-2-yl)methanamine (**46a**).

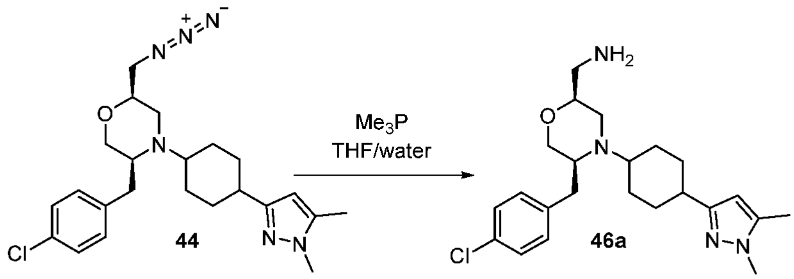

44
46a

An azide **44** (as a free amine)(590 mg; 1.33 mmol) was dissolved in THF/$H_2O$ (20 mL; *9:1, v/v*) and then 1 M solution of $Me_3P$ in THF (2 mL; 2.00 mmol) was added and a whole was stirred at room temperature for 2 hours. The reaction progress was monitored by LC-MS. When analysis indicated completion of the reaction, the reaction was quenched with 5% $NaHCO_3$ and a whole was extracted with diethyl ether (2 x). The combined organic solutions were washed with water and then dried over anhydrous $MgSO_4$, filtered and concentrated *in vacuo.* The all crude product mixture was used to the next step without additional purification.
ESI-MS m/z for $C_{23}H_{34}ClN_4O$ found 417.1/419.1 $[M+H]^+$; Rt = 0.76 min (Method A)

Step 2.

Synthesis of *tert*-butyl (((2*S*,5*S*)-5-(4-chlorobenzyl)-4-(4-(1,5-dimethyl-1*H*-pyrazol-3-yl)cyclohexyl)morpholin-2-yl) methyl)carbamate (**46b**).

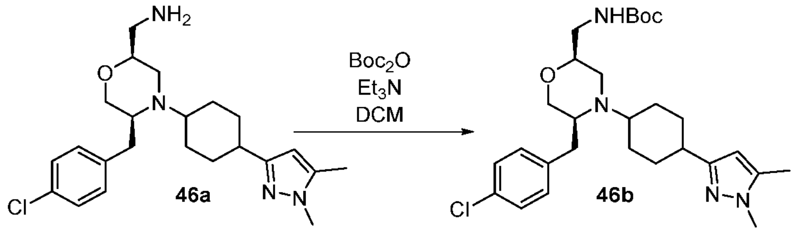

46a
46b

The title compound (**46b**) was obtained in 74% yield (504 mg; 0.98 mmol) from **46a** (the crude mixture) according to the General Procedure **X.**
ESI-MS m/z for $C_{28}H_{42}ClN_4O_3$ found 517.2/519.2 $[M+H]^+$; Rt = 1.08 min (Method A)

Step 3.

Synthesis of ((2*S*,5*S*)-5-(4-chlorobenzyl)-4-(4-(1,5-dimethyl-1*H*-pyrazol-3-yl)cyclohexyl)morpholin-2-yl)methanamine (**46c**).

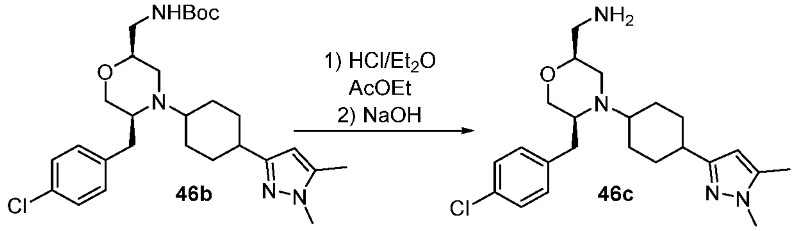

46b
46c

The title compound (**46c**) was obtained as a free amine in 87% yield (355 mg; 0.85 mmol) from **46b** (504 mg; 0.98 mmol) according to the General Procedure **IVa** and then after basic (NaOH) extraction with DCM.
ESI-MS m/z for $C_{23}H_{34}ClN_4O$ found 417.2/419.1 $[M+H]^+$; Rt = 1.05 min (Method A)

Step 4.

Synthesis of *N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-pyrazole-3-carboxamide hydrochloride (**46**).

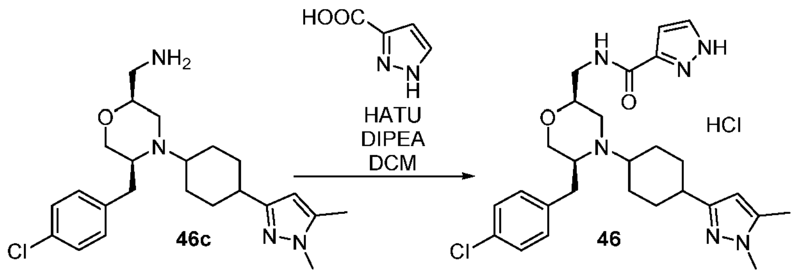

**46c** **46**

To the solution of **46c** (50 mg; 0.120 mmol) in dry DCM (2 mL) diisopropylethylamine (DIPEA; 0.03 mL; 0.180 mmol), 1*H*-pyrazole-3-carboxylic acid (14 mg; 0.126 mmol) and HATU (48 mg; 0.126 mmol) were added and the reaction mixture was stirred at room temperature overnight. The reaction progress was monitored by TLC and LC-MS. When analyses indicated completion of the reaction, the mixture was concentrated *in vacuo.* The crude product was purified by preparative reversed-phase column chromatography (column: Cosmosil Cholester 20 x 250 mm, water + 0.3‰ HCl (36%)/MeCN, 98:2 to 10:90, 30 min, 20 mL/min). The title compound (**46**) was obtained as a hydrochloride salt as a single isomer in 37% yield (24 mg; 0.044 mmol).
ESI-MS m/z for $C_{27}H_{36}ClN_6O_2$ found 511.3/513.3 $[M+H]^+$; Rt = 0.88 min (Method A); $^1H$ NMR (700 MHz, DMSO-$d_6$ + $D_2O$, 348 K) δ 7.72 (d, $J$ = 2.3 Hz, 1H), 7.37 (d, $J_{AA'}$ = 8.5 Hz, 2H), 7.31 *(d,* $J_{BB'}$ = 8.5 Hz, 2H), 6.70 (d, $J$ = 2.3 Hz, 1H), 5.86 (s, 1H), 4.01 - 3.99 (m, 1H), 3.79 - 3.75 (m, 2H), 3.64 - 3.62 (m, 4H), 3.54 - 3.51 (m, 4H), 3.16 - 3.09 (m, 3H), 2.51 - 2.46 (m, 1H), 2.28 - 2.23 (m, 2H), 2.18 (s, 3H), 2.06 - 2.02 (m, 2H), 1.60 - 1.55 (m, 2H), 1.47 - 1.40 (m, 2H).

**Example 47.**

Synthesis of (2*R*,5*S*)-5-(4-chlorobenzyl)-4-(4-(1,5-dimethyl-1*H*-pyrazol-3-yl)cyclohexyl)-*N*-((1-(37-oxo-41-((3*aS*,4*S*,6*aR*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide hydrochloride (**47**).

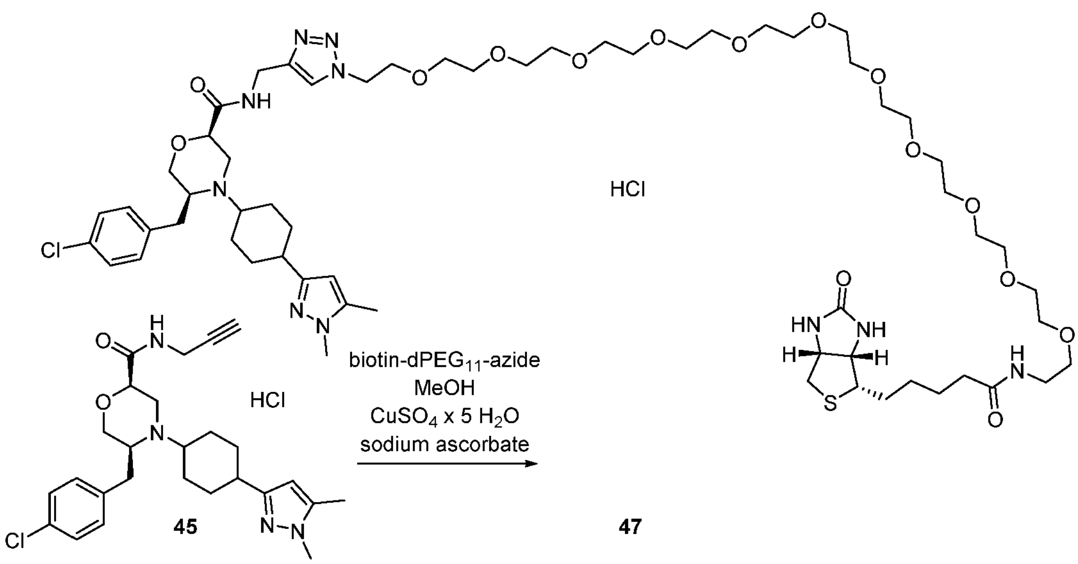

**45** **47**

To the solution of **45** (59 mg; 0.117 mmol) in MeOH (2 mL) $CuSO_4$ x 5 $H_2O$ (15 mg; 0.060 mmol) was added and after 5 minutes to a light green solution sodium L-ascorbate (23 mg; 0.117 mmol) was added in one portion. A whole was stirred for 25 minutes at room temperature and then a solution of an azide (93 mg; 0.117 mmol) in MeOH (2 mL) was added and the reaction mixture was stirred at room temperature overnight. After analytical control (LC-MS) indicated completion

of the reaction, the reaction mixture was concentrated *in vacuo* and the residue was dissolved in water/MeCN/$HCl_{aq}$, filtered off and taken for preparative reversed-phase column chromatography (first: column: Cosmosil Cholester 20 x 250 mm, water + 0.3‰ HCl (36%)/MeCN, 98:2 to 10:90, 30 min, 20 mL/min, second: column: Cosmosil Cholester 20 x 250 mm, water + 0.3‰ HCl (36%)/MeCN, 99:1 to 20:80, 30 min, 18 mL/min, third: column: Cosmosil Cholester 20 x 250 mm, water + 0.3‰ HCl (36%)/MeCN, 99:1 to 10:90, 30 min, 18 mL/min). The title compound (**47**) was obtained as a hydrochloride salt as a single isomer in 20% yield (31 mg; 0.023 mmol).
ESI-MS m/z for $C_{60}H_{98}ClN_{10}O_{15}S$ found 1265.7/1267.7 $[M+H]^+$; Rt = 1.06 min (Method A)

**Example 48.**

Synthesis of *N*-(((2*S*,5*S*)-5-(4-chlorobenzyl)-4-(4-(1,5-dimethyl-1*H*-pyrazol-3-yl)cyclohexyl)morpholin-2-yl) methyl)-2*H*-1,2,3-triazole-4-carboxamide hydrochloride (**48**).

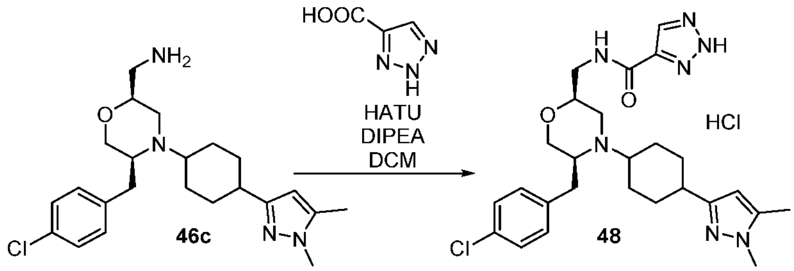

46c 48

To the solution of **46c** (50 mg; 0.120 mmol) in dry DCM (2 mL) diisopropylethylamine (DIPEA; 0.03 mL; 0.180 mmol), 2*H*-1,2,3-triazole-4-carboxylic acid (15 mg; 0.126 mmol) and HATU (48 mg; 0.126 mmol) were added and the reaction mixture was stirred at room temperature overnight. The reaction progress was monitored by TLC and LC-MS. When analyses indicated completion of the reaction, the mixture was concentrated *in vacuo.* The crude product was purified twice by preparative reversed-phase column chromatography (first: column: Cosmosil Cholester 20 x 250 mm, water + 0.3‰ HCl (36%)/MeCN, 99:1 to 20:80, 30 min, 18 mL/min, second: C-18, water + 0.3‰ HCl (36%)/MeCN, 98:2 to 30:70, 30 min, 18 mL/min). The title compound (**48**) was obtained as a hydrochloride salt as a single isomer in 9% yield (6 mg; 0.011 mmol).
ESI-MS m/z for $C_{26}H_{35}ClN_7O_2$ found 512.2/514.2 $[M+H]^+$; Rt = 0.88 min (Method A); $^1H$ NMR (700 MHz, DMSO-$d_6$ + $D_2O$, 348 K) δ 8.28 (brs, 1H), 7.38 (d, $J_{AA'}$ = 8.5 Hz, 2H), 7.31 (d, $J_{BB'}$ = 8.5 Hz, 2H), 5.84 (s, 1H), 4.02 - 4.00 (m, 1H), 3.79 - 3.76 (m, 2H), 3.65 - 3.63 (m, 1H), 3.61 (s, 3H), 3.56 - 3.52 (m, 3H), 3.46 - 3.44 (m, 1H), 3.16 - 3.10 (m, 3H), 2.50 - 2.45 (m, 1H), 2.29 - 2.23 (m, 2H), 2.17 (s, 3H), 2.06 - 2.02 (m, 2H), 1.60 - 1.54 (m, 2H), 1.47 - 1.40 (m, 2H).

**Example 49.**

Synthesis of 3-(((2*R*,5*S*)-5-(4-chlorobenzyl)-4-(4-(1,5-dimethyl-1*H*-pyrazol-3-yl)cyclohexyl)morpholin-2-yl)methyl)imidazolidine-2,4-dione hydrochloride (**49**).

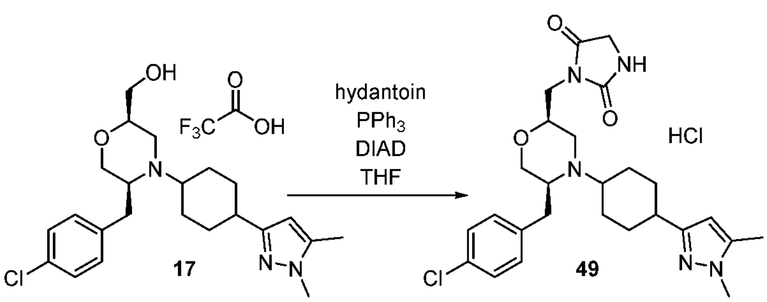

17 49

To the solution of an alcohol **17** (133 mg; 0.25 mmol) and hydantoin (30 mg; 0.30 mmol) in THF (3 mL) $PPh_3$ (158 mg; 0.60 mmol) was added. Then a whole was cooled to -10 °C and DIAD (65 μL; 0.33 mmol) was added dropwise and stirred at room temperature for 2 days. Then to this mixture another portion of hydantoin (30 mg; 0.30 mmol), $PPh_3$ (79 mg;

0.30 mmol) and DIAD (65 μL; 0.33 mmol) were added at -10 °C and the reaction mixture was stirred at room temperature overnight. The reaction progress was monitored by LC-MS. Then a solid was filtered off and a light yellow filtrate was concentrated *in vacuo.* The residue was dissolved in water/MeCN/$HCl_{aq}$, filtered off and taken for preparative reversed-phase column chromatography (first: column: Cosmosil Cholester 20 x 250 mm, water + 0.3‰ HCl (36%)/MeCN, 98:2 to 30:70, 30 min, 20 mL/min, second: C-18, water + 0.3‰ HCl (36%)/MeCN, 98:2 to 20:80, 30 min, 20 mL/min). The title compound (**49**) was obtained as a hydrochloride salt as a single isomer in 40% yield (53 mg; 0.10 mmol).
ESI-MS m/z for $C_{26}H_{35}ClN_5O_3$ found 500.2/502.2 $[M+H]^+$; Rt = 0.87 min (Method A); $^1H$ NMR (700 MHz, DMSO-$d_6$ + $D_2O$, 348 K) δ 7.37 (d, $J_{AA'}$ = 8.5 Hz, 2H), 7.29 (d, $J_{BB'}$ = 8.5 Hz, 2H), 5.82 (s, 1H), 4.07 - 4.04 (m, 1H), 3.92 (s, 2H), 3.76 - 3.74 (m, 1H), 3.71 - 3.67 (m, 2H), 3.62 - 3.60 (m, 4H), 3.55 - 3.50 (m, 3H), 3.17 - 3.08 (m, 3H), 2.47 (tt, $J$ = 12.3 Hz, $J$ = 3.6 Hz, 1H), 2.26 - 2.20 (m, 2H), 2.16 (s, 3H), 2.06 - 2.01 (m, 2H), 1.57 - 1.51 (m, 2H), 1.45 - 1.39 (m, 2H).

**Example 50.**

Synthesis of (2*R*,5*S*)-5-(4-chlorobenzyl)-4-(4-(1,5-dimethyl-1*H*-1,2,4-triazol-3-yl)cyclohexyl)-*N*-ethylmorpholine-2-carboxamide hydrochloride (**50**).

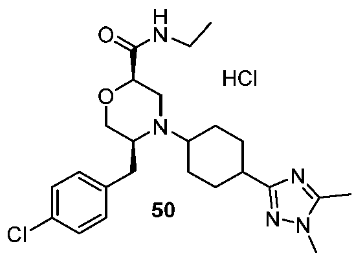

The title compound (**50**) was obtained as a hydrochloride salt as a single isomer in 7% overall yield in a similar way to Example 30 with the exception that, in the first step of the synthesis, ethylamine hydrochloride was used instead of propan-2-amine and in the third step of the synthesis, ketone **15c** was used instead of ketone **2d.** The crude product was purified by silica-gel column chromatography (hexane/AcOEt, 1:1, *v/v*, then AcOEt, 100%, then: AcOEt/MeOH, 50:1 to 6:1, *v/v*) and then purified by preparative reversed-phase column chromatography (column: Cosmosil Cholester 20 x 250 mm, water + 0.3‰ HCl (36%)/MeCN, 95:5 to 20:80, 30 min, 18 mL/min).
ESI-MS m/z for $C_{24}H_{35}ClN_5O_2$ found 460.1/462.1 $[M+H]^+$; Rt = 0.77 min (Method A); $^1H$ NMR (700 MHz, DMSO-$d_6$ + $D_2O$, 348 K) δ 7.38 (d, $J_{AA'}$ = 8.5 Hz, 2H), 7.34 (d, $J_{BB'}$ = 8.5 Hz, 2H), 4.39 - 4.37 (m, 1H), 3.87 (dd, $J$ = 13.2 Hz, $J$ = 2.3 Hz, 1H), 3.80 - 3.78 (m, 1H), 3.73 - 3.71 (m, 4H), 3.60 - 3.58 (m, 1H), 3.53 - 3.49 (m, 1H), 3.35 - 3.32 (m, 1H), 3.22 - 3.09 (m, 4H), 2.67 (tt, $J$ = 12.2 Hz, $J$ = 3.6 Hz, 1H), 2.41 (s, 3H), 2.28 - 2.22 (m, 2H), 2.16 - 2.11 (m, 2H), 1.71 - 1.62 (m, 2H), 1.58 - 1.50 (m, 2H) 1.07 (t, $J$ = 7.2 Hz, 3H).

**Example 51.**

Synthesis of *N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-imidazole-4-carboxamide hydrochloride (**51**).

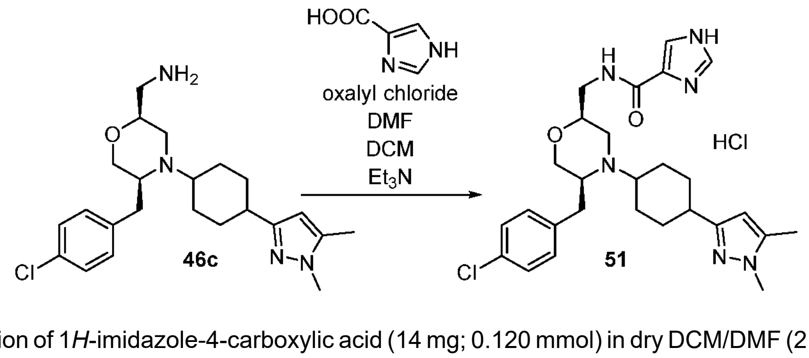


To the solution of 1*H*-imidazole-4-carboxylic acid (14 mg; 0.120 mmol) in dry DCM/DMF (2 mL; 1:1 *v/v)* an oxalyl

chloride (11 µL; 0.130 mmol) was added (gas evolution). The reaction mixture was stirred at room temperature for 1 hour and then to this mixture a solution of an amine **46c** (50 mg; 0.120 mmol) in dry DCM (3 mL) and triethylamine (0.05 mL; 0.360 mmol) were added and the reaction mixture was stirred at room temperature overnight. The reaction progress was monitored by LC-MS. When analysis indicated completion of the reaction, the mixture was concentrated *in vacuo.* The crude product was purified by preparative reversed-phase column chromatography (column: Cosmosil Cholester 20 x 250 mm, water + 0.3‰ HCl (36%)/MeCN, 98:2 to 10:90, 30 min, 19 mL/min). The title compound (**51**) was obtained as a hydrochloride salt as a single isomer in 26% yield (17 mg; 0.031 mmol).
ESI-MS m/z for $C_{27}H_{36}ClN_6O_2$ found 511.3/513.3 $[M+H]^+$; Rt = 0.82 min (Method A); $^1H$ NMR (700 MHz, DMSO-$d_6$ + $D_2O$, 348 K) δ 8.58 (s, 1H), 8.01 (d, $J$ = 0.9 Hz, 1H), 7.38 (d, $J_{AA'}$ = 8.5 Hz, 2H), 7.31 (d, $J_{BB'}$ = 8.5 Hz, 2H), 5.86 (s, 1H), 4.04 - 4.02 (m, 1H), 3.84 - 3.83 (m, 1H), 3.78 - 3.76 (m, 1H), 3.64 - 3.62 (m, 4H), 3.56 - 3.49 (m, 3H), 3.47 - 3.43 (m, 1H), 3.17 - 3.10 (m, 3H), 2.51 - 2.47 (m, 1H), 2.29 - 2.24 (m, 2H), 2.18 (s, 3H), 2.07 - 2.02 (m, 2H), 1.66 - 1.59 (m, 2H), 1.47 - 1.40 (m, 2H).

**Example 52.**

Synthesis of (2*R*,5*S*)-5-(4-chlorobenzyl)-4-(4-(4,5-dimethylthiazol-2-yl)cyclohex-3-en-1-yl)-2-((methylsulfonyl)methyl) morpholine 2,2,2-trifluoroacetate (**52**).

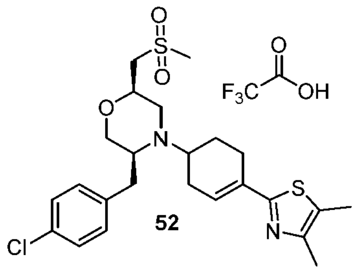

**52**

Step 1.

Synthesis of 4-(4,5-dimethylthiazol-2-yl)cyclohex-3-en-1-one (**52a**).

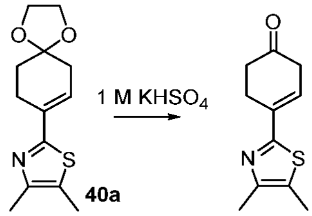


**40a** **52a**

The solution of **40a** (861 mg; 3.40 mmol) in 1 M $KHSO_{4aq}$ (60 mL) was stirred at 30 °C for 5 hours. The reaction progress was monitored by LC-MS. When analysis indicated completion of the reaction, to this mixture aqueous solution of NaOH (4 M) was added to pH 12 and then extracted with DCM (2 x). The combined organic solutions were dried over anhydrous $MgSO_4$, filtered and concentrated *in vacuo.* The crude product was purified by flash column chromatography on silica (hexane/AcOEt, 100:0 to 45:55, *v*/*v,* 35 min). Compound **52a** was obtained in 44% yield (310 mg; 1.50 mmol). ESI-MS m/z for $C_{11}H_{14}NOS$ found 208.1 $[M+H]^+$; Rt = 1.10 min (Method A)

Step 2.

Synthesis of (2*R*,5*S*)-5-(4-chlorobenzyl)-4-(4-(4,5-dimethylthiazol-2-yl)cyclohex-3-en-1-yl)-2-((methylsulfonyl)methyl) morpholine 2,2,2-trifluoroacetate (**52**).

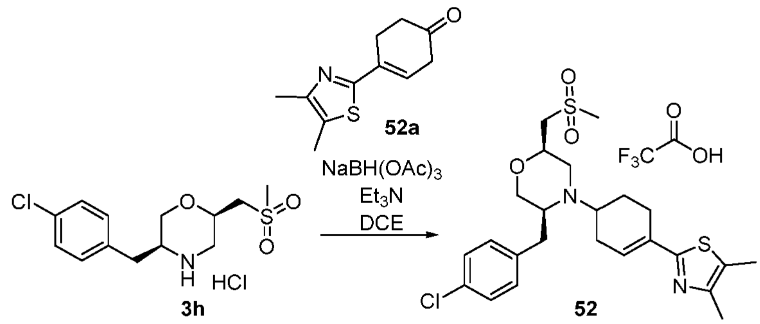

52a
3h
52

The title compound (**52**) was obtained as a TFA salt from **3h** (85 mg; 0.25 mmol) and ketone **52a** (50 mg; 0.24 mmol) according to the General Procedure **VII** in 37% yield (56 mg; 0.09 mmol). The crude product was purified by flash column chromatography on silica (hexane/AcOEt, 100:0 to 35:65, *v*/*v*) and then purified twice by preparative reversed-phase column chromatography (column: Cosmosil Cholester 20 x 250 mm, water/MeCN + 1‰ TFA, 95:5 to 10:90, 30 min, 20 mL/min).

ESI-MS m/z for $C_{24}H_{32}ClN_2O_3S_2$ found 495.0/497.0 $[M+H]^+$; $R_t$ = 1.34 min (Method A); $^1H$ NMR (700 MHz, DMSO-$d_6$ + $D_2O$) δ 7.39 - 7.37 (m, 2H), 7.34 - 7.32 (m, 2H), 6.38 - 6.36 (m, 1H), 4.29 - 4.24 (m, 1H), 3.82 - 3.63 (m, 4H), 3.61 - 3.56 (m, 1.5H), 3.51 - 3.49 (m, 0.5H), 3.38 - 3.37 (m, 1H), 3.31 - 3.26 (m, 1H), 3.19 (td, *J* = 13.8 Hz, *J* = 10.9 Hz, 1H), 3.13 - 3.09 (m, 1H), 3.03 (s, 3H), 2.81 - 2.72 (m, 2H), 2.48 - 2.33 (m, 3H), 2.29 (s, 3H), 2.22 (s, 3H), 1.74 - 1.66 (m, 1H).

**Example 53.**

Synthesis of (2*R*,5*S*)-5-(4-chlorobenzyl)-4-(4-(4,5-dimethylthiazol-2-yl)cyclohex-3-en-1-yl)-*N*-ethylmorpholine-2-carboxamide 2,2,2-trifluoroacetate (**53**).

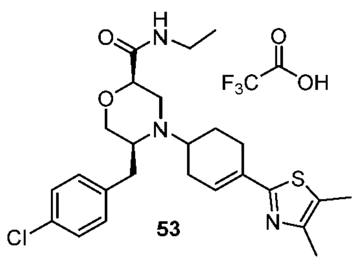
53

The title compound (**53**) was obtained as a TFA salt in 24% overall yield in a similar way to Example 30 with the exception that, in the first step of the synthesis, ethylamine hydrochloride was used instead of propan-2-amine and in the third step of the synthesis, ketone **52a** was used instead of ketone **2d.** The crude product was purified by flash column chromatography on silica (hexane/AcOEt, 100:0 to 10:90, *v*/*v,* 30 min) and then purified by preparative reversed-phase column chromatography (column: Cosmosil Cholester 20 x 250 mm, water/MeCN + 1‰ TFA, 98:2 to 10:90, 30 min, 20 mL/min).

ESI-MS m/z for $C_{25}H_{33}ClN_3O_2S$ found 474.2/476.2 $[M+H]^+$; Rt = 1.25 min (Method A); $^1H$ NMR (700 MHz, DMSO-$d_6$ + $D_2O$, 348 K) δ 7.39 - 7.34 (m, 4H), 6.37 - 6.36 (m, 1H), 4.30 - 4.27 (m, 1H), 3.81 - 3.74 (m, 2H), 3.71 - 3.66 (m, 2H), 3.61 - 3.52 (m, 1H), 3.34 - 3.30 (m, 1H), 3.23 - 3.11 (m, 4H), 2.80 - 2.71 (m, 2H), 2.48 - 2.38 (m, 2H), 2.36 - 2.29 (m, 4H), 2.21 (brs, 3H), 1.77 - 1.67 (m, 1H), 1.08 - 1.06 (m, 3H).

**Example 54.**

Synthesis of (2*R*,5*S*)-5-(4-chlorobenzyl)-4-(4-(4,5-dimethylthiazol-2-yl)cyclohexyl)-*N*-(37-oxo-41-((3*aS*,4*S*,6*aR*)-2-oxohexahydro-1*H*-thieno[3,4-*d*]imidazol-4-yl)-3,6,9,12,15,18,21,24,27,30,33-undecaoxa-36-azahentetracontyl)morpholine-2-carboxamide hydrochloride (**54**).

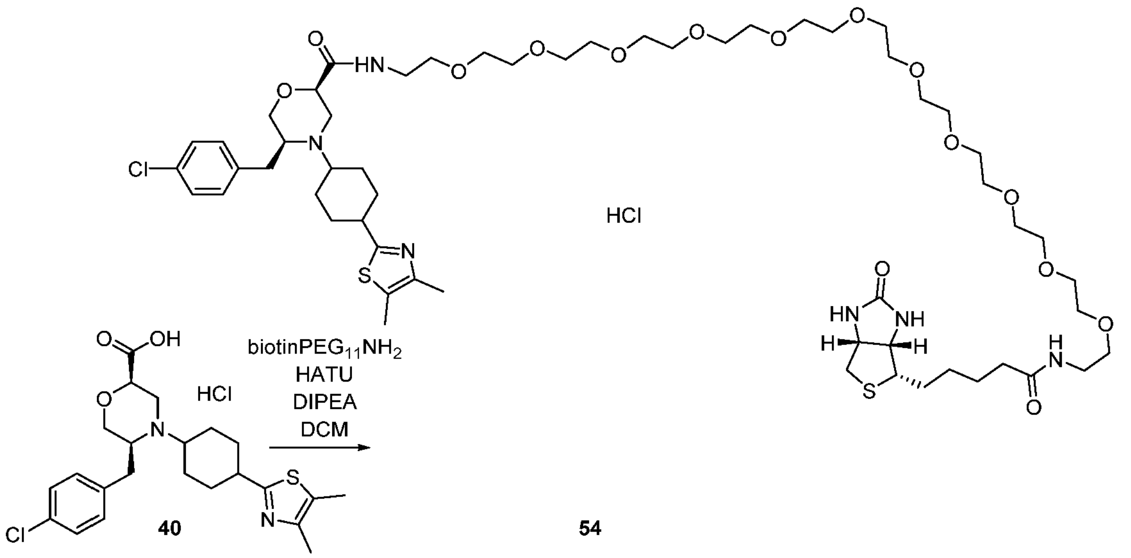


40

54

The title compound (**54**) was obtained as a single isomer as a hydrochloride salt from **40** (17 mg; 0.035 mmol) according to the General Procedure **XI** in 77% yield (34 mg; 0.027 mmol). The crude product was purified twice by preparative reversed-phase column chromatography (column: Cosmosil Cholester 20 x 250 mm, water + 0.3‰ HCl (36%)/MeCN, 95:5 to 20:80, 30 min, 18 mL/min).

ESI-MS m/z for $C_{57}H_{94}ClN_6O_{15}S_2$ found 1201.7/1203.7 $[M+H]^+$; $R_t$ = 1.22 min (Method A)

**Example 55.**

Synthesis of (2*R*,5*S*)-2-(azidomethyl)-5-(4-chlorobenzyl)-4-(4-(4,5-dimethylthiazol-2-yl)cyclohexyl)morpholine hydrochloride (**55**).

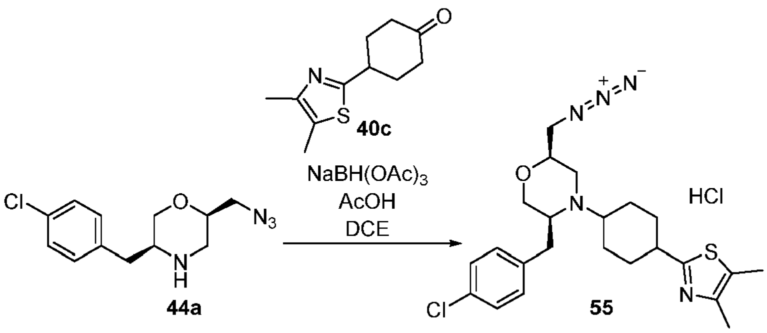


44a

55

The title compound (**55**) was obtained as a single isomer as a free amine from **44a** (682 mg; 2.56 mmol) and ketone **40c** (563 mg; 2.69 mmol) according to the General Procedure **VII** in 42% yield (500 mg; 1.09 mmol). The crude product was purified by flash column chromatography on silica (hexane/AcOEt, 100:0 to 0:100, *v/v,* 40 min). The sample of this product (25 mg) was then purified by preparative reversed-phase column chromatography (column: Cosmosil Cholester 20 x 250 mm, water + 0.3‰ HCl (36%)/MeCN, 95:5 to 10:90, 30 min, 20 mL/min) to give 9 mg of the title product as a hydrochloride salt.

ESI-MS m/z for $C_{23}H_{31}ClN_5OS$ found 460.3/462.3 $[M+H]^+$; Rt = 1.31 min (Method A); $^1$H NMR (700 MHz, $D_2O$, 333 K) δ 7.86 (d, $J_{AA'}$ = 8.3 Hz, 2H), 7.74 (d, $J_{BB'}$ = 8.3 Hz, 2H), 4.52 - 4.50 (m, 1H), 4.35 - 4.29 (m, 2H), 4.24 - 4.21 (m, 1H), 4.12 (dd, *J* = 13.7 Hz, *J* = 3.3 Hz, 1H), 4.04 - 3.99 (m, 2H), 3.91 (dd, *J* = 13.7 Hz, *J* = 5.3 Hz, 1H), 3.81 - 3.78 (m, 1H), 3.71 - 3.67 (m, 1H), 3.63 - 3.58 (m, 2H), 2.88 - 2.87 (m, 2H), 2.78 - 2.75 (m, 5H), 2.74 (s, 3H), 2.15 - 2.04 (m, 4H).

**Example 56.**

Synthesis of (2*R*,5*S*)-5-(4-chlorobenzyl)-4-(4-(4,5-dimethylthiazol-2-yl)cyclohexyl)-*N*-((1-(37-oxo-41-((3*aS*,4*S*,6*aR*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide hydrochloride (**56**).

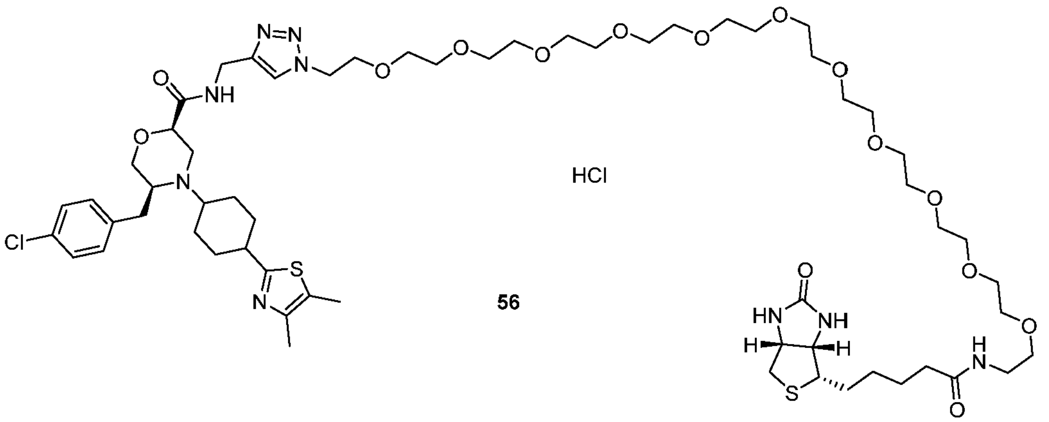

**56**

Step 1.

Synthesis of (2*R*,5*S*)-5-(4-chlorobenzyl)-4-(4-(4,5-dimethylthiazol-2-yl)cyclohexyl)-*N*-(prop-2-yn-1-yl)morpholine-2-carboxamide (**56a**).

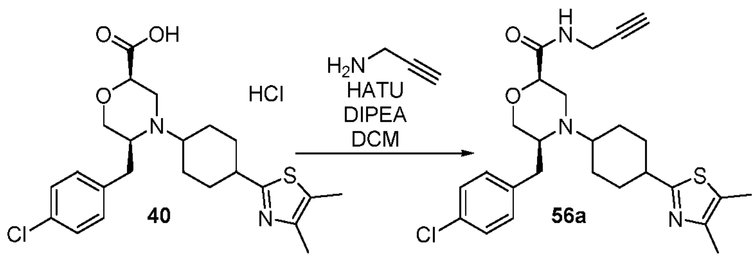


**40** **56a**

To the solution of **40** (22 mg; 0.045 mmol) in dry DCM (2 mL) diisopropylethylamine (DIPEA; 23 μL; 0.135 mmol), propargylamine (4.5 μL; 0.068 mmol) were added and after 5 minutes HATU (21 mg; 0.055 mmol) was added and the reaction mixture was stirred at room temperature overnight. The reaction progress was monitored by TLC and LC-MS. When analyses indicated completion of the reaction, the mixture was concentrated *in vacuo*. The crude product was used to the next step without additional purification.
ESI-MS m/z for $C_{26}H_{33}ClN_3O_2S$ found 486.0/488.0 $[M+H]^+$; Rt = 1.25 min (Method A)

Step 2.

Synthesis of (2*R*,5*S*)-5-(4-chlorobenzyl)-4-(4-(4,5-dimethylthiazol-2-yl)cyclohexyl)-N-((1-(37-oxo-41-((3*aS*,4*S*,6*aR*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide hydrochloride (**56**).

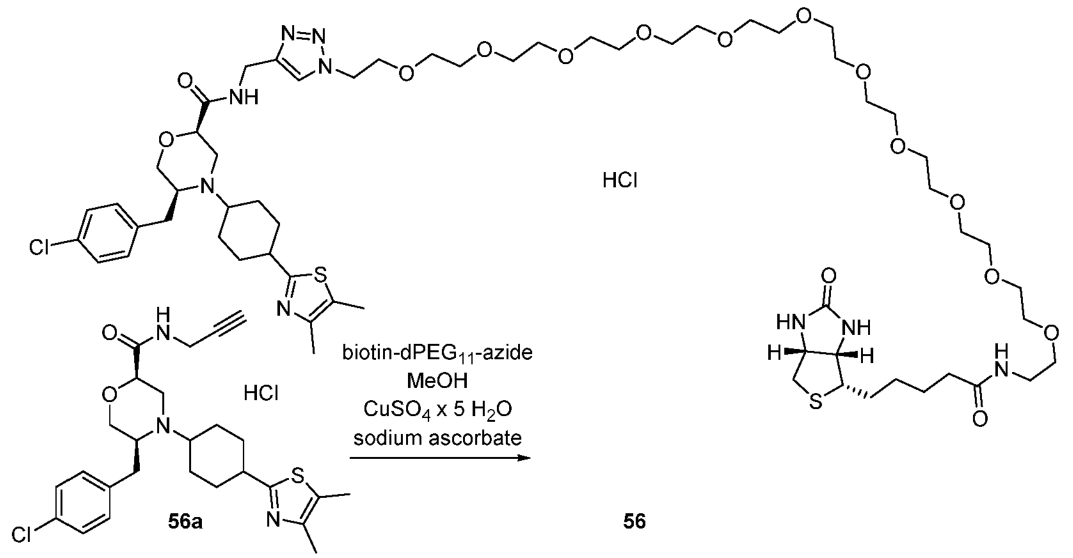


56a

56

To the solution of **56a** (the crude product) in MeOH (2 mL) $CuSO_4$ x 5 $H_2O$ (6 mg; 0.022 mmol) was added and after 5 minutes to a light green solution sodium L-ascorbate (9 mg; 0.045 mmol) was added in one portion. A whole was stirred for 25 minutes at room temperature and then a solution of an azide (36 mg; 0.045 mmol) in MeOH (2 mL) was added and the reaction mixture was stirred at room temperature for 3 hours. After analytical control (LC-MS) indicated completion of the reaction, the reaction mixture was concentrated *in vacuo* and the residue was dissolved in water/MeCN/$HCl_{aq}$, filtered off and taken for preparative reversed-phase column chromatography (first: column: Cosmosil Cholester 20 x 250 mm, water + 0.3‰ HCl (36%)/MeCN, 95:5 to 20:80, 30 min, 20 mL/min, second: C-18, water + 0.3‰ HCl (36%)/MeCN, 95:5 to 20:80, 30 min, 20 mL/min). The title compound (**56**) was obtained as a hydrochloride salt as a single isomer in 27% yield (per two steps)(16 mg; 0.012 mmol).
ESI-MS m/z for $C_{60}H_{97}ClN_9O_{15}S_2$ found 1282.2/1284.2 $[M+H]^+$; Rt = 1.23 min (Method A)

**Example 57.**

Synthesis of *N*-(((2*S*,5*S*)-5-(4-chlorobenzyl)-4-(4-(4,5-dimethylthiazol-2-yl)cyclohexyl)morpholin-2-yl) methyl)-1*H*-1,2,3-triazole-5-carboxamide hydrochloride (**57**).

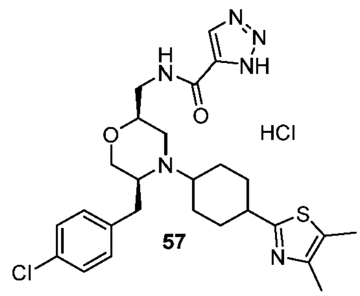


57

The title compound (**57**) was obtained as a hydrochloride salt as a single isomer in 3% overall yield in a similar way to Example 46 with the exception that, in the first step of the synthesis, the compound **55** was used instead of the compound **44** and in the fourth step of the synthesis, 2*H*-1,2,3-triazole-4-carboxylic acid was used instead of 1*H*-pyrazole-3-carboxylic acid. The crude product was purified twice by preparative reversed-phase column chromatography (first: column: Cosmosil Cholester 20 x 250 mm, water + 0.3‰ HCl (36%)/MeCN, 98:2 to 30:70, 30 min, 20 mL/min, second: C-18, water + 0.3‰ HCl (36%)/MeCN, 98:2 to 20:80, 30 min, 20 mL/min).
ESI-MS m/z for $C_{26}H_{34}ClN_6O_2S$ found 529.1/531.1 $[M+H]^+$; Rt = 1.17 min (Method A); $^1H$ NMR (700 MHz, DMSO-$d_6$) $\delta$ 10.60 (brs, 1H), 8.63 - 8.61 (m, 1H), 7.43 (d, $J_{AA'}$ = 8.4 Hz, 2H), 7.35 (d, $J_{BB'}$ = 8.4 Hz, 2H), 4.04 - 4.00 (m, 1H), 3.82 - 3.80 (m, 2H), 3.62 - 3.58 (m, 2H), 3.54 - 3.48 (m, 3H), 3.14 - 3.07 (m, 3H), 2.88 (tt, $J$ = 12.0 Hz, $J$ = 3.6 Hz, 1H), 2.33 - 2.28 (m, 5H), 2.21 - 2.14 (m, 5H), 1.70 - 1.59 (m, 3H), 1.53 - 1.47 (m, 1H).

**Example 58.**

Synthesis of *N*-(((2*S*,5*S*)-5-(4-chlorobenzyl)-4-(4-(1,5-dimethyl-1*H*-pyrazol-3-yl)cyclohexyl)morpholin-2-yl)methyl)-1-methyl-1*H*-imidazole-4-carboxamide hydrochloride (**58**).

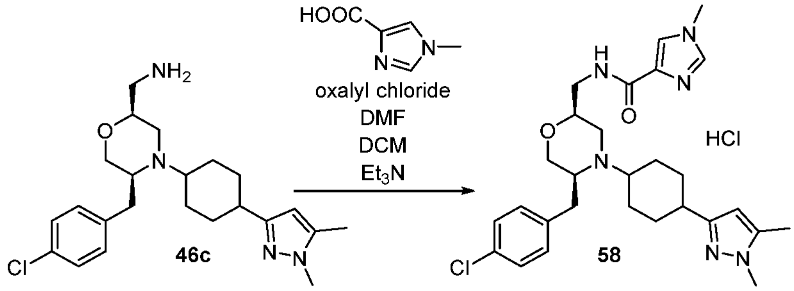


To the solution of 1-methyl-1*H*-imidazole-4-carboxylic acid (25 mg; 0.190 mmol) in dry DCM/DMF (2 mL; 1:1 *v*/*v*) an oxalyl chloride (18 μL; 0.210 mmol) was added (gas evolution). The reaction mixture was stirred at room temperature for 1 hour and then to this mixture a solution of an amine **46c** (80 mg; 0.190 mmol) in dry DCM (3 mL) and triethylamine (0.08 mL; 0.600 mmol) were added and the reaction mixture was stirred at room temperature overnight. The reaction progress was monitored by LC-MS. When analysis indicated completion of the reaction, the mixture was concentrated *in vacuo.* The crude product was purified twice by preparative reversed-phase column chromatography (first: C-18, water + 0.3‰ HCl (36%)/MeCN, 98:2 to 10:90, 30 min, 20 mL/min, second: column: Cosmosil Cholester 20 x 250 mm, water + 0.3‰ HCl (36%)/MeCN, 98:2 to 30:70, 30 min, 20 mL/min). The title compound (**58**) was obtained as a hydrochloride salt as a single isomer in 11% yield (12 mg; 0.021 mmol).
ESI-MS m/z for $C_{28}H_{38}ClN_6O_2$ found 525.4/527.4 $[M+H]^+$; Rt = 1.03 min (Method A); $^1$H NMR (700 MHz, DMSO-$d_6$ + $D_2O$, 348 K) δ 8.01 (brs, 1H), 7.76 (s, 1H), 7.37 (d, $J_{AA'}$ = 8.5 Hz, 2H), 7.31 (d, $J_{BB'}$ = 8.5 Hz, 2H), 5.83 (s, 1H), 3.99 - 3.97 (m, 1H), 3.78 - 3.73 (m, 5H), 3.64 - 3.60 (m, 4H), 3.53 - 3.49 (m, 3H), 3.45 - 3.42 (m, 1H), 3.15 - 3.08 (m, 3H), 2.50 - 2.45 (m, 1H), 2.28 - 2.22 (m, 2H), 2.17 (s, 3H), 2.05 - 2.01 (m, 2H), 1.60 - 1.54 (m, 2H), 1.46 - 1.39 (m, 2H).

**Examples 59 and 60.**

Synthesis of methyl 2-((2*S*,5*S*)-5-(4-chlorobenzyl)-4-(4-(1,5-dimethyl-1*H*-pyrazol-3-yl)cyclohexyl)morpholin-2-yl)acetate hydrochloride (**59**) and 2-((2*S*,5*S*)-5-(4-chlorobenzyl)-4-(4-(1,5-dimethyl-1*H*-pyrazol-3-yl)cyclohexyl)morpholin-2-yl)acetic acid hydrochloride (**60**).

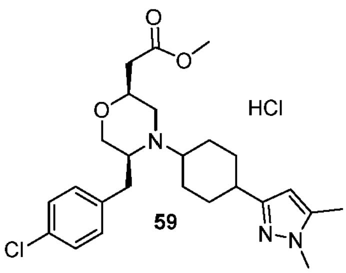
**59**

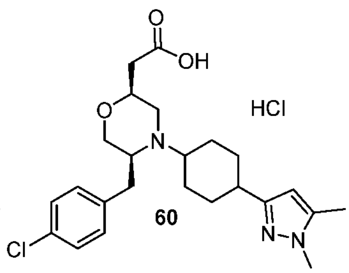
**60**

Step 1.

Synthesis of *tert*-butyl (2*S*,5*S*)-5-(4-chlorobenzyl)-2-(cyanomethyl)morpholine-4-carboxylate (**59a**).

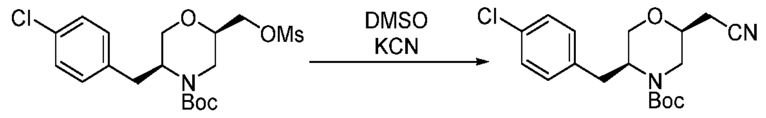


3e 59a

To a solution of **3e** (1.5 g; 4.39 mmol) in an anhydrous DMSO (10 mL) KCN (530 mg; 4.61 mmol) was added and the reaction mixture was stirred at 85 °C for 3 hours and then at room temperature overnight. The reaction progress was monitored by LC-MS analysis. When analysis indicated completion of the reaction, the reaction mixture was diluted with water and extracted with $Et_2O$ (3 x 50 mL). The combined organic solutions were dried over anhydrous $Na_2SO_4$, filtered and concentrated *in vacuo*. The crude product was purified by flash column chromatography on silica (hexane/AcOEt, 100:0 to 75:25, *v/v,* 20 min). Compound **59a** was obtained in 75% yield (1.16 g; 3.31 mmol).
ESI-MS m/z for $C_{13}H_{16}ClN_2O$ found 251.0/253.0 $[M+H-Boc]^+$; Rt = 6.35 min (Method C)

Step 2.

Synthesis of 2-((2*S*,5*S*)-4-(tert-butoxycarbonyl)-5-(4-chlorobenzyl)morpholin-2-yl)acetic acid (**59b**).

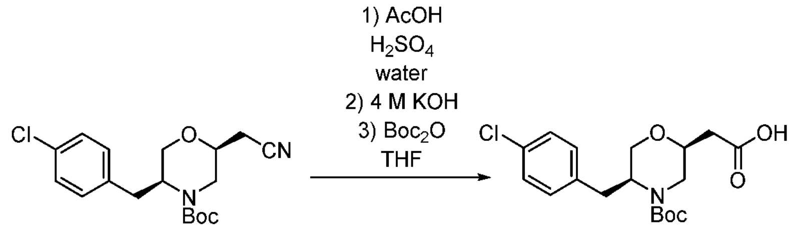


59a 59b

To a solution of **59a** (830 mg; 2.37 mmol) in AcOH (10 mL) a concentrated $H_2SO_4$ (5 mL) and water (5 mL) were added and the reaction mixture was stirred at 90 °C overnight. The reaction progress was monitored by LC-MS analysis. When analysis indicated completion of the reaction, the reaction mixture was cooled down to room temperature and alkalized to pH 11 - 12 with 4 M $KOH_{aq}$. Then to this solution a solution of $Boc_2O$ (1.2 g; 5.50 mmol) in THF (50 mL) was added and stirred at room temperature for 4 hours. Then another portion of $Boc_2O$ (1.2 g; 5.50 mmol) in THF (50 mL) was added and stirred at room temperature for 2 days. When analysis indicated completion of the reaction, the reaction mixture was acidified to pH 2 - 3 with 2 M $HCl_{aq}$ and extracted with AcOEt. The combined organic solutions were washed with brine, dried over anhydrous $Na_2SO_4$, filtered and concentrated *in vacuo.* The crude product was used to the next step without additional purification. Compound **59b** was obtained in 99% yield (867 mg; 2.35 mmol).
ESI-MS m/z for $C_{18}H_{23}ClNO_5$ found 368.0/370.0 $[M-H]^-$; $R_t$ = 5.92 min (Method C)

Step 3.

Synthesis of methyl 2-((2*S*,5*S*)-5-(4-chlorobenzyl)morpholin-2-yl)acetate (**59c**).

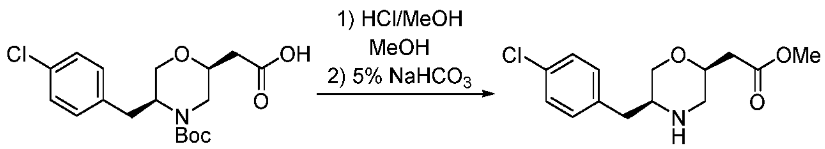


59b 59c

The title compound (**59c**) was obtained as a free amine in 43% yield (250 mg; 0.88 mmol) from **59b** (750 mg; 2.03 mmol) according to the General Procedure **IVa** and then after basic (5% $NaHCO_3$) extraction with DCM.
ESI-MS m/z for $C_{14}H_{19}ClNO_3$ found 284.0/286.0 $[M+H]^+$; $R_t$ = 3.53 min (Method C)

Step 4.

Synthesis of methyl 2-((2*S*,5*S*)-5-(4-chlorobenzyl)-4-(4-(1,5-dimethyl-1*H*-pyrazol-3-yl)cyclohexyl)morpholin-2-yl)acetate (**59d**).

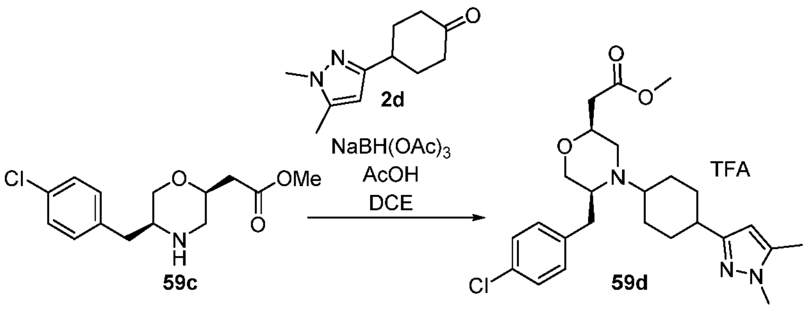


The title compound (**59d**) was obtained as a TFA salt from **59c** (155 mg; 0.55 mmol) and ketone **2d** (105 mg; 0.55 mmol) according to the General Procedure **VII** in 22% yield (70 mg; 0.12 mmol). The crude product was purified by silica-gel column chromatography (AcOEt, 100%, then: AcOEt/MeOH, 95:45, *v/v*) and then by preparative reversed-phase column chromatography (Phenyl-Hexyl, water/MeCN + 1‰ TFA, 95:5 to 5:95, 60 min, 15 mL/min).
ESI-MS m/z for $C_{25}H_{35}ClN_3O_3$ found 460.0/462.0 $[M+H]^+$; $R_t$ = 4.37 min (Method C)

Step 5.

Synthesis of methyl 2-((2*S*,5*S*)-5-(4-chlorobenzyl)-4-(4-(1,5-dimethyl-1*H*-pyrazol-3-yl)cyclohexyl)morpholin-2-yl)acetate hydrochloride (**59**) and 2-((2*S*,5*S*)-5-(4-chlorobenzyl)-4-(4-(1,5-dimethyl-1*H*-pyrazol-3-yl)cyclohexyl)morpholin-2-yl)acetic acid hydrochloride (**60**).

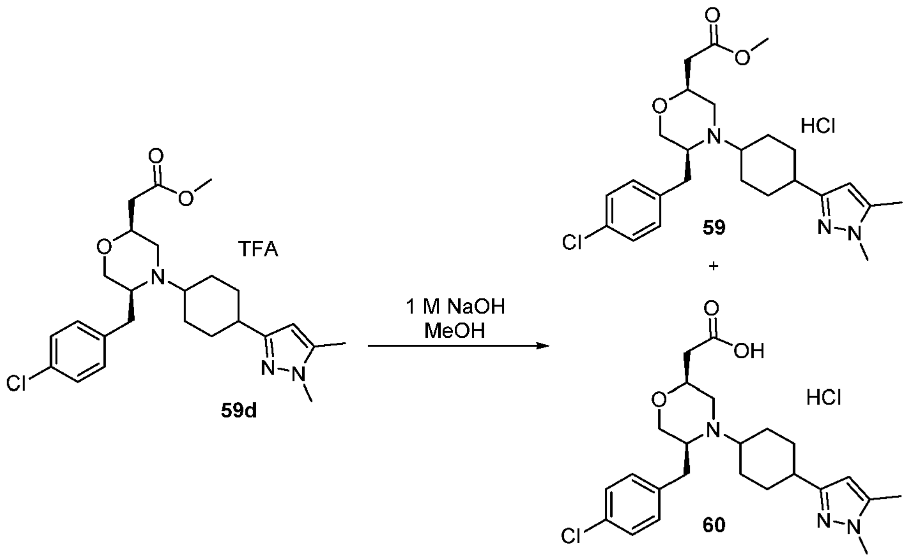


To the solution of **59d** (70 mg; 0.120 mmol) in MeOH (3 mL) 1 M NaOH (0.5 mL) was added and a whole was stirred at room temperature for 1 hour. The reaction progress was monitored by LC-MS. Then the solvent was evaporated *in vacuo* and the residue was purified by preparative reversed-phase column chromatography (Luna® 5μm Phenyl-Hexyl, LC Column 250 x 21.2 mm, water/MeCN + 1‰ TFA, 95:5 to 5:95, 30 min, 20 mL/min). Then the obtained products were transformed into hydrochloride salts with using HCl/AcOEt (2.6 M; 0.5 mL). The obtained solids of the hydrochloride salts were then lyophilized. The title compounds (**59** and **60**) were obtained as separated single isomers as hydrochloride salts in 10% yield (for **59**)(6 mg; 0.012 mmol) and in 53% yield (for **60**)(31 mg; 0.064 mmol).

For **59:** ESI-MS m/z for $C_{25}H_{35}ClN_3O_3$ found 460.2/462.2 $[M+H]^+$; Rt = 4.42 min (Method C); $^1H$ NMR (400 MHz,

Methanol-$d_4$) δ 7.41 - 7.26 (m, 4H), 6.45 (s, 1H), 4.38 - 4.22 (m, 1H), 3.95 (s, 3H), 3.90 - 3.80 (m, 2H), 3.76 - 3.66 (m, 5H), 3.63 - 3.53 (m, 1H), 3.40 - 3.33 (m, 1H), 3.28 - 3.11 (m, 2H), 2.95 - 2.83 (m, 1H), 2.81 - 2.67 (m, 2H), 2.56 - 2.37 (m, 5H), 2.30 - 2.20 (m, 2H), 1.90 - 1.63 (m, 4H).

For **60:** ESI-MS m/z for $C_{24}H_{33}ClN_3O_3$ found 446.2/448.2 $[M+H]^+$; Rt = 4.03 min (Method C); $^1H$ NMR (400 MHz, Methanol-$d_4$) δ 7.40 - 7.29 (m, 4H), 6.48 - 6.40 (m, 1H), 4.34 - 4.19 (m, 1H), 3.95 (s, 3H), 3.90 - 3.83 (m, 2H), 3.78 - 3.69 (m, 2H), 3.64 - 3.55 (m, 1H), 3.40 - 3.33 (m, 1H), 3.30 - 3.14 (m, 2H), 2.94 - 2.81 (m, 1H), 2.78 - 2.61 (m, 2H), 2.59 - 2.45 (m, 2H), 2.42 (s, 3H), 2.31 - 2.16 (m, 2H), 1.85 - 1.63 (m, 4H).

**Example 61.**

Synthesis of 2-((2*S*,5*S*)-5-(4-chlorobenzyl)-4-(4-(1,5-dimethyl-1*H*-pyrazol-3-yl)cyclohexyl)morpholin-2-yl)-*N*-ethylacetamide hydrochloride (**61**).

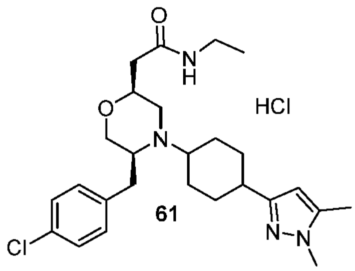

The title compound (**61**) was obtained as a hydrochloride salt as a single isomer in 2% overall yield in a similar way to Example 30 with the exception that, in the first step of the synthesis, ethylamine hydrochloride was used instead of propan-2-amine and the compound **59b** was used instead of the compound **5a** and in the second step of the synthesis, 1 M NaOH was used instead of 5% $NaHCO_3$. The crude product was purified by flash column chromatography on silica (AcOEt, 100%, then: AcOEt/MeOH, 80:20, v/v) and then purified by preparative reversed-phase column chromatography (Luna® 5μm Phenyl-Hexyl, LC Column 250 x 21.2 mm, water/MeCN + 1‰ TFA, 95:5 to 5:95, 30 min, 20 mL/min). Then the obtained product was transformed into hydrochloride salt with using HCl/AcOEt (3.6 M; 0.2 mL). The obtained solid of the hydrochloride salt was then lyophilized.

ESI-MS m/z for $C_{26}H_{38}ClN_4O_2$ found 473.2/475.2 $[M+H]^+$; Rt = 4.06 min (Method C); $^1H$ NMR (400 MHz, Methanol-$d_4$) δ 7.43 - 7.25 (m, 4H), 6.55 - 6.43 (m, 1H), 4.32 - 4.18 (m, 1H), 3.97 (s, 3H), 3.90 - 3.80 (m, 2H), 3.77 - 3.66 (m, 2H), 3.64 - 3.55 (m, 1H), 3.43 - 3.32 (m, 1H), 3.28 - 3.13 (m, 4H), 2.95 - 2.85 (m, 1H), 2.62 - 2.41 (m, 7H), 2.34 - 2.18 (m, 2H), 1.89 - 1.65 (m, 4H), 1.14 (t, *J* = 7.3 Hz, 3H).

**Example 62.**

Synthesis of (2*R*,5*S*)-5-(4-chlorobenzyl)-4-(4-(1,5-dimethyl-1*H*-1,2,4-triazol-3-yl)cyclohexyl)morpholine-2-carboxylic acid hydrochloride (**62**).

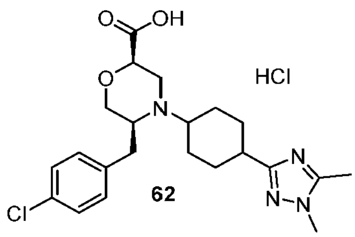

The title compound (**62**) was obtained as a hydrochloride salt as a single isomer in 1% overall yield in a similar way to Example 9 with the exception that, in the third step of the synthesis, ketone **15c** was used instead of ketone **1g**. The crude product was purified by preparative reversed-phase column chromatography (Luna® 5μm Phenyl-Hexyl, LC Column 250 x 21.2 mm, water/MeCN + 1‰ TFA, 95:5 to 20:80, 40 min, 12 mL/min). Then the obtained product was transformed into hydrochloride salt with using of 1 M HCl (2 mL). The obtained solid of the hydrochloride salt was then lyophilized.

ESI-MS m/z for $C_{22}H_{30}ClN_4O_3$ found 433.2/435.2 $[M+H]^+$; Rt = 3.40 min (Method C); $^1H$ NMR (700 MHz, DMSO-$d_6$ + $D_2O$, 348 K) δ 7.40 - 7.36 (m, 2H), 7.33 - 7.30 (m, 2H), 4.52 - 4.44 (m, 1H), 3.80 - 3.76 (m, 1H), 3.76 - 3.71 (m, 2H), 3.68 (s, 3H),

3.56 - 3.52 (m, 1H), 3.50 - 3.44 (m, 2H), 3.16 - 3.10 (m, 1H), 3.10 - 3.00 (m, 1H), 2.64 - 2.58 (m, 1H), 2.35 (s, 3H), 2.23 - 2.06 (m, 4H), 1.68 - 1.42 (m, 4H).

**Example 63.**

Synthesis of (2*R*,5*S*)-5-(4-bromobenzyl)-4-(4-(1,5-dimethyl-1*H*-pyrazol-3-yl)cyclohexyl)-2-((methylsulfonyl)methyl) morpholine hydrochloride (**63**).

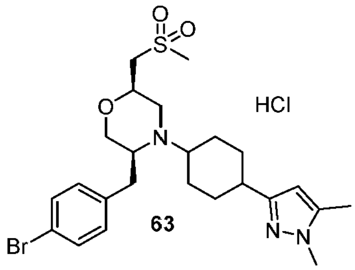

**63**

Step 1.

Synthesis of (2*S*)-2-amino-3-(4-bromophenyl)propan-1-ol (**63a**).

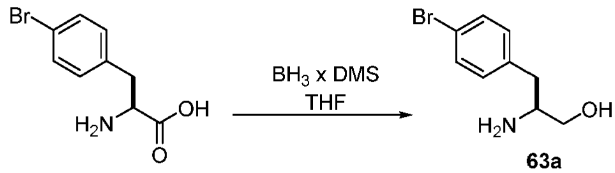


**63a**

The title compound (**63a**) was obtained from optically pure L-*p*-bromophenylalanine ((2*S*)-2-amino-3-(4-bromophenyl)propanoic acid) (2.8 g; 11.47 mmol) according to the General Procedure **Ia** in 87% yield (2.6 g; 11.36 mmol).

Step 2.

Synthesis of (*R*)-2-bromo-*N*-((*S*)-1-(4-bromophenyl)-3-hydroxypropan-2-yl)-3-(tert-butoxy)-propanamide (**63b**).

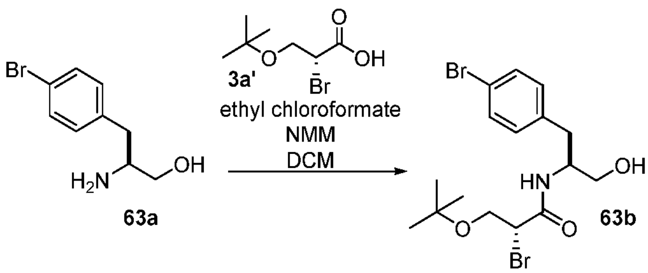


**63a** **63b**

The title compound (**63b**) was obtained from the compound **63a** (7.8 g; 34.0 mmol) and (*R*)-2-bromo-3-(*tert*-butoxy)propanoic acid (**3a**') (9.5 g; 42.0 mmol) according to the General Procedure **V** in 99% yield (14.7 g; 33.7 mmol).

Step 3.

Synthesis of (2*S*,5*S*)-5-(4-bromobenzyl)-2-(*tert*-butoxymethyl)morpholin-3-one (**63c**).

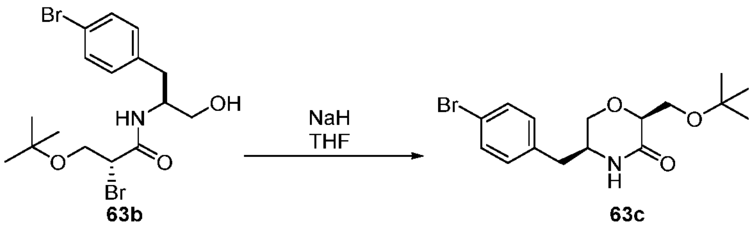


**63b** **63c**

The title compound (**63c**) was obtained from **63b** (16.05 g, 37.0 mmol) according to the General Procedure **II** in 51% yield (6.7 g; 18.8 mmol).
ESI-MS m/z for $C_{12}H_{15}BrNO_3$ found 300.0/302.0 [M+H-*t*Bu]$^+$; Rt= 5.75 min (Method C)

Step 4.

Synthesis of ((2*R*,5*S*)-5-(4-bromobenzyl)morpholin-2-yl)methanol (**63d**).

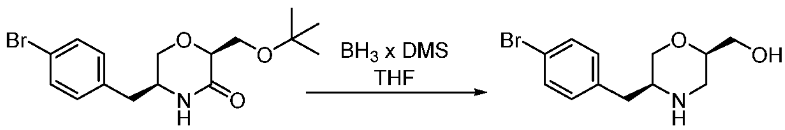


**63c** **63d**

The title compound (**63d**) was obtained from **63c** (5.7 g; 16.1 mmol) according to the General Procedure **Ib** in 68% yield (3.1 g; 10.9 mmol).
ESI-MS m/z for $C_{12}H_{17}BrNO_2$ found 285.7/287.7 [M+H]$^+$; Rt= 2.96 min (Method C)

Step 5.

Synthesis of *tert*-butyl (2*R*,5*S*)-5-(4-bromobenzyl)-2-(*tert*-butoxymethyl)morpholine-4-carboxylate (**63e**).

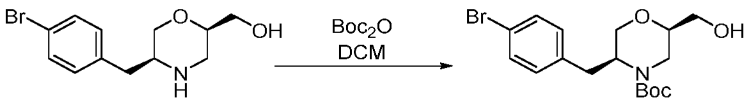


**63d** **63e**

To a solution of amino alcohol **63d** (2.25 g, 7.86 mmol) in dichloromethane (80 mL), di-*tert*-butyl dicarbonate ($Boc_2O$) (1.62 g, 7.47 mmol) was added and the reaction mixture was stirred at room temperature overnight, after which time TLC showed almost complete consumption of the starting material. The volatiles were removed *in vacuo* and the residue was purified by silica-gel column chromatography (DCM/MeOH, 100:1, *v*/*v*). Compound **63e** was obtained in 82% yield (2.48 g; 6.44 mmol).
ESI-MS m/z for $C_{12}H_{17}BrNO$; found 286.0/288.0 [M+H-Boc]$^+$; Rt = 5.81 min (Method C); $^1$H NMR (700 MHz, DMSO-$d_6$+ $D_2O$, 348 K) δ 7.44 - 7.39 (m, 2H), 7.15 - 7.10 (m, 2H), 4.08 - 4.00 (m, 1H), 3.84 - 3.75 (m, 1H), 3.72 - 3.66 (m, 1H), 3.53 - 3.46 (m, 2H), 3.46 - 3.40 (m, 2H), 3.36 - 3.30 (m, 1H), 2.96 - 2.84 (m, 2H), 2.84 - 2.77 (m, 1H), 1.22 (s, 9H).

Step 6.

Synthesis of *tert*-butyl (2*R*,5*S*)-5-(4-bromobenzyl)-2-(((methylsulfonyl)oxy)methyl)morpholine-4-carboxylate (**63f**).

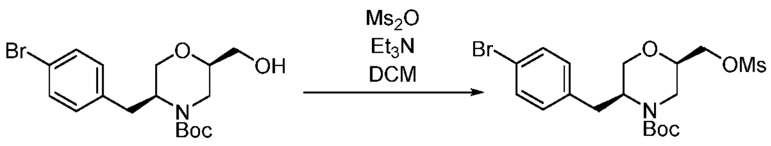


63e 63f

The title compound (**63f**) was obtained from **63e** (965 mg; 2.5 mmol) according to the General Procedure **VIII** in 98% yield (1.14 g; 2.46 mmol).
ESI-MS m/z for $C_{18}H_{26}BrNO_6SNa$ found 485.9/487.9 $[M+Na]^+$; Rt= 6.35 min (Method C)

Step 7.

Synthesis of *tert*-butyl (2*R*,5*S*)-5-(4-bromobenzyl)-2-((methylthio)methyl)morpholine-4-carboxylate (**63g**).

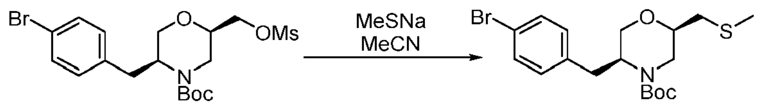


63f 63g

To a solution of **63f** (0.9 g; 1.94 mmol) in acetonitrile (3.5 mL) MeSNa (365 mg; 5.21 mmol) was added and the reaction mixture was stirred at 105 °C overnight. The reaction progress was monitored by LC-MS analysis. After analytical control indicated completion of the reaction, the mixture was dissolved in water (5.6 mL) and MeCN was removed *in vacuo.* An aqueous phase was extracted with DCM (4 x). The combined organic solutions were washed with 1 M NaOH and brine, dried over anhydrous $Na_2SO_4$, filtered and concentrated *in vacuo* and the crude product was used to the next step without additional purification. Compound **63g** was obtained in 87% yield (0.7 g; 1.69 mmol).
ESI-MS m/z for $C_{13}H_{19}BrNOS$ found 316.0/318.0 $[M+H-Boc]^+$; Rt= 6.65 min (Method C)

Step 8.

Synthesis of *tert*-butyl (2*R*,5*S*)-5-(4-bromobenzyl)-2-((methylsulfonyl)methyl)morpholine-4-carboxylate (**63h**).

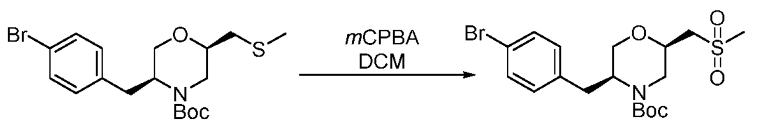


63g 63h

To a solution of **63g** (0.7 g; 1.69 mmol) in DCM (9.9 mL) *m*CPBA (1.39 g; 8.0 mmol) was added portionwise at 0 °C. Then the cooling bath was removed and the mixture was stirred at room temperature overnight. The reaction progress was monitored by LC-MS analysis. When analysis indicated completion of the reaction, the reaction was quenched with 10% $Na_2SO_3$ and stirred for 30 minutes at room temperature. Then phases were separated and the organic one was washed with 1 M NaOH (4 x) and brine, dried over anhydrous $Na_2SO_4$, filtered and concentrated *in vacuo* and the crude product was used to the next step without additional purification. Compound **63h** was obtained in 86% yield (0.65 g; 1.45 mmol).
ESI-MS m/z for $C_{13}H_{19}BrNO_3S$ found 348.0/350.0 $[M+H-Boc]^+$; Rt= 6.19 min (Method C)

Step 9.

Synthesis of (2*R*,5*S*)-5-(4-bromobenzyl)-2-((methylsulfonyl)methyl)morpholine (**63i**).

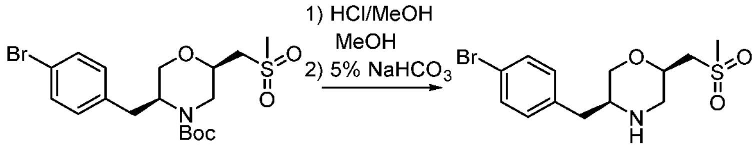


63h 63i

The title compound (**63i**) was obtained as a free amine in 91% yield (423 mg; 1.22 mmol) from **63h** (600 mg; 1.34 mmol) according to the General Procedure **IVa** and then after basic (5% $NaHCO_3$) extraction with DCM. ESI-MS m/z for $C_{13}H_{19}BrNO_3S$ found 348.0/350.0 $[M+H]^+$; $R_t$ = 3.29 min (Method C)

Step 10.

Synthesis of (2*R*,5*S*)-5-(4-bromobenzyl)-4-(4-(1,5-dimethyl-1*H*-pyrazol-3-yl)cyclohexyl)-2-((methylsulfonyl)methyl) morpholine hydrochloride (**63**).

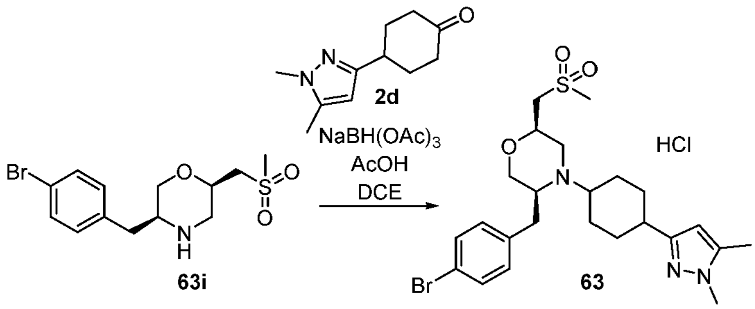


2d 63i 63

The title compound (**63**) was obtained as a single isomer as a hydrochloride salt from **63i** (180 mg; 0.52 mmol) and ketone **2d** (100 mg; 0.52 mmol) according to the General Procedure **VII** in 15% yield (45 mg; 0.08 mmol). The crude product was purified by flash column chromatography on silica (hexane/AcOEt, 6:4 to 0:100, v/v, then: AcOEt/MeOH, 98:2 to 96:4, v/v) and then the obtained product was transformed into hydrochloride salt with using of 1 M HCl (1 mL). The obtained solid of the hydrochloride salt was then lyophilized.
ESI-MS m/z for $C_{24}H_{35}BrN_3O_3S$ found 524.1/526.1 $[M+H]^+$; Rt = 4.19 min (Method C); $^1H$ NMR (700 MHz, DMSO-$d_6$ + $D_2O$, 348 K) δ 7.54 - 7.50 (m, 2H), 7.28 - 7.24 (m, 2H), 5.82 (s, 1H), 4.33 - 4.27 (m, 1H), 3.87 - 3.75 (m, 2H), 3.70 - 3.66 (m, 1H), 3.61 (s, 3H), 3.60 - 3.55 (m, 2H), 3.47 - 3.35 (m, 3H), 3.23 - 3.16 (m, 2H), 3.12 - 3.08 (m, 1H), 3.03 (s, 3H), 2.28 - 2.22 (m, 2H), 2.18 (s, 3H), 2.09 - 2.02 (m, 2H), 1.60 - 1.52 (m, 2H), 1.52 - 1.41 (m, 2H).

**Example 64.**

Synthesis of *N*-(((2*R*,5*S*)-5-(4-chlorobenzyl)-4-(4-(1,5-dimethyl-1*H*-pyrazol-3-yl)cyclohexyl)morpholin-2-yl)methyl) methanesulfonamide hydrochloride (**64**).

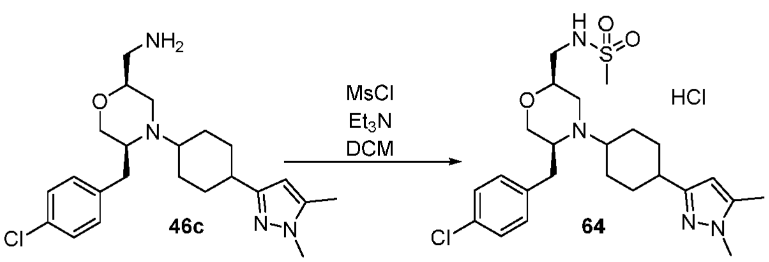


46c 64

The title compound (**64**) was obtained as a hydrochloride salt as a single isomer in 30% yield (44 mg; 0.09 mmol) from **46c** (126 mg; 0.30 mmol) according to the General Procedure **X.** The crude product was purified by preparative reversed-phase column chromatography (Luna® 5µm Phenyl-Hexyl, LC Column 250 x 21.2 mm, water/MeCN + 1‰ TFA,

95:5 to 20:80, 40 min, 12 mL/min). Then the obtained product was transformed into hydrochloride salt with using of 1 M HCl (2 mL). The obtained solid of the hydrochloride salt was then lyophilized.
ESI-MS m/z for $C_{24}H_{36}ClN_4O_3S$ found 495.2/497.2 $[M+H]^+$; Rt = 4.10 min (Method C); $^1H$ NMR (400 MHz, Methanol-$d_4$) δ 7.43 - 7.28 (m, 4H), 6.44 (s, 1H), 4.02 - 3.96 (m, 1H), 3.94 (s, 3H), 3.92 - 3.75 (m, 3H), 3.69 - 3.55 (m, 2H), 3.42 - 3.33 (m, 2H), 3.27 - 3.16 (m, 2H), 3.21 - 3.11 (m, 1H), 2.99 (s, 3H), 2.95 - 2.80 (m, 1H), 2.56 - 2.44 (m, 2H), 2.42 (s, 3H), 2.30 - 2.18 (m, 2H), 1.88 - 1.60 (m, 4H).

**Example 65.**

Synthesis of (2*R*,5*S*)-5-(4-bromobenzyl)-4-(4-(1,5-dimethyl-1*H*-pyrazol-3-yl)cyclohexyl)morpholine-2-carboxylic acid hydrochloride (**65**).

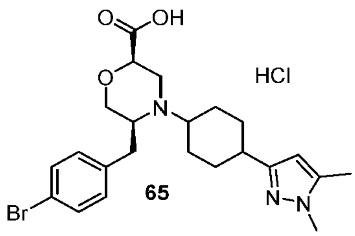

**65**

Step 1.

Synthesis of (2*R*,5*S*)-5-(4-bromobenzyl)-4-(tert-butoxycarbonyl)morpholine-2-carboxylic acid (**65a**).

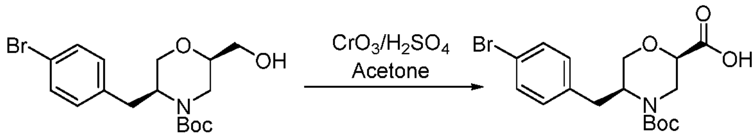


**63e** **65a**

To a solution of an alcohol **63e** (0.7 g; 1.80 mmol) in acetone (18.5 mL) at 0 °C, Jones reagent (2.1 mL; 2.6 M) was added dropwise. The reaction mixture was stirred at 0 °C for 2 hours, and then isopropanol (*i*PrOH) (4.5 mL) was added. After 10 minutes ethyl acetate was added and the mixture was filtered through a pad of the Celite. The filtrate was washed with brine, dried over anhydrous $Na_2SO_4$ and evaporated *in vacuo* affording the title compound **65a** in 79% yield (566 mg; 1.42 mmol).
ESI-MS m/z for $C_{12}H_{15}BrNO_3$ found 300.0/302.0 $[M+H-Boc]^+$; $R_t$ = 5.87 min (Method C)

Step 2.

Synthesis of methyl (2*R*,5*S*)-5-(4-bromobenzyl)morpholine-2-carboxylate (**65b**).

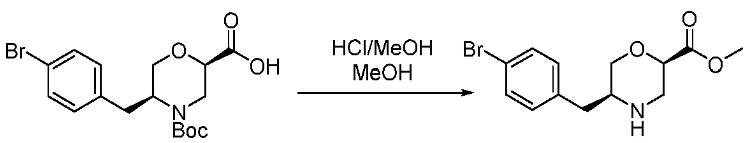


**65a** **65b**

To a solution of **65a** (566 mg; 1.42 mmol) in MeOH (12 mL) a solution of HCl/MeOH (3.8 M; 2.2 mL) was added dropwise at 0 °C. Then the cooling bath was removed and the reaction mixture was stirred at room temperature overnight. After reaction was completed as judged by LC-MS, the solvent was evaporated *in vacuo.* Then another portion of

HCl/MeOH (3.8 M; 4 mL) was added and the reaction mixture was stirred at 45 °C for 3 hours. The residue was taken between 5% $NaHCO_3$ and DCM and an aqueous phase was extracted with DCM (2 x). The combined organic solutions were dried over anhydrous $Na_2SO_4$, filtered and concentrated *in vacuo.* The crude product was used to the next step without additional purification. Compound **65b** was obtained in 72% yield (321 mg; 1.02 mmol).
ESI-MS m/z for $C_{13}H_{17}BrNO_3$ found 314.0/316.0 $[M+H]^+$; $R_t$ = 3.37 min (Method C)

Step 3.

Synthesis of methyl (2*R*,5*S*)-5-(4-bromobenzyl)-4-(4-(1,5-dimethyl-1*H*-pyrazol-3-yl)cyclohexyl)morpholine-2-carboxylate (**65c**).

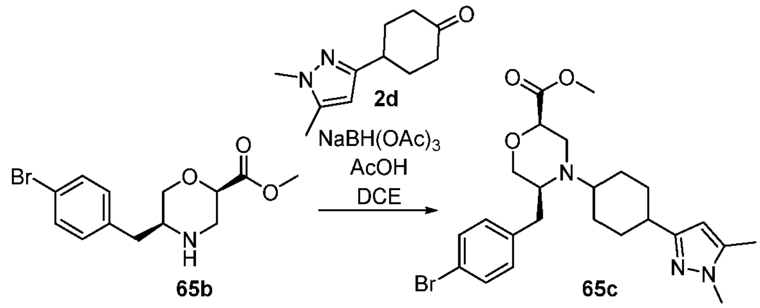


65b 65c

The title compound (**65c**) was obtained from **65b** (220 mg; 0.70 mmol) and ketone **2d** (135 mg; 0.70 mmol) according to the General Procedure **VII** in 21% yield (74 mg; 0.15 mmol).
ESI-MS m/z for $C_{24}H_{33}BrN_3O_3$ found 490.0/492.0 $[M+H]^+$; Rt = 4.21 min (Method C)

Step 4.

Synthesis of (2*R*,5*S*)-5-(4-bromobenzyl)-4-(4-(1,5-dimethyl-1*H*-pyrazol-3-yl)cyclohexyl)morpholine-2-carboxylic acid hydrochloride (**65**).

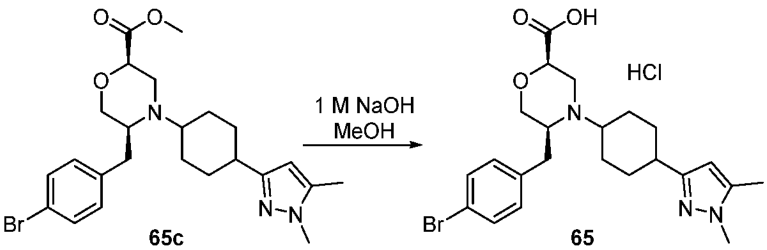


65c 65

To the solution of **65c** (74 mg; 0.15 mmol) in MeOH (1 mL) 1 M NaOH (1 mL) was added and whole was stirred at room temperature for 2 hours. The reaction progress was monitored by LC-MS. When analysis indicated completion of the reaction, the solvent was evaporated *in vacuo* and the residue was dissolved in MeCN/water and to this solution 5% TFA was added to pH 3. The crude product was then purified by preparative reversed-phase column chromatography (Luna® 5μm Phenyl-Hexyl, LC Column 250 x 21.2 mm, water/MeCN + 1‰ TFA, 95:5 to 20:80, 40 min, 12 mL/min) and then the obtained product was transformed into hydrochloride salt with using of 1 M HCl (2 mL). The title compound **(65)** was obtained as a single isomer as a hydrochloride salt in 73% yield (58 mg; 0.11 mmol).
ESI-MS m/z for $C_{23}H_{31}BrN_3O_3$ found 476.1/478.1 $[M+H]^+$; Rt = 4.12 min (Method C); $^1H$ NMR (400 MHz, Methanol-$d_4$) δ 7.60 - 7.47 (m, 2H), 7.33 - 7.22 (m, 2H), 6.33 (s, 1H), 4.71 - 4.49 (m, 1H), 4.07 - 3.79 (m, 7H), 3.71 - 3.41 (m, 2H), 3.27 - 3.03 (m, 2H), 2.90 - 2.76 (m, 1H), 2.59 - 2.34 (m, 5H), 2.28 - 2.16 (m, 2H), 1.92 - 1.57 (m, 4H).

**Example 66.**

Synthesis of (2R,5S)-5-(4-bromobenzyl)-4-(4-(5-methyloxazol-2-yl)cyclohexyl)morpholine-2-carboxylic acid 2,2,2-trifluoroacetate **(66).**

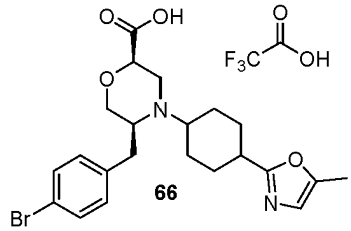

66

The title compound **(66)** was obtained as a TFA salt as a single isomer in 2% overall yield in a similar way to Example 65 with the exception that, in the third step of the synthesis, ketone **1g** was used instead of ketone **2d.** The crude product was purified by preparative reversed-phase column chromatography (Luna® 5μm Phenyl-Hexyl, LC Column 250 x 21.2 mm, water/MeCN + 1‰ TFA, 95:5 to 20:80, 40 min, 12 mL/min).
ESI-MS m/z for $C_{22}H_{28}BrN_2O_4$ found 463.1/465.1 $[M+H]^+$; Rt = 4.14 min (Method C); $^1H$ NMR (400 MHz, Methanol-$d_4$) δ 7.57 - 7.50 (m, 2H), 7.31 - 7.23 (m, 2H), 6.70 (s, 1H), 4.62 - 4.51 (m, 1H), 3.96 - 3.59 (m, 6H), 3.25 - 3.06 (m, 2H), 2.92 - 2.73 (m, 1H), 2.49 - 2.20 (m, 7H), 1.77 - 1.62 (m, 4H).

**Example 67.**

Synthesis of (2*R*,5*S*)-5-(4-bromobenzyl)-4-(4-(5-methyloxazol-2-yl)cyclohexyl)-2-((methylsulfonyl)methyl)morpholine 2,2,2-trifluoroacetate **(67).**

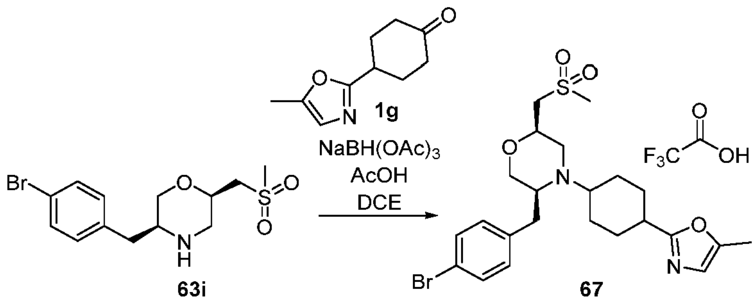


63i

67

The title compound **(67)** was obtained as a single isomer as a TFA salt from **63i** (105 mg; 0.30 mmol) and ketone **1g** (54 mg; 0.30 mmol) according to the General Procedure **VII** in 47% yield (87 mg; 0.14 mmol). The crude product was purified by preparative reversed-phase column chromatography (Luna® 5μm Phenyl-Hexyl, LC Column 250 x 21.2 mm, water/MeCN + 1‰ TFA, 95:5 to 20:80, 40 min, 12 mL/min).
ESI-MS m/z for $C_{23}H_{32}BrN_2O_4S$ found 511.1/513.1 $[M+H]^+$; Rt = 4.19 min (Method C); $^1H$ NMR (400 MHz, Methanol-$d_4$) δ 7.58 - 7.49 (m, 2H), 7.31 - 7.22 (m, 2H), 6.71 (s, 1H), 4.42 - 4.28 (m, 1H), 3.96 - 3.52 (m, 6H), 3.47 - 3.36 (m, 2H), 3.29 - 3.23 (m, 1H), 3.20 - 3.12 (m, 1H), 3.10 (s, 3H), 2.91 - 2.79 (m, 1H), 2.54 - 2.40 (m, 2H), 2.38 - 2.27 (m, 5H), 1.80 - 1.52 (m, 4H).

**Example 68.**

Synthesis of (2*R*,5*S*)-5-(4-bromobenzyl)-4-(4-(1,5-dimethyl-1*H*-1,2,4-triazol-3-yl)cyclohexyl)-2-((methylsulfonyl) methyl)morpholine hydrochloride **(68).**

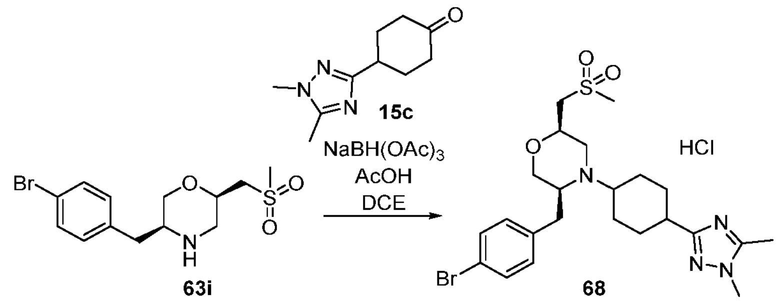


63i 68

The title compound **(68)** was obtained as a single isomer as a hydrochloride salt from **63i** (118 mg; 0.34 mmol) and ketone **15c** (61 mg; 0.34 mmol) according to the General Procedure **VII** in 15% yield (30 mg; 0.05 mmol). The crude product was purified by preparative reversed-phase column chromatography (Luna® 5μm Phenyl-Hexyl, LC Column 250 x 21.2 mm, water/MeCN + 1‰ TFA, 95:5 to 20:80, 40 min, 12 mL/min) and then the obtained product was transformed into hydrochloride salt with using of 1 M HCl (2 mL).

ESI-MS m/z for $C_{23}H_{34}BrN_4O_3S$ found 525.1/527.1 $[M+H]^+$; Rt = 3.67 min (Method C); $^1H$ NMR (400 MHz, Methanol-$d_4$) δ 7.58 - 7.42 (m, 2H), 7.35 - 7.17 (m, 2H), 4.53 - 4.35 (m, 1H), 4.02 - 3.85 (m, 5H), 3.85 - 3.52 (m, 4H), 3.47 - 3.35 (m, 2H), 3.28 - 3.22 (m, 1H), 3.21 - 3.12 (m, 1H), 3.07 (s, 3H), 3.03 - 2.92 (m, 1H), 2.68 (s, 3H), 2.55 - 2.14 (m, 4H), 2.06 - 1.69 (m, 4H).

**Example** 69.

Synthesis of (2R,5S)-5-(4-bromobenzyl)-4-(4-(1,5-dimethyl-1H-1,2,4-triazol-3-yl)cyclohexyl)morpholine-2-carboxylic acid hydrochloride **(69).**

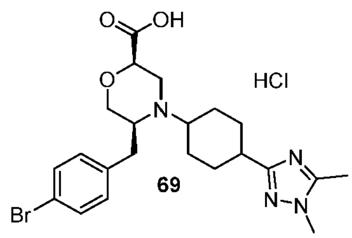

69

Step 1.

Synthesis of methyl (2R,55)-5-(4-bromobenzyl)-4-(4-(1,5-dimethyl-1H-1,2,4-triazol-3-yl)cyclohexyl)morpholine-2-carboxylate **(69a).**

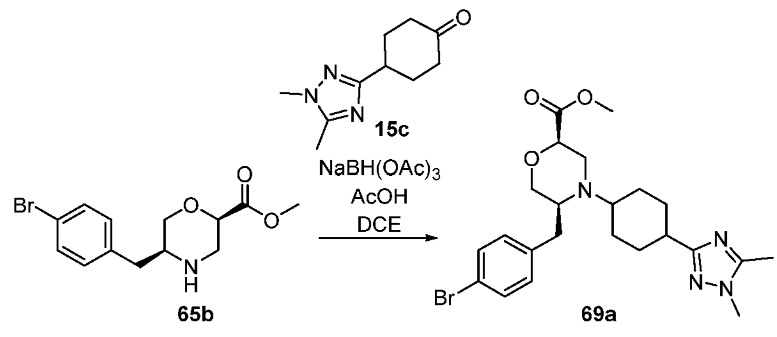


65b 69a

The title compound **(69a)** was obtained from **65b** (154 mg; 0.49 mmol) and ketone 15c (95 mg; 0.49 mmol) according to the General Procedure **VII** in 47% yield (112 mg; 0.23 mmol). ESI-MS m/z for $C_{23}H_{32}BrN_4O_3$ found 491.0/493.0 $[M+H]^+$; Rt = 3.78 min (Method C)

Step 2.

Synthesis of (2*R*,5*S*)-5-(4-bromobenzyl)-4-(4-(1,5-dimethyl-1*H*-1,2,4-triazol-3-yl)cyclohexyl)morpholine-2-carboxylic acid hydrochloride **(69).**

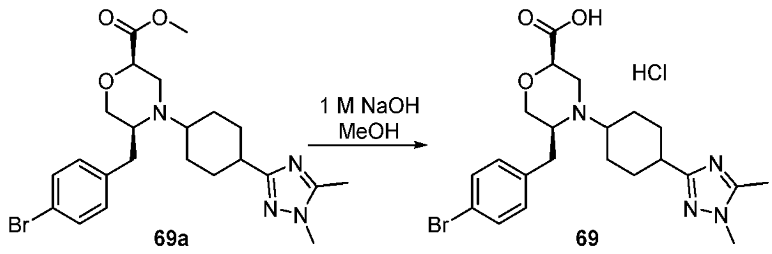


To the solution of **69a** (112 mg; 0.23 mmol) in MeOH (1 mL) 1 M NaOH (1 mL) was added and whole was stirred at room temperature for 2 hours. The reaction progress was monitored by LC-MS. When analysis indicated completion of the reaction, the solvent was evaporated *in vacuo* and the residue was dissolved in MeCN/water and to this solution 5% TFA was added to pH 3. The crude product was then purified by preparative reversed-phase column chromatography (Luna® 5μm Phenyl-Hexyl, LC Column 250 x 21.2 mm, water/MeCN + 1‰ TFA, 95:5 to 20:80, 40 min, 12 mL/min) and then the obtained product was transformed into hydrochloride salt with using of 1 M HCl (2 mL). The title compound **(69)** was obtained as a single isomer as a hydrochloride salt in 43% yield (53 mg; 0.10 mmol).
ESI-MS m/z for $C_{22}H_{30}BrN_4O_3$ found 477.1/479.1 $[M+H]^+$; Rt = 3.30 min (Method C); $^1H$ NMR (400 MHz, Methanol-$d_4$) δ 7.58 - 7.47 (m, 2H), 7.34 - 7.23 (m, 2H), 4.72 - 4.54 (m, 1H), 4.01 - 3.77 (m, 7H), 3.70 - 3.58 (m, 1H), 3.57 - 3.41 (m, 1H), 3.39 - 3.31 (m, 1H), 3.29 - 3.10 (m, 1H), 3.07 - 2.93 (m, 1H), 2.71 (s, 3H), 2.58 - 2.13 (m, 4H), 2.08 - 1.60 (m, 4H).

**Example 70.**

Synthesis of *N*-(((2*R*,5*S*)-5-(4-chlorobenzyl)-4-(4-(1,5-dimethyl-1*H*-pyrazol-3-yl)cyclohexyl)morpholin-2-yl)methyl)-1, 1, 1-trifluoromethanesulfonamide hydrochloride **(70).**

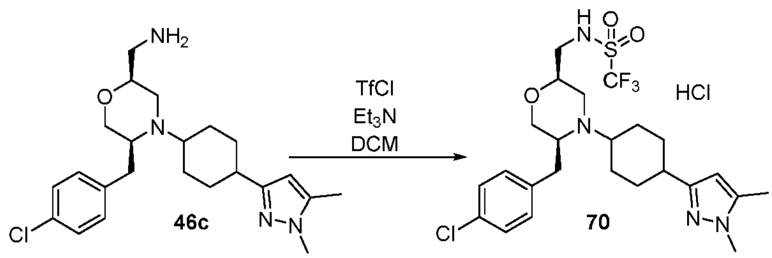


The title compound **(70)** was obtained as a hydrochloride salt as a single isomer in 24% yield (54 mg; 0.09 mmol) from **46c** (153 mg; 0.37 mmol) according to the General Procedure **X** with the exception that, the TfCl was added to the reaction mixture at 0 °C and then the reaction was allowed to room temperature. The crude product was purified by preparative reversed-phase column chromatography (Luna® 5μm Phenyl-Hexyl, LC Column 250 x 21.2 mm, water/MeCN + 1‰ TFA, 95:5 to 20:80, 40 min, 12 mL/min). Then the obtained product was transformed into hydrochloride salt with using HCl/AcOEt (3.6 M; 0.2 mL). The obtained solid of the hydrochloride salt was then lyophilized.
ESI-MS m/z for $C_{24}H_{33}ClF_3N_4O_3S$ found 549.2/551.2 $[M+H]^+$; Rt = 4.67 min (Method C); $^1H$ NMR (400 MHz, Methanol-$d_4$) δ 7.46 - 7.21 (m, 4H), 6.30 (s, 1H), 4.09 - 3.94 (m, 1H), 3.94 - 3.74 (m, 6H), 3.74 - 3.35 (m, 5H), 3.24 - 3.12 (m, 2H), 2.88 - 2.71 (m, 1H), 2.55 - 2.42 (m, 2H), 2.37 (s, 3H), 2.29 - 2.12 (m, 2H), 1.86 - 1.54 (m, 4H).

**Example 71.**

Synthesis of (2*R*,5*S*)-5-(4-bromobenzyl)-4-(4-(1,5-dimethyl-1*H*-pyrazol-3-yl)cyclohexyl)-*N*-ethylmorpholine-2-carboxamide hydrochloride **(71).**

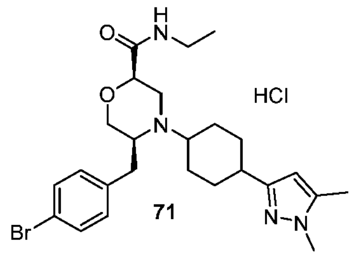

The title compound **(71)** was obtained as a hydrochloride salt as a single isomer in 10% overall yield in a similar way to Example 30 with the exception that, in the first step of the synthesis, ethylamine hydrochloride was used instead of propan-2-amine and the compound **65a** was used instead of the compound **5a** and in the second step of the synthesis, the General Procedure **IVb** was used instead of the General Procedure **IVa.** The crude product was purified by preparative reversed-phase column chromatography (Luna® 5μm Phenyl-Hexyl, LC Column 250 x 21.2 mm, water/MeCN + 1‰ TFA, 95:5 to 20:80, 30 min, 12 mL/min). Then the obtained product was transformed into hydrochloride salt with using HCl/AcOEt (3.6 M; 0.2 mL). The obtained solid of the hydrochloride salt was then lyophilized.
ESI-MS m/z for $C_{25}H_{36}BrN_4O_2$ found 503.2/505.2 $[M+H]^+$; Rt = 4.21 min (Method C); $^1$H NMR (400 MHz, Methanol-$d_4$) δ 7.57 - 7.52 (m, 2H), 7.35 - 7.28 (m, 2H), 6.43 (s, 1H), 4.65 - 4.38 (m, 1H), 4.01 - 3.79 (m, 7H), 3.68 - 3.54 (m, 1H), 3.42 - 3.32 (m, 2H), 3.29 - 3.02 (m, 3H), 2.93 - 2.74 (m, 1H), 2.58 - 2.45 (m, 2H), 2.42 (s, 3H), 2.32 - 2.17 (m, 2H), 1.88 - 1.57 (m, 4H), 1.17 (t, *J* = 7.2 Hz, 3H).

**Example 72.**

Synthesis of (2*R*,5*S*)-*N*-(2-(3-(but-3-yn-1-yl)-3*H*-diazirin-3-yl)ethyl)-5-(4-chlorobenzyl)-4-(4-(1,5-dimethyl-1*H*-pyrazol-3-yl)cyclohexyl)morpholine-2-carboxamide hydrochloride **(72).**

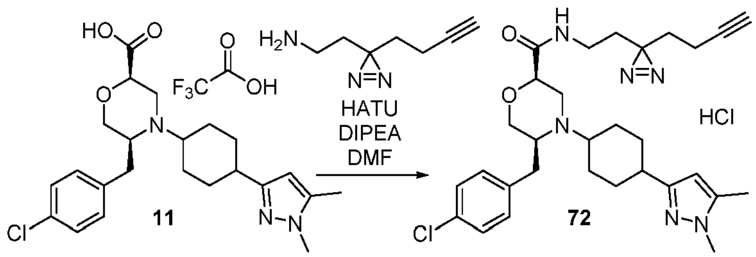


To the solution of **11** (30 mg; 0.055 mmol) in dry DMF (0.33 mL) diisopropylethylamine (DIPEA; 38 μL; 0.220 mmol), 2-(3-(but-3-yn-1-yl)-3H-diazirin-3-yl)ethan-1-amine (10 mg; 0.072 mmol) were added and after 5 minutes HATU (23 mg; 0.060 mmol) was added and the reaction mixture was stirred at room temperature overnight. The reaction progress was monitored by TLC and LC-MS. When analyses indicated completion of the reaction, the mixture was concentrated *in vacuo,* diluted with water and the crude product was purified by preparative reversed-phase column chromatography (Luna® 5μm Phenyl-Hexyl, LC Column 250 x 21.2 mm, water/MeCN + 1‰ TFA, 95:5 to 20:80, 30 min, 12 mL/min). Then the obtained product was transformed into hydrochloride salt with using HCl/AcOEt (3.6 M; 0.2 mL). The obtained solid of the hydrochloride salt was then lyophilized. The title compound **(72)** was obtained as a hydrochloride salt as a single isomer in 67% yield (22 mg; 0.037 mmol).
ESI-MS m/z for $C_{30}H_{40}ClN_6O_2$ found 551.2/553.2 $[M+H]^+$; Rt = 4.73 min (Method C); $^1$H NMR (400 MHz, Methanol-$d_4$) δ 7.42 - 7.34 (m, 4H), 6.48 (s, 1H), 4.65 - 4.47 (m, 1H), 4.09 - 3.73 (m, 8H), 3.72 - 3.51 (m, 1H), 3.47 - 3.32 (m, 1H), 3.27 - 3.14 (m, 3H), 2.94 - 2.83 (m, 1H), 2.58 - 2.45 (m, 2H), 2.43 (s, 3H), 2.34 - 2.20 (m, 3H), 2.10 - 2.00 (m, 2H), 1.95 - 1.81 (m, 2H), 1.79 - 1.58 (m, 6H).

**Example 73.**

Synthesis of 2-(((2*R*,5*S*)-5-(4-chlorobenzyl)-4-(4-(4,5-dimethylthiazol-2-yl)cyclohexyl)morpholin-2-yl)methoxy)acetic acid hydrochloride **(73).**

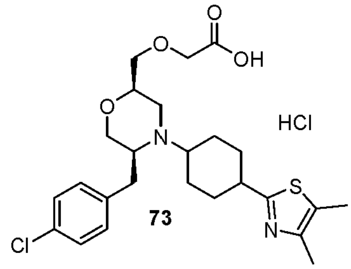

**73**

Step 1.

Synthesis of *tert*-butyl (2*R*,5*S*)-5-(4-chlorobenzyl)-2-((2-ethoxy-2-oxoethoxy)methyl)morpholine-4-carboxylate **(73a).**

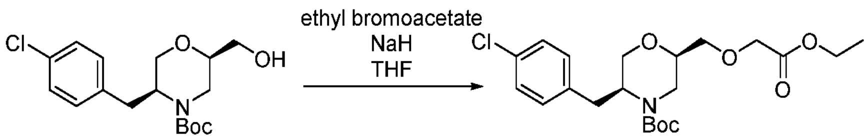


**3d** **73a**

NaH (60% in oil; 88 mg; 2.2 mmol) was suspended in the solvent (2 x hexane and 2 x THF) and after that the solvent was removed by syringe. Then a solution of **3d** (0.5 g; 1.47 mmol) in THF (3 mL) was added at 0 °C. After 2 minutes the bath was removed and ethyl bromoacetate (327 μL; 2.93 mmol) was slowly added and then stirred at room temperature overnight. The reaction progress was monitored by LC-MS analysis of small aliquots of the crude reaction mixture. When analysis indicated completion of the reaction, the mixture was quenched with EtOH. The volatiles were removed *in vacuo* and the crude product was dissolved in DCM/water. Then an aqueous phase was extracted with DCM (2 x). The combined organic phases were dried over anhydrous $Na_2SO_4$, concentrated *in vacuo.* The crude product was used to the next step without additional purification. Compound **73a** was obtained in 99% yield (619 mg; 1.45 mmol). ESI-MS m/z for $C_{21}H_{30}ClNO_6Na$ found 450.0/452.0 $[M+Na]^+$; Rt = 4.00 min (Method D) Step 2.

Synthesis of methyl 2-(((2*R*,5*S*)-5-(4-chlorobenzyl)morpholin-2-yl)methoxy)acetate **(73b).**

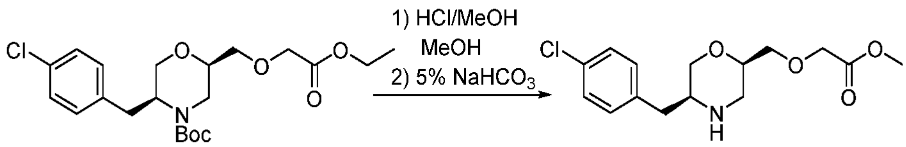


**73a** **73b**

The title compound **(73b)** was obtained as a free amine in 64% yield (172 mg; 0.55 mmol) from **73a** (366 mg; 0.86 mmol) according to the General Procedure **IVa** and then after basic (5% $NaHCO_3$) extraction with DCM.
ESI-MS m/z for $C_{15}H_{21}ClNO_4$ found 314.0/316.0 $[M+H]^+$; Rt = 1.25 min (Method D)

Step 3.

Synthesis of methyl 2-(((2*R*,5*S*)-5-(4-chlorobenzyl)-4-(4-(4,5-dimethylthiazol-2-yl)cyclohexyl)morpholin-2-yl)methoxy) acetate **(73c).**

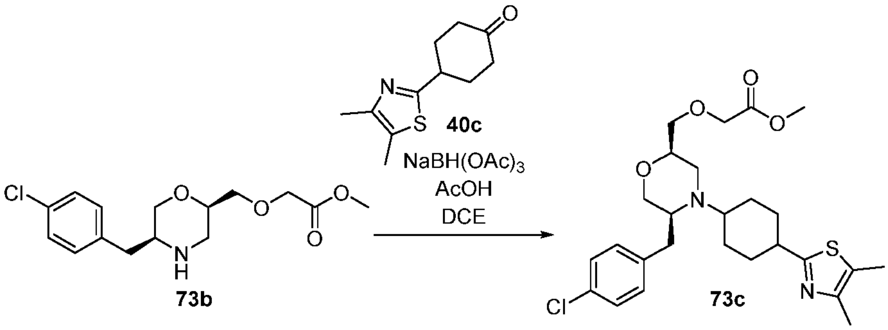


73b

73c

The title compound **(73c)** was obtained from **73b** (173 mg; 0.55 mmol) and ketone **40c** (116 mg; 0.55 mmol) according to the General Procedure **VII.** The crude product mixture was purified by flash column chromatography on silica (hexane/AcOEt, 100:0 to 0:100, v/v) and give two separated isomers in 49% yield (for the first isomer)(138 mg; 0.27 mmol) and in 22% (for the second isomer)(60 mg; 0.12 mmol).

For the first isomer: ESI-MS m/z for $C_{26}H_{36}ClN_2O_4S$ found 507.0/509.0 $[M+H]^+$; Rt = 2.38 min (Method D);
For the second isomer: ESI-MS m/z for $C_{26}H_{36}ClN_2O_4S$ found 507.0/509.0 $[M+H]^+$; Rt = 2.62 min

(Method D)

Step 4.

Synthesis of 2-(((2*R*,5*S*)-5-(4-chlorobenzyl)-4-(4-(4,5-dimethylthiazol-2-yl)cyclohexyl)morpholin-2-yl)methoxy)acetic acid hydrochloride **(73).**

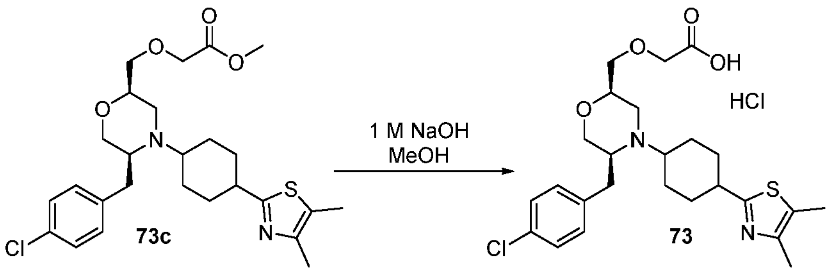


73c

73

To the solution of **73c** (the second isomer)(60 mg; 0.12 mmol) in MeOH (5 mL) 1 M NaOH (2 mL) was added and whole was stirred at room temperature for 1.5 hours. The reaction progress was monitored by LC-MS. When analysis indicated completion of the reaction, the solvent was evaporated *in vacuo* and the residue was dissolved in MeCN/water and to this solution 5% TFA was added to pH 3. The crude product was then purified by preparative reversed-phase column chromatography (Luna® 5μm Phenyl-Hexyl, LC Column 250 x 21.2 mm, water/MeCN + 1‰ TFA, 95:5 to 20:80, 50 min, 12 mL/min) and then the obtained product was transformed into hydrochloride salt with using of 1 M HCl (2 mL). The title compound (73) was obtained as a single isomer as a hydrochloride salt in 50% yield (31 mg; 0.06 mmol).
ESI-MS m/z for $C_{25}H_{34}ClN_2O_4S$ found 493.0/495.0 $[M+H]^+$; Rt = 2.08 min (Method D); $^1H$ NMR (400 MHz, DMSO-$d_6$) δ 7.47 - 7.37 (m, 2H), 7.36 - 7.27 (m, 2H), 4.08 (s, 2H), 4.05 - 3.97 (m, 1H), 3.89 - 3.84 (m, 1H), 3.64 - 3.62 (m, 2H), 3.58 - 3.42 (m, 3H), 3.17 - 3.03 (m, 2H), 2.95 - 2.80 (m, 1H), 2.34 - 2.25 (m, 5H), 2.20 (s, 3H), 2.16 - 1.95 (m, 2H), 1.75 - 1.43 (m, 4H), 1.25 - 1.18 (m, 2H).

**Example 74.**

Synthesis of 2-((2*R*,5*S*)-5-(4-chlorobenzyl)-4-(4-(1,5-dimethyl-1*H*-pyrazol-3-yl)cyclohexyl)morpholin-2-yl)propan-2-ol **(74).**

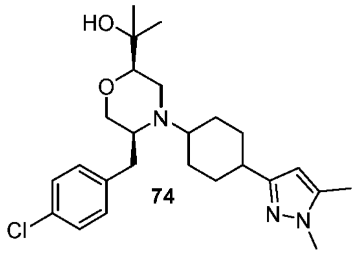

74

Step 1.

Synthesis of *tert*-butyl (2*R*,5*S*)-5-(4-chlorobenzyl)-2-(2-hydroxypropan-2-yl)morpholine-4-carboxylate **(74a).**

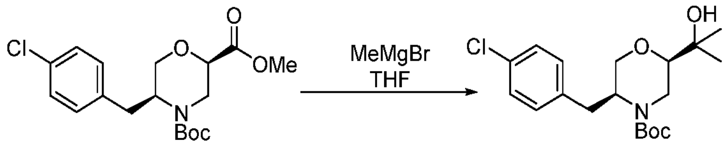


22a

74a

The title compound **(74a)** was obtained from **22a** (0.50 g; 1.41 mmol) according to the General Procedure **VI** in 64% yield (332 mg; 0.90 mmol).
ESI-MS $C_{19}H_{28}ClNO_4Na$ found 393.2/395.2 $[M+Na]^+$, 270.0/272.0 $[M+H\text{-}Boc]^+$; Rt = 4.20 min

(Method C)

Step 2.

Synthesis of 2-((2*R*,5*S*)-5-(4-chlorobenzyl)morpholin-2-yl)propan-2-ol **(74b).**

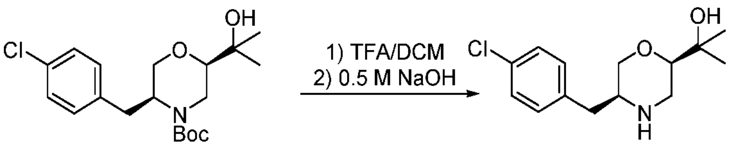


74a

74b

The title compound **(74b)** was obtained as a free amine in 92% yield (223 mg; 0.83 mmol) from **74a** (332 mg; 0.90 mmol) according to the General Procedure **IVb** and then after basic (0.5 M NaOH) extraction with DCM.
ESI-MS m/z for $C_{14}H_{21}ClNO_2$ found 270.0/272.0 $[M+H]^+$; $R_t$ = 3.40 min (Method C)

Step 3.

Synthesis of 2-((2*R*,5*S*)-5-(4-chlorobenzyl)-4-(4-(1,5-dimethyl-1*H*-pyrazol-3-yl)cyclohexyl)morpholin-2-yl)propan-2-ol **(74).**

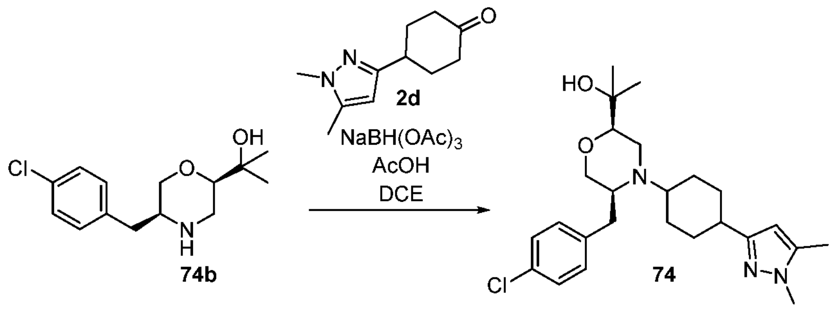


The title compound **(74)** was obtained as a free amine as a single isomer from **74b** (223 mg; 0.83 mmol) and ketone **2d** (179 mg; 0.91 mmol) according to the General Procedure **VII** in 12% yield (46 mg; 0.10 mmol). The crude product was purified by flash column chromatography on silica (AcOEt/MeOH, 100:4 to 70:30, *v/v,* 24 min).
ESI-MS m/z for $C_{25}H_{37}ClN_3O_2$ found 446.2/448.2 $[M+H]^+$; Rt = 7.50 min (Method C); $^1$H NMR (400 MHz, Methanol-$d_4$) δ 7.30 - 7.26 (m, 2H), 7.23 - 7.18 (m, 2H), 5.89 - 5.85 (m, 1H), 3.68 (s, 3H), 3.67 - 3.62 (m, 1H), 3.51 - 3.45 (m, 1H), 3.06 - 2.93 (m, 3H), 2.83 - 2.76 (m, 1H), 2.63 - 2.48 (m, 3H), 2.33 - 2.17 (m, 5H), 2.08 - 1.98 (m, 2H), 1.58 - 1.43 (m, 2H), 1.39 - 1.27 (m, 3H), 1.27 - 1.22 (m, 6H).

**Example 75.**

Synthesis of (2*R*,5*S*)-5-(4-chlorobenzyl)-4-(4-(1,5-dimethyl-1*H*-pyrazol-3-yl)cyclohexyl)-2-(5-methyl-1*H*-pyrazol-3-yl) morpholine hydrochloride **(75).**

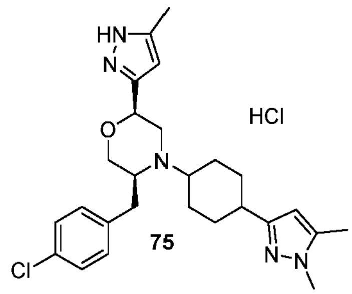


Step 1.

Synthesis of *tert*-butyl (2*R*,5*S*)-2-(but-2-ynoyl)-5-(4-chlorobenzyl)morpholine-4-carboxylate **(75a).**

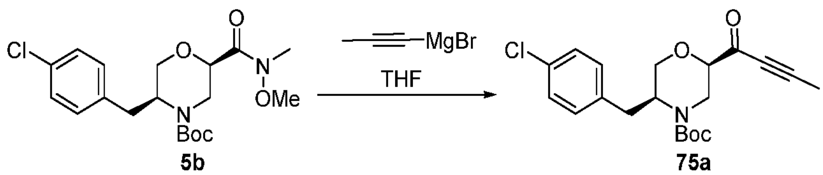


The title compound **(75a)** was obtained from **5b** (300 mg; 0.750 mmol) and from prop-1-yn-1-ylmagnesium bromide (0.5 M; 2.25 mL; 1.125 mmol) according to the General Procedure **VI** and the crude product was used to the next step without additional purification.
ESI-MS m/z for $C_{20}H_{25}ClNO_4$ found 378.0/380.0 $[M+H]^+$; Rt = 6.50 min (Method C)

Step 2.

Synthesis of *tert*-butyl (2*R*,5*S*)-5-(4-chlorobenzyl)-2-(5-methyl-1*H*-pyrazol-3-yl)morpholine-4-carboxylate **(75b).**

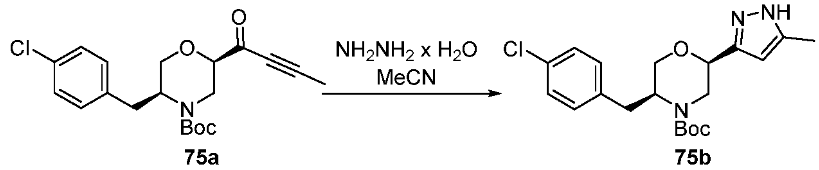


To the suspension of **75a** (the crude product) in MeCN (2 mL) hydrazine monohydrate (73 μL; 1.50 mmol) was added and the reaction mixture was stirred at room temperature for 2 hours. The reaction progress was monitored by LC-MS. When analysis indicated completion of the reaction, the whole was concentrated *in vacuo* and the crude product was used to the next step without additional purification.
ESI-MS m/z for $C_{20}H_{27}ClN_3O_3$ found 392.0/394 $[M+H]^+$; Rt = 6.20 min (Method C)

Step 3.

Synthesis of (2*R*,5*S*)-5-(4-chlorobenzyl)-2-(5-methyl-1*H*-pyrazol-3-yl)morpholine **(75c).**

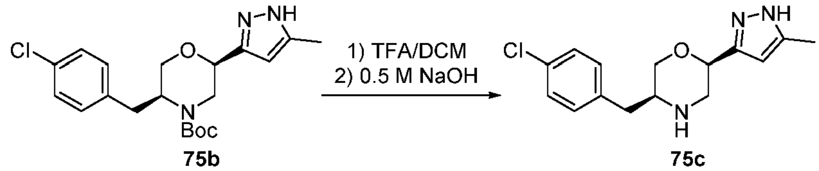


The title compound **(75c)** was obtained as a free amine from **75b** (the crude product) according to the General Procedure **IVb** and then after basic (0.5 M NaOH) extraction with DCM. The crude product was used to the next step without additional purification.
ESI-MS m/z for $C_{14}H_{21}ClNO_2$ found 270.0/272.0 $[M+H]^+$; Rt = 3.60 min (Method C)

Step 4.

Synthesis of (2*R*,5*S*)-5-(4-chlorobenzyl)-4-(4-(1,5-dimethyl-1*H*-pyrazol-3-yl)cyclohexyl)-2-(5-methyl-1*H*-pyrazol-3-yl) morpholine hydrochloride **(75).**

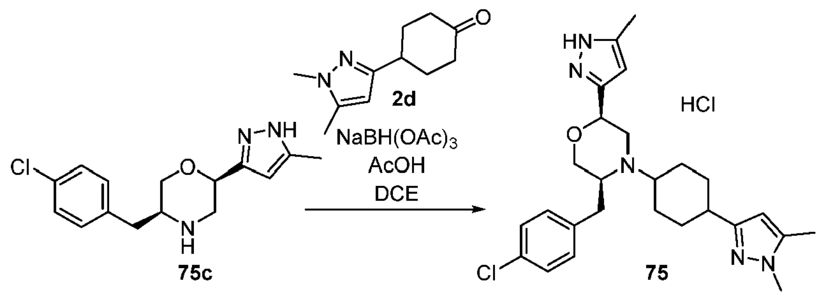


The title compound **(75)** was obtained as a hydrochloride salt as a single isomer from **75c** (the crude product) and ketone **2d** (144 mg; 0.75 mmol) according to the General Procedure **VII** in 5% yield (per four steps)(20 mg; 0.04 mmol). The crude product was purified by flash column chromatography on silica (AcOEt/MeOH, 100:0 to 70:30, *v/v,* 24 min) and then by preparative reversed-phase column chromatography (Luna® 5μm Phenyl-Hexyl, LC Column 250 x 21.2 mm, water + 1‰ HCl (36%)/MeCN, 90:10 to 5:95, 35 min, Rt = 26 min, 12 mL/min).
ESI-MS m/z for $C_{26}H_{35}ClN_5O$ found 468.2/470.2 $[M+H]^+$; Rt = 4.25 min (Method C); $^1H$ NMR (400 MHz, Methanol-$d_4$) δ

7.47 - 7.30 (m, 4H), 6.45 (s, 2H), 5.21 - 5.07 (m, 1H), 4.11 - 4.03 (m, 1H), 4.02 - 3.84 (m, 6H), 3.75 - 3.49 (m, 2H), 3.40 - 3.34 (m, 1H), 3.27 - 3.18 (m, 1H), 2.94 - 2.82 (m, 1H), 2.58 - 2.46 (m, 2H), 2.42 (s, 3H), 2.39 (d, *J* = 0.7 Hz, 3H), 2.31 - 2.22 (m, 2H), 1.93 - 1.80 (m, 2H), 1.77 - 1.64 (m, 2H).

**Example 76.**

Synthesis of (2R,5R)-2-(4-chlorobenzyl)-1-(4-(1,5-dimethyl-1H-pyrazol-3-yl)cyclohexyl)-5-(methylsulfonyl)piperidine hydrochloride **(76).**

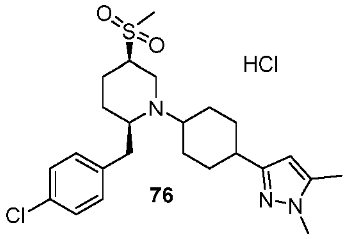

HCl

**76**

Step 1.

Synthesis of (*S*)-2-((*tert*-butoxycarbonyl)amino)-3-(4-chlorophenyl)propanoic acid **(76a).**

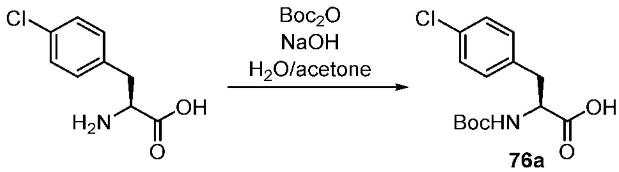


**76a**

To a solution of *L*-*p*-chlorophenylalanine (18.0 g, 75 mmol) in acetone-water (150 mL : 150 mL) sodium hydroxide (6 g, 150 mmol) was added at 0 °C. Then di-*tert*-butyl dicarbonate (16.4 g, 75 mmol) was added. The reaction mixture was stirred at room temperature overnight. Acetone was evaporated *in vacuo.* An aqueous layer was acidified to pH 2 with 2 M HCl and extracted with ethyl acetate. An organic layer was dried over anhydrous $MgSO_4$, filtered and concentrated under reduced pressure. The crude product was crystallized from hexane to obtain **76a** as a white solid in 80% yield (18.0 g; 60 mmol).

ESI-MS m/z for $C_{14}H_{19}ClNO_4$ found 299.8/301.8 $[M+H]^+$; Rt = 7.50 min (Method C); $^1H$ NMR (500 MHz, DMSO-$d_6$) δ 7.29 (d, *J* = 8.3 Hz, 2H), 7.21 (d, *J* = 8.3 Hz, 2H), 7.02 (d, *J* = 7.3 Hz, 1H), 4.08-3.99 (m, 1H), 2.96 (dd, *J* = 4.3, 13.7Hz, 1H), 2.76 (dd, *J* = 10.5, 13.6 Hz, 1H).

Step 2.

Synthesis of (*S*)-tert-butyl (3-(4-chlorophenyl)-1-(2,2-dimethyl-4,6-dioxo-1,3-dioxan-5-yl)-1-oxopropan-2-yl)carbamate **(76b).**

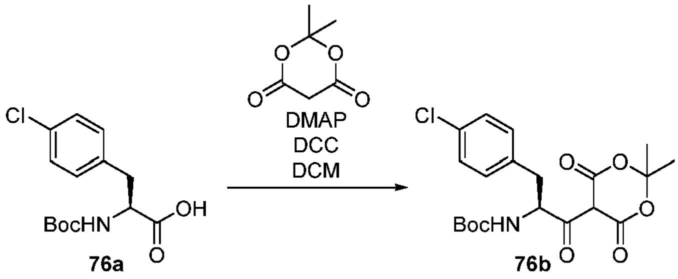

76a 76b

To a solution of **76a** (4 g, 13.34 mmol), DMAP (2.45 g; 20.02 mmol) and Meldrum's acid (2.31 g; 16.01 mmol) in anhydrous DCM (27 mL) DCC (3.3 g; 16.01 mmol) was added portionwise at 0 °C over 10 minutes and the mixture was allowed to room temperature and stirred for 22 hours. The reaction progress was monitored by LC-MS. When analysis indicated completion of the reaction, the mixture was filtered through the Celite pad in order to remove insoluble solid which was washed with DCM and discarded. The filtrate was washed with 1 M HCl (30 mL) and brine (30 mL), dried over anhydrous $Na_2SO_4$, filtered and concentrated *in vacuo* and the crude product was used to the next step without additional purification. Compound **76b** was obtained in 99% yield (5.61 g; 13.21 mmol).
ESI-MS m/z for $C_{20}H_{24}ClNO_7Na$ found 448.1/450.1 $[M+Na]^+$; Rt = 4.40 min (Method D)

Step 3.

Synthesis of (*R*)-tert-butyl (1-(4-chlorophenyl)-3-(2,2-dimethyl-4,6-dioxo-1,3-dioxan-5-yl)-propan-2-yl)carbamate **(76c).**

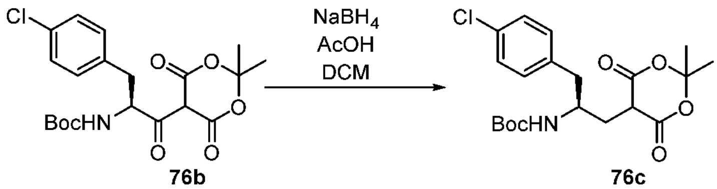

76b 76c

To a cooled to 0 °C solution of **76b** (9.35 g, 21.96 mmol) and glacial acetic acid (12.6 mL; 219.56 mmol) in DCM (110 mL) $NaBH_4$ (2.08 g; 54.89 mmol) was added portionwise over 20 minutes and the mixture was allowed to room temperature and stirred for 18 hours. The reaction progress was monitored by LC-MS. When analysis indicated completion of the reaction, phases were separated and an organic phase was washed with brine (100 mL). An aqueous phase was washed additionally with DCM (3 x 40 mL) and discarded. The combined organic solutions were washed with brine (2 x 50 mL), then with saturated aqueous $NaHCO_3$ diluted with water (100 mL; 1:1 v/v) until pH 6-7 and with brine (50 mL). Due to emulsion $CHCl_3$ was added to the organic phase during a final washing. The combined organic solutions were dried over anhydrous $Na_2SO_4$, filtered and concentrated *in vacuo* and the crude product was used to the next step without additional purification. Compound 76c was obtained in 99% yield (8.94 g; 21.74 mmol).
ESI-MS m/z for $C_{20}H_{26}ClNO_6Na$ found 434.1/436.1 $[M+Na]^+$; Rt = 3.80 min (Method D)

Step 4.

Synthesis of (R)-tert-butyl 2-(4-chlorobenzyl)-5-oxopyrrolidine-1-carboxylate **(76d).**

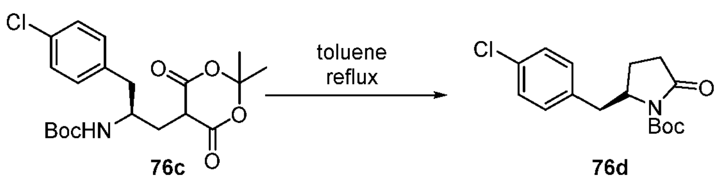

76c 76d

The solution of **76c** (8.94 g; 21.74 mmol) in toluene (110 mL) was heated to reflux for 15 hours. The reaction

progress was monitored by LC-MS. When analysis indicated completion of the reaction, the mixture was concentrated *in vacuo* and the remaining oily residue was dissolved in AcOEt and then coevaporated with silica gel. The crude product was purified by flash column chromatography on silica (hexane/AcOEt, 80:20 to 70:30, *v/v,* 24 min). Compound **76d** was obtained as a dense orange oil in 80% yield (5.35 g; 17.31 mmol).
ESI-MS m/z for $C_{16}H_{20}ClNO_3Na$ found 332.1/334.1 $[M+Na]^+$; Rt = 3.60 min (Method D)

Step 5.

Synthesis of ((*R*)-1-(4-chlorobenzyl)-4-oxo-5-dimethylsulfoxonium-pentyl)-carbamic acid *tert*-butyl ester **(76e).**

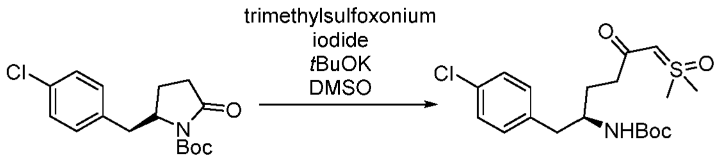


**76d** **76e**

To a suspension of trimethylsulfoxonium iodide (4.56 g; 20.72 mmol) in anhydrous DMSO (18 mL) under nitrogen atmosphere, tBuOK (2.13 g; 19 mmol) was added and the mixture was stirred at room temperature for 2 hours until the mixture became clear. In an another flask **76d** (5.35 g, 17.31 mmol) was dissolved in DMSO (17 mL) and to this solution the previously prepared ylide was transferred via syringe and the mixture was stirred at room temperature for 17 hours. The reaction progress was monitored by LC-MS. When analysis indicated completion of the reaction, water (200 mL) was added and the product was extracted with AcOEt (200 mL, then 2 x 50 mL). The combined organic solutions were washed with water (50 mL) and brine (50 mL), dried over anhydrous $Na_2SO_4$, filtered and concentrated *in vacuo* and the crude product was used to the next step without additional purification. Compound **76e** was obtained as a white solid in 93% yield (6.46 g; 16.1 mmol).
ESI-MS m/z for $C_{19}H_{29}ClNO_4S$ found 402.1/404.1 $[M+Na]^+$; Rt = 2.30 min (Method D)

Step 6.

Synthesis of (*R*)-*tert*-butyl 2-(4-chlorobenzyl)-5-oxopiperidine-1-carboxylate **(76f).**

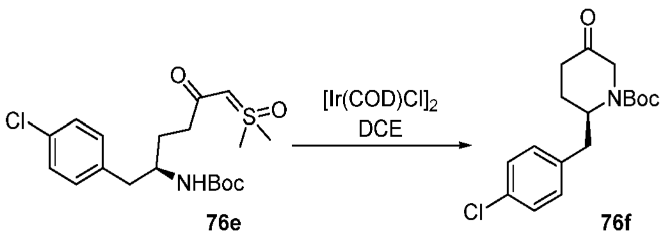


**76e** **76f**

To a solution of iridium catalyst (42 mg; 0.06 mmol) in a degassed DCE (20 mL) under nitrogen atmosphere, a solution of **76e** (2.5 g; 6.22 mmol) in a degassed DCE (105 mL) was added dropwise via syringe pump (20 mL/hour) over 5 hours at 70 °C. After this time the mixture was stirred at 70 °C for 30 minutes. The reaction progress was monitored by LC-MS. When analysis indicated completion of the reaction, the mixture was concentrated *in vacuo* and the oily residue was dissolved in DCM and co-evaporated with silica gel. The crude product was purified by flash column chromatography on silica (hexane/AcOEt, 100:0 to 70:30, *v/v,* 24 min). Compound **76f** was obtained as a thick orange oil in 86% yield (1.73 g; 5.35 mmol).
ESI-MS m/z for $C_{17}H_{23}ClNO_3$ found 325.0/327.0 $[M+Na]^+$; Rt = 3.70 min (Method D)

Step 7.

Synthesis of (2*R*,5*R*)-*tert*-butyl 2-(4-chlorobenzyl)-5-hydroxypiperidine-1-carboxylate **(76g).**

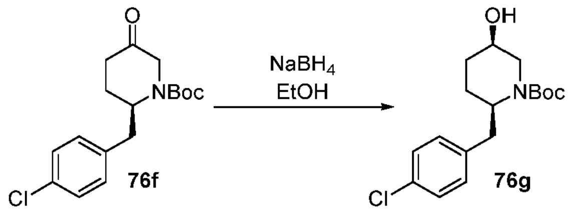

76f 76g

To a solution of **76f** (136 mg; 0.42 mmol) in EtOH (8.4 mL) $NaBH_4$ (24 mg; 0.63 mmol) was added at -5 °C and the mixture was stirred at this temperature for 4 hours. The reaction progress was monitored by LC-MS. When analysis indicated completion of the reaction, the mixture was concentrated *in vacuo* and saturated aqueous solution of $NH_4Cl$ (20 mL) was added and the product was extracted with DCM (4 x 15 mL). The combined organic solutions were dried over anhydrous $Na_2SO_4$, filtered and concentrated *in vacuo* and the crude product was used to the next step without additional purification. Compound **76g** was obtained in 99% yield (137 mg; 0.42 mmol).
ESI-MS m/z for $C_{12}H_{17}ClNO$ found 226.1/228.1 $[M+H-Boc]^+$; Rt = 6.00 min (Method C)

Step 8.

Synthesis of *tert*-butyl (2*R*,5*S*)-5-(benzoyloxy)-2-(4-chlorobenzyl)piperidine-1-carboxylate **(76h).**

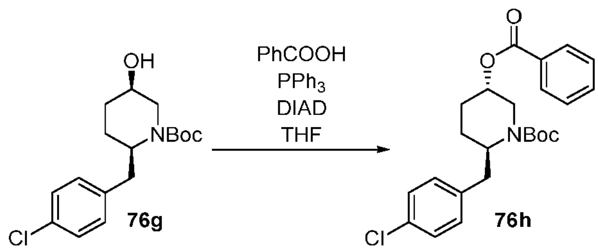

76g 76h

The solution of **76g** (2 g; 6.20 mmol), $PPh_3$ (2.1 g; 8.06 mmol) and benzoic acid (9.84 g; 8.06 mmol) in THF (25 mL) was cooled to 0 °C and then DIAD (1.83 mL; 9.30 mmol) was added dropwise. The reaction mixture was stirred under argon atmosphere for 30 minutes below 0 °C, then at room temperature. The reaction progress was monitored by LC-MS. When analysis indicated completion of the reaction, the solvent was removed *in vacuo* and the residue was dissolved in AcOEt and washed with brine (2 x 30 mL). An organic phase was dried over anhydrous $Na_2SO_4$, filtered and concentrated *in vacuo* and the crude product was purified by flash column chromatography on silica (hexane/AcOEt, 100:0 to 0:100, *v*/*v*, 24 min). Compound **76h** was obtained in 99% yield (2.63 g; 6.14 mmol).
ESI-MS m/z for $C_{24}H_{29}ClNO_4$ found 430.1/432.1 $[M+H]^+$; Rt = 6.80 min (Method C)

Step 9.

Synthesis of *tert*-butyl (2*R*,5*S*)-2-(4-chlorobenzyl)-5-hydroxypiperidine-1-carboxylate **(76i).**

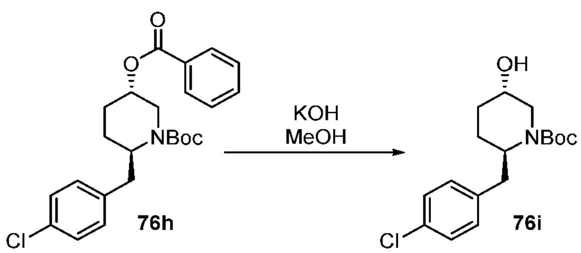

76h 76i

To the solution of **76h** (2.63 g; 6.14 mmol) in MeOH (15 mL) KOH (382 mg; 6.82 mmol) was added and whole was stirred at room temperature overnight. The reaction progress was monitored by LC-MS. When analysis indicated completion of the reaction, the solvent was evaporated *in vacuo* and the residue was dissolved in AcOEt and washed with 0.5 M NaOH. An organic phase was dried over anhydrous $Na_2SO_4$, filtered and concentrated *in vacuo* and the crude product was used to the next step without additional purification. Compound 76i was obtained in 65% yield (1.3 g; 4.00 mmol).
ESI-MS m/z for $C_{12}H_{17}ClNO$ found 226.1/228.1 $[M+H-Boc]^+$; Rt = 6.00 min (Method C)

Step 10.

Synthesis of *tert-butyl* (2*R*,5*S*)-2-(4-chlorobenzyl)-5-((methylsulfonyl)oxy)piperidine-1-carboxylate **(76j).**

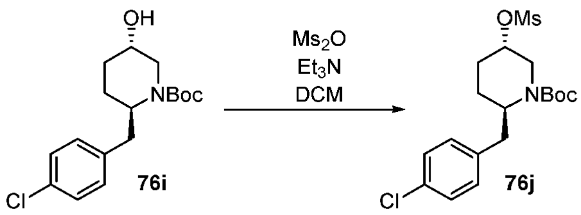


The title compound **(76j)** was obtained from **76i** (1.3 g; 4.00 mmol) according to the General Procedure **XI.** The crude product was used to the next step without additional purification. ESI-MS m/z for $C_{18}H_{27}ClNO_5S$ found 404.1/406.1 $[M+H]^+$; Rt = 6.40 min (Method C)

Step 11.

Synthesis of *tert*-butyl (2*R*,5*R*)-2-(4-chlorobenzyl)-5-(methylthio)piperidine-1-carboxylate **(76k).**

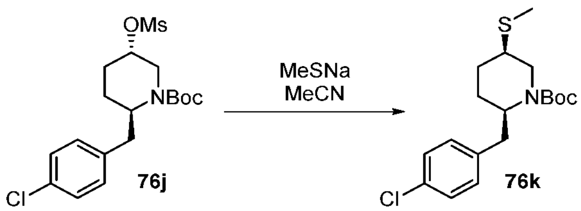


To a solution of **76j** (the crude product) in acetonitrile (15 mL) MeSNa (561 mg; 8.00 mmol) was added and the reaction mixture was stirred at 80 °C overnight. The reaction progress was monitored by LC-MS analysis. After analytical control indicated completion of the reaction, the mixture was dissolved in DCM and then washed with 2 M NaOH and brine, dried over anhydrous $Na_2SO_4$, filtered and concentrated *in vacuo* and the crude product was used to the next step without additional purification.
ESI-MS m/z for $C_{18}H_{27}ClNO_2S$ found 356.1/358.1 $[M+H]^+$; Rt = 6.80 min (Method C)

Step 12.

Synthesis of *tert*-butyl (2*R*,5*R*)-2-(4-chlorobenzyl)-5-(methylsulfonyl)piperidine-1-carboxylate **(761).**

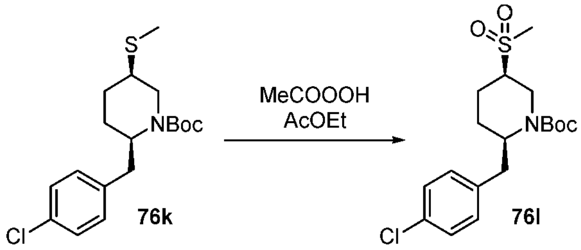

76k 76l

The reaction of **76k** (the crude product) with peracetic acid (35% in AcOH; 2.3 mL; 12.0 mmol) was stirred at room temperature overnight. Then the reaction was concentrated *in vacuo* and the residue was diluted with AcOEt, washed with 1 M NaOH and $Na_2S_2O_3$. Phases were separated and the organic one was washed with brine, dried over anhydrous $Na_2SO_4$, filtered and concentrated *in vacuo* and the crude product was purified by flash column chromatography on silica (hexane/AcOEt, 100:0 to 0:100, *v/v,* 24 min). Compound **761** was obtained in 60% yield (per three steps)(0.93 g; 2.4 mmol).
ESI-MS m/z for $C_{18}H_{27}ClNO_4S$ found 388.1/390.1 $[M+H]^+$; Rt = 6.30 min (Method C)

Step 13.

Synthesis of (2*R*,5*R*)-2-(4-chlorobenzyl)-5-(methylsulfonyl)piperidine **(76m).**

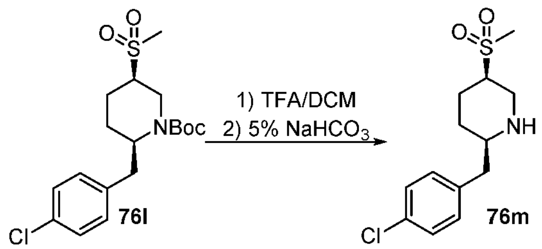

76l 76m

The title compound **(76m)** was obtained as a free amine from **761** (0.45 g; 1.12 mmol) according to the General Procedure **IVb** and then after basic (5% $NaHCO_3$) extraction with DCM. The crude product was used to the next step without additional purification.
ESI-MS m/z for $C_{13}H_{19}ClNO_2S$ found 288.0/290.0 $[M+H]^+$; Rt = 3.30 min (Method C)

Step 14.

Synthesis of (2*R*,5*R*)-2-(4-chlorobenzyl)-1-(4-(1,5-dimethyl-1*H*-pyrazol-3-yl)cyclohexyl)-5-(methylsulfonyl)piperidine hydrochloride **(76).**

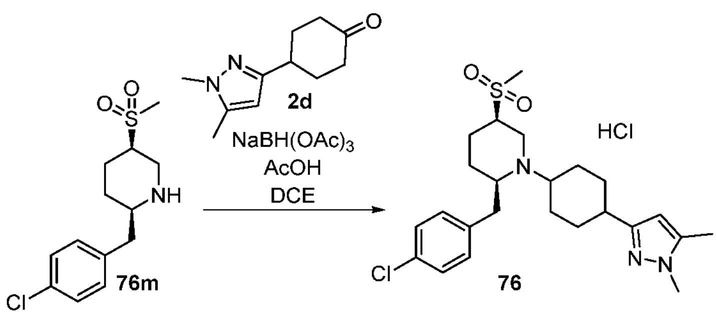

76m 76

The title compound **(76)** was obtained as a hydrochloride salt as a single isomer from **76m** (the crude product) and ketone **2d** (236 mg; 1.23 mmol) according to the General Procedure **VII** in 7% yield (per two steps)(40 mg; 0.08 mmol). The crude product was purified by flash column chromatography on silica (AcOEt/MeOH, 100:0 to 70:30, *v/v,* 24 min) and then

by preparative reversed-phase column chromatography (Luna® 5μm Phenyl-Hexyl, LC Column 250 x 21.2 mm, water + 1‰ HCl (36%)/MeCN, 90:10 to 5:95, 35 min, Rt = 26 min, 12 mL/min).
ESI-MS m/z for $C_{24}H_{35}ClN_3O_2S$ found 464.1/466.1 $[M+H]^+$; Rt = 4.20 min (Method C); $^1H$ NMR (400 MHz, Methanol-$d_4$) δ 7.43 - 7.30 (m, 4H), 6.43 (s, 1H), 4.21 - 3.60 (m, 8H), 3.17 - 3.12 (m, 3H), 3.12 - 3.04 (m, 1H), 2.93 - 2.79 (m, 1H), 2.61 - 2.45 (m, 1H), 2.42 (s, 3H), 2.37 - 2.19 (m, 4H), 2.19 - 2.00 (m, 2H), 2.00 - 1.79 (m, 4H), 1.74 - 1.60 (m, 2H).

**Example 77.**

Synthesis of (2*R*,5*S*)-5-(4-chlorobenzyl)-4-(4-(1,5-dimethyl-1*H*-pyrazol-3-yl)cyclohexyl)morpholine-2-carboxamide **(77).**

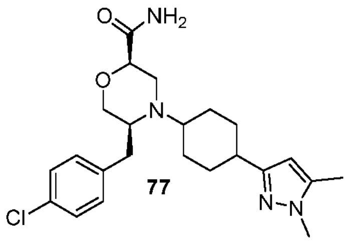

Step 1.

Synthesis of *tert*-butyl (2*R*,5*S*)-2-carbamoyl-5-(4-chlorobenzyl)morpholine-4-carboxylate **(77a).**

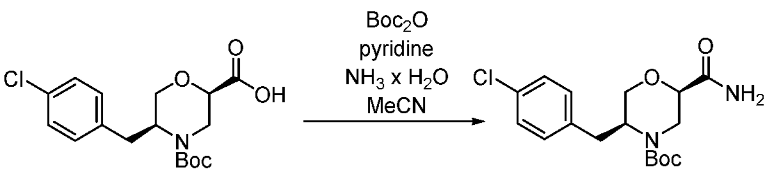


**5a** **77a**

The solution of **5a** (0.80 g; 2.25 mmol), $Boc_2O$ (0.74 g; 3.37 mmol) and pyridine (1 mL) in acetonitrile (16 mL) was stirred at room temperature for 30 minutes. Then to this mixture $NH_3$ x $H_2O$ (4.8 mL) was added dropwise over 30 minutes and the reaction mixture was stirred at room temperature. The reaction progress was monitored by LC-MS analysis. After analytical control indicated completion of the reaction, the mixture was concentrated *in vacuo* and the crude product was used to the next step without additional purification.
ESI-MS m/z for $C_{17}H_{24}ClN_2O_4$ found 355.1/357.1 $[M+H]^+$; Rt = 5.70 (Method C)

Step 2.

Synthesis of (2*R*,5*S*)-5-(4-chlorobenzyl)morpholine-2-carboxamide **(77b).**

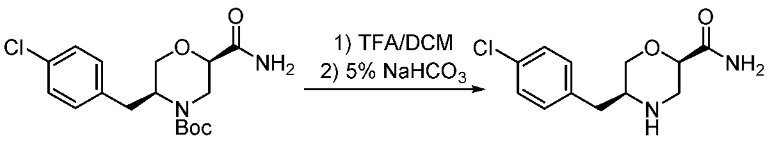


**77a** **77b**

The title compound **(77b)** was obtained in 80% yield (per two steps)(457 mg; 1.8 mmol) as a free amine from 77a (the crude product) according to the General Procedure IVb and then after basic (5% $NaHCO_3$) extraction with DCM.
ESI-MS m/z for $C_{12}H_{16}ClN_2O_2$ found 255.0/257.0 $[M+H]^+$; Rt = 3.00 min (Method C)

Step 3.

Synthesis of (2*R*,5*S*)-5-(4-chlorobenzyl)-4-(4-(1,5-dimethyl-1*H*-pyrazol-3-yl)cyclohexyl)-morpholine-2-carboxamide (77).

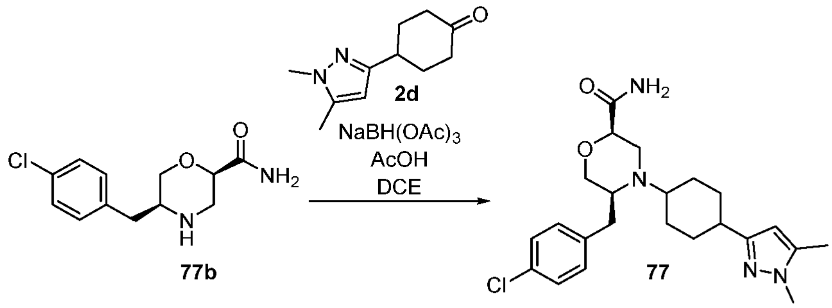


77b

77

The title compound (77) was obtained as a free amine as a single isomer from 77b (457 mg; 1.80 mmol) and ketone 2d (380 mg; 1.98 mmol) according to the General Procedure VII in 14% yield (110 mg; 0.25 mmol). The crude product was purified by flash column chromatography on silica (AcOEt/MeOH, 100:0 to 70:30, *v/v,* 24 min).
ESI-MS m/z for $C_{23}H_{32}ClN_4O_2$ found 431.1/433.2 $[M+H]^+$; Rt = 3.90 min (Method C); $^1H$ NMR (400 MHz, Methanol-$d_4$) $\delta$ 7.32 - 7.26 (m, 2H), 7.26 - 7.21 (m, 2H), 5.87 (s, 1H), 4.02 - 3.94 (m, 1H), 3.74 - 3.64 (m, 4H), 3.59 - 3.48 (m, 1H), 3.24 - 3.17 (m, 1H), 3.12 - 3.03 (m, 1H), 3.02 - 2.95 (m, 1H), 2.86 - 2.77 (m, 1H), 2.69 - 2.58 (m, 2H), 2.56 - 2.44 (m, 1H), 2.25 - 2.12 (m, 5H), 2.09 - 1.97 (m, 2H), 1.58 - 1.43 (m, 2H), 1.39 - 1.28 (m, 2H).

**Example 78.**

Synthesis of 2-(4-((2*R*,5*R*)-2-(4-chlorobenzyl)-5-(methylsulfonyl)piperidin-1-yl)cyclohexyl)-5-methyloxazole hydrochloride **(78).**

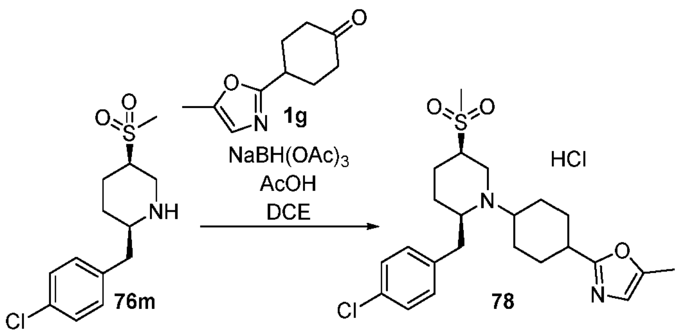


76m

78

The title compound **(78)** was obtained as a hydrochloride salt as a single isomer from **76m** (333 mg; 1.16 mmol) and ketone **1g** (229 mg; 1.28 mmol) according to the General Procedure **VII** in 3% yield (15 mg; 0.03 mmol). The crude product was purified by flash column chromatography on silica (AcOEt/MeOH, 100:0 to 70:30, *v/v,* 24 min) and then by preparative reversed-phase column chromatography (Luna® 5μm Phenyl-Hexyl, LC Column 250 x 21.2 mm, water + 1‰ HCl (36%)/MeCN, 90:10 to 5:95, 35 min, Rt = 26 min, 12 mL/min).
ESI-MS m/z for $C_{23}H_{32}ClN_2O_3S$ found 451.1/453.1 $[M+H]^+$; Rt = 4.20 min (Method C); $^1H$ NMR (400 MHz, Methanol-$d_4$) $\delta$ 7.45 - 7.29 (m, 4H), 7.20 - 7.05 (m, 1H), 4.21 - 3.61 (m, 4H), 3.47 - 3.34 (m, 1H), 3.28 - 3.04 (m, 6H), 2.60 - 2.50 (m, 1H), 2.46 - 2.24 (m, 6H), 2.17 - 1.70 (m, 8H).

**Example 79.**

Synthesis of (2*R*,5*S*)-5-(4-chlorobenzyl)-4-(4-(1,5-dimethyl-1*H*-pyrazol-3-yl)cyclohexyl)-2-((methylsulfinyl)methyl)morpholine **(79).**

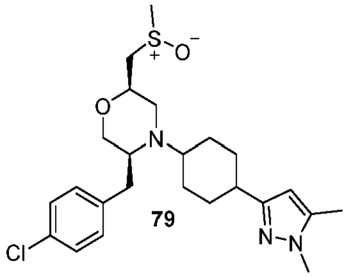

79

Step 1.

Synthesis of *tert*-butyl (2*R*,5*S*)-5-(4-chlorobenzyl)-2-((methylsulfinyl)methyl)morpholine-4-carboxylate **(79a).**

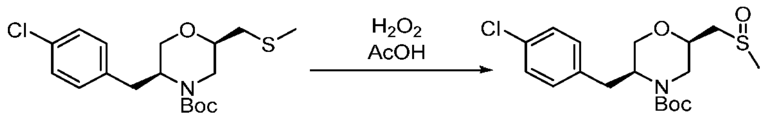


**3f** **79a**

To a cooled to 0 °C solution of **3f** (1.75 g; 4.70 mmol) in AcOH (2 mL) $H_2O_2$ (140 mL; 14.10 mmol) was added dropwise and stirred at room temperature overnight. Then to this reaction 0.5 M $NaOH/Na_2S_2O_3$ were added and extracted with AcOEt. The organic one was dried over anhydrous $Na_2SO_4$, filtered and concentrated *in vacuo* and the crude product was used to the next step without additional purification. Compound **79a** was obtained in 99% yield (1.80 g; 4.65 mmol).
ESI-MS m/z for $C_{18}H_{27}ClNO_4S$ found 388.1/390.1 $[M+H]^+$; Rt = 5.60 min (Method C)

Step 2.

Synthesis of (2*R*,5*S*)-5-(4-chlorobenzyl)-2-((methylsulfinyl)methyl)morpholine **(79b).**

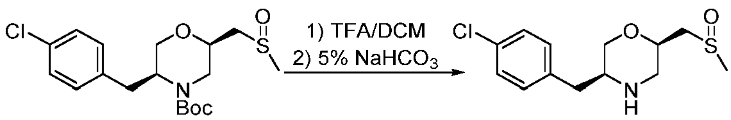


**79a** **79b**

The title compound **(79b)** was obtained in 80% yield (1.44 g; 3.72 mmol) as a free amine from 79a (1.80 g; 4.65 mmol) according to the General Procedure IVb and then after basic (5% $NaHCO_3$) extraction with DCM.
ESI-MS m/z for $C_{13}H_{19}ClNO_2S$ found 288.0/290.0 $[M+H]^+$; Rt = 3.10 min (Method C)

Step 3.

Synthesis of (2*R*,5*S*)-5-(4-chlorobenzyl)-4-(4-(1,5-dimethyl-1*H*-pyrazol-3-yl)cyclohexyl)-2-((methylsulfinyl)methyl)morpholine **(79).**

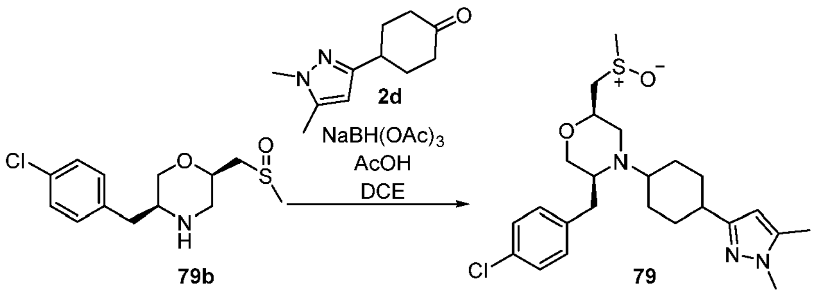


79b

79

The title compound **(79)** was obtained as a free amine as a single isomer from **79b** (1.47 g; 3.80 mmol) and ketone **2d** (803 mg; 4.18 mmol) according to the General Procedure **VII** in 1% yield (22 mg; 0.05 mmol). The crude product was purified by flash column chromatography on silica (AcOEt/MeOH, 100:0 to 70:30, *v/v,* 24 min) and then by chiral preparative reversed-phase column chromatography (Lux Amylose-1 5μm, 250 x 21.2 mm, hexane/*i*PrOH, 70:30, *v/v; 10* mL/min, Rt = 22.5 min).
ESI-MS m/z for $C_{24}H_{35}ClN_3O_2S$ found 464.1/466.1 $[M+H]^+$; Rt = 1.80 min (Method C); $^1$H NMR (400 MHz, Methanol-$d_4$) δ 7.31 - 7.26 (m, 2H), 7.22 - 7.16 (m, 2H), 5.89 - 5.83 (m, 1H), 4.09 - 3.97 (m, 1H), 3.68 (s, 3H), 3.64 - 3.58 (m, 1H), 3.55 - 3.46 (m, 1H), 3.10 - 2.91 (m, 5H), 2.86 - 2.78 (m, 1H), 2.77 - 2.67 (m, 4H), 2.67 - 2.57 (m, 1H), 2.58 - 2.46 (m, 1H), 2.25 - 2.12 (m, 5H), 2.08 - 1.97 (m, 2H), 1.57 - 1.42 (m, 2H), 1.38 - 1.28 (m, 2H).

**Example 80.**

Synthesis of (2*R*,5*S*)-5-(4-chlorobenzyl)-4-(4-(1,5-dimethyl-1*H*-pyrazol-3-yl)cyclohexyl)-2-((methylsulfinyl)methyl)morpholine **(80).**

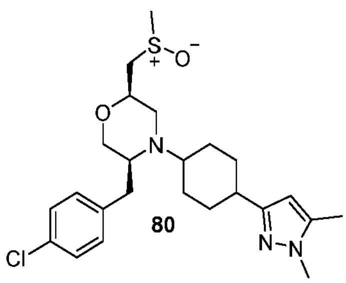

80

The title compound **(80)** was obtained during the synthesis of compound 79 as a free amine as a second single isomer from **79b** (1.47 g; 3.80 mmol) and ketone **2d** (803 mg; 4.18 mmol) according to the General Procedure **VII** in 3% yield (62 mg; 0.13 mmol). The crude product was purified by flash column chromatography on silica (AcOEt/MeOH, 100:0 to 70:30, *v/v,* 24 min) and then by chiral preparative reversed-phase column chromatography (Lux Amylose-1 5μm, 250 x 21.2 mm, hexane/*i*PrOH, 70:30, *v/v;* 10 mL/min, Rt = 25.0 min).
ESI-MS m/z for $C_{24}H_{35}ClN_3O_2S$ found 464.0/466.0 $[M+H]^+$; Rt = 1.80 min (Method C); $^1$H NMR (400 MHz, Methanol-$d_4$) δ 7.30 - 7.25 (m, 2H), 7.22 - 7.16 (m, 2H), 5.89 - 5.85 (m, 1H), 4.02 - 3.93 (m, 1H), 3.68 (s, 3H), 3.65 - 3.59 (m, 1H), 3.56 - 3.47 (m, 1H), 3.10 - 3.03 (m, 2H), 3.03 - 2.99 (m, 1H), 2.97 - 2.89 (m, 2H), 2.85 - 2.80 (m, 1H), 2.69 (s, 3H), 2.67 - 2.57 (m, 2H), 2.55 - 2.46 (m, 1H), 2.27 - 2.10 (m, 5H), 2.10 - 1.97 (m, 2H), 1.56 - 1.42 (m, 2H), 1.39 - 1.25 (m, 2H).

**Example 81.**

Synthesis of (2*R*,5*S*)-5-(4-chlorobenzyl)-4-(4-(1,5-dimethyl-1*H*-pyrazol-3-yl)cyclohexyl)-2-((methylthio)methyl)morpholine hydrochloride **(81).**

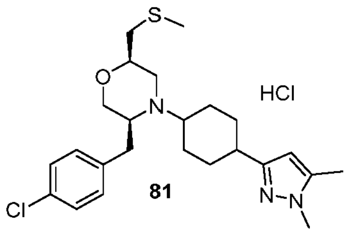

Step 1.

Synthesis of (2*R*,5*S*)-5-(4-chlorobenzyl)-2-((methylthio)methyl)morpholine **(81a).**

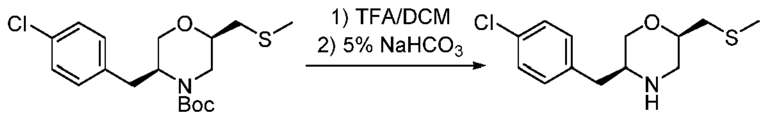


3f 81a

The title compound **(81a)** was obtained as a free amine from **3f** (148 mg; 0.40 mmol) according to the General Procedure **IVb** and then after basic (5% $NaHCO_3$) extraction with DCM. The crude product was used to the next step without additional purification.
ESI-MS m/z for $C_{13}H_{19}ClNOS$ found 272.0/274.0 $[M+H]^+$; Rt = 1.40 min (Method D)

Step 2.

Synthesis of (2*R*,5*S*)-5-(4-chlorobenzyl)-4-(4-(1,5-dimethyl-1*H*-pyrazol-3-yl)cyclohexyl)-2-((methylthio)methyl)morpholine hydrochloride **(81).**

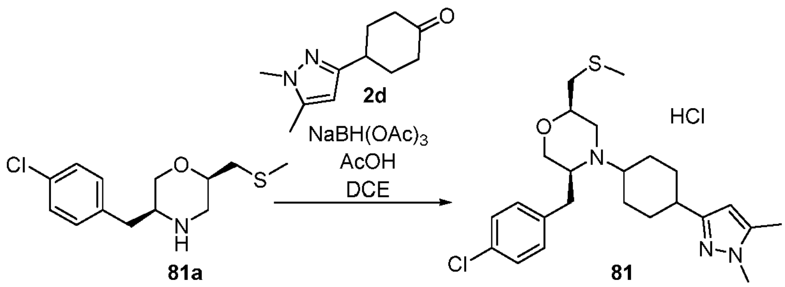


81a 81

The title compound **(81)** was obtained as a hydrochloride salt as a single isomer from **81a** (the crude product) and ketone **2d** (86 mg; 0.45 mmol) according to the General Procedure **VII** in 5% yield (8 mg; 0.02 mmol). The crude product was purified by flash column chromatography on silica (AcOEt/MeOH, 100:0 to 70:30, *v*/*v*, 24 min) and then by preparative reversed-phase column chromatography (Luna® 5µm Phenyl-Hexyl, LC Column 250 x 21.2 mm, water + 1‰ HCl (36%)/MeCN, 90:10 to 5:95, 35 min, Rt = 27 min, 12 mL/min).
ESI-MS m/z for $C_{24}H_{35}ClN_3OS$ found 448.1/450.1 $[M+H]^+$; Rt = 2.19 min (Method D); $^1H$ NMR (400 MHz, Methanol-$d_4$) δ 7.43 - 7.37 (m, 2H), 7.36 - 7.29 (m, 2H), 6.39 (s, 1H), 4.13 - 4.02 (m, 1H), 3.92 (s, 3H), 3.89 - 3.66 (m, 4H), 3.62 - 3.50 (m, 1H), 3.38 - 3.32 (m, 1H), 3.29 - 3.23 (m, 1H), 3.24 - 3.11 (m, 1H), 2.89 - 2.72 (m, 3H), 2.60 - 2.44 (m, 2H), 2.41 (s, 3H), 2.31 - 2.18 (m, 5H), 1.82 - 1.59 (m, 4H).

**Example 82.**

Synthesis of 1-(((2*R*,5*S*)-5-(4-chlorobenzyl)-4-(4-(1,5-dimethyl-1*H*-pyrazol-3-yl)cyclohexyl)morpholin-2-yl)methyl)pyrrolidine-2,5-dione 2,2,2-trifluoroacetate **(82).**

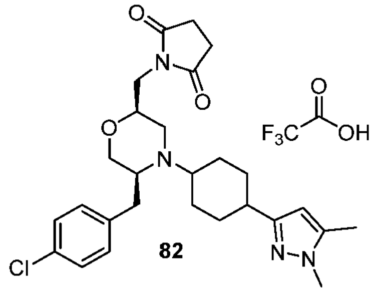

**82**

Step 1.

Synthesis of *tert*-butyl (2*S*,5*S*)-5-(4-chlorobenzyl)-2-((2,5-dioxopyrrolidin-1-yl)methyl)morpholine-4-carboxylate **(82a).**

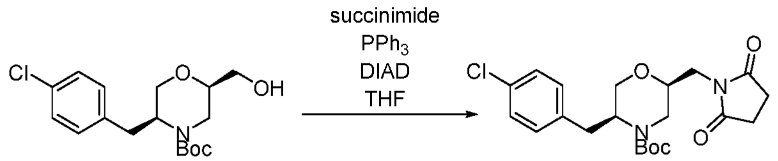


**3d** **82a**

To the solution of an alcohol **3d** (1 g; 3.00 mmol) and succinimide (330 mg; 3.30 mmol) in THF (10 mL) $PPh_3$ (860 mg; 3.30 mmol) was added. Then a whole was cooled to -10 °C and DIAD (650 μL; 3.30 mmol) was added dropwise and stirred at room temperature for 2 days. The reaction progress was monitored by LC-MS. Then a solid was filtered off and the filtrate was concentrated *in vacuo.* The residue was purified by flash column chromatography on silica (hexane/AcOEt, 100:0 to 0:100, *v/v,* 24 min). The title compound **(82a)** was obtained in 19% yield (240 mg; 0.57 mmol).
ESI-MS m/z for $C_{21}H_{28}ClN_2O_5$ found 423.0/425.0 $[M+H]^+$; $R_t$ = 3.50 min (Method D)

Step 2.

Synthesis of 1-(((2*R*,5*S*)-5-(4-chlorobenzyl)morpholin-2-yl)methyl)pyrrolidine-2,5-dione 2,2,2-trifluoroacetate **(82b).**

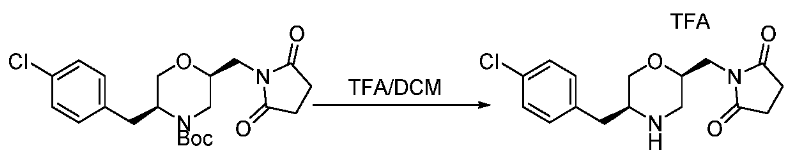


**82a** **82b**

The title compound **(82b)** was obtained as a TFA salt from **82a** (240 mg; 0.57 mmol) according to the General Procedure **IVb.** The crude product was used to the next step without additional purification.
ESI-MS m/z for $C_{16}H_{20}ClN_2O_3$ found 323.0/325.0 $[M+H]^+$; Rt = 1.10 min (Method D)

Step 3.

Synthesis of 1-(((2*R*,5*S*)-5-(4-chlorobenzyl)-4-(4-(1,5-dimethyl-1*H*-pyrazol-3-yl)cyclohexyl)morpholin-2-yl)methyl)pyrrolidine-2,5-dione 2,2,2-trifluoroacetate **(82).**

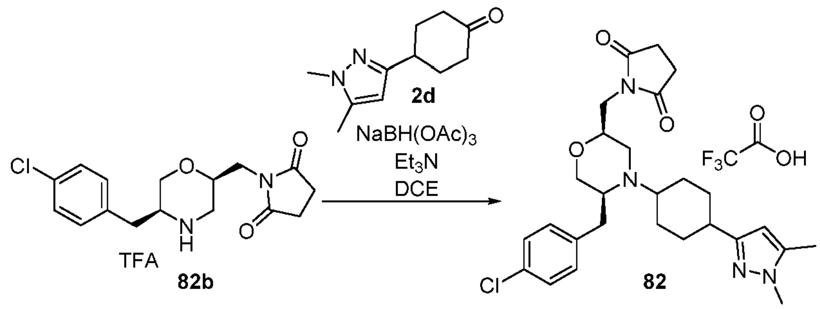


The title compound **(82)** was obtained as a TFA salt as a single isomer from **82b** (the crude product) and ketone **2d** (160 mg; 0.82 mmol) according to the General Procedure **VII** in 16% yield (per two steps)(58 mg; 0.09 mmol). The crude product was purified by flash column chromatography on silica (AcOEt/MeOH, 100:0 to 70:30, *v/v,* 24 min) and then by preparative reversed-phase column chromatography (Luna® 5μm Phenyl-Hexyl, LC Column 250 x 21.2 mm, water + 1‰ HCl (36%)/MeCN, 90:10 to 5:95, 35 min, Rt = 27.5 min, 12 mL/min).
ESI-MS m/z for $C_{27}H_{36}ClN_4O_3$ found 499.2/501.2 $[M+H]^+$; Rt = 2.00 min (Method D); $^1$H NMR (400 MHz, Methanol-$d_4$) δ 7.40 - 7.35 (m, 2H), 7.34 - 7.29 (m, 2H), 5.99 - 5.94 (m, 1H), 4.06 - 3.96 (m, 1H), 3.90 - 3.48 (m, 11H), 3.28 - 3.20 (m, 1H), 3.17 - 3.11 (m, 1H), 2.80 - 2.73 (m, 4H), 2.65 - 2.55 (m, 1H), 2.49 - 2.40 (m, 2H), 2.26 (s, 3H), 2.22 - 2.13 (m, 2H), 1.69 - 1.53 (m, 4H).

**Example 83.**

Synthesis of (2*S*,5*S*)-5-(4-chlorobenzyl)-4-(4-(4,5-dimethylthiazol-2-yl)cyclohexyl)-2-ethynylmorpholine hydrochloride **(83).**

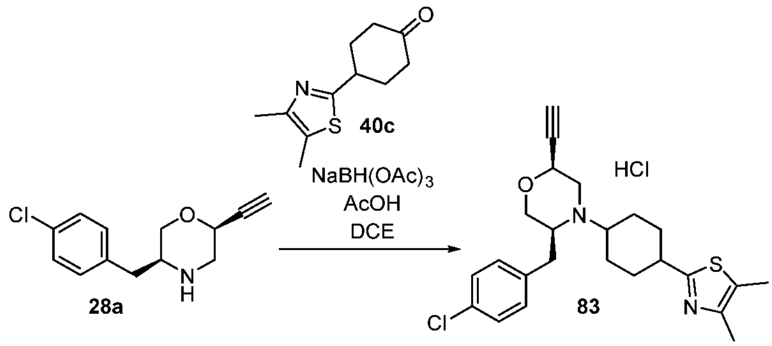


The title compound **(83)** was obtained as a single isomer as a free amine from **28a** (495 mg; 2.10 mmol) and ketone **40c** (462 mg; 2.20 mmol) according to the General Procedure **VII** in 28% yield (250 mg; 0.58 mmol). The crude product was purified by flash column chromatography on silica (hexane/MeCN, 100:0 to 40:60, *v/v,* 40 min) and then a small fraction of the product was purified by preparative reversed-phase column chromatography (C-18, water + 0.3‰ HCl (36%)/MeCN, 95:5 to 10:90, 30 min, 20 mL/min) to obtain the product **83** a s a hydrochloride salt. ESI-MS m/z for $C_{24}H_{30}ClN_2OS$ found 429.2/431.2 $[M+H]^+$; Rt = 1.38 min (Method A); $^1$H NMR (700 MHz, DMSO-$d_6$ + $D_2O$, 348 K) δ 7.38 (d, $J_{AA'}$ = 8.5 Hz, 2H), 7.31 (d, $J_{BB'}$ = 8.5 Hz, 2H), 4.72 - 4.70 (m, 1H), 3.74 - 3.68 (m, 3H), 3.54 (d, *J* = 2.2 Hz, 1H), 3.52 -3.47 (m, 2H), 3.35 (dd, J = 13.1 Hz, J = 8.9 Hz, 1H), 3.18 - 3.15 (m, 1H), 3.05 - 3.02 (m, 1H), 2.85 (tt, *J* = 12.3 Hz, *J* = 3.7 Hz, 1H), 2.26 (brs, 3H), 2.23 - 2.11 (m, 7H), 1.65 - 1.44 (m, 4H).

Example 84.

Synthesis of 2-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)acetic acid hydrochloride **(84).**

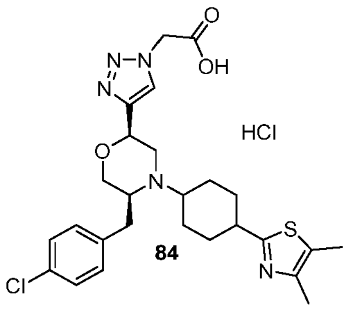


84

Step 1.

Synthesis of ethyl 2-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)acetate **(84a).**

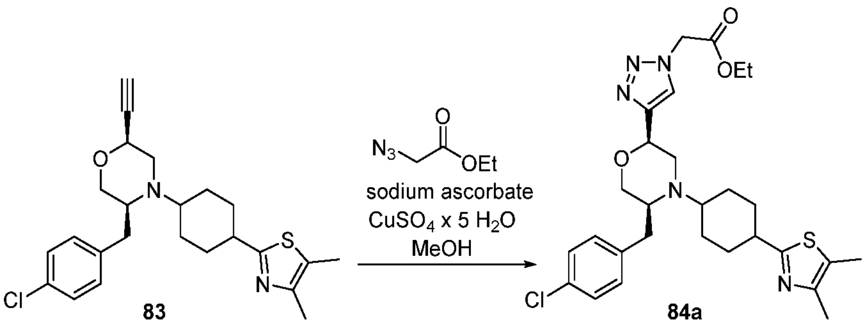


83 84a

To a solution of 83 (75 mg; 0.175 mmol) in MeOH (4 mL) $CuSO_4$ x 5 $H_2O$ (22 mg; 0.087 mmol) was added and after 5 minutes sodium ascorbate (35 mg; 0.175 mmol) was added in one portion and the mixture was stirred at room temperature for 25 minutes. Then to this mixture a solution of ethyl 2-azidoacetate (45 mg; 0.350 mmol) in MeOH (5 mL) was added and the mixture was stirred at room temperature for 2 days. The reaction progress was monitored by LC-MS. When analysis indicated completion of the reaction, the all crude reaction mixture was used to the next step without additional purification.
ESI-MS m/z for $C_{28}H_{37}ClN_5O_3S$ found 558.1/560.1 $[M+H]^+$; Rt = 1.40 min (Method A)

Step 2.

Synthesis of 2-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)acetic acid hydrochloride **(84).**

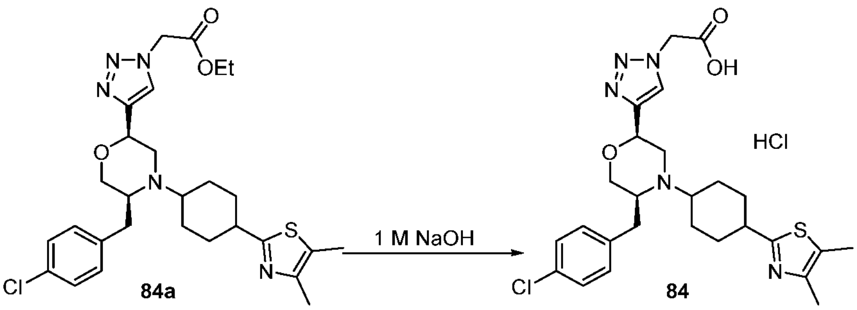


84a 84

To the **84a** (the crude reaction mixture) 1 M NaOH (3 mL) was added and whole was stirred at room temperature for 3 hours. The reaction progress was monitored by LC-MS. When analysis indicated completion of the reaction, the solvent was evaporated *in vacuo* and the residue was dissolved in MeCN/water with $HCl_{aq}$. An insoluble acid was filtered

off and the filtrate was taken for the preparative reversed-phase column chromatography (C-18, water + 0.3‰ HCl (36%)/MeCN, 95:5 to 20:80, 30 min, 20 mL/min). The title compound **(84)** was obtained as a single isomer as a hydrochloride salt in 33% yield (per two steps)(33 mg; 0.058 mmol).
ESI-MS m/z for $C_{26}H_{33}ClN_5O_3S$ found 530.2/532.2 $[M+H]^+$; Rt = 1.16 min (Method A); $^1$H NMR (700 MHz, DMSO-$d_6$ + $D_2O$, 348 K) δ 8.21 (s, 1H), 7.38 (d, $J_{AA'}$ = 8.5 Hz, 2H), 7.32 (d, $J_{BB'}$ = 8.5 Hz, 2H), 5.26 (s, 2H), 5.07 - 5.05 (m, 1H), 3.92 - 3.90 (m, 1H), 3.81 - 3.79 (m, 1H), 3.71 (dd, *J* = 13.1 Hz, *J* = 1.3 Hz, 1H), 3.67 - 3.65 (m, 1H), 3.60 - 3.57 (m, 1H), 3.54 - 3.50 (m, 1H), 3.23 (dd, *J* = 13.8 Hz, *J* = 10.6 Hz, 1H), 3.17 (dd, *J* = 13.8 Hz, *J* = 4.0 Hz, 1H), 2.87 (tt, *J* = 11.8 Hz, *J* = 3.5 Hz, 1H), 2.33 - 2.28 (m, 2H), 2.26 (s, 3H), 2.18 - 2.16 (m, 5H), 1.65 - 1.51 (m, 4H).

**Example 85.**

Synthesis of 3-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)propanoic acid hydrochloride **(85).**

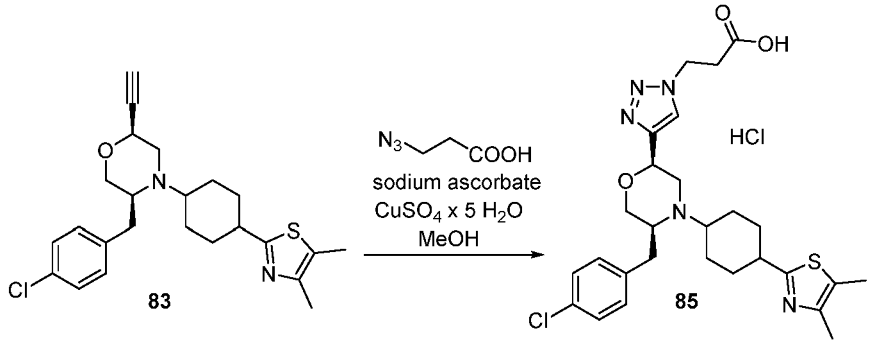

83 85

To a solution of **83** (50 mg; 0.116 mmol) in MeOH (4 mL) $CuSO_4$ x 5 $H_2O$ (15 mg; 0.058 mmol) was added and after 5 minutes sodium ascorbate (23 mg; 0.116 mmol) was added in one portion and the mixture was stirred at room temperature for 25 minutes. Then to this mixture a solution of 3-azidopropanoic acid (27 mg; 0.233 mmol) in MeOH (5 mL) was added and the mixture was stirred at room temperature overnight. The reaction progress was monitored by LC-MS. When analysis indicated completion of the reaction, the solvent was evaporated *in vacuo* and the residue was dissolved in MeCN/water with $HCl_{aq}$. An insoluble acid was filtered off and the filtrate was taken for the preparative reversed-phase column chromatography (C-18, water + 0.3‰ HCl (36%)/MeCN, 95:5 to 20:80, 30 min, 20 mL/min). The title compound (85) was obtained as a single isomer as a hydrochloride salt in 49% yield (33 mg; 0.057 mmol).
ESI-MS m/z for $C_{26}H_{35}ClN_5O_3S$ found 544.2/546.2 $[M+H]^+$; $R_t$ = 1.06 min (Method A); $^1$H NMR (700 MHz, DMSO-$d_6$ + $D_2O$, 348 K) δ 8.18 (s, 1H), 7.38 (d, $J_{AA'}$ = 8.5 Hz, 2H), 7.33 (d, $J_{BB'}$ = 8.5 Hz, 2H), 5.07 - 5.05 (m, 1H), 4.59 (t, *J* = 6.6 Hz, 2H), 3.93 - 3.92 (m, 1H), 3.85 - 3.83 (m, 1H), 3.71 - 3.66 (m, 2H), 3.61 - 3.56 (m, 2H), 3.24 (dd, *J* = 13.9 Hz, *J* = 10.6 Hz, 1H), 3.20 - 3.17 (m, 1H), 2.92 - 2.85 (m, 3H), 2.34 - 2.30 (m, 2H), 2.26 (s, 3H), 2.19 - 2.16 (m, 5H), 1.67 - 1.51 (m, 4H).

**Example 86.**

Synthesis of (2*R*,5*S*)-5-(4-chlorobenzyl)-4-(4-(1,5-dimethyl-1*H*-pyrazol-3-yl)cyclohex-3-en-1-yl)-2-((methylsulfonyl) methyl)morpholine hydrochloride **(86).**

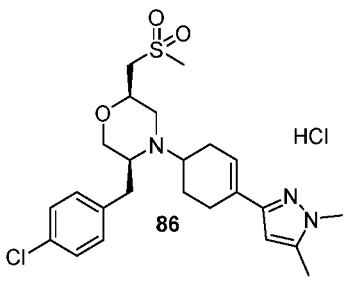

86

Step 1.

Synthesis of 1,5-dimethyl-3-(1,4-dioxaspiro[4.5]dec-7-en-8-yl)-1*H*-pyrazole **(86a).**

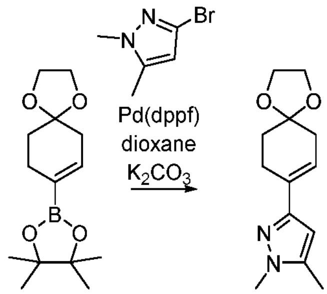


**86a**

The solution of 4,4,5,5-tetramethyl-2-(1,4-dioxaspiro[4.5]dec-7-en-8-yl)-1,3,2-dioxaborolane (980 mg; 3.68 mmol), 3-bromo-1,5-dimethyl-1H-pyrazole (613 mg, 3.50 mmol), Pd(dppf) (143 mg; 0.175 mmol) in mixture of dioxane and 2 M aqueous solution of $K_2CO_3$ (20 mL; 4:1 v/v) was heated at 100 °C for 3 hours under argon atmosphere. LC-MS was showed complete consumption of the starting material. Then a whole was concentrated *in vacuo* and the residue was purified by flash column chromatography on silica (hexane/AcOEt, 90:10 to 0:100, *v/v,* 30 min, 30 mL/min). Compound **86a** was obtained in 79% yield (650 mg; 2.78 mmol).
ESI-MS m/z for $C_{13}H_{19}N_2O_2$ found 235.1 $[M+H]^+$; $R_t$ = 0.99 min (Method A)

Step 2.

Synthesis of 4-(1,5-dimethyl-1*H*-pyrazol-3-yl)cyclohex-3-en-1-one **(86b).**

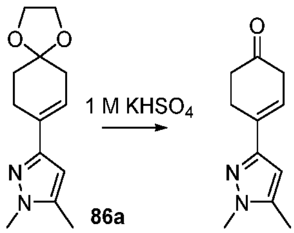


**86a** **86b**

The solution of **86a** (200 mg; 0.855 mmol) in 1 M $KHSO_{4aq}$ (15 mL) was stirred at 30 °C for 3 hours. The reaction progress was monitored by LC-MS. When analysis indicated completion of the reaction, to this mixture water was added and a whole was taken for lyophilization. Then all crude product was used to the next step without additional purification.
ESI-MS m/z for $C_{13}H_{19}N_2O_2$ found 235.1 $[M+H]^+$; $R_t$ = 0.98 min (Method A)

Step 3.

Synthesis of (2*R*,5*S*)-5-(4-chlorobenzyl)-4-(4-(1,5-dimethyl-1*H*-pyrazol-3-yl)cyclohex-3-en-1-yl)-2-((methylsulfonyl) methyl)morpholine hydrochloride **(86).**

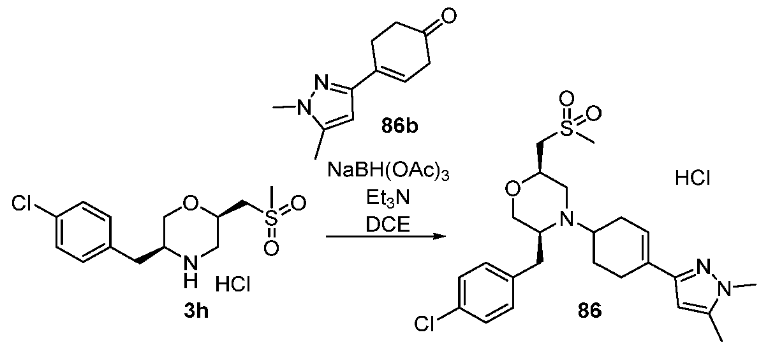

3h
86

The title compound **(86)** was obtained as a hydrochloride salt from **3h** (288 mg; 0.85 mmol) and ketone **86b** (the crude product) according to the General Procedure **VII** in % yield (4 mg; 0.09 mmol). The crude product was purified by flash column chromatography on silica (hexane/AcOEt, 100:0 to 30:70, v/v) and then purified three times by preparative reversed-phase column chromatography (twice: C-18, water + 0.3‰ HCl (36%)/MeCN, 95:5 to 20:80, 30 min, 20 mL/min; third: column: Cosmosil Cholester 20 x 250 mm, water + 0.3‰ HCl (36%)/MeCN, 95:5 to 20:80, 30 min, 20 mL/min). ESI-MS m/z for $C_{24}H_{33}ClN_3O_3S$ found 478.0/480.0 $[M+H]^+$; $R_t$ = 1.00 min (Method A); $^1H$ NMR (700 MHz, $D_2O$, 333 K) δ 7.83 - 7.81 (m, 2H), 7.73 - 7.70 (m, 2H), 6.67- 6.66 (m, 1H), 6.60 - 6.59 (m, 1H), 4.41 - 4.39 (m, 1H), 4.35 - 4.10 (m, 8H), 4.07 - 4.05 (m, 1H), 3.94 - 3.91 (m, 1H), 3.88 - 3.84 (m, 1H), 3.71 - 3.66 (m, 1H), 3.62 - 3.59 (m, 4H), 3.26 - 3.20 (m, 1H), 3.11 - 3.05 (m, 1H), 2.91 - 2.79 (m, 3H), 2.65 (s, 3H), 2.26 - 2.20 (m, 1H).

**Example 87.**

Synthesis of *N*-(35-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)-3,6,9,12,15,18,21,24,27,30,33-undecaoxapentatriacontyl)-5-((3*aS*,4*S*,6*aR*)-2-oxohexahydro-1*H*-thieno [3,4-*d*]imidazol-4-yl)pentanamide hydrochloride **(87).**

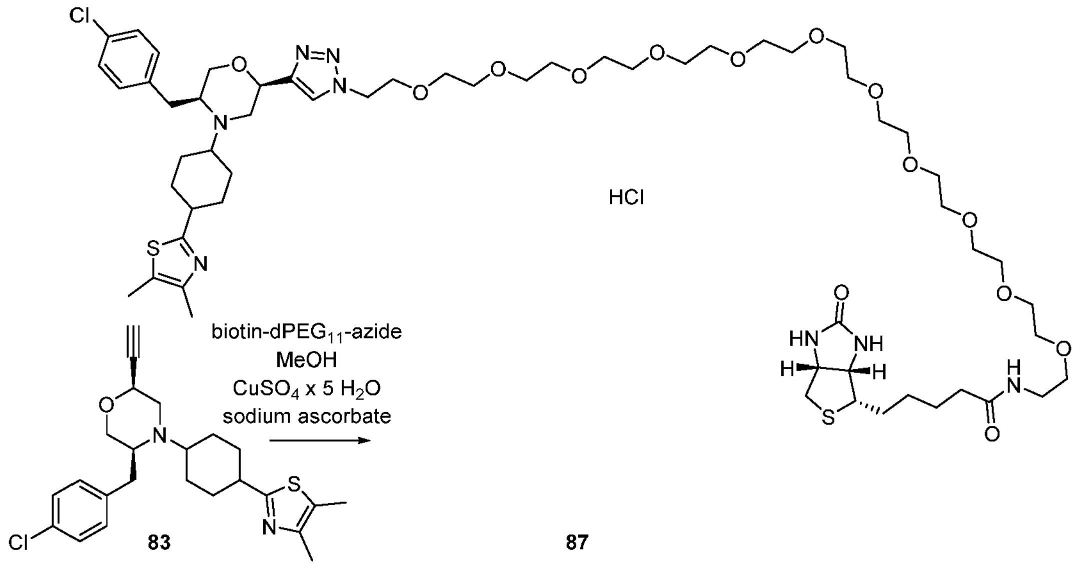

83
87

To the solution of 83 (20 mg; 0.047 mmol) in MeOH (02 mL) $CuSO_4$ x 5 $H_2O$ (6 mg; 0.023 mmol) was added and after 5 minutes to a light green solution sodium L-ascorbate (10 mg; 0.047 mmol) was added in one portion. A whole was stirred for 25 minutes at room temperature and then a solution of an azide (37 mg; 0.047 mmol) in MeOH (0.5 mL) was added and the reaction mixture was stirred at room temperature overnight. After analytical control (LC-MS) indicated completion of the reaction, the reaction mixture was concentrated *in vacuo* and the residue was mixed with water/MeCN and then this mixture was filtered and purified twice by preparative reversed-phase column chromatography (first: C-18, water + 0.3‰ HCl (36%)/MeCN, 95:5 to 20:80, 30 min, 20 mL/min, second: column: Cosmosil Cholester 20 x 250 mm, water + 0.3‰ HCl (36%)/MeCN, 95:5 to 20:80, 30 min, 20 mL/min). Compound **87** was obtained as a single isomer as a hydrochloride salt in 36% yield (21 mg; 0.017 mmol).

ESI-MS m/z for $C_{58}H_{94}ClN_8O_{14}S_2$ found 1225.4/1227.4 $[M+H]^+$; Rt = 1.24 min (Method A)

**Example 88.**

Synthesis of 2-((2*S*,5*S*)-5-(4-chlorobenzyl)-4-(4-(4,5-dimethylthiazol-2-yl)cyclohexyl)morpholin-2-yl)acetic acid hydrochloride **(88).**

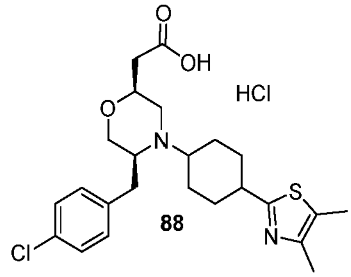

88

Step 1.

Synthesis of methyl 2-((2*S*,5*S*)-5-(4-chlorobenzyl)-4-(4-(4,5-dimethylthiazol-2-yl)cyclohexyl)morpholin-2-yl)acetate **(88a).**

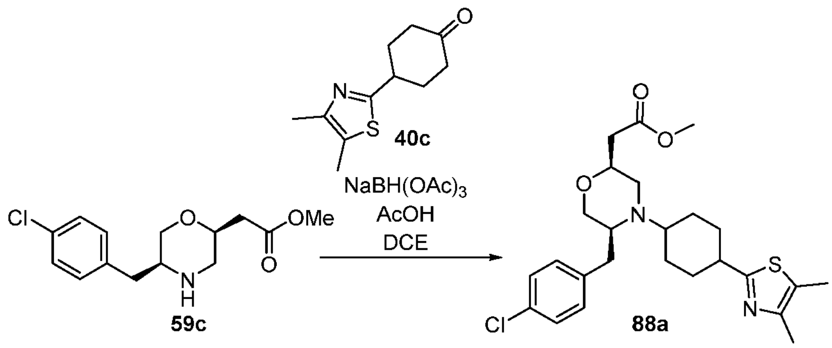


40c, 59c, 88a

The title compound **(88a)** was obtained from **59c** (150 mg; 0.48 mmol) and ketone **40c** (100 mg; 0.48 mmol) according to the General Procedure **VII** in 27% yield (62 mg; 0.13 mmol). ESI-MS m/z for $C_{25}H_{34}ClN_2O_3S$ found 477.1/479.1 $[M+H]^+$; Rt = 2.27 min (Method D)

Step 2.

Synthesis of 2-((2*S*,5*S*)-5-(4-chlorobenzyl)-4-(4-(4,5-dimethylthiazol-2-yl)cyclohexyl)morpholin-2-yl)acetic acid hydrochloride **(88).**

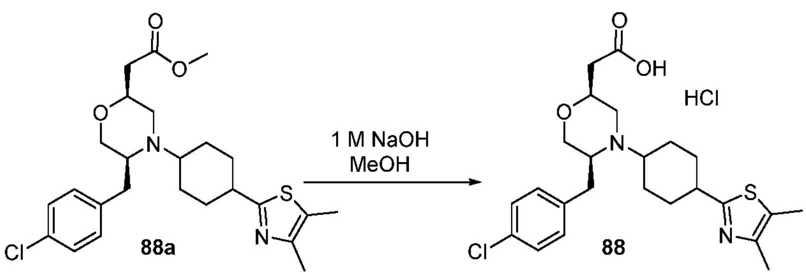


88a, 88

To the solution of 88a (62 mg; 0.13 mmol) in MeOH (3 mL) 1 M NaOH (2 mL) was added and a whole was stirred at room temperature for 4 hours. The reaction progress was monitored by LC-MS. Then to this mixture 1M HCl was added to pH = 2. The crude product was purified by preparative reversed-phase column chromatography (Luna® 5μm Phenyl-Hexyl, LC Column 250 x 21.2 mm, water/MeCN, 95:5 to 5:95, 50 min, 12 mL/min). The title compound **(88)** was obtained as a single isomer as a hydrochloride salt in 61% yield (42 mg; 0.08 mmol).
ESI-MS m/z for $C_{24}H_{32}ClN_2O_3S$ found 463.1/465.1 $[M+H]^+$; $R_t$ = 2.16 min (Method D); $^1H$ NMR (400 MHz, DMSO-$d_6$) δ 7.46 - 7.11 (m, 4H), 4.30 - 4.16 (m, 1H), 3.96 - 3.84 (m, 1H), 3.49 - 3.33 (m, 2H), 3.17 - 2.98 (m, 2H), 2.92 - 2.78 (m, 1H), 2.73 - 2.57 (m, 2H), 2.29 - 2.06 (m, 8H), 1.95 - 1.66 (m, 5H), 1.62 - 1.33 (m, 2H), 1.32 - 1.15 (m, 2H).

**Example 89.**

Synthesis of 3-((((2*R*,5*S*)-5-(4-chlorobenzyl)-4-(4-(4,5-dimethylthiazol-2-yl)cyclohexyl)morpholin-2-yl)methyl)sulfonyl) propanoic acid hydrochloride **(89).**

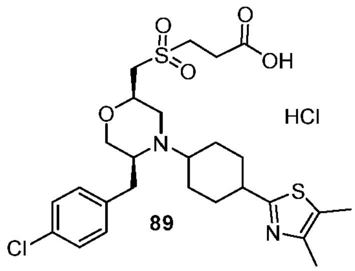

89

Step 1.

Synthesis of 3-((((2*R*,5*S*)-4-(tert-butoxycarbonyl)-5-(4-chlorobenzyl)morpholin-2-yl)methyl)thio)propanoic acid **(89a).**

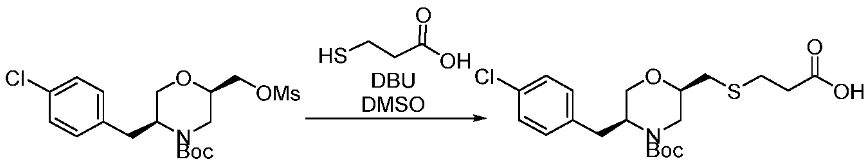


3e 89a

To a DMSO (5 mL) DBU (1.91 g; 11.93mmol) was added and degassed. Then to this solution 3-mercaptopropanoic acid (666 mg; 5.97 mmol) was added. Then the compound **3e** (1.0 g; 2.39 mmol) was dissolved in DMSO (9 mL) and degassed and added to the mixture with DBU and all mixture was stirred at room temperature. The reaction progress was monitored by LC-MS analysis. After analytical control indicated completion of the reaction, the mixture was extracted with DCM and then an organic solution was washed with HCl and dried over anhydrous $Na_2SO_4$, filtered and concentrated *in vacuo* and the crude product was used to the next step without additional purification.
ESI-MS m/z for $C_{15}H_{21}ClNO_3S$ found 330.0/332.0 $[M+H-Boc]^+$; $R_t$ = 3.59 min (Method D) Step 2.

Synthesis of 3-((((2*R*,5*S*)-4-(tert-butoxycarbonyl)-5-(4-chlorobenzyl)morpholin-2-yl)methyl)sulfonyl)propanoic acid **(89b).**

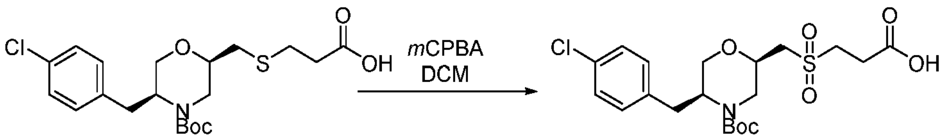


89a 89b

To a solution of **89a** (the crude product) in DCM (15 mL) mCPBA (1.65 g; 9.54 mmol) was added portionwise at 0 °C. Then the cooling bath was removed and the mixture was stirred at room temperature overnight. The reaction progress was monitored by LC-MS analysis. When analysis indicated completion of the reaction, the reaction was quenched with 10% $Na_2SO_3$ and stirred for 30 minutes at room temperature. Then phases were separated and the organic one was washed with 1 M NaOH (4 x) and brine, dried over anhydrous $Na_2SO_4$, filtered and concentrated *in vacuo* and the crude product was used to the next step without additional purification. Compound **89b** was obtained in 54% yield (per two steps) (0.60 g; 1.30 mmol).
ESI-MS m/z for $C_{20}H_{28}ClNO_7SNa$ found 484.1/486.1 $[M+Na]^+$; $R_t$ = 3.32 min (Method D)

Step 3.

Synthesis of methyl 3-((((2*R*,5*S*)-5-(4-chlorobenzyl)morpholin-2-yl)methyl)sulfonyl)propanoate **(89c).**

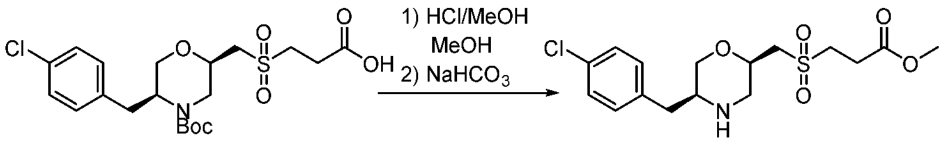


**89b** **89c**

The title compound **(89c)** was obtained as a free amine in 99% yield (484 mg; 1.29 mmol) from **89b** (0.60 g; 1.30 mmol) according to the General Procedure **IVa** and then after basic (saturated solution of $NaHCO_3$) extraction with DCM.
ESI-MS m/z for $C_{16}H_{23}ClNO_5S$ found 376.0/378.0 $[M+H]^+$; Rt = 2.21 min (Method D)

Step 4.

Synthesis of 3-((((2*R*,5*S*)-5-(4-chlorobenzyl)-4-(4-(4,5-dimethylthiazol-2-yl)cyclohexyl)morpholin-2-yl)methyl)sulfonyl) propanoic acid hydrochloride **(89).**

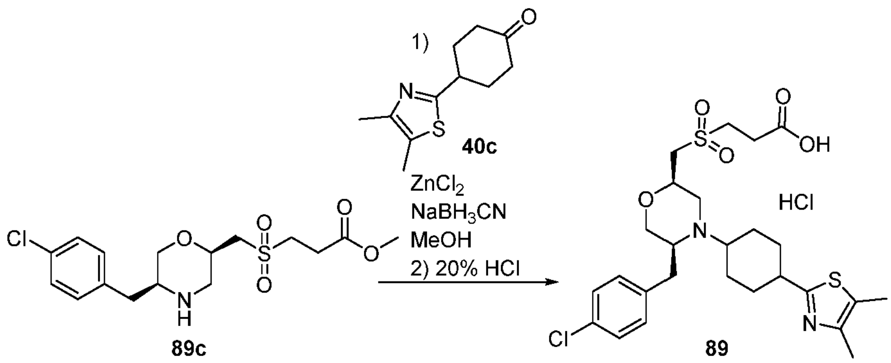


To the solution of **89c** (256 mg; 0.68 mmol) in MeOH (2mL) a ketone **40c** (150 mg; 0.72 mmol) and $ZnCl_2$ (107 mg; 0.78 mmol) were added. After everything was dissolved $NaBH_3CN$ (50 mg; 0.78 mmol) was added and the mixture was stirred at room temperature overnight. The reaction progress was monitored by LC-MS analysis. After analytical control indicated completion of the reaction, the mixture was extracted with DCM and then an organic solution was washed with water and dried over anhydrous $Na_2SO_4$, filtered and concentrated *in vacuo* and the crude product was purified by flash column chromatography on silica (hexane/AcOEt, 6:4 to 0:100, *v*/*v*) and then the obtained product (25% yield; 96 mg; 0.17 mmol) was dissolved in 20% HCl (5 mL) and all was stirred at room temperature for 2.5 hours. After analytical control indicated completion of the reaction, the solvents were removed *in vacuo* and the residue was purified by preparative reversed-phase column chromatography (Luna® 5μm Phenyl-Hexyl, LC Column 250 x 21.2 mm, water/MeCN, 95:5 to 5:95, 50 min, 12 mL/min). The title compound **(89)** was obtained as a single isomer as a hydrochloride salt in 45% yield (45 mg; 0.076 mmol).
ESI-MS m/z for $C_{26}H_{36}ClN_2O_5S_2$ found 555.1/557.1 $[M+H]^+$; Rt = 2.20 min (Method D); $^1H$ NMR (400 MHz, DMSO-$d_6$) δ 7.43 - 7.37 (m, 2H), 7.36 - 7.31 (m, 2H), 4.38 - 4.28 (m, 1H), 3.96 - 3.88 (m, 1H), 3.83 - 3.70 (m, 2H), 3.47 - 3.40 (m, 3H), 3.37

- 3.28 (m, 1H), 3.21 - 3.07 (m, 2H), 2.94 - 2.63 (m, 3H), 2.37 - 2.30 (m, 2H), 2.28 (s, 3H), 2.24 - 2.11 (m, 6H), 2.04 - 1.93 (m, 1H), 1.80 - 1.41 (m, 5H).

**Example 90.**

Synthesis of 3-(((2*R*,5*S*)-5-(4-chlorobenzyl)-4-(4-(1,5-dimethyl-1*H*-pyrazol-3-yl)cyclohexyl)morpholin-2-yl)methyl)-6,6-dimethyl-3-azabicyclo[3.1.0]hexane-2,4-dione hydrochloride **(90).**

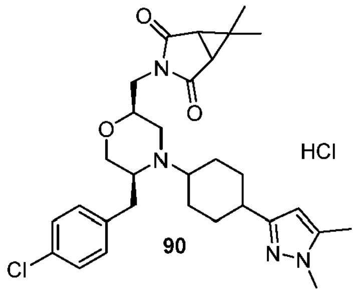

The title compound **(90)** was obtained as a hydrochloride salt as a single isomer in 4% overall yield in a similar way to Example 82 with the exception that, in the first step of the synthesis, 6,6-dimethyl-3-azabicyclo[3.1.0]hexane-2,4-dione was used instead of a succinimide and in the second step of the synthesis, the obtained TFA salt was transformed into a free amine with the basic (0.5 M NaOH) extraction with DCM. The crude product was purified by flash column chromatography on silica (AcOEt/MeOH, 100:0 to 70:30, *v/v,* 24 min) and then by preparative reversed-phase column chromatography (Luna® 5μm Phenyl-Hexyl, LC Column 250 x 21.2 mm, water + 1‰ HCl (36%)/MeCN, 90:10 to 5:95, 35 min, Rt = 25 min, 12 mL/min).
ESI-MS m/z for $C_{30}H_{40}ClN_4O_3$ found 539.2/541.2 $[M+H]^+$; Rt = 2.39 min (Method D); $^1$H NMR (400 MHz, $D_2O$) δ 7.27 - 7.14 (m, 2H), 7.13 - 7.01 (m, 2H), 6.09 (s, 1H), 3.85 - 3.74 (m, 1H), 3.73 - 3.65 (m, 1H), 3.63 (s, 3H), 3.59 - 3.44 (m, 3H), 3.43 - 3.27 (m, 3H), 3.03 - 2.90 (m, 3H), 2.67 - 2.53 (m, 1H), 2.50 - 2.34 (m, 2H), 2.34 - 2.17 (m, 2H), 2.12 (s, 3H), 2.06 - 1.92 (m, 2H), 1.45 - 1.26 (m, 4H), 1.04 (s, 3H), 1.00 (s, 3H).

**Example 91.**

Synthesis of 3-(((2*R*,5*S*)-5-(4-chlorobenzyl)-4-(4-(4,5-dimethylthiazol-2-yl)cyclohexyl)morpholin-2-yl)methyl)-6,6-dimethyl-3-azabicyclo[3.1.0]hexane-2,4-dione hydrochloride **(91).**

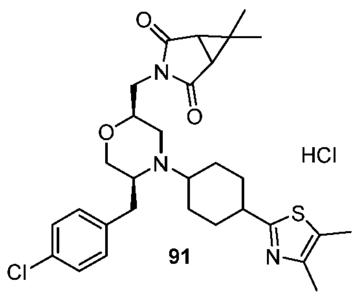

The title compound **(91)** was obtained as a hydrochloride salt as a single isomer in 5% overall yield in a similar way to Example 82 with the exception that, in the first step of the synthesis, 6,6-dimethyl-3-azabicyclo[3.1.0]hexane-2,4-dione was used instead of a succinimide and in the second step of the synthesis, the obtained TFA salt was transformed into a free amine with the basic (0.5 M NaOH) extraction with DCM and in the third step of the synthesis, the ketone **40c** was used instead of the ketone **2d.** The crude product was purified by flash column chromatography on silica (AcOEt/MeOH, 100:0 to 70:30, *v/v,* 24 min) and then by preparative reversed-phase column chromatography (Luna® 5μm Phenyl-Hexyl, LC Column 250 x 21.2 mm, water + 1‰ HCl (36%)/MeCN, 90:10 to 5:95, 35 min, Rt = 24 min, 12 mL/min).
ESI-MS m/z for $C_{30}H_{39}ClN_3O_3S$ found 556.1/558.1 $[M+H]^+$; Rt = 2.54 min (Method D); $^1$H NMR (400 MHz, $D_2O$) δ 7.26 -

7.19 (m, 2H), 7.13 - 7.05 (m, 2H), 3.90 - 3.77 (m, 1H), 3.77 - 3.67 (m, 1H), 3.66 - 3.32 (m, 6H), 3.19 - 3.10 (m, 1H), 3.07 - 2.93 (m, 3H), 2.51 - 2.40 (m, 2H), 2.34 - 2.25 (m, 2H), 2.23 - 2.10 (m, 8H), 1.66 - 1.36 (m, 4H), 1.07 (s, 3H), 1.02 (s, 3H).

**Example 92.**

Synthesis of 3-((((2*S*,5*S*)-5-(4-chlorobenzyl)-4-(4-(1,5-dimethyl-1*H*-pyrazol-3-yl)cyclohexyl)morpholin-2-yl)methyl)amino)-4-hydroxycyclobut-3-ene-1,2-dione hydrochloride **(92).**

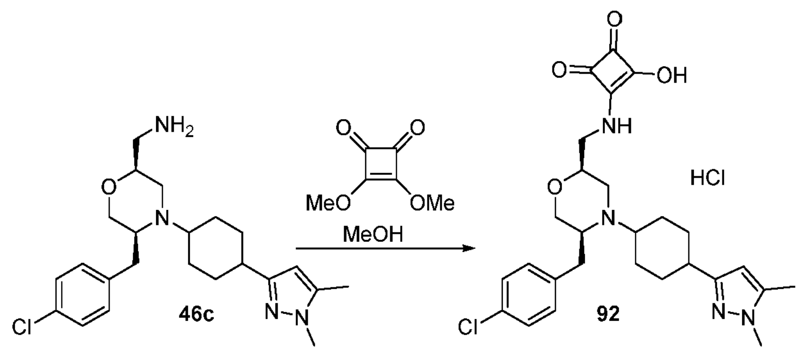


46c 92

To the solution of **46c** (120 mg; 0.288 mmol) in MeOH (2 mL) 3,4-dimethoxycyclobut-3-ene-1,2-dione (41 mg; 0.288 mmol) weas added and the reaction mixture was stirred at room temperature for 30 minutes. The reaction progress was monitored by LC-MS. When analysis indicated completion of the reaction, the mixture was concentrated *in vacuo.* The crude product was treated with water and evaporated *in vacuo.* Then to this residue a solution of $NH_4Cl$ was added and stirred at room temperature for 30 minutes. Then the mixture was concentrated *in vacuo* and the crude product was purified by preparative reversed-phase column chromatography (Luna® 5μm Phenyl-Hexyl, LC Column 250 x 21.2 mm, water + 1‰ HCl (36%)/MeCN, 90:10 to 5:95, 35 min, Rt = 22.5 min, 12 mL/min). The title compound **(92)** was obtained as a hydrochloride salt as a single isomer in 49% yield (78 mg; 0.142 mmol).
ESI-MS m/z for $C_{27}H_{34}ClN_4O_4$ found 513.1/515.1 $[M+H]^+$; Rt = 2.14 min (Method D); $^1H$ NMR (400 MHz, Methanol-$d_4$) δ 7.40 - 7.35 (m, 2H), 7.35 - 7.30 (m, 2H), 6.39 (s, 1H), 4.15 - 3.99 (m, 1H), 3.92 (s, 3H), 3.89 - 3.73 (m, 5H), 3.73 - 3.65 (m, 1H), 3.65 - 3.54 (m, 1H), 3.27 - 3.13 (m, 3H), 2.89 - 2.77 (m, 1H), 2.55 - 2.43 (m, 2H), 2.40 (s, 3H), 2.31 - 2.20 (m, 2H), 1.86 - 1.60 (m, 4H).

**Example 93.**

Synthesis of 3-amino-4-((((2*S*,5*S*)-5-(4-chlorobenzyl)-4-(4-(1,5-dimethyl-1*H*-pyrazol-3-yl)cyclohexyl)morpholin-2-yl) methyl)amino)cyclobut-3-ene-1,2-dione hydrochloride (93).

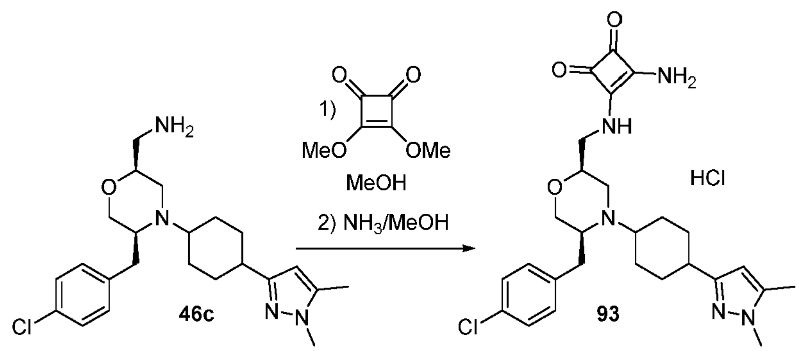


46c 93

To the solution of 46c (120 mg; 0.288 mmol) in MeOH (2 mL) 3,4-dimethoxycyclobut-3-ene-1,2-dione (41 mg; 0.288 mmol) weas added and the reaction mixture was stirred at room temperature overnight. Then to this mixture $NH_3$ (7 N solution in MeOH; 0.1mL) was added and stirred at room temperature overnight. The reaction progress was monitored by LC-MS. When analysis indicated completion of the reaction, the mixture was concentrated *in vacuo.* The crude product

was purified by preparative reversed-phase column chromatography (Luna® 5μm Phenyl-Hexyl, LC Column 250 x 21.2 mm, water + 1‰ HCl (36%)/MeCN, 90:10 to 5:95, 35 min, Rt = 21.5 min, 12 mL/min). The title compound **(93)** was obtained as a hydrochloride salt as a single isomer in 18% yield (29 mg; 0.053 mmol).
ESI-MS m/z for $C_{27}H_{35}ClN_5O_3$ found 512.1/514.1 $[M+H]^+$; Rt = 1.79 min (Method D); $^1H$ NMR (400 MHz, Methanol-$d_4$) δ 7.39 - 7.26 (m, 4H), 6.38 (s, 1H), 4.09 - 3.99 (m, 1H), 3.95 - 3.85 (m, 5H), 3.86 - 3.70 (m, 4H), 3.64 - 3.52 (m, 1H), 3.28 - 3.10 (m, 3H), 2.89 - 2.80 (m, 1H), 2.55 - 2.45 (m, 2H), 2.40 (s, 3H), 2.33 - 2.22 (m, 2H), 1.86 - 1.60 (m, 4H).

**Example 94.**

Synthesis of (2*R*,5*S*)-5-(4-chlorobenzyl)-4-(4-(4,5-dimethylthiazol-2-yl)cyclohexyl)-2-((ethylsulfonyl)methyl)morpholine hydrochloride **(94).**

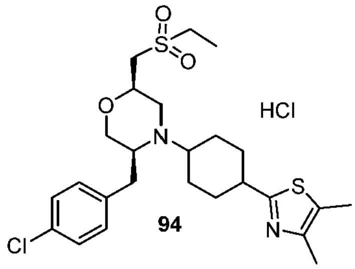

**94**

Step 1.

Synthesis of *tert-butyl* (2*R*,5*S*)-5-(4-chlorobenzyl)-2-((ethylthio)methyl)morpholine-4-carboxylate **(94a).**

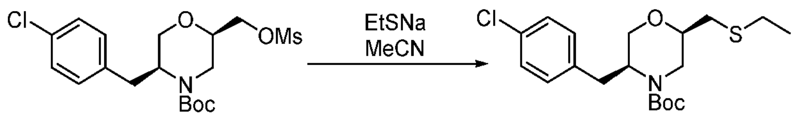


**3e** **94a**

To a solution of **3e** (700 mg; 1.66 mmol) in acetonitrile (3 mL) EtSNa (460 mg; 3.33 mmol) was added and the reaction mixture was stirred at 82 °C overnight. The reaction progress was monitored by LC-MS analysis. After analytical control indicated completion of the reaction, the mixture was diluted with water and the MeCN was evaporated off. An aqueous phase was extracted with DCM (3 x). The combined organic phases were washed with 1 M NaOH and brine, dried over anhydrous $Na_2SO_4$, filtered and concentrated *in vacuo* and the crude product was used to the next step without additional purification. Compound **94a** was obtained in 99% yield (632 mg; 1.64 mmol).
ESI-MS m/z for $C_{19}H_{29}ClNO_3S$ found 386.1/388.1 $[M+H]^+$; Rt = 4.59 min (Method D).

Step 2.

Synthesis of *tert-butyl* (2*R*,5*S*)-5-(4-chlorobenzyl)-2-((ethylsulfonyl)methyl)morpholine-4-carboxylate **(94b).**

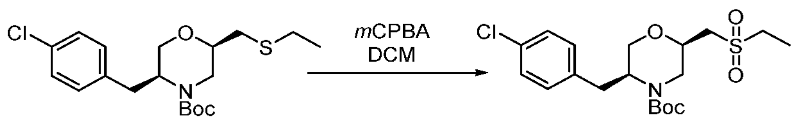


**94a** **94b**

To a solution of **94a** (632 mg; 1.64 mmol) in DCM (6.6 mL) mCPBA (719 mg; 4.16 mmol) was added portionwise at 0 °C. Then the cooling bath was removed and the mixture was stirred at room temperature overnight. The reaction

progress was monitored by LC-MS analysis. Then the reaction mixture was cooled to 0 °C and the another portion of mCPBA (719 mg; 4.16 mmol) was added and stirred at room temperature for 4 hours. When analysis indicated completion of the reaction, the reaction was quenched with 10% $Na_2SO_3$ (20 mL) and stirred for 30 minutes at room temperature. Then phases were separated and the organic one was washed with 1 M NaOH (4 x) and brine, dried over anhydrous $Na_2SO_4$, filtered and concentrated *in vacuo* and the crude product was purified by flash column chromatography on silica (dry load, DCM/AcOEt, 100:0 to 0:100, v/v). Compound **94b** was obtained in 99% yield (677 mg; 1.62 mmol).
ESI-MS m/z for $C_{19}H_{29}ClNO_5S$ found 418.1/420.1 $[M+H]^+$; Rt = 3.67 min (Method D).

Step 3.

Synthesis of (2*R*,5*S*)-5-(4-chlorobenzyl)-2-((ethylsulfonyl)methyl)morpholine 2,2,2-trifluoroacetate **(94c).**

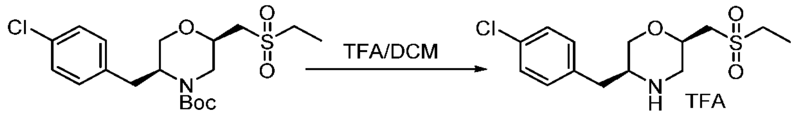


**94b** **94c**

The title compound **(94c)** was obtained as a TFA salt in 99% yield (207 mg; 0.48 mmol) from **94b** (200 mg; 0.48 mmol) according to the General Procedure **IVb.**
ESI-MS m/z for $C_{14}H_{21}ClNO_3S$ found 318.0/320.0 $[M+H]^+$; $R_t$ = 1.38 min (Method D).

Step 4.

Synthesis of (2*R*,5*S*)-5-(4-chlorobenzyl)-4-(4-(4,5-dimethylthiazol-2-yl)cyclohexyl)-2-((ethylsulfonyl)methyl)morpholine hydrochloride **(94).**

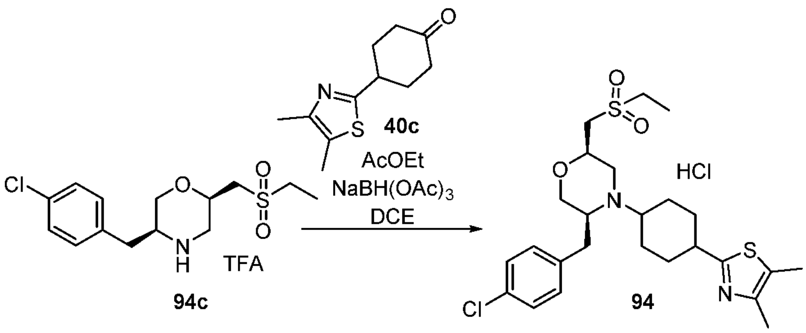


**94c** **94**

The title compound **(94)** was obtained as a hydrochloride salt as a single isomer from **94c** (207 mg; 0.48 mmol) and ketone **40c** (100 mg; 0.48 mmol) according to the General Procedure **VII** in 19% yield (48 mg; 0.09 mmol). The crude product was purified by flash column chromatography on silica (DCM/AcOEt, 100:0 to 0:100, v/v, then: AcOEt/MeOH, 92:8, v/v) and then by preparative reversed-phase column chromatography (Luna® 5μm Phenyl-Hexyl, LC Column 250 x 21.2 mm, water + 1‰ HCl (36%)/MeCN, 95:5 to 20:80, 30 min, 15 mL/min).
ESI-MS m/z for $C_{25}H_{36}ClN_2O_3S_2$ found 511.0/513.0 $[M+H]^+$; Rt = 2.15 min (Method D); $^1H$ NMR (400 MHz, $D_2O$) δ 7.20 - 7.16 (m, 2H), 7.09 - 7.04 (m, 2H), 4.22 - 4.14 (m, 1H), 3.77 - 3.69 (m, 1H), 3.68 - 3.62 (m, 1H), 3.58 - 3.37 (m, 4H), 3.28 - 3.15 (m, 2H), 3.15 - 3.01 (m, 4H), 2.99 - 2.93 (m, 1H), 2.35 - 2.20 (m, 2H), 2.16 (s, 3H), 2.13 (s, 3H), 1.78 - 1.72 (m, 2H), 1.59 - 1.38 (m, 4H), 1.13 (t, *J* = 7.4 Hz, 3H).

**Example 95.**

Synthesis of (2*R*,5*S*)-5-(4-chlorobenzyl)-4-(4-(1,5-dimethyl-1*H*-pyrazol-3-yl)cyclohexyl)-2-((ethylsulfonyl)methyl)morpholine hydrochloride **(95).**

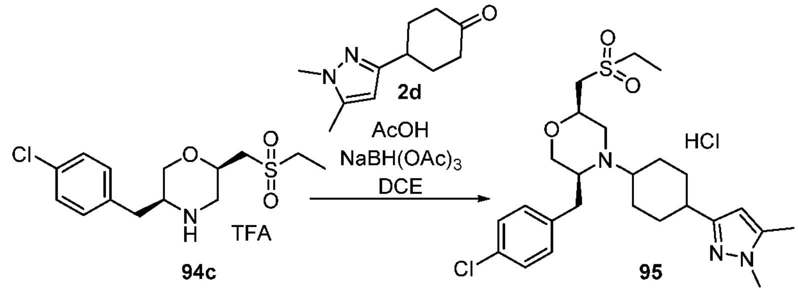

94c 95

The title compound **(95)** was obtained as a hydrochloride salt as a single isomer from **94c** (207 mg; 0.48 mmol) and ketone **2d** (192 mg; 1.00 mmol) according to the General Procedure **VII** in 14% yield (35 mg; 0.07 mmol). The crude product was purified by flash column chromatography on silica (DCM/AcOEt, 100:0 to 40:60, v/v) and then by preparative reversed-phase column chromatography (Luna® 5μm Phenyl-Hexyl, LC Column 250 x 21.2 mm, water + 1‰ HCl (36%)/MeCN, 95:5 to 20:80, 30 min, 15 mL/min).
ESI-MS m/z for $C_{25}H_{37}ClN_3O_3S$ found 494.1/496.1 $[M+H]^+$; Rt = 2.35 min (Method D); $^1H$ NMR (400 MHz, $D_2O$) δ 7.19 - 7.10 (m, 2H), 7.08 - 6.99 (m, 2H), 6.06 (s, 1H), 4.17 - 4.10 (m, 1H), 3.72 - 3.65 (m, 1H), 3.64 - 3.61 (m, 1H), 3.59 (s, 3H), 3.51 - 3.40 (m, 3H), 3.35 - 3.27 (m, 1H), 3.25 - 3.14 (m, 2H), 3.09 - 2.99 (m, 3H), 2.96 - 2.88 (m, 1H), 2.62 - 2.51 (m, 1H), 2.27 - 2.12 (m, 2H), 2.07 (s, 3H), 2.01 - 1.89 (m, 2H), 1.48 - 1.23 (m, 4H), 1.09 (t, *J* = 7.2 Hz, 3H).

**Example 96.**

Synthesis of 3-(((2*R*,5*S*)-5-(4-chlorobenzyl)-4-(4-(1,5-dimethyl-1*H*-pyrazol-3-yl)cyclohexyl)morpholin-2-yl)methyl)-1-methylimidazolidine-2,4-dione hydrochloride **(96).**

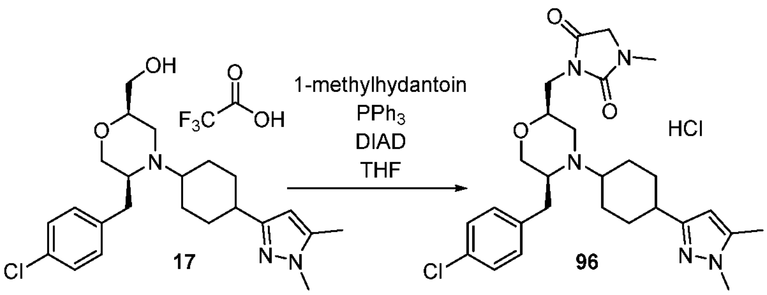

17 96

To the solution of an alcohol **17** (133 mg; 0.25 mmol) and 1-methylhydantoin (69 mg; 0.60 mmol) in THF (3 mL) $PPh_3$ (158 mg; 0.60 mmol) was added. Then a whole was cooled to -10 °C and DIAD (130 μL; 0.65 mmol) was added dropwise and stirred at room temperature overnight. The reaction progress was monitored by LC-MS. Then a solid was filtered off and a light yellow filtrate was concentrated *in vacuo*. The residue was dissolved in water/MeCN/$HCl_{aq}$, filtered off and taken for preparative reversed-phase column chromatography (first: column: Cosmosil Cholester 20 x 250 mm, water + 0.3‰ HCl (36%)/MeCN, 98:2 to 30:70, 30 min, 20 mL/min, second: C-18, water + 0.3‰ HCl (36%)/MeCN, 98:2 to 20:80, 30 min, 18 mL/min). The title compound **(96)** was obtained as a hydrochloride salt as a single isomer in 36% yield (51 mg; 0.09 mmol).
ESI-MS m/z for $C_{27}H_{37}ClN_5O_3$ found 514.4/516.4 $[M+H]^+$; Rt = 0.99 min (Method D); $^1H$ NMR (700 MHz, DMSO-$d_6$ + $D_2O$, 348 K) δ 7.41 - 7.36 (m, 2H), 7.32 - 7.28 (m, 2H), 5.83 (s, 1H), 4.11 - 4.03 (m, 1H), 4.01 - 3.94 (m, 2H), 3.79 - 3.74 (m, 1H), 3.73 - 3.68 (m, 2H), 3.66 - 3.62 (m, 1H), 3.61 (s, 3H), 3.56 - 3.51 (m, 2H), 3.47 - 3.44 (m, 1H), 3.19 - 3.09 (m, 3H), 2.88 (s, 3H), 2.48 - 2.44 (m, 1H), 2.28 - 2.21 (m, 2H), 2.17 (s, 3H), 2.07 - 2.01 (m, 2H), 1.59 - 1.52 (m, 2H), 1.46 - 1.40 (m, 2H).

**Example 97.**

Synthesis of (2*R*,5*S*)-5-(4-chlorobenzyl)-4-(4-(4,5-dimethylthiazol-2-yl)cyclohexyl)-*N*-(1-methyl-1*H*-imidazol-4-yl)morpholine-2-carboxamide hydrochloride **(97).**

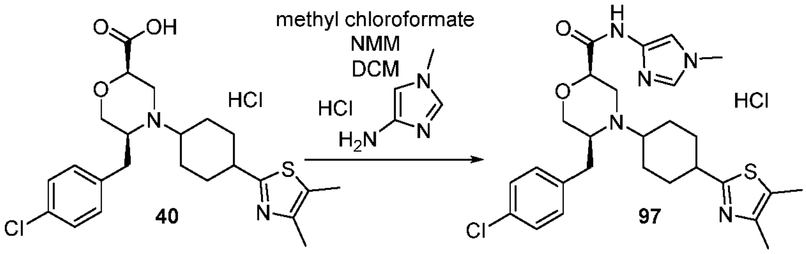


The title compound **(97)** was obtained as a hydrochloride salt as a single isomer from **40** (43 mg; 0.089 mmol) and from 1-methyl-1*H*-imidazol-4-amine hydrochloride (15 mg; 0.107 mmol) according to the General Procedure **V** in 30% yield (15 mg; 0.027 mmol). The crude product was purified by preparative reversed-phase column chromatography (column: Cosmosil Cholester 20 x 250 mm, water + 0.3‰ HCl (36%)/MeCN, 98:2 to 20:80, 30 min, 18 mL/min).
ESI-MS m/z for $C_{27}H_{35}ClN_5O_2S$ found 528.4/530.4 $[M+H]^+$; $R_t$ = 1.02 min (Method A); $^1$H NMR (700 MHz, DMSO-$d_6$ + $D_2O$, 348 K) δ 8.31 (s, 1H), 7.43 (d, *J* = 1.7 Hz, 1H), 7.41 (d, $I_{AA'}$ = 8.5 Hz, 2H), 7.36 (d, $J_{BB'}$ = 8.5 Hz, 2H), 4.63 - 4.61 (m, 1H), 3.88 - 3.86 (m, 2H), 3.75 - 3.73 (m, 4H), 3.66 - 3.64 (m, 1H), 3.57 - 3.53 (m, 1H), 3.51 - 3.47 (m, 1H), 3.16 - 3.09 (m, 2H), 2.89 (tt, J= 12.1 Hz, *J* = 3.5 Hz, 1H), 2.27 - 2.24 (m, 5H), 2.18 - 2.13 (m, 5H), 1.68 - 1.58 (m, 2H), 1.56 - 1.50 (m, 2H).

**Example 98.**

Synthesis of 4-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)butanoic acid hydrochloride **(98).**

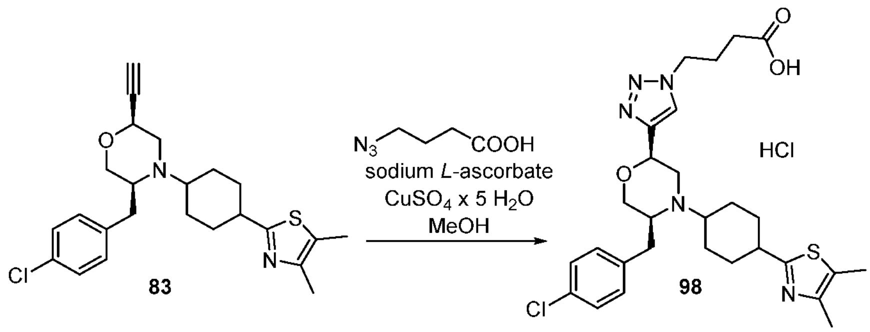


To a solution of 83 (35 mg; 0.082 mmol) in MeOH (1.5 mL) $CuSO_4$ x 5 $H_2O$ (11 mg; 0.041 mmol) was added and after 5 minutes sodium L-ascorbate (17 mg; 0.082 mmol) was added in one portion and the mixture was stirred at room temperature for 25 minutes. Then to this mixture a solution of 4-azidobutanoic acid (21 mg; 0.164 mmol) in MeOH (0.5 mL) was added and the mixture was stirred at room temperature for 2.5 hours. The reaction progress was monitored by LC-MS. When analysis indicated completion of the reaction, the solvent was evaporated *in vacuo* and the residue was dissolved in MeCN/water with $HCl_{aq}$. An insoluble acid was filtered off and the filtrate was taken for the preparative reversed-phase column chromatography (C-18, water + 0.3‰ HCl (36%)/MeCN, 95:5 to 20:80, 30 min, 19 mL/min). The title compound (98) was obtained as a single isomer as a hydrochloride salt in 49% yield (24 mg; 0.040 mmol).
ESI-MS m/z for $C_{28}H_{37}ClN_5O_3S$ found 558.4/560.4 $[M+H]^+$; Rt = 1.03 min (Method A); $^1$H NMR (700 MHz, DMSO-$d_6$ + $D_2O$, 348 K) δ 8.21 (s, 1H), 7.38 (d, $J_{AA'}$ = 8.6 Hz, 2H), 7.34 (d, $J_{BB'}$ = 8.6 Hz, 2H), 5.08 - 5.06 (m, 1H), 4.41 (t, *J* = 7.1 Hz, 2H), 3.95 - 3.93 (m, 1H), 3.86 - 3.84 (m, 1H), 3.72 - 3.68 (m, 2H), 3.62 - 3.56 (m, 2H), 3.25 (dd, *J* = 13.9 Hz, *J* = 10.7 Hz, 1H), 3.19 (dd, *J* = 13.9 Hz, *J* = 4.3 Hz, 1H), 2.88 (tt, *J* = 12.1 Hz, *J* = 3.6 Hz, 1H), 2.35 - 2.26 (m, 7H), 2.19 - 2.17 (m, 5H), 2.10 - 2.06 (m, 2H), 1.68 - 1.52 (m, 4H).

**Example 99.**

Synthesis of (2R,5S)-5-(4-chlorobenzyl)-4-(4-(4,5-dimethylthiazol-2-yl)cyclohexyl)-N-hydroxymorpholine-2-carboxamide hydrochloride **(99).**

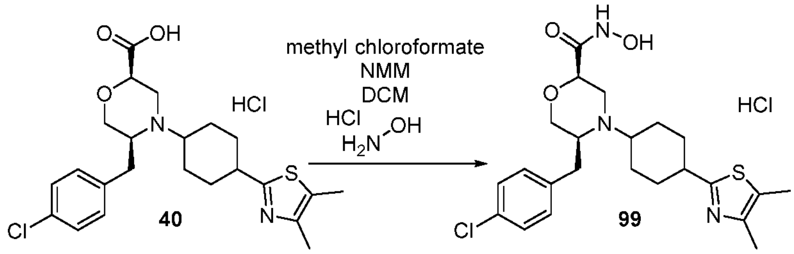

40 99

The title compound **(99)** was obtained as a hydrochloride salt as a single isomer from **40** (43 mg; 0.089 mmol) and from hydroxylamine hydrochloride (8 mg; 0.107 mmol) according to the General Procedure **V** in 13% yield (6 mg; 0.012 mmol). The crude product was purified by preparative reversed-phase column chromatography (column: Cosmosil Cholester 20 x 250 mm, water + 0.3‰ HCl (36%)/MeCN, 98:2 to 30:70, 30 min, 18 mL/min).
ESI-MS m/z for $C_{23}H_{31}ClN_3O_3S$ found 464.3/466.3 $[M+H]^+$; Rt = 0.96 min (Method A); $^1H$ NMR (700 MHz, DMSO-$d_6$ + $D_2O$, 348 K) δ 7.38 (d, $J_{AA'}$ = 8.6 Hz, 2H), 7.34 (d, $J_{BB'}$ = 8.6 Hz, 2H), 4.43 - 4.42 (m, 1H), 3.86 - 3.84 (m, 1H), 3.79 - 3.77 (m, 1H), 3.68 (dd, $J$ = 13.3 Hz, $J$ = 2.3 Hz, 1H), 3.57 - 3.54 (m, 2H), 3.40 - 3.37 (m, 1H), 3.16 - 3.10 (m, 2H), 2.94 (tt, $J$ = 12.2 Hz, $J$ = 3.6 Hz, 1H), 2.29 - 2.16 (m, 10H), 1.75 - 1.65 (m, 2H), 1.59 - 1.52 (m, 2H).

**Example 100.**

Synthesis of (2*R*,5*S*)-5-(4-chlorobenzyl)-N-(1,5-dimethyl-1*H*-pyrazol-3-yl)-4-(4-(4,5-dimethylthiazol-2-yl)cyclohexyl) morpholine-2-carboxamide hydrochloride **(100).**

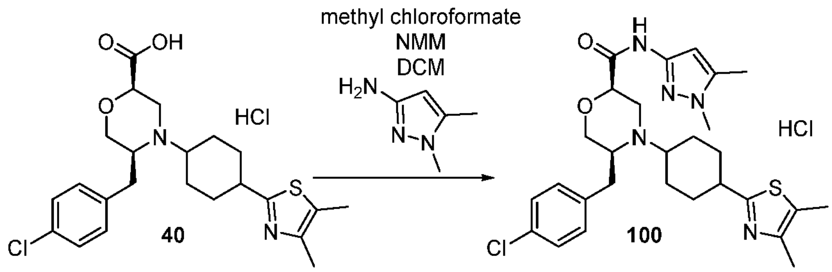

40 100

The title compound **(100)** was obtained as a hydrochloride salt as a single isomer from **40** (43 mg; 0.089 mmol) and from 1,5-dimethyl-1*H*-pyrazol-3-amine (12 mg; 0.110 mmol) according to the General Procedure **V** in 45% yield (23 mg; 0.040 mmol). The crude product was purified by preparative reversed-phase column chromatography (column: Cosmosil Cholester 20 x 250 mm, water + 0.3‰ HCl (36%)/MeCN, 98:2 to 10:90, 30 min, 18 mL/min).
ESI-MS m/z for $C_{28}H_{37}ClN_5O_2S$ found 542.5/544.5 $[M+H]^+$; Rt = 1.17 min (Method A); $^1H$ NMR (700 MHz, DMSO-$d_6$ + $D_2O$, 348 K) δ 7.38 (d, $J_{AA'}$ = 8.6 Hz, 2H), 7.35 (d, $J_{BB'}$ = 8.6 Hz, 2H), 6.25 (s, 1H), 4.51 - 4.49 (m, 1H), 3.83 (dd, $J$ = 13.1 Hz, $J$ = 2.1 Hz, 1H), 3.79 - 3.74 (m, 2H), 3.61 - 3.60 (m, 4H), 3.54 - 3.50 (m, 2H), 3.17 - 3.12 (m, 2H), 2.87 (tt, $J$ = 12.2 Hz, $J$ = 3.7 Hz, 1H), 2.28 - 2.14 (m, 13H), 1.67 - 1.59 (m, 2H), 1.58 - 1.50 (m, 2H).

**Example 101.**

Synthesis of 5-(4-((2*R*,5*S*)-5-(4-chlorobenzyl)-4-(4-(4,5-dimethylthiazol-2-yl)cyclohexyl)-morpholin-2-yl)-1*H*-1,2,3-triazol-1-yl)pentanoic acid hydrochloride **(101).**

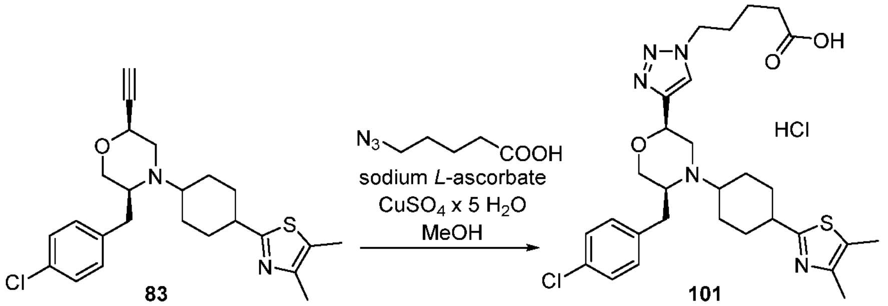


To a solution of **83** (35 mg; 0.082 mmol) in MeOH (3 mL) $CuSO_4$ x 5 $H_2O$ (11 mg; 0.041 mmol) was added and after 5 minutes sodium L-ascorbate (17 mg; 0.082 mmol) was added in one portion and the mixture was stirred at room temperature for 25 minutes. Then to this mixture 5-azidopentanoic acid (24 mg; 0.164 mmol) was added and the mixture was stirred at room temperature overnight. The reaction progress was monitored by LC-MS. When analysis indicated completion of the reaction, the solvent was evaporated *in vacuo* and the residue was dissolved in MeCN/water with $HCl_{aq}$. An insoluble acid was filtered off and the filtrate was taken for the preparative reversed-phase column chromatography (C-18, water + 0.3‰ HCl (36%)/MeCN, 95:5 to 20:80, 30 min, 20 mL/min). The title compound **(101)** was obtained as a single isomer as a hydrochloride salt in 30% yield (15 mg; 0.025 mmol).
ESI-MS m/z for $C_{29}H_{39}ClN_5O_3S$ found 572.4/574.4 $[M+H]^+$; $R_t$ = 1.07 min (Method A); $^1H$ NMR (700 MHz, DMSO-$d_6$ + $D_2O$, 348 K) δ 8.19 (s, 1H), 7.38 (d, $J_{AA'}$ = 8.6 Hz, 2H), 7.33 (d, $J_{BB'}$ = 8.6 Hz, 2H), 5.07 - 5.05 (m, 1H), 4.38 (t, *J* = 7.0 Hz, 2H), 3.94 - 3.92 (m, 1H), 3.85 - 3.83 (m, 1H), 3.72 - 3.67 (m, 2H), 3.62 - 3.56 (m, 2H), 3.24 (dd, *J* = 13.9 Hz, *J* = 10.7 Hz, 1H), 3.20 - 3.18 (m, 1H), 2.88 (tt, *J* = 12.0 Hz, *J* = 3.7 Hz, 1H), 2.35 - 2.30 (m, 2H), 2.26 - 2.24 (m, 5H), 2.19 - 2.17 (m, 5H), 1.89 - 1.85 (m, 2H), 1.67 - 1.49 (m, 6H).

**Example 102.**

Synthesis of (2*R*,5*S*)-5-(4-chlorobenzyl)-*N*-(1,5-dimethyl-1*H*-pyrazol-3-yl)-4-(4-(1,5-dimethyl-1*H*-pyrazol-3-yl)cyclohexyl)morpholine-2-carboxamide hydrochloride **(102).**

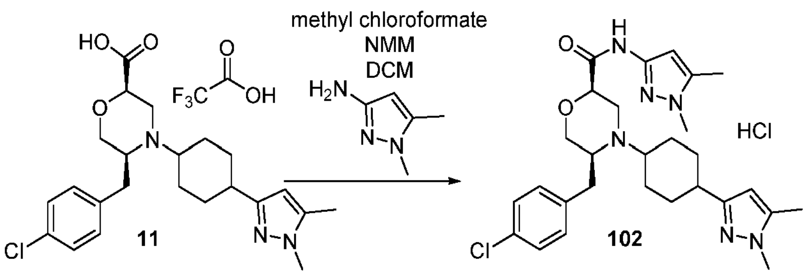


The title compound **(102)** was obtained as a hydrochloride salt as a single isomer from **11** (35 mg; 0.064 mmol) and from 1,5-dimethyl-1*H*-pyrazol-3-amine (9 mg; 0.080 mmol) according to the General Procedure **V** in 39% yield (16 mg; 0.025 mmol). The crude product was purified by preparative reversed-phase column chromatography (column: Cosmosil Cholester 20 x 250 mm, water + 0.3‰ HCl (36%)/MeCN, 98:2 to 30:70, 30 min, 18 mL/min).
ESI-MS m/z for $C_{28}H_{38}ClN_6O_2S$ found 525.5/527.5 $[M+H]^+$; $R_t$ = 1.04 min (Method A); $^1H$ NMR (700 MHz, DMSO-$d_6$ + $D_2O$, 348 K) δ 7.38 (d, $J_{AA'}$ = 8.5 Hz, 2H), 7.35 (d, $J_{BB'}$ = 8.5 Hz, 2H), 6.24 (s, 1H), 5.82 (s, 1H), 4.51 - 4.50 (m, 1H), 3.85 - 3.82 (m, 1H), 3.80 - 3.74 (m, 2H), 3.62 - 3.60 (m, 7H), 3.51 - 3.46 (m, 2H), 3.17 - 3.11 (m, 2H), 2.47 (tt, *J* = 12.0 Hz, *J* = 3.7 Hz, 1H), 2.25 - 2.16 (m, 8H), 2.06 - 2.01 (m, 2H), 1.63 - 1.54 (m, 2H), 1.47 - 1.38 (m, 2H).

**Example 103.**

Synthesis of *N*-((2*R*,5*S*)-5-(4-chlorobenzyl)-4-(4-(4,5-dimethylthiazol-2-yl)cyclohexyl)-morpholine-2-carbonyl)-*N*-methylglycine hydrochloride **(103).**

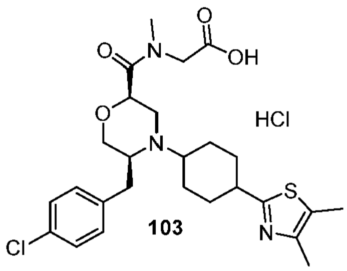

103

Step 1.

Synthesis of methyl *N*-((2*R*,5*S*)-5-(4-chlorobenzyl)-4-(4-(4,5-dimethylthiazol-2-yl)cyclohexyl)morpholine-2-carbonyl)-*N*-methylglycinate hydrochloride **(103a).**

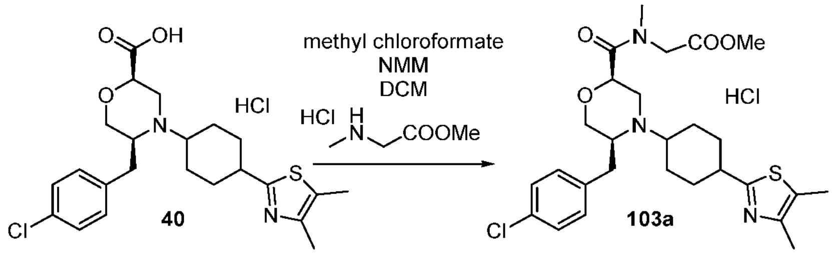


The title compound **(103a)** was obtained as a hydrochloride salt from **40** (35 mg; 0.072 mmol) and from methyl methylglycinate hydrochloride (13 mg; 0.094 mmol) according to the General Procedure **V.** The crude product was purified by preparative reversed-phase column chromatography (C-18, water + 0.3‰ HCl (36%)/MeCN, 98:2 to 10:90, 30 min, 18 mL/min) and was taken to the next step.
ESI-MS m/z for $C_{27}H_{37}ClN_3O_4S$ found 534.2/536.1 $[M+H]^+$; Rt = 1.10 min (Method A)

Step 2.

Synthesis of *N*-((2*R*,5*S*)-5-(4-chlorobenzyl)-4-(4-(4,5-dimethylthiazol-2-yl)cyclohexyl)-morpholine-2-carbonyl)-*N*-methylglycine hydrochloride **(103).**

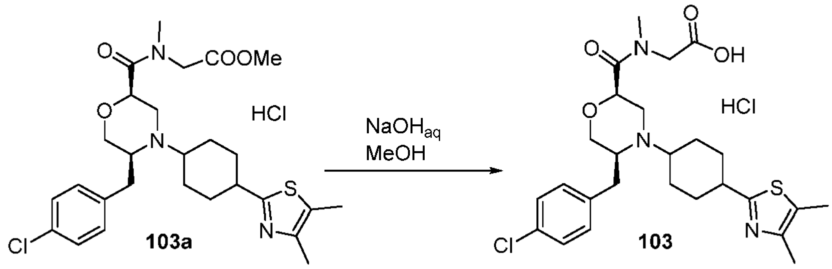


To the aqueous solution of **103a** (the crude product) in MeOH (2 mL) 4 M NaOH (0.5 mL) was added and whole was stirred at room temperature for 2 hours. The reaction progress was monitored by LC-MS. When analysis indicated completion of the reaction, the solvent was evaporated *in vacuo* and the residue was purified by preparative reversed-phase column chromatography (C-18, water + 0.3‰ HCl (36%)/MeCN, 98:2 to 10:90, 30 min, 18 mL/min). The title compound **(103)** was obtained as a single isomer as a hydrochloride salt in 19% yield (per two steps)(8 mg; 0.014 mmol).
ESI-MS m/z for $C_{26}H_{35}ClN_3O_4S$ found 520.4/522.4 $[M+H]^+$; Rt = 1.04 min (Method A); $^1H$ NMR (700 MHz, DMSO-$d_6$ + $D_2O$) δ 7.38 (d, $J_{AA'}$ = 8.2 Hz, 2H), 7.31 - 7.29 (m, 2H), 4.81 - 4.74 (m, 1H), 4.20 - 4.16 (m, 1H), 4.10 - 3.99 (m, 1H), 3.87 - 3.80 (m, 2H), 3.74 - 3.54 (m, 4H), 3.23 - 3.21 (m, 1H), [3.09 (s, 1.8H), 2.92 (s, 1.2H), rotamers, 3H], 3.02 - 2.97 (m, 1H), 2.87 (tt, *J* = 12.5 Hz, *J* = 3.3 Hz, 1H), 2.26 - 2.11 (m, 10H), 1.72 - 1.68 (m, 1H), 1.64 - 1.52 (m, 2H), 1.47 - 1.44 (m, 1H).

**Example 104.**

Synthesis of (2*R*,5*S*)-4-(4-(1-methyl-1*H*-1,2,4-triazol-3-yl)cyclohexyl)-2-((methylsulfonyl)-methyl)-5-(4-(trifluoromethyl) benzyl)morpholine 2,2,2-trifluoroacetate **(104).**

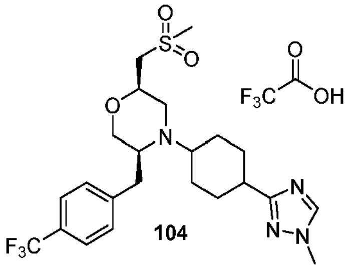

**104**

Step 1.

Synthesis of (*S*)-2-amino-3-(4-(trifluoromethyl)phenyl)propan-1-ol **(104a).**

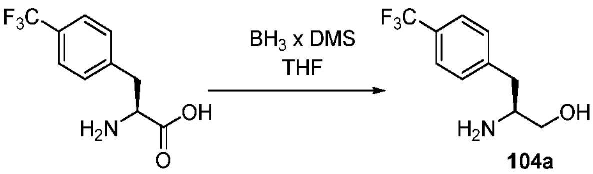


**104a**

The title compound **(104a)** was obtained from optically pure (*S*)-2-amino-3-(4-(trifluoromethyl)phenyl)propanoic acid (7 g; 30 mmol) according to the General Procedure **Ia** in 99% yield (6.5 g; 29.7 mmol).
ESI-MS m/z for $C_{10}H_{13}F_3NO$ found 220.1 $[M+H]^+$; Rt = 1.17 min (Method A)

Step 2.

Synthesis of 2-bromo-3-(*tert*-butoxy)-*N*-((*S*)-1-hydroxy-3-(4-(trifluoromethyl)phenyl)propan-2-yl)propanamide **(104b).**

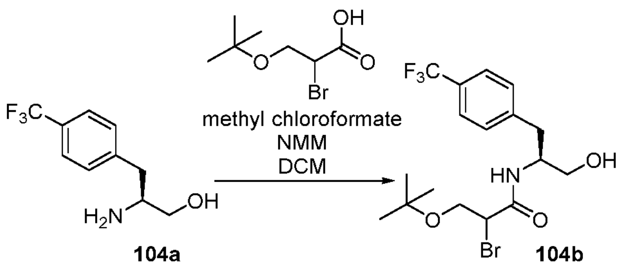


**104a** **104b**

The title compound **(104b)** was obtained from **104a** (7.3 g; 33.3 mmol) according to the General Procedure **V** in 89% yield (12.66 g; 29.78 mmol).
ESI-MS m/z for $C_{17}H_{24}BrF_3NO_3$ found 426.1 $[M+H]^+$; Rt = 1.43 min (Method A)

Step 3.

Synthesis of (2*S*,5*S*)-2-(*tert*-butoxymethyl)-5-(4-(trifluoromethyl)benzyl)morpholin-3-one **(104c).**

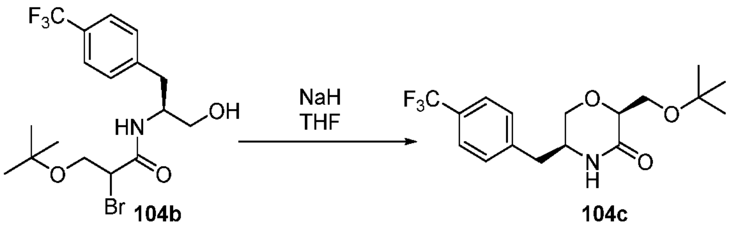


104b 104c

The title compound **(104c)** was obtained from **104b** (12.66 g; 29.78 mmol) according to the General Procedure **II** in 99% yield (10.18 g; 29.48 mmol).
ESI-MS m/z for $C_{17}H_{23}F_3NO_3$ found 346.2 $[M+H]^+$; Rt = 1.43 min (Method A)

Step 4.

Synthesis of ((2*R*,5*S*)-5-(4-(trifluoromethyl)benzyl)morpholin-2-yl)methanol **(104d).**

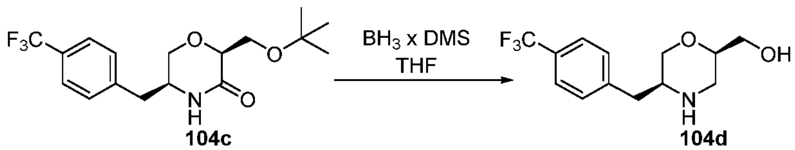


104c 104d

The title compound **(104d)** was obtained from **104c** (10.18 g; 29.48 mmol) according to the General Procedure **Ib** in 99% yield (8.03 g; 29.19 mmol).
ESI-MS m/z for $C_{13}H_{17}F_3NO_2$ found 276.1 $[M+H]^+$; $R_t$ = 0.55 min (Method A)

Step 5.

Synthesis of *tert-butyl* (2*R*,5*S*)-2-(hydroxymethyl)-5-(4-(trifluoromethyl)benzyl)morpholine-4-carboxylate **(104e).**

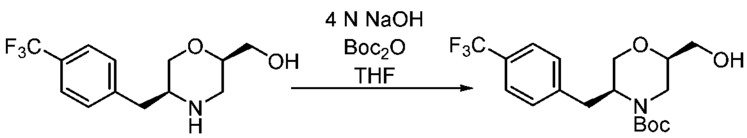


104d 104e

To a solution of an amino alcohol **104d** (8.03 g; 29.19 mmol) in 4 N NaOH (110 mL) the solution of di-tert-butyl dicarbonate ($Boc_2O$) (9.45 g; 43.3 mmol) in THF (50 mL) was added and the reaction mixture was stirred at room temperature overnight. The reaction progress was monitored by LC-MS. When analysis indicated completion of the reaction, the phases were separated and an aqueous one was extracted with $Et_2O$ (3 x 100 mL). The combined organic solutions were dried over anhydrous $Na_2SO_4$, filtered and concentrated *in vacuo* and the residue was purified by silica-gel column chromatography (hexane/AcOEt, 1:5 to 2.5:1, *v*/*v*). Compound **104e** was obtained as a colorless oil in 99% yield (10.84 g; 28.9 mmol).
ESI-MS m/z for $C_{18}H_{25}F_3NO_4$ found 376.2 $[M+H]^+$; $R_t$ = 1.45 min (Method A)

Step 6.

Synthesis of *tert*-butyl (2*R*,5*S*)-2-(((methylsulfonyl)oxy)methyl)-5-(4-(trifluoromethyl)benzyl)-morpholine-4-carboxylate **(104f).**

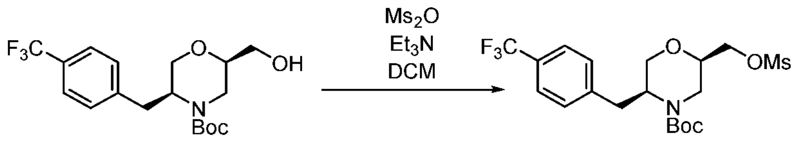


**104e** **104f**

The title compound **(104f)** was obtained from **104e** (2.1 g; 5.59 mmol) according to the General Procedure **VIII** in 99% yield (2.51 g; 5.53 mmol).
ESI-MS m/z for $C_{19}H_{27}F_3NO_6S$ found 454.1 $[M+H]^+$; Rt = 1.70 min (Method A)

Step 7.

Synthesis of *tert*-butyl (2*R*,5*S*)-2-((methylthio)methyl)-5-(4-(trifluoromethyl)benzyl)morpholine-4-carboxylate **(104g).**

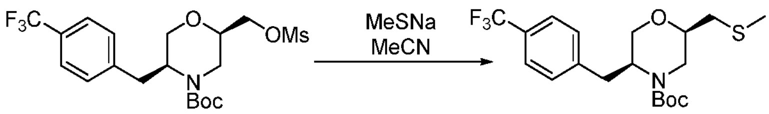


**104f** **104g**

To a solution of **104f** (2.51 g; 5.53 mmol) in acetonitrile (20 mL) MeSNa (1 g; 14 mmol) was added and the reaction mixture was stirred at 80 °C overnight. The reaction progress was monitored by LC-MS analysis. After analytical control indicated completion of the reaction, the mixture was dissolved in $Et_2O$ and then washed with 1 M NaOH and brine, extracted with $Et_2O$ (3 x 70 mL), dried over anhydrous $Na_2SO_4$, filtered and concentrated *in vacuo* and the crude product was used to the next step without additional purification. Compound **104g** was obtained in 99% yield (2.22 g; 5.47 mmol).
ESI-MS m/z for $C_{19}H_{27}F_3NO_3S$ found 406.2 $[M+H]^+$; Rt = 1.48 min (Method A)

Step 8.

Synthesis of *tert*-butyl (2*R*,5*S*)-2-((methylsulfonyl)methyl)-5-(4-(trifluoromethyl)benzyl)-morpholine-4-carboxylate **(104h).**

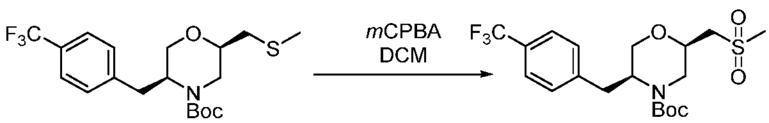


**104g** **104h**

To the pre-cooled (0 °C) solution of **104g** (2.22 g; 5.47 mmol) in DCM (40 mL) mCPBA (4.13 g; 16.77 mmol) was added in several portions. The reaction was stirred at room temperature for 3 hours. The reaction progress was monitored by LC-MS analysis. After analytical control indicated completion of the reaction, the reaction was washed with 10% $Na_2S_2O_3$, saturated $NaHCO_3$, brine, dried over anhydrous $Na_2SO_4$, filtered and concentrated *in vacuo* and the crude product was used to the next step without additional purification. Compound **104h** was obtained in 99% yield (2.37 g; 5.42 mmol).
ESI-MS m/z for $C_{19}H_{27}F_3NO_5S$ found 438.2 $[M+H]^+$; Rt = 1.55 min (Method A)

Step 9.

Synthesis of (2R,5S)-2-((methylsulfonyl)methyl)-5-(4-(trifluoromethyl)benzyl)morpholine (104i).

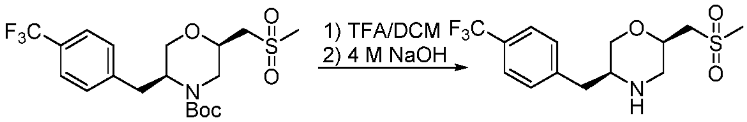


104h 104i

The title compound (104i) was obtained as a free base in 86% yield (1.57 g; 4.66 mmol) from **104h** (2.37 g; 5.42 mmol) according to the General Procedure **IVb** and then after basic (4 M NaOH) extraction with DCM.

Step 10.

Synthesis of 1-methyl-3-(1,4-dioxaspiro[4.5]dec-7-en-8-yl)-1*H*-1,2,4-triazole **(104j).**

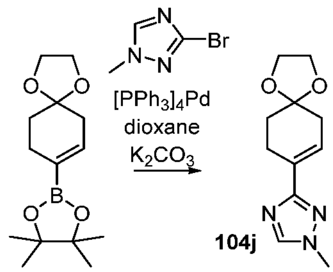


104j

The solution of 4,4,5,5-tetramethyl-2-(1,4-dioxaspiro[4.5]dec-7-en-8-yl)-1,3,2-dioxaborolane (1.02 g; 3.80 mmol), 3-bromo-1-methyl-1*H*-1,2,4-triazole (650 mg, 4.00 mmol), $[PPh_3]_4Pd$ (130 mg; 0.11 mmol) in mixture of dioxane (16 mL) and 2 M aqueous solution of $K_2CO_3$ (4 mL) was heated at 100 °C for 24 hours under an argon atmosphere. LC-MS showed complete consumption of the starting material. Then AcOEt (40 mL) was added and this mixture was washed with water and brine. An aqueous layer was extracted with chloroform. The combined organic layers were concentrated *in vacuo* and the residue was purified by flash column chromatography on silica (hexane/AcOEt, 1:1, *v*/*v*, then: AcOEt/-MeOH, 140:1 to 15:1, *v*/*v*). Compound **104j** was obtained as pale yellow oil in 69% yield (580 mg; 2.62 mmol).
ESI-MS m/z for $C_{11}H_{16}N_3O_2$ found 222.0 $[M+H]^+$; Rt = 0.56 min (Method A); $^1H$ NMR (700 MHz, DMSO-$d_6$ + $D_2O$, 348 K) δ 8.27 - 8.23 (m, 1H), 6.52 - 6.48 (m, 1H), 3.95 - 3.92 (m, 4H), 3.83 (s, 3H), 2.62 - 2.58 (m, 2H), 2.39 - 2.36 (m, 2H), 1.82 - 1.76 (m, 2H).

Step 11.

Synthesis of 1-methyl-3-(1,4-dioxaspiro[4.5]decan-8-yl)-1*H*-1,2,4-triazole **(104k).**

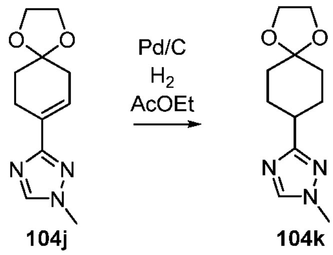


104j 104k

To the solution of **104j** (570 mg; 2.57 mmol) in AcOEt (15 mL) 10% Pd/C (5 mol%; 137 mg, 0.13 mmol) was added and the reaction mixture was conducted under hydrogen atmosphere at room temperature overnight. The reaction progress was monitored by LC-MS. When analysis indicated completion of the reaction, Pd/C was filtered off through the Celite pad and the solvents were evaporated *in vacuo.* The crude product was used to the next step without additional purification.
ESI-MS m/z for $C_{11}H_{18}N_3O_2$ found 224.2 $[M+H]^+$; Rt = 0.45 min (Method A)

Step 12.

Synthesis of 4-(1-methyl-1*H*-1,2,4-triazol-3-yl)cyclohexan-1-one **(1041).**

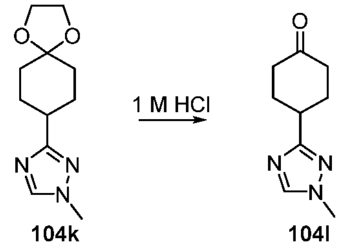


**104k** **104l**

The solution of **104k** (the crude product) in 1 M HCl (15 mL) was stirred at room temperature for overnight. The reaction progress was monitored by LC-MS. When analysis indicated completion of the reaction, to this mixture an aqueous solution of NaOH (4 M) was added to pH 13 and then extracted with $CHCl_3$ (2 x). The combined organic solutions were dried over anhydrous $MgSO_4$, filtered and concentrated *in vacuo.* The crude product was used to the next step without additional purification. Compound **1041** was obtained in 92% yield (per two steps)(424 mg; 2.37 mmol).
ESI-MS m/z for $C_9H_{14}N_3O$ found 180.2 $[M+H]^+$; $R_t$ = 0.27 min (Method A); $^1H$ NMR (700 MHz, DMSO-$d_6$ + $D_2O$, 300 K) δ 8.33 (s, 1H), 3.78 (s, 3H), 3.19 - 3.12 (m, 1H), 2.49 - 2.45 (m, 2H), 2.31 - 2.26 (m, 2H), 2.24 - 2.16 (m, 2H), 1.92 - 1.84 (m, 2H).

Step 13.

Synthesis of (2*R*,5*S*)-4-(4-(1-methyl-1H-1,2,4-triazol-3-yl)cyclohexyl)-2-((methylsulfonyl)-methyl)-5-(4-(trifluoromethyl) benzyl)morpholine 2,2,2-trifluoroacetate (**104**).

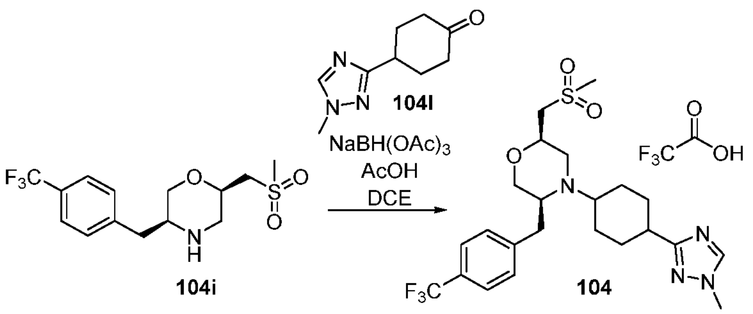


**104i** **104**

The title compound (**104**) was obtained as a TFA salt as a single isomer from **104i** (135 mg; 0.40 mmol) and ketone **104l** (72 mg; 0.40 mmol) according to the General Procedure **VII** in 15% yield (35 mg; 0.06 mmol). The crude product was purified by silica-gel column chromatography (DCM/MeOH, 200:1 to 20:1, v/v) and then by preparative reversed-phase column chromatography (column: Cosmosil Cholester 20 x 250 mm, water/MeCN + 1‰ TFA, 99:1 to 50:50, 30 min, 18 mL/min).
ESI-MS m/z for $C_{23}H_{32}F_3N_4O_3S$ found 501.3 $[M+H]^+$; Rt = 0.83 min (Method A); $^1H$ NMR (700 MHz, DMSO-$d_6$ + $D_2O$, 348 K) δ 8.28 - 8.24 (m, 1H), 7.70 - 7.66 (m, 2H), 7.55 - 7.52 (m, 2H), 4.34 - 4.27 (m, 1H), 3.90 - 3.85 (m, 1H), 3.84 - 3.80 (m, 1H), 3.78 (s, 3H), 3.71 - 3.66 (m, 1H), 3.63 - 3.57 (m, 2H), 3.54 - 3.48 (m, 1H), 3.45 - 3.44 (m, 1H), 3.36 - 3.29 (m, 2H), 3.26 - 3.21 (m, 1H), 3.04 (s, 3H), 2.70 - 2.64 (m, 1H), 2.33 - 2.25 (m, 2H), 2.20 - 2.11 (m, 2H), 1.62 - 1.50 (m, 4H).

**Example 105.**

Synthesis of (2*R*,5*S*)-5-(4-chlorobenzyl)-4-(4-(1-methyl-1*H*-imidazol-4-yl)cyclohexyl)-2-((methylsulfonyl)methyl)morpholine 2,2,2-trifluoroacetate (**105**).

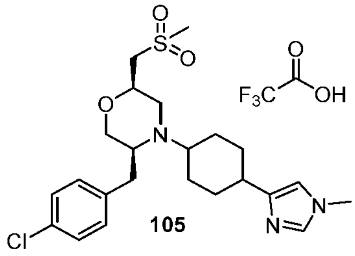

105

The title compound (**105**) was obtained as a TFA salt as a single isomer in 1% overall yield in a similar way to Example 15 with the exception that, in the first step of the synthesis, 4-bromo-1-methyl-1*H*-imidazole was used instead of 3-bromo-1,5-dimethyl-1*H*-1,2,4-triazole.
ESI-MS m/z for $C_{23}H_{33}ClN_3O_3S$ found 466.2/468.2 $[M+H]^+$; Rt = 0.96 min (Method A); $^1H$ NMR (700 MHz, Methanol-*d₄*) δ 8.82 - 8.80 (m, 1H), 7.41 - 7.38 (m, 2H), 7.38 - 7.36 (m, 1H), 7.35 - 7.31 (m, 2H), 4.41 - 4.32 (m, 1H), 3.95 - 3.91 (m, 1H), 3.90 (d, *J* = 0.4 Hz, 3H), 3.87 - 3.80 (m, 2H), 3.77 - 3.71 (m, 1H), 3.66 - 3.55 (m, 2H), 3.50 - 3.40 (m, 2H), 3.37 - 3.32 (m, 1H), 3.20 - 3.16 (m, 1H), 3.10 (s, 3H), 2.87 - 2.77 (m, 1H), 2.54 - 2.45 (m, 2H), 2.36 - 2.26 (m, 2H), 1.76 - 1.55 (m, 4H).

**Example 106.**

Synthesis of (2*R*,5*S*)-4-(4-(1-methyl-1*H*-1,2,4-triazol-3-yl)cyclohexyl)-5-(4-(trifluoromethyl)-benzyl)morpholine-2-carboxylic acid 2,2,2-trifluoroacetate (**106**).

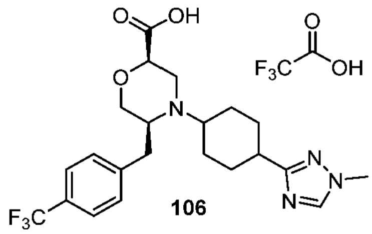

106

The title compound (**106**) was obtained as a TFA salt as a single isomer in 2% overall yield in a similar way to Example 65 with the exception that, in the first step of the synthesis, the compound **104e** was used instead of the compound **63e** and in the third step of the synthesis, ketone **104l** was used instead of ketone **2d.** The crude product was purified by preparative reversed-phase column chromatography (C-18, water/MeCN + 1‰ TFA, 99:1 to 40:60, 30 min, 20 mL/min).
ESI-MS m/z for $C_{22}H_{28}F_3N_4O_3$ found 453.3 $[M+H]^+$; Rt = 0.90 min (Method A); $^1H$ NMR (700 MHz, $D_2O$) δ 9.22 (s, 1H), 7.82 - 7.78 (m, 2H), 7.62 - 7.58 (m, 2H), 4.69 - 4.55 (m, 1H), 4.12 - 4.08 (m, 1H), 4.07 (s, 3H), 3.99 - 3.81 (m, 3H), 3.79 - 3.51 (m, 2H), 3.43 - 3.31 (m, 2H), 3.09 - 3.02 (m, 1H), 2.62 - 2.30 (m, 4H), 1.84 - 1.61 (m, 4H).

**Example 107.**

Synthesis of (2*S*,5*S*)-5-(4-chlorobenzyl)-2-methyl-4-(4-(5-methyl-1*H*-pyrazol-3-yl)cyclohexyl)-morpholine 2,2,2-trifluoroacetate (**107**).

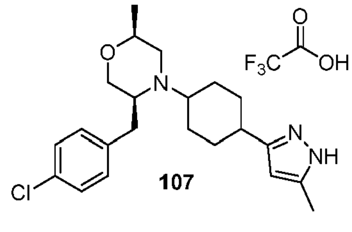

107

The title compound (**107**) was obtained as a TFA salt as a single isomer in 9% overall yield in a similar way to

Example 2 with the exception that, in the third step of the synthesis, methylhydrazine was used instead of hydrazine monohydrate. The crude product was purified by flash column chromatography on silica (hexane/AcOEt, 1:1 to 0:1, *v*/*v*, then: AcOEt/MeOH, 10:1, *v*/*v*) and then purified by preparative reversed-phase column chromatography (C-18, water/-MeCN + 1‰ TFA, 90:10 to 10:90, 30 min, 20 mL/min).
ESI-MS m/z for $C_{22}H_{31}ClN_3O$ found 388.1/390.1 $[M+H]^+$; Rt = 0.95 min (Method A); $^1H$ NMR (700 MHz, DMSO-$d_6$ + $D_2O$, 348 K) δ 7.38 (d, $J_{AA'}$ = 8.4 Hz, 2H), 7.31 (d, $J_{BB'}$ = 8.4 Hz, 2H), 5.84 (s, 1H), 3.85 - 3.83 (m, 1H), 3.73 - 3.69 (m, 2H), 3.60 - 3.59 (m, 1H), 3.47 - 3.44 (m, 2H), 3.17 - 3.14 (m, 1H), 3.08 (dd, *J* = 13.7 Hz, *J* = 3.8 Hz, 1H), 3.03 - 3.01 (m, 1H), 2.57 - 2.53 (m, 1H), 2.32 - 2.29 (m, 1H), 2.27 - 2.24 (m, 1H), 2.16 (s, 3H), 2.09 - 2.05 (m, 2H), 1.56 - 1.42 (m, 4H), 1.23 (d, *J* = 6.2 Hz, 3H).

**Example 108.**

Synthesis of (2*R*,5*S*)-5-(4-chlorobenzyl)-4-(4-(1-methyl-1*H*-1,2,4-triazol-3-yl)cyclohexyl)-2-((methylsulfonyl)methyl) morpholine 2,2,2-trifluoroacetate (**108**).

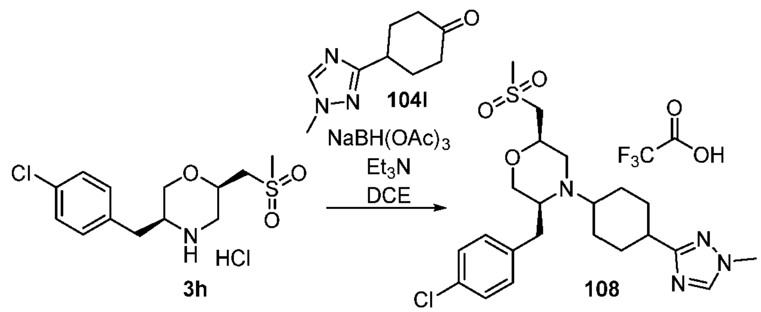


The title compound (**108**) was obtained as a TFA salt as a single isomer from **3h** (288 mg; 0.95 mmol) and ketone **104l** (170 mg; 0.95 mmol) according to the General Procedure **VII** in 14% yield (75 mg; 0.13 mmol). The crude product was purified by flash column chromatography on silica (hexane/AcOEt, 100:0 to 0:100, *v*/*v*, then: AcOEt/MeOH, 50:1 to 2:1, *v*/*v*) and then transferred into TFA salt with using TFA/DCM and then purified by preparative reversed-phase column chromatography (C-18, water/MeCN + 1‰ TFA, 95:5 to 20:80, 30 min, 20 mL/min).
ESI-MS m/z for $C_{22}H_{32}ClN_4O_3S$ found 467.1/469.1 $[M+H]^+$; Rt = 0.78 min (Method A); $^1H$ NMR (700 MHz, DMSO-$d_6$ + $D_2O$, 348 K) δ 8.22 (s, 1H), 7.37-7.38 (m, 2H), 7.32 - 7.31 (m, 2H), 4.29 - 4.25 (m, 1H), 3.79 - 3.77 (m, 5H), 3.68 (d, *J* = 12.1 Hz, 1H), 3.59 - 3.54 (m, 2H), 3.48 - 3.45 (m, 1H), 3.41 - 3.39 (m, 1H), 3.29 (t, *J* = 12.0 Hz, 1H), 3.21 - 3.17 (m, 1H), 3.11 (dd, *J* = 13.8 Hz, *J* = 4.3 Hz, 1H), 3.03 (s, 3H), 3.68 - 3.63 (m, 1H), 2.28 - 2.26 (m, 2H), 2.16 - 2.12 (m, 2H), 1.60 - 1.51 (m, 4H).

**Example 109.**

Synthesis of (2*R*,5*S*)-5-(4-chlorobenzyl)-4-(4-(1-methyl-1*H*-1,2,4-triazol-3-yl)cyclohexyl)-morpholine-2-carboxylic acid 2,2,2-trifluoroacetate (**109**).

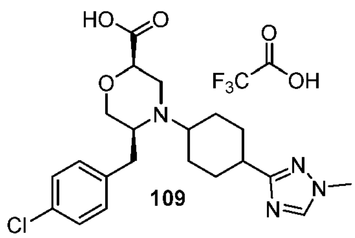

109

The title compound (**109**) was obtained as a TFA salt as a single isomer in 37% overall yield in a similar way to Example 65 with the exception that, in the first step of the synthesis, the compound **3d** was used instead of the compound **63e** and in the third step of the synthesis, ketone **104l** was used instead of ketone **2d.** The crude product was purified by preparative reversed-phase column chromatography (column: Cosmosil Cholester 20 x 250 mm, water/MeCN + 1‰ TFA, 98:2 to 30:70, 30 min, 20 mL/min).
ESI-MS m/z for $C_{21}H_{28}ClN_4O_3$ found 419.0/421.0 $[M+H]^+$; Rt = 0.75 min (Method A); $^1H$ NMR (700 MHz, DMSO-$d_6$ + $D_2O$,

348 K) δ 8.21 (s, 1H), 7.37 (d, $J_{AA'}$ = 8.4 Hz, 2H), 7.31 (d, $J_{BB'}$ = 8.4 Hz, 2H), ), 4.46 - 4.45 (m, 1H), 3.77 (s, 3H), 3.75 - 3.70 (m, 3H), 3.53 - 3.44 (m, 3H), 3.15 - 3.12 (m, 1H), 3.02 (dd, $J$ = 13.9 Hz, $J$ = 9.8 Hz, 1H), 2.65 (tt, $J$ = 11.7 Hz, $J$ = 3.6 Hz, 1H), 2.21 - 2.09 (m, 4H), 1.64 - 1.48 (m, 4H).

**Example 110.**

Synthesis of (2*R*,5*S*)-5-(4-chlorobenzyl)-4-(4-(1-methyl-1*H*-1,2,3-triazol-4-yl)cyclohexyl)-2-((methylsulfonyl)methyl) morpholine 2,2,2-trifluoroacetate (**110**).

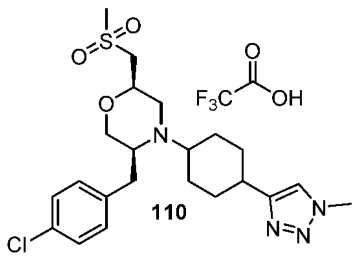

**110**

Step 1.

Synthesis of 4-(1,4-dioxaspiro[4.5]decan-8-yl)-1-((trimethylsilyl)methyl)-1*H*-1,2,3-triazole (**110a**).

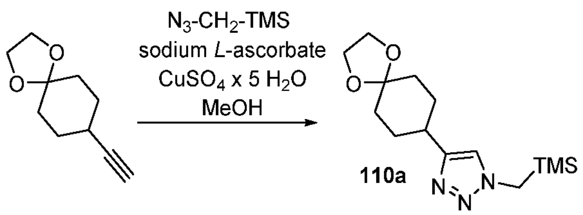


**110a**

To a solution of 8-ethynyl-1,4-dioxaspiro[4.5]decane (458 mg; 2.76 mmol) in MeOH (23 mL) $CuSO_4$ x 5 $H_2O$ (350 mg; 1.40 mmol) was added and after 5 minutes sodium *L*-ascorbate (555 mg; 2.80 mmol) was added in one portion and the mixture was stirred at room temperature for 15 minutes. Then to an yellow mixture trimethylsilylmethyl azide (413 μL; 2.80 mmol) was added and the mixture was stirred at room temperature for 2 hours. The reaction progress was monitored by LC-MS. When analysis indicated completion of the reaction, the green suspension was concentrated *in vacuo* and the residue was taken between water/DCM. An organic layer was washed with brine, dried over anhydrous $MgSO_4$, filtered and concentrated *in vacuo* and the crude product mixture was purified by flash column chromatography on silica (AcOEt/MeOH, 100:1, v/v). Compound **110a** was obtained in 92% yield (750 mg; 2.54 mmol).
ESI-MS m/z for $C_{14}H_{26}N_3O_2Si$ found 296.2 $[M+H]^+$; Rt = 1.28 min (Method A); $^1H$ NMR (700 MHz, DMSO-$d_6$ + $D_2O$) δ 7.64 (s, 1H), 3.90 (s, 2H), 3.87 - 3.82 (m, 4H), 2.71 - 2.66 (m, 1H), 1.93 - 1.88 (m, 2H), 1.71 - 1.66 (m, 2H), 1.64 - 1.54 (m, 4H).

Step 2.

Synthesis of 4-(1-((trimethylsilyl)methyl)-1*H*-1,2,3-triazol-4-yl)cyclohexan-1-one (**110b**).

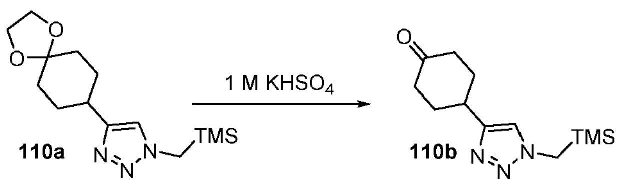


**110a** **110b**

The solution of **110a** (750 mg; 2.54 mmol) and 1 M $KHSO_4$ (10 mL) was stirred at room temperature overnight. The reaction progress was monitored by LC-MS analysis. After analytical control indicated completion of the reaction, to this mixture 4 M NaOH was added to pH 12. Then an aqueous suspension was extracted with DCM (2 x). The combined organic solutions were washed with brine, dried over anhydrous $Na_2SO_4$, filtered and concentrated *in vacuo.* The crude ketone was used to the next step without additional purification. Compound **110b** was obtained in 68% yield (435 mg; 1.73 mmol).
ESI-MS m/z for $C_{12}H_{22}N_3OSi$ found 252.1 $[M+H]^+$; Rt = 1.12 min (Method A)

Step 3.

Synthesis of (2*R*,5*S*)-5-(4-chlorobenzyl)-2-((methylsulfonyl)methyl)-4-(4-(1-((trimethylsilyl)-methyl)-1*H*-1,2,3-triazol-4-yl)cyclohexyl)morpholine (**110c**).

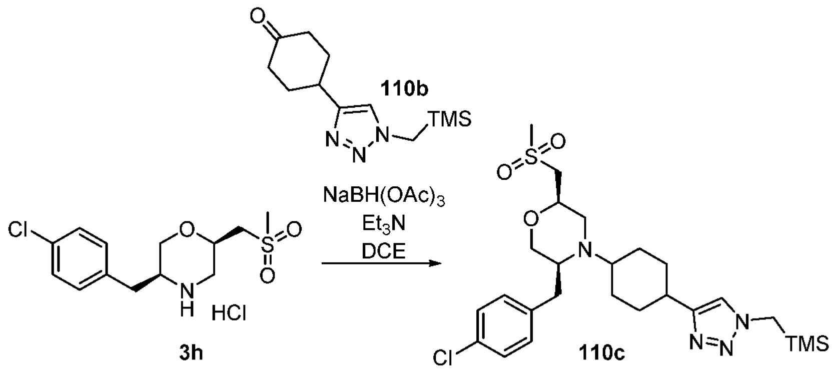

**110b**
**3h**
**110c**

The title compound (**110c**) was obtained from **3h** (299 mg; 0.88 mmol) and ketone **110b** (200 mg; 0.79 mmol) according to the General Procedure **VII** in 30% yield (130 mg; 0.24 mmol). The crude product was purified by flash column chromatography on silica (hexane/AcOEt, 5:4 to 0:100, *v*/*v*, then: AcOEt/MeOH, 50:1, *v*/*v*).
ESI-MS m/z for $C_{25}H_{40}ClN_4O_3SSi$ found 539.1/541.1 $[M+H]^+$; Rt = 1.11 min (Method A)

Step 4.

Synthesis of (2*R*,5*S*)-5-(4-chlorobenzyl)-4-(4-(1-methyl-1*H*-1,2,3-triazol-4-yl)cyclohexyl)-2-((methylsulfonyl)methyl) morpholine 2,2,2-trifluoroacetate (**110**).

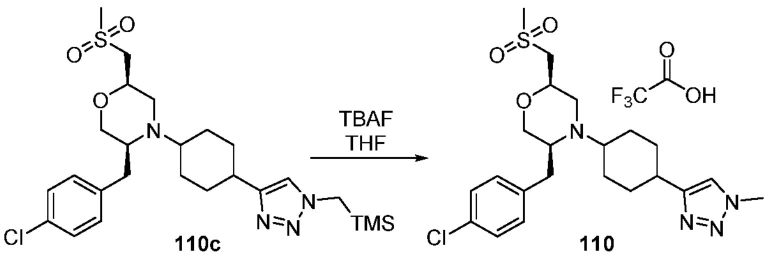

**110c**
**110**

To a solution of **110c** (130 mg; 0.240 mmol) in THF (3 mL) at -70 °C TBAF (1 M in THF; 25 μL; 0.025 mmol) was added and the cooling bath was removed and the mixture was stirred at room temperature overnight. The reaction progress was monitored by LC-MS. When analysis indicated completion of the reaction, the reaction mixture was evaporated *in vacuo* and the crude product was purified by preparative reversed-phase column chromatography (C-18, water/MeCN + 1‰ TFA, 98:2 to 30:70, 30 min, 20 mL/min). The title compound **110** was obtained as a TFA salt as a single isomer in 80% yield (112 mg; 0.193 mmol).
ESI-MS m/z for $C_{22}H_{32}ClN_4O_3S$ found 467.0/469.0 $[M+H]^+$; Rt = 0.81 min (Method A); $^1H$ NMR (700 MHz, DMSO-$d_6$ + $D_2O$, 348 K) δ 7.68 (s, 1H), 7.37 (d, $J_{AA'}$ = 8.4 Hz, 2H), 7.31 (d, $J_{BB'}$ = 8.4 Hz, 2H), 4.28 - 4.24 (m, 1H), 3.97 (s, 3H), 3.78 - 3.74 (m, 2H), 3.67 (d, J = 12.5 Hz, 2H), 3.57 (dd, *J* = 15.0 Hz, *J* = 7.7 Hz, 1H), 3.54 - 3.52 (m, 1H), 3.44 - 3.38 (m, 2H), 3.28 - 2.24

(m, 1H), 3.18 (dd, *J* = 13.7 Hz, *J* = 10.7 Hz, 1H), 3.09 (dd, *J* = 13.7 Hz, *J* = 4.2 Hz, 1H), 3.02 (s, 3H), 2.67 (tt, *J* = 11.6 Hz, *J* = 3.5 Hz, 1H), 2.27 - 2.25 (m, 2H), 2.15 - 2.12 (m, 2H), 1.58 - 1.46 (m, 4H).

**Example 111.**

Synthesis of methyl (2*R*,5*S*)-5-(4-chlorobenzyl)-4-(4-(1-methyl-1*H*-1,2,3-triazol-4-yl)cyclohexyl)morpholine-2-carboxylate 2,2,2-trifluoroacetate (**111**).

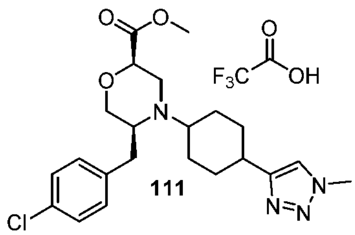

**111**

The title compound (**111**) was obtained as a TFA salt as a single isomer in 9% overall yield in a similar way to Example 110 with the exception that, in the third step of the synthesis, the compound **9b** was used instead of the compound **3h** and in this reaction AcOH was used instead of $Et_3N$. The crude product was purified by flash column chromatography on silica (AcOEt/MeOH, 100:0 to 10:1, v/v) and then purified by preparative reversed-phase column chromatography (column: Cosmosil Cholester 20 x 250 mm, water/MeCN + 1‰ TFA, 90:10 to 20:80, 30 min, 20 mL/min).
ESI-MS m/z for $C_{22}H_{30}ClN_4O_3$ found 433.2/435.2 $[M+H]^+$; Rt = 0.83 min (Method A); $^1H$ NMR (700 MHz, DMSO-$d_6$ + $D_2O$, 348 K) δ 7.66 (s, 1H), 7.36 - 7.35 (m, 2H), 7.29 - 7.28 (m, 2H), 4.49 - 4.47 (m, 1H), 3.96 (s, 3H), 3.74 (s, 3H), 3.68 (d, *J* = 3.3 Hz, 2H), 3.60 - 3.58 (m, 1H), 3.39 - 3.36 (m, 3H), 3.07 (dd, *J* = 13.8 Hz, *J* = 4.2 Hz, 1H), 2.94 (dd, *J* = 13.8 Hz, *J* = 9.9 Hz, 1H), 2.65 (tt, *J* = 12.0 Hz, *J* = 3.4 Hz, 1H), 2.13 - 2.07 (m, 4H), 1.61 - 1.38 (m, 4H).

**Example 112.**

Synthesis of (2*R*,5*S*)-5-(4-chlorobenzyl)-4-(4-(1-methyl-1*H*-1,2,3-triazol-4-yl)cyclohexyl)-morpholine-2-carboxylic acid 2,2,2-trifluoroacetate (**112**).

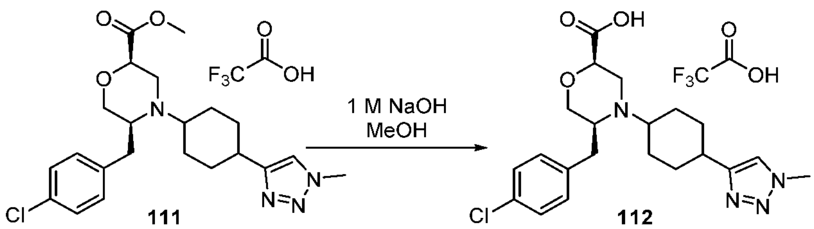


**111** **112**

To a solution of **111** (73 mg; 0.134 mmol) in MeOH (2 mL) 1 M NaOH (1 mL) was added and the mixture was stirred at room temperature for 3 hours. The reaction progress was monitored by LC-MS. When analysis indicated completion of the reaction, methanol was evaporated off and the residue was dissolved in MeCN/water and this mixture was acidified to pH 2 by addition of TFA. The crude product was purified by preparative reversed-phase column chromatography (column: Cosmosil Cholester 20 x 250 mm, water/MeCN + 1‰ TFA, 98:2 to 30:70, 30 min, 20 mL/min). The title compound **112** was obtained as a TFA salt as a single isomer in 42% yield (30 mg; 0.056 mmol).
ESI-MS m/z for $C_{21}H_{28}ClN_4O_3$ found 419.1/421.1 $[M+H]^+$; Rt = 0.83 min (Method A); $^1H$ NMR (700 MHz, DMSO-$d_6$ + $D_2O$) δ 7.72 (s, 1H), 7.31 (d, $J_{AA'}$ = 8.4 Hz, 2H), 7.22 (d, $J_{BB'}$ = 8.4 Hz, 2H), 3.94 (s, 3H), 3.92 (dd, *J* = 8.4 Hz, *J* = 3.3 Hz, 1H), 3.50 (dd, *J* = 11.4 Hz, *J* = 3.9 Hz, 1H), 3.34 (dd, *J* = 11.3 Hz, *J* = 2.5 Hz, 1H), 2.95 - 2.90 (m, 2H), 2.79 - 2.71 (m, 3H), 2.60 - 2.65 (m, 1H), 2.56 (tt, *J* = 12.3 Hz, *J* = 3.6 Hz, 1H), 2.00 - 1.94 (m, 3H), 1.92 - 1.89 (m, 1H), 1.42 - 1.23 (m, 4H).

**Example 113.**

Synthesis of (2*R*,5*S*)-5-(4-chlorobenzyl)-4-(4-(1,5-dimethyl-1*H*-pyrazol-4-yl)cyclohexyl)-2-((methylsulfonyl)methyl) morpholine 2,2,2-trifluoroacetate (**113**).

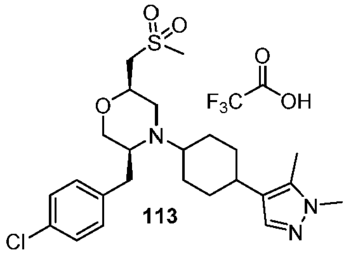

113

The title compound (**113**) was obtained as a TFA salt as a single isomer in 9% overall yield in a similar way to Example 15 with the exception that, in the first step of the synthesis, 4-bromo-1,5-dimethyl-1*H*-pyrazole was used instead of 3-bromo-1,5-dimethyl-1*H*-1,2,4-triazole and $Pd(dppf)_2Cl_2$ was used instead of $[PPh_3]_4Pd$. The crude product was purified by flash column chromatography on silica (hexane/AcOEt, 1:1 to 0:100, v/v; then: AcOEt/MeOH, 20:1, v/v) and then by preparative normal-phase column chromatography (Silica-based HPLC column, Hex:IPA, 1.5:1 (*v*/*v*), isocratic elution) and then by preparative reversed-phase column chromatography (column: Cosmosil Cholester 20 x 250 mm, water/MeCN + 1‰ TFA, 95:5 to 20:80, 30 min, 20 mL/min).
ESI-MS m/z for $C_{24}H_{35}ClN_3O_3S$ found 480.2/482.2 $[M+H]^+$; $R_t$ = 0.89 min (Method A); $^1$H NMR (700 MHz, DMSO-$d_6$ + $D_2O$, 348 K) δ 7.41 - 7.37 (m, 2H), 7.34 - 7.31 (m, 2H), 7.14 (s, 1H), 4.31 - 4.23 (m, 1H), 3.81 - 3.76 (m, 2H), 3.70 - 3.67 (m, 1H), 3.65 (s, 3H), 3.61 - 3.54 (m, 2H), 3.49 - 3.45 (m, 1H), 3.40 - 3.38 (m, 1H), 3.32 - 3.26 (m, 1H), 3.22 - 3.18 (m, 1H), 3.14 - 3.09 (m, 1H), 3.03 (s, 3H), 2.43 - 2.35 (m, 1H), 2.28 - 2.22 (m, 2H), 2.16 (s, 3H), 1.95 - 1.88 (m, 2H), 1.58 - 1.49 (m, 2H), 1.49 - 1.41 (m, 2H).

**Example 114.**

Synthesis of (2*R*,5*S*)-5-(4-chlorobenzyl)-4-(4-(1-methyl-1*H*-pyrazol-3-yl)cyclohexyl)-2-((methylsulfonyl)methyl)morpholine 2,2,2-trifluoroacetate (**114**).

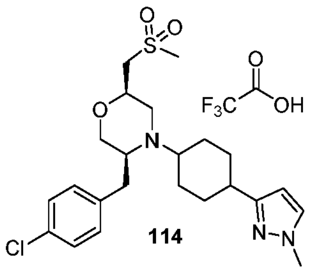

114

The title compound (**114**) was obtained as a TFA salt as a single isomer in 12% overall yield in a similar way to Example 15 with the exception that, in the first step of the synthesis, 3-bromo-1-methyl-1*H*-pyrazole was used instead of 3-bromo-1,5-dimethyl-1*H*-1,2,4-triazole. The crude product was purified by flash column chromatography on silica (pentane/AcOEt, 1:1 to 0:100, *v*/*v*; then: AcOEt/MeOH, 50:1 to 20:1, *v*/*v*) and then by preparative reversed-phase column chromatography (column: Cosmosil Cholester 20 x 250 mm, water/MeCN + 1‰ TFA, 95:5 to 20:80, 30 min, 20 mL/min).
ESI-MS m/z for $C_{23}H_{33}ClN_3O_3S$ found 466.0/468.0 $[M+H]^+$; Rt = 0.95 min (Method A); $^1$H NMR (700 MHz, DMSO-$d_6$ + $D_2O$, 348 K) δ 7.46 (d, *J* = 2.2 Hz, 1H), 7.38 (d, $J_{AA'}$ = 8.5 Hz, 2H), 7.32 *(d,* $J_{BB'}$ = 8.5 Hz, 2H), 6.02 (d, *J* = 2.2 Hz, 1H), 4.28 - 4.25 (m, 1H), 3.80 - 3.77 (m, 2H), 3.73 (s, 3H), 3.68 - 3.67 (m, 1H), 3.61 - 3.56 (m, 2H), 3.46 - 3.41 (m, 2H), 3.30 - 3.26 (m, 1H), 3.19 (dd, *J* = 13.7 Hz, *J* = 11.0 Hz, 1H), 3.11 (dd, *J* = 13.8 Hz, *J* = 4.2 Hz, 1H), 3.03 (s, 3H), 2.57 - 2.53 (m, 1H), 2.27 - 2.25 (m, 2H), 2.10 - 2.06 (m, 2H), 1.56 - 1.44 (m, 4H).

**Example 115.**

Synthesis of (2*R*,5*S*)-5-(4-chlorobenzyl)-4-(4-(2-methyl-2*H*-1,2,3-triazol-4-yl)cyclohexyl)-2-((methylsulfonyl)methyl) morpholine 2,2,2-trifluoroacetate (**115**).

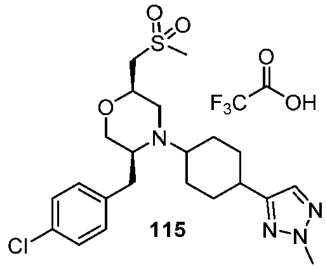

**115**

The title compound (**115**) was obtained as a TFA salt as a single isomer in 14% overall yield in a similar way to Example 15 with the exception that, in the first step of the synthesis, 4-bromo-2-methyl-2*H*-1,2,3-triazole was used instead of 3-bromo-1,5-dimethyl-1*H*-1,2,4-triazole. The crude product was purified by flash column chromatography on silica (hexane/AcOEt, 1:1 to 1:7, v/v; then: AcOEt/MeOH, 70:1, *v*/*v*) and then by preparative reversed-phase column chromatography (column: Cosmosil Cholester 20 x 250 mm, water/MeCN + 1‰ TFA, 95:5 to 20:80, 30 min, 20 mL/min).
ESI-MS m/z for $C_{22}H_{32}ClN_4O_3S$ found 467.0/469.0 $[M+H]^+$; Rt = 0.95 min (Method A); $^1H$ NMR (700 MHz, DMSO-$d_6$ + $D_2O$, 348 K) δ 7.49 (s, 1H), 7.38 (d, $J_{AA'}$ = 8.5 Hz, 2H), 7.31 (d, $J_{BB'}$ = 8.5 Hz, 2H), 4.26 - 4.24 (m, 1H), 4.03 (s, 3H), 3.76 - 3.73 (m, 2H), 3.67 - 3.66 (m, 1H), 3.58 (dd, *J* = 14.9 Hz, *J* = 7.9 Hz, 1H), 3.54 - 3.51 (m, 1H), 3.40 - 3.38 (m, 2H), 3.27 - 3.24 (m, 1H), 3.17 (dd, *J* = 13.7 Hz, *J* = 11.0 Hz, 1H), 3.09 (dd, *J* = 13.8 Hz, *J* = 4.1 Hz, 1H), 3.02 (s, 3H), 2.69 - 2.65 (m, 1H), 2.27 - 2.25 (m, 2H), 2.14 - 2.10 (m, 2H), 1.57 - 1.46 (m, 4H).

**Example 116.**

Synthesis of (2*R*,5*S*)-5-(4-chlorobenzyl)-4-(4-(2-methyl-2*H*-tetrazol-5-yl)cyclohexyl)-2-((methylsulfonyl)methyl)morpholine 2,2,2-trifluoroacetate (**116**).

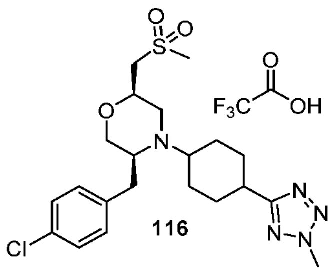

**116**

The title compound (**116**) was obtained as a TFA salt as a single isomer in 6% overall yield in a similar way to Example 15 with the exception that, in the first step of the synthesis, 5-bromo-2-methyl-2*H*-tetrazole was used instead of 3-bromo-1,5-dimethyl-1*H*-1,2,4-triazole. The crude product was purified by flash column chromatography on silica (pentane/AcOEt, 5:1 to 0:100, *v*/*v*) and then by preparative reversed-phase column chromatography (column: Cosmosil Cholester 20 x 250 mm, water/MeCN + 1‰ TFA, 95:5 to 20:80, 30 min, 20 mL/min).
ESI-MS m/z for $C_{21}H_{31}ClN_5O_3S$ found 468.2/470.2 $[M+H]^+$; Rt = 0.90 min (Method A); $^1H$ NMR (700 MHz, DMSO-$d_6$ + $D_2O$, 348 K) δ 7.38 (d, $J_{AA'}$ = 8.5 Hz, 2H), 7.32 (d, $J_{BB'}$ = 8.5 Hz, 2H), 4.27 - 4.24 (m, 4H), 3.77 - 3.73 (m, 2H), 3.68 - 3.66 (m, 1H), 3.58 (dd, *J* = 15.0 Hz, *J* = 7.9 Hz, 1H), 3.54 - 3.51 (m, 1H), 3.47 - 3.44 (m, 1H), 3.40 - 3.38 (m, 1H), 3.28 - 3.24 (m, 1H), 3.17 (dd, *J* = 13.7 Hz, *J* = 10.9 Hz, 1H), 3.10 (dd, *J* = 13.6 Hz, *J* = 4.0 Hz, 1H), 3.03 (s, 3H), 2.91 (tt *J* = 12.0 Hz, *J* = 3.6 Hz, 1H), 2.30 - 2.28 (m, 2H), 2.22 - 2.19 (m, 2H), 1.67 - 1.54 (m, 4H).

**Example 117.**

Synthesis of (2R,5S)-5-(4-chlorobenzyl)-*N,N*-dimethyl-4-(4-(5-methyl-1,3,4-thiadiazol-2-yl)cyclohexyl)morpholine-2-carboxamide 2,2,2-trifluoroacetate (**117**).

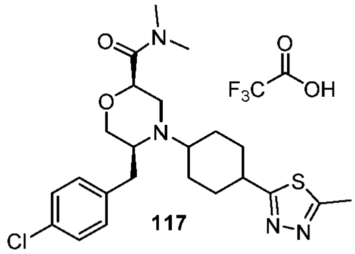

**117**

The title compound (**117**) was obtained as a TFA salt as a single isomer in 18% overall yield in a similar way to Example 15 with the exception that, in the first step of the synthesis, 2-bromo-5-methyl-1,3,4-thiadiazole was used instead of 3-bromo-1,5-dimethyl-1*H*-1,2,4-triazole and in the fourth step of the synthesis, the compound **117b** (the synthesis of this compound wass described below) was used instead of the compound **3h.** The crude product was purified by silica-gel column chromatography (pentane/AcOEt, 3:1 to 0:100, *v/v*; then: AcOEt/MeOH, 140:1 to 10:1, v/v) and then by preparative reversed-phase column chromatography (column: Cosmosil Cholester 20 x 250 mm, water/MeCN + 1‰ TFA, 95:5 to 30:70, 30 min, 20 mL/min).
ESI-MS m/z for $C_{23}H_{32}ClN_4O_2S$ found 463.2/465.2 $[M+H]^+$; $R_t$ = 0.95 min (Method A); $^1H$ NMR (700 MHz, DMSO-$d_6$ + $D_2O$, 348 K) δ 7.37 (d, $J_{AA'}$ = 8.4 Hz, 2H), 7.31 (d, $J_{BB'}$ = 8.4 Hz, 2H), 4.74 - 4.73 (m, 1H), 3.83 - 3.79 (m, 2H), 3.65 - 3.60 (m, 2H), 3.54 - 3.52 (m, 1H), 3.41 - 3.39 (m, 1H), 3.22 - 3.19 (m, 1H), 3.11 (tt, *J* = 11.8 Hz, *J* = 3.4 Hz, 1H), 3.04 - 3.01 (m, 4H), 2.89 (brs, 3H), 2.66 (3H), 2.26 - 2.17 (m, 4H), 1.72 - 1.60 (m, 3H), 1.58 - 1.52 (m, 1H).

Synthesis of the compound **117b:**

Step 1.

Synthesis of *tert*-butyl (2*R*,5*S*)-5-(4-chlorobenzyl)-2-(dimethylcarbamoyl)morpholine-4-carboxylate (**117a**).

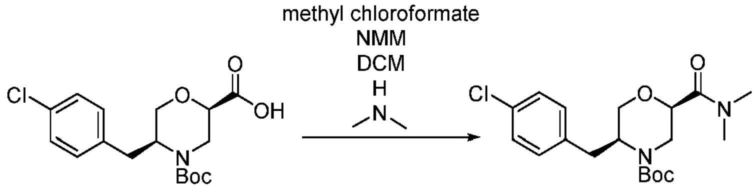


**5a** **117a**

The title compound (**117a**) was obtained from **5a** (480 mg; 1.35 mmol) according to the General Procedure **V** in 97% yield (500 mg; 1.31 mmol).
ESI-MS m/z for $C_{19}H_{28}ClN_2O_4$ found 383.2/385.2 $[M+H]^+$; Rt = 1.47 min (Method A)

Step 2.

Synthesis of (2*R*,5*S*)-5-(4-chlorobenzyl)-*N,N*-dimethylmorpholine-2-carboxamide hydrochloride (**117b**).

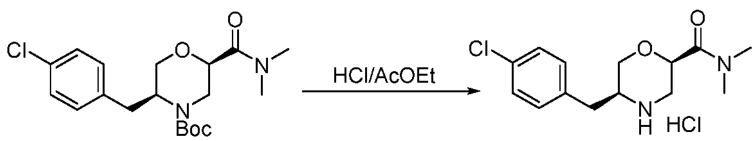


**117a** **117b**

The title compound (**117b**) was obtained as a hydrochloride salt from **117a** (180 mg; 0.47 mmol) according to the General Procedure **IVa** in 99% yield (150 mg; 0.47 mmol).
ESI-MS m/z for $C_{14}H_{20}ClN_2O_2$ found 283.1/285.1 $[M+H]^+$; Rt = 0.67 min (Method A)

**Example 118.**

Synthesis of (2*R*,5*S*)-5-(4-chlorobenzyl)-4-(4-(5-methyl-1,3,4-thiadiazol-2-yl)cyclohexyl)-2-((methylsulfonyl)methyl) morpholine hydrochloride (**118**).

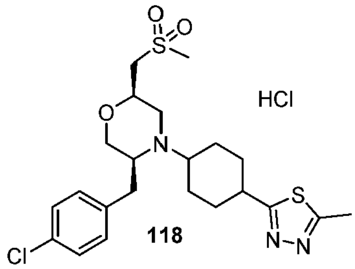

**118**

The title compound (**118**) was obtained as a hydrochloride salt as a single isomer in 18% overall yield in a similar way to Example 15 with the exception that, in the first step of the synthesis, 2-bromo-5-methyl-1,3,4-thiadiazole was used instead of 3-bromo-1,5-dimethyl-1*H*-1,2,4-triazole. The crude product was purified by silica-gel column chromatography (pentane/AcOEt, 4:1 to 0:100, *v/v*; then: AcOEt/MeOH, 80:1 to 20:1, *v/v*) and then by preparative reversed-phase column chromatography (column: Cosmosil Cholester 20 x 250 mm, water/MeCN + 1‰ TFA, 90:10 to 20:80, 30 min, 20 mL/min). Then a white solid of TFA salt (after lyophilization) was dissolved in water and to this solution 2 N HCl (2 mL) was added, then concentrated *in vacuo* and lyophilized from water to give the product as a hydrochloride salt.
ESI-MS m/z for $C_{22}H_{31}ClN_3O_3S_2$ found 484.1/486.1 $[M+H]^+$; $R_t$ = 0.91 min (Method A); $^1H$ NMR (700 MHz, DMSO-$d_6$ + $D_2O$, 348 K) δ 7.37 (d, $J_{AA'}$ = 8.4 Hz, 2H), 7.33 (d, $J_{BB'}$ = 8.4 Hz, 2H), 4.38 - 4.36 (m, 1H), 3.90 (d, $J$ = 13.2 Hz, 1H), 3.80 - 3.78 (m, 1H), 3.67 (d, $J$ = 13.3 Hz, 1H), 3.60 - 3.52 (m, 3H), 3.42 (dd, $J$ = 15.1 Hz, $J$ = 3.4 Hz, 1H), 3.31 - 3.30 (m, 1H), 3.20 (dd, $J$ = 13.8 Hz, $J$ = 10.8 Hz, 1H), 3.16 - 3.10 (m, 2H), 3.03 (s, 3H), 2.66 (3H), 2.32 - 2.29 (m, 2H), 2.27 - 2.22 (m, 2H), 1.75 - 1.69 (m, 2H), 1.67 - 1.60 (m, 2H).

**Example 119.**

Synthesis of (2*R*,5*S*)-5-(4-chlorobenzyl)-4-(4-(5-methoxy-1-methyl-1*H*-1,2,4-triazol-3-yl)cyclohexyl)-2-((methylsulfonyl)methyl)morpholine 2,2,2-trifluoroacetate (**119**).

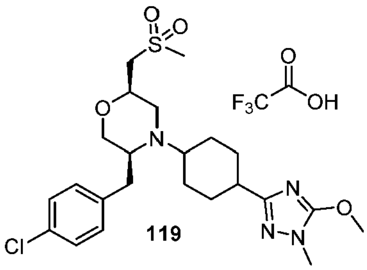

**119**

The title compound (**119**) was obtained as a TFA salt as a single isomer in 5% overall yield in a similar way to Example 15 with the exception that, in the first step of the synthesis, 3-bromo-5-methoxy-1-methyl-1*H*-1,2,4-triazole was used instead of 3-bromo-1,5-dimethyl-1*H*-1,2,4-triazole and Pd(dppf) was used instead of $[PPh_3]_4Pd$. The crude product was purified by silica-gel column chromatography (hexane/AcOEt, 1:1 to 0:100, v/v; then: AcOEt/MeOH, 50:1 to 20:1, *v/v*) and then transferred into HCl salt with using HCl/DCM and then purified twice by preparative reversed-phase column chromatography (first: column: Cosmosil Cholester 20 x 250 mm, water + 0.3‰ HCl (36%)/MeCN, 95:5 to 20:80, 30 min, 20 mL/min, second: column: Cosmosil Cholester 20 x 250 mm, water/MeCN + 1‰ TFA, 95:5 to 20:80, 30 min, 20 mL/min).
ESI-MS m/z for $C_{23}H_{34}ClN_4O_4S$ found 497.4/499.4 $[M+H]^+$; $R_t$ = 0.94 min (Method A); $^1H$ NMR (700 MHz, DMSO-$d_6$ +

$D_2O$, 348 K) δ 7.37 (d, $J_{AA'}$ = 8.4 Hz, 2H), 7.31 (d, $J_{BB'}$ = 8.4 Hz, 2H), 4.28 - 4.24 (m, 1H), 3.97 (s, 3H), 3.78 - 3.73 (m, 2H), 3.68 - 3.66 (m, 1H), 3.58 - 3.55 (m, 1H), 3.52 - 3.51 (m, 1H), 3.45 (s, 3H), 3.42 - 3.38 (m, 2H), 3.27 - 3.24 (m, 2H), 3.17 (dd, *J* = 13.8 Hz, *J* = 10.7 Hz, 1H), 3.09 (dd, *J* = 13.8 Hz, *J* = 4.1 Hz, 1H), 3.02 (s, 3H), 2.25 - 2.24 (m, 2H), 2.11 - 2.08 (m, 2H), 1.57 - 1.48 (m, 4H).

**Example 120.**

Synthesis of 3-(4-((2*R*,5*S*)-5-(4-chlorobenzyl)-2-((methylsulfonyl)methyl)morpholino)-cyclohexyl)-1-methyl-1*H*-1,2,4-triazol-5-ol 2,2,2-trifluoroacetate (**120**).

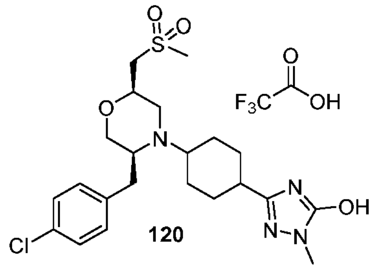

**120**

The title compound (**120**) was obtained during the synthesis of the compound **119** as a TFA salt as a single isomer as a by-product in 5% overall yield. The crude product was purified by silica-gel column chromatography (hexane/AcOEt, 1:1 to 0:100, v/v; then: AcOEt/MeOH, 50:1 to 20:1, v/v) and then transferred into HCl salt with using HCl/DCM and then purified twice by preparative reversed-phase column chromatography (first: column: Cosmosil Cholester 20 x 250 mm, water + 0.3‰ HCl (36%)/MeCN, 95:5 to 20:80, 30 min, 20 mL/min, second: column: Cosmosil Cholester 20 x 250 mm, water/MeCN + 1‰ TFA, 95:5 to 20:80, 30 min, 20 mL/min).
ESI-MS m/z for $C_{22}H_{32}ClN_4O_4S$ found 483.3/485.3 $[M+H]^+$; $R_t$ = 0.80 min (Method A); $^1H$ NMR (700 MHz, DMSO-$d_6$ + $D_2O$, 348 K) δ 7.36 (d, $J_{AA'}$ = 8.4 Hz, 2H), 7.29 (d, $J_{BB'}$ = 8.4 Hz, 2H), 4.25 - 4.21 (m, 1H), 3.74 - 3.72 (m, 1H), 3.68 - 3.64 (m, 2H), 3.57 - 3.53 (m, 1H), 3.46 - 3.44 (m, 1H), 3.34 - 3.31 (m, 2H), 3.23 - 3.20 (m, 4H), 3.15 (dd, *J* = 13.6 Hz, *J* = 10.8 Hz, 1H), 3.05 (dd, *J* = 14.1 Hz, *J* = 4.0 Hz, 1H), 3.01 (s, 3H), 2.47 - 2.44 (m, 1H), 2.23 - 2.21 (m, 2H), 2.09 - 2.06 (m, 2H), 1.51 - 1.45 (m, 4H)

**Example 121.**

Synthesis of (2*R*,5*S*)-5-(4-chlorobenzyl)-4-(4-(1-isopropyl-1*H*-pyrazol-3-yl)cyclohexyl)-2-((methylsulfonyl)methyl)morpholine 2,2,2-trifluoroacetate (**121**).

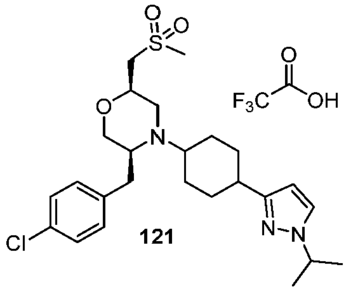

**121**

The title compound (**121**) was obtained as a TFA salt as a single isomer in 18% overall yield in a similar way to Example 15 with the exception that, in the first step of the synthesis, 3-bromo-1-isopropyl-1*H*-pyrazole was used instead of 3-bromo-1,5-dimethyl-1*H*-1,2,4-triazole and Pd(dppf) was used instead of $[PPh_3]_4Pd$. The crude product was purified by silica-gel column chromatography (cyclohexane/AcOEt, 12:1 to 0:100, *v*/*v*) and then transferred into TFA salt with using TFA/DCM and then purified by preparative reversed-phase column chromatography (column: Cosmosil Cholester 20 x 250 mm, water/MeCN + 1‰ TFA, 95:5 to 10:90, 30 min, 20 mL/min).
ESI-MS m/z for $C_{25}H_{37}ClN_3O_3S$ found 494.5/496.5 $[M+H]^+$; $R_t$ = 1.08 min (Method A); $^1H$ NMR (700 MHz, DMSO-$d_6$ +

$D_2O$, 348 K) δ 7.51 (d, $J_{,}$ = 2.2 Hz, 1H), 7.37 (d, $J_{AA'}$ = 8.4 Hz, 2H), 7.31 (d, $J_{BB'}$ = 8.4 Hz, 2H), 6.01 (d, $J_{,}$ = 2.2 Hz, 1H), 4.37 (hept, $J$ = 6.7 Hz, 1H), 4.30 - 4.26 (m, 1H), 3.79 - 3.77 (m, 2H), 3.69 - 3.68 (m, 1H), 3.59 - 3.55 (m, 2H), 2.47 - 2.41 (m, 2H), 2.30 - 2.27 (m, 1H), 3.19 (dd, $J$ = 13.8 Hz, $J$ = 10.7 Hz, 1H), 3.11 (dd, $J$ = 13.8 Hz, $J$ = 4.4 Hz, 1H), 3.03 (s, 3H), 2.59 - 2.54 (m, 1H), 2.27 - 2.25 (m, 2H), 2.10 - 2.07 (m, 2H), 1.57 - 1.44 (m, 4H), 1.36 (d, $J$ = 6.7 Hz, 6H).

**Example 122.**

Synthesis of (2*R*,5*S*)-5-(4-chlorobenzyl)-4-(4-(5-methoxy-1-methyl-1*H*-1,2,4-triazol-3-yl)cyclohexyl)-*N,N*-dimethylmorpholine-2-carboxamide 2,2,2-trifluoroacetate (**122**).

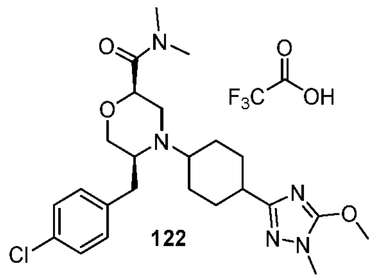

**122**

The title compound (**122**) was obtained as a TFA salt as a single isomer in 5% overall yield in a similar way to Example 15 with the exception that, in the first step of the synthesis, 3-bromo-5-methoxy-1-methyl-1*H*-1,2,4-triazole was used instead of 3-bromo-1,5-dimethyl-1*H*-1,2,4-triazole and in this reaction, Pd(dppf) was used instead of $[PPh_3]_4Pd$ and in the fourth step of the synthesis, the compound **117b** was used instead of the compound **3h.** The crude product was purified by silica-gel column chromatography (hexane/AcOEt, 1:1 to 0:100, v/v; then: AcOEt/MeOH, 40:1 to 5:1, *v*/*v*) and then purified twice by preparative reversed-phase column chromatography (column: Cosmosil Cholester 20 x 250 mm, water/MeCN + 1‰ TFA, 95:5 to 30:70, 30 min, 20 mL/min).
ESI-MS m/z for $C_{24}H_{35}ClN_5O_3$ found 476.2/478.2 $[M+H]^+$; Rt = 0.93 min (Method A); $^1H$ NMR (700 MHz, DMSO-$d_6$ + $D_2O$, 348 K) δ 7.37 (d, $J_{AA'}$ = 8.4 Hz, 2H), 7.30 (d, $J_{BB'}$ = 8.4 Hz, 2H), 4.75 - 4.73 (m, 1H), 3.97 (s, 3H), 3.83 - 3.80 (m, 2H), 3.65 - 3.63 (m, 1H), 3.59 - 3.51 (m, 2H), 3.44 - 3.40 (m, 4H), 3.21 - 3.19 (m, 1H), 3.03 - 3.01 (m, 4H), 2.89 (brs, 3H), 2.50 - 2.48 (m, 1H), 2.22 - 2.20 (m, 1H), 2.14 - 2.05 (m, 3H), 1.63 - 1.51 (m, 3H), 1.48 - 1.42 (m, 1H).

**Example 123.**

Synthesis of (2*R*,5*S*)-5-(4-chlorobenzyl)-4-(4-(5-hydroxy-1-methyl-1*H*-1,2,4-triazol-3-yl)cyclohexyl)-*N,N*-dimethylmorpholine-2-carboxamide 2,2,2-trifluoroacetate (**123**).

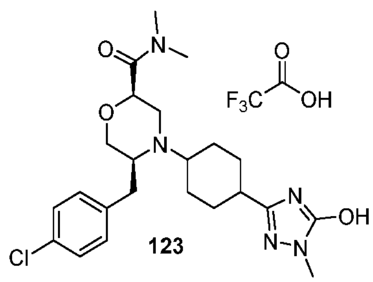

**123**

The title compound (**123**) was obtained during the synthesis of the compound **122** as a TFA salt as a single isomer as a by-product in 1% overall yield. The crude product was purified by silica-gel column chromatography (hexane/AcOEt, 1:1 to 0:100, v/v; then: AcOEt/MeOH, 40:1 to 5:1, *v*/*v*) and then purified twice by preparative reversed-phase column chromatography (column: Cosmosil Cholester 20 x 250 mm, water/MeCN + 1‰ TFA, 95:5 to 30:70, 30 min, 20 mL/min).
ESI-MS m/z for $C_{23}H_{33}ClN_5O_3$ found 462.2/464.2 $[M+H]^+$; Rt = 0.82 min (Method A); $^1H$ NMR (700 MHz, DMSO-$d_6$ + $D_2O$, 348 K) δ 7.36 (d, $J_{AA'}$ = 8.5 Hz, 2H), 7.28 (d, $J_{BB'}$ = 8.5 Hz, 2H), 4.70 - 4.68 (m, 1H), 3.78 (dd, $J$ = 13.0 Hz, $J$ = 2.2 Hz, 1H), 3.73 - 3.72 (m, 1H), 3.63 - 3.61 (m, 1H), 3.48 - 3.46 (m, 2H), 3.37 - 3.35 (m, 1H), 3.21 (s, 3H), 3.16 - 3.14 (m, 1H), 3.03 - 2.99 (m,

4H), 2.88 (brs, 3H), 2.48 - 2.44 (m, 1H), 2.21 - 2.19 (m, 1H), 2.13 - 2.02 (m, 3H), 1.58 - 1.47 (m, 3H), 1.44 - 1.38 (m, 1H).

**Example 124.**

Synthesis of (2*R*,5*S*)-5-(4-chlorobenzyl)-4-(4-(1,2-dimethyl-1*H*-imidazol-4-yl)cyclohexyl)-2-((methylsulfonyl)methyl) morpholine hydrochloride (**124**).

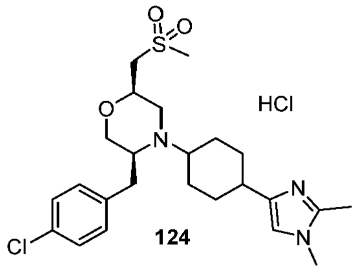

**124**

The title compound (**124**) was obtained as a hydrochloride salt as a single isomer in 1% overall yield in a similar way to Example 15 with the exception that, in the first step of the synthesis, 4-bromo-1,2-dimethyl-1*H*-imidazole was used instead of 3-bromo-1,5-dimethyl-1*H*-1,2,4-triazole. The crude product was purified by silica-gel column chromatography (hexane/AcOEt, 4:1 to 0:100, *v/v*, then: AcOEt/MeOH, 40:1 to 2:1, *v/v*) and then purified by preparative reversed-phase column chromatography (column: Cosmosil Cholester 20 x 250 mm, water + 0.3‰ HCl (36%)/MeCN, 99:1 to 20:80, 30 min, 20 mL/min).
ESI-MS m/z for $C_{24}H_{35}ClN_3O_3S$ found 480.4/482.4 $[M+H]^+$; Rt = 0.58 min (Method A); $^1H$ NMR (700 MHz, DMSO-$d_6$ + $D_2O$) δ 7.37 (d, $J_{AA'}$ = 8.4 Hz, 2H), 7.30 (d, $J_{BB'}$ = 8.4 Hz, 2H), 7.17 (s, 1H), 4.34 - 4.30 (m, 1H), 3.86 - 3.84 (m, 1H), 3.71 - 3.69 (m, 1H), 3.66 - 3.64 (m, 4H), 3.57 (dd, *J* = 15.0 Hz, *J* = 7.7 Hz, 1H), 3.50 - 3.48 (m, 1H), 3.41 - 3.38 (m, 2H), 3.26 - 3.22 (m, 1H), 3.17 (dd, *J* = 13.7 Hz, *J* = 10.8 Hz, 1H), 3.09 - 3.07 (m, 1H), 3.02 (s, 3H), 2.63 (tt, *J* = 12.3 Hz, *J* = 3.5 Hz, 1H), 2.51 (s, 3H), 2.27 - 2.24 (m, 2H), 2.14 - 2.10 (m, 2H), 1.67 - 1.60 (m, 2H), 1.49 - 1.43 (m, 2H).

**Example 125.**

Synthesis of (2*R*,5*S*)-5-(4-chlorobenzyl)-4-(4-(1-methyl-5-(trifluoromethyl)-1*H*-pyrazol-3-yl)cyclohexyl)-2-((methylsulfonyl)methyl)morpholine hydrochloride (**125**).

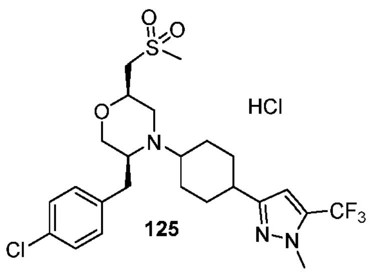

**125**

The title compound (**125**) was obtained as a hydrochloride salt as a single isomer in 2% overall yield in a similar way to Example 15 with the exception that, in the first step of the synthesis, 3-bromo-1-methyl-5-(trifluoromethyl)-1*H*-pyrazole was used instead of 3-bromo-1,5-dimethyl-1*H*-1,2,4-triazole and in this reaction, Pd(dppf) was used instead of $[PPh_3]_4Pd$. The crude product was purified by silica-gel column chromatography (hexane/AcOEt, 5:1 to 0:100, v/v) and then purified twice by preparative reversed-phase column chromatography (first: column: Cosmosil Cholester 20 x 250 mm, water + 0.3‰ HCl (36%)/MeCN, 95:5 to 10:90, 30 min, 20 mL/min, second: column: Cosmosil Cholester 20 x 250 mm, water + 0.3‰ HCl (36%)/MeCN, 95:5 to 30:70, 30 min, 20 mL/min).
ESI-MS m/z for $C_{24}H_{32}ClF_3N_3O_3S$ found 534.2/536.2 $[M+H]^+$; Rt = 1.20 min (Method A); $^1H$ NMR (700 MHz, DMSO-$d_6$ + $D_2O$, 348 K) δ 7.37 (d, $J_{AA'}$ = 8.5 Hz, 2H), 7.31 (d, $J_{BB'}$ = 8.5 Hz, 2H), 6.63 (s, 1H), 4.26 - 4.24 (m, 1H), 3.86 (brd, *J* = 0.7 Hz, 3H), 3.77 - 3.75 (m, 1H), 3.71 - 3.70 (m, 1H), 3.66 - 3.64 (m, 1H), 3.57 (dd, *J* = 15.0 Hz, *J* = 7.9 Hz, 1H), 3.51 - 3.49 (m, 1H), 3.40 - 3.37 (m, 2H), 3.23 - 3.20 (m, 1H), 3.16 (dd, *J* = 13.6 Hz, *J* = 11.0 Hz, 1H), 3.07 (dd, *J* = 13.6 Hz, *J* = 3.8 Hz, 1H), 3.02 (s,

3H), 2.62 - 2.58 (m, 1H), 2.25 - 2.23 (m, 2H), 2.10 - 2.06 (m, 2H), 1.56 - 1.44 (m, 4H); $^{19}F$ NMR (250 MHz, DMSO-$d_6$ + $D_2O$) δ -59.5 (s).

**Example 126.**

Synthesis of (2*R*,5*S*)-5-(4-chlorobenzyl)-4-(4-(4,5-dimethylthiophen-2-yl)cyclohexyl)-2-((methylsulfonyl)methyl)morpholine hydrochloride (**126**).

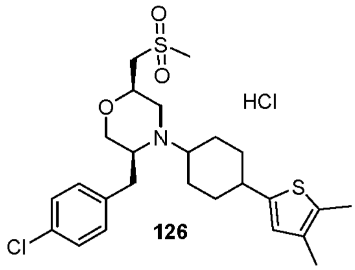

**126**

The title compound (**126**) was obtained as a hydrochloride salt as a single isomer in 3% overall yield in a similar way to Example 15 with the exception that, in the first step of the synthesis, 5-bromo-2,3-dimethylthiophene was used instead of 3-bromo-1,5-dimethyl-1*H*-1,2,4-triazole and in this reaction Pd(dppf) was used instead of $[PPh_3]_4Pd$. The crude product was purified by flash column chromatography on silica (hexane/AcOEt, 100:0 to 45:55, *v*/*v*, 35 min) and then purified by preparative reversed-phase column chromatography (column: Cosmosil Cholester 20 x 250 mm, water + 0.3‰ HCl (36%)/MeCN, 95:5 to 10:90, 30 min, 20 mL/min).
ESI-MS m/z for $C_{25}H_{35}ClNO_33_2$ found 496.3/498.3 $[M+H]^+$; $R_t$ = 1.43 min (Method A); $^1H$ NMR (700 MHz, DMSO-$d_6$ + $D_2O$, 348 K) δ 7.37 (d, $J_{AA'}$ = 8.5 Hz, 2H), 7.31 (d, $J_{BB'}$ = 8.5 Hz, 2H), 6.50 (s, 1H), 4.29 - 4.27 (m, 1H), 3.81 - 3.79 (m, 1H), 3.74 - 3.72 (m, 1H), 3.66 - 3.64 (m, 1H), 3.57 (dd, *J* = 15.0 Hz, *J* = 7.9 Hz, 1H), 3.52 - 3.50 (m, 1H), 3.42 - 3.37 (m, 2H), 3.26 - 3.22 (m, 1H), 3.16 (dd, *J* = 13.8 Hz, *J* = 10.8 Hz, 1H), 3.09 (dd, *J* = 13.8 Hz, *J* = 4.2 Hz, 1H), 3.02 (s, 3H), 2.67 (tt, *J* = 12.0 Hz, *J* = 3.4 Hz, 1H), 2.24 - 2.22 (m, 2H), 2.20 (s, 3H), 2.09 - 2.04 (m, 2H), 1.99 (s, 3H), 1.59 - 1.53 (m, 2H), 1.46 - 1.40 (m, 2H).

**Example 127.**

Synthesis of 4-(4-((2*R*,5*S*)-5-(4-chlorobenzyl)-2-((methylsulfonyl)methyl)morpholino)-cyclohexyl)-1-methyl-1*H*-pyrrole-2-carbonitrile hydrochloride (**127**).

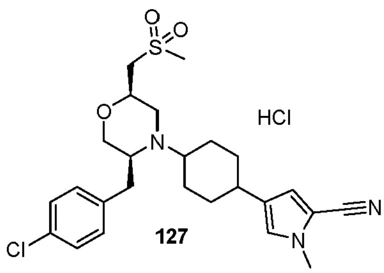

**127**

Step 1.

Synthesis of 1-methyl-4-(1,4-dioxaspiro[4.5]dec-7-en-8-yl)-1*H*-pyrrole-2-carbonitrile (**127a**).

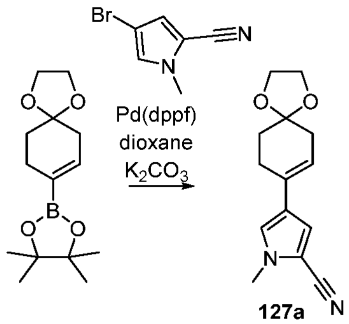


127a

The solution of 4,4,5,5-tetramethyl-2-(1,4-dioxaspiro[4.5]dec-7-en-8-yl)-1,3,2-dioxaborolane (420 mg; 1.58 mmol), 4-bromo-1-methyl-1*H*-pyrrole-2-carbonitrile (278 mg, 1.50 mmol), Pd(dppf) (122 mg; 0.15 mmol) in mixture of dioxane (8 mL) and 2 M aqueous solution of $K_2CO_3$ (2 mL) was heated at 100 °C for 2.5 hours under argon atmosphere. LC-MS showed complete consumption of the starting material. Then a whole was concentrated *in vacuo* and the residue was purified by flash column chromatography on silica (hexane/AcOEt, 100:0 to 60:40, *v/v,* 35 min). Compound **127a** was obtained in 62% yield (226 mg; 0.93 mmol).
ESI-MS m/z for $C_{14}H_{17}N_2O_2$ found 244.9 $[M+H]^+$; Rt = 1.25 min (Method A); $^1H$ NMR (700 MHz, DMSO-$d_6$ + $D_2O$) δ 7.18 (d, *J* = 1.8 Hz, 1H), 7.00 (d, *J* = 1.9 Hz, 1H), 5.84 - 5.79 (m, 1H), 3.88 (s, 4H), 3.68 (s, 3H), 2.39 - 2.34 (m, 2H), 2.27 - 2.21 (m, 2H), 1.78 - 1.68 (m, 2H).

Step 2.

Synthesis of 1-methyl-4-(1,4-dioxaspiro[4.5]decan-8-yl)-1*H*-pyrrole-2-carbonitrile (**127b**).

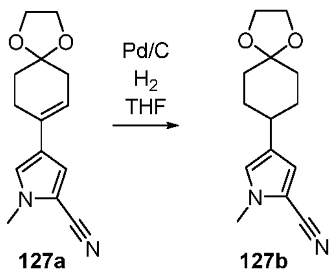


127a 127b

To the solution of **127a** (224 mg; 0.917 mmol) in THF (5 mL) 10% Pd/C (25 mol%; 24 mg, 0.23 mmol) was added and the reaction mixture was conducted under hydrogen atmosphere at room temperature for 4 hours. The reaction progress was monitored by LC-MS. When analysis indicated completion of the reaction, Pd/C was filtered off through Celite and the filtrate was concentrated *in vacuo.* The crude product was used to the next step without additional purification. Compound **127b** was obtained in 95% yield (215 mg; 0.873 mmol).
ESI-MS m/z for $C_{14}H_{19}N_2O_2$ found 247.0 $[M+H]^+$; $R_t$ = 1.20 min (Method A)

Step 3.

Synthesis of 1-methyl-4-(4-oxocyclohexyl)-1*H*-pyrrole-2-carbonitrile (**127c**).

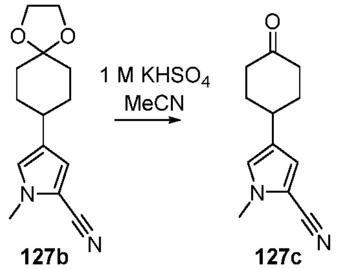


127b 127c

The solution of **127b** (215 mg; 0.873 mmol) and 1 M $KHSO_4$ (15 mL) in MeCN (3 mL) was stirred at room temperature overnight. The reaction progress was monitored by LC-MS analysis. After analytical control indicated completion of the reaction, to this mixture 4 M NaOH was added to pH 12 and a whole was extracted with DCM (2 x). The combined organic solutions were dried over anhydrous $Na_2SO_4$, filtered and concentrated *in vacuo.* The crude ketone was used to the next step without additional purification. Compound **127c** was obtained in 95% yield (168 mg; 0.831 mmol). ESI-MS m/z for $C_{12}H_{15}N_2O$ found 203.1 $[M+H]^+$; Rt = 1.06 min (Method A); $^1H$ NMR (700 MHz, DMSO-$d_6$ + $D_2O$) δ 7.01 (d, $J$ = 1.8 Hz, 1H), 6.82 (d, $J$ = 1.9 Hz, 1H), 3.66 (s, 3H), 2.96 - 2.87 (m, 1H), 2.50 - 2.44 (m, 2H), 2.25 - 2.17 (m, 2H), 2.13 - 2.06 (m, 2H), 1.72 - 1.59 (m, 2H). Step 4.

Synthesis of 4-(4-((2*R*,5*S*)-5-(4-chlorobenzyl)-2-((methylsulfonyl)methyl)morpholino)-cyclohexyl)-1-methyl-1*H*-pyrrole-2-carbonitrile hydrochloride (**127**).

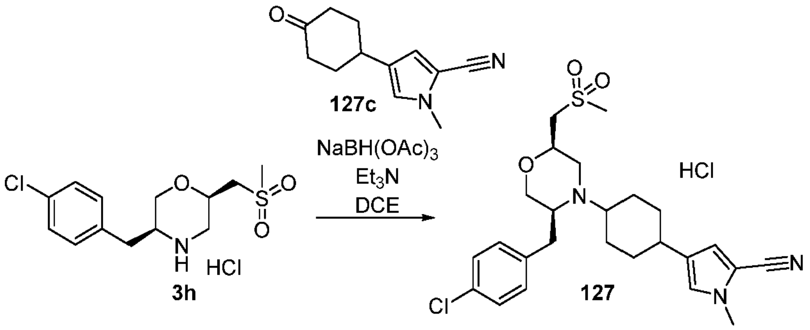


The title compound (**127**) was obtained as a hydrochloride salt as a single isomer from **3h** (102 mg; 0.30 mmol) and from ketone **127c** (60 mg; 0.30 mmol) according to the General Procedure **VII** in 10% yield (17 mg; 0.03 mmol). The crude product was purified by flash column chromatography on silica (hexane/AcOEt, 100:0 to 35:65, *v/v,* 36 min, 11 mL/min) and then purified by preparative reversed-phase column chromatography (column: Cosmosil Cholester 20 x 250 mm, water + 0.3‰ HCl (36%)/MeCN, 95:5 to 20:80, 30 min, 20 mL/min).
ESI-MS m/z for $C_{25}H_{33}ClN_3O_3S$ found 490.4/492.4 $[M+H]^+$; $R_t$ = 1.04 min (Method A); $^1H$ NMR (700 MHz, DMSO-$d_6$ + $D_2O$, 348 K) δ 7.37 (d, $J_{AA'}$ = 8.4 Hz, 2H), 7.30 (d, $J_{BB'}$ = 8.4 Hz, 2H), 6.91 (d, $J$ = 1.8 Hz, 1H), 6.72 (d, $J$ = 1.8 Hz, 1H), 4.27 - 4.24 (m, 1H), 3.78 - 3.76 (m, 1H), 3.72 - 3.70 (m, 1H), 3.66 - 3.64 (m, 4H), 3.57 (dd, $J$ = 15.0 Hz, $J$ = 7.9 Hz, 1H), 3.51 - 3.49 (m, 1H), 3.41 - 3.35 (m, 2H), 3.24 - 3.20 (m, 1H), 3.18 - 3.14 (m, 1H), 3.08 - 3.06 (m, 1H), 3.02 (s, 3H), 2.42 - 2.38 (m, 1H), 2.22 - 2.20 (m, 2H), 2.03 - 1.99 (m, 2H), 1.58 - 1.54 (m, 2H), 1.39 - 1.32 (m, 2H).

**Example 128.**

Synthesis of (2*R*,5*S*)-5-(4-chlorobenzyl)-4-(4-(5-cyano-1-methyl-1*H*-pyrrol-3-yl)cyclohexyl)morpholine-2-carboxylic acid hydrochloride (**128**).

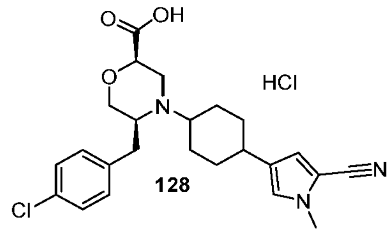

128

The title compound (**128**) was obtained as a hydrochloride salt as a single isomer in 7% overall yield in a similar way to Example 69 with the exception that, in the first step of the synthesis, the ketone **127c** and the compound **9b** were used instead of the ketone **15c** and the compound **65b** and in the second step of the synthesis, 4 M NaOH was used instead of 1 M NaOH. The crude product was purified twice by preparative reversed-phase column chromatography (first: column: Cosmosil Cholester 20 x 250 mm, water + 0.3‰ HCl (36%)/MeCN, 98:2 to 10:90, 30 min, 18 mL/min, second: C-18, water + 0.3‰ HCl (36%)/MeCN, 98:2 to 10:90, 30 min, 18 mL/min).
ESI-MS m/z for $C_{24}H_{29}ClN_3O_3$ found 442.3/444.3 $[M+H]^+$; Rt = 1.09 min (Method A); $^1H$ NMR (700 MHz, DMSO-$d_6$ + $D_2O$, 348 K) δ 7.37 (d, $J_{AA'}$ = 8.4 Hz, 2H), 7.30 (d, $J_{BB'}$ = 8.4 Hz, 2H), 6.90 (d, *J* = 1.8 Hz, 1H), 6.71 (d, *J* = 1.8 Hz, 1H), 4.42 - 4.40 (m, 1H), 3.72 - 3.66 (m, 5H), 3.63 - 3.62 (m, 1H), 3.42 - 3.31 (m, 3H), 3.08 (dd, *J* = 14.0 Hz, *J* = 4.5 Hz, 1H), 2.97 (dd, *J* = 13.7 Hz, *J* = 10.3 Hz, 1H), 2.39 (tt, *J* = 12.1 Hz, *J* = 3.6 Hz, 1H), 2.11 - 2.07 (m, 2H), 2.02 - 1.95 (m, 2H), 1.59 - 1.46 (m, 2H), 1.38 - 1.25 (m, 2H).

**Example 129.**

Synthesis of (2*R*,5*S*)-5-(4-chlorobenzyl)-4-(4-(1-ethyl-5-methyl-1*H*-pyrazol-3-yl)cyclohexyl)-2-((methylsulfonyl)methyl) morpholine hydrochloride (**129**).

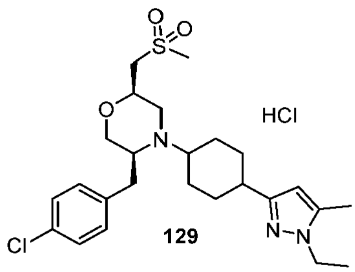

129

Step 1.

Synthesis of 1-ethyl-5-methyl-3-(1,4-dioxaspiro[4.5]dec-7-en-8-yl)-1*H*-pyrazole (**129a**).

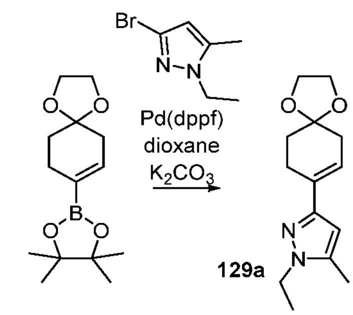


129a

The solution of 4,4,5,5-tetramethyl-2-(1,4-dioxaspiro[4.5]dec-7-en-8-yl)-1,3,2-dioxaborolane (739 mg; 2.78 mmol), 3-bromo-1-ethyl-5-methyl-1*H*-pyrazole (500 mg, 2.64 mmol), Pd(dppf) (212 mg; 0.26 mmol) in mixture of dioxane (16 mL) and 2 M aqueous solution of $K_2CO_3$ (4 mL) was heated at 100 °C for 5 hours under argon atmosphere. LC-MS showed complete consumption of the starting material. Then a whole was concentrated *in vacuo* and the residue was

purified by flash column chromatography on silica (hexane/AcOEt, 100:0 to 25:75, *v/v,* 35 min). Compound **129a** was obtained in 86% yield (565 mg; 2.28 mmol).
ESI-MS m/z for $C_{14}H_{21}N_2O_2$ found 249.3 $[M+H]^+$; $R_t$ = 1.07 min (Method A); $^1H$ NMR (700 MHz, DMSO-$d_6$ + $D_2O$) δ 6.09 (s, 1H), 5.99 - 5.97 (m, 1H), 4.00 - 3.92 (m, 2H), 3.88 (s, 4H), 2.48 - 2.42 (m, 2H), 2.32 - 2.24 (m, 2H), 2.19 (s, 3H), 1.74 - 1.65 (m, 2H), 1.22 (t, *J* = 7.2 Hz, 3H). Step 2.

Synthesis of 1-ethyl-5-methyl-3-(1,4-dioxaspiro[4.5]decan-8-yl)-1*H*-pyrazole (**129b**).

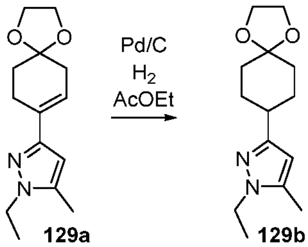


129a 129b

To the solution of **129a** (560 mg; 2.25 mmol) in AcOEt (20 mL) 10% Pd/C (5 mol%; 14 mg, 0.13 mmol) was added and the reaction mixture was conducted under hydrogen atmosphere at room temperature for 4 hours. The reaction progress was monitored by LC-MS. When analysis indicated completion of the reaction, Pd/C was filtered off through the Celite pad and the filtrate was concentrated *in vacuo.* The crude product was used to the next step without additional purification. Compound **129b** was obtained in 99% yield (558 mg; 2.23 mmol).
ESI-MS m/z for $C_{14}H_{23}N_2O_2$ found 251.3 $[M+H]^+$; $R_t$ = 0.99 min (Method A)

Step 3.

Synthesis of 4-(1-ethyl-5-methyl-1*H*-pyrazol-3-yl)cyclohexan-1-one (**129c**).

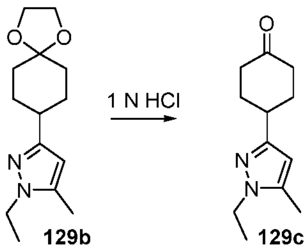


129b 129c

The solution of **129b** (558 mg; 2.23 mmol) in 1 N HCl (15 mL) was stirred at room temperature overnight. The reaction progress was monitored by LC-MS. When analysis indicated completion of the reaction, to this mixture aqueous solution of NaOH (4 M) was added to pH 12 and then extracted with DCM (2 x). The combined organic solutions were dried over anhydrous $MgSO_4$, filtered and concentrated *in vacuo.* The crude product was used to the next step without additional purification. Compound **129c** was obtained in 89% yield (409 mg; 1.98 mmol).
ESI-MS m/z for $C_{12}H_{19}N_2O$ found 207.1 $[M+H]^+$; $R_t$ = 0.85 min (Method A)

Step 4.

Synthesis of (2*R*,5*S*)-5-(4-chlorobenzyl)-4-(4-(1-ethyl-5-methyl-1*H*-pyrazol-3-yl)cyclohexyl)-2-((methylsulfonyl)methyl) morpholine hydrochloride (**129**).

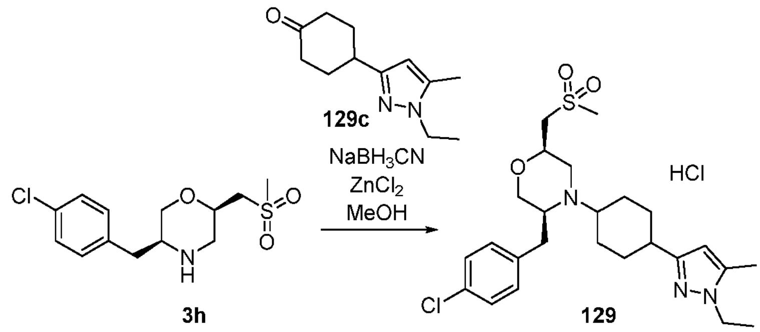


3h

129

The solution of free amine of compound **3h** (after basic (0.5 M NaOH) extraction with DCM)(91 mg; 0.30 mmol), ketone **129c** (65 mg; 0.31 mmol) in MeOH (2 mL) was stirred at room temperature for 30 minutes and then to this mixture $ZnCl_2$ (49 mg; 0.36 mmol) was added and stirred at room temperature for 10 minutes. Then $NaBH_3CN$ (23 mg; 0.36 mmol) was added and this mixture was then stirred at room temperature overnight. The reaction progress was monitored by LC-MS. When analysis indicated completion of the reaction, the reaction mixture was diluted with 5% $NaHCO_3$ and after 10 minutes a whole was concentrated *in vacuo.* The residue was suspended in DCM and washed with water. An organic phase was dried over anhydrous $Na_2SO_4$, filtered and concentrated *in vacuo.* The crude product was purified by flash column chromatography on silica (hexane/AcOEt, 100:0 to 0:100, v/v, then: AcOEt/MeOH, 100:0 to 80:20, v/v) and then by preparative reversed-phase column chromatography (column: Cosmosil Cholester 20 x 250 mm, water + 0.3‰ HCl (36%)/MeCN, 95:5 to 20:80, 30 min, 20 mL/min). The title compound **129** was obtained as a hydrochloride salt as a single isomer in 7% yield (13 mg; 0.02 mmol).

ESI-MS m/z for $C_{25}H_{37}ClN_3O_3S$ found 494.4/496.4 $[M+H]^+$; Rt = 1.00 min (Method A); $^1H$ NMR (700 MHz, DMSO-$d_6$ + $D_2O$) δ 7.40 (d, $J_{AA'}$ = 8.5 Hz, 2H), 7.32 (d, $J_{BB'}$ = 8.5 Hz, 2H), 5.86 (s, 1H), 4.24 - 4.21 (m, 1H), 3.94 (q, *J* = 7.2 Hz, 2H), 3.81 - 3.80 (m, 1H), 3.75 - 3.72 (m, 1H), 3.64 - 3.61 (m, 3H), 3.40 - 3.38 (m, 2H), 3.22 - 3.15 (m, 2H), 3.09 - 3.07 (m, 1H), 3.03 (s, 3H), 2.48 (tt, *J* = 11.8 Hz, *J* = 3.4 Hz, 1H), 2.27 - 2.23 (m, 2H), 2.18 (d, *J* = 0.6 Hz, 3H), 2.03 - 2.01 (m, 2H), 1.53 - 1.38 (m, 4H), 1.22 (t, *J* = 7.2 Hz, 2H).

**Example 130.**

Synthesis of (2*R*,5*S*)-5-(4-chlorobenzyl)-4-(4-(1-ethyl-5-methyl-1*H*-pyrazol-3-yl)cyclohexyl)-morpholine-2-carboxylic acid hydrochloride (**130**).

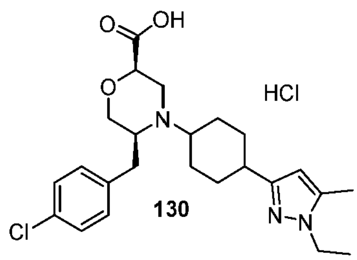


130

Step 1.

Synthesis of methyl (2*R*,5*S*)-5-(4-chlorobenzyl)-4-(4-(1-ethyl-5-methyl-1*H*-pyrazol-3-yl)cyclohexyl)morpholine-2-carboxylate (**130a**).

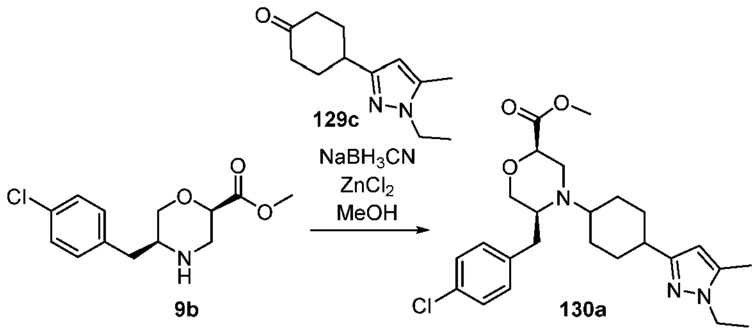


The solution of **9b** (136 mg; 0.50 mmol), ketone **129c** (105 mg; 0.50 mmol) in MeOH (2.5 mL) was stirred at room temperature for 30 minutes and then to this mixture $ZnCl_2$ (83 mg; 0.61 mmol) was added and stirred at room temperature for 10 minutes. Then $NaBH_3CN$ (38 mg; 0.61 mmol) was added and this mixture was then stirred at room temperature overnight. The reaction progress was monitored by LC-MS. When analysis indicated completion of the reaction, the reaction mixture was diluted with 5% $NaHCO_3$ and after 10 minutes a whole was concentrated *in vacuo.* The residue was suspended in DCM and washed with water. An organic phase was dried over anhydrous $Na_2SO_4$, filtered and concentrated *in vacuo.* The crude product was purified by flash column chromatography on silica (hexane/AcOEt, 100:0 to 0:100, *v/v,* 35 min). The title compound **130a** was obtained in 34% yield (78 mg; 0.17 mmol).
ESI-MS m/z for $C_{25}H_{35}ClN_3O_3$ found 460.4/462.4 $[M+H]^+$; Rt = 1.06 min (Method A)

Step 2.

Synthesis of (2*R*,5*S*)-5-(4-chlorobenzyl)-4-(4-(1-ethyl-5-methyl-1*H*-pyrazol-3-yl)cyclohexyl)-morpholine-2-carboxylic acid hydrochloride (**130**).

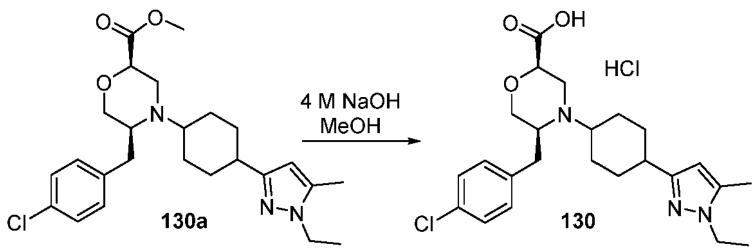


To a solution of **130a** (78 mg; 0.17 mmol) in MeOH (10 mL) 4 M NaOH (0.4 mL) was added and the mixture was stirred at room temperature for 4.5 hours. The reaction progress was monitored by LC-MS. When analysis indicated completion of the reaction, methanol was evaporated off and the residue was dissolved in MeCN/water and this mixture was acidified to pH 4 by addition of $HCl_{aq}$. The crude product was purified by preparative reversed-phase column chromatography (column: Cosmosil Cholester 20 x 250 mm, water + 0.3‰ HCl (36%)/MeCN, 98:2 to 10:90, 30 min, 18 mL/min). The title compound **130** was obtained as a hydrochloride salt as a single isomer in 29% yield (24 mg; 0.05 mmol).
ESI-MS m/z for $C_{24}H_{33}ClN_3O_3$ found 446.4/448.4 $[M+H]^+$; Rt = 1.03 min (Method A); $^1H$ NMR (700 MHz, DMSO-$d_6$ + $D_2O$, 348 K) $\delta$ 7.37 (d, $J_{AA'}$ = 8.5 Hz, 2H), 7.31 (d, $J_{BB'}$ = 8.5 Hz, 2H), 5.79 (s, 1H), 4.47 (dd, *J* = 8.9 Hz, *J* = 3.5 Hz, 1H), 3.93 (q, *J* = 7.2 Hz, 2H), 3.77 - 3.69 (m, 3H), 3.53 - 3.42 (m, 3H), 3.12 (dd, *J* = 13.9 Hz, *J* = 4.1 Hz, 1H), 3.02 (dd, *J* = 13.9 Hz, *J* = 10.2 Hz, 1H), 2.47 (tt, *J* = 12.2 Hz, *J* = 3.6 Hz, 1H), 2.17 - 2.12 (m, 5H), 2.05 - 1.99 (m, 2H), 1.63 - 1.51 (m, 2H), 1.46 - 1.33 (m, 2H), 1.23 (t, *J* = 7.2 Hz, 2H).

**Example 131.**

Synthesis of (2*R*,5*S*)-5-(4-chlorobenzyl)-*N*-ethyl-4-(4-(1-ethyl-5-methyl-1*H*-pyrazol-3-yl)cyclohexyl)morpholine-2-carboxamide hydrochloride (**131**).

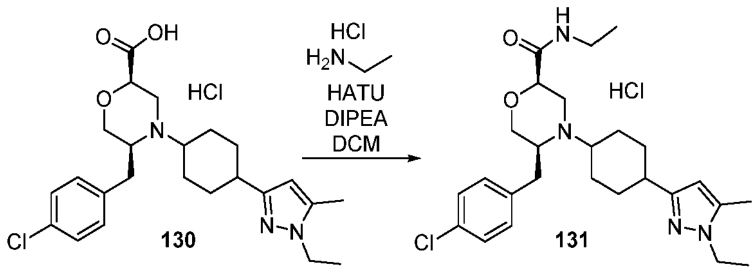


To the solution of **130** (15 mg; 0.031 mmol) and ethylamine hydrochloride (4 mg; 0.047 mmol) in DCM (1.5 mL) diisopropylethylamine (DIPEA; 27 μL; 0.155 mmol) was added and after 5 minutes HATU (15 mg; 0.040 mmol) was added in one portion and the reaction mixture was stirred at room temperature overnight. The reaction progress was monitored by TLC and LC-MS. When analyses indicated completion of the reaction, a whole was concentrated *in vacuo.* The crude product was purified by preparative reversed-phase column chromatography (column: Cosmosil Cholester 20 x 250 mm, water + 0.3‰ HCl (36%)/MeCN, 95:5 to 20:80, 30 min, 19 mL/min). The title compound **131** was obtained as a hydrochloride salt as a single isomer in 58% yield (9 mg; 0.018 mmol).
ESI-MS m/z for $C_{26}H_{38}ClN_4O_2$ found 473.5/475.5 $[M+H]^+$; Rt = 1.03 min (Method A); $^1$H NMR (700 MHz, DMSO-$d_6$ + $D_2O$, 348 K) δ 7.41 - 7.38 (m, 2H), 7.37 - 7.32 (m, 2H), 5.87 - 5.81 (m, 1H), 4.40 - 4.31 (m, 1H), 3.96 (q, *J* = 7.2 Hz, 2H), 3.85 - 3.79 (m, 2H), 3.75 - 3.70 (m, 1H), 3.63 - 3.57 (m, 1H), 3.53 - 3.49 (m, 1H), 3.39 - 3.34 (m, 1H), 3.24 - 3.09 (m, 5H), 2.26 - 2.17 (m, 5H), 2.08 - 2.01 (m, 2H), 1.65 - 1.55 (m, 2H), 1.47 - 1.37 (m, 2H), 1.25 (t, *J* = 7.2 Hz, 3H), 1.08 (t, *J* = 7.2 Hz, 3H).

**Example 132.**

Synthesis of (2*R*,5*S*)-5-(4-chlorobenzyl)-4-(4-(4-methyl-1*H*-pyrazol-1-yl)cyclohexyl)-2-((methylsulfonyl)methyl)morpholine hydrochloride (**132**).

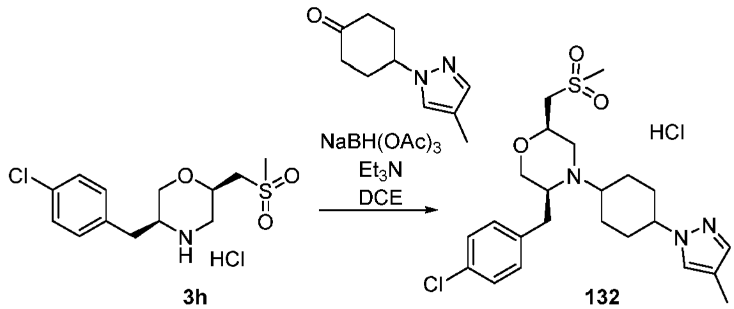


The title compound (**132**) was obtained as a hydrochloride salt as a single isomer from **3h** (220 mg; 0.648 mmol) and from 4-(4-methyl-1*H*-pyrazol-1-yl)cyclohexan-1-one (110 mg; 0.617 mmol) according to the General Procedure **VII** in 18% yield (55 mg; 0.110 mmol). The crude product was purified by flash column chromatography on silica (hexane/AcOEt, 100:0 to 10:90, *v/v,* 45 min) and then purified by preparative reversed-phase column chromatography (column: Cosmosil Cholester 20 x 250 mm, water + 0.3‰ HCl (36%)/MeCN, 95:5 to 20:80, 30 min, 18 mL/min).
ESI-MS m/z for $C_{23}H_{33}ClN_3O_3S$ found 466.4/468.4 $[M+H]^+$; $R_t$ = 1.04 min (Method A); $^1$H NMR (700 MHz, DMSO-$d_6$ + $D_2O$, 348 K) δ 7.41 (s, 1H), 7.37 (d, $J_{AA'}$ = 8.5 Hz, 2H), 7.31 (d, $J_{BB'}$ = 8.5 Hz, 2H), 7.21 (s, 1H), 4.30 - 4.26 (m, 1H), 4.08 (tt, *J* = 11.9 Hz, *J* = 3.8 Hz, 1H), 3.80 - 3.78 (m, 1H), 3.76 - 3.74 (m, 1H), 3.66 (dd, *J* = 13.3 Hz, *J* = 1.1 Hz, 1H), 3.58 (dd, *J* = 15.0 Hz, *J* = 8.0 Hz, 1H), 3.54 - 3.52 (m, 1H), 3.51 - 3.47 (m, 1H), 3.40 - 3.38 (m, 1H), 3.30 - 3.26 (m, 1H), 3.18 (dd, *J* = 13.7 Hz, *J* = 10.9 Hz, 1H), 3.12 - 3.09 (m, 1H), 3.02 (s, 3H), 2.30 - 2.28 (m, 2H), 2.17 - 2.13 (m, 2H), 1.98 (s, 3H), 1.84 - 1.76 (m, 2H), 1.66 - 1.60 (m, 2H).

**Example 133.**

Synthesis of (2*R*,5*S*)-5-(4-chlorobenzyl)-4-(4-(4-methyl-1*H*-pyrazol-1-yl)cyclohexyl)-morpholine-2-carboxylic acid hydrochloride (**133**).

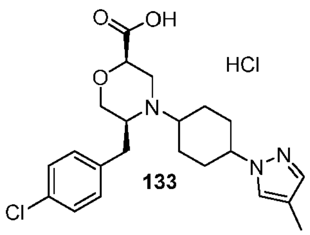

133

The title compound (**133**) was obtained as a hydrochloride salt as a single isomer in 18% overall yield in a similar way to Example 69 with the exception that, in the first step of the synthesis, 4-(4-methyl-1*H*-pyrazol-1-yl)cyclohexan-1-one and the compound **9b** were used instead of the ketone **15c** and the compound **65b** and, in the second step of the synthesis, 4 M NaOH was used instead of 1 M NaOH. The crude product was purified twice by preparative reversed-phase column chromatography (first: column: Cosmosil Cholester 20 x 250 mm, water + 0.3‰ HCl (36%)/MeCN, 98:2 to 30:70, 30 min, 20 mL/min, second: C-18, water + 0.3‰ HCl (36%)/MeCN, 98:2 to 30:70, 30 min, 20 mL/min).
ESI-MS m/z for $C_{22}H_{29}ClN_3O_3$ found 418.0/420.0 $[M+H]^+$; Rt = 1.16 min (Method A); $^1H$ NMR (700 MHz, DMSO-$d_6$ + $D_2O$, 348 K) $\delta$ 7.40 (s, 1H), 7.37 (d, $J_{AA'}$ = 8.4 Hz, 2H), 7.30 (d, $J_{BB'}$ = 8.4 Hz, 2H), 7.20 (s, 1H), 4.41 (dd, *J* = 8.5 Hz, *J* = 3.8 Hz, 1H), 4.06 (tt, *J* = 11.8 Hz, *J* = 3.8 Hz, 1H), 3.71 - 3.66 (m, 2H), 3.63 - 3.61 (m, 1H), 3.43 - 3.36 (m, 3H), 3.10 (dd, *J* = 13.9 Hz, *J* = 4.4 Hz, 1H), 2.98 (dd, *J* = 13.9 Hz, *J* = 10.2 Hz, 1H), 2.19 - 2.09 (m, 4H), 1.98 (s, 3H), 1.82 - 1.70 (m, 2H), 1.68 - 1.56 (m, 2H).

**Example 134.**

Synthesis of *N*-(39-((((2*R*,5*S*)-5-(4-chlorobenzyl)-4-(4-(4,5-dimethylthiazol-2-yl)cyclohexyl)-morpholin-2-yl)methyl)sulfonyl)-37-oxo-3,6,9,12,15,18,21,24,27,30,33-undecaoxa-36-azanonatriacontyl)-5-((3a*S*,4*S*,6a*R*)-2-oxohexahydro-1*H*-thieno[3,4-*d*]imidazol-4-yl)pentanamide hydrochloride (**134**).

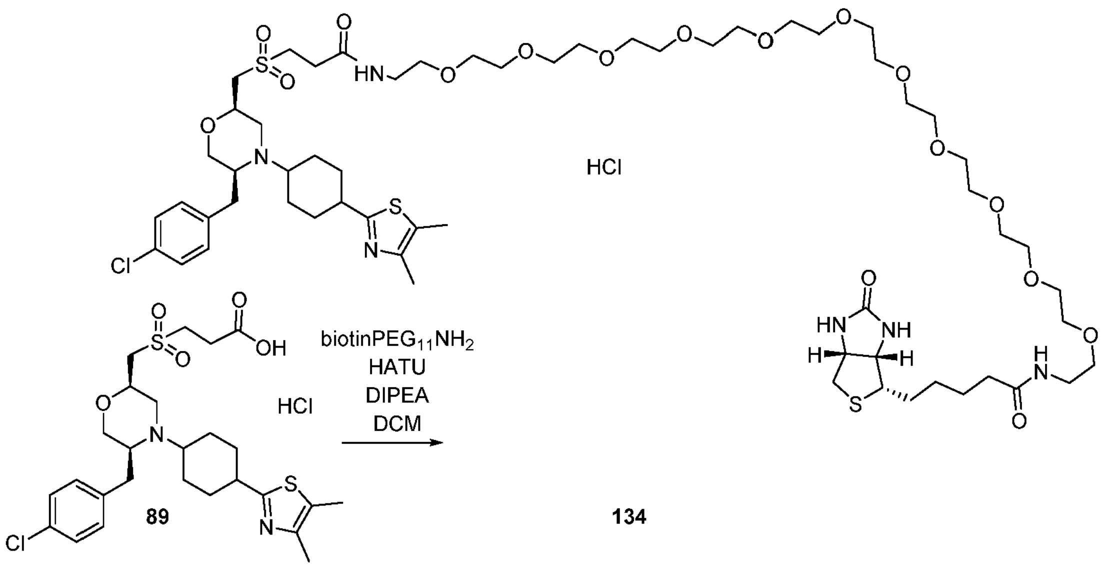


89

134

The title compound (**134**) was obtained as a single isomer as a hydrochloride salt from **89** (30 mg; 0.051 mmol) according to the General Procedure **XI** in 53% yield (36 mg; 0.027 mmol). The crude product was purified by preparative reversed-phase column chromatography (column: Cosmosil Cholester 20 x 250 mm, water + 0.3‰ HCl (36%)/MeCN, 95:5 to 10:90, 30 min, 18 mL/min).
ESI-MS m/z for $C_{60}H_{100}ClN_6O_{17}S_3$ found 1308.0/1310.0 $[M+H]^+$; $R_t$ = 1.12 min (Method A)

**Example 135.**

Synthesis of (2*R*,5*S*)-5-(4-chlorobenzyl)-4-(4-(1,5-dimethyl-1*H*-pyrazol-3-yl)cyclohexyl)-*N*-(1H-tetrazol-5-yl)morpholine-2-carboxamide hydrochloride (**135**).

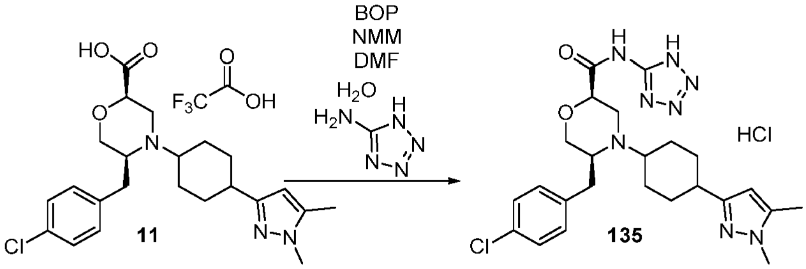


11

135

To the solution of **11** (56 mg; 0.102 mmol) in dry DMF (3 mL) *N*-methylmorpholine (50 μL; 0.459 mmol) and BOP (benzotriazol-1-yloxytris(dimethylamino)phosphonium hexafluorophosphate) (68 mg; 0.153 mmol) were added followed by 1*H*-tetrazol-5-amine monohydrate (26 mg; 0.255 mmol) and the reaction mixture was stirred at room temperature for 5 days. The reaction progress was monitored by LC-MS. When analysis indicated completion of the reaction, the reaction mixture was concentrated *in vacuo.* The crude product was purified by preparative reversed-phase column chromatography (column: Cosmosil Cholester 20 x 250 mm, water + 0.3‰ HCl (36%)/MeCN, 98:2 to 30:70, 30 min, 18 mL/min, Rt = 15.8 - 16.7 min). The title compound **135** was obtained as a single isomer as a hydrochloride sat in 31% yield (17 mg; 0.032 mmol).
ESI-MS m/z for $C_{24}H_{32}ClN_8O_2$ found 499.5/501.5 $[M+H]^+$; Rt = 0.99 min (Method A); $^1H$ NMR (700 MHz, DMSO-$d_6$ + $D_2O$, 348 K) δ 7.41 - 7.37 (m, 2H), 7.37 - 7.34 (m, 2H), 5.82 (s, 1H), 4.72 - 4.65 (m, 1H), 3.90 - 3.84 (m, 1H), 3.83 - 3.75 (m, 2H), 3.71 - 3.65 (m, 1H), 3.60 (s, 3H), 3.56 - 3.48 (m, 2H), 3.17 - 3.08 (m, 2H), 2.48 - 2.44 (m, 1H), 2.26 - 2.19 (m, 2H), 2.17 (s, 3H), 2.08 - 1.98 (m, 2H), 1.66 - 1.54 (m, 2H), 1.49 - 1.38 (m, 2H).

**Example 136.**

Synthesis of (2*R*,5*S*)-4-(4-(1*H*-tetrazol-5-yl)cyclohexyl)-5-(4-chlorobenzyl)-2-((methylsulfonyl)-methyl)morpholine hydrochloride (**136**).

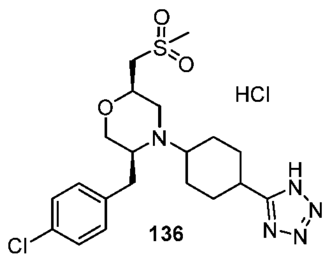

136

Step 1.

Synthesis of 4-((2*R*,5*S*)-5-(4-chlorobenzyl)-2-((methylsulfonyl)methyl)morpholino)cyclohexane-1-carbonitrile (**136a**).

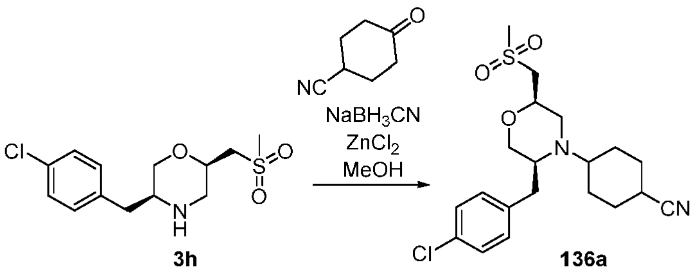


3h 136a

The solution of **3h** (as a free amine - obtained after basic (0.5 M NaOH) extraction with DCM) (200 mg; 0.66 mmol), 4-oxocyclohexane-1-carbonitrile (90 mg; 0.73 mmol) in MeOH (3 mL) was stirred at room temperature for 30 minutes and then to this mixture $ZnCl_2$ (100 mg; 0.73 mmol) was added and stirred at room temperature for 10 minutes. Then $NaBH_3CN$ (48 mg; 0.76 mmol) was added and this mixture was then stirred at room temperature overnight. The reaction progress was monitored by LC-MS. When analysis indicated completion of the reaction, the reaction mixture was diluted with 5% $NaHCO_3$ and extracted with AcOEt. An organic phase was dried over anhydrous $Na_2SO_4$, filtered and concentrated *in vacuo.* The crude product was purified by flash column chromatography on silica (hexane/AcOEt, 100:0 to 0:100, *v*/*v,* 35 min). The title compound **136a** was obtained in 41% yield (168 mg; 0.27 mmol).
ESI-MS m/z for $C_{20}H_{28}ClN_2O_{3S}$ found 411.1/413.1 $[M+H]^+$; $R_t$ = 1.75 min (Method F)

Step 2.

Synthesis of (2*R*,5*S*)-4-(4-(1*H*-tetrazol-5-yl)cyclohexyl)-5-(4-chlorobenzyl)-2-((methylsulfonyl)-methyl)morpholine hydrochloride (**136**).

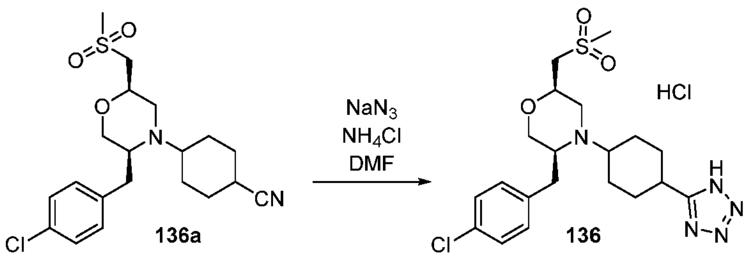


136a 136

To a solution of **136a** (111 mg; 0.271 mmol) in DMF (2 mL) $NaN_3$ (71 mg; 1.100 mmol) and $NH_4Cl$ (60 mg; 1.100 mmol) were added and the mixture was heated at 120 °C overnight. Then another portions of $NaN_3$ (71 mg; 1.100 mmol) and $NH_4Cl$ (60 mg; 1.100 mmol) were added and the mixture was heated at 120 °C for 2 days. The reaction progress was monitored by LC-MS. When analysis indicated completion of the reaction, the reaction mixture was quenched with saturated aqueous $NaHCO_3$ and extracted with AcOEt. An organic layer was then dried over anhydrous $Na_2SO_4$, filtered and concentrated *in vacuo* and the crude product was purified by preparative reversed-phase column chromatography (Luna® 5μm Phenyl-Hexyl, LC Column 250 x 21.2 mm, water + 1‰ HCl/MeCN, 95:5 to 20:80, 40 min, 12 mL/min, Rt = 23 min). The title compound **136** was obtained as a hydrochloride salt as a single diastereoisomer in 3% yield (4 mg; 0.008 mmol).
ESI-MS m/z for $C_{20}H_{29}ClN_5O_3S$ found 454.1/456.1 $[M+H]^+$; Rt = 1.51 min (Method F); $^1H$ NMR (400 MHz, Methanol-$d_4$, δ 7.43 - 7.36 (m, 2H), 7.36 - 7.31 (m, 2H), 4.49 - 4.22 (m, 1H), 4.03 - 3.38 (m, 8H), 3.37 - 3.31 (m, 1H), 3.30 - 3.26 (m, 1H), 3.26 - 3.16 (m, 1H), 3.10 (s, 3H), 2.66 - 2.30 (m, 4H), 1.91 - 1.64 (m, 4H).

**Example 137.**

Synthesis of (2*R*,5*S*)-4-(4-(1*H*-1,2,4-triazol-1-yl)cyclohexyl)-5-(4-chlorobenzyl)-2-((methylsulfonyl)methyl)morpholine hydrochloride (**137**).

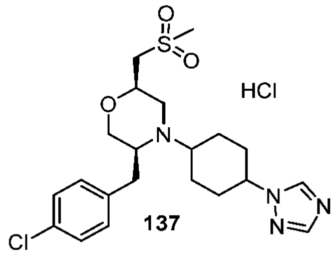

137

Step 1.

Synthesis of 1-(1,4-dioxaspiro[4.5]decan-8-yl)-1*H*-1,2,4-triazole (**137a**).

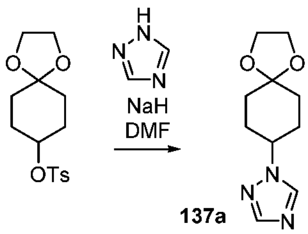

137a

To the solution of 1,4-dioxaspiro[4.5]decan-8-yl 4-methylbenzenesulfonate (250 mg; 0.80 mmol) and 1*H*-1,2,4-triazole (78 mg, 1.12 mmol) in anhydrous DMF (2 mL) NaH (60% in oil; 45 mg; 1.12 mmol) was added and the reaction mixture was stirred at 75 °C for 24 hours under inert atmosphere. The reaction progress was monitored by LC-MS analysis of small aliquots of the crude reaction mixture. When analysis indicated completion of the reaction, water and $NaHCO_3$ were added and this mixture was extracted with AcOEt. An organic phase was dried over anhydrous $Na_2SO_4$, filtered and concentrated *in vacuo.* The crude product was used to the next step without additional purification.
ESI-MS m/z for $C_{10}H_{16}N_3O_2$ found 210.0 $[M+H]^+$; Rt = 1.00 min (Method F)

Step 2.

Synthesis of 4-(1*H*-1,2,4-triazol-1-yl)cyclohexan-1-one (**137b**).

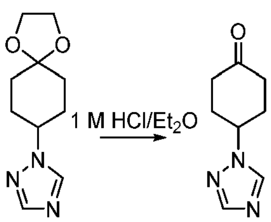

**137a** **137b**

The solution of **137a** (the crude product) in 1 M HCl/$Et_2O$ (3 mL/3 mL) was stirred at room temperature overnight. The reaction progress was monitored by LC-MS. When analysis indicated completion of the reaction, to this mixture aqueous solution of NaOH (4 M) was added to pH 13 and then extracted with AcOEt (2 x). The combined organic solutions were dried over anhydrous $Na_2SO_4$, filtered and concentrated *in vacuo.* The crude product was used to the next step without additional purification.
ESI-MS m/z for $C_8H_{12}N_3O$ found 166.0 $[M+H]^+$; Rt = 0.50 min (Method F)

Step 3.

Synthesis of (2*R*,5*S*)-4-(4-(1*H*-1,2,4-triazol-1-yl)cyclohexyl)-5-(4-chlorobenzyl)-2-((methylsulfonyl)methyl)morpholine hydrochloride (**137**).

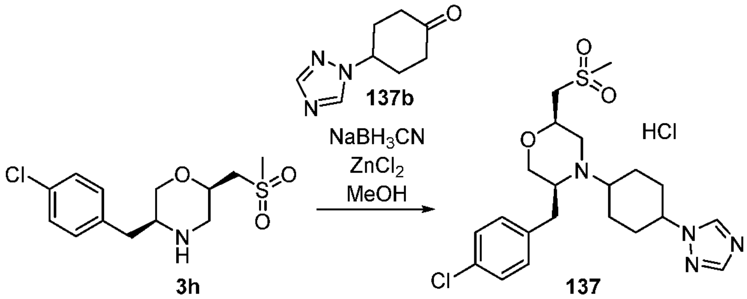


3h 137

The solution of **3h** (as a free amine - obtained after basic (0.5 M NaOH) extraction with DCM) (185 mg; 0.61 mmol), **137b** (100 mg; 0.61 mmol) in MeOH (3 mL) was stirred at room temperature for 30 minutes and then to this mixture $ZnCl_2$ (92 mg; 0.67 mmol) was added and stirred at room temperature for 10 minutes. Then $NaBH_3CN$ (42 mg; 0.67 mmol) was added and this mixture was then stirred at room temperature overnight. The reaction progress was monitored by LC-MS. When analysis indicated completion of the reaction, the reaction mixture was diluted with 5% $NaHCO_3$ and extracted with AcOEt. An organic phase was dried over anhydrous $Na_2SO_4$, filtered and concentrated *in vacuo.* The crude product was purified by flash column chromatography on silica (AcOEt/MeOH, 100:0 to 70:30, *v*/*v*, 28 min, Rt = 11.5 min) and then by preparative reversed-phase column chromatography (Luna® 5μm Phenyl-Hexyl, LC Column 250 x 21.2 mm, water + 1‰ HCl/MeCN, 95:5 to 20:80, 40 min, 12 mL/min, Rt = 22 min). The title compound **137** was obtained as a hydrochloride salt as a single isomer in 13% yield (40 mg; 0.08 mmol).

ESI-MS m/z for $C_{21}H_{30}ClN_4O_3S$ found 453.1/455.1 $[M+H]^+$; Rt = 1.61 min (Method F); $^1H$ NMR (400 MHz, Methanol-$d_4$) δ 9.72 (s, 1H), 8.81 (s, 1H), 7.43 - 7.27 (m, 4H), 4.72 - 4.63 (m, 1H), 4.04 - 3.60 (m, 6H), 3.56 - 3.39 (m, 2H), 3.34 (s, 2H), 3.09 (s, 3H), 2.64 - 2.37 (m, 4H), 2.20 - 2.00 (m, 2H), 2.00 - 1.83 (m, 2H).

**Example 138.**

Synthesis of (2*R*,5*S*)-4-(4-(1*H*-pyrazol-1-yl)cyclohexyl)-5-(4-chlorobenzyl)-2-((methylsulfonyl)-methyl)morpholine hydrochloride (**138**).

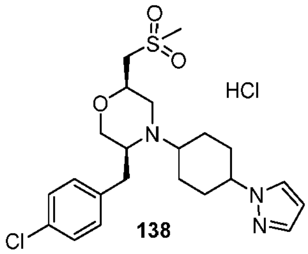


138

Step 1.

Synthesis of 1-(1,4-dioxaspiro[4.5]decan-8-yl)-1*H*-pyrazole (**138a**).

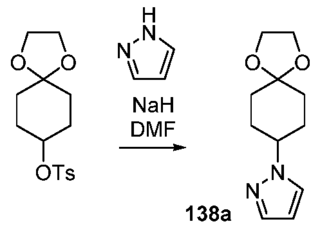


NaH (60% in mineral oil; 147 mg; 3.688 mmol) was added to DMF (1.6 mL) and stirred at room temperature for 5 minutes. Then to this suspension 1*H*-pyrazole (251 mg; 3.688 mmol) was added in several portions within 10 minutes and the mixture was stirred at room temperature for 1 hour. Then to this mixture 1,4-dioxaspiro[4.5]decan-8-yl 4-methylbenzenesulfonate (960 mg; 3.073 mmol) was added and the reaction mixture was vigorously stirred at 60 °C for 16 hours. The reaction progress was monitored by LC-MS and TLC analyses of small aliquots of the crude reaction mixture. When analyses indicated completion of the reaction, the reaction was cooling down to room temperature and then to this mixture water (30 mL) was added and then extracted with AcOEt (3 x 20 mL) and $Et_2O$ (20 mL). The combined organic solutions were washed with water (5 mL) and brine (5 mL), dried over anhydrous $Na_2SO_4$, and concentrated *in vacuo.* The crude product was purified by flash column chromatography on silica (hexane/AcOEt, 100:0 to 50:50, *v/v,* 25 min). The title compound **138a** was obtained as a colorless oil in 72% yield (459 mg; 2.205 mmol).
ESI-MS m/z for $C_{11}H_{17}N_2O_2$ found 209.0 $[M+H]^+$; $R_t$ = 1.67 min (Method F)

Step 2.

Synthesis of 4-(1*H*-pyrazol-1-yl)cyclohexan-1-one (**138b**).

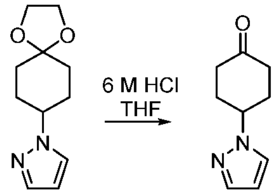


**138a** **138b**

To the solution of **138a** (459 mg; 2.205 mmol) in THF (8.8 mL) an aqueous solution of HCl (6 M; 4.4 mL) was added and a whole was stirred at room temperature for 40 hours. The reaction progress was monitored by LC-MS. When analysis indicated completion of the reaction, THF was evaporated under reduced pressure and the residue was alkalized with saturated aqueous solution of $K_2CO_3$ and then extracted with AcOEt (3 x 20 mL). The combined organic solutions were dried over anhydrous $Na_2SO_4$, filtered and concentrated *in vacuo.* The crude product was used to the next step without additional purification.
ESI-MS m/z for $C_9H_{13}N_2O$ found 165.0 $[M+H]^+$; Rt = 0.87 min (Method F)

Step 3.

Synthesis of (2*R*,5*S*)-4-(4-(1*H*-pyrazol-1-yl)cyclohexyl)-5-(4-chlorobenzyl)-2-((methylsulfonyl)-methyl)morpholine hydrochloride (**138**).

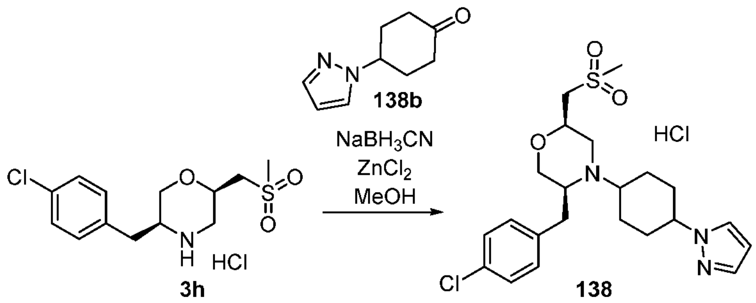


The solution of **3h** (as a free amine - obtained after basic (4 M NaOH) extraction with chloroform) (160 mg; 0.527 mmol), **138b** (104 mg; 0.632 mmol) in MeOH (5.3 mL) was stirred at room temperature for 30 minutes and then to this mixture $ZnCl_2$ (86 mg; 0.632 mmol) was added and stirred at room temperature for 10 minutes. Then $NaBH_3CN$ (40 mg; 0.632 mmol) was added and this mixture was then stirred at room temperature overnight. The reaction progress was monitored by LC-MS. When analysis indicated completion of the reaction, to the reaction mixture water (10 mL) and an aqueous solution of NaOH (4 M; 15 mL) were added followed by MTBE (25 mL), and the mixture was vigorously stirred at room temperature for 1 hour. Then the phases were separated and an aqueous one was washed with MTBE (2 x 25 mL) and discarded. The combined organic solutions were washed with 2 M NaOH (5 mL) and brine (5 mL), dried over anhydrous $Na_2SO_4$, filtered and concentrated *in vacuo*. The crude product was purified by flash column chromatography on silica (hexane/AcOEt, 100:0 to 0:100, *v/v,* 25 min) and then by preparative reversed-phase column chromatography (Luna® 5μm Phenyl-Hexyl, LC Column 250 x 21.2 mm, water + 1‰ HCl (36%)/MeCN, 90:10 to 20:80, 50 min, 12 mL/min, Rt = 32 min). The title compound **138** was obtained as a hydrochloride salt as a single isomer in 18% yield (48 mg; 0.098 mmol).

ESI-MS m/z for $C_{22}H_{31}ClN_3O_3S$ found 452.1/454.1 $[M+H]^+$; Rt = 1.89 min (Method F); $^1H$ NMR (400 MHz, Methanol-$d_4$) δ 7.90 - 7.86 (m, 1H), 7.74 - 7.71 (m, 1H), 7.42 - 7.31 (m, 4H), 6.45 - 6.42 (m, 1H), 4.48 - 4.34 (m, 2H), 3.96 - 3.59 (m, 6H), 3.51 - 3.33 (m, 2H), 3.27 - 3.17 (m, 1H), 3.10 (s, 3H), 2.59 - 2.48 (m, 2H), 2.39 - 2.32 (m, 2H), 2.11 - 1.94 (m, 2H), 1.93 - 1.79 (m, 2H), 1.40 - 1.25 (m, 1H).

**Example 139.**

Synthesis of (2R,5S)-5-(4-chlorobenzyl)-4-(4-(4-fluoro-1*H*-pyrazol-1-yl)cyclohexyl)-2-((methylsulfonyl)methyl)morpholine hydrochloride (**139**).

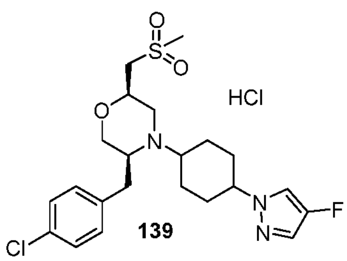
HCl

139

The title compound (**139**) was obtained as a hydrochloride salt as a single isomer in 10% overall yield in a similar way to Example 138 with the exception that, in the first step of the synthesis, 4-fluoro-1*H*-pyrazole was used instead of 1*H*-pyrazole and in the last step of the synthesis, the crude product was purified by flash column chromatography on silica (hexane/AcOEt, 100:0 to 0:100, *v/v*, 25 min) and then by preparative reversed-phase column chromatography (Luna® 5μm Phenyl-Hexyl, LC Column 250 x 21.2 mm, water + 1‰ HCl (36%)/MeCN, 90:10 to 10:90, 40 min, 12 mL/min, $R_t$ = 25.9 min).

ESI-MS m/z for $C_{22}H_{30}ClFN_3O_3S$ found 470.0/472.0 $[M+H]^+$; $R_t$ = 2.09 min (Method F); $^1H$ NMR (400 MHz, Methanol-$d_4$) δ 7.71 - 7.67 (m, 1H), 7.42 - 7.32 (m, 5H), 4.49 - 4.39 (m, 1H), 4.26 - 4.14 (m, 1H), 3.96 - 3.53 (m, 5H), 3.51 - 3.37 (m, 2H), 3.37 - 3.31 (m, 1H), 3.30 - 3.28 (m, 1H), 3.25 - 3.17 (m, 1H), 3.10 (s, 3H), 2.58 - 2.43 (m, 2H), 2.35 - 2.23 (m, 2H), 2.04 - 1.91 (m, 2H), 1.91 - 1.75 (m, 2H).

**Example 140.**

Synthesis of (2R,5S)-5-(4-chlorobenzyl)-4-(4-(5-fluoro-1-methyl-1*H*-pyrazol-3-yl)cyclohexyl)-morpholine-2-carboxylic acid hydrochloride (**140**).

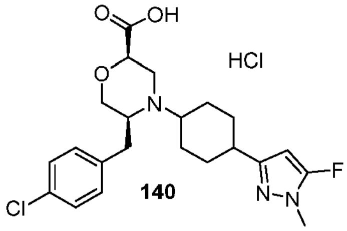

**140**

The title compound (**140**) was obtained as a hydrochloride salt as a single isomer in 7% overall yield in a similar way to Example 69 with the exception that, in the first step of the synthesis, the ketone 4-(5-fluoro-1-methyl-1*H*-pyrazol-3-yl)cyclohexan-1-one (the synthesis of this ketone was the same as for the ketone **15c** with the exception that, in the first step of the synthesis, 3-bromo-5-fluoro-1-methyl-1*H*-pyrazole and Pd(dppf) x DCM were used instead of 3-bromo-1,5-dimethyl-1*H*-1,2,4-triazole and $[PPh_3]_4Pd$) and the compound **9b** were used instead of the ketone **15c** and the compound **65b** and in the last step of the synthesis, the crude product was purified by preparative reversed-phase column chromatography (column: Cosmosil Cholester 20 x 250 mm, water + 0.3‰ HCl (36%)/MeCN, 98:2 to 10:90, 30 min, 20 mL/min, $R_t$ = 14.65 - 15.66 min).
ESI-MS m/z for $C_{22}H_{28}ClFN_3O_3$ found 436.4/438.4 $[M+H]^+$; Rt = 1.03 min (Method A); $^1$H NMR (700 MHz, DMSO-$d_6$ + $D_2O$, 348 K) δ 7.38 - 7.34 (m, 2H), 7.32 - 7.28 (m, 2H), 5.69 (d, *J* = 5.6 Hz, 1H), 4.45 - 4.41 (m, 1H), 3.75 - 3.72 (m, 1H), 3.72 - 3.68 (m, 1H), 3.67 - 3.63 (m, 1H), 3.57 (s, 3H), 3.47 - 3.42 (m, 1H), 3.41 - 3.36 (m, 2H), 3.12 - 3.06 (m, 1H), 3.03 - 2.95 (m, 1H), 2.48 - 2.42 (m, 1H), 2.17 - 2.09 (m, 2H), 2.06 - 1.97 (m, 2H), 1.62 - 1.48 (m, 2H), 1.47 - 1.32 (m, 2H).

**Example 141.**

Synthesis of *N*[1]-(1,31-bis(((2*R*,3*R*,4*R*,5*R*,6*R*)-3-acetamido-4,5-dihydroxy-6-(hydroxymethyl)-tetrahydro-2*H*-pyran-2-yl)oxy)-16-((3-((3-(5-(((2R,3R,4R,5R,6R)-3-acetamido-4,5-dihydroxy-6-(hydroxymethyl)tetrahydro-2*H*-pyran-2-yl)oxy)pentanamido)propyl)amino)-3-oxopropoxy)-methyl)-5,11,21,27-tetraoxo-14,18-dioxa-6,10,22,26-tetraazahentriacontan-16-yl)-*N*[12]-(3-(4-(((2*R*,5*S*)-5-(4-chlorobenzyl)-4-(4-(1,5-dimethyl-1*H*-pyrazol-3-yl)cyclohexyl)morpholine-2-carboxamido)methyl)-1*H*-1,2,3-triazol-1-yl)propyl)dodecanediamide hydrochloride (**141**).

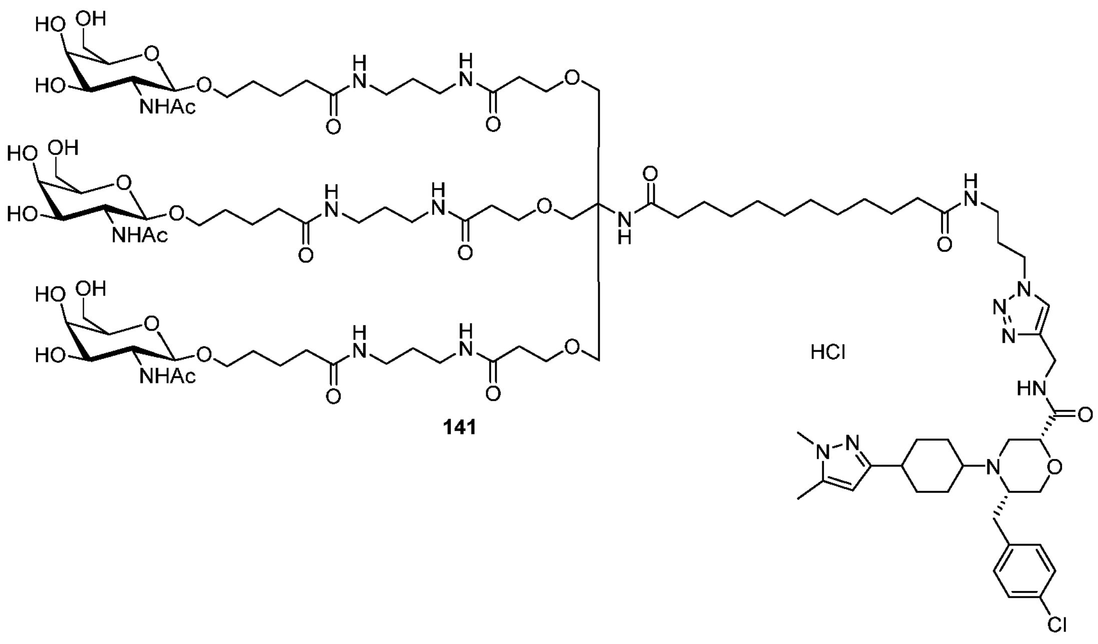

**141**

Step 1.

Synthesis of N1-(1,31-bis(((2R,3R,4R,5R,6R)-3-acetamido-4,5-dihydroxy-6-(hydroxymethyl)-tetrahydro-2H-pyran-2-yl)oxy)-16-((3-((3-(5-(((2R,3R,4R,5R,6R)-3-acetamido-4,5-diacetoxy-6-(acetoxymethyl)tetrahydro-2H-pyran-2-yl)oxy) pentanamido)propyl)amino)-3-oxopropoxy)-methyl)-5,11,21,27-tetraoxo-14,18-dioxa-6,10,22,26-tetraazahentriacontan-16-yl)-N12-(3-(4-(((2R,5S)-5-(4-chlorobenzyl)-4-(4-(1,5-dimethyl-1H-pyrazol-3-yl)cyclohexyl)morpholine-2-carboxamido)methyl)-1H-1,2,3-triazol-1-yl)propyl)dodecanediamide hydrochloride (**141a**).

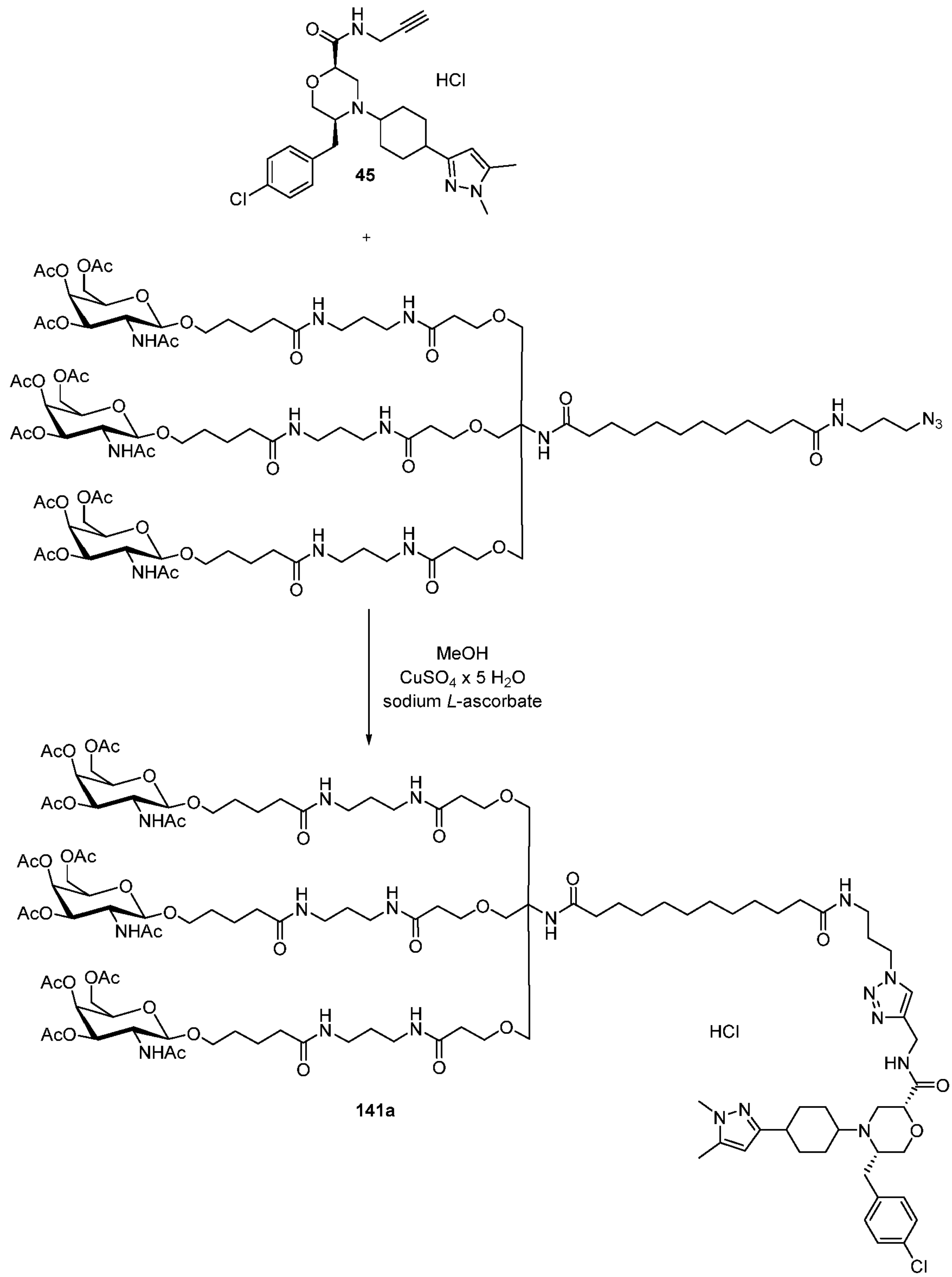


45

141a

To the solution of **45** (8 mg; 0.0160 mmol) and an azide (3-Azido-propylamino-C12-tris-β-GalNAc-PerAc)(20 mg; 0.0096 mmol) in MeOH (0.5 mL) $CuSO_4$ x 5 $H_2O$ (1.3 mg; 0.0050 mmol) was added and after 10 minutes sodium *L*-ascorbate (2 mg; 0.0100 mmol) was added in one portion. Then the reaction mixture was stirred at room temperature over week. After analytical control (LC-MS) indicated completion of the reaction, the reaction mixture was concentrated *in vacuo* and the residue was dissolved in water/MeCN/$HCl_{aq}$, filtered off and taken for preparative reversed-phase column chromatography (first: column: Cosmosil Cholester 20 x 250 mm, water + 0.3‰ HCl (36%)/MeCN, 95:5 to 20:80, 30 min, 19 mL/min, second: C-18, water + 0.3‰ HCl (36%)/MeCN, 95:5 to 20:80, 30 min, 19 mL/min). The title compound (**141a**) was obtained as a hydrochloride salt as a single isomer in 52% yield (13 mg; 0.0050 mmol).
ESI-MS m/z for $C_{120}H_{188}ClN_{18}O_{40}$ found 1279.4 $[(M+2)/2]^+$; $R_t$ = 1.34 min (Method A)

Step 2.

Synthesis of *N*[1]-(1,31-bis(((2*R*,3*R*,4*R*,5*R*,6*R*)-3-acetamido-4,5-dihydroxy-6-(hydroxymethyl)-tetrahydro-2*H*-pyran-2-yl)oxy)-16-((3-((3-(5-(((2*R*,3*R*,4*R*,5*R*,6*R*)-3-acetamido-4,5-dihydroxy-6-(hydroxymethyl)tetrahydro-2*H*-pyran-2-yl)oxy) pentanamido)propyl)amino)-3-oxopropoxy)-methyl)-5,11,21,27-tetraoxo-14,18-dioxa-6,10,22,26-tetraazahentriacontan-16-yl)-*N*[12]-(3-(4-(((2*R*,5*S*)-5-(4-chlorobenzyl)-4-(4-(1,5-dimethyl-1*H*-pyrazol-3-yl)cyclohexyl)morpholine-2-carboxamido)methyl)-1*H*-1,2,3-triazol-1-yl)propyl)dodecanediamide hydrochloride (**141**).

141a

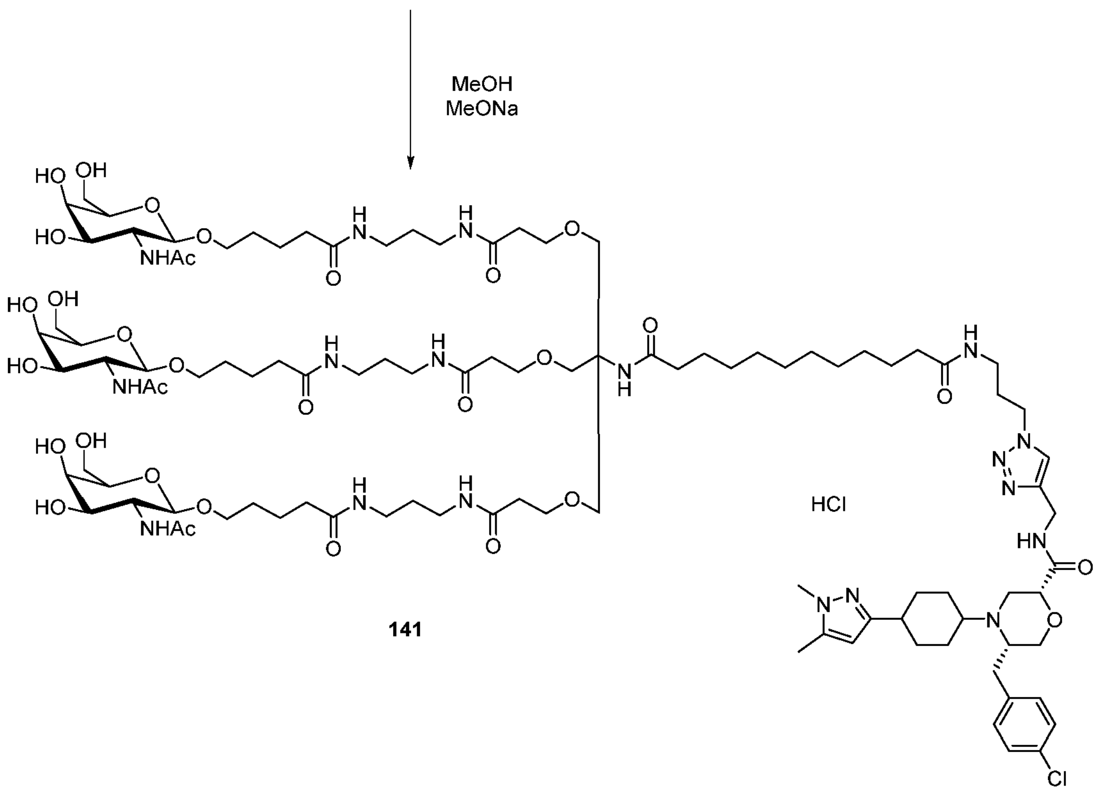


141

To the cooled to 0 °C solution of **141a** (13 mg; 0.0050 mmol) in MeOH (0.9 mL) sodium methoxide (2.5 mg; 0.0460 mmol) was added in one portion. Then the cooling bath was removed and another portion of ) sodium methoxide (3 mg; 0.0550 mmol) was added and stirred at room temperature for 1 hour. After analytical control (LC-MS) indicated completion of the reaction, to the reaction mixture Dowex 50WX8 was added until the solution was weakly acidic. The resin was filtered off and washed with MeOH. The filtrate was concentrated *in vacuo* and the pink residue was taken for preparative reversed-phase column chromatography (C-18, water + 0.3‰ HCl (36%)/MeCN, 95:5 to 10:90, 30 min, 20 mL/min). The title compound (**141**) was obtained as a hydrochloride salt as a single isomer in 32% yield (3.5 mg; 0.0016 mmol).
ESI-MS m/z for $C_{102}H_{170}ClN_{18}O_{31}$ found 1090.2 $[(M+2)/2]^+$; $R_t$ = 1.03 min (Method A)

**Example 142.**

Synthesis of (2R,5S)-5-(4-chlorobenzyl)-4-(4-(1,5-dimethyl-1*H*-pyrazol-3-yl)cyclohexyl)-N-(5-(2-(2,6-dioxopiperidin-3-yl)-3-oxoisoindolin-4-yl)pentyl)morpholine-2-carboxamide hydrochloride (**142**).

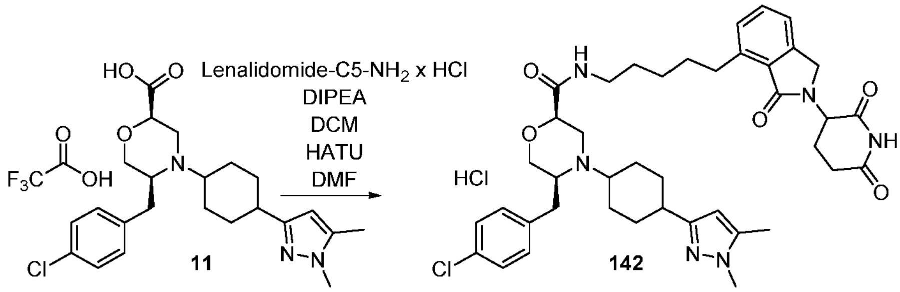

11
142

To the solution of **11** (47 mg; 0.086 mmol) and Lenalidomide-C5-$NH_2$ hydrochloride (37 mg; 0.100 mmol) in dry DMF/DCM (0.05 mL/3 mL) diisopropylethylamine (DIPEA; 70 μL; 0.400 mmol) was added and after 5 minutes HATU (46 mg; 0.120 mmol) was added and the reaction mixture was stirred at room temperature overnight. The reaction progress was monitored by LC-MS. When analysis indicated completion of the reaction, the reaction mixture was diluted with DCM, washed with 0.5 M $HCl_{aq}$ and then concentrated *in vacuo.* The crude product was purified twice by preparative reversed-phase column chromatography (first: C-18, water + 0.3‰ HCl (36%)/MeCN, 95:5 to 10:90, 30 min, 20 mL/min, second: column: Cosmosil Cholester 20 x 250 mm, water + 0.3‰ HCl (36%)/MeCN, 95:5 to 10:90, 30 min, 19 mL/min, Rt = 12.5 - 14.9 min). The obtained solid of the hydrochloride salt was then lyophilized. The title compound (**142**) was obtained as a hydrochloride salt as a single isomer in 37% yield (25 mg; 0.032 mmol).
ESI-MS m/z for $C_{41}H_{52}ClN_6O_5$ found 743.6/745.5 $[M+H]^+$; Rt = 1.35 min (Method A); $^1H$ NMR (700 MHz, DMSO-$d_6$ + $D_2O$) δ 7.56 - 7.55 (m, 1H), 7.45 - 7.44 (m, 2H), 7.40 (d, $J_{AA'}$ = 8.3 Hz, 2H), 7.33 (d, $J_{BB'}$ = 8.3 Hz, 2H), 5.86 (s, 1H), 5.05 (dd, *J* = 13.3 Hz, *J* = 5.1 Hz, 1H), 4.45 (d, *J* = 17.1 Hz, 1H), 4.30 (d, *J* = 17.1 Hz, 1H), 4.27 - 4.23 (m, 1H), 3.75 - 3.67 (m, 2H), 3.60 (s, 3H), 3.59 - 3.44 (m, 2H), 3.17 - 3.03 (m, 5H), 2.88 - 2.83 (m, 1H), 2.63 - 2.60 (m, 3H), 2.47 - 2.37 (m, 2H), 2.25 - 2.19 (m, 2H), 2.16 (s, 3H), 2.03 - 1.99 (m, 3H), 1.61 - 1.38 (m, 8H), 1.30 - 1.27 (m, 2H).

## Claims

1. A compound represented by structural formula (I):

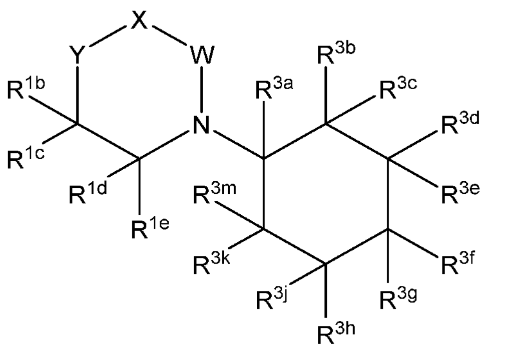
(I)

wherein:

W represents -C(-$R^{1f}$)(-$R^{18}$)-;
X represents -C(-$R^{1h}$)(-$R^{1j}$)-;
Y represents -C(-$R^{1k}$)(-$R^{1m}$)-, -N(-$R^{1k}$)-, or -O-;
$R^{1b}$ represents -H;
$R^{1c}$ represents -H;
$R^{1d}$ represents 4-$R^{10}$-benzyl;
$R^{1e}$ represents -H;
$R^{1f}$ represents -H;

$R^{1g}$ represents -H;

$R^{1h}$ is selected from the group consisting of $C_1$-$C_6$ alkyl, $C_2$-$C_6$ alkenyl, $C_2$-$C_6$ alkynyl, hydroxy-$C_1$-$C_6$ alkyl, azido-$C_1$-$C_6$ alkyl, hydroxycarbonyl-$C_1$-$C_3$ alkoxy-$C_1$-$C_6$ alkyl, $C_3$-$C_6$ alkynyloxy-$C_1$-$C_6$ alkyl, halo-$C_1$-$C_6$ alkyl, $C_1$-$C_3$ alkylthio-$C_1$-$C_6$ alkyl, $C_1$-$C_3$ alkylsulfinyl-$C_1$-$C_6$ alkyl, $C_1$-$C_3$ alkylsulfonyl-$C_1$-$C_6$ alkyl, hydroxycarbonyl-$C_1$-$C_3$ alkylsulfonyl-$C_1$-$C_6$ alkyl, hydroxycarbonyl-$C_1$-$C_6$ alkyl, $C_1$-$C_3$ alkylcarbonyl-$C_1$-$C_6$ alkyl, $C_1$-$C_3$ alkylaminocarbonyl-$C_1$-$C_6$ alkyl, di($C_1$-$C_3$ alkyl)aminocarbonyl-$C_1$-$C_6$ alkyl, $C_1$-$C_3$ alkylsulfonylamino-$C_1$-$C_6$ alkyl, halo-$C_1$-$C_3$ alkylsulfonylamino-$C_1$-$C_6$ alkyl, 5- or 6-membered monocyclic heteroaryl-carbonylamino-$C_1$-$C_6$ alkyl, 5- or 6-membered monocyclic heteroaryl-$C_1$-$C_3$ alkoxy-$C_1$-$C_6$ alkyl, 5- or 6-membered monocyclic heteroaryl-$C_1$-$C_6$ alkyl, hydroxycarbonyl, $C_1$-$C_3$ alkoxycarbonyl, aminocarbonyl, $C_1$-$C_3$ alkylaminocarbonyl, di($C_1$-$C_3$ alkyl)aminocarbonyl, $C_3$-$C_6$ alkynylaminocarbonyl, hydroxycarbonyl-$C_1$-$C_3$ alkyl($C_1$-$C_3$ alkyl)aminocarbonyl, (biotinyl-$PEG_{11}$)aminocarbonyl, (biotinyl-$PEG_{11}$)aminocarbonyl-$C_1$-$C_3$ alkylsulfonyl-$C_1$-$C_6$ alkyl, hydroxylaminocarbonyl, *N*[2]-acetylhydrazinecarbonyl, (5- or 6-membered monocyclic heteroaryl)-$C_1$-$C_3$ alkylaminocarbonyl, (5- or 6-membered monocyclic heteroaryl)aminocarbonyl, $C_1$-$C_3$ alkylsulfonyl, 5- or 6-membered monocyclic heteroaryl, (3-azabicyclo[3.1.0]hexan-3-yl)-$C_1$-$C_3$ alkyl, (4-, 5-, or 6-membered heterocyclyl)amino-$C_1$-$C_3$ alkyl, hydroxycarbonyl-$C_1$-$C_6$ alkyl-(5- or 6-membered monocyclic heteroaryl), $R^{20}$, and 5-(2-(2,6-dioxopiperidin-3-yl)-3-oxoisoindolin-4-yl)pentyl,

where any available position of any heteroaryl ring in $R^{1h}$ can be substituted with a substituent selected from the group consisting of $C_1$-$C_6$ alkyl, $C_3$-$C_6$ alkynyl, halo, trifluoromethyl, 2,2,2-trifluoroethyl, biotinyl-$PEG_{11}$, and any available position of any carbon chain in $R^{1h}$ excluding heteroaryl rings can be substituted with a substituent selected from halogen, amino, hydroxy, and oxo;

$R^{1j}$ represents -H;

$R^{1k}$ represents -H;

$R^{1m}$ represents -H;

or, $R^{1k}$ and $R^{1h}$ taken along with nitrogen and carbon atoms bearing them, respectively, represent a 6-membered heterocyclic ring of structural formula (II):

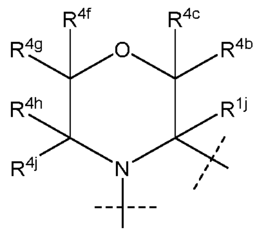

(II)

$R^{3a}$ represents -H;

$R^{3b}$ represents -H;

$R^{3c}$ represents -H;

$R^{3d}$ represents -H;

$R^{3e}$ represents -H;

$R^{3f}$ is selected from the group consisting of 1-methyl-1*H*-imidazol-4-yl, 1,2-dimethyl-1*H*-imidazol-4-yl, 5-methyloxazol-2-yl, 4,5-dimethyloxazol-2-yl, 1*H*-pyrazol-1-yl, 1-methyl-1*H*-pyrazol-3-yl, 4-methyl-1*H*-pyrazol-1-yl, 4-fluoro-1*H*-pyrazol-1-yl, 1-isopropyl-1*H*-pyrazol-3-yl, 5-methyl-1*H*-pyrazol-3-yl, 1,5-dimethyl-1*H*-pyrazol-3-yl, 1-ethyl-5-methyl-1*H*-pyrazol-3-yl, 1,5-dimethyl-1*H*-pyrazol-3-yl, 1,5-dimethyl-1*H*-pyrazol-4-yl, 1-methyl-5-(trifluoromethyl)-1*H*-pyrazol-3-yl, 1-methyl-2-cyano-1*H*-pyrrol-4-yl, 1*H*-tetrazol-5-yl, 2-methyl-2*H*-tetrazol-5-yl, 5-methyl-1,3,4-thiadiazol-2-yl, 5-methylthiazol-2-yl, 4,5-dimethylthiophen-2-yl, 1*H*-1,2,4-triazol-1-yl, 1-methyl-1*H*-1,2,3-triazol-4-yl, 1-methyl-1*H*-1,2,4-triazol-3-yl, 2-methyl-2*H*-1,2,3-triazol-4-yl, 1,5-dimethyl-1*H*-1,2,4-triazol-3-yl, 5-hydroxy-1-methyl-1*H*-1,2,4-triazol-3-yl, and 5-methoxy-1-methyl-1*H*-1,2,4-triazol-3-yl;

$R^{3g}$ represents -H;

$R^{3h}$ represents -H;

$R^{3j}$ represents -H;

$R^{3k}$ represents -H;

$R^{3m}$ represents -H;

or $R^{3d}$ and $R^{3g}$ taken along with the single bond linking two carbon atoms bearing them represent a double bond between these two carbon atoms;

$R^{4b}$ represents -H;

$R^{4c}$ represents -H;

$R^{4f}$ represents -H;
$R^{4g}$ represents -H;
$R^{4h}$ represents -H;
$R^{4j}$ represents -H;
$R^{10}$ represents halogen or trifluoromethyl;
and $R^{20}$ represents

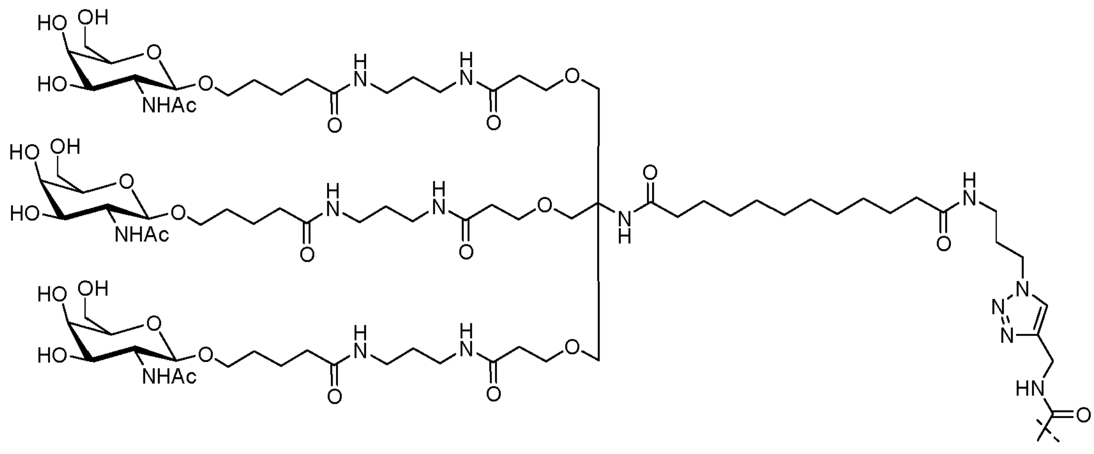

;

or a tautomer, stereoisomer, *N*-oxide, methiodide, pharmaceutically acceptable salt, solvate, or polymorph thereof.

**2.** Compound according to claim 1, represented by structural formula (I):

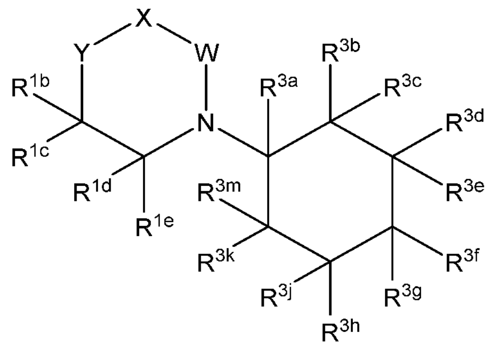

(I)

wherein:

W represents $-C(-R^{1f})(-R^{1g})-$;
X represents $-C(-R^{1h})(-R^{1j})-$;
Y represents $-C(-R^{1k})(-R^{1m})-$, $-N(-R^{1k})-$, or -O-;
$R^{16}$ represents -H;
$R^{1c}$ represents -H;
$R^{1d}$ represents 4-$R^{10}$-benzyl;
$R^{1e}$ represents -H;
$R^{1f}$ represents -H;
$R^{1g}$ represents -H;
$R^{1h}$ is selected from the group consisting of $C_1$-$C_6$ alkyl, $C_2$-$C_6$ alkenyl, $C_2$-$C_6$ alkynyl, hydroxy-$C_1$-$C_6$ alkyl, azido-$C_1$-$C_6$ alkyl, hydroxycarbonyl-$C_1$-$C_3$ alkoxy-$C_1$-$C_6$ alkyl, $C_3$-$C_6$ alkynyloxy-$C_1$-$C_6$ alkyl, halo-$C_1$-$C_6$ alkyl, $C_1$-$C_3$ alkylthio-$C_1$-$C_6$ alkyl, $C_1$-$C_3$ alkylsulfinyl-$C_1$-$C_6$ alkyl, $C_1$-$C_3$ alkylsulfonyl-$C_1$-$C_6$ alkyl, hydroxycarbonyl-$C_1$-$C_3$ alkylsulfonyl-$C_1$-$C_6$ alkyl, hydroxycarbonyl-$C_1$-$C_6$ alkyl, $C_1$-$C_3$ alkylcarbonyl-$C_1$-$C_6$ alkyl, $C_1$-$C_3$ alkylaminocarbonyl-$C_1$-$C_6$ alkyl, di($C_1$-$C_3$ alkyl)aminocarbonyl-$C_1$-$C_6$ alkyl, $C_1$-$C_3$ alkylsulfonylamino-$C_1$-$C_6$ alkyl, halo-$C_1$-$C_3$ alkylsulfonylamino-$C_1$-$C_6$ alkyl, 5- or 6-membered monocyclic heteroaryl-carbonylamino-$C_1$-$C_6$ alkyl, 5- or 6-membered monocyclic heteroaryl-$C_1$-$C_3$ alkoxy-$C_1$-$C_6$ alkyl, 5- or 6-membered monocyclic heteroaryl-$C_1$-$C_6$

alkyl, hydroxycarbonyl, $C_1$-$C_3$ alkoxycarbonyl, aminocarbonyl, $C_1$-$C_3$ alkylaminocarbonyl, di($C_1$-$C_3$ alkyl)aminocarbonyl, $C_3$-$C_6$ alkynylaminocarbonyl, hydroxycarbonyl-$C_1$-$C_3$ alkyl($C_1$-$C_3$ alkyl)aminocarbonyl, (biotinyl-$PEG_{11}$)aminocarbonyl, hydroxylaminocarbonyl, *N*[2]-acetylhydrazinecarbonyl, (5- or 6-membered monocyclic heteroaryl)-$C_1$-$C_3$ alkylaminocarbonyl, (5- or 6-membered monocyclic heteroaryl)aminocarbonyl, $C_1$-$C_3$ alkylsulfonyl, 5- or 6-membered monocyclic heteroaryl, (3-azabicyclo[3.1.0]hexan-3-yl)-$C_1$-$C_3$ alkyl, (4-, 5-, or 6-membered heterocyclyl)amino-$C_1$-$C_3$ alkyl, and hydroxycarbonyl-$C_1$-$C_6$ alkyl-(5- or 6-membered monocyclic heteroaryl),

where any available position of any heteroaryl ring in $R^{1h}$ can be substituted with a substituent selected from the group consisting of $C_1$-$C_6$ alkyl, $C_3$-$C_6$ alkynyl, halo, trifluoromethyl, 2,2,2-trifluoroethyl, biotinyl-$PEG_{11}$, and any available position of any carbon chain in $R^{1h}$ excluding heteroaryl rings can be substituted with a substituent selected from halogen, amino, hydroxy, and oxo;

$R^{1j}$ represents -H;

$R^{1k}$ represents -H;

$R^{1m}$ represents -H;

or $R^{1k}$ and $R^{1h}$ taken along with nitrogen and carbon atoms bearing them, respectively, represent a 6-membered heterocyclic ring of structural formula (II):

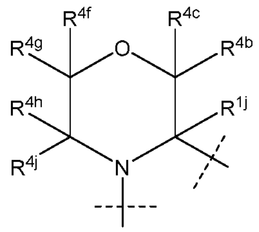

(II)

$R^{3a}$ represents -H;

$R^{3b}$ represents -H;

$R^{3c}$ represents -H;

$R^{3d}$ represents -H;

$R^{3e}$ represents -H;

$R^{3f}$ is selected from the group consisting of 5-methyloxazol-2-yl, 1,5-dimethyl-1*H*-pyrazol-3-yl, 1,5-dimethyl-1*H*-1,2,4-triazol-3-yl, 4,5-dimethyloxazol-2-yl, 5-fluoro-1-methyl-1*H*-pyrazol-3-yl, 4,5-dimethylthiazol-2-yl, and 5-methylthiazol-2-yl;

$R^{3g}$ represents -H;

$R^{3h}$ represents -H;

$R^{3j}$ represents -H;

$R^{3k}$ represents -H;

$R^{3m}$ represents -H;

or $R^{3d}$ and $R^{3g}$ taken along with the single bond linking two carbon atoms bearing them represent a double bond between these two carbon atoms;

$R^{4b}$ represents -H;

$R^{4c}$ represents -H;

$R^{4f}$ represents -H;

$R^{4g}$ represents -H;

$R^{4h}$ represents -H;

$R^{4j}$ represents -H;

and $R^{10}$ represents halogen or trifluoromethyl;

or a tautomer, stereoisomer, *N*-oxide, methiodide, pharmaceutically acceptable salt, solvate, or polymorph thereof.

**3.** Compound according to claim 1 or 2, having the structural formula (Ia):

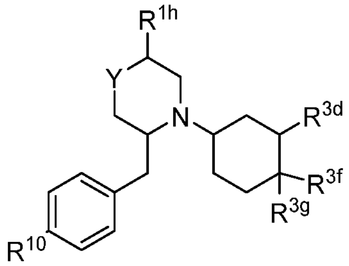

(Ia)

wherein:

Y represents $-CH_2-$, $-N(-R^{1k})-$, or -O-;
$R^{1h}$ is selected from the group consisting of methyl, ethynyl, 2-hydroxyprop-2-yl, azidomethyl, hydroxymethyl, hydroxycarbonylmethoxymethyl, (prop-2-yn-1-yloxy)methyl, fluoromethyl, methylthiomethyl, methylsulfinylmethyl, methylsulfonylmethyl, ethylsulfonylmethyl, 2-(hydroxycarbonyl)ethylsulfonylmethyl, hydroxycarbonylmethyl, methoxycarbonylmethyl, ethylaminocarbonylmethyl, methylsulfonylaminomethyl, trifluoromethylsulfonylaminomethyl, (1*H*-pyrazol-3-yl)carbonylaminomethyl, 1-(trifluoromethyl)-1*H*-1,2,3-triazol-4-yl, (1-(biotinyl-$PEG_{11}$)-1*H*-1,2,3-triazol-4-yl)methoxymethyl, (2*H*-1,2,3-triazol-4-yl)carbonylaminomethyl, (1*H*-1,2,3-triazol-5-yl)carbonylaminomethyl, (1-methyl-1*H*-imidazol-4-yl)carbonylaminomethyl, (1*H*-imidazol-4-yl)carbonylaminomethyl, (1,5-dimethyl-1*H*-pyrazol-3-yl)carbonylaminomethyl, (2,5-dioxopyrrolidin-1-yl)methyl, (2,4-dioxoimidazolidin-3-yl)methyl, (1-methyl-2,4-dioxoimidazolidin-3-yl)methyl, hydroxycarbonyl, methoxycarbonyl, aminocarbonyl, methylaminocarbonyl, dimethylaminocarbonyl, ethylaminocarbonyl, isopropylaminocarbonyl, (prop-2-yn-1-yl)aminocarbonyl, hydroxycarbonylmethyl(methyl)aminocarbonyl, (biotinyl-$PEG_{11}$)aminocarbonyl, hydroxylaminocarbonyl, *N*[2]-acetylhydrazinecarbonyl, (1-(biotinyl-$PEG_{11}$)-1*H*-1,2,3-triazol-4-yl)methylaminocarbonyl, (1-methyl-1*H*-imidazol-4-yl)aminocarbonyl, (1,5-dimethyl-1*H*-pyrazol-3-yl)aminocarbonyl, (2-(3-(but-3-yn-1-yl)-3*H*-diazirin-3-yl)ethyl)aminocarbonyl, methylsulfonyl, 3-methylisoxazol-5-yl, 1-(2,2,2-trifluoroethyl)-1*H*-1,2,3-triazol-4-yl, 5-methyl-1,3,4-oxadiazol-2-yl, (6,6-dimethyl-2,4-dioxo-3-azabicyclo[3.1.0]hexan-3-yl)methyl, (1,2-dioxo-3-hydroxy-cyclobut-3-en-4-yl)aminomethyl, (1,2-dioxo-3-amino-cyclobut-3-en-4-yl) aminomethyl, 1-(biotinyl-$PEG_{11}$)-1*H*-1,2,3-triazol-4-yl, 1-(hydroxycarbonylmethyl)-1*H*-1,2,3-triazol-4-yl, 1-(2-(hydroxycarbonyl)ethyl)-1*H*-1,2,3-triazol-4-yl, 1-(3-(hydroxycarbonyl)prop-1-yl)-1*H*-1,2,3-triazol-4-yl, 1-(4-(hydroxycarbonyl)but-1-yl)-1*H*-1,2,3-triazol-4-yl, and 5-methyl-1*H*-pyrazol-3-yl;
or $R^{1k}$ and $R^{1h}$ taken along with nitrogen and carbon atoms bearing them, respectively, represent a 6-membered heterocyclic ring of structural formula (IIa):

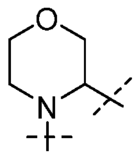

(IIa)

$R^{3d}$ represents H;
$R^{3g}$ represents H;
or $R^{3d}$ and $R^{3g}$ taken along with the single bond linking two carbon atoms bearing them represent a double bond between these two carbon atoms;
$R^{3f}$ is selected from the group consisting of 5-methyloxazol-2-yl, 1,5-dimethyl-1*H*-pyrazol-3-yl, 1,5-dimethyl-1*H*-1,2,4-triazol-3-yl, 4,5-dimethyloxazol-2-yl, 5-fluoro-1-methyl-1*H*-pyrazol-3-yl, 4,5-dimethylthiazol-2-yl, and 5-methylthiazol-2-yl;
and $R^{10}$ represents chloro, bromo, or trifluoromethyl;
or a tautomer, stereoisomer, pharmaceutically acceptable salt, solvate, or polymorph thereof.

**4.** Compound according to claim 1 or 2 or 3, wherein:

X represents $-CH(-R^{1h})-$;
Y represents $-CH_2-$; and
$R^{1h}$ represents methanesulfonyl.

**5.** Compound according to claim 1 or 2 or 3, wherein:

X represents -CH(-$R^{1h}$)-.
Y represents -N(-$R^{1k}$)-.
and $R^{1k}$ and $R^{1h}$ taken along with nitrogen and carbon atoms bearing them, respectively, represent a 6-membered heterocyclic ring of structural formula (IIb):

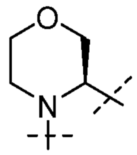

(IIb).

**6.** Compound according to claim 1 or 2 or 3, wherein Y represents -O-.

**7.** Compound according to any one of claims 1 to 6, wherein $R^{1d}$ and $R^{1h}$ are in *cis* relationship.

**8.** Compound according to any one of claims 1 to 7, wherein the absolute stereochemistry at the carbon atom bearing $R^{1d}$ is S.

**9.** Compound according to any one of claims 1, 2, 3, 6, 7, and 8, having the structural formula (Ib):

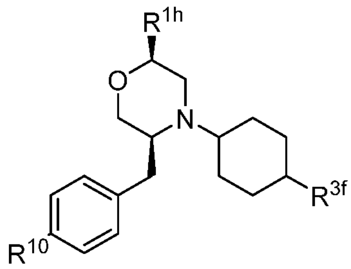

(Ib)

wherein:

$R^{1h}$ is selected from the group consisting of methyl, ethynyl, 2-hydroxyprop-2-yl, azidomethyl, hydroxymethyl, hydroxycarbonylmethoxymethyl, (prop-2-yn-1-yloxy)methyl, fluoromethyl, methylthiomethyl, methylsulfinylmethyl, methylsulfonylmethyl, ethylsulfonylmethyl, 2-(hydroxycarbonyl)ethylsulfonylmethyl, hydroxycarbonylmethyl, methoxycarbonylmethyl, ethylaminocarbonylmethyl, methylsulfonylaminomethyl, trifluoromethylsulfonylaminomethyl, (1*H*-pyrazol-3-yl)carbonylaminomethyl, 1-(trifluoromethyl)-1*H*-1,2,3-triazol-4-yl, (1-(biotinyl-$PEG_{11}$)-1*H*-1,2,3-triazol-4-yl)methoxymethyl, (2*H*-1,2,3-triazol-4-yl)carbonylaminomethyl, (1*H*-1,2,3-triazol-5-yl)carbonylaminomethyl, (1-methyl-1*H*-imidazol-4-yl)carbonylaminomethyl, (1*H*-imidazol-4-yl)carbonylaminomethyl, (1,5-dimethyl-1*H*-pyrazol-3-yl)carbonylaminomethyl, (2,5-dioxopyrrolidin-1-yl)methyl, (2,4-dioxoimidazolidin-3-yl)methyl, (1-methyl-2,4-dioxoimidazolidin-3-yl)methyl, hydroxycarbonyl, methoxycarbonyl, aminocarbonyl, methylaminocarbonyl, dimethylaminocarbonyl, ethylaminocarbonyl, isopropylaminocarbonyl, (prop-2-yn-1-yl)aminocarbonyl, (biotinyl-$PEG_{11}$)aminocarbonyl, hydroxylaminocarbonyl, $N^2$-acetylhydrazinecarbonyl, (1-(biotinyl-$PEG_{11}$)-1*H*-1,2,3-triazol-4-yl)methylaminocarbonyl, (1-methyl-1*H*-imidazol-4-yl)aminocarbonyl, (1,5-dimethyl-1H-pyrazol-3-yl)aminocarbonyl, (2-(3-(but-3-yn-1-yl)-3*H*-diazirin-3-yl)ethyl)aminocarbonyl, 3-methylisoxazol-5-yl, 1-(2,2,2-trifluoroethyl)-1*H*-1,2,3-triazol-4-yl, 5-methyl-1,3,4-oxadiazol-2-yl, (6,6-dimethyl-2,4-dioxo-3-azabicyclo[3.1.0]hexan-3-yl)methyl, (1,2-dioxo-3-hydroxy-cyclobut-3-en-4-yl)aminomethyl, (1,2-dioxo-3-amino-cyclobut-3-en-4-yl)aminomethyl, 1-(biotinyl-$PEG_{11}$)-1*H*-1,2,3-triazol-4-yl, 1-(hydroxycarbonylmethyl)-1*H*-1,2,3-triazol-4-yl, 1-(2-(hydroxycarbonyl)ethyl)-1*H*-1,2,3-triazol-4-yl, 1-(3-(hydroxycarbonyl)prop-1-yl)-1*H*-1,2,3-triazol-4-yl, 1-(4-(hydroxycarbonyl)but-1-yl)-1*H*-1,2,3-triazol-4-yl, and 5-methyl-1*H*-pyrazol-3-yl; and
$R^{3f}$ is selected from the group consisting of 5-methyloxazol-2-yl, 1,5-dimethyl-*1*H-pyrazol-3-yl, 1,5-dimethyl-1*H*-1,2,4-triazol-3-yl, 4,5-dimethyloxazol-2-yl, 5-fluoro-1-methyl-1*H*-pyrazol-3-yl, 4,5-dimethylthiazol-2-yl, and 5-methylthiazol-2-yl;
or a tautomer, stereoisomer, pharmaceutically acceptable salt, solvate, or polymorph thereof.

**10.** Compound according to any one of claims 1, 2, 3, 6, 7, 8, and 9, wherein $R^{10}$ is bromo.

**11.** Compound according to any one of claims 1 to 9, wherein $R^{10}$ is chloro.

**12.** Compound according to any one of claims 1 to 11, wherein $R^{3f}$ is 5-methyloxazol-2-yl.

**13.** Compound according to any one of claims 1 to 11, wherein $R^{3f}$ is 1,5-dimethyl-1*H*-pyrazol-3-yl.

**14.** Compound according to any one of claims 1 to 11, wherein $R^{3f}$ is 1,5-dimethyl-1*H*-1,2,4-triazol-3-yl.

**15.** Compound according to any one of claims 1, 2, 3, 6, 7, 8, 9, and 11, wherein $R^{3f}$ is 4,5-dimethyloxazol-2-yl.

**16.** Compound according to any one of claims 1, 2, 3, 6, 7, 8, 9, and 11, wherein $R^{3f}$ is 5-fluoro-1-methyl-1*H*-pyrazol-3-yl.

**17.** Compound according to any one of claims 1 to 11, wherein $R^{3f}$ is 4,5-dimethylthiazol-2-yl.

**18.** Compound according to any one of claims 1, 2, 3, 6, 7, 8, 9, and 11, wherein $R^{3f}$ is 5-methylthiazol-2-yl.

**19.** Compound according to any one of claims 1 to 18, wherein

$R^{3a}$ represents H;
$R^{3g}$ represents H; and
the $R^{3f}$ substituent and the heterocyclic ring attached by its nitrogen ring member atom to the cyclohexane ring in 1,4 substitution pattern are in *trans* relationship.

**20.** Compound according to claim 1, selected from the group consisting of the following compounds:

(2*S*,5*S*)-5-(4-chlorobenzyl)-2-methyl-4-(4-(5-methyloxazol-2-yl)cyclohexyl)morpholine;
(2*S*,5*S*)-5-(4-chlorobenzyl)-4-(4-(1,5-dimethyl-1*H*-pyrazol-3-yl)cyclohexyl)-2-methylmorpholine;
(2*R*,5*S*)-5-(4-chlorobenzyl)-4-(4-(5-methyloxazol-2-yl)cyclohexyl)-2-((methylsulfonyl)methyl)morpholine;
(2*R*,5*S*)-5-(4-chlorobenzyl)-4-((1*r*,4*S*)-4-(1,5-dimethyl-1*H*-pyrazol-3-yl)cyclohexyl)-2-((methylsulfonyl)methylymorpholine;
(2*R*,5*S*)-5-(4-chlorobenzyl)-4-(4-(1,5-dimethyl-1*H*-pyrazol-3-yl)cyclohexyl)-2-(3-methylisoxazol-5-yl)morpholine;
(7*S*,9a*R*)-7-(4-chlorobenzyl)-8-(4-(5-methyloxazol-2-yl)cyclohexyl)octahydropyrazino[2,1-c][1,4]oxazine;
(7*S*,9a*R*)-7-(4-chlorobenzyl)-8-(4-(1,5-dimethyl-1*H*-pyrazol-3-yl)cyclohexyl)octahydropyrazino[2,1-*c*][1,4]oxazine;
(2*R*,5*S*)-5-(4-chlorobenzyl)-4-(4-(5-methyloxazol-2-yl)cyclohexyl)morpholine-2-carboxylic acid;
methyl (2*R*,5*S*)-5-(4-chlorobenzyl)-4-(4-(1,5-dimethyl-1*H*-pyrazol-3-yl)cyclohexyl)morpholine-2-carboxylate;
(2*R*,5*S*)-5-(4-chlorobenzyl)-4-(4-(1,5-dimethyl-1*H*-pyrazol-3-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholine;
(2*R*,5*S*)-5-(4-chlorobenzyl)-4-(4-(5-methyloxazol-2-yl)cyclohexyl)-2-(1-(2,2,2-trifluoroethyl)-1*H*-1,2,3-triazol-4-yl)morpholine;
(2*R*,5*S*)-5-(4-chlorobenzyl)-4-(4-(1,5-dimethyl-1*H*-pyrazol-3-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl) morpholine-2-carboxamide;
(2*R*,5*S*)-5-(4-chlorobenzyl)-4-(4-(1,5-dimethyl-1*H*-1,2,4-triazol-3-yl)cyclohexyl)-2-((methylsulfonyl)methyl) morpholine;
(2*R*,5*S*)-5-(4-chlorobenzyl)-4-(4-(1,5-dimethyl-1*H*-pyrazol-3-yl)cyclohexyl)-2-(fluoromethyl)morpholine;
((2*R*,5*S*)-5-(4-chlorobenzyl)-4-(4-(1,5-dimethyl-1*H*-pyrazol-3-yl)cyclohexyl)morpholin-2-yl)methanol;
(2*R*,5*S*)-5-(4-chlorobenzyl)-*N*-ethyl-4-(4-(5-methyloxazol-2-yl)cyclohexyl)morpholine-2-carboxamide;
(2*R*,5*S*)-5-(4-chlorobenzyl)-4-(4-(1,5-dimethyl-1*H*-pyrazol-3-yl)cyclohexyl)-*N*-ethylmorpholine-2-carboxamide;
(2*R*,5*S*)-5-(4-chlorobenzyl)-4-(4-(1,5-dimethyl-1*H*-pyrazol-3-yl)cyclohexyl)-*N,N*-dimethylmorpholine-2-carboxamide;
(2R,5S)-5-(4-chlorobenzyl)-*N,N*-dimethyl-4-(4-(5-methyloxazol-2-yl)cyclohexyl)morpholine-2-carboxamide;
(2*R*,5*S*)-5-(4-chlorobenzyl)-4-(4-(1,5-dimethyl-1*H*-pyrazol-3-yl)cyclohexyl)-2-(5-methyl-1,3,4-oxadiazol-2-yl) morpholine;
(2*R*,5*S*)-*N*'-acetyl-5-(4-chlorobenzyl)-4-(4-(1,5-dimethyl-1*H*-pyrazol-3-yl)cyclohexyl)morpholine-2-carbohydrazide;
(2*R*,5*S*)-5-(4-chlorobenzyl)-2-(5-methyl-1,3,4-oxadiazol-2-yl)-4-(4-(5-methyloxazol-2-yl)cyclohexyl)morpholine;
(2*R*,5*S*)-*N'*-acetyl-5-(4-chlorobenzyl)-4-(4-(5-methyloxazol-2-yl)cyclohexyl)morpholine-2-carbohydrazide;
(2*R*,5*S*)-5-(4-chlorobenzyl)-4-(4-(4,5-dimethyloxazol-2-yl)cyclohexyl)-2-((methylsulfonyl)methyl)morpholine;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1,5-dimethyl-1*H*-pyrazole-3-carboxamide;
(2*S*,5*S*)-5-(4-chlorobenzyl)-4-(4-(1,5-dimethyl-1*H*-pyrazol-3-yl)cyclohexyl)-2-ethynylmorpholine;
*N*-(35-(4-((2*R*,5*S*)-5-(4-chlorobenzyl)-4-(4-(1,5-dimethyl-1*H*-pyrazol-3-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
(2*R*,5*S*)-5-(4-chlorobenzyl)-4-(4-(1,5-dimethyl-1*H*-pyrazol-3-yl)cyclohexyl)-*N*-isopropylmorpholine-2-carboxamide;
(2*R*,5*S*)-5-(4-chlorobenzyl)-4-(4-(1,5-dimethyl-1*H*-pyrazol-3-yl)cyclohexyl)-*N*-methylmorpholine-2-carboxamide;
(2*R*,5*S*)-5-(4-chlorobenzyl)-4-(4-(4,5-dimethyloxazol-2-yl)cyclohexyl)-*N*,*N*-dimethylmorpholine-2-carboxamide;
(2*R*,5*S*)-5-(4-chlorobenzyl)-4-(4-(4,5-dimethyloxazol-2-yl)cyclohexyl)-*N*-isopropylmorpholine-2-carboxamide;
(2*R*,5*S*)-5-(4-chlorobenzyl)-*N*-methyl-4-(4-(5-methyloxazol-2-yl)cyclohexyl)morpholine-2-carboxamide;
(2*R*,5*S*)-5-(4-chlorobenzyl)-4-(4-(4,5-dimethyloxazol-2-yl)cyclohexyl)-*N*-ethylmorpholine-2-carboxamide;
(2*R*,5*S*)-5-(4-chlorobenzyl)-4-(4-(5-fluoro-1-methyl-1*H*-pyrazol-3-yl)cyclohexyl)-2-((methylsulfonyl)methyl) morpholine;
(2*R*,5*S*)-5-(4-chlorobenzyl)-4-(4-(4,5-dimethylthiazol-2-yl)cyclohexyl)-2-((methylsulfonyl)methyl)morpholine;
(2*R*,5*S*)-5-(4-chlorobenzyl)-2-((methylsulfonyl)methyl)-4-(4-(5-methylthiazol-2-yl)cyclohexyl)morpholine;
(2*R*,5*S*)-5-(4-chlorobenzyl)-4-(4-(4,5-dimethyloxazol-2-yl)cyclohexyl)morpholine-2-carboxylic acid;
(2*R*,5*S*)-5-(4-chlorobenzyl)-4-(4-(4,5-dimethylthiazol-2-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)-2-((prop-2-yn-1-yloxy)methyl)morpholine;
(2*R*,5*S*)-5-(4-chlorobenzyl)-4-(4-(4,5-dimethylthiazol-2-yl)cyclohexyl)-*N*-ethylmorpholine-2-carboxamide;
*N*-(35-(4-((((2*R*,5*S*)-5-(4-chlorobenzyl)-4-(4-(1,5-dimethyl-1*H*-pyrazol-3-yl)cyclohexyl)morpholin-2-yl)methoxy)methyl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
(2*R*,5*S*)-2-(azidomethyl)-5-(4-chlorobenzyl)-4-(4-(1,5-dimethyl-1*H*-pyrazol-3-yl)cyclohexyl)morpholine;
(2*R*,5*S*)-5-(4-chlorobenzyl)-4-(4-(1,5-dimethyl-1*H*-pyrazol-3-yl)cyclohexyl)-*N*-(prop-2-yn-1-yl)morpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-pyrazole-3-carboxamide;
(2*R*,5*S*)-5-(4-chlorobenzyl)-4-(4-(1,5-dimethyl-1*H*-pyrazol-3-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl) methyl)-2*H*-1,2,3-triazole-4-carboxamide;
3-(((2*R*,5*S*)-5-(4-chlorobenzyl)-4-(4-(1,5-dimethyl-1*H*-pyrazol-3-yl)cyclohexyl)morpholin-2-yl)methyl)imidazolidine-2,4-dione;

(2*R*,5*S*)-5-(4-chlorobenzyl)-4-(4-(1,5-dimethyl-1*H*-1,2,4-triazol-3-yl)cyclohexyl)-*N*-ethylmorpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-imidazole-4-carboxamide;
(2*R*,5*S*)-5-(4-chlorobenzyl)-4-(4-(4,5-dimethylthiazol-2-yl)cyclohex-3-en-1-yl)-2-((methylsulfonyl)methyl)morpholine;
(2*R*,5*S*)-5-(4-chlorobenzyl)-4-(4-(4,5-dimethylthiazol-2-yl)cyclohex-3-en-1-yl)-*N*-ethylmorpholine-2-carboxamide;
(2*R*,5*S*)-5-(4-chlorobenzyl)-4-(4-(4,5-dimethylthiazol-2-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)morpholine-2-carboxamide;
(2*R*,5*S*)-2-(azidomethyl)-5-(4-chlorobenzyl)-4-(4-(4,5-dimethylthiazol-2-yl)cyclohexyl)morpholine;
(2*R*,5*S*)-5-(4-chlorobenzyl)-4-(4-(4,5-dimethylthiazol-2-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide;
*N*-(((2*S*,5*S*)-5-(4-chlorobenzyl)-4-(4-(4,5-dimethylthiazol-2-yl)cyclohexyl)morpholin-2-yl)methyl)-1*H*-1,2,3-triazole-5-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1-methyl-1*H*-imidazole-4-carboxamide;
methyl 2-((2*S*,5*S*)-5-(4-chlorobenzyl)-4-(4-(1,5-dimethyl-1*H*-pyrazol-3-yl)cyclohexyl)morpholin-2-yl)acetate;
2-((2*S*,5*S*)-5-(4-chlorobenzyl)-4-(4-(1,5-dimethyl-1*H*-pyrazol-3-yl)cyclohexyl)morpholin-2-yl)acetic acid;
2-((2*S*,5*S*)-5-(4-chlorobenzyl)-4-(4-(1,5-dimethyl-1*H*-pyrazol-3-yl)cyclohexyl)morpholin-2-yl)-*N*-ethylacetamide;
(2*R*,5*S*)-5-(4-chlorobenzyl)-4-(4-(1,5-dimethyl-1*H*-1,2,4-triazol-3-yl)cyclohexyl)morpholine-2-carboxylic acid;
(2*R*,5*S*)-5-(4-bromobenzyl)-4-(4-(1,5-dimethyl-1*H*-pyrazol-3-yl)cyclohexyl)-2-((methylsulfonyl)methyl)morpholine;
*N*-(((2*R*,5*S*)-5-(4-chlorobenzyl)-4-(4-(1,5-dimethyl-1*H*-pyrazol-3-yl)cyclohexyl)morpholin-2-yl)methyl)methanesulfonamide;
(2*R*,5*S*)-5-(4-bromobenzyl)-4-(4-(1,5-dimethyl-1*H*-pyrazol-3-yl)cyclohexyl)morpholine-2-carboxylic acid;
(2*R*,5*S*)-5-(4-bromobenzyl)-4-(4-(5-methyloxazol-2-yl)cyclohexyl)morpholine-2-carboxylic acid;
(2*R*,5*S*)-5-(4-bromobenzyl)-4-(4-(5-methyloxazol-2-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)-2-((methylsulfonyl)methyl) morpholine;
(2*R*,5*S*)-5-(4-bromobenzyl)-4-(4-(1,5-dimethyl-1*H*-1,2,4-triazol-3-yl)cyclohexyl)morpholine-2-carboxylic acid;
*N*-(((2*R*,5*S*)-5-(4-chlorobenzyl)-4-(4-(1,5-dimethyl-1*H*-pyrazol-3-yl)cyclohexyl)morpholin-2-yl)methyl)-1,1,1-trifluoromethanesulfonamide;
(2*R*,5*S*)-5-(4-bromobenzyl)-4-(4-(1,5-dimethyl-1*H*-pyrazol-3-yl)cyclohexyl)-*N*-ethylmorpholine-2-carboxamide;
(2*R*,5*S*)-*N*-(2-(3-(but-3-yn-1-yl)-3*H*-diazirin-3-yl)ethyl)-5-(4-chlorobenzyl)-4-(4-(1,5-dimethyl-1*H*-pyrazol-3-yl) cyclohexyl)morpholine-2-carboxamide;
2-(((2*R*,5*S*)-5-(4-chlorobenzyl)-4-(4-(4,5-dimethylthiazol-2-yl)cyclohexyl)morpholin-2-yl)methoxy)acetic acid;
2-((2*R*,5*S*)-5-(4-chlorobenzyl)-4-(4-(1,5-dimethyl-1*H*-pyrazol-3-yl)cyclohexyl)morpholin-2-yl)propan-2-ol;
(2*R*,5*S*)-5-(4-chlorobenzyl)-4-(4-(1,5-dimethyl-1*H*-pyrazol-3-yl)cyclohexyl)-2-(5-methyl-1*H*-pyrazol-3-yl)morpholine;
(2*R*,5*R*)-2-(4-chlorobenzyl)-1-(4-(1,5-dimethyl-1*H*-pyrazol-3-yl)cyclohexyl)-5-(methylsulfonyl)piperidine;
(2*R*,5*S*)-5-(4-chlorobenzyl)-4-(4-(1,5-dimethyl-1*H*-pyrazol-3-yl)cyclohexyl)morpholine-2-carboxamide;
2-(4-((2*R*,5*R*)-2-(4-chlorobenzyl)-5-(methylsulfonyl)piperidin-1-yl)cyclohexyl)-5-methyloxazole;
(2*R*,5*S*)-5-(4-chlorobenzyl)-4-(4-(1,5-dimethyl-1*H*-pyrazol-3-yl)cyclohexyl)-2-((methylsulfinyl)methyl)morpholine;
(2*R*,5*S*)-5-(4-chlorobenzyl)-4-(4-(1,5-dimethyl-1*H*-pyrazol-3-yl)cyclohexyl)-2-((methylthio)methyl)morpholine;
1-(((2*R*,5*S*)-5-(4-chlorobenzyl)-4-(4-(1,5-dimethyl-1*H*-pyrazol-3-yl)cyclohexyl)morpholin-2-yl)methyl)pyrrolidine-2,5-dione;
(2*S*,5*S*)-5-(4-chlorobenzyl)-4-(4-(4,5-dimethylthiazol-2-yl)cyclohexyl)-2-ethynylmorpholine;
2-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)acetic acid;
3-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)propanoic acid;
(2*R*,5*S*)-5-(4-chlorobenzyl)-4-(4-(1,5-dimethyl-1*H*-pyrazol-3-yl)cyclohex-3-en-1-yl)-2-((methylsulfonyl)methyl)morpholine;
*N*-(35-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
2-((2*S*,5*S*)-5-(4-chlorobenzyl)-4-(4-(4,5-dimethylthiazol-2-yl)cyclohexyl)morpholin-2-yl)acetic acid;
3-((((2*R*,5*S*)-5-(4-chlorobenzyl)-4-(4-(4,5-dimethylthiazol-2-yl)cyclohexyl)morpholin-2-yl)methyl)sulfonyl)propanoic acid;
3-(((2*R*,5*S*)-5-(4-chlorobenzyl)-4-(4-(1,5-dimethyl-1*H*-pyrazol-3-yl)cyclohexyl)morpholin-2-yl)methyl)-6,6-dimethyl-3-azabicyclo[3.1.0]hexane-2,4-dione;
3-(((2*R*,5*S*)-5-(4-chlorobenzyl)-4-(4-(4,5-dimethylthiazol-2-yl)cyclohexyl)morpholin-2-yl)methyl)-6,6-dimethyl-3-azabicyclo[3.1.0]hexane-2,4-dione;
3-((((2*S*,5*S*)-5-(4-chlorobenzyl)-4-(4-(1,5-dimethyl-1*H*-pyrazol-3-yl)cyclohexyl)morpholin-2-yl)methyl)amino)-4-hydroxycyclobut-3-ene-1,2-dione;
3-amino-4-((((2*S*,5*S*)-5-(4-chlorobenzyl)-4-(4-(1,5-dimethyl-1*H*-pyrazol-3-yl)cyclohexyl)morpholin-2-yl)methyl)amino)cyclobut-3-ene-1,2-dione;
(2*R*,5*S*)-5-(4-chlorobenzyl)-4-(4-(4,5-dimethylthiazol-2-yl)cyclohexyl)-2-((ethylsulfonyl)methyl)morpholine;
(2*R*,5*S*)-5-(4-chlorobenzyl)-4-(4-(1,5-dimethyl-1*H*-pyrazol-3-yl)cyclohexyl)-2-((ethylsulfonyl)methyl)morpholine;
3-(((2*R*,5*S*)-5-(4-chlorobenzyl)-4-(4-(1,5-dimethyl-1*H*-pyrazol-3-yl)cyclohexyl)morpholin-2-yl)methyl)-1-methylimidazolidine-2,4-dione;
(2*R*,5*S*)-5-(4-chlorobenzyl)-4-(4-(4,5-dimethylthiazol-2-yl)cyclohexyl)-*N*-(1-methyl-1*H*-imidazol-4-yl)morpholine-2-carboxamide;
4-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)butanoic acid;
(2*R*,5*S*)-5-(4-chlorobenzyl)-4-(4-(4,5-dimethylthiazol-2-yl)cyclohexyl)-*N*-hydroxymorpholine-2-carboxamide;
(2*R*,5*S*)-5-(4-chlorobenzyl)-*N*-(1,5-dimethyl-1*H*-pyrazol-3-yl)-4-(4-(4,5-dimethylthiazol-2-yl)cyclohexyl)morpholine-2-carboxamide;
5-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)pentanoic acid;
(2*R*,5*S*)-5-(4-chlorobenzyl)-*N*-(1,5-dimethyl-1*H*-pyrazol-3-yl)-4-(4-(1,5-dimethyl-1*H*-pyrazol-3-yl)cyclohexyl) morpholine-2-carboxamide;
*N*-((2*R*,5*S*)-5-(4-chlorobenzyl)-4-(4-(4,5-dimethylthiazol-2-yl)cyclohexyl)morpholine-2-carbonyl)-*N*-methylglycine;
(2*R*,5*S*)-4-(4-(1-methyl-1*H*-1,2,4-triazol-3-yl)cyclohexyl)-2-((methylsulfonyl)methyl)-5-(4-(trifluoromethyl)benzyl)morpholine;
(2*R*,5*S*)-5-(4-chlorobenzyl)-4-(4-(1-methyl-1*H*-imidazol-4-yl)cyclohexyl)-2-((methylsulfonyl)methyl)morpholine;
(2*R*,5*S*)-4-(4-(1-methyl-1*H*-1,2,4-triazol-3-yl)cyclohexyl)-5-(4-(trifluoromethyl)benzyl)-morpholine-2-carboxylic acid;
(2*S*,5*S*)-5-(4-chlorobenzyl)-2-methyl-4-(4-(5-methyl-1*H*-pyrazol-3-yl)cyclohexyl)morpholine;
(2*R*,5*S*)-5-(4-chlorobenzyl)-4-(4-(1-methyl-1*H*-1,2,4-triazol-3-yl)cyclohexyl)-2-((methylsulfonyl)methyl)morpholine;
(2*R*,5*S*)-5-(4-chlorobenzyl)-4-(4-(1-methyl-1*H*-1,2,4-triazol-3-yl)cyclohexyl)morpholine-2-carboxylic acid;
(2*R*,5*S*)-5-(4-chlorobenzyl)-4-(4-(1-methyl-1*H*-1,2,3-triazol-4-yl)cyclohexyl)-2-((methylsulfonyl)methyl)morpholine;
methyl (2*R*,5*S*)-5-(4-chlorobenzyl)-4-(4-(1-methyl-1*H*-1,2,3-triazol-4-yl)cyclohexyl)morpholine-2-carboxylate;
(2*R*,5*S*)-5-(4-chlorobenzyl)-4-(4-(1-methyl-1*H*-1,2,3-triazol-4-yl)cyclohexyl)morpholine-2-carboxylic acid;
(2*R*,5*S*)-5-(4-chlorobenzyl)-4-(4-(1,5-dimethyl-1*H*-pyrazol-4-yl)cyclohexyl)-2-((methylsulfonyl)methyl)morpholine;
(2*R*,5*S*)-5-(4-chlorobenzyl)-4-(4-(1-methyl-1*H*-pyrazol-3-yl)cyclohexyl)-2-((methylsulfonyl)methyl)morpholine;
(2*R*,5*S*)-5-(4-chlorobenzyl)-4-(4-(2-methyl-2*H*-1,2,3-triazol-4-yl)cyclohexyl)-2-((methylsulfonyl)methyl)morpholine;
(2*R*,5*S*)-5-(4-chlorobenzyl)-4-(4-(2-methyl-2*H*-tetrazol-5-yl)cyclohexyl)-2-((methylsulfonyl)methyl)morpholine;
(2*R*,5*S*)-5-(4-chlorobenzyl)-*N*,*N*-dimethyl-4-(4-(5-methyl-1,3,4-thiadiazol-2-yl)cyclohexyl)morpholine-2-carboxamide;
(2*R*,5*S*)-5-(4-chlorobenzyl)-4-(4-(5-methyl-1,3,4-thiadiazol-2-yl)cyclohexyl)-2-((methylsulfonyl)methyl)morpholine;
(2*R*,5*S*)-5-(4-chlorobenzyl)-4-(4-(5-methoxy-1-methyl-1*H*-1,2,4-triazol-3-yl)cyclohexyl)-2-((methylsulfonyl)methyl)morpholine;
3-(4-((2*R*,5*S*)-5-(4-chlorobenzyl)-2-((methylsulfonyl)methyl)morpholino)cyclohexyl)-1-methyl-1*H*-1,2,4-triazol-5-ol;
(2*R*,5*S*)-5-(4-chlorobenzyl)-4-(4-(1-isopropyl-1*H*-pyrazol-3-yl)cyclohexyl)-2-((methylsulfonyl)methyl)morpholine;
(2*R*,5*S*)-5-(4-chlorobenzyl)-4-(4-(5-methoxy-1-methyl-1*H*-1,2,4-triazol-3-yl)cyclohexyl)-*N*,*N*-dimethylmorpholine-2-carboxamide;
(2*R*,5*S*)-5-(4-chlorobenzyl)-4-(4-(5-hydroxy-1-methyl-1*H*-1,2,4-triazol-3-yl)cyclohexyl)-*N*,*N*-dimethylmorpholine-2-carboxamide;
(2*R*,5*S*)-5-(4-chlorobenzyl)-4-(4-(1,2-dimethyl-1*H*-imidazol-4-yl)cyclohexyl)-2-((methylsulfonyl)methyl)morpholine;
(2*R*,5*S*)-5-(4-chlorobenzyl)-4-(4-(1-methyl-5-(trifluoromethyl)-1*H*-pyrazol-3-yl)cyclohexyl)-2-((methylsulfonyl)methyl)morpholine;
(2*R*,5*S*)-5-(4-chlorobenzyl)-4-(4-(4,5-dimethylthiophen-2-yl)cyclohexyl)-2-((methylsulfonyl)methyl)morpholine;
4-(4-((2*R*,5*S*)-5-(4-chlorobenzyl)-2-((methylsulfonyl)methyl)morpholino)cyclohexyl)-1-methyl-1*H*-pyrrole-2-carbonitrile;
(2*R*,5*S*)-5-(4-chlorobenzyl)-4-(4-(5-cyano-1-methyl-1*H*-pyrrol-3-yl)cyclohexyl)morpholine-2-carboxylic acid;
(2*R*,5*S*)-5-(4-chlorobenzyl)-4-(4-(1-ethyl-5-methyl-1*H*-pyrazol-3-yl)cyclohexyl)-2-((methylsulfonyl)methyl)morpholine;
(2*R*,5*S*)-5-(4-chlorobenzyl)-4-(4-(1-ethyl-5-methyl-1*H*-pyrazol-3-yl)cyclohexyl)morpholine-2-carboxylic acid;
(2*R*,5*S*)-5-(4-chlorobenzyl)-*N*-ethyl-4-(4-(1-ethyl-5-methyl-1*H*-pyrazol-3-yl)cyclohexyl)morpholine-2-carboxamide;
(2*R*,5*S*)-5-(4-chlorobenzyl)-4-(4-(4-methyl-1*H*-pyrazol-1-yl)cyclohexyl)-2-((methylsulfonyl)methyl)morpholine;
(2*R*,5*S*)-5-(4-chlorobenzyl)-4-(4-(4-methyl-1*H*-pyrazol-1-yl)cyclohexyl)morpholine-2-carboxylic acid;
*N*-(39-((((2*R*,5*S*)-5-(4-chlorobenzyl)-4-(4-(4,5-dimethylthiazol-2-yl)cyclohexyl)morpholin-2-yl)methyl)sulfonyl)-37-oxo-3,6,9,12,15,18,21,24,27,30,33-undecaoxa-36-azanonatriacontyl)-5-((3a*S*,4*S*,6a*R*)-2-oxohexahydro-1*H*-thieno[3,4-*d*]imidazol-4-yl)pentanamide;
(2*R*,5*S*)-5-(4-chlorobenzyl)-4-(4-(1,5-dimethyl-1*H*-pyrazol-3-yl)cyclohexyl)-*N*-(1*H*-tetrazol-5-yl)morpholine-2-carboxamide;
(2*R*,5*S*)-4-(4-(1*H*-tetrazol-5-yl)cyclohexyl)-5-(4-chlorobenzyl)-2-((methylsulfonyl)methyl)-morpholine;
(2*R*,5*S*)-4-(4-(1*H*-1,2,4-triazol-1-yl)cyclohexyl)-5-(4-chlorobenzyl)-2-((methylsulfonyl)methyl)-morpholine;
(2*R*,5*S*)-4-(4-(1*H*-pyrazol-1-yl)cyclohexyl)-5-(4-chlorobenzyl)-2-((methylsulfonyl)methyl)-morpholine;
(2*R*,5*S*)-5-(4-chlorobenzyl)-4-(4-(4-fluoro-1*H*-pyrazol-1-yl)cyclohexyl)-2-((methylsulfonyl)methyl)morpholine;
(2*R*,5*S*)-5-(4-chlorobenzyl)-4-(4-(5-fluoro-1-methyl-1*H*-pyrazol-3-yl)cyclohexyl)morpholine-2-carboxylic acid;
$N^1$-(1,31-bis(((2*R*,3*R*,4*R*,5*R*,6*R*)-3-acetamido-4,5-dihydroxy-6-(hydroxymethyl)tetrahydro-2*H*-pyran-2-yl)oxy)-16-((3-((3-(5-(((2*R*,3*R*,4*R*,5*R*,6*R*)-3-acetamido-4,5-dihydroxy-6-(hydroxymethyl)tetrahydro-2*H*-pyran-2-yl)oxy)pentanamido)propyl)amino)-3-oxopropoxy)methyl)-5,11,21,27-tetraoxo-14,18-dioxa-6,10,22,26-tetraazahentriacontan-16-yl)-$N^{12}$-(3-(4-(((2*R*,5*S*)-5-(4-chlorobenzyl)-4-(4-(1,5-dimethyl-1*H*-pyrazol-3-yl)cyclohexyl)morpholine-2-carboxamido)methyl)-1*H*-1,2,3-triazol-1-yl)propyl)dodecanediamide; and
(2*R*,5*S*)-5-(4-chlorobenzyl)-4-(4-(1,5-dimethyl-1*H*-pyrazol-3-yl)cyclohexyl)-*N*-(5-(2-(2,6-dioxopiperidin-3-yl)-3-oxoisoindolin-4-yl)pentyl)morpholine-2-carboxamide;
or is a tautomer, stereoisomer, pharmaceutically acceptable salt, solvate, or polymorph thereof.

**21.** Compound according to claim 2, selected from the group consisting of the following compounds:

(2*S*,5*S*)-5-(4-chlorobenzyl)-2-methyl-4-(4-(5-methyloxazol-2-yl)cyclohexyl)morpholine;
(2*S*,5*S*)-5-(4-chlorobenzyl)-4-(4-(1,5-dimethyl-1*H*-pyrazol-3-yl)cyclohexyl)-2-methylmorpholine;
(2*R*,5*S*)-5-(4-chlorobenzyl)-4-(4-(5-methyloxazol-2-yl)cyclohexyl)-2-((methylsulfonyl)methyl)morpholine;
(2*R*,5*S*)-5-(4-chlorobenzyl)-4-((1*r*,4*S*)-4-(1,5-dimethyl-1*H*-pyrazol-3-yl)cyclohexyl)-2-((methylsulfonyl)methyl)morpholine;
(2*R*,5*S*)-5-(4-chlorobenzyl)-4-(4-(1,5-dimethyl-1*H*-pyrazol-3-yl)cyclohexyl)-2-(3-methylisoxazol-5-yl)morpholine;
(7*S*,9a*R*)-7-(4-chlorobenzyl)-8-(4-(5-methyloxazol-2-yl)cyclohexyl)octahydropyrazino[2,1-*c*][1,4]oxazine;
(7*S*,9a*R*)-7-(4-chlorobenzyl)-8-(4-(1,5-dimethyl-1*H*-pyrazol-3-yl)cyclohexyl)octahydropyrazino[2,1-*c*][1,4]oxazine;
(2*R*,5*S*)-5-(4-chlorobenzyl)-4-(4-(5-methyloxazol-2-yl)cyclohexyl)morpholine-2-carboxylic acid;
methyl (2*R*,5*S*)-5-(4-chlorobenzyl)-4-(4-(1,5-dimethyl-1*H*-pyrazol-3-yl)cyclohexyl)morpholine-2-carboxylate;
(2*R*,5*S*)-5-(4-chlorobenzyl)-4-(4-(1,5-dimethyl-1*H*-pyrazol-3-yl)cyclohexyl)morpholine-2-carboxylic acid;

(2*R*,5*S*)-5-(4-chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholine;
(2*R*,5*S*)-5-(4-chlorobenzyl)-4-(4-(5-methyloxazol-2-yl)cyclohexyl)-2-(1-(2,2,2-trifluoroethyl)-1*H*-1,2,3-triazol-4-yl)morpholine;
(2*R*,5*S*)-5-(4-chlorobenzyl)-4-(4-(1,5-dimethyl-1*H*-pyrazol-3-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl) morpholine-2-carboxamide;
(2*R*,5*S*)-5-(4-chlorobenzyl)-4-(4-(1,5-dimethyl-1*H*-1,2,4-triazol-3-yl)cyclohexyl)-2-((methylsulfonyl)methyl) morpholine;
(2*R*,5*S*)-5-(4-chlorobenzyl)-4-(4-(1,5-dimethyl-1*H*-pyrazol-3-yl)cyclohexyl)-2-(fluoromethyl)morpholine;
((2*R*,5*S*)-5-(4-chlorobenzyl)-4-(4-(1,5-dimethyl-1*H*-pyrazol-3-yl)cyclohexyl)morpholin-2-yl)methanol;
(2R,5S)-5-(4-chlorobenzyl)-N-ethyl-4-(4-(5-methyloxazol-2-yl)cyclohexyl)morpholine-2-carboxamide;
(2*R*,5*S*)-5-(4-chlorobenzyl)-4-(4-(1,5-dimethyl-1*H*-pyrazol-3-yl)cyclohexyl)-*N*-ethylmorpholine-2-carboxamide;
(2*R*,5*S*)-5-(4-chlorobenzyl)-4-(4-(1,5-dimethyl-1*H*-pyrazol-3-yl)cyclohexyl)-*N,N*-dimethylmorpholine-2-carboxamide;
(2*R*,5*S*)-5-(4-chlorobenzyl)-*N*,*N*-dimethyl-4-(4-(5-methyloxazol-2-yl)cyclohexyl)morpholine-2-carboxamide;
(2*R*,5*S*)-5-(4-chlorobenzyl)-4-(4-(1,5-dimethyl-1*H*-pyrazol-3-yl)cyclohexyl)-2-(5-methyl-1,3,4-oxadiazol-2-yl) morpholine;
(2*R*,5*S*)-*N'*-acetyl-5-(4-chlorobenzyl)-4-(4-(1,5-dimethyl-1*H*-pyrazol-3-yl)cyclohexyl)morpholine-2-carbohydrazide;
(2*R*,5*S*)-5-(4-chlorobenzyl)-2-(5-methyl-1,3,4-oxadiazol-2-yl)-4-(4-(5-methyloxazol-2-yl)cyclohexyl)morpholine;
(2*R*,5*S*)-*N'*-acetyl-5-(4-chlorobenzyl)-4-(4-(5-methyloxazol-2-yl)cyclohexyl)morpholine-2-carbohydrazide;
(2*R*,5*S*)-5-(4-chlorobenzyl)-4-(4-(4,5-dimethyloxazol-2-yl)cyclohexyl)-2-((methylsulfonyl)methyl)morpholine;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1,5-dimethyl-1*H*-pyrazole-3-carboxamide;
(2*S*,5*S*)-5-(4-chlorobenzyl)-4-(4-(1,5-dimethyl-1*H*-pyrazol-3-yl)cyclohexyl)-2-ethynylmorpholine;
*N*-(35-(4-((2*R*,5*S*)-5-(4-chlorobenzyl)-4-(4-(1,5-dimethyl-1*H*-pyrazol-3-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
(2*R*,5*S*)-5-(4-chlorobenzyl)-4-(4-(1,5-dimethyl-1*H*-pyrazol-3-yl)cyclohexyl)-*N*-isopropylmorpholine-2-carboxamide;
(2*R*,5*S*)-5-(4-chlorobenzyl)-4-(4-(1,5-dimethyl-1*H*-pyrazol-3-yl)cyclohexyl)-*N*-methylmorpholine-2-carboxamide;
(2*R*,5*S*)-5-(4-chlorobenzyl)-4-(4-(4,5-dimethyloxazol-2-yl)cyclohexyl)-*N,N*-dimethylmorpholine-2-carboxamide;
(2*R*,5*S*)-5-(4-chlorobenzyl)-4-(4-(4,5-dimethyloxazol-2-yl)cyclohexyl)-*N*-isopropylmorpholine-2-carboxamide;
(2*R*,5*S*)-5-(4-chlorobenzyl)-*N*-methyl-4-(4-(5-methyloxazol-2-yl)cyclohexyl)morpholine-2-carboxamide;
(2*R*,5*S*)-5-(4-chlorobenzyl)-4-(4-(4,5-dimethyloxazol-2-yl)cyclohexyl)-*N*-ethylmorpholine-2-carboxamide;
(2*R*,5*S*)-5-(4-chlorobenzyl)-4-(4-(5-fluoro-1-methyl-1*H*-pyrazol-3-yl)cyclohexyl)-2-((methylsulfonyl)methyl) morpholine;
(2*R*,5*S*)-5-(4-chlorobenzyl)-4-(4-(4,5-dimethylthiazol-2-yl)cyclohexyl)-2-((methylsulfonyl)methyl)morpholine;
(2*R*,5*S*)-5-(4-chlorobenzyl)-2-((methylsulfonyl)methyl)-4-(4-(5-methylthiazol-2-yl)cyclohexyl)morpholine;
(2*R*,5*S*)-5-(4-chlorobenzyl)-4-(4-(4,5-dimethyloxazol-2-yl)cyclohexyl)morpholine-2-carboxylic acid;
(2*R*,5*S*)-5-(4-chlorobenzyl)-4-(4-(4,5-dimethylthiazol-2-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)-2-((prop-2-yn-1-yloxy)methyl)morpholine;
(2*R*,5*S*)-5-(4-chlorobenzyl)-4-(4-(4,5-dimethylthiazol-2-yl)cyclohexyl)-*N*-ethylmorpholine-2-carboxamide;
*N*-(35-(4-((((2*R*,5*S*)-5-(4-chlorobenzyl)-4-(4-(1,5-dimethyl-1*H*-pyrazol-3-yl)cyclohexyl)morpholin-2-yl)methoxy)methyl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamide;
(2*R*,5*S*)-2-(azidomethyl)-5-(4-chlorobenzyl)-4-(4-(1,5-dimethyl-1*H*-pyrazol-3-yl)cyclohexyl)morpholine;
(2*R*,5*S*)-5-(4-chlorobenzyl)-4-(4-(1,5-dimethyl-1*H*-pyrazol-3-yl)cyclohexyl)-*N*-(prop-2-yn-1-yl)morpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-pyrazole-3-carboxamide;
(2*R*,5*S*)-5-(4-chlorobenzyl)-4-(4-(1,5-dimethyl-1*H*-pyrazol-3-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl) methyl)-2*H*-1,2,3-triazole-4-carboxamide;
3-(((2*R*,5*S*)-5-(4-chlorobenzyl)-4-(4-(1,5-dimethyl-1*H*-pyrazol-3-yl)cyclohexyl)morpholin-2-yl)methyl)imidazolidine-2,4-dione;
(2*R*,5*S*)-5-(4-chlorobenzyl)-4-(4-(1,5-dimethyl-1*H*-1,2,4-triazol-3-yl)cyclohexyl)-*N*-ethylmorpholine-2-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1*H*-imidazole-4-carboxamide;
(2*R*,5*S*)-5-(4-chlorobenzyl)-4-(4-(4,5-dimethylthiazol-2-yl)cyclohex-3-en-1-yl)-2-((methylsulfonyl)methyl)morpholine;
(2*R*,5*S*)-5-(4-chlorobenzyl)-4-(4-(4,5-dimethylthiazol-2-yl)cyclohex-3-en-1-yl)-*N*-ethylmorpholine-2-carboxamide;
(2*R*,5*S*)-5-(4-chlorobenzyl)-4-(4-(4,5-dimethylthiazol-2-yl)cyclohexyl)-*N*-(37-oxo-41-((3a*S*,4*S*,6a*R*)-2-oxohexahydro-1*H*-thieno[3,4-d]imidazol-4-yl)-3,6,9,12,15,18,21,24,27,30,33-undecaoxa-36-azahentetracontyl)morpholine-2-carboxamide;
(2*R*,5*S*)-2-(azidomethyl)-5-(4-chlorobenzyl)-4-(4-(4,5-dimethylthiazol-2-yl)cyclohexyl)morpholine;
(2*R*,5*S*)-5-(4-chlorobenzyl)-4-(4-(4,5-dimethylthiazol-2-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholine-2-carboxamide;
*N*-(((2*S*,5*S*)-5-(4-chlorobenzyl)-4-(4-(4,5-dimethylthiazol-2-yl)cyclohexyl)morpholin-2-yl)methyl)-1*H*-1,2,3-triazole-5-carboxamide;
*N*-(((2*S*,5*S*)-5-(4-chlorobenzyl)-4-(4-(1,5-dimethyl-1*H*-pyrazol-3-yl)cyclohexyl)morpholin-2-yl)methyl)-1-methyl-1*H*-imidazole-4-carboxamide;
methyl 2-((2*S*,5*S*)-5-(4-chlorobenzyl)-4-(4-(1,5-dimethyl-1*H*-pyrazol-3-yl)cyclohexyl)morpholin-2-yl) acetate;
2-((2*S*,5*S*)-5-(4-chlorobenzyl)-4-(4-(1,5-dimethyl-1*H*-pyrazol-3-yl)cyclohexyl)morpholin-2-yl)acetic acid;
2-((2*S*,5*S*)-5-(4-chlorobenzyl)-4-(4-(1,5-dimethyl-1*H*-pyrazol-3-yl)cyclohexyl)morpholin-2-yl)-*N*-ethylacetamide;
(2*R*,5*S*)-5-(4-chlorobenzyl)-4-(4-(1,5-dimethyl-1*H*-1,2,4-triazol-3-yl)cyclohexyl)morpholine-2-carboxylic acid;
(2*R*,5*S*)-5-(4-bromobenzyl)-4-(4-(1,5-dimethyl-1*H*-pyrazol-3-yl)cyclohexyl)-2-((methylsulfonyl)methyl)morpholine;
*N*-(((2*R*,5*S*)-5-(4-chlorobenzyl)-4-(4-(1,5-dimethyl-1*H*-pyrazol-3-yl)cyclohexyl)morpholin-2-yl)methyl)methanesulfonamide;
(2*R*,5*S*)-5-(4-bromobenzyl)-4-(4-(1,5-dimethyl-1*H*-pyrazol-3-yl)cyclohexyl)morpholine-2-carboxylic acid;
(2*R*,5*S*)-5-(4-bromobenzyl)-4-(4-(5-methyloxazol-2-yl)cyclohexyl)morpholine-2-carboxylic acid;
(2*R*,5*S*)-5-(4-bromobenzyl)-4-(4-(5-methyloxazol-2-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)-2-((methylsulfonyl)methyl) morpholine;
(2*R*,5*S*)-5-(4-bromobenzyl)-4-(4-(1,5-dimethyl-1*H*-1,2,4-triazol-3-yl)cyclohexyl)morpholine-2-carboxylic acid;
*N*-(((2*R*,5*S*)-5-(4-chlorobenzyl)-4-(4-(1,5-dimethyl-1*H*-pyrazol-3-yl)cyclohexyl)morpholin-2-yl)methyl)-1,1,1-trifluoromethanesulfonamide;
(2*R*,5*S*)-5-(4-bromobenzyl)-4-(4-(1,5-dimethyl-1*H*-pyrazol-3-yl)cyclohexyl)-*N*-ethylmorpholine-2-carboxamide;
(2*R*,5*S*)-*N*-(2-(3-(but-3-yn-1-yl)-3*H*-diazirin-3-yl)ethyl)-5-(4-chlorobenzyl)-4-(4-(1,5-dimethyl-1*H*-pyrazol-3-yl) cyclohexyl)morpholine-2-carboxamide;
2-(((2*R*,5*S*)-5-(4-chlorobenzyl)-4-(4-(4,5-dimethylthiazol-2-yl)cyclohexyl)morpholin-2-yl)methoxy)acetic acid;
2-((2*R*,5*S*)-5-(4-chlorobenzyl)-4-(4-(1,5-dimethyl-1*H*-pyrazol-3-yl)cyclohexyl)morpholin-2-yl)propan-2-ol;
(2*R*,5*S*)-5-(4-chlorobenzyl)-4-(4-(1,5-dimethyl-1*H*-pyrazol-3-yl)cyclohexyl)-2-(5-methyl-1*H*-pyrazol-3-yl)morpholine;
(2*R*,5*R*)-2-(4-chlorobenzyl)-1-(4-(1,5-dimethyl-1*H*-pyrazol-3-yl)cyclohexyl)-5-(methylsulfonyl)piperidine;
(2*R*,5*S*)-5-(4-chlorobenzyl)-4-(4-(1,5-dimethyl-1*H*-pyrazol-3-yl)cyclohexyl)morpholine-2-carboxamide;
2-(4-((2*R*,5*R*)-2-(4-chlorobenzyl)-5-(methylsulfonyl)piperidin-1-yl)cyclohexyl)-5-methyloxazole;
(2*R*,5*S*)-5-(4-chlorobenzyl)-4-(4-(1,5-dimethyl-1*H*-pyrazol-3-yl)cyclohexyl)-2-((methylsulfinyl)methyl)morpholine;
(2*R*,5*S*)-5-(4-chlorobenzyl)-4-(4-(1,5-dimethyl-1*H*-pyrazol-3-yl)cyclohexyl)-2-((methylthio)methyl)morpholine;
1-(((2*R*,5*S*)-5-(4-chlorobenzyl)-4-(4-(1,5-dimethyl-1*H*-pyrazol-3-yl)cyclohexyl)morpholin-2-yl)methyl)pyrrolidine-2,5-dione;
(2*S*,5*S*)-5-(4-chlorobenzyl)-4-(4-(4,5-dimethylthiazol-2-yl)cyclohexyl)-2-ethynylmorpholine;

2-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)acetic acid;
3-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)propanoic acid;
(2*R*,5*S*)-5-(4-chlorobenzyl)-4-(4-(1,5-dimethyl-1*H*-pyrazol-3-yl)cyclohex-3-en-1-yl)-2-((methylsulfonyl)methyl) morpholine;
*N*-(35-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-d]imidazol-4-yl)pentanamide;
2-((2*S*,5*S*)-5-(4-chlorobenzyl)-4-(4-(4,5-dimethylthiazol-2-yl)cyclohexyl)morpholin-2-yl)acetic acid;
3-((((2*R*,5*S*)-5-(4-chlorobenzyl)-4-(4-(4,5-dimethylthiazol-2-yl)cyclohexyl)morpholin-2-yl)methyl)sulfonyl)propanoic acid;
3-(((2*R*,5*S*)-5-(4-chlorobenzyl)-4-(4-(1,5-dimethyl-1*H*-pyrazol-3-yl)cyclohexyl)morpholin-2-yl)methyl)-6,6-dimethyl-3-azabicyclo[3.1.0]hexane-2,4-dione;
3-(((2*R*,5*S*)-5-(4-chlorobenzyl)-4-(4-(4,5-dimethylthiazol-2-yl)cyclohexyl)morpholin-2-yl)methyl)-6,6-dimethyl-3-azabicyclo[3.1.0]hexane-2,4-dione;
3-((((2*S*,5*S*)-5-(4-chlorobenzyl)-4-(4-(1,5-dimethyl-1*H*-pyrazol-3-yl)cyclohexyl)morpholin-2-yl)methyl)amino)-4-hydroxycyclobut-3-ene-1,2-dione;
3-amino-4-((((2*S*,5*S*)-5-(4-chlorobenzyl)-4-(4-(1,5-dimethyl-1*H*-pyrazol-3-yl)cyclohexyl)morpholin-2-yl)methyl) amino)cyclobut-3-ene-1,2-dione;
(2*R*,5*S*)-5-(4-chlorobenzyl)-4-(4-(4,5-dimethylthiazol-2-yl)cyclohexyl)-2-((ethylsulfonyl)methyl)morpholine;
(2*R*,5*S*)-5-(4-chlorobenzyl)-4-(4-(1,5-dimethyl-1*H*-pyrazol-3-yl)cyclohexyl)-2-((ethylsulfonyl)methyl)morpholine;
3-(((2*R*,5*S*)-5-(4-chlorobenzyl)-4-(4-(1,5-dimethyl-1*H*-pyrazol-3-yl)cyclohexyl)morpholin-2-yl)methyl)-1-methylimidazolidine-2,4-dione;
(2*R*,5*S*)-5-(4-chlorobenzyl)-4-(4-(4,5-dimethylthiazol-2-yl)cyclohexyl)-*N*-(1-methyl-1*H*-imidazol-4-yl)morpholine-2-carboxamide;
4-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)butanoic acid;
(2*R*,5*S*)-5-(4-chlorobenzyl)-4-(4-(4,5-dimethylthiazol-2-yl)cyclohexyl)-*N*-hydroxymorpholine-2-carboxamide;
(2*R*,5*S*)-5-(4-chlorobenzyl)-*N*-(1,5-dimethyl-1*H*-pyrazol-3-yl)-4-(4-(4,5-dimethylthiazol-2-yl)cyclohexyl)morpholine-2-carboxamide;
5-(4-((2*R*,5*S*)-5-(4-chlorobenzyl)-4-(4-(4,5-dimethylthiazol-2-yl)cyclohexyl)morpholin-2-yl)-1*H*-1,2,3-triazol-1-yl)pentanoic acid;
(2*R*,5*S*)-5-(4-chlorobenzyl)-*N*-(1,5-dimethyl-1*H*-pyrazol-3-yl)-4-(4-(1,5-dimethyl-1*H*-pyrazol-3-yl)cyclohexyl) morpholine-2-carboxamide; and
*N*-((2*R*,5*S*)-5-(4-chlorobenzyl)-4-(4-(4,5-dimethylthiazol-2-yl)cyclohexyl)morpholine-2-carbonyl)-*N*-methylglycine;
or a tautomer, stereoisomer, pharmaceutically acceptable salt, solvate, or polymorph thereof.

**22.** Compound according to claim 2 or claim 21, selected from the group consisting of the following compounds:

(2*S*,5*S*)-5-(4-chlorobenzyl)-2-methyl-4-(4-(5-methyloxazol-2-yl)cyclohexyl)morpholine;
(2*S*,5*S*)-5-(4-chlorobenzyl)-4-(4-(1,5-dimethyl-1*H*-pyrazol-3-yl)cyclohexyl)-2-methylmorpholine;
(2*R*,5*S*)-5-(4-chlorobenzyl)-4-(4-(5-methyloxazol-2-yl)cyclohexyl)-2-((methylsulfonyl)methyl)morpholine;
(2*R*,5*S*)-5-(4-chlorobenzyl)-4-((1*r*,4*S*)-4-(1,5-dimethyl-1*H*-pyrazol-3-yl)cyclohexyl)-2-((methylsulfonyl)methyl) morpholine;
(2*R*,5*S*)-5-(4-chlorobenzyl)-4-(4-(1,5-dimethyl-1*H*-pyrazol-3-yl)cyclohexyl)-2-(3-methylisoxazol-5-yl)morpholine;
(2*R*,5*S*)-5-(4-chlorobenzyl)-4-(4-(5-methyloxazol-2-yl)cyclohexyl)morpholine-2-carboxylic acid;
(2*R*,5*S*)-5-(4-chlorobenzyl)-4-(4-(1,5-dimethyl-1*H*-pyrazol-3-yl)cyclohexyl)morpholine-2-carboxylic acid;
(2*R*,5*S*)-5-(4-chlorobenzyl)-4-(4-(1,5-dimethyl-1*H*-1,2,4-triazol-3-yl)cyclohexyl)-2-((methylsulfonyl)methyl) morpholine;
(2*R*,5*S*)-5-(4-chlorobenzyl)-4-(4-(1,5-dimethyl-1*H*-pyrazol-3-yl)cyclohexyl)-2-(fluoromethyl)morpholine;
((2*R*,5*S*)-5-(4-chlorobenzyl)-4-(4-(1,5-dimethyl-1*H*-pyrazol-3-yl)cyclohexyl)morpholin-2-yl)methanol;
(2*R*,5*S*)-5-(4-chlorobenzyl)-4-(4-(1,5-dimethyl-1*H*-pyrazol-3-yl)cyclohexyl)-*N,N*-dimethylmorpholine-2-carboxamide;
(2*R*,5*S*)-5-(4-chlorobenzyl)-4-(4-(4,5-dimethyloxazol-2-yl)cyclohexyl)-2-((methylsulfonyl)methyl)morpholine;

(2*R*,5*S*)-5-(4-chlorobenzyl)-4-(4-(1,5-dimethyl-1*H*-pyrazol-3-yl)cyclohexyl)-N-methylmorpholine-2-carboxamide;
(2*R*,5*S*)-5-(4-chlorobenzyl)-4-(4-(5-fluoro-1-methyl-1*H*-pyrazol-3-yl)cyclohexyl)-2-((methylsulfonyl)methyl)morpholine;
(2*R*,5*S*)-5-(4-chlorobenzyl)-4-(4-(4,5-dimethylthiazol-2-yl)cyclohexyl)-2-((methylsulfonyl)methyl)morpholine;
(2*R*,5*S*)-5-(4-chlorobenzyl)-4-(4-(4,5-dimethyloxazol-2-yl)cyclohexyl)morpholine-2-carboxylic acid;
(2*R*,5*S*)-5-(4-chlorobenzyl)-4-(4-(4,5-dimethylthiazol-2-yl)cyclohexyl)morpholine-2-carboxylic acid;
(2*R*,5*S*)-5-(4-chlorobenzyl)-4-(4-(4,5-dimethylthiazol-2-yl)cyclohex-3-en-1-yl)-2-((methylsulfonyl)methyl)morpholine;
2-((2*S*,5*S*)-5-(4-chlorobenzyl)-4-(4-(1,5-dimethyl-1*H*-pyrazol-3-yl)cyclohexyl)morpholin-2-yl)acetic acid;
(2*R*,5*S*)-5-(4-chlorobenzyl)-4-(4-(1,5-dimethyl-1*H*-1,2,4-triazol-3-yl)cyclohexyl)morpholine-2-carboxylic acid;
(2*R*,5*S*)-5-(4-bromobenzyl)-4-(4-(1,5-dimethyl-1*H*-pyrazol-3-yl)cyclohexyl)-2-((methylsulfonyl)methyl)morpholine;
*N*-(((2*R*,5*S*)-5-(4-chlorobenzyl)-4-(4-(1,5-dimethyl-1*H*-pyrazol-3-yl)cyclohexyl)morpholin-2-yl)methyl)methanesulfonamide;
(2*R*,5*S*)-5-(4-bromobenzyl)-4-(4-(1,5-dimethyl-1*H*-pyrazol-3-yl)cyclohexyl)morpholine-2-carboxylic acid;
(2*R*,5*S*)-5-(4-bromobenzyl)-4-(4-(5-methyloxazol-2-yl)cyclohexyl)morpholine-2-carboxylic acid;
(2*R*,5*S*)-5-(4-bromobenzyl)-4-(4-(5-methyloxazol-2-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)-2-((methylsulfonyl)methyl)morpholine;
(2*R*,5*S*)-5-(4-bromobenzyl)-4-(4-(1,5-dimethyl-1*H*-1,2,4-triazol-3-yl)cyclohexyl)morpholine-2-carboxylic acid;
(2*R*,5*R*)-2-(4-chlorobenzyl)-1-(4-(1,5-dimethyl-1*H*-pyrazol-3-yl)cyclohexyl)-5-(methylsulfonyl)piperidine;
(2*R*,5*S*)-5-(4-chlorobenzyl)-4-(4-(1,5-dimethyl-1*H*-pyrazol-3-yl)cyclohexyl)morpholine-2-carboxamide;
2-(4-((2*R*,5*R*)-2-(4-chlorobenzyl)-5-(methylsulfonyl)piperidin-1-yl)cyclohexyl)-5-methyloxazole;
(2*R*,5*S*)-5-(4-chlorobenzyl)-4-(4-(1,5-dimethyl-1*H*-pyrazol-3-yl)cyclohexyl)-2-((methylsulfinyl)methyl)morpholine;
(2*R*,5*S*)-5-(4-chlorobenzyl)-4-(4-(1,5-dimethyl-1*H*-pyrazol-3-yl)cyclohexyl)-2-((methylthio)methyl)morpholine;
(2*R*,5*S*)-5-(4-chlorobenzyl)-4-(4-(1,5-dimethyl-1*H*-pyrazol-3-yl)cyclohex-3-en-1-yl)-2-((methylsulfonyl)methyl)morpholine;
2-((2*S*,5*S*)-5-(4-chlorobenzyl)-4-(4-(4,5-dimethylthiazol-2-yl)cyclohexyl)morpholin-2-yl)acetic acid; and
(2*R*,5*S*)-5-(4-chlorobenzyl)-4-(4-(1,5-dimethyl-1*H*-pyrazol-3-yl)cyclohexyl)-2-((ethylsulfonyl)methyl)morpholine;
or a tautomer, stereoisomer, pharmaceutically acceptable salt, solvate, or polymorph thereof.

**23.** A pharmaceutical composition comprising a therapeutically effective amount of at least one compound according to any one of claims 1 - 22, and a pharmaceutically acceptable carrier therefor.

**24.** A compound according to any one of claims 1 - 22, or a pharmaceutical composition according to claim 23, for use the treatment or prevention of a disease, disorder, or condition associated with aberrant expression or activity of the chitinase-like protein YKL-40.

**25.** Compound for use according to claim 24, wherein the disease, disorder, or condition is selected from the group consisting of cancer, allergic diseases, acute and chronic inflammatory diseases, autoimmune diseases, dental diseases, neurologic diseases, metabolic diseases, liver diseases, kidney diseases, polycystic ovary syndrome, endometriosis, asthma, and fibrotic disorders.

**26.** Compound for use according to claim 25, wherein the disease, disorder, or condition is cancer selected from the group consisting of glioblastoma, breast cancer, colon cancer, kidney cancer, uterine cancer, primary and metastatic lung cancer, mesothelioma, osteosarcoma, malignant melanoma, ovarian cancer, cervical cancer, prostate cancer, liver cancer, gastric cancer, metastatic renal cancer, leukemia, lymphoma, oligodendroglioma, glioblastoma, and germ cell tumors.

**27.** Compound for use according to claim 25, wherein the disease, disorder, or condition is an allergic disease selected from the group consisting of asthma, allergic rhinitis, seasonal allergic rhinitis, chronic rhinosinusitis with or without nasal polyps, conjunctivitis, keratoconjunctivitis, seasonal allergic conjunctivitis, dry eye syndrome, eosinophilic esophagitis, celiac disease, food allergy, irritable bowel syndrome, irritable bowel disease, atopic eczema, atopic dermatitis, allergic contact dermatitis, eosinophilic otitis media, eosinophilic pneumonia, and IgG4 mediated diseases.

**28.** Compound for use according to claim 25, wherein the disease, disorder, or condition is an acute or chronic inflammatory disease selected from the group consisting of fungal diseases, parasitic infections, celiac disease, microscopic colitis, chronic obstructive pulmonary disease (COPD), idiopathic pulmonary fibrosis, interstitial lung diseases, cystic fibrosis, Hermansky-Pudlak syndrome, and Alzheimer's disease.

**29.** Compound for use according to claim 25, wherein the disease, disorder, or condition is an autoimmune disorder selected from the group consisting of inflammatory bowel disease, ulcerative colitis, Crohn's disease, rheumatoid arthritis, osteoarthritis, psoriasis, scleroderma, multiple sclerosis, Sjögren's syndrome, atherosclerosis, and sarcoidosis.

**30.** Compound for use according to claim 25, wherein the disease, disorder, or condition is a metabolic disease selected from the group consisting of insulin-dependent diabetes mellitus and non-insulin-dependent diabetes mellitus.

**31.** Compound for use according to claim 25, wherein the disease, disorder, or condition is a liver disease selected from the group consisting of non-alcoholic fatty liver disease, non-alcoholic steatohepatitis, hepatitis-C virus-induced fibrosis and cirrhosis, and alcoholic fibrosis.

**32.** Compound for use according to claim 25, wherein the disease, disorder, or condition is a kidney disease selected from the group consisting of nephropathy (*e.g.*, diabetic nephropathy), focal segmental glomerulosclerosis, tubulointerstitial fibrosis, postransplant fibrosis, and retroperitoneal fibrosis (Ormond's disease).

**33.** Compound for use according to claim 25, wherein the disease, disorder, or condition is a fibrotic disorder, in particular idiopathic pulmonary fibrosis (IPF).

**34.** Compound for use according to claim 25, wherein the the disease, disorder, or condition is a storage disease selected from the group consisting of Gaucher disease, Fabry disease, lysosomal storage disorders, Niemann-Pick disease, nephropathic cystinosis, and X-linked globotriaosylceramidosis.

**35.** A method of identifying compounds that interfere with the chitinase-like protein YKL-40 and asialofetuin interaction by:

(1) in a binding competition assay, subjecting (i) a compound according to any one of claims 1 to 22 and (ii) biotinylated asialofetuin to YKL-40 or derivative of YKL-40 comprising a purification tag; and
(2) assessing the resulting AlphaScreen luminescence signal; wherein an emission signal decline from the high level at lowest inhibitor concentration to the lower level (diminished by the value of at least 3x standard deviation of the noise displayed at plateau values at highest concentrations of inhibitor in the response plots for IC50 determinations) at highest inhibitor concentration indicates that the compound interferes with the chitinase-like protein YKL-40 and asialofetuin interaction.

## Patentansprüche

**1.** Eine Verbindung, die durch die Strukturformel (I) dargestellt wird:

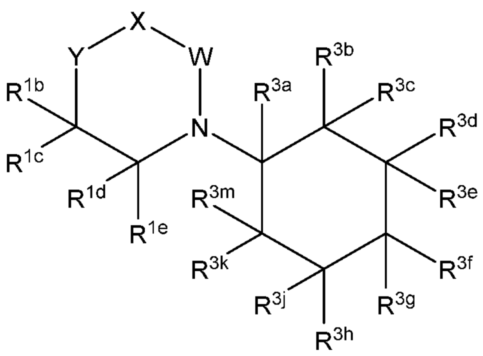

(I)

wobei:

W steht für $-C(-R^{1f})(-R^{1g})-$;
X steht für $-C(-R^{1h})(-R^{1j})-$;
Y steht für $-C(-R^{1k})(-R^{1m})-$, $-N(-R^{1k})-$, oder -O-;
$R^{1b}$ steht für -H;
$R^{1c}$ steht für -H;
$R^{1d}$ bedeutet 4-$R^{10}$-Benzyl;
$R^{1e}$ steht für -H;
$R^{1f}$ steht für -H;
$R^{1g}$ steht für -H;
$R^{1h}$, ausgewählt aus der Gruppe bestehend aus: $C_1$-$C_6$ Alkyl, $C_2$-$C_6$ Alkenyl, $C_2$-$C_6$ Alkinyl, hydroxy-$C_1$-$C_6$ Alkyl, azido-$C_1$-$C_6$ Alkyl, hydroxycarbonyl-$C_1$-$C_3$ alkoxy-$C_1$-$C_6$ Alkyl, $C_3$-$C_6$ alkinyloxy-$C_1$-$C_6$ Alkyl, halo-$C_1$-$C_6$ Alkyl, $C_1$-$C_3$ alkylthio-$C_1$-$C_6$ Alkyl, $C_1$-$C_3$ Alkylsulfinyl-$C_1$-$C_6$ Alkyl, $C_1$-$C_3$ Alkylsulfonyl-$C_1$-$C_6$ Alkyl, Hydroxycarbonyl-$C_1$-$C_3$ Alkylsulfonyl-$C_1$-$C_6$ Alkyl, Hydroxycarbonyl-$C_1$-$C_6$ Alkyl, $C_1$-$C_3$ Alkylcarbonyl-$C_1$-$C_6$ Alkyl, $C_1$-$C_3$ Alkylaminocarbonyl-$C_1$-$C_6$ Alkyl, Di($C_1$-$C_3$ alkyl)aminocarbonyl-$C_1$-$C_6$ Alkyl, $C_1$-$C_3$ Alkylsulfonylamino-$C_1$-$C_6$ Alkyl, Halo-$C_1$-$C_3$ alkylsulfonylamino-$C_1$-$C_6$ Alkyl, 5- oder 6-gliedriges monozyklisches Heteroaryl-carbonylamino-$C_1$-$C_6$ Alkyl, 5- oder 6-gliedriges monozyklisches Heteroaryl-$C_1$-$C_3$ alkoxy-$C_1$-$C_6$ Alkyl, 5- oder 6-gliedriges monozyklisches Heteroaryl-$C_1$-$C_6$ Alkyl, Hydroxycarbonyl, $C_1$-$C_3$ Alkoxycarbonyl, Aminocarbonyl, $C_1$-$C_3$ Alkylaminocarbonyl, Di($C_1$-$C_3$ alkyl)aminocarbonyl, $C_3$-$C_6$ Alkinylaminocarbonyl, Hydroxycarbonyl-$C_1$-$C_3$ Alkyl($C_1$-$C_3$ alkyl)aminocarbonyl, (Biotinyl-$PEG_{11}$)aminocarbonyl, (Biotinyl-$PEG_{11}$)aminocarbonyl-$C_1$-$C_3$ Alkylsulfonyl-$C_1$-$C_6$ Alkyl, Hydroxylaminocarbonyl, $N^2$-Acetylhydrazinecarbonyl, (5- oder 6-gliedriges monozyklisches Heteroaryl)-$C_1$-$C_3$ Alkylaminocarbonyl, (5- oder 6-gliedriges monozyklisches Heteroaryl)aminocarbonyl, $C_1$-$C_3$ Alkylsulfonyl, 5- oder 6-gliedriges monozyklisches Heteroaryl, (3-Azabizyklo[3.1.0]hexan-3-yl)-$C_1$-$C_3$ Alkyl, (4-, 5-, oder 6-gliedriges Heterocyclyl)amino-$C_1$-$C_3$ Alkyl, Hydroxycarbonyl-$C_1$-$C_6$ Alkyl-(5- oder 6-gliedriges monozyklisches Heteroaryl), $R^{20}$, und 5-(2-(2,6-dioxopiperidin-3-yl)-3-oxoisoindolin-4-yl)Pentyl,
wobei jede verfügbare Position eines beliebigen Heteroaryl-Rings in $R^{1h}$ mit einem Substituent ersetzt werden kann, der ausgewählt ist aus der Gruppe bestehend aus: $C_1$-$C_6$ Alkyl, $C_3$-$C_6$ Alkinyl, Halogen, Trifluoromethyl, 2,2,2-Trifluorethyl, Biotinyl-$PEG_{11}$, und jede verfügbare Position einer beliebigen Kohlenstoffkette in $R^{1h}$ mit Ausnahme von Heteroaryl-Ringen mit einem Substituent, ausgewählt aus Halogen, Amino, Hydroxy und Oxo, ersetzt werden kann;
$R^{1j}$ steht für -H;
$R^{1k}$ steht für -H;
$R^{1m}$ steht für -H;
oder $R^{1k}$ und $R^{1h}$ zusammen mit den Stickstoff- bzw. Kohlenstoffatomen, die sie tragen, einen 6-teiligen heterocyclischen Ring der Strukturformel (II) darstellen:

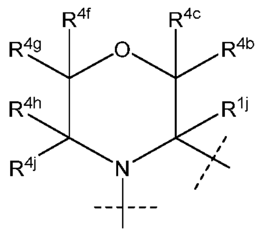
(II)

$R^{3a}$ steht für -H;
$R^{3b}$ steht für -H;
$R^{3c}$ steht für -H;
$R^{3d}$ steht für -H;
$R^{3e}$ steht für -H;
$R^{3f}$, ausgewählt aus der Gruppe bestehend aus: 1-Methyl-1*H*-imidazol-4-yl, 1,2-Dimethyl-1*H*-imidazol-4-yl, 5-Methyloxazol-2-yl, 4,5-Dimethyloxazol-2-yl, 1*H*-pyrazol-1-yl, 1-Methyl-1*H*-pyrazol-3-yl, 4-Methyl-1*H*-pyrazol-1-yl, 4-Fluoro-1*H*-pyrazol-1-yl, 1-Isopropyl-1*H*-pyrazol-3-yl, 5-Methyl-1*H*-pyrazol-3-yl, 1,5-Dimethyl-1*H*-pyrazol-3-yl, 1-Ethyl-5-methyl-1*H*-pyrazol-3-yl, 1,5-Dimethyl-1*H*-pyrazol-3-yl, 1,5-Dimethyl-1*H*-pyrazol-4-yl, 1-Methyl-5-(trifluoromethyl)-1*H*-pyrazol-3-yl, 1-Methyl-2-cyano-1*H*-pyrrol-4-yl, 1*H*-Tetrazol-5-yl, 2-Methyl-2*H*-tetrazol-5-yl, 5-Methyl-1,3,4-thiadiazol-2-yl, 5-Methylthiazol-2-yl, 4,5-Dimethylthiophen-2-yl, 1*H*-1,2,4-Triazol-1-yl, 1-Methyl-1*H*-1,2,3-triazol-4-yl, 1-Methyl-1*H*-1,2,4-triazol-3-yl, 2-Methyl-2*H*-1,2,3-triazol-4-yl, 1,5-Dimethyl-1*H*-1,2,4-triazol-3-yl, 5-Hydroxy-1-methyl-1*H*-1,2,4-triazol-3-yl, und 5-Methoxy-1-methyl-1*H*-1,2,4-triazol-3-yl;

$R^{3g}$ steht für -H;
$R^{3h}$ steht für -H;
$R^{3j}$ steht für -H;
$R^{3k}$ steht für -H;
$R^{3m}$ steht für -H;
oder $R^{3d}$ und $R^{3g}$ zusammen mit der Einfachbindung zwischen den zwei sie tragenden Kohlenstoffatomen, die eine Doppelbindung zwischen diesen beiden Kohlenstoffatomen darstellen;
$R^{4b}$ steht für -H;
$R^{4c}$ steht für -H;
$R^{4f}$ steht für -H;
$R^{4g}$ steht für -H;
$R^{4h}$ steht für -H;
$R^{4j}$ steht für -H;
$R^{10}$ steht für Halogen oder Trifluoromethyl;
und $R^{20}$ steht für

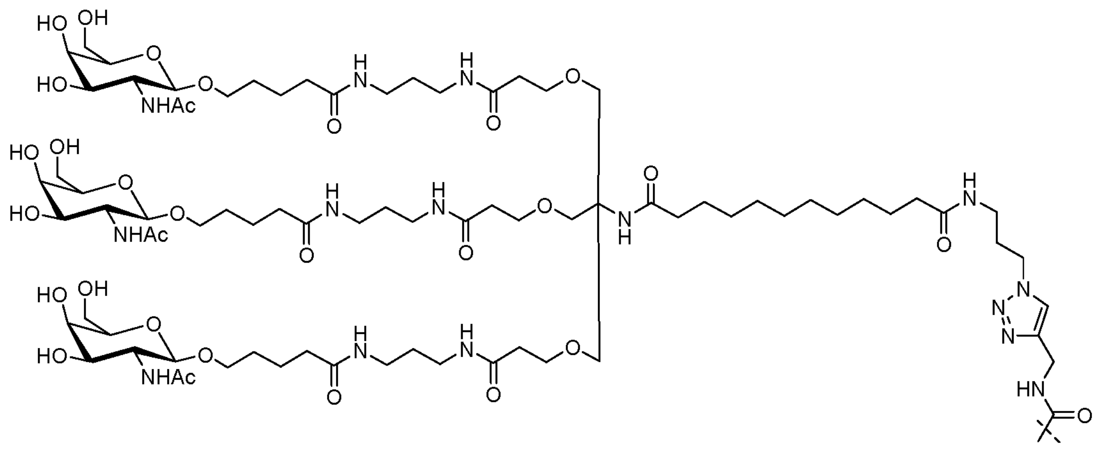

;

oder ein Tautomer, Stereoisomer, N-Oxid, Methiodid, pharmazeutisch zugelassenes Salz, Solvat oder Polymorph davon.

**2.** Eine Verbindung nach Anspruch 1, dargestellt mit der Strukturformel (I):

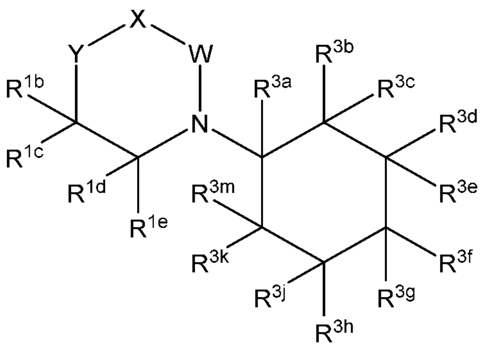

(I)

wobei:

W steht für $-C(-R^{1f})(-R^{1g})-$;
X steht für $-C(-R^{1h})(-R^{1j})-$;
Y steht für $-C(-R^{1k})(-R^{1m})-$, $-N(-R^{1k})-$, oder -O-;
$R^{1b}$ steht für -H;
$R^{1c}$ steht für -H;
$R^{1d}$ bedeutet 4-$R^{10}$-Benzyl;
$R^{1e}$ steht für -H;

$R^{1f}$ steht für -H;
$R^{1g}$ steht für -H;
$R^{1h}$, ausgewählt aus der Gruppe bestehend aus: $C_1$-$C_6$ Alkyl, $C_2$-$C_6$ Alkenyl, $C_2$-$C_6$ Alkinyl, hydroxy-$C_1$-$C_6$ Alkyl, azido-$C_1$-$C_6$ Alkyl, Hydroxycarbonyl-$C_1$-$C_3$ alkoxy-$C_1$-$C_6$ Alkyl, $C_3$-$C_6$ Alkinyloxy-$C_1$-$C_6$ Alkyl, Halo-$C_1$-$C_6$ Alkyl, $C_1$-$C_3$ Alkylthio-$C_1$-$C_6$ Alkyl, $C_1$-$C_3$ Alkylsulfinyl-$C_1$-$C_6$ Alkyl, $C_1$-$C_3$ Alkylsulfonyl-$C_1$-$C_6$ Alkyl, Hydroxycarbonyl-$C_1$-$C_3$ Alkylsulfonyl-$C_1$-$C_6$ Alkyl, Hydroxycarbonyl-$C_1$-$C_6$ Alkyl, $C_1$-$C_3$ Alkylcarbonyl-$C_1$-$C_6$ Alkyl, $C_1$-$C_3$ Alkylaminocarbonyl-$C_1$-$C_6$ Alkyl, Di($C_1$-$C_3$ alkyl)aminocarbonyl-$C_1$-$C_6$ Alkyl, $C_1$-$C_3$ Alkylsulfonylamino-$C_1$-$C_6$ Alkyl, Halo-$C_1$-$C_3$ Alkylsulfonylamino-$C_1$-$C_6$ Alkyl, 5- oder 6-gliedriges monozyklisches Heteroaryl-carbonylamino-$C_1$-$C_6$ Alkyl, 5- oder 6-gliedriges monozyklisches Heteroaryl-$C_1$-$C_3$ Alkoxy-$C_1$-$C_6$ Alkyl, 5- oder 6-gliedriges monozyklisches Heteroaryl-$C_1$-$C_6$ Alkyl, Hydroxycarbonyl, $C_1$-$C_3$ Alkoxycarbonyl, Aminocarbonyl, $C_1$-$C_3$ Alkylaminocarbonyl, Di($C_1$-$C_3$ alkyl)aminocarbonyl, $C_3$-$C_6$ Alkinylaminocarbonyl, Hydroxycarbonyl-$C_1$-$C_3$ Alkyl($C_1$-$C_3$ alkyl)aminocarbonyl, (Biotinyl-$PEG_{11}$)aminocarbonyl, Hydroxylaminocarbonyl, *N*²-Acetylhydrazinecarbonyl, (5- oder 6-gliedriges monozyklisches Heteroaryl)-$C_1$-$C_3$ Alkylaminocarbonyl, (5- oder 6-gliedriges monozyklisches Heteroaryl)aminocarbonyl, $C_1$-$C_3$ Alkylsulfonyl, 5- oder 6-gliedriges monozyklisches Heteroaryl, (3-Azabizyklo[3.1.0]hexan-3-yl)-$C_1$-$C_3$ Alkyl, (4-, 5-, oder 6-gliedriges Heterocyclyl)amino-$C_1$-$C_3$ Alkyl, und Hydroxycarbonyl-$C_1$-$C_6$ Alkyl-(5- oder 6-gliedriges monozyklisches Heteroaryl),
wobei jede verfügbare Position eines beliebigen Heteroaryl-Rings in $R^{1h}$ mit einem Substituent ersetzt werden kann, der ausgewählt ist aus der Gruppe bestehend aus: $C_1$-$C_6$ Alkyl, $C_3$-$C_6$ Alkinyl, Halogen, Trifluoromethyl, 2,2,2-Trifluorethyl, Biotinyl-$PEG_{11}$, und jede verfügbare Position einer beliebigen Kohlenstoffkette in $R^{1h}$ mit Ausnahme von Heteroaryl-Ringen mit einem Substituent, ausgewählt aus Halogen, Amino, Hydroxy und Oxo, ersetzt werden kann;
$R^{1j}$ steht für -H;
$R^{1k}$ steht für -H;
$R^{1m}$ steht für -H;
oder $R^{1k}$ und $R^{1h}$ zusammen mit den Stickstoff- bzw. Kohlenstoffatomen, die sie tragen, einen 6-gliedrigen heterocyclischen Ring mit der Strukturformel (II) darstellen:

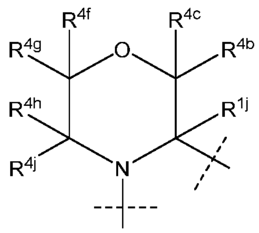

(II)

$R^{3a}$ steht für -H;
$R^{3b}$ steht für -H;
$R^{3c}$ steht für -H;
$R^{3d}$ steht für -H;
$R^{3e}$ steht für -H;
$R^{3f}$, ausgewählt aus der Gruppe bestehend aus: 5-Methyloxazol-2-yl, 1 ,5-Dimethyl-1*H*-pyrazol-3-yl, 1,5-Dimethyl-1*H*-1,2,4-triazol-3-yl, 4,5-Dimethyloxazol-2-yl, 5-Fluoro-1-methyl-1*H*-pyrazol-3-yl, 4,5-Dimethylthiazol-2-yl, und 5-Methylthiazol-2-yl;
$R^{3g}$ steht für -H;
$R^{3h}$ steht für -H;
$R^{3j}$ steht für -H;
$R^{3k}$ steht für -H;
$R^{3m}$ steht für -H;
oder $R^{3d}$ und $R^{3g}$ zusammen mit der Einfachbindung zwischen den zwei sie tragenden Kohlenstoffatomen, die eine Doppelbindung zwischen diesen beiden Kohlenstoffatomen darstellen;
$R^{4b}$ steht für -H;
$R^{4c}$ steht für -H;
$R^{4f}$ steht für -H;
$R^{4g}$ steht für -H;
$R^{4h}$ steht für -H;
$R^{4j}$ steht für -H;
und $R^{10}$, der für Halogen oder Trifluoromethyl steht;

oder ein Tautomer, Stereoisomer, *N*-Oxid, Methiodid, pharmazeutisch zugelassenes Salz, Solvat oder Polymorph davon.

**3.** Eine Verbindung nach Anspruch 1 oder 2, die die Strukturformel (Ia) aufweist:

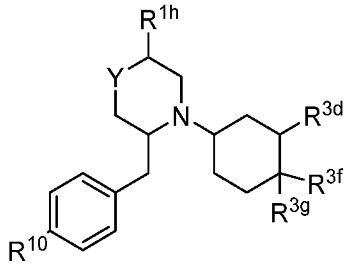

(Ia)

wobei:

Y steht für $-CH_2-$, $-N(-R^{1k})-$ oder -O-;

R1h, ausgewählt aus der Gruppe bestehend aus: Methyl, Ethinyl, 2-Hydroxyprop-2-yl, Azidomethyl, Hydroxymethyl, Hydroxycarbonylmethoxymethyl, (Prop-2-yn-1-yloxy)methyl, Fluoromethyl, Methylthiomethyl, Methylsulfinylmethyl, Methylsulfonylmethyl, Ethylsulfonylmethyl, 2-(Hydroxycarbonyl)ethylsulfonylmethyl, Hydroxycarbonylmethyl, Methoxycarbonylmethyl, Ethylaminocarbonylmethyl, Methylsulfonylaminomethyl, Trifluoromethylsulfonylaminomethyl, (1*H*-Pyrazol-3-yl)carbonylaminomethyl, 1-(Trifluoromethyl)-1*H*-1,2,3-triazol-4-yl, (1-(Biotinyl-$PEG_{11}$)-1*H*-1,2,3-triazol-4-yl)methoxymethyl, (2*H*-1,2,3-Triazol-4-yl)carbonylaminomethyl, (1*H*-1,2,3-Triazol-5-yl)carbonylaminomethyl, (1-Methyl-1*H*-imidazol-4-yl)carbonylaminomethyl, (1*H*-Imidazol-4-yl) carbonylaminomethyl, (1,5-Dimethyl-1*H*-pyrazol-3-yl)carbonylaminomethyl, (2,5-Dioxopyrrolidin-1-yl)methyl, (2,4-Dioxolmidazolidin-3-yl)methyl, (1-Methyl-2,4-dioxolmidazolidin-3-yl)methyl, Hydroxycarbonyl, Methoxycarbonyl, Aminocarbonyl, Methylaminocarbonyl, Dimethylaminocarbonyl, Ethylaminocarbonyl, Isopropylaminocarbonyl, (Prop-2-yn-1-yl)aminocarbonyl, Hydroxycarbonylmethyl(methyl)aminocarbonyl, (Biotinyl-$PEG_{11}$) aminocarbonyl, Hydroxylaminocarbonyl, $N^2$-Acetylhydrazinecarbonyl, (1-(Biotinyl-$PEG_{11}$)-1*H*-1,2,3-triazol-4-yl)methylaminocarbonyl, (1-Methyl-1*H*-imidazol-4-yl)aminocarbonyl, (1,5-Dimethyl-1*H*-pyrazol-3-yl)aminocarbonyl, (2-(3-(But-3-yn-1-yl)-3*H*-diazirin-3-yl)ethyl)aminocarbonyl, Methylsulfonyl, 3-Methylisoxazol-5-yl, 1-(2,2,2-Trifluoroethyl)-1*H*-1,2,3-triazol-4-yl, 5-Methyl-1,3,4-oxadiazol-2-yl, (6,6-Dimethyl-2,4-dioxo-3-azabizyklo[3.1.0]hexan-3-yl)methyl, (1,2-Dioxo-3-hydroxy-cyclobut-3-en-4-yl)aminomethyl, (1,2-Dioxo-3-amino-cyclobut-3-en-4-yl)aminomethyl, 1-(Biotinyl-$PEG_{11}$)-1*H*-1,2,3-triazol-4-yl, 1-(Hydroxycarbonylmethyl)-1*H*-1,2,3-triazol-4-yl, 1-(2-(Hydroxycarbonyl)ethyl)-1*H*-1,2,3-triazol-4-yl, 1-(3-(Hydroxycarbonyl)prop-1-yl)-1*H*-1,2,3-triazol-4-yl, 1-(4-(Hydroxycarbonyl)but-1-yl)-1*H*-1,2,3-triazol-4-yl, und 5-Methyl-1*H*-pyrazol-3-yl;

oder $R^{1k}$ und $R^{1h}$ zusammen mit den Stickstoff- bzw. Kohlenstoffatomen, die sie tragen, einen 6-gliedrigen heterocyclischen Ring mit der Strukturformel (IIa) darstellen:

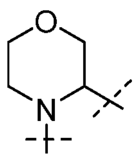

(IIa)

$R^{3d}$ steht für H;

$R^{3g}$ steht für H;

oder $R^{3d}$ und $R^{3g}$ zusammen mit der Einfachbindung zwischen den zwei sie tragenden Kohlenstoffatomen, die eine Doppelbindung zwischen diesen beiden Kohlenstoffatomen darstellen;

$R^{3f}$, ausgewählt aus der Gruppe bestehend aus: 5-Methyloxazol-2-yl, 1,5-Dimethyl-1*H*-pyrazol-3-yl, 1,5-Dimethyl-1*H*-1,2,4-triazol-3-yl, 4,5-Dimethyloxazol-2-yl, 5-Fluoro-1-methyl-1*H*-pyrazol-3-yl, 4,5-Dimethylthiazol-2-yl, und 5-Methylthiazol-2-yl;

und $R^{10}$ steht für Chlor, Brom oder Trifluoromethyl;

oder ein Tautomer, Stereoisomer, pharmazeutisch zulässiges Salz, Solvat oder Polymorph davon.

**4.** Eine Verbindung nach Anspruch 1 oder 2 oder 3,

wobei:

X steht für -CH(-$R^{1h}$)-;
Y steht für -$CH_2$-; und
$R^{1h}$ steht für Methansulfonyl.

**5.** Eine Verbindung nach Anspruch 1 oder 2 oder 3,
wobei:

X steht für -CH(-$R^{1h}$)-;
Y steht für -N(-$R^{1k}$)-.
und $R^{1k}$ und $R^{1h}$ zusammen mit den Stickstoff- bzw. Kohlenstoffatomen, die sie tragen, einen 6-gliedrigen heterocyclischen Ring mit der Strukturformel (IIb) darstellen:

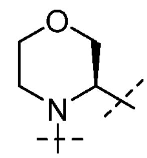
(IIb).

**6.** Eine Verbindung nach Anspruch 1 oder 2 oder 3,
wobei Y für -O- steht.

**7.** Eine Verbindung nach einem der Ansprüche 1 bis 6,
wobei $R^{1d}$ und $R^{1h}$ in *cis* Beziehung zueinander stehen.

**8.** Eine Verbindung nach einem der Ansprüche 1 bis 7,
wobei die absolute Stereochemie an dem $R^{1d}$-tragenden Kohlenstoffatom S ist.

**9.** Eine Verbindung nach einem der Ansprüche 1, 2, 3, 6, 7 und 8, die die folgende Strukturformel (Ib) aufweist:

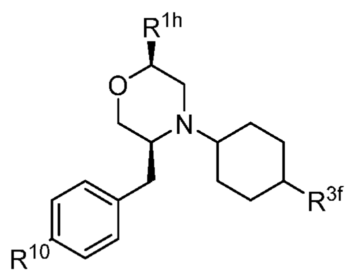
(Ib)

wobei:

$R^{1h}$, ausgewählt aus der Gruppe bestehend aus: Methyl, Ethinyl, 2-Hydroxyprop-2-yl, Azidomethyl, Hydroxymethyl, Hydroxycarbonylmethoxymethyl, (Prop-2-yn-1-yloxy)methyl, Fluoromethyl, Methylthiomethyl, Methylsulfinylmethyl, Methylsulfonylmethyl, Ethylsulfonylmethyl, 2-(Hydroxycarbonyl)ethylsulfonylmethyl, Hydroxycarbonylmethyl, Methoxycarbonylmethyl, Ethylaminocarbonylmethyl, Methylsulfonylaminomethyl, Trifluoromethylsulfonylaminomethyl, (1*H*-Pyrazol-3-yl)carbonylaminomethyl, 1-(Trifluoromethyl)-1*H*-1,2,3-triazol-4-yl, (1-(Biotinyl-$PEG_{11}$)-1*H*-1,2,3-triazol-4-yl)methoxymethyl, (2*H*-1,2,3-Triazol-4-yl)carbonylaminomethyl, (1*H*-1,2,3-Triazol-5-yl)carbonylaminomethyl, (1-Methyl-1*H*-imidazol-4-yl)carbonylaminomethyl, (1H-Imidazol-4-yl) carbonylaminomethyl, (1,5-Dimethyl-1*H*-pyrazol-3-yl)carbonylaminomethyl, (2,5-Dioxopyrrolidin-1-yl)methyl, (2,4-Dioxolmidazolidin-3-yl)methyl, (1-Methyl-2,4-dioxolmidazolidin-3-yl)methyl, Hydroxycarbonyl, Methoxycarbonyl, Aminocarbonyl, Methylaminocarbonyl, Dimethylaminocarbonyl, Ethylaminocarbonyl, Isopropylaminocarbonyl, (Prop-2-yn-1-yl)aminocarbonyl, (Biotinyl-$PEG_{11}$)aminocarbonyl, Hydroxylaminocarbonyl, $N^2$-Acetylhydrazinecarbonyl, (1-(Biotinyl-$PEG_{11}$)-1*H*-1,2,3-triazol-4-yl)methylaminocarbonyl, (1-Methyl-1*H*-imidazol-4-yl)aminocarbonyl, (1,5-Dimethyl-1H-pyrazol-3-yl)aminocarbonyl, (2-(3-(But-3-yn-1-yl)-3*H*-diazirin-3-yl) ethyl)aminocarbonyl, 3-Methylisoxazol-5-yl, 1-(2,2,2-Trifluoroethyl)-1*H*-1,2,3-triazol-4-yl, 5-Methyl-1,3,4-oxadiazol-2-yl, (6,6-Dimethyl-2,4-dioxo-3-azabizyklo[3.1.0]hexan-3-yl)methyl, (1,2-Dioxo-3-hydroxy-zyklobut-3-en-4-yl)aminomethyl, (1,2-Dioxo-3-amino-zyklobut-3-en-4-yl)aminomethyl, 1-(Biotinyl-$PEG_{11}$)-1*H*-1,2,3-triazol-4-yl, 1-(Hydroxycarbonylmethyl)-1*H*-1,2,3-triazol-4-yl, 1-(2-(Hydroxycarbonyl)ethyl)-1*H*-1,2,3-triazol-4-yl, 1-(3-(Hydroxycarbonyl)prop-1-yl)-1*H*-1,2,3-triazol-4-yl, 1-(4-(Hydroxycarbonyl)but-1-yl)-1*H*-1,2,3-triazol-4-yl, und 5-Methyl-1*H*-pyrazol-3-yl; und

$R^{3f}$, ausgewählt aus der Gruppe bestehend aus: 5-Methyloxazol-2-yl, 1,5-Dimethyl-1*H*-pyrazol-3-yl, 1,5-Dimethyl-1*H*-1,2,4-triazol-3-yl, 4,5-Dimethyloxazol-2-yl, 5-Fluoro-1-methyl-1*H*-pyrazol-3-yl, 4,5-Dimethylthiazol-2-yl, und 5-Methylthiazol-2-yl;

oder ein Tautomer, Stereoisomer, pharmazeutisch zulässiges Salz, Solvat oder Polymorph davon.

**10.** Eine Verbindung nach einem der Ansprüche 1, 2, 3, 6, 7, 8 und 9, wobei $R^{10}$ Brom ist.

**11.** Eine Verbindung nach einem der Ansprüche 1 bis 9, wobei $R^{10}$ Chlor ist.

**12.** Eine Verbindung nach einem der Ansprüche 1 bis 11, wobei $R^{3f}$ 5-Methyloxazol-2-yl ist.

**13.** Eine Verbindung nach einem der Ansprüche 1 bis 11, wobei $R^{3f}$ 1,5-Dimethyl-1*H*-pyrazol-3-yl ist.

**14.** Eine Verbindung nach einem der Ansprüche 1 bis 11, wobei $R^{3f}$ 1,5-Dimethyl-1*H*-1,2,4-triazol-3-yl ist.

**15.** Eine Verbindung nach einem der Ansprüche 1, 2, 3, 6, 7, 8, 9 und 11, wobei $R^{3f}$ 4,5-Dimethyloxazol-2-yl ist.

**16.** Eine Verbindung nach einem der Ansprüche 1, 2, 3, 6, 7, 8, 9 und 11, wobei $R^{3f}$ 5-Fluor-1-methyl-1*H*-pyrazol-3-yl ist.

**17.** Eine Verbindung nach einem der Ansprüche 1 bis 11, wobei $R^{3f}$ 4,5-Dimethylthiazol-2-yl ist.

**18.** Eine Verbindung nach einem der Ansprüche 1, 2, 3, 6, 7, 8, 9 und 11, wobei $R^{3f}$ 5-Methylthiazol-2-yl ist.

**19.** Eine Verbindung nach einem der Ansprüche 1 bis 18, wobei

$R^{3a}$ für H steht;
$R^{3g}$ für H steht; und
der Substituent $R^{3f}$ und der heterozyklische Ring, der durch sein Stickstoffringgliedatom an den Cyclohexan-Ring nach dem 1,4-Substitutionsmuster gebunden ist, stehen zueinander in der *trans*-Beziehung.

**20.** Eine Verbindung nach Anspruch 1, ausgewählt aus einer Gruppe, die die folgenden Verbindungen umfasst:

(2*S*,5*S*)-5-(4-Chlorobenzyl)-2-methyl-4-(4-(5-methyloxazol-2-yl)cyclohexyl)morpholin;
(2*S*,5*S*)-5-(4-Chlorobenzyl)-4-(4-(1,5-dimethyl-1*H*-Pyrazol-3-yl)cyclohexyl)-2-methylmorpholin;
(2*R*,5*S*)-5-(4-Chlorobenzyl)-4-(4-(5-methyloxazol-2-yl)cyclohexyl)-2-((methylsulfonyl)methyl)morpholin;
(2*R*,5*S*)-5-(4-Chlorobenzyl)-4-((1*r*,4*S*)-4-(1,5-dimethyl-1*H*-Pyrazol-3-yl)cyclohexyl)-2-((methylsulfonyl)methyl) morpholin;
(2*R*,5*S*)-5-(4-Chlorobenzyl)-4-(4-(1,5-dimethyl-1*H*-Pyrazol-3-yl)cyclohexyl)-2-(3-methylisoxazol-5-yl)morpholin;
(7*S*,9a*R*)-7-(4-Chlorobenzyl)-8-(4-(5-Methyloxazol-2-yl)cyclohexyl)octahydropyrazino[2,1-*c*][1,4]oxazin;
(7*S*,9a*R*)-7-(4-Chlorobenzyl)-8-(4-(1,5-dimethyl-1*H*-Pyrazol-3-yl)cyclohexyl)octahydropyrazino[2,1-*c*][1,4]oxazin;
(2*R*,5*S*)-5-(4-Chlorobenzyl)-4-(4-(5-methyloxazo1-2-yl)cyclohexyl)morpholin-2-carbonsäure;
Methyl (2*R*,5*S*)-5-(4-chlorobenzyl)-4-(4-(1,5-dimethyl-1*H*-pyrazol-3-yl)cyclohexyl)morpholin-2-carboxylat;

(2*R*,5*S*)-5-(4-Chlorobenzyl)-4-(4-(1,5-dimethyl-1*H*-pyrazol-3-yl)cyclohexyl)morpholin-2-carbonsäure;
(2*R*,5*S*)-5-(4-Chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholin;
(2*R*,5*S*)-5-(4-Chlorobenzyl)-4-(4-(5-methyloxazol-2-yl)cyclohexyl)-2-(1-(2,2,2-trifluoroethyl)-1*H*-1,2,3-triazol-4-yl)morpholin;
(2*R*,5*S*)-5-(4-Chlorobenzyl)-4-(4-(1,5-dimethyl-1*H*-pyrazol-3-yl)cyclohexyl)-*N*-(37-oxo-41-((3a*S*,4*S*,6a*R*)-2-oxohexahydro-1*H*-thieno[3,4-*d*]Imidazol-4-yl)-3,6,9,12,15,18,21,24,27,30,33-undecaoxa-36-azahentetracontyl)morpholin-2-carboxamid;
(2*R*,5*S*)-5-(4-Chlorobenzyl)-4-(4-(1,5-dimethyl-1*H*-1,2,4-triazol-3-yl)cyclohexyl)-2-((methylsulfonyl)methyl) morpholin;
(2*R*,5*S*)-5-(4-Chlorobenzyl)-4-(4-(1,5-dimethyl-1*H*-pyrazol-3-yl)cyclohexyl)-2-(fluoromethyl)morpholin;
((2*R*,5*S*)-5-(4-Chlorobenzyl)-4-(4-(1,5-dimethyl-1*H*-pyrazol-3-yl)cyclohexyl)morpholin-2-yl)methanol;
(2*R*,5*S*)-5-(4-Chlorobenzyl)-*N*-ethyl-4-(4-(5-methyloxazol-2-yl)cyclohexyl)morpholin-2-carboxamid;
(2*R*,5*S*)-5-(4-Chlorobenzyl)-4-(4-(1,5-dimethyl-1*H*-pyrazol-3-yl)cyclohexyl)-*N*-ethylmorpholin-2-carboxamid;
(2*R*,5*S*)-5-(4-Chlorobenzyl)-4-(4-(1,5-dimethyl-1*H*-pyrazol-3-yl)cyclohexyl)-*N,N*-dimethylmorpholin-2-carboxamid;
(2*R*,5*S*)-5-(4-Chlorobenzyl)-*N,N*-dimethyl-4-(4-(5-methyloxazol-2-yl)cyclohexyl)morpholin-2-carboxamid;
(2*R*,5*S*)-5-(4-Chlorobenzyl)-4-(4-(1,5-dimethyl-1*H*-pyrazol-3-yl)cyclohexyl)-2-(5-methyl-1,3,4-oxadiazol-2-yl) morpholin;
(2*R*,5*S*)-*N*-Acetyl-5-(4-chlorobenzyl)-4-(4-(1,5-dimethyl-1*H*-pyrazol-3-yl)cyclohexyl)morpholin-2-carbohydrazid;
(2*R*,5*S*)-5-(4-Chlorobenzyl)-2-(5-methyl-1,3,4-oxadiazol-2-yl)-4-(4-(5-methyloxazol-2-yl)cyclohexyl)morpholin;
(2*R*,5*S*)-*N*-Acetyl-5-(4-chlorobenzyl)-4-(4-(5-methyloxazol-2-yl)cyclohexyl)morpholin-2-carbohydrazid;
(2*R*,5*S*)-5-(4-Chlorobenzyl)-4-(4-(4,5-dimethyloxazol-2-yl)cyclohexyl)-2-((methylsulfonyl)methyl)morpholin;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-1,5-dimethyl-1*H*-pyrazol-3-carboxamid;
(2*S*,5*S*)-5-(4-Chlorobenzyl)-4-(4-(1,5-dimethyl-1*H*-pyrazol-3-yl)cyclohexyl)-2-ethinylmorpholin;
*N*-(35-(4-((2*R*,5*S*)-5-(4-Chlorobenzyl)-4-(4-(1,5-dimethyl-1*H*-pyrazol-3-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamid;
(2*R*,5*S*)-5-(4-Chlorobenzyl)-4-(4-(1,5-dimethyl-1*H*-pyrazol-3-yl)cyclohexyl)-*N*-isopropylmorpholin-2-carboxamid;
(2*R*,5*S*)-5-(4-Chlorobenzyl)-4-(4-(1,5-dimethyl-1*H*-pyrazol-3-yl)cyclohexyl)-*N*-methylmorpholin-2-carboxamid;
(2*R*,5*S*)-5-(4-Chlorobenzyl)-4-(4-(4,5-dimethyloxazol-2-yl)cyclohexyl)-*N,N*-dimethylmorpholin-2-carboxamid;
(2*R*,5*S*)-5-(4-Chlorobenzyl)-4-(4-(4,5-dimethyloxazol-2-yl)cyclohexyl)-*N*-isopropylmorpholin-2-carboxamid;
(2*R*,5*S*)-5-(4-Chlorobenzyl)-*N*-methyl-4-(4-(5-methyloxazol-2-yl)cyclohexyl)morpholin-2-carboxamid;
(2*R*,5*S*)-5-(4-Chlorobenzyl)-4-(4-(4,5-dimethyloxazol-2-yl)cyclohexyl)-*N*-ethylmorpholin-2-carboxamid;
(2*R*,5*S*)-5-(4-Chlorobenzyl)-4-(4-(5-fluoro-1-methyl-1*H*-pyrazol-3-yl)cyclohexyl)-2-((methylsulfonyl)methyl) morpholin;
(2*R*,5*S*)-5-(4-Chlorobenzyl)-4-(4-(4,5-dimethylthiazol-2-yl)cyclohexyl)-2-((methylsulfonyl)methyl)morpholin;
(2*R*,5*S*)-5-(4-Chlorobenzyl)-2-((methylsulfonyl)methyl)-4-(4-(5-methylthiazol-2-yl)cyclohexyl)morpholin;
(2*R*,5*S*)-5-(4-Chlorobenzyl)-4-(4-(4,5-dimethyloxazol-2-yl)cyclohexyl)morpholin-2-carbonsäure;
(2*R*,5*S*)-5-(4-Chlorobenzyl)-4-(4-(4,5-dimethylthiazol-2-yl)cyclohexyl)morpholin-2-carbonsäure;
(2*R*,5*S*)-5-(4-Chlorobenzyl)-4-(4-(1,5-dimethyl-1*H*-pyrazol-3-yl)cyclohexyl)-2-((prop-2-yn-1-yloxy)methyl)morpholin;
(2*R*,5*S*)-5-(4-Chlorobenzyl)-4-(4-(4,5-dimethylthiazol-2-yl)cyclohexyl)-*N*-ethylmorpholin-2-carboxamid;
*N*-(35-(4-((((2*R*,5*S*)-5-(4-chlorobenzyl)-4-(4-(1,5-dimethyl-1*H*-pyrazol-3-yl)cyclohexyl)morpholin-2-yl)methoxy)methyl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]Imidazol-4-yl)pentanamid;
(2*R*,5*S*)-2-(Azidomethyl)-5-(4-chlorobenzyl)-4-(4-(1,5-dimethyl-1*H*-pyrazol-3-yl)cyclohexyl)morpholin;
(2*R*,5*S*)-5-(4-Chlorobenzyl)-4-(4-(1,5-dimethyl-1*H*-pyrazol-3-yl)cyclohexyl)-*N*-(prop-2-yn-1-yl)morpholin-2-carboxamid;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-1*H*-pyrazole-3-carboxamid;
(2*R*,5*S*)-5-(4-Chlorobenzyl)-4-(4-(1,5-dimethyl-1*H*-pyrazol-3-yl)cyclohexyl)-*N*-((1-(37-oxo-41-((3a*S*,4*S*,6a*R*)-2-oxohexahydro-1*H*-thieno[3,4-*d*]Imidazol-4-yl)-3,6,9,12,15,18,21,24,27,30,33-undecaoxa-36-azahentetracontyl)-1*H*-1,2,3-triazol-4-yl)methyl)morpholin-2-carboxamid;

*N*-(((2*S*,5*S*)-5-(4-Chlorobenzyl)-4-(4-(1,5-dimethyl-1*H*-pyrazol-3-yl)cyclohexyl)morpholin-2-yl)methyl)-2*H*-1,2,3-triazole-4-carboxamid;
3-(((2*R*,5*S*)-5-(4-Chlorobenzyl)-4-(4-(1,5-dimethyl-1*H*-pyrazol-3-yl)cyclohexyl)morpholin-2-yl)methyl)imidazolidine-2,4-dion;
(2*R*,5*S*)-5-(4-Chlorobenzyl)-4-(4-(1,5-dimethyl-1*H*-1,2,4-triazol-3-yl)cyclohexyl)-*N*-ethylmorpholin-2-carboxamid;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-1*H*-imidazol-4-carboxamid;
(2*R*,5*S*)-5-(4-Chlorobenzyl)-4-(4-(4,5-dimethylthiazol-2-yl)zyklohex-3-en-1-yl)-2-((methylsulfonyl)methyl)morpholin;
(2*R*,5*S*)-5-(4-Chlorobenzyl)-4-(4-(4,5-dimethylthiazol-2-yl)zyklohex-3-en-1-yl)-*N*-ethylmorpholin-2-carboxamid;
(2*R*,5*S*)-5-(4-Chlorobenzyl)-4-(4-(4,5-dimethylthiazol-2-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)morpholin-2-carboxamid;
(2*R*,5*S*)-2-(Azidomethyl)-5-(4-chlorobenzyl)-4-(4-(4,5-dimethylthiazol-2-yl)cyclohexyl)morpholin;
(2*R*,5*S*)-5-(4-Chlorobenzyl)-4-(4-(4,5-dimethylthiazol-2-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholin-2-carboxamid;
*N*-(((2*S*,5*S*)-5-(4-Chlorobenzyl)-4-(4-(4,5-dimethylthiazol-2-yl)cyclohexyl)morpholin-2-yl)methyl)-1*H*-1,2,3-triazol-5-carboxamid;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-1-methyl-1*H*-imidazol-4-carboxamid;
Methyl 2-((2*S*,5*S*)-5-(4-chlorobenzyl)-4-(4-(1,5-dimethyl-1*H*-pyrazol-3-yl)cyclohexyl)morpholin-2-yl)acetat;
2-((2*S*,5*S*)-5-(4-Chlorobenzyl)-4-(4-(1,5-dimethyl-1*H*-pyrazol-3-yl)cyclohexyl)morpholin-2-yl)essigsäure;
2-((2*S*,5*S*)-5-(4-Chlorobenzyl)-4-(4-(1,5-dimethyl-1*H*-pyrazol-3-yl)cyclohexyl)morpholin-2-yl)-*N*-ethylacetamid;
(2*R*,5*S*)-5-(4-Chlorobenzyl)-4-(4-(1,5-dimethyl-1*H*-1,2,4-triazol-3-yl)cyclohexyl)morpholin-2-carbonsäure;
(2*R*,5*S*)-5-(4-Bromobenzyl)-4-(4-(1,5-dimethyl-1*H*-pyrazol-3-yl)cyclohexyl)-2-((methylsulfonyl)methyl)morpholin;
*N*-(((2*R*,5*S*)-5-(4-Chlorobenzyl)-4-(4-(1,5-dimethyl-1*H*-pyrazol-3-yl)cyclohexyl)}morpholin-2-yl)methyl)methanesulfonamid;
(2*R*,5*S*)-5-(4-Bromobenzyl)-4-(4-(1,5-dimethyl-1*H*-pyrazol-3-yl)cyclohexyl)morpholin-2-carbonsäure;
(2*R*,5*S*)-5-(4-Bromobenzyl)-4-(4-(5-methyloxazol-2-yl)cyclohexyl)morpholin-2-carbonsäure;
(2*R*,5*S*)-5-(4-Bromobenzyl)-4-(4-(5-methyloxazol-2-yl)cyclohexyl)-2-((methylsulfonyl)methyl)morpholin;
(2*R*,5*S*)-5-(4-Bromobenzyl)-4-(4-(1,5-dimethyl-1*H*-1,2,4-triazol-3-yl)cyclohexyl)-2-((methylsulfonyl)methyl) morpholin;
(2*R*,5*S*)-5-(4-Bromobenzyl)-4-(4-(1,5-dimethyl-1*H*-1,2,4-triazol-3-yl)cyclohexyl)morpholin-2-carbonsäure;
*N*-(((2*R*,5*S*)-5-(4-Chlorobenzyl)-4-(4-(1,5-dimethyl-1*H*-Pyrazol-3-yl)cyclohexyl)morpholin-2-yl)methyl)-1,1,1-trifluoromethanesulfonamid;
(2*R*,5*S*)-5-(4-Bromobenzyl)-4-(4-(1,5-dimethyl-1*H*-pyrazol-3-yl)cyclohexyl)-*N*-ethylmorpholin-2-carboxamid;
(2*R*,5*S*)-*N*-(2-(3-(But-3-yn-1-yl)-3*H*-diazirin-3-yl)ethyl)-5-(4-chlorobenzyl)-4-(4-(1,5-dimethyl-1*H*-pyrazol-3-yl) cyclohexyl)morpholin-2-carboxamid;
2-(((2*R*,5*S*)-5-(4-Chlorobenzyl)-4-(4-(4,5-dimethylthiazol-2-yl)cyclohexyl)morpholin-2-yl)methoxy)essigsäure;
2-((2*R*,5*S*)-5-(4-Chlorobenzyl)-4-(4-(1,5-dimethyl-1*H*-pyrazol-3-yl)cyclohexyl)morpholin-2-yl)propan-2-ol;
(2*R*,5*S*)-5-(4-Chlorobenzyl)-4-(4-(1,5-dimethyl-1*H*-pyrazol-3-yl)cyclohexyl)-2-(5-methyl-1*H*-pyrazol-3-yl)morpholin;
(2*R*,5*R*)-2-(4-Chlorobenzyl)-1-(4-(1,5-dimethyl-1*H*-pyrazol-3-yl)cyclohexyl)-5-(methylsulfonyl)piperidin;
(2*R*,5*S*)-5-(4-Chlorobenzyl)-4-(4-(1,5-dimethyl-1*H*-pyrazol-3-yl)cyclohexyl)morpholin-2-carboxamid;
2-(4-((2*R*,5*R*)-2-(4-Chlorobenzyl)-5-(methylsulfonyl)piperidin-1-yl)cyclohexyl)-5-methyloxazol;
(2*R*,5*S*)-5-(4-Chlorobenzyl)-4-(4-(1,5-dimethyl-1*H*-pyrazol-3-yl)cyclohexyl)-2-((methylsulfinyl)methyl)morpholin;
(2*R*,5*S*)-5-(4-Chlorobenzyl)-4-(4-(1,5-dimethyl-1*H*-pyrazol-3-yl)cyclohexyl)-2-((methylthio)methyl)morpholin;
1-(((2*R*,5*S*)-5-(4-Chlorobenzyl)-4-(4-(1,5-dimethyl-1*H*-pyrazol-3-yl)cyclohexyl)morpholin-2-yl)methyl)pyrrolidine-2,5-dion;
(2*S*,5*S*)-5-(4-Chlorobenzyl)-4-(4-(4,5-dimethylthiazol-2-yl)cyclohexyl)-2-ethinylmorpholin;
2-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)essigsäure;

3-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)propionsäure;
(2*R*,5*S*)-5-(4-Chlorobenzyl)-4-(4-(1,5-dimethyl-1*H*-pyrazol-3-yl)zyklohex-3-en-1-yl)-2-((methylsulfonyl)methyl) morpholin;
*N*-(35-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]Imidazol-4-yl)pentanamid;
2-((2*S*,5*S*)-5-(4-Chlorobenzyl)-4-(4-(4,5-dimethylthiazol-2-yl)cyclohexyl)morpholin-2-yl)essigsäure;
3-((((2*R*,5*S*)-5-(4-Chlorobenzyl)-4-(4-(4,5-dimethylthiazol-2-yl)cyclohexyl)morpholin-2-yl)methyl)sulfonyl)propionsäure;
3-(((2*R*,5*S*)-5-(4-Chlorobenzyl)-4-(4-(1,5-dimethyl-1*H*-pyrazol-3-yl)cyclohexyl)morpholin-2-yl)methyl)-6,6-dimethyl-3-azabizyklo[3.1.0]hexane-2,4-dion;
3-(((2*R*,5*S*)-5-(4-Chlorobenzyl)-4-(4-(4,5-dimethylthiazol-2-yl)cyclohexyl)morpholin-2-yl)methyl)-6,6-dimethyl-3-azabizyklo[3.1.0]hexane-2,4-dion;
3-((((2*S*,5*S*)-5-(4-Chlorobenzyl)-4-(4-(1,5-dimethyl-1*H*-pyrazol-3-yl)cyclohexyl)morpholin-2-yl)methyl)amino)-4-hydroxyzyklobut-3-ene-1,2-dion;
3-Amino-4-((((2*S*,5*S*)-5-(4-chlorobenzyl)-4-(4-(1,5-dimethyl-1*H*-pyrazol-3-yl)cyclohexyl)morpholin-2-yl)methyl)amino)zyklobut-3-ene-1,2-dion;
(2*R*,5*S*)-5-(4-Chlorobenzyl)-4-(4-(4,5-dimethylthiazol-2-yl)cyclohexyl)-2-((ethylsulfonyl)methyl)morpholin;
(2*R*,5*S*)-5-(4-Chlorobenzyl)-4-(4-(1,5-dimethyl-1*H*-pyrazol-3-yl)cyclohexyl)-2-((ethylsulfonyl)methyl)morpholin;
3-(((2*R*,5*S*)-5-(4-Chlorobenzyl)-4-(4-(1,5-dimethyl-1*H*-pyrazol-3-yl)cyclohexyl)morpholin-2-yl)methyl)-1-methylImidazolidine-2,4-dion;
(2*R*,5*S*)-5-(4-Chlorobenzyl)-4-(4-(4,5-dimethylthiazol-2-yl)cyclohexyl)-*N*-(1-methyl-1*H*-imidazol-4-yl)morpholin-2-carboxamid;
4-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)buttersäure;
(2*R*,5*S*)-5-(4-Chlorobenzyl)-4-(4-(4,5-dimethylthiazol-2-yl)cyclohexyl)-*N*-hydroxymorpholin-2-carboxamid;
(2*R*,5*S*)-5-(4-Chlorobenzyl)-*N*-(1,5-dimethyl-1*H*-pyrazol-3-yl)-4-(4-(4,5-dimethylthiazol-2-yl)cyclohexyl)morpholin-2-carboxamid;
5-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)valeriansäure;
(2*R*,5*S*)-5-(4-Chlorobenzyl)-*N*-(1,5-dimethyl-1*H*-pyrazol-3-yl)-4-(4-(1,5-dimethyl-1*H*-pyrazol-3-yl)cyclohexyl) morpholin-2-carboxamid;
*N*-((2*R*,5*S*)-5-(4-Chlorobenzyl)-4-(4-(4,5-dimethylthiazol-2-yl)cyclohexyl)morpholin-2-carbonyl)-*N*-methylglyzin;
(2*R*,5*S*)-4-(4-(1-Methyl-1*H*-1,2,4-triazol-3-yl)cyclohexyl)-2-((methylsulfonyl)methyl)-5-(4-(trifluoromethyl)benzyl)morpholin;
(2*R*,5*S*)-5-(4-Chlorobenzyl)-4-(4-(1-methyl-1H-Imidazol-4-yl)cyclohexyl)-2-((methylsulfonyl)methyl)morpholin;
(2*R*,5*S*)-4-(4-(1-Methyl-1*H*-1,2,4-triazol-3-yl)cyclohexyl)-5-(4-(triFluoromethyl)benzyl)morpholin-2-carbonsäure;
(2*S*,5*S*)-5-(4-Chlorobenzyl)-2-methyl-4-(4-(5-methyl-1*H*-pyrazol-3-yl)cyclohexyl)morpholin;
(2*R*,5*S*)-5-(4-Chlorobenzyl)-4-(4-(1-methyl-1*H*-1,2,4-triazol-3-yl)cyclohexyl)-2-((methylsulfonyl)methyl)morpholin;
(2*R*,5*S*)-5-(4-Chlorobenzyl)-4-(4-(1-methyl-1*H*-1,2,4-triazol-3-yl)cyclohexyl)morpholin-2-carbonsäure;
(2*R*,5*S*)-5-(4-Chlorobenzyl)-4-(4-(1-methyl-1*H*-1,2,3-triazol-4-yl)cyclohexyl)-2-((methylsulfonyl)methyl)morpholin;
Methyl (2*R*,5*S*)-5-(4-chlorobenzyl)-4-(4-(1-methyl-1*H*-1,2,3-triazol-4-yl)cyclohexyl)morpholin-2-carboxylat;
(2*R*,5*S*)-5-(4-Chlorobenzyl)-4-(4-(1-methyl-1*H*-1,2,3-triazol-4-yl)cyclohexyl)morpholin-2-carbonsäure;
(2*R*,5*S*)-5-(4-Chlorobenzyl)-4-(4-(1,5-dimethyl-1*H*-pyrazol-4-yl)cyclohexyl)-2-((methylsulfonyl)methyl)morpholin;
(2*R*,5*S*)-5-(4-Chlorobenzyl)-4-(4-(1-methyl-1*H*-pyrazol-3-yl)cyclohexyl)-2-((methylsulfonyl)methyl)morpholin;
(2*R*,5*S*)-5-(4-Chlorobenzyl)-4-(4-(2-methyl-2*H*-1,2,3-triazol-4-yl)cyclohexyl)-2-((methylsulfonyl)methyl)morpholin;
(2*R*,5*S*)-5-(4-Chlorobenzyl)-4-(4-(2-methyl-2*H*-tetrazol-5-yl)cyclohexyl)-2-((methylsulfonyl)methyl)morpholin;
(2*R*,5*S*)-5-(4-Chlorobenzyl)-*N,N*-dimethyl-4-(4-(5-methyl-1,3,4-thiadiazol-2-yl)cyclohexyl)morpholin-2-carboxamid;
(2*R*,5*S*)-5-(4-Chlorobenzyl)-4-(4-(5-methyl-1,3,4-thiadiazol-2-yl)cyclohexyl)-2-((methylsulfonyl)methyl)morpholin;
(2*R*,5*S*)-5-(4-Chlorobenzyl)-4-(4-(5-methoxy-1-methyl-1*H*-1,2,4-triazol-3-yl)cyclohexyl)-2-((methylsulfonyl) methyl)morpholin;
3-(4-((2*R*,5*S*)-5-(4-Chlorobenzyl)-2-((methylsulfonyl)methyl)morpholino)cyclohexyl)-1-methyl-1*H*-1,2,4-triazol-5-ol;
(2*R*,5*S*)-5-(4-Chlorobenzyl)-4-(4-(1-isopropyl-1*H*-pyrazol-3-yl)cyclohexyl)-2-((methylsulfonyl)methyl)morpholin;
(2*R*,5*S*)-5-(4-Chlorobenzyl)-4-(4-(5-methoxy-1-methyl-1*H*-1,2,4-triazol-3-yl)cyclohexyl)-*N*,*N*-dimethylmorpholin-2-carboxamid;
(2*R*,5*S*)-5-(4-Chlorobenzyl)-4-(4-(5-hydroxy-1-methyl-1*H*-1,2,4-triazol-3-yl)cyclohexyl)-*N*,*N*-dimethylmorpholin-2-carboxamid;
(2*R*,5*S*)-5-(4-Chlorobenzyl)-4-(4-(1,2-dimethyl-1*H*-imidazol-4-yl)cyclohexyl)-2-((methylsulfonyl)methyl)morpholin;
(2*R*,5*S*)-5-(4-Chlorobenzyl)-4-(4-(1-methyl-5-(trifluoromethyl)-1*H*-pyrazol-3-yl)cyclohexyl)-2-((methylsulfonyl) methyl)morpholin;
(2*R*,5*S*)-5-(4-Chlorobenzyl)-4-(4-(4,5-dimethylthiophen-2-yl)cyclohexyl)-2-((methylsulfonyl)methyl)morpholin;
4-(4-((2*R*,5*S*)-5-(4-Chlorobenzyl)-2-((methylsulfonyl)methyl)morpholino)cyclohexyl)-1-methyl-1*H*-pyrrol-2-carbonitril;
(2*R*,5*S*)-5-(4-Chlorobenzyl)-4-(4-(5-cyano-1-methyl-1*H*-pyrrol-3-yl)cyclohexyl)morpholin-2-carbonsäure;
(2*R*,5*S*)-5-(4-Chlorobenzyl)-4-(4-(1-ethyl-5-methyl-1*H*-pyrazol-3-yl)cyclohexyl)-2-((methylsulfonyl)methyl) morpholin;
(2*R*,5*S*)-5-(4-Chlorobenzyl)-4-(4-(1-ethyl-5-methyl-1*H*-pyrazol-3-yl)cyclohexyl)morpholin-2-carbonsäure;
(2*R*,5*S*)-5-(4-Chlorobenzyl)-*N*-ethyl-4-(4-(1-ethyl-5-methyl-1*H*-pyrazol-3-yl)cyclohexyl)morpholin-2-carboxamid;
(2*R*,5*S*)-5-(4-Chlorobenzyl)-4-(4-(4-methyl-1H-pyrazol-1-yl)cyclohexyl)-2-((methylsulfonyl)methyl)morpholin;
(2*R*,5*S*)-5-(4-Chlorobenzyl)-4-(4-(4-methyl-1*H*-pyrazol-1-yl)cyclohexyl)morpholin-2-carbonsäure;
*N*-(39-((((2*R*,5*S*)-5-(4-Chlorobenzyl)-4-(4-(4,5-dimethylthiazol-2-yl)cyclohexyl)morpholin-2-yl)methyl)sulfonyl)-37-oxo-3,6,9,12,15,18,21,24,27,30,33-undecaoxa-36-azanonatriacontyl)-5-((3a*S*,4*S*,6a*R*)-2-oxohexahydro-1*H*-thieno[3,4-*d*]Imidazol-4-yl)pentanamid;
(2*R*,5*S*)-5-(4-Chlorobenzyl)-4-(4-(1,5-dimethyl-1*H*-pyrazol-3-yl)cyclohexyl)-*N*-(1*H*-tetrazol-5-yl)morpholin-2-carboxamid;
(2*R*,5*S*)-4-(4-(1*H*-Tetrazol-5-yl)cyclohexyl)-5-(4-chlorobenzyl)-2-((methylsulfonyl)methyl)morpholin;
(2*R*,5*S*)-4-(4-(1*H*-1,2,4-Triazol-1-yl)cyclohexyl)-5-(4-chlorobenzyl)-2-((methylsulfonyl)methyl)morpholin;
(2*R*,5*S*)-4-(4-(1*H*-Pyrazol-1-yl)cyclohexyl)-5-(4-chlorobenzyl)-2-((methylsulfonyl)methyl)morpholin;
(2*R*,5*S*)-5-(4-Chlorobenzyl)-4-(4-(4-fluoro-1*H*-pyrazol-1-yl)cyclohexyl)-2-((methylsulfonyl)methyl)morpholin;
(2*R*,5*S*)-5-(4-Chlorobenzyl)-4-(4-(5-fluoro-1-methyl-1*H*-pyrazol-3-yl)cyclohexyl)morpholin-2-carbonsäure;
*N*1-(1,31-Bis(((2*R*,3*R*,4*R*,5*R*,6*R*)-3-acetamido-4,5-dihydroxy-6-(hydroxymethyl)tetrahydro-2*H*-pyran-2-yl) oxy)-16-((3-((3-(5-(((2*R*,3*R*,4*R*,5*R*,6*R*)-3-acetamido-4,5-dihydroxy-6-(hydroxymethyl)tetrahydro-2*H*-pyran-2-yl)oxy)pentanamido)propyl)amino)-3-oxopropoxy)methyl)-5,11,21,27-tetraoxo-14,18-dioxa-6,10,22,26-tetraazahentriacontan-16-yl)-*N*12-(3-(4-(((2*R*,5*S*)-5-(4-chlorobenzyl)-4-(4-(1,5-dimethyl-1*H*-pyrazol-3-yl)cyclohexyl)morpholin-2-carboxamido)methyl)-1*H*-1,2,3-triazol-1-yl)propyldDodecanediamid; und
(2*R*,5*S*)-5-(4-Chlorobenzyl)-4-(4-(1,5-dimethyl-1*H*-pyrazol-3-yl)cyclohexyl)-*N*-(5-(2-(2,6-dioxopiperidin-3-yl)-3-oxoisoindolin-4-yl)pentyl)morpholin-2-carboxamid;
oder ist ein Tautomer, Stereoisomer, pharmazeutisch zulässiges Salz, Solvat oder Polymorph davon.

**21.** Eine Verbindung nach Anspruch 2, ausgewählt aus einer Gruppe, die die folgenden Verbindungen umfasst:

(2*S*,5*S*)-5-(4-Chlorobenzyl)-2-methyl-4-(4-(5-methyloxazol-2-yl)cyclohexyl)morpholin;
(2*S*,5*S*)-5-(4-Chlorobenzyl)-4-(4-(1,5-dimethyl-1*H*-pyrazol-3-yl)cyclohexyl)-2-methylmorpholin;
(2*R*,5*S*)-5-(4-Chlorobenzyl)-4-(4-(5-methyloxazol-2-yl)cyclohexyl)-2-((methylsulfonyl)methyl)morpholin;
(2*R*,5*S*)-5-(4-Chlorobenzyl)-4-((1*r*,4*S*)-4-(1,5-dimethyl-1*H*-pyrazol-3-yl)cyclohexyl)-2-((methylsulfonyl)methyl) morpholin;
(2*R*,5*S*)-5-(4-Chlorobenzyl)-4-(4-(1,5-dimethyl-1*H*-pyrazol-3-yl)cyclohexyl)-2-(3-methylisoxazol-5-yl)morpholin;
(7*S*,9a*R*)-7-(4-Chlorobenzyl)-8-(4-(5-Methyloxazol-2-yl)cyclohexyl)octahydropyrazino[2,1-*c*][1,4]oxazin;
(7*S*,9a*R*)-7-(4-Chlorobenzyl)-8-(4-(1,5-dimethyl-1*H*-pyrazol-3-yl)cyclohexyl)octahydropyrazino[2,1-*c*][1,4]oxazin;
(2*R*,5*S*)-5-(4-Chlorobenzyl)-4-(4-(5-methyloxazol-2-yl)cyclohexyl)morpholin-2-carbonsäure;

Methyl (2*R*,5*S*)-5-(4-chlorobenzyl)-4-(4-(1,5-dimethyl-1*H*-pyrazol-3-yl)cyclohexyl)morpholin-2-carboxylat;
(2*R*,5*S*)-5-(4-Chlorobenzyl)-4-(4-(1,5-dimethyl-1*H*-pyrazol-3-yl)cyclohexyl)morpholin-2-carbonsäure;
(2*R*,5*S*)-5-(4-Chlorobenzyl)-4-(4-(1,5-dimethyl-1*H*-pyrazol-3-yl)cyclohexyl)-2-(1-(2,2,2-trifluoroethyl)-1*H*-1,2,3-triazol-4-yl)morpholin;
(2*R*,5*S*)-5-(4-Chlorobenzyl)-4-(4-(5-methyloxazol-2-yl)cyclohexyl)-2-(1-(2,2,2-trifluoroethyl)-1*H*-1,2,3-triazol-4-yl)morpholin;
(2*R*,5*S*)-5-(4-Chlorobenzyl)-4-(4-(1,5-dimethyl-1*H*-pyrazol-3-yl)cyclohexyl)-*N*-(37-oxo-41-((3a*S*,4*S*,6a*R*)-2-oxohexahydro-1*H*-thieno[3,4-*d*]Imidazol-4-yl)-3,6,9,12,15,18,21,24,27,30,33-undecaoxa-36-azahentetracontyl)morpholin-2-carboxamid;
(2*R*,5*S*)-5-(4-Chlorobenzyl)-4-(4-(1,5-dimethyl-1*H*-1,2,4-triazol-3-yl)cyclohexyl)-2-((methylsulfonyl)methyl) morpholin;
(2*R*,5*S*)-5-(4-Chlorobenzyl)-4-(4-(1,5-dimethyl-1*H*-pyrazol-3-yl)cyclohexyl)-2-(fluoromethyl)morpholin;
((2*R*,5*S*)-5-(4-Chlorobenzyl)-4-(4-(1,5-dimethyl-1*H*-pyrazol-3-yl)cyclohexyl)morpholin-2-yl)methanol;
(2*R*,5*S*)-5-(4-Chlorobenzyl)-*N*-ethyl-4-(4-(5-methyloxazol-2-yl)cyclohexyl)morpholin-2-carboxamid;
(2*R*,5*S*)-5-(4-Chlorobenzyl)-4-(4-(1,5-dimethyl-1*H*-pyrazol-3-yl)cyclohexyl)-*N*-ethylmorpholin-2-carboxamid;
(2*R*,5*S*)-5-(4-Chlorobenzyl)-4-(4-(1,5-dimethyl-1*H*-pyrazol-3-yl)cyclohexyl)-*N,N*-dimethylmorpholin-2-carboxamid;
(2*R*,5*S*)-5-(4-Chlorobenzyl)-*N,N*-dimethyl-4-(4-(5-methyloxazol-2-yl)cyclohexyl)morpholin-2-carboxamid;
(2*R*,5*S*)-5-(4-Chlorobenzyl)-4-(4-(1,5-dimethyl-1*H*-pyrazol-3-yl)cyclohexyl)-2-(5-methyl-1,3,4-oxadiazol-2-yl) morpholin;
(2*R*,5*S*)-*N*-Acetyl-5-(4-chlorobenzyl)-4-(4-(1,5-dimethyl-1*H*-pyrazol-3-yl)cyclohexyl)morpholin-2-carbohydrazid;
(2*R*,5*S*)-5-(4-Chlorobenzyl)-2-(5-methyl-1,3,4-oxadiazol-2-yl)-4-(4-(5-methyloxazol-2-yl)cyclohexyl)morpholin;
(2*R*,5*S*)-*N'*-Acetyl-5-(4-chlorobenzyl)-4-(4-(5-methyloxazol-2-yl)cyclohexyl)morpholin-2-carbohydrazid;
(2*R*,5*S*)-5-(4-Chlorobenzyl)-4-(4-(4,5-dimethyloxazol-2-yl)cyclohexyl)-2-((methylsulfonyl)methyl)morpholin;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-1,5-dimethyl-1*H*-pyrazol-3-carboxamid;
(2*S*,5*S*)-5-(4-Chlorobenzyl)-4-(4-(1,5-dimethyl-1*H*-pyrazol-3-yl)cyclohexyl)-2-ethinylmorpholin;
*N*-(35-(4-((2*R*,5*S*)-5-(4-Chlorobenzyl)-4-(4-(1,5-dimethyl-1*H*-pyrazol-3-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]imidazol-4-yl)pentanamid;
(2*R*,5*S*)-5-(4-Chlorobenzyl)-4-(4-(1,5-dimethyl-1*H*-pyrazol-3-yl)cyclohexyl)-*N*-isopropylmorpholin-2-carboxamid;
(2*R*,5*S*)-5-(4-Chlorobenzyl)-4-(4-(1,5-dimethyl-1*H*-pyrazol-3-yl)cyclohexyl)-*N*-methylmorpholin-2-carboxamid;
(2*R*,5*S*)-5-(4-Chlorobenzyl)-4-(4-(4,5-dimethyloxazol-2-yl)cyclohexyl)-*N,N*-dimethylmorpholin-2-carboxamid;
(2*R*,5*S*)-5-(4-Chlorobenzyl)-4-(4-(4,5-dimethyloxazol-2-yl)cyclohexyl)-*N*-isopropylmorpholin-2-carboxamid;
(2*R*,5*S*)-5-(4-Chlorobenzyl)-*N*-methyl-4-(4-(5-methyloxazol-2-yl)cyclohexyl)morpholin-2-carboxamid;
(2*R*,5*S*)-5-(4-Chlorobenzyl)-4-(4-(4,5-dimethyloxazol-2-yl)cyclohexyl)-*N*-ethylmorpholin-2-carboxamid;
(2*R*,5*S*)-5-(4-Chlorobenzyl)-4-(4-(5-fluoro-1-methyl-1*H*-pyrazol-3-yl)cyclohexyl)-2-((methylsulfonyl)methyl) morpholin;
(2*R*,5*S*)-5-(4-Chlorobenzyl)-4-(4-(4,5-dimethylthiazol-2-yl)cyclohexyl)-2-((methylsulfonyl)methyl)morpholin;
(2*R*,5*S*)-5-(4-Chlorobenzyl)-2-((methylsulfonyl)methyl)-4-(4-(5-methylthiazol-2-yl)cyclohexyl)morpholin;
(2*R*,5*S*)-5-(4-Chlorobenzyl)-4-(4-(4,5-dimethyloxazol-2-yl)cyclohexyl)morpholin-2-carbonsäure;
(2*R*,5*S*)-5-(4-Chlorobenzyl)-4-(4-(4,5-dimethylthiazol-2-yl)cyclohexyl)morpholin-2-carbonsäure;
(2*R*,5*S*)-5-(4-Chlorobenzyl)-4-(4-(1,5-dimethyl-1*H*-pyrazol-3-yl)cyclohexyl)-2-((prop-2-yn-1-yloxy)methyl)morpholin;
(2*R*,5*S*)-5-(4-Chlorobenzyl)-4-(4-(4,5-dimethylthiazol-2-yl)cyclohexyl)-*N*-ethylmorpholin-2-carboxamid;
*N*-(35-(4-((((2*R*,5*S*)-5-(4-chlorobenzyl)-4-(4-(1,5-dimethyl-1*H*-pyrazol-3-yl)cyclohexyl)morpholin-2-yl)methoxy)methyl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]Imidazol-4-yl)pentanamid;
(2*R*,5*S*)-2-(Azidomethyl)-5-(4-chlorobenzyl)-4-(4-(1,5-dimethyl-1H-pyrazol-3-yl)cyclohexyl)morpholin;
(2*R*,5*S*)-5-(4-Chlorobenzyl)-4-(4-(1,5-dimethyl-1*H*-pyrazol-3-yl)cyclohexyl)-*N*-(prop-2-yn-1-yl)morpholin-2-carboxamid;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-1*H*-pyrazole-3-carboxamid;
(2*R*,5*S*)-5-(4-Chlorobenzyl)-4-(4-(1,5-dimethyl-1*H*-pyrazol-3-yl)cyclohexyl)-*N*-((1-(37-oxo-41-((3a*S*,4*S*,6a*R*)-2-oxohexahydro-1*H*-thieno[3,4-*d*]Imidazol-4-yl)-3,6,9,12,15,18,21,24,27,30,33-undecaoxa-36-azahentetracontyl)-1*H*-1,2,3-triazol-4-yl)methyl)morpholin-2-carboxamid;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-2*H*-1,2,3-triazole-4-carboxamid;
3-(((2*R*,5*S*)-5-(4-Chlorobenzyl)-4-(4-(1,5-dimethyl-1*H*-pyrazol-3-yl)cyclohexyl)morpholin-2-yl)methyl)imidazolidine-2,4-dion;
(2*R*,5*S*)-5-(4-Chlorobenzyl)-4-(4-(1,5-dimethyl-1*H*-1,2,4-triazol-3-yl)cyclohexyl)-*N*-ethylmorpholin-2-carboxamid;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-1*H*-imidazol-4-carboxamid;
(2*R*,5*S*)-5-(4-Chlorobenzyl)-4-(4-(4,5-dimethylthiazol-2-yl)zyklohex-3-en-1-yl)-2-((methylsulfonyl)methyl)morpholin;
(2*R*,5*S*)-5-(4-Chlorobenzyl)-4-(4-(4,5-dimethylthiazol-2-yl)zyklohex-3-en-1-yl)-*N*-ethylmorpholin-2-carboxamid;
(2*R*,5*S*)-5-(4-Chlorobenzyl)-4-(4-(4,5-dimethylthiazol-2-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)morpholin-2-carboxamid;
(2*R*,5*S*)-2-(Azidomethyl)-5-(4-chlorobenzyl)-4-(4-(4,5-dimethylthiazol-2-yl)cyclohexyl)morpholin;
(2*R*,5*S*)-5-(4-Chlorobenzyl)-4-(4-(4,5-dimethylthiazol-2-yl)cyclohexyl)-*N*-((1-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undecaoxa-36-azahentetracontyl)-1*H*-1,2,3-triazol-4-yl)methyl)morpholin-2-carboxamid;
*N*-(((2*S*,5*S*)-5-(4-Chlorobenzyl)-4-(4-(4,5-dimethylthiazol-2-yl)cyclohexyl)morpholin-2-yl)methyl)-1*H*-1,2,3-triazol-5-carboxamid;
*N*-(((2*S*,5*S*)-5-(4-Chlorobenzyl)-4-(4-(1,5-dimethyl-1*H*-pyrazol-3-yl)cyclohexyl)morpholin-2-yl)methyl)-1-methyl-1*H*-imidazol-4-carboxamid;
Methyl 2-((2*S*,5*S*)-5-(4-chlorobenzyl)-4-(4-(1,5-dimethyl-1*H*-pyrazol-3-yl)cyclohexyl)morpholin-2-yl)acetat;
2-((2*S*,5*S*)-5-(4-Chlorobenzyl)-4-(4-(1,5-dimethyl-1*H*-pyrazol-3-yl)cyclohexyl)morpholin-2-yl)essigsäure;
2-((2*S*,5*S*)-5-(4-Chlorobenzyl)-4-(4-(1,5-dimethyl-1*H*-pyrazol-3-yl)cyclohexyl)morpholin-2-yl)-*N*-ethylacetamid;
(2*R*,5*S*)-5-(4-Chlorobenzyl)-4-(4-(1,5-dimethyl-1*H*-1,2,4-triazol-3-yl)cyclohexyl)morpholin-2-carbonsäure;
(2*R*,5*S*)-5-(4-Bromobenzyl)-4-(4-(1,5-dimethyl-1*H*-pyrazol-3-yl)cyclohexyl)-2-((methylsulfonyl)methyl)morpholin;
*N*-(((2*R*,5*S*)-5-(4-Chlorobenzyl)-4-(4-(1,5-dimethyl-1*H*-pyrazol-3-yl)cyclohexyl)morpholin-2-yl)methyl)methanesulfonamid;
(2*R*,5*S*)-5-(4-Bromobenzyl)-4-(4-(1,5-dimethyl-1*H*-pyrazol-3-yl)cyclohexyl)morpholin-2-carbonsäure;
(2*R*,5*S*)-5-(4-Bromobenzyl)-4-(4-(5-methyloxazol-2-yl)cyclohexyl)morpholin-2-carbonsäure;
(2*R*,5*S*)-5-(4-Bromobenzyl)-4-(4-(5-methyloxazol-2-yl)cyclohexyl)-2-((methylsulfonyl)methyl)morpholin;
(2*R*,5*S*)-5-(4-Bromobenzyl)-4-(4-(1,5-dimethyl-1*H*-1,2,4-triazol-3-yl)cyclohexyl)-2-((methylsulfonyl)methyl) morpholin;
(2*R*,5*S*)-5-(4-Bromobenzyl)-4-(4-(1,5-dimethyl-1*H*-1,2,4-triazol-3-yl)cyclohexyl)morpholin-2-carbonsäure;
*N*-(((2*R*,5*S*)-5-(4-Chlorobenzyl)-4-(4-(1,5-dimethyl-1*H*-pyrazol-3-yl)cyclohexyl)morpholin-2-yl)methyl)-1,1,1-trifluoromethanesulfonamid;
(2*R*,5*S*)-5-(4-Bromobenzyl)-4-(4-(1,5-dimethyl-1*H*-pyrazol-3-yl)cyclohexyl)-*N*-ethylmorpholin-2-carboxamid;
(2iR,5*S*)-*N*-(2-(3-(But-3-yn-1-yl)-3*H*-diazirin-3-yl)ethyl)-5-(4-chlorobenzyl)-4-(4-(1,5-dimethyl-1*H*-pyrazol-3-yl) cyclohexyl)morpholin-2-carboxamid;
2-(((2*R*,5*S*)-5-(4-Chlorobenzyl)-4-(4-(4,5-dimethylthiazol-2-yl)cyclohexyl)morpholin-2-yl)methoxy)essigsäure;
2-((2*R*,5*S*)-5-(4-Chlorobenzyl)-4-(4-(1,5-dimethyl-1*H*-pyrazol-3-yl)cyclohexyl)morpholin-2-yl)propan-2-ol;
(2*R*,5*S*)-5-(4-Chlorobenzyl)-4-(4-(1,5-dimethyl-1*H*-pyrazol-3-yl)cyclohexyl)-2-(5-methyl-1H-pyrazol-3-yl)morpholin;
(2*R*,5*R*)-2-(4-Chlorobenzyl)-1-(4-(1,5-dimethyl-1*H*-pyrazol-3-yl)cyclohexyl)-5-(methylsulfonyl)piperidin;
(2*R*,5*S*)-5-(4-Chlorobenzyl)-4-(4-(1,5-dimethyl-1*H*-pyrazol-3-yl)cyclohexyl)morpholin-2-carboxamid;
2-(4-((2*R*,5*R*)-2-(4-Chlorobenzyl)-5-(methylsulfonyl)piperidin-1-yl)cyclohexyl)-5-methyloxazol;
(2*R*,5*S*)-5-(4-Chlorobenzyl)-4-(4-(1,5-dimethyl-1*H*-pyrazol-3-yl)cyclohexyl)-2-((methylsulfinyl)methyl)morpholin;
(2*R*,5*S*)-5-(4-Chlorobenzyl)-4-(4-(1,5-dimethyl-1*H*-pyrazol-3-yl)cyclohexyl)-2-((methylthio)methyl)morpholin;
1-(((2*R*,5*S*)-5-(4-Chlorobenzyl)-4-(4-(1,5-dimethyl-1*H*-pyrazol-3-yl)cyclohexyl)morpholin-2-yl)methyl)pyrrolidine-2,5-dion;
(2*S*,5*S*)-5-(4-Chlorobenzyl)-4-(4-(4,5-dimethylthiazol-2-yl)cyclohexyl)-2-ethinylmorpholin;
2-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)essigsäure;
3-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)propionsäure;
(2*R*,5*S*)-5-(4-Chlorobenzyl)-4-(4-(1,5-dimethyl-1*H*-pyrazol-3-yl)zyklohex-3-en-1-yl)-2-((methylsulfonyl)methyl) morpholin;
*N*-(35-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)-3,6,9,12,15,18,21,24,27,30,33-undecaoxapentatriacontyl)-5-((3a*S*,4*S*,6a*R*)-2-oxohexahydro-1*H*-thieno[3,4-*d*]Imidazol-4-yl)pentanamid;
2-((2*S*,5*S*)-5-(4-Chlorobenzyl)-4-(4-(4,5-dimethylthiazol-2-yl)cyclohexyl)morpholin-2-yl)essigsäure;
3-((((2*R*,5*S*)-5-(4-Chlorobenzyl)-4-(4-(4,5-dimethylthiazol-2-yl)cyclohexyl)morpholin-2-yl)methyl)sulfonyl)propionsäure;
3-(((2*R*,5*S*)-5-(4-Chlorobenzyl)-4-(4-(1,5-dimethyl-1*H*-pyrazol-3-yl)cyclohexyl)morpholin-2-yl)methyl)-6,6-dimethyl-3-azabizyklo[3.1.0]hexane-2,4-dion;
3-(((2*R*,5*S*)-5-(4-Chlorobenzyl)-4-(4-(4,5-dimethylthiazol-2-yl)cyclohexyl)morpholin-2-yl)methyl)-6,6-dimethyl-3-azabizyklo[3.1.0]hexane-2,4-dion;
3-((((2*S*,5*S*)-5-(4-Chlorobenzyl)-4-(4-(1,5-dimethyl-1*H*-pyrazol-3-yl)cyclohexyl)morpholin-2-yl)methyl)amino)-4-hydroxyzyklobut-3-ene-1,2-dion;
3-Amino-4-((((2*S*,5*S*)-5-(4-chlorobenzyl)-4-(4-(1,5-dimethyl-1*H*-pyrazol-3-yl)cyclohexyl)morpholin-2-yl)methyl)amino)zyklobut-3-ene-1,2-dion;
(2*R*,5*S*)-5-(4-Chlorobenzyl)-4-(4-(4,5-dimethylthiazol-2-yl)cyclohexyl)-2-((ethylsulfonyl)methyl)morpholin;
(2*R*,5*S*)-5-(4-Chlorobenzyl)-4-(4-(1,5-dimethyl-1*H*-pyrazol-3-yl)cyclohexyl)-2-((ethylsulfonyl)methyl)morpholin;
3-(((2*R*,5*S*)-5-(4-Chlorobenzyl)-4-(4-(1,5-dimethyl-1*H*-pyrazol-3-yl)cyclohexyl)morpholin-2-yl)methyl)-1-methylImidazolidine-2,4-dion;
(2*R*,5*S*)-5-(4-Chlorobenzyl)-4-(4-(4,5-dimethylthiazol-2-yl)cyclohexyl)-*N*-(1-methyl-1*H*-imidazol-4-yl)morpholin-2-carboxamid;
4-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)buttersäure;
(2*R*,5*S*)-5-(4-Chlorobenzyl)-4-(4-(4,5-dimethylthiazol-2-yl)cyclohexyl)-*N*-hydroxymorpholin-2-carboxamid;
(2*R*,5*S*)-5-(4-Chlorobenzyl)-*N*-(1,5-dimethyl-1*H*-pyrazol-3-yl)-4-(4-(4,5-dimethylthiazol-2-yl)cyclohexyl)morpholin-2-carboxamid;
5-(4-((2*R*,5*S*)-5-(4-Chlorobenzyl)-4-(4-(4,5-dimethylthiazol-2-yl)cyclohexyl)morpholin-2-yl)-1*H*-1,2,3-triazol-1-yl)valeriansäure;
(2*R*,5*S*)-5-(4-Chlorobenzyl)-*N*-(1,5-dimethyl-1*H*-pyrazol-3-yl)-4-(4-(1,5-dimethyl-1*H*-pyrazol-3-yl)cyclohexyl) morpholin-2-carboxamid; und
*N*-((2*R*,5*S*)-5-(4-Chlorobenzyl)-4-(4-(4,5-dimethylthiazol-2-yl)cyclohexyl)morpholin-2-carbonyl)-*N*-methylglyzin;
oder ein Tautomer, Stereoisomer, pharmazeutisch zulässiges Salz, Solvat oder Polymorph davon.

**22.** Eine Verbindung nach Anspruch 2 oder Anspruch 21, ausgewählt aus der Gruppe, die die folgenden Verbindungen umfasst:

(2*S*,5*S*)-5-(4-Chlorobenzyl)-2-methyl-4-(4-(5-methyloxazol-2-yl)cyclohexyl)morpholin;
(2*S*,5*S*)-5-(4-Chlorobenzyl)-4-(4-(1,5-dimethyl-1*H*-pyrazol-3-yl)cyclohexyl)-2-methylmorpholin;
(2*R*,5*S*)-5-(4-Chlorobenzyl)-4-(4-(5-methyloxazol-2-yl)cyclohexyl)-2-((methylsulfonyl)methyl)morpholin;
(2*R*,5*S*)-5-(4-Chlorobenzyl)-4-((1*r*,4*S*)-4-(1,5-dimethyl-1*H*-pyrazol-3-yl)cyclohexyl)-2-((methylsulfonyl)methyl) morpholin;
(2*R*,5*S*)-5-(4-Chlorobenzyl)-4-(4-(1,5-dimethyl-1*H*-pyrazol-3-yl)cyclohexyl)-2-(3-methylisoxazol-5-yl)morpholin;
(2*R*,5*S*)-5-(4-Chlorobenzyl)-4-(4-(5-methyloxazol-2-yl)cyclohexyl)morpholin-2-carbonsäure;
(2*R*,5*S*)-5-(4-Chlorobenzyl)-4-(4-(1,5-dimethyl-1*H*-pyrazol-3-yl)cyclohexyl)morpholin-2-carbonsäure;
(2*R*,5*S*)-5-(4-Chlorobenzyl)-4-(4-(1,5-dimethyl-1*H*-1,2,4-triazol-3-yl)cyclohexyl)-2-((methylsulfonyl)methyl) morpholin;
(2*R*,5*S*)-5-(4-Chlorobenzyl)-4-(4-(1,5-dimethyl-1*H*-pyrazol-3-yl)cyclohexyl)-2-(fluoromethyl)morpholin;
((2*R*,5*S*)-5-(4-Chlorobenzyl)-4-(4-(1,5-dimethyl-1*H*-pyrazol-3-yl)cyclohexyl)morpholin-2-yl)methanol;
(2*R*,5*S*)-5-(4-Chlorobenzyl)-4-(4-(1,5-dimethyl-1*H*-pyrazol-3-yl)cyclohexyl)-*N,N*-dimethylmorpholin-2-carboxamid;
(2*R*,5*S*)-5-(4-Chlorobenzyl)-4-(4-(4,5-dimethyloxazol-2-yl)cyclohexyl)-2-((methylsulfonyl)methyl)morpholin;

(2*R*,5*S*)-5-(4-Chlorobenzyl)-4-(4-(1,5-dimethyl-1*H*-pyrazol-3-yl)cyclohexyl)-*N*-methylmorpholin-2-carboxamid;
(2*R*,5*S*)-5-(4-Chlorobenzyl)-4-(4-(5-fluoro-1-methyl-1*H*-pyrazol-3-yl)cyclohexyl)-2-((methylsulfonyl)methyl) morpholin;
(2*R*,5*S*)-5-(4-Chlorobenzyl)-4-(4-(4,5-dimethylthiazol-2-yl)cyclohexyl)-2-((methylsulfonyl)methyl)morpholin;
(2*R*,5*S*)-5-(4-Chlorobenzyl)-4-(4-(4,5-dimethyloxazol-2-yl)cyclohexyl)morpholin-2-carbonsäure;
(2*R*,5*S*)-5-(4-Chlorobenzyl)-4-(4-(4,5-dimethylthiazol-2-yl)cyclohexyl)morpholin-2-carbonsäure;
(2*R*,5*S*)-5-(4-Chlorobenzyl)-4-(4-(4,5-dimethylthiazol-2-yl)zyklohex-3-en-1-yl)-2-((methylsulfonyl)methyl)morpholin;
2-((2*S*,5*S*)-5-(4-Chlorobenzyl)-4-(4-(1,5-dimethyl-1*H*-pyrazol-3-yl)cyclohexyl)morpholin-2-yl)essigsäure;
(2*R*,5*S*)-5-(4-Chlorobenzyl)-4-(4-(1,5-dimethyl-1*H*-1,2,4-triazol-3-yl)cyclohexyl)morpholin-2-carbonsäure;
(2*R*,5*S*)-5-(4-Bromobenzyl)-4-(4-(1,5-dimethyl-1*H*-pyrazol-3-yl)cyclohexyl)-2-((methylsulfonyl)methyl)morpholin;
*N*-(((2*R*,5*S*)-5-(4-Chlorobenzyl)-4-(4-(1,5-dimethyl-1*H*-pyrazol-3-yl)cyclohexyl)morpholin-2-yl)methyl)methanesulfonamid;
(2*R*,5*S*)-5-(4-Bromobenzyl)-4-(4-(1,5-dimethyl-1*H*-pyrazol-3-yl)cyclohexyl)morpholin-2-carbonsäure;
(2*R*,5*S*)-5-(4-Bromobenzyl)-4-(4-(5-methyloxazol-2-yl)cyclohexyl)morpholin-2-carbonsäure;
(2*R*,5*S*)-5-(4-Bromobenzyl)-4-(4-(5-methyloxazol-2-yl)cyclohexyl)-2-((methylsulfonyl)methyl)morpholin;
(2*R*,5*S*)-5-(4-Bromobenzyl)-4-(4-(1,5-dimethyl-1*H*-1,2,4-triazol-3-yl)cyclohexyl)-2-((methylsulfonyl)methyl) morpholin;
(2*R*,5*S*)-5-(4-Bromobenzyl)-4-(4-(1,5-dimethyl-1*H*-1,2,4-triazol-3-yl)cyclohexyl)morpholin-2-carbonsäure;
(2*R*,5*R*)-2-(4-Chlorobenzyl)-1-(4-(1,5-dimethyl-1*H*-pyrazol-3-yl)cyclohexyl)-5-(methylsulfonyl)piperidin;
(2*R*,5*S*)-5-(4-Chlorobenzyl)-4-(4-(1,5-dimethyl-1*H*-pyrazol-3-yl)cyclohexyl)morpholin-2-carboxamid;
2-(4-((2*R*,5*R*)-2-(4-Chlorobenzyl)-5-(methylsulfonyl)piperidin-1-yl)cyclohexyl)-5-methyloxazol;
(2*R*,5*S*)-5-(4-Chlorobenzyl)-4-(4-(1,5-dimethyl-1*H*-pyrazol-3-yl)cyclohexyl)-2-((methylsulfinyl)methyl)morpholin;
(2*R*,5*S*)-5-(4-Chlorobenzyl)-4-(4-(1,5-dimethyl-1*H*-pyrazol-3-yl)cyclohexyl)-2-((methylthio)methyl)morpholin;
(2*R*,5*S*)-5-(4-Chlorobenzyl)-4-(4-(1,5-dimethyl-1*H*-pyrazol-3-yl)zyklohex-3-en-1-yl)-2-((methylsulfonyl)methyl) morpholin;
2-((2*S*,5*S*)-5-(4-Chlorobenzyl)-4-(4-(4,5-dimethylthiazol-2-yl)cyclohexyl)morpholin-2-yl)Essigsäure; und
(2*R*,5*S*)-5-(4-Chlorobenzyl)-4-(4-(1,5-dimethyl-1*H*-pyrazol-3-yl)cyclohexyl)-2-((ethylsulfonyl)methyl)morpholin;
oder ein Tautomer, Stereoisomer, pharmazeutisch zulässiges Salz, Solvat oder Polymorph davon.

**23.** Eine pharmazeutische Zubereitung, die eine therapeutisch wirksame Menge mindestens einer Verbindung nach einem der Ansprüche 1 bis 22 und einen pharmazeutisch akzeptablen Trägerstoff dafür enthält.

**24.** Eine Verbindung nach einem der Ansprüche 1 bis 22 oder eine pharmazeutische Zubereitung nach Anspruch 23 zur Verwendung bei der Behandlung oder Vorbeugung einer Krankheit, einer Störung oder eines Zustands, der mit einer abweichenden Expression oder Aktivität des Chitinase-ähnlichen Proteins YKL-40 verbunden ist.

**25.** Eine Verbindung zur Verwendung nach Anspruch 24, wobei die Krankheit, Störung oder der Zustand aus einer Gruppe ausgewählt ist, die bestehend aus: Krebs, allergischen Krankheiten, akuten und chronischen entzündlichen Krankheiten, Autoimmunkrankheiten, Zahnerkrankungen, neurologischen Krankheiten, Stoffwechselkrankheiten, Leberkrankheiten, Nierenkrankheiten, polyzystischem Ovarsyndrom, Endometriose, Asthma und fibrotischen Störungen.

**26.** Eine Verbindung zur Verwendung nach Anspruch 25, wobei die Krankheit, Störung oder der Zustand Krebs ist, ausgewählt aus der Gruppe bestehend aus: Glioblastom, Brustkrebs, Dickdarmkrebs, Nierenkrebs, Gebärmutterkrebs, primärem und metastasiertem Lungenkrebs, Mesotheliom, Osteosarkom, malignem Melanom, Eierstockkrebs, Gebärmutterhalskrebs, Prostatakrebs, Leberkrebs, Magenkrebs, metastasiertem Nierenkrebs, Leukämie, Lymphom, Oligodendrogliom, Glioblastom und Keimzelltumoren.

**27.** Eine Verbindung zur Verwendung nach Anspruch 25, wobei die Krankheit, Störung oder der Zustand eine allergische Krankheit ist, ausgewählt aus der Gruppe bestehend aus: Asthma, allergischer Rhinitis, saisonaler allergischer Rhinitis, chronischer Rhinosinusitis mit oder ohne Nasenpolypen, Konjunktivitis, Keratokonjunktivitis, saisonaler allergischer Konjunktivitis, Trockenes-Auge-Syndrom, eosinophiler Ösophagitis, Zöliakie, Nahrungsmittelallergie, Reizdarmsyndrom, Reizdarmerkrankung, atopischem Ekzem, atopischer Dermatitis, allergischer Kontaktdermatitis, eosinophiler Otitis media, eosinophiler Pneumonie und IgG4-assozierten Krankheiten.

**28.** Eine Verbindung zur Verwendung nach Anspruch 25, wobei die Krankheit, Störung oder der Zustand eine akute oder chronische entzündliche Krankheit ist, ausgewählt aus der Gruppe bestehend aus: Pilzerkrankungen, parasitären Infektionen, Zöliakie, mikroskopischer Kolitis, chronisch obstruktiver Lungenerkrankung (COPD), idiopathischer Lungenfibrose, interstitiellen Lungenerkrankungen, zystischer Fibrose, Hermansky-Pudlak-Syndrom und Alzheimer-Krankheit.

**29.** Eine Verbindung zur Verwendung nach Anspruch 25, wobei die Krankheit, Störung oder der Zustand eine Autoimmunerkrankung ist, ausgewählt aus der Gruppe bestehend aus: entzündlicher Darmerkrankung, Colitis ulcerosa, Morbus Crohn, rheumatoider Arthritis, Osteoarthritis, Psoriasis, Sklerodermie, multipler Sklerose, Sjögren-Syndrom, Atherosklerose und Sarkoidose.

**30.** Eine Verbindung zur Verwendung nach Anspruch 25, wobei die Krankheit, Störung oder der Zustand eine Stoffwechselerkrankung ist, ausgewählt aus der Gruppe bestehend aus: insulinabhängigem Diabetes mellitus und nicht insulinabhängigem Diabetes mellitus.

**31.** Eine Verbindung zur Verwendung nach Anspruch 25, wobei die Krankheit, Störung oder der Zustand eine Lebererkrankung ist, ausgewählt aus der Gruppe bestehend aus: nichtalkoholischer Fettlebererkrankung, nichtalkoholischer Steatohepatitis, durch Hepatitis-C-Virus induzierter Fibrose und Zirrhose und alkoholischer Fibrose.

**32.** Eine Verbindung zur Verwendung nach Anspruch 25, wobei die Krankheit, Störung oder der Zustand eine Nierenerkrankung ist, ausgewählt aus der Gruppe bestehend aus: Nephropathie (z. B. diabetischer Nephropathie), fokal segmentaler Glomerulosklerose, tubulointerstitieller Fibrose, Posttransplantationsfibrose und retroperitonealer Fibrose (Ormondsche Krankheit).

**33.** Eine Verbindung zur Verwendung nach Anspruch 25, wobei die Krankheit, Störung oder der Zustand eine fibrotische Störung ist, insbesondere idiopathische Lungenfibrose (IPF).

**34.** Eine Verbindung zur Verwendung nach Anspruch 25, wobei die Krankheit, die Störung oder der Zustand eine Speicherkrankheit ist, ausgewählt aus der Gruppe bestehend aus: Gaucher-Krankheit, Fabry-Krankheit, lysosomalen Speicherstörungen, Niemann-Pick-Krankheit, nephropathischer Cystinose und X-vererbter Globotriaosylceramidose.

**35.** Ein Verfahren zur Identifizierung von Verbindungen, die die Wechselwirkung mit dem Chitinase-ähnlichen Protein YKL-40 und Asialofetuin beeinträchtigen und zwar durch:

(1) mit einem Bindungskonkurrenz-Assay, der (i) eine Verbindung nach einem der Ansprüche 1 bis 22 und ein (ii) biotinyliertes Asialofetuin mit YKL-40 oder einem Derivat von YKL-40 mit einem Reinigungsmarker umfasst; und
(2) eine Bewertung des sich ergebenden AlphaScreen-Lumineszenzsignals; wobei eine Abnahme des Emissionssignals vom hohen Niveau bei der niedrigsten Konzentration des Inhibitors auf das niedrigere Niveau (vermindert um den Wert von mindestens dreifacher Standardabweichung des Rauschens, das bei den Plateauwerten bei den höchsten Konzentrationen des Inhibitors in den Reaktionsdiagrammen für IC50-Bestimmungen angezeigt wird) bei der höchsten Konzentration es Inhibitors anzeigt, dass die Verbindung die Wechselwirkung mit dem Chitinase-ähnlichen Protein YKL-40 und Asialofetuin beeinträchtigt.

## Revendications

**1.** Composé représenté par la formule structurelle (I) :

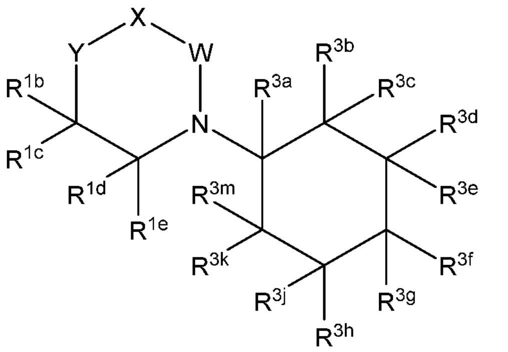

(I)

dans lequel :

W représente -C(-$R^{1f}$)(-$R^{1g}$)- ;
X représente -C(-$R^{1h}$)(-$R^{1j}$)- ;
Y représente -C(-$R^{1k}$)(-$R^{1m}$)-, -N(-$R^{1k}$)-, ou -O- ;
$R^{1b}$ représente -H ;
$R^{1c}$ représente -H ;
$R^{1d}$ représente 4-$R^{10}$-benzyle ;
$R^{1e}$ représente -H ;
$R^{1f}$ représente -H ;
$R^{1g}$ représente -H ;
$R^{1h}$ est choisi dans le groupe constitué de $C_1$-$C_6$alkyle, $C_2$-$C_6$alcényle, $C_2$-$C_6$alcynyle, hydroxy-$C_1$-$C_6$alkyle, azido-$C_1$-$C_6$alkyle, hydroxycarbonyl-$C_1$-$C_3$ alkoxy-$C_1$-$C_6$alkyle, $C_3$-$C_6$ alkynyloxy-$C_1$-$C_6$ alkyle, halo-$C_1$-$C_6$alkyle, $C_1$-$C_3$alkylthio-$C_1$-$C_6$alkyle, $C_1$-$C_3$alkylsulfinyl-$C_1$-$C_6$alkyle, $C_1$-$C_3$alkylsulfonyl-$C_1$-$C_6$alkyle, hydroxycarbonyl-$C_1$-$C_3$ alkylsulfonyl-$C_1$-$C_6$alkyle, hydroxycarbonyl-$C_1$-$C_6$alkyle, $C_1$-$C_3$ alkylcarbonyl-$C_1$-$C_6$alkyle, $C_1$-$C_3$alkylaminocarbonyl-$C_1$-$C_6$alkyle, di($C_1$-$C_3$alkyl)aminocarbonyl-$C_1$-$C_6$alkyle, $C_1$-$C_3$alkylsulfonylamino-$C_1$-$C_6$alkyle, halo-$C_1$-$C_3$alkylsulfonylamino-$C_1$-$C_6$alkyle, hétéroaryl-carbonylamino-$C_1$-$C_6$alkyle à 5 ou 6 chaînons monocycliques, hétéroaryle monocyclique à 5 ou 6 chaînons-$C_1$-$C_3$ alcoxy-$C_1$-$C_6$alkyle, hétéroaryle monocyclique à 5 ou 6 chaînons-$C_1$-$C_6$alkyle, hydroxycarbonyle, $C_1$-$C_3$alcoxycarbonyle, aminocarbonyle, $C_1$-$C_3$ alkylaminocarbonyle, di($C_1$-$C_3$alkyle)aminocarbonyle, $C_3$-$C_6$ alcynylaminocarbonyle, hydroxycarbonyle-$C_1$-$C_3$alkyl($C_1$-$C_3$ alkyle)aminocarbonyle, (biotinyl-$PEG_{11}$)aminocarbonyle, (biotinyl-$PEG_{11}$)aminocarbonyle-$C_1$-$C_3$ alkylsulfonyle-$C_1$-$C_6$alkyle, hydroxylaminocarbonyle, *N*2-acétylhydrazinocarbonyle, (hétéroaryle monocyclique à 5 ou 6 chaînons)-$C_1$-$C_3$alkylaminocarbonyle, (hétéroaryle monocyclique à 5 ou 6 chaînons) aminocarbonyle, $C_1$-$C_3$alkylsulfonyle, hétéroaryle monocyclique à 5 ou 6 chaînons, (3-azabicyclo[3.1.0]hexan-3-yl)-$C_1$-$C_3$alkyle, (hétérocyclyle à 4, 5 ou 6 chaînons)amino-$C_1$-$C_3$alkyle, hydroxycarbonyl-$C_1$-$C_6$alkyle-(hétéroaryle monocyclique à 5 ou 6 chaînons), $R^{20}$, et 5-(2-(2,6-dioxopipéridin-3-yl)-3-oxoisoindoline-4-yl) pentyle,
où toute position disponible de tout anneau hétéroaryle dans $R^{1h}$ peut être substituée par un substituant choisi dans le groupe constitué de $C_1$-$C_6$alkyle, $C_3$-$C_6$alcynyle, halo, trifluorométhyle, 2,2,2-trifluoroéthyle, biotinyl-$PEG_{11}$, et toute position disponible de toute chaîne de carbone dans $R^{1h}$ à l'exclusion des anneaux hétéroaryles peut être substituée par un substituant choisi parmi l'halogène, l'amino, l'hydroxy et l'oxo ;
$R^{1j}$ représente -H ;
$R^{1k}$ représente -H ;
$R^{1m}$ représente -H ;
ou, $R^{1k}$ et $R^{1h}$ pris avec les atomes d'azote et de carbone qui les portent, respectivement, représentent un anneau hétérocyclique à 6 chaînons de formule structurelle (II) :

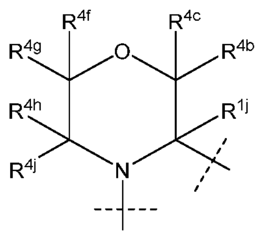

(II)

$R^{3a}$ représente -H ;
$R^{3b}$ représente -H ;
$R^{3c}$ représente -H ;
$R^{3d}$ représente -H ;
$R^{3e}$ représente -H ;
$R^{3f}$ est sélectionné de le groupe consisting de 1-methyl-1*H*-imidazol-4-yle, 1,2-diméthyl-1*H*-imidazol-4-yle, 5-méthyloxazol-2-yle, 4,5-diméthyloxazol-2-yle, 1*H*-pyrazol-1-yle, 1-méthyl-1*H*-pyrazol-3-yle, 4-méthyl-1*H*-pyrazol-1-yle, 4-fluoro-1*H*-pyrazol-1-yle, 1-isopropyl-1*H*-pyrazol-3-yle, 5-méthyl-1*H*-pyrazol-3-yle, 1,5-diméthyl-1*H*-pyrazol-3-yle, 1-éthyl-5-méthyl-1*H*-pyrazol-3-yle, 1,5-diméthyl-1*H*-pyrazol-3-yle, 1,5-diméthyl-1*H*-pyrazol-4-yle, 1-méthyl-5-(trifluorométhyl)-1*H*-pyrazol-3-yle, 1-méthyl-2-cyano-1*H*-pyrrol-4-yle, 1*H*-tétrazol-5-yle, 2-méthyl-2*H*-tétrazol-5-yle, 5-méthyl-1,3,4-thiadiazol-2-yle, 5-méthylthiazol-2-yle, 4,5-diméthylthiophén-2-yle, 1*H*-1,2,4-triazol-1-yle, 1-méthyl-1*H*-1,2,3-triazol-4-yle, 1-méthyl-1*H*-1,2,4-triazol-3-yle, 2-méthyl-2*H*-1,2,3-triazol-4-yle, 1,5-diméthyl-1*H*-1,2,4-triazol-3-yle, 5-hydroxy-1-méthyl-1*H*-1,2,4-triazol-3-yle, et 5-méthoxy-1-méthyl-1*H*-1,2,4-triazol-3-yle ;
$R^{3g}$ représente -H ;
$R^{3h}$ représente -H ;
$R^{3j}$ représente -H ;
$R^{3k}$ représente -H ;
$R^{3m}$ représente -H ;
ou $R^{3d}$ et $R^{3g}$ pris avec la liaison simple reliant deux atomes de carbone qui les portent représentent une double liaison entre ces deux atomes de carbone ;
$R^{4b}$ représente -H ;
$R^{4c}$ représente -H ;
$R^{4f}$ représente -H ;
$R^{4g}$ représente -H ;
$R^{4h}$ représente -H ;
$R^{4j}$ représente -H ;
$R^{10}$ représente l'halogène ou le trifluorométhyle ;
et $R^{20}$ représente

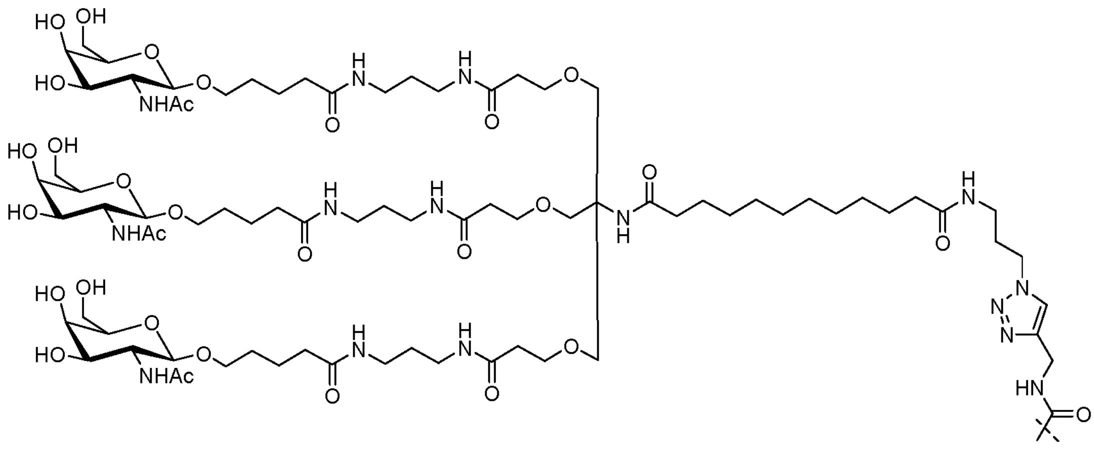
;

ou un tautomère, un stéréo-isomère, un *N*-oxyde, un méthiodure, un sel pharmaceutiquement acceptable, un solvate ou un polymorphe de celui-ci.

**2.** Composé selon la revendication 1, représenté par la formule structurelle (I) :

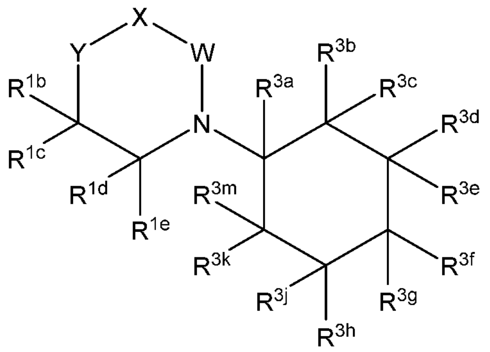

(I)

dans lequel :

W représente -C(-$R^{1f}$)(-$R^{1g}$)- ;
X représente -C(-$R^{1h}$)(-$R^{1j}$)- ;
Y représente -C(-$R^{1k}$)(-$R^{1m}$)-, -N(-$R^{1k}$)-, ou -O- ;
$R^{1b}$ représente -H ;
$R^{1c}$ représente -H ;
$R^{1d}$représente 4-$R^{10}$-benzyle ;
$R^{1e}$ représente -H ;
$R^{1f}$ représente -H ;
$R^{1g}$ représente -H ;
$R^{1h}$ est choisi dans le groupe constitué de $C_1$-$C_6$alkyle, $C_2$-$C_6$ alcényle, $C_2$-$C_6$alkynyle, hydroxy-$C_1$-$C_6$alkyle, azido-$C_1$-$C_6$alkyle, hydroxycarbonyl-$C_1$-$C_3$ alkoxy-$C_1$-$C_6$alkyle, $C_3$-$C_6$ alkynyloxy-$C_1$-$C_6$alkyle, halo-$C_1$-$C_6$alkyle, $C_1$-$C_3$ alkylthio-$C_1$-$C_6$alkyle, $C_1$-$C_3$ alkylsulfinyl-$C_1$-$C_6$alkyle, $C_1$-$C_3$ alkylsulfonyl-$C_1$-$C_6$ alkyle, hydroxycarbonyl-$C_1$-$C_3$ alkylsulfonyl-$C_1$-$C_6$alkyle, hydroxycarbonyl-$C_1$-$C_6$alkyle, $C_1$-$C_3$ alkylcarbonyl-$C_1$-$C_6$alkyle, $C_1$-$C_3$alkylaminocarbonyl-$C_1$-$C_6$alkyle, di($C_1$-$C_3$alkyle)aminocarbonyl-$C_1$-$C_6$alkyle, $C_1$-$C_3$ alkylsulfonylamino-$C_1$-$C_6$alkyle, halo-$C_1$-$C_3$ alkylsulfonylamino-$C_1$-$C_6$alkyle, hétéroaryl-carbonylamino-$C_1$-$C_6$alkyle à 5 ou 6 chaînons monocycliques, hétéroaryle monocyclique à 5 ou 6 chaînons-$C_1$-$C_3$alcoxy-$C_1$-$C_6$alkyle, hétéroaryle monocyclique à 5 ou 6 chaînons-$C_1$-$C_6$alkyle, hydroxycarbonyle, $C_1$-$C_3$alcoxycarbonyle, aminocarbonyle, $C_1$-$C_3$alkylaminocarbonyle, di($C_1$-$C_3$alkyle)aminocarbonyl, $C_3$-$C_6$alkynylaminocarbonyl, hydroxycarbonyl-$C_1$-$C_3$alkyle($C_1$-$C_3$alkyle)aminocarbonyl, (biotinyl-$PEG_{11}$)aminocarbonyle, hydroxylaminocarbonyle, *N*[2]-acétylhydrazinecarbonyle, (hétéroaryle monocyclique à 5 ou 6 chaînons)-$C_1$-$C_3$alkylaminocarbonyle, (hétéroaryle monocyclique à 5 ou 6 chaînons) aminocarbonyle, $C_1$-$C_3$alkylsulfonyle, hétéroaryle monocyclique à 5 ou 6 chaînons, (3-azabicyclo[3.1.0]hexan-3-yl)-$C_1$-$C_3$alkyle, (hétérocyclyle à 4, 5 ou 6 chaînons)amino-$C_1$-$C_3$alkyle, et hydroxycarbonyle-$C_1$-$C_6$alkyle-(hétéroaryle monocyclique à 5 ou 6 chaînons),
où toute position disponible de tout anneau hétéroaryle dans $R^{1h}$ peut être substituée par un substituant choisi dans le groupe constitué de $C_1$-$C_6$alkyle, $C_3$-$C_6$alcynyle, halo, trifluorométhyle, 2,2,2-trifluoroéthyle, biotinyl-$PEG_{11}$, et toute position disponible de toute chaîne de carbone dans $R^{1h}$ à l'exclusion des anneaux hétéroaryles peut être substituée par un substituant choisi parmi l'halogène, l'amino, l'hydroxy et l'oxo ;
$R^{1j}$ représente -H ;
$R^{1k}$ représente -H ;
$R^{1m}$ représente -H ;
ou $R^{1k}$ et $R^{1h}$ pris avec les atomes d'azote et de carbone qui les portent, respectivement, représentent un anneau hétérocyclique à 6 chaînons de formule structurelle (II) :

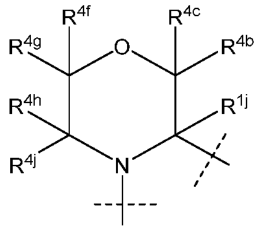

(II)

$R^{3a}$ représente -H ;

$R^{3b}$ représente -H ;
$R^{3c}$ représente -H ;
$R^{3d}$ représente -H ;
$R^{3e}$ représente -H ;
$R^{3f}$ est choisi dans le groupe constitué de 5-méthyloxazol-2-yl, 1,5-diméthyl-1*H*-pyrazol-3-yle, 1,5-diméthyl-1*H*-1,2,4-triazol-3-yle, 4,5-diméthyloxazol-2-yle, 5-fluoro-1-méthyl-1*H*-pyrazol-3-yle, 4,5-diméthylthiazol-2-yle et 5-méthylthiazol-2-yle ;
$R^{3g}$ représente -H ;
$R^{3h}$ représente -H ;
$R^{3j}$ représente -H ;
$R^{3k}$ représente -H ;
$R^{3m}$ représente -H ;
ou $R^{3d}$ et $R^{3g}$ pris avec la liaison simple reliant deux atomes de carbone qui les portent représentent une double liaison entre ces deux atomes de carbone ;
$R^{4b}$ représente -H ;
$R^{4c}$ représente -H ;
$R^{4f}$ représente -H ;
$R^{4g}$ représente -H ;
$R^{4h}$ représente -H ;
$R^{4j}$ représente -H ;
et $R^{10}$ représente l'halogène ou le trifluorométhyle ;
ou un tautomère, un stéréo-isomère, un *N*-oxyde, un méthiodure, un sel pharmaceutiquement acceptable, un solvate ou un polymorphe de celui-ci.

**3.** Composé selon la revendication 1 ou 2, ayant la formule structurelle (Ia) :

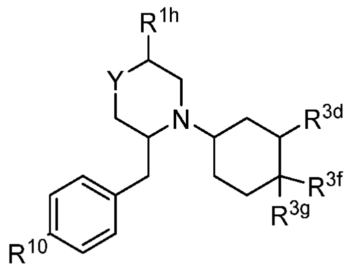

(Ia)

dans lequel :

Y représente $-CH_2-$, $-N(-R^{1k})-$, ou -O- ;
$R^{1h}$ est choisi dans le groupe constitué de méthyle, éthynyle, 2-hydroxyprop-2-yle, azidométhyle, hydroxyméthyle, hydroxycarbonylméthoxyméthyle, (prop-2-yn-1-yloxy)méthyle, fluorométhyle, méthylthiométhyle, méthylsulfinylméthyle, méthylsulfonylméthyle, éthylsulfonylméthyle, 2-(hydroxycarbonyl)éthylsulfonylméthyle, hydroxycarbonylméthyle, méthoxycarbonylméthyle, éthylaminocarbonylméthyle, méthylsulfonylaminométhyle, trifluorométhylsulfonylaminométhyle, (1*H*-pyrazol-3-yl)carbonylaminométhyle, 1-(trifluorométhyl)-1*H*-1,2,3-triazol-4-yle, (1-(biotinyl-$PEG_{11}$)-1*H*-1,2,3-triazol-4-yl)méthoxyméthyle, (2*H*-1,2,3-triazol-4-yl)carbonylaminométhyle, (1*H*-1,2,3-triazol-5-yl)carbonylaminométhyle, (1-méthyl-1*H*-imidazol-4-yl)carbonylaminométhyle, (1*H*-imidazol-4-yl)carbonylaminométhyle, (1,5-diméthyl-1*H*-pyrazol-3-yl)carbonylaminométhyle, (2,5-dioxopyrrolidin-1-yl)méthyle, (2,4-dioxoimidazolidin-3-yl)méthyle, (1-méthyl-2,4-dioxoimidazolidin-3-yl)méthyle, hydroxycarbonyle, méthoxycarbonyle, aminocarbonyle, méthylaminocarbonyle, diméthylaminocarbonyle, éthylaminocarbonyle, isopropylaminocarbonyle, (prop-2-yn-1-yl)aminocarbonyle, hydroxycarbonylméthyl(méthyl)aminocarbonyle, (biotinyl-$PEG_{11}$)aminocarbonyle, hydroxylaminocarbonyle, $N^2$-acétylhydrazinecarbonyle, (1-(biotinyl-$PEG_{11}$)-1*H*-1,2,3-triazol-4-yl)méthylaminocarbonyl, (1-méthyl-1*H*-imidazol-4-yl)aminocarbonyle, (1,5-diméthyl-1*H*-pyrazol-3-yl)aminocarbonyle, (2-(3-(but-3-yn-1-yl)-3*H*-diazirin-3-yl)éthyl)aminocarbonyle, méthylsulfonyle, 3-méthylisoxazol-5-yle, 1-(2,2,2-trifluoroéthyl)-1*H*-1,2,3-triazol-4-yle, 5-méthyl-1,3,4-oxadiazol-2-yle, (6,6-diméthyl-2,4-dioxo-3-azabicyclo[3.1.0]hexan-3-yl)méthyle, (1,2-dioxo-3-hydroxy-cyclobut-3-en-4-yl)aminométhyle, (1,2-dioxo-3-amino-cyclobut-3-en-4-yl)aminométhyle, 1-(biotinyl-$PEG_{11}$)-1*H*-1,2,3-triazol-4-yle, 1-(hydroxycarbonylméthyl)-1*H*-1,2,3-triazol-4-yle, 1-(2-(hydroxycarbonyl)éthyl)-1*H*-1,2,3-triazol-4-yle, 1-(3-(hydroxycarbonyl)prop-1-yl)-1*H*-1,2,3-triazol-4-yle, 1-(4-(hydroxycarbonyl)but-1-yl)-1*H*-1,2,3-triazol-4-yle, et 5-méthyl-1*H*-pyrazol-3-yle ;
ou $R^{1k}$ et $R^{1h}$ pris avec les atomes d'azote et de carbone qui les portent, respectivement, représentent un anneau hétérocyclique à 6 chaînons de formule structurelle (IIa) :

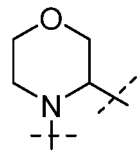

(IIa)

$R^{3d}$ représente H ;
$R^{3g}$ représente H ;
ou $R^{3d}$ et $R^{3g}$ pris avec la liaison simple reliant deux atomes de carbone qui les portent représentent une double liaison entre ces deux atomes de carbone ;
$R^{3f}$ est choisi dans le groupe constitué de 5-méthyloxazol-2-yl, 1,5-diméthyl-1*H*-pyrazol-3-yle, 1,5-diméthyl-1*H*-1,2,4-triazol-3-yle, 4,5-diméthyloxazol-2-yle, 5-fluoro-1-méthyl-1*H*-pyrazol-3-yle, 4,5-diméthylthiazol-2-yle et 5-méthylthiazol-2-yle ;
et $R^{10}$ représente le chloro, le bromo ou le trifluorométhyle ;
ou un tautomère, un stéréo-isomère, un sel pharmaceutiquement acceptable, un solvate ou un polymorphe de celui-ci.

**4.** Composé selon la revendication 1, 2 ou 3,
dans lequel :

X représente $-CH(-R^{1h})-$ ;
Y représente $-CH_2-$ ; et
$R^{1h}$ représente le méthanesulfonyle.

**5.** Composé selon la revendication 1, 2 ou 3,
dans lequel :

X représente $-CH(-R^{1h})-$ ;
Y représente $-N(-R^{1k})-$.
et $R^{1k}$ et $R^{1h}$ pris avec les atomes d'azote et de carbone qui les portent, respectivement, représentent un anneau hétérocyclique à 6 chaînons de formule structurelle (IIb) :

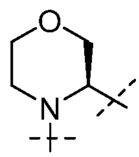

(IIb).

**6.** Composé selon la revendication 1, 2 ou 3,
où Y représente -O-.

**7.** Composé selon l'une quelconque des revendications 1 à 6,
où $R^{1d}$ et $R^{1h}$ sont en relation *cis* .

**8.** Composé selon l'une quelconque des revendications 1 à 7,
dans lequel la stéréochimie absolue au niveau de l'atome de carbone portant $R^{1d}$ est S.

**9.** Composé selon l'une quelconque des revendications 1, 2, 3, 6, 7 et 8, ayant la formule structurelle (Ib) :

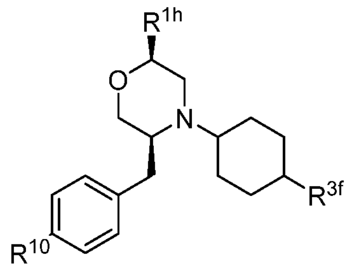

(Ib)

dans lequel :

$R^{1h}$ est choisi dans le groupe constitué de méthyle, éthynyle, 2-hydroxyprop-2-yle, azidométhyle, hydroxyméthyle, hydroxycarbonylméthoxyméthyle, (prop-2-yn-1-yloxy)méthyle, fluorométhyle, méthylthiométhyle, méthylsulfinylméthyle, méthylsulfonylméthyle, éthylsulfonylméthyle, 2-(hydroxycarbonyl)éthylsulfonylméthyle, hydroxycarbonylméthyle, méthoxycarbonylméthyle, éthylaminocarbonylméthyle, méthylsulfonylaminométhyle, trifluorométhylsulfonylaminométhyle, (1*H*-pyrazol-3-yl)carbonylaminométhyle, 1-(trifluorométhyl)-1*H*-1,2,3-triazol-4-yle, (1-(biotinyl-$PEG_{11}$)-1*H*-1,2,3-triazol-4-yl)méthoxyméthyle, (2*H*-1,2,3-triazol-4-yl)carbonylaminométhyl, (1*H*-1,2,3-triazol-5-yl)carbonylaminométhyle, (1-méthyl-1*H*-imidazol-4-yl)carbonylaminométhyle, (1*H*-imidazol-4-yl)carbonylaminométhyle, (1,5-diméthyl-1*H*-pyrazol-3-yl)carbonylaminométhyle, (2,5-dioxopyrrolidin-1-yl)méthyle, (2,4-dioxoimidazolidin-3-yl)méthyle, (1-méthyl-2,4-dioxoimidazolidin-3-yl)méthyle, hydroxycarbonyle, méthoxycarbonyle, aminocarbonyle, méthylaminocarbonyle, diméthylaminocarbonyle, éthylaminocarbonyle, isopropylaminocarbonyle, (prop-2-yn-1-yl)aminocarbonyle, (biotinyl-$PEG_{11}$)aminocarbonyle, hydroxylaminocarbonyle, $N^2$-acétylhydrazinecarbonyle, (1-(biotinyl-$PEG_{11}$)-1*H*-1,2,3-triazol-4-yl)méthylaminocarbonyle, (1-méthyl-1*H*-imidazol-4-yl)aminocarbonyle, (1,5-diméthyl-1H-pyrazol-3-yl)aminocarbonyle, (2-(3-(but-3-yn-1-yl)-3*H*-diazirin-3-yl)éthyl)aminocarbonyle, 3-méthylisoxazol-5-yle, 1-(2,2,2-trifluoroéthyl)-1*H*-1,2,3-triazol-4-yle, 5-méthyl-1,3,4-oxadiazol-2-yle, (6,6-diméthyl-2,4-dioxo-3-azabicyclo[3.1.0]hexan-3-yl)méthyle, (1,2-dioxo-3-hydroxy-cyclobut-3-én-4-yl)aminométhyl, (1,2-dioxo-3-amino-cyclobut-3-en-4-yl)aminométhyle, 1-(biotinyl-$PEG_{11}$)-1*H*-1,2,3-triazol-4-yle, 1-(hydroxycarbonylméthyl)-1*H*-1,2,3-triazol-4-yle, 1-(2-(hydroxycarbonyl)éthyl)-1*H*-1,2,3-triazol-4-yle, 1-(3-(hydroxycarbonyl)prop-1-yl)-1*H*-1,2,3-triazol-4-yle, 1-(4-(hydroxycarbonyl)but-1-yl)-1*H*-1,2,3-triazol-4-yle, et 5-méthyl-1*H*-pyrazol-3-yle ; et
$R^{3f}$ est choisi dans le groupe constitué de 5-méthyloxazol-2-yl, 1,5-diméthyl-1*H*-pyrazol-3-yle, 1,5-diméthyl-1*H*-1,2,4-triazol-3-yle, 4,5-diméthyloxazol-2-yle, 5-fluoro-1-méthyl-1*H*-pyrazol-3-yle, 4,5-diméthylthiazol-2-yle et 5-méthylthiazol-2-yle ;
ou un tautomère, un stéréo-isomère, un sel pharmaceutiquement acceptable, un solvate ou un polymorphe de celui-ci.

**10.** Composé selon l'une quelconque des revendications 1, 2, 3, 6, 7, 8 et 9, dans lequel $R^{10}$ est un bromo.

**11.** Composé selon l'une quelconque des revendications 1 à 9, dans lequel $R^{10}$ est un chloro.

**12.** Composé selon l'une quelconque des revendications 1 à 11, dans lequel $R^{3f}$ est un 5-méthyloxazol-2-yle.

**13.** Composé selon l'une quelconque des revendications 1 à 11, dans lequel $R^{3f}$ est un 1,5-diméthyl-1*H*-pyrazol-3-yle.

**14.** Composé selon l'une quelconque des revendications 1 à 11, dans lequel $R^{3f}$ est un 1,5-diméthyl-1*H*-1,2,4-triazol-3-yle.

**15.** Composé selon l'une quelconque des revendications 1, 2, 3, 6, 7, 8, 9 et 11, dans lequel $R^{3f}$ est un 4,5-diméthyloxazol-2-yle.

**16.** Composé selon l'une quelconque des revendications 1, 2, 3, 6, 7, 8, 9 et 11, dans lequel $R^{3f}$ est un 5-fluoro-1-méthyl-1*H*-pyrazol-3-yle.

**17.** Composé selon l'une quelconque des revendications 1 à 11, dans lequel $R^{3f}$ est un 4,5-diméthylthiazol-2-yle.

**18.** Composé selon l'une quelconque des revendications 1, 2, 3, 6, 7, 8, 9 et 11, dans lequel $R^{3f}$ est un 5-méthylthiazol-2-yle.

**19.** Composé selon l'une quelconque des revendications 1 à 18, dans lequel

$R^{3a}$ représente H ;
$R^{3g}$ représente H ; et
le substituant $R^{3f}$ et l'anneau hétérocyclique attaché par son atome d'azote à l'anneau cyclohexane selon le schéma de substitution 1,4 sont en relation *trans* .

**20.** Composé selon la revendication 1, choisi dans le groupe constitué des composés suivants :

(2*S*,5*S*)-5-(4-chlorobenzyl)-2-méthyl-4-(4-(5-méthyloxazol-2-yl)cyclohexyl)morpholine ;
(2*S*,5*S*)-5-(4-chlorobenzyl)-4-(4-(1,5-diméthyl-1*H*-pyrazol-3-yl)cyclohexyl)-2-méthylmorpholine ;
(2*R*,5*S*)-5-(4-chlorobenzyl)-4-(4-(5-méthyloxazol-2-yl)cyclohexyl)-2-((méthylsulfonyl)méthyl)morpholine ;
(2*R*,5*S*)-5-(4-chlorobenzyl)-4-((1*r*,4*S*)-4-(1,5-diméthyl-1*H*-pyrazol-3-yl)cyclohexyl)-2-((méthylsulfonyl)méthyl) morpholine ;
(2*R*,5*S*)-5-(4-chlorobenzyl)-4-(4-(1,5-diméthyl-1*H*-pyrazol-3-yl)cyclohexyl)-2-(3-méthylisoxazol-5-yl)morpholine ;
(7*S*,9a*R*)-7-(4-chlorobenzyl)-8-(4-(5-méthyloxazol-2-yl)cyclohexyl)octahydropyrazino[2,1-*c*][1,4]oxazine ;
(7*S*,9a*R*)-7-(4-chlorobenzyl)-8-(4-(1,5-diméthyl-1*H*-pyrazol-3-yl)cyclohexyl)octahydropyrazino[2,1-*c*][1,4]oxazine ;
acide (2*R*,5*S*)-5-(4-chlorobenzyl)-4-(4-(5-méthyloxazol-2-yl)cyclohexyl)morpholine-2-carboxylique ;
méthyl (2*R*,5*S*)-5-(4-chlorobenzyl)-4-(4-(1,5-diméthyl-1*H*-pyrazol-3-yl)cyclohexyl)morpholine-2-carboxylate ;
acide (2*R*,5*S*)-5-(4-chlorobenzyl)-4-(4-(1,5-diméthyl-1*H*-pyrazol-3-yl)cyclohexyl)morpholine-2-carboxylique ;
(2*R*,5*S*)-5-(4-chlorobenzyl)-4-(4-(1,5-diméthyl-1*H*-pyrazol-3-yl)cyclohexyl)-2-(1-(2,2,2-trifluoroéthyl)-1*H*-1,2,3-triazol-4-yl)morpholine ;
(2*R*,5*S*)-5-(4-chlorobenzyl)-4-(4-(5-méthyloxazol-2-yl)cyclohexyl)-2-(1-(2,2,2-trifluoroéthyl)-1*H*-1,2,3-triazol-4-yl)morpholine ;
(2*R*,5*S*)-5-(4-chlorobenzyl)-4-(4-(1,5-diméthyl-1*H*-pyrazol-3-yl)cyclohexyl)-*N*-(37-oxo-41-((3a*S*,4*S*,6a*R*)-2-oxohexahydro-1*H*-thiéno[3,4-*d*]imidazol-4-yl)-3,6,9,12,15,18,21,24,27,30,33-undécaoxa-36-azahentétracontyl)morpholine-2-carboxamide ;
(2*R*,5*S*)-5-(4-chlorobenzyl)-4-(4-(1,5-diméthyl-1*H*-1,2,4-triazol-3-yl)cyclohexyl)-2-((méthylsulfonyl)méthyl) morpholine ;
(2*R*,5*S*)-5-(4-chlorobenzyl)-4-(4-(1,5-diméthyl-1*H*-pyrazol-3-yl)cyclohexyl)-2-(fluorométhyl)morpholine ;
((2*R*,5*S*)-5-(4-chlorobenzyl)-4-(4-(1,5-diméthyl-1*H*-pyrazol-3-yl)cyclohexyl)morpholin-2-yl)methanol ;
(2*R*,5*S*)-5-(4-chlorobenzyl)-*N*-éthyl-4-(4-(5-méthyloxazol-2-yl)cyclohexyl)morpholine-2-carboxamide ;
(2*R*,5*S*)-5-(4-chlorobenzyl)-4-(4-(1,5-diméthyl-1*H*-pyrazol-3-yl)cyclohexyl)-*N*-éthylmorpholine-2-carboxamide;
(2*R*,5*S*)-5-(4-chlorobenzyl)-4-(4-(1,5-diméthyl-1*H*-pyrazol-3-yl)cyclohexyl)-*N,N*-diméthylmorpholine-2-carboxamide ;
(2*R*,5*S*)-5-(4-chlorobenzyl)-*N,N*-diméthyl-4-(4-(5-méthyloxazol-2-yl)cyclohexyl)morpholine-2-carboxamide ;
(2*R*,5*S*)-5-(4-chlorobenzyl)-4-(4-(1,5-diméthyl-1*H*-pyrazol-3-yl)cyclohexyl)-2-(5-méthyl-1,3,4-oxadiazol-2-yl) morpholine ;
(2*R*,5*S*)-*N'*-acétyl-5-(4-chlorobenzyl)-4-(4-(1,5-diméthyl-1*H*-pyrazol-3-yl)cyclohexyl)morpholine-2-carbohydrazide ;
(2*R*,5*S*)-5-(4-chlorobenzyl)-2-(5-méthyl-1,3,4-oxadiazol-2-yl)-4-(4-(5-méthyloxazol-2-yl)cyclohexyl)morpholine ;
(2*R*,5*S*)-*N'*-acétyl-5-(4-chlorobenzyl)-4-(4-(5-méthyloxazol-2-yl)cyclohexyl)morpholine-2-carbohydrazide ;
(2*R*,5*S*)-5-(4-chlorobenzyl)-4-(4-(4,5-diméthyloxazol-2-yl)cyclohexyl)-2-((méthylsulfonyl)méthyl)morpholine ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-1,5-diméthyl-1*H*-pyrazole-3-carboxamide ;
(2*S*,5*S*)-5-(4-chlorobenzyl)-4-(4-(1,5-diméthyl-1*H*-pyrazol-3-yl)cyclohexyl)-2-éthynylmorpholine ;
*N*-(35-(4-((2*R*,5*S*)-5-(4-chlorobenzyl)-4-(4-(1,5-diméthyl-1*H*-pyrazol-3-yl)cyclohexyl)morpholin-2-yl)-1*H*-1,2,3-triazol-1-yl)-3,6,9,12,15,18,21,24,27,30,33-undécaoxapentatriacontyl)-5-((3a*S*,4*S*,6a*R*)-2-oxohexahydro-1*H*-thiéno[3,4-*d*]imidazol-4-yl)pentanamide ;
(2*R*,5*S*)-5-(4-chlorobenzyl)-4-(4-(1,5-diméthyl-1*H*-pyrazol-3-yl)cyclohexyl)-*N*-isopropylmorpholine-2-carboxamide ;
(2*R*,5*S*)-5-(4-chlorobenzyl)-4-(4-(1,5-diméthyl-1*H*-pyrazol-3-yl)cyclohexyl)-*N*-méthylmorpholine-2-carboxamide ;
(2*R*,5*S*)-5-(4-chlorobenzyl)-4-(4-(4,5-diméthyloxazol-2-yl)cyclohexyl)-*N,N*-diméthylmorpholine-2-carboxamide ;
(2*R*,5*S*)-5-(4-chlorobenzyl)-4-(4-(4,5-diméthyloxazol-2-yl)cyclohexyl)-*N*-isopropylmorpholine-2-carboxamide ;
(2*R*,5*S*)-5-(4-chlorobenzyl)-*N*-méthyl-4-(4-(5-méthyloxazol-2-yl)cyclohexyl)morpholine-2-carboxamide ;
(2*R*,5*S*)-5-(4-chlorobenzyl)-4-(4-(4,5-diméthyloxazol-2-yl)cyclohexyl)-*N*-éthylmorpholine-2-carboxamide ;
(2*R*,5*S*)-5-(4-chlorobenzyl)-4-(4-(5-fluoro-1-méthyl-1*H*-pyrazol-3-yl)cyclohexyl)-2-((méthylsulfonyl)méthyl) morpholine ;
(2*R*,5*S*)-5-(4-chlorobenzyl)-4-(4-(4,5-diméthylthiazol-2-yl)cyclohexyl)-2-((méthylsulfonyl)méthyl)morpholine ;
(2*R*,5*S*)-5-(4-chlorobenzyl)-2-((méthylsulfonyl)méthyl)-4-(4-(5-méthylthiazol-2-yl)cyclohexyl)morpholine ;
acide (2*R*,5*S*)-5-(4-chlorobenzyl)-4-(4-(4,5-diméthyloxazol-2-yl)cyclohexyl)morpholine-2-carboxylique ;
acide (2*R*,5*S*)-5-(4-chlorobenzyl)-4-(4-(4,5-diméthylthiazol-2-yl)cyclohexyl)morpholine-2-carboxylique ;
(2*R*,5*S*)-5-(4-chlorobenzyl)-4-(4-(1,5-diméthyl-1*H*-pyrazol-3-yl)cyclohexyl)-2-((prop-2-yn-1-yloxy)méthyl)morpholine ;
(2*R*,5*S*)-5-(4-chlorobenzyl)-4-(4-(4,5-diméthylthiazol-2-yl)cyclohexyl)-*N*-éthylmorpholine-2-carboxamide ;
*N*-(35-(4-((((2*R*,5*S*)-5-(4-chlorobenzyl)-4-(4-(1,5-diméthyl-1*H*-pyrazol-3-yl)cyclohexyl)morpholin-2-yl)methoxy)méthyl)-1*H*-1,2,3-triazol-1-yl)-3,6,9,12,15,18,21,24,27,30,33-undécaoxapentatriacontyl)-5-((3a*S*,4*S*,6a*R*)-2-oxohexahydro-1*H*-thiéno[3,4-*d*]imidazol-4-yl)pentanamide ;
(2*R*,5*S*)-2-(azidométhyl)-5-(4-chlorobenzyl)-4-(4-(1,5-diméthyl-1*H*-pyrazol-3-yl)cyclohexyl)morpholine ;
(2*R*,5*S*)-5-(4-chlorobenzyl)-4-(4-(1,5-diméthyl-1*H*-pyrazol-3-yl)cyclohexyl)-*N*-(prop-2-yn-1-yl)morpholine-2-carboxamide ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-1*H*-pyrazole-3-carboxamide ;
(2*R*,5*S*)-5-(4-chlorobenzyl)-4-(4-(1,5-diméthyl-1*H*-pyrazol-3-yl)cyclohexyl)-*N*-((1-(37-oxo-41-((3a*S*,4*S*,6a*R*)-2-oxohexahydro-1*H*-thiéno[3,4-*d*]imidazol-4-yl)-3,6,9,12,15,18,21,24,27,30,33-undécaoxa-36-azahentétracontyl)-1*H*-1,2,3-triazol-4-yl)méthyl)morpholine-2-carboxamide ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-2*H*-1,2,3-triazole-4-carboxamide ;
3-(((2*R*,5*S*)-5-(4-chlorobenzyl)-4-(4-(1,5-diméthyl-1*H*-pyrazol-3-yl)cyclohexyl)morpholin-2-yl)méthyl)imidazolidine-2,4-dione ;
(2*R*,5*S*)-5-(4-chlorobenzyl)-4-(4-(1,5-diméthyl-1*H*-1,2,4-triazol-3-yl)cyclohexyl)-*N*-éthylmorpholine-2-carboxamide ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-1*H*-imidazole-4-carboxamide ;
(2*R*,5*S*)-5-(4-chlorobenzyl)-4-(4-(4,5-diméthylthiazol-2-yl)cyclohex-3-én-1-yl)-2-((méthylsulfonyl)méthyl)morpholine ;
(2*R*,5*S*)-5-(4-chlorobenzyl)-4-(4-(4,5-diméthylthiazol-2-yl)cyclohex-3-én-1-yl)-*N*-éthylmorpholine-2-carboxamide ;
(2*R*,5*S*)-5-(4-chlorobenzyl)-4-(4-(4,5-diméthylthiazol-2-yl)cyclohexyl)-*N*-(37-oxo-41-((3a*S*,4*S*,6a*R*)-2-oxohexahydro-1*H*-thiéno[3,4-*d*]imidazol-4-yl)-3,6,9,12,15,18,21,24,27,30,33-undécaoxa-36-azahentétracontyl)morpholine-2-carboxamide ;
(2*R*,5*S*)-2-(azidométhyl)-5-(4-chlorobenzyl)-4-(4-(4,5-diméthylthiazol-2-yl)cyclohexyl)morpholine ;
(2*R*,5*S*)-5-(4-chlorobenzyl)-4-(4-(4,5-diméthylthiazol-2-yl)cyclohexyl)-*N*-((1-(37-oxo-41-((3a*S*,4*S*,6a*R*)-2-oxohexahydro-1*H*-thiéno[3,4-*d*]imidazol-4-yl)-3,6,9,12,15,18,21,24,27,30,33-undécaoxa-36-azahentétracontyl)-1*H*-1,2,3-triazol-4-yl)méthyl)morpholine-2-carboxamide ;
*N*-(((2*S*,5*S*)-5-(4-chlorobenzyl)-4-(4-(4,5-diméthylthiazol-2-yl)cyclohexyl)morpholin-2-yl)méthyl)-1*H*-1,2,3-triazole-5-carboxamide ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-1-méthyl-1*H*-imidazole-4-carboxamide ;
méthyl 2-((2*S*,5*S*)-5-(4-chlorobenzyl)-4-(4-(1,5-diméthyl-1*H*-pyrazol-3-yl)cyclohexyl)morpholin-2-yl)acétate ;
acide 2-((2*S*,5*S*)-5-(4-chlorobenzyl)-4-(4-(1,5-diméthyl-1*H*-pyrazol-3-yl)cyclohexyl)morpholin-2-yl)acétique ;
2-((2*S*,5*S*)-5-(4-chlorobenzyl)-4-(4-(1,5-diméthyl-1*H*-pyrazol-3-yl)cyclohexyl)morpholin-2-yl)-*N*-éthylacetamide ;
acide (2*R*,5*S*)-5-(4-chlorobenzyl)-4-(4-(1,5-diméthyl-1*H*-1,2,4-triazol-3-yl)cyclohexyl)morpholine-2-carboxylique ;
(2*R*,5*S*)-5-(4-bromobenzyl)-4-(4-(1,5-diméthyl-1*H*-pyrazol-3-yl)cyclohexyl)-2-((méthylsulfonyl)méthyl)morpholine ;
*N*-(((2*R*,5*S*)-5-(4-chlorobenzyl)-4-(4-(1,5-diméthyl-1*H*-pyrazol-3-yl)cyclohexyl)morpholin-2-yl)méthyl)methanesulfonamide ;
acide (2*R*,5*S*)-5-(4-bromobenzyl)-4-(4-(1,5-diméthyl-1*H*-pyrazol-3-yl)cyclohexyl)morpholine-2-carboxylique ;
acide (2*R*,5*S*)-5-(4-bromobenzyl)-4-(4-(5-méthyloxazol-2-yl)cyclohexyl)morpholine-2-carboxylique ;
(2*R*,5*S*)-5-(4-bromobenzyl)-4-(4-(5-méthyloxazol-2-yl)cyclohexyl)-2-((méthylsulfonyl)méthyl)morpholine ;
(2*R*,5*S*)-5-(4-bromobenzyl)-4-(4-(1,5-diméthyl-1*H*-1,2,4-triazol-3-yl)cyclohexyl)-2-((méthylsulfonyl)méthyl) morpholine ;
acide (2*R*,5*S*)-5-(4-bromobenzyl)-4-(4-(1,5-diméthyl-1*H*-1,2,4-triazol-3-yl)cyclohexyl)morpholine-2-carboxylique ;
*N*-(((2*R*,5*S*)-5-(4-chlorobenzyl)-4-(4-(1,5-diméthyl-1*H*-pyrazol-3-yl)cyclohexyl)morpholin-2-yl)méthyl)-1,1,1-trifluoromethanesulfonamide ;
(2*R*,5*S*)-5-(4-bromobenzyl)-4-(4-(1,5-diméthyl-1*H*-pyrazol-3-yl)cyclohexyl)-*N*-éthylmorpholine-2-carboxamide ;
(2*R*,5*S*)-*N*-(2-(3-(but-3-yn-1-yl)-3*H*-diazirin-3-yl)éthyl)-5-(4-chlorobenzyl)-4-(4-(1,5-diméthyl-1*H*-pyrazol-3-yl) cyclohexyl)morpholine-2-carboxamide ;
acide 2-(((2*R*,5*S*)-5-(4-chlorobenzyl)-4-(4-(4,5-diméthylthiazol-2-yl)cyclohexyl)morpholin-2-yl)méthoxy)acétique ;
2-((2*R*,5*S*)-5-(4-chlorobenzyl)-4-(4-(1,5-diméthyl-1*H*-pyrazol-3-yl)cyclohexyl)morpholin-2-yl)propan-2-ol ;
(2*R*,5*S*)-5-(4-chlorobenzyl)-4-(4-(1,5-diméthyl-1*H*-pyrazol-3-yl)cyclohexyl)-2-(5-méthyl-1*H*-pyrazol-3-yl)morpholine ;
(2*R*,5*R*)-2-(4-chlorobenzyl)-1-(4-(1,5-diméthyl-1*H*-pyrazol-3-yl)cyclohexyl)-5-(méthylsulfonyl)pipéridine ;
(2*R*,5*S*)-5-(4-chlorobenzyl)-4-(4-(1,5-diméthyl-1*H*-pyrazol-3-yl)cyclohexyl)morpholine-2-carboxamide ;
2-(4-((2*R*,5*R*)-2-(4-chlorobenzyl)-5-(méthylsulfonyl)pipéridin-1-yl)cyclohexyl)-5-méthyloxazole ;
(2*R*,5*S*)-5-(4-chlorobenzyl)-4-(4-(1,5-diméthyl-1*H*-pyrazol-3-yl)cyclohexyl)-2-((méthylsulfinyl)méthyl)morpholine ;
(2*R*,5*S*)-5-(4-chlorobenzyl)-4-(4-(1,5-diméthyl-1*H*-pyrazol-3-yl)cyclohexyl)-2-((méthylthio)méthyl)morpholine ;
1-(((2*R*,5*S*)-5-(4-chlorobenzyl)-4-(4-(1,5-diméthyl-1*H*-pyrazol-3-yl)cyclohexyl)morpholin-2-yl)méthyl)pyrrolidine-2,5-dione ;
(2*S*,5*S*)-5-(4-chlorobenzyl)-4-(4-(4,5-diméthylthiazol-2-yl)cyclohexyl)-2-éthynylmorpholine ;
acide 2-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)acétique ;
acide 3-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)propanoïque ;
(2*R*,5*S*)-5-(4-chlorobenzyl)-4-(4-(1,5-diméthyl-1*H*-pyrazol-3-yl)cyclohex-3-én-1-yl)-2-((méthylsulfonyl)méthyl) morpholine ;
*N*-(35-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)-3,6,9,12,15,18,21,24,27,30,33-undécaoxapentatriacontyl)-5-((3a*S*,4*S*,6a*R*)-2-oxohexahydro-1*H*-thiéno[3,4-*d*]imidazol-4-yl)pentanamide ;
acide 2-((2*S*,5*S*)-5-(4-chlorobenzyl)-4-(4-(4,5-diméthylthiazol-2-yl)cyclohexyl)morpholin-2-yl)acétique ;
acide 3-((((2*R*,5*S*)-5-(4-chlorobenzyl)-4-(4-(4,5-diméthylthiazol-2-yl)cyclohexyl)morpholin-2-yl)méthyl)sulfonyl)acide propanoïque ;
3-(((2*R*,5*S*)-5-(4-chlorobenzyl)-4-(4-(1,5-diméthyl-1*H*-pyrazol-3-yl)cyclohexyl)morpholin-2-yl)méthyl)-6,6-diméthyl-3-azabicyclo[3.1.0]hexane-2,4-dione ;
3-(((2*R*,5*S*)-5-(4-chlorobenzyl)-4-(4-(4,5-diméthylthiazol-2-yl)cyclohexyl)morpholin-2-yl)méthyl)-6,6-diméthyl-3-azabicyclo[3.1.0]hexane-2,4-dione ;
3-((((2*S*,5*S*)-5-(4-chlorobenzyl)-4-(4-(1,5-diméthyl-1*H*-pyrazol-3-yl)cyclohexyl)morpholin-2-yl)méthyl)amino)-4-hydroxycyclobut-3-ène-1,2-dione ;
3-amino-4-((((2*S*,5*S*)-5-(4-chlorobenzyl)-4-(4-(1,5-diméthyl-1*H*-pyrazol-3-yl)cyclohexyl)morpholin-2-yl)méthyl) amino)cyclobut-3-ène-1,2-dione ;
(2*R*,5*S*)-5-(4-chlorobenzyl)-4-(4-(4,5-diméthylthiazol-2-yl)cyclohexyl)-2-((éthylsulfonyl)méthyl)morpholine ;
(2*R*,5*S*)-5-(4-chlorobenzyl)-4-(4-(1,5-diméthyl-1*H*-pyrazol-3-yl)cyclohexyl)-2-((éthylsulfonyl)méthyl)morpholine ;
3-(((2*R*,5*S*)-5-(4-chlorobenzyl)-4-(4-(1,5-diméthyl-1*H*-pyrazol-3-yl)cyclohexyl)morpholin-2-yl)méthyl)-1-méthylimidazolidine-2,4-dione ;
(2*R*,5*S*)-5-(4-chlorobenzyl)-4-(4-(4,5-diméthylthiazol-2-yl)cyclohexyl)-*N*-(1-méthyl-1*H*-imidazol-4-yl)morpholine-2-carboxamide ;
acide 4-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)butanolque ;
(2*R*,5*S*)-5-(4-chlorobenzyl)-4-(4-(4,5-diméthylthiazol-2-yl)cyclohexyl)-*N*-hydroxymorpholine-2-carboxamide ;
(2*R*,5*S*)-5-(4-chlorobenzyl)-*N*-(1,5-diméthyl-1*H*-pyrazol-3-yl)-4-(4-(4,5-diméthylthiazol-2-yl)cyclohexyl)morpholine-2-carboxamide ;
acide 5-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)pentanoïque ;
(2*R*,5*S*)-5-(4-chlorobenzyl)-*N*-(1,5-diméthyl-1*H*-pyrazol-3-yl)-4-(4-(1,5-diméthyl-1*H*-pyrazol-3-yl)cyclohexyl) morpholine-2-carboxamide ;
*N*-((2*R*,5*S*)-5-(4-chlorobenzyl)-4-(4-(4,5-diméthylthiazol-2-yl)cyclohexyl)morpholine-2-carbonyl)-*N*-méthylglycine ;
(2*R*,5*S*)-4-(4-(1-méthyl-1*H*-1,2,4-triazol-3-yl)cyclohexyl)-2-((méthylsulfonyl)méthyl)-5-(4-(trifluorométhyl)benzyl)morpholine ;
(2*R*,5*S*)-5-(4-chlorobenzyl)-4-(4-(1-méthyl-1*H*-imidazol-4-yl)cyclohexyl)-2-((méthylsulfonyl)méthyl)morpholine ;
acide (2*R*,5*S*)-4-(4-(1-méthyl-1*H*-1,2,4-triazol-3-yl)cyclohexyl)-5-(4-(trifluorométhyl)benzyl)morpholine-2-carboxylique ;
(2*S*,5*S*)-5-(4-chlorobenzyl)-2-méthyl-4-(4-(5-méthyl-1*H*-pyrazol-3-yl)cyclohexyl)morpholine ;
(2*R*,5*S*)-5-(4-chlorobenzyl)-4-(4-(1-méthyl-1*H*-1,2,4-triazol-3-yl)cyclohexyl)-2-((méthylsulfonyl)méthyl)morpholine ;
acide (2*R*,5*S*)-5-(4-chlorobenzyl)-4-(4-(1-méthyl-1*H*-1,2,4-triazol-3-yl)cyclohexyl)morpholine-2-carboxylique ;
(2*R*,5*S*)-5-(4-chlorobenzyl)-4-(4-(1-méthyl-1*H*-1,2,3-triazol-4-yl)cyclohexyl)-2-((méthylsulfonyl)méthyl)morpholine ;
méthyl (2*R*,5*S*)-5-(4-chlorobenzyl)-4-(4-(1-méthyl-1*H*-1,2,3-triazol-4-yl)cyclohexyl)morpholine-2-carboxylate ;
acide (2*R*,5*S*)-5-(4-chlorobenzyl)-4-(4-(1-méthyl-1*H*-1,2,3-triazol-4-yl)cyclohexyl)morpholine-2-carboxylique ;
(2*R*,5*S*)-5-(4-chlorobenzyl)-4-(4-(1,5-diméthyl-1*H*-pyrazol-4-yl)cyclohexyl)-2-((méthylsulfonyl)méthyl)morpholine ;
(2*R*,5*S*)-5-(4-chlorobenzyl)-4-(4-(1-méthyl-1*H*-pyrazol-3-yl)cyclohexyl)-2-((méthylsulfonyl)méthyl)morpholine ;
(2*R*,5*S*)-5-(4-chlorobenzyl)-4-(4-(2-méthyl-2*H*-1,2,3-triazol-4-yl)cyclohexyl)-2-((méthylsulfonyl)méthyl)morpholine ;
(2*R*,5*S*)-5-(4-chlorobenzyl)-4-(4-(2-méthyl-2*H*-tétrazol-5-yl)cyclohexyl)-2-((méthylsulfonyl)méthyl)morpholine ;
(2*R*,5*S*)-5-(4-chlorobenzyl)-*N,N*-diméthyl-4-(4-(5-méthyl-1,3,4-thiadiazol-2-yl)cyclohexyl)morpholine-2-carboxamide ;
(2*R*,5*S*)-5-(4-chlorobenzyl)-4-(4-(5-méthyl-1,3,4-thiadiazol-2-yl)cyclohexyl)-2-((méthylsulfonyl)méthyl)morpholine ;
(2*R*,5*S*)-5-(4-chlorobenzyl)-4-(4-(5-méthoxy-1-méthyl-1*H*-1,2,4-triazol-3-yl)cyclohexyl)-2-((méthylsulfonyl)méthyl)morpholine ;
3-(4-((2*R*,5*S*)-5-(4-chlorobenzyl)-2-((méthylsulfonyl)méthyl)morpholino)cyclohexyl)-1-méthyl-1*H*-1,2,4-triazol-5-ol ;
(2*R*,5*S*)-5-(4-chlorobenzyl)-4-(4-(1-isopropyl-1*H*-pyrazol-3-yl)cyclohexyl)-2-((méthylsulfonyl)méthyl)morpholine ;
(2*R*,5*S*)-5-(4-chlorobenzyl)-4-(4-(5-méthoxy-1-méthyl-1*H*-1,2,4-triazol-3-yl)cyclohexyl)-*N*,*N*-diméthylmorpholine-2-carboxamide ;
(2*R*,5*S*)-5-(4-chlorobenzyl)-4-(4-(5-hydroxy-1-méthyl-1*H*-1,2,4-triazol-3-yl)cyclohexyl)-*N*,*N*-diméthylmorpholine-2-carboxamide ;
(2*R*,5*S*)-5-(4-chlorobenzyl)-4-(4-(1,2-diméthyl-1*H*-imidazol-4-yl)cyclohexyl)-2-((méthylsulfonyl)méthyl)morpholine ;
(2*R*,5*S*)-5-(4-chlorobenzyl)-4-(4-(1-méthyl-5-(trifluorométhyl)-1*H*-pyrazol-3-yl)cyclohexyl)-2-((méthylsulfonyl) méthyl)morpholine ;
(2*R*,5*S*)-5-(4-chlorobenzyl)-4-(4-(4,5-diméthylthiophène-2-yl)cyclohexyl)-2-((méthylsulfonyl)méthyl)morpholine ;
4-(4-((2*R*,5*S*)-5-(4-chlorobenzyl)-2-((méthylsulfonyl)méthyl)morpholino)cyclohexyl)-1-méthyl-1*H*-pyrrole-2-carbonitrile ;
acide (2*R*,5*S*)-5-(4-chlorobenzyl)-4-(4-(5-cyano-1-méthyl-1*H*-pyrrol-3-yl)cyclohexyl)morpholine-2-carboxylique ;
(2*R*,5*S*)-5-(4-chlorobenzyl)-4-(4-(1-éthyl-5-méthyl-1*H*-pyrazol-3-yl)cyclohexyl)-2-((méthylsulfonyl)méthyl)morpholine ;

acide (2*R*,5*S*)-5-(4-chlorobenzyl)-4-(4-(1-éthyl-5-méthyl-1*H*-pyrazol-3-yl)cyclohexyl)morpholine-2-carboxylique ;
(2*R*,5*S*)-5-(4-chlorobenzyl)-*N*-éthyl-4-(4-(1-éthyl-5-méthyl-1*H*-pyrazol-3-yl)cyclohexyl)morpholine-2-carboxamide ;
(2*R*,5*S*)-5-(4-chlorobenzyl)-4-(4-(4-méthyl-1*H*-pyrazol-1-yl)cyclohexyl)-2-((méthylsulfonyl)méthyl)morpholine ;
acide (2*R*,5*S*)-5-(4-chlorobenzyl)-4-(4-(4-méthyl-1*H*-pyrazol-1-yl)cyclohexyl)morpholine-2-carboxylique ;
*N*-(39-((((2*R*,5*S*)-5-(4-chlorobenzyl)-4-(4-(4,5-diméthylthiazol-2-yl)cyclohexyl)morpholin-2-yl)méthyl)sulfonyl)-37-oxo-3,6,9,12,15,18,21,24,27,30,33-undécaoxa-36-azanonatriacontyl)-5-((3a*S*,4*S*,6a*R*)-2-oxohexahydro-1*H*-thiéno[3,4-*d*]imidazol-4-yl)pentanamide ;
(2*R*,5*S*)-5-(4-chlorobenzyl)-4-(4-(1,5-diméthyl-1*H*-pyrazol-3-yl)cyclohexyl)-*N*-(1*H*-tétrazol-5-yl)morpholine-2-carboxamide ;
(2*R*,5*S*)-4-(4-(1*H*-tétrazol-5-yl)cyclohexyl)-5-(4-chlorobenzyl)-2-((méthylsulfonyl)méthyl)morpholine ;
(2*R*,5*S*)-4-(4-(1*H*-1,2,4-triazol-1-yl)cyclohexyl)-5-(4-chlorobenzyl)-2-((méthylsulfonyl)méthyl)morpholine ;
(2*R*,5*S*)-4-(4-(1*H*-pyrazol-1-yl)cyclohexyl)-5-(4-chlorobenzyl)-2-((méthylsulfonyl)méthyl)morpholine ;
(2*R*,5*S*)-5-(4-chlorobenzyl)-4-(4-(4-fluoro-1*H*-pyrazol-1-yl)cyclohexyl)-2-((méthylsulfonyl)méthyl)morpholine ;
acide (2*R*,5*S*)-5-(4-chlorobenzyl)-4-(4-(5-fluoro-1-méthyl-1*H*-pyrazol-3-yl)cyclohexyl)morpholine-2-carboxylique ;
*N*[1]-(1,31-bis(((2*R*,3*R*,4*R*,5*R*,6*R*)-3-acétamido-4,5-dihydroxy-6-(hydroxyméthyl)tétrahydro-2*H*-pyran-2-yl)oxy)-16-((3-((3-(5-(((2*R*,3*R*,4*R*,5*R*,6*R*)-3-acétamido-4,5-dihydroxy-6-(hydroxyméthyl)tétrahydro-2*H*-pyran-2-yl)oxy)pentanamido)propyl)amino)-3-oxopropoxy)méthyl)-5,11,21,27-tétraoxo-14,18-dioxa-6,10,22,26-tétraazahentriacontan-16-yl)-*N*[12]-(3-(4-(((2*R*,5*S*)-5-(4-chlorobenzyl)-4-(4-(1,5-diméthyl-1*H*-pyrazol-3-yl)cyclohexyl)morpholine-2-carboxamido)méthyl)-1*H*-1,2,3-triazol-1-yl)propyl)dodécanediamide ; et
(2*R*,5*S*)-5-(4-chlorobenzyl)-4-(4-(1,5-diméthyl-1*H*-pyrazol-3-yl)cyclohexyl)-*N*-(5-(2-(2,6-dioxopipéridin-3-yl)-3-oxoisoindolin-4-yl)pentyl)morpholine-2-carboxamide ;
ou un tautomère, un stéréo-isomère, un sel pharmaceutiquement acceptable, un solvant ou un polymorphe de celui-ci.

**21.** Composé selon la revendication 2, choisi dans le groupe constitué des composés suivants :

(2*S*,5*S*)-5-(4-chlorobenzyl)-2-méthyl-4-(4-(5-méthyloxazol-2-yl)cyclohexyl)morpholine ;
(2*S*,5*S*)-5-(4-chlorobenzyl)-4-(4-(1,5-diméthyl-1*H*-pyrazol-3-yl)cyclohexyl)-2-méthylmorpholine ;
(2*R*,5*S*)-5-(4-chlorobenzyl)-4-(4-(5-méthyloxazol-2-yl)cyclohexyl)-2-((méthylsulfonyl)méthyl)morpholine ;
(2*R*,5*S*)-5-(4-chlorobenzyl)-4-((1*r*,4*S*)-4-(1,5-diméthyl-1*H*-pyrazol-3-yl)cyclohexyl)-2-((méthylsulfonyl)méthyl)morpholine ;
(2*R*,5*S*)-5-(4-chlorobenzyl)-4-(4-(1,5-diméthyl-1*H*-pyrazol-3-yl)cyclohexyl)-2-(3-méthylisoxazol-5-yl)morpholine ;
(7*S*,9a*R*)-7-(4-chlorobenzyl)-8-(4-(5-méthyloxazol-2-yl)cyclohexyl)octahydropyrazino[2,1-*c*][1,4]oxazine ;
(7*S*,9a*R*)-7-(4-chlorobenzyl)-8-(4-(1,5-diméthyl-1*H*-pyrazol-3-yl)cyclohexyl)octahydropyrazino[2,1-c][1,4]oxazine ;
acide (2*R*,5*S*)-5-(4-chlorobenzyl)-4-(4-(5-méthyloxazol-2-yl)cyclohexyl)morpholine-2-carboxylique ;
méthyl (2*R*,5*S*)-5-(4-chlorobenzyl)-4-(4-(1,5-diméthyl-1*H*-pyrazol-3-yl)cyclohexyl)morpholine-2-carboxylate ;
acide (2*R*,5*S*)-5-(4-chlorobenzyl)-4-(4-(1,5-diméthyl-1*H*-pyrazol-3-yl)cyclohexyl)morpholine-2-carboxylique ;
(2*R*,5*S*)-5-(4-chlorobenzyl)-4-(4-(1,5-diméthyl-1*H*-pyrazol-3-yl)cyclohexyl)-2-(1-(2,2,2-trifluoroéthyl)-1*H*-1,2,3-triazol-4-yl)morpholine ;
(2*R*,5*S*)-5-(4-chlorobenzyl)-4-(4-(5-méthyloxazol-2-yl)cyclohexyl)-2-(1-(2,2,2-trifluoroéthyl)-1*H*-1,2,3-triazol-4-yl)morpholine ;
(2*R*,5*S*)-5-(4-chlorobenzyl)-4-(4-(1,5-diméthyl-1*H*-pyrazol-3-yl)cyclohexyl)-*N*-(37-oxo-41-((3a*S*,4*S*,6a*R*)-2-oxohexahydro-1*H*-thieno[3,4-*d*]imidazol-4-yl)-3,6,9,12,15,18,21,24,27,30,33-undécaoxa-36-azahentétracontyl)morpholine-2-carboxamide ;
(2*R*,5*S*)-5-(4-chlorobenzyl)-4-(4-(1,5-diméthyl-1*H*-1,2,4-triazol-3-yl)cyclohexyl)-2-((méthylsulfonyl)méthyl)morpholine ;
(2*R*,5*S*)-5-(4-chlorobenzyl)-4-(4-(1,5-diméthyl-1*H*-pyrazol-3-yl)cyclohexyl)-2-(fluorométhyl)morpholine ;
((2*R*,5*S*)-5-(4-chlorobenzyl)-4-(4-(1,5-diméthyl-1*H*-pyrazol-3-yl)cyclohexyl)morpholin-2-yl)methanol ;
(2*R*,5*S*)-5-(4-chlorobenzyl)-*N*-éthyl-4-(4-(5-méthyloxazol-2-yl)cyclohexyl)morpholine-2-carboxamide ;
(2*R*,5*S*)-5-(4-chlorobenzyl)-4-(4-(1,5-diméthyl-1*H*-pyrazol-3-yl)cyclohexyl)-*N*-éthylmorpholine-2-carboxamide;
(2*R*,5*S*)-5-(4-chlorobenzyl)-4-(4-(1,5-diméthyl-1*H*-pyrazol-3-yl)cyclohexyl)-*N,N*-diméthylmorpholine-2-carboxamide ;
(2*R*,5*S*)-5-(4-chlorobenzyl)-*N,N*-diméthyl-4-(4-(5-méthyloxazol-2-yl)cyclohexyl)morpholine-2-carboxamide ;

(2*R*,5*S*)-5-(4-chlorobenzyl)-4-(4-(1,5-diméthyl-1*H*-pyrazol-3-yl)cyclohexyl)-2-(5-méthyl-1,3,4-oxadiazol-2-yl) morpholine ;
(2*R*,5*S*)-*N'*-acétyl-5-(4-chlorobenzyl)-4-(4-(1,5-diméthyl-1*H*-pyrazol-3-yl)cyclohexyl)morpholine-2-carbohydrazide ;
(2*R*,5*S*)-5-(4-chlorobenzyl)-2-(5-méthyl-1,3,4-oxadiazol-2-yl)-4-(4-(5-méthyloxazol-2-yl)cyclohexyl)morpholine ;
(2*R*,5*S*)-*N'*-acétyl-5-(4-chlorobenzyl)-4-(4-(5-méthyloxazol-2-yl)cyclohexyl)morpholine-2-carbohydrazide ;
(2*R*,5*S*)-5-(4-chlorobenzyl)-4-(4-(4,5-diméthyloxazol-2-yl)cyclohexyl)-2-((méthylsulfonyl)méthyl)morpholine ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-1,5-diméthyl-1*H*-pyrazole-3-carboxamide ;
(2*S*,5*S*)-5-(4-chlorobenzyl)-4-(4-(1,5-diméthyl-1*H*-pyrazol-3-yl)cyclohexyl)-2-éthynylmorpholine ;
*N*-(35-(4-((2*R*,5*S*)-5-(4-chlorobenzyl)-4-(4-(1,5-diméthyl-1*H*-pyrazol-3-yl)cyclohexyl)morpholin-2-yl)-1*H*-1,2,3-triazol-1-yl)-3,6,9,12,15,18,21,24,27,30,33-undécaoxapentatriacontyl)-5-((3a*S*,4*S*,6a*R*)-2-oxohexahydro-1*H*-thiéno[3,4-*d*]imidazol-4-yl)pentanamide ;
(2*R*,5*S*)-5-(4-chlorobenzyl)-4-(4-(1,5-diméthyl-1*H*-pyrazol-3-yl)cyclohexyl)-*N*-isopropylmorpholine-2-carboxamide ;
(2*R*,5*S*)-5-(4-chlorobenzyl)-4-(4-(1,5-diméthyl-1*H*-pyrazol-3-yl)cyclohexyl)-*N*-méthylmorpholine-2-carboxamide ;
(2*R*,5*S*)-5-(4-chlorobenzyl)-4-(4-(4,5-diméthyloxazol-2-yl)cyclohexyl)-*N,N*-diméthylmorpholine-2-carboxamide ;
(2*R*,5*S*)-5-(4-chlorobenzyl)-4-(4-(4,5-diméthyloxazol-2-yl)cyclohexyl)-*N*-isopropylmorpholine-2-carboxamide ;
(2*R*,5*S*)-5-(4-chlorobenzyl)-*N*-méthyl-4-(4-(5-méthyloxazol-2-yl)cyclohexyl)morpholine-2-carboxamide ;
(2*R*,5*S*)-5-(4-chlorobenzyl)-4-(4-(4,5-diméthyloxazol-2-yl)cyclohexyl)-*N*-éthylmorpholine-2-carboxamide ;
(2*R*,5*S*)-5-(4-chlorobenzyl)-4-(4-(5-fluoro-1-méthyl-1*H*-pyrazol-3-yl)cyclohexyl)-2-((méthylsulfonyl)méthyl) morpholine ;
(2*R*,5*S*)-5-(4-chlorobenzyl)-4-(4-(4,5-diméthylthiazol-2-yl)cyclohexyl)-2-((méthylsulfonyl)méthyl)morpholine ;
(2*R*,5*S*)-5-(4-chlorobenzyl)-2-((méthylsulfonyl)méthyl)-4-(4-(5-méthylthiazol-2-yl)cyclohexyl)morpholine ;
acide (2*R*,5*S*)-5-(4-chlorobenzyl)-4-(4-(4,5-diméthyloxazol-2-yl)cyclohexyl)morpholine-2-carboxylique ;
acide (2*R*,5*S*)-5-(4-chlorobenzyl)-4-(4-(4,5-diméthylthiazol-2-yl)cyclohexyl)morpholine-2-carboxylique ;
(2*R*,5*S*)-5-(4-chlorobenzyl)-4-(4-(1,5-diméthyl-1*H*-pyrazol-3-yl)cyclohexyl)-2-((prop-2-yn-1-yloxy)méthyl)morpholine ;
(2*R*,5*S*)-5-(4-chlorobenzyl)-4-(4-(4,5-diméthylthiazol-2-yl)cyclohexyl)-*N*-éthylmorpholine-2-carboxamide ;
*N*-(35-(4-((((2*R*,5*S*)-5-(4-chlorobenzyl)-4-(4-(1,5-diméthyl-1*H*-pyrazol-3-yl)cyclohexyl)morpholin-2-yl)methoxy)méthyl)-1*H*-1,2,3-triazol-1-yl)-3,6,9,12,15,18,21,24,27,30,33-undécaoxapentatriacontyl)-5-((3a*S*,4*S*,6a*R*)-2-oxohexahydro-1*H*-thiéno[3,4-*d*]imidazol-4-yl)pentanamide ;
(2*R*,5*S*)-2-(azidométhyl)-5-(4-chlorobenzyl)-4-(4-(1,5-diméthyl-1*H*-pyrazol-3-yl)cyclohexyl)morpholine ;
(2*R*,5*S*)-5-(4-chlorobenzyl)-4-(4-(1,5-diméthyl-1*H*-pyrazol-3-yl)cyclohexyl)-*N*-(prop-2-yn-1-yl)morpholine-2-carboxamide ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-1*H*-pyrazole-3-carboxamide ;
(2*R*,5*S*)-5-(4-chlorobenzyl)-4-(4-(1,5-diméthyl-1*H*-pyrazol-3-yl)cyclohexyl)-*N*-((1-(37-oxo-41-((3a*S*,4*S*,6a*R*)-2-oxohexahydro-1*H*-thiéno[3,4-*d*]imidazol-4-yl)-3,6,9,12,15,18,21,24,27,30,33-undécaoxa-36-azahentétracontyl)-1*H*-1,2,3-triazol-4-yl)méthyl)morpholine-2-carboxamide ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-2*H*-1,2,3-triazole-4-carboxamide ;
3-(((2*R*,5*S*)-5-(4-chlorobenzyl)-4-(4-(1,5-diméthyl-1*H*-pyrazol-3-yl)cyclohexyl)morpholin-2-yl)méthyl)imidazolidine-2,4-dione ;
(2*R*,5*S*)-5-(4-chlorobenzyl)-4-(4-(1,5-diméthyl-1*H*-1,2,4-triazol-3-yl)cyclohexyl)-*N*-éthylmorpholine-2-carboxamide ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-1*H*-imidazole-4-carboxamide ;
(2*R*,5*S*)-5-(4-chlorobenzyl)-4-(4-(4,5-diméthylthiazol-2-yl)cyclohex-3-én-1-yl)-2-((méthylsulfonyl)méthyl)morpholine ;
(2*R*,5*S*)-5-(4-chlorobenzyl)-4-(4-(4,5-diméthylthiazol-2-yl)cyclohex-3-én-1-yl)-*N*-éthylmorpholine-2-carboxamide ;
(2*R*,5*S*)-5-(4-chlorobenzyl)-4-(4-(4,5-diméthylthiazol-2-yl)cyclohexyl)-*N*-(37-oxo-41-((3a*S*,4*S*,6a*R*)-2-oxohexahydro-1*H*-thiéno[3,4-*d*]imidazol-4-yl)-3,6,9,12,15,18,21,24,27,30,33-undécaoxa-36-azahentétracontyl)morpholine-2-carboxamide ;

(2*R*,5*S*)-2-(azidométhyl)-5-(4-chlorobenzyl)-4-(4-(4,5-diméthylthiazol-2-yl)cyclohexyl)morpholine ;
(2*R*,5*S*)-5-(4-chlorobenzyl)-4-(4-(4,5-diméthylthiazol-2-yl)cyclohexyl)-*N*-((1-(37-oxo-41-((3a*S*,4*S*,6a*R*)-2-oxo-hexahydro-1*H*-thiéno[3,4-*d*]imidazol-4-yl)-3,6,9,12,15,18,21,24,27,30,33-undécaoxa-36-azahentétracontyl)-1*H*-1,2,3-triazol-4-yl)méthyl)morpholine-2-carboxamide ;
*N*-(((2*S*,5*S*)-5-(4-chlorobenzyl)-4-(4-(4,5-diméthylthiazol-2-yl)cyclohexyl)morpholin-2-yl)méthyl)-1H-1,2,3-triazole-5-carboxamide ;
*N*-(((2*S*,5*S*)-5-(4-chlorobenzyl)-4-(4-(1,5-diméthyl-1*H*-pyrazol-3-yl)cyclohexyl)morpholin-2-yl)méthyl)-1-méthyl-1*H*-imidazole-4-carboxamide ;
méthyl 2-((2*S*,5*S*)-5-(4-chlorobenzyl)-4-(4-(1,5-diméthyl-1*H*-pyrazol-3-yl)cyclohexyl)morpholin-2-yl)acétate ;
acide 2-((2*S*,5*S*)-5-(4-chlorobenzyl)-4-(4-(1,5-diméthyl-1*H*-pyrazol-3-yl)cyclohexyl)morpholin-2-yl)acétique ;
2-((2*S*,5*S*)-5-(4-chlorobenzyl)-4-(4-(1,5-diméthyl-1*H*-pyrazol-3-yl)cyclohexyl)morpholin-2-yl)-*N*-éthylacetamide ;
acide (2*R*,5*S*)-5-(4-chlorobenzyl)-4-(4-(1,5-diméthyl-1*H*-1,2,4-triazol-3-yl)cyclohexyl)morpholine-2-carboxyliique ;
(2*R*,5*S*)-5-(4-bromobenzyl)-4-(4-(1,5-diméthyl-1*H*-pyrazol-3-yl)cyclohexyl)-2-((méthylsulfonyl)méthyl)morpholine ;
*N*-(((2*R*,5*S*)-5-(4-chlorobenzyl)-4-(4-(1,5-diméthyl-1*H*-pyrazol-3-yl)cyclohexyl)morpholin-2-yl)méthyl)methanesulfonamide ;
acide (2*R*,5*S*)-5-(4-bromobenzyl)-4-(4-(1,5-diméthyl-1*H*-pyrazol-3-yl)cyclohexyl)morpholine-2-carboxylique ;
acide (2*R*,5*S*)-5-(4-bromobenzyl)-4-(4-(5-méthyloxazol-2-yl)cyclohexyl)morpholine-2-carboxylique ;
(2*R*,5*S*)-5-(4-bromobenzyl)-4-(4-(5-méthyloxazol-2-yl)cyclohexyl)-2-((méthylsulfonyl)méthyl)morpholine ;
(2*R*,5*S*)-5-(4-bromobenzyl)-4-(4-(1,5-diméthyl-1*H*-1,2,4-triazol-3-yl)cyclohexyl)-2-((méthylsulfonyl)méthyl) morpholine ;
acide (2*R*,5*S*)-5-(4-bromobenzyl)-4-(4-(1,5-diméthyl-1*H*-1,2,4-triazol-3-yl)cyclohexyl)morpholine-2-carboxylique ;
*N*-(((2*R*,5*S*)-5-(4-chlorobenzyl)-4-(4-(1,5-diméthyl-1*H*-pyrazol-3-yl)cyclohexyl)morpholin-2-yl)méthyl)-1,1,1-trifluoromethanesulfonamide ;
(2*R*,5*S*)-5-(4-bromobenzyl)-4-(4-(1,5-diméthyl-1*H*-pyrazol-3-yl)cyclohexyl)-*N*-éthylmorpholine-2-carboxamide ;
(2*R*,5*S*)-*N*-(2-(3-(but-3-yn-1-yl)-3*H*-diazirin-3-yl)éthyl)-5-(4-chlorobenzyl)-4-(4-(1,5-diméthyl-1*H*-pyrazol-3-yl) cyclohexyl)morpholine-2-carboxamide ;
acide 2-(((2*R*,5*S*)-5-(4-chlorobenzyl)-4-(4-(4,5-diméthylthiazol-2-yl)cyclohexyl)morpholin-2-yl)méthoxy)acétique ;
2-((2*R*,5*S*)-5-(4-chlorobenzyl)-4-(4-(1,5-diméthyl-1*H*-pyrazol-3-yl)cyclohexyl)morpholin-2-yl)propan-2-ol ;
(2*R*,5*S*)-5-(4-chlorobenzyl)-4-(4-(1,5-diméthyl-1*H*-pyrazol-3-yl)cyclohexyl)-2-(5-méthyl-1*H*-pyrazol-3-yl)morpholine ;
(2*R*,5*R*)-2-(4-chlorobenzyl)-1-(4-(1,5-diméthyl-1*H*-pyrazol-3-yl)cyclohexyl)-5-(méthylsulfonyl)pipéridine ;
(2*R*,5*S*)-5-(4-chlorobenzyl)-4-(4-(1,5-diméthyl-1*H*-pyrazol-3-yl)cyclohexyl)morpholine-2-carboxamide ;
2-(4-((2*R*,5*R*)-2-(4-chlorobenzyl)-5-(méthylsulfonyl)pipéridin-1-yl)cyclohexyl)-5-méthyloxazole ;
(2*R*,5*S*)-5-(4-chlorobenzyl)-4-(4-(1,5-diméthyl-1*H*-pyrazol-3-yl)cyclohexyl)-2-((méthylsulfinyl)méthyl)morpholine ;
(2*R*,5*S*)-5-(4-chlorobenzyl)-4-(4-(1,5-diméthyl-1*H*-pyrazol-3-yl)cyclohexyl)-2-((méthylthio)méthyl)morpholine ;
1-(((2*R*,5*S*)-5-(4-chlorobenzyl)-4-(4-(1,5-diméthyl-1*H*-pyrazol-3-yl)cyclohexyl)morpholin-2-yl)méthyl)pyrrolidine-2,5-dione ;
(2*S*,5*S*)-5-(4-chlorobenzyl)-4-(4-(4,5-diméthylthiazol-2-yl)cyclohexyl)-2-éthynylmorpholine ;
acide 2-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)acétique ;
acide 3-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)propanoïque ;
(2*R*,5*S*)-5-(4-chlorobenzyl)-4-(4-(1,5-diméthyl-1*H*-pyrazol-3-yl)cyclohex-3-én-1-yl)-2-((méthylsulfonyl)méthyl) morpholine ;
*N*-(35-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)-3,6,9,12,15,18,21,24,27,30,33-undécaoxapentatriacontyl)-5-((3a*S*,4*S*,6a*R*)-2-oxohexahydro-1*H*-thiéno[3,4-*d*]imidazol-4-yl)pentanamide ;
acide 2-((2*S*,5*S*)-5-(4-chlorobenzyl)-4-(4-(4,5-diméthylthiazol-2-yl)cyclohexyl)morpholin-2-yl)acétique ;
acide 3-((((2*R*,5*S*)-5-(4-chlorobenzyl)-4-(4-(4,5-diméthylthiazol-2-yl)cyclohexyl)morpholin-2-yl)méthyl)sulfonyl)acide propanoïque ;
3-(((2*R*,5*S*)-5-(4-chlorobenzyl)-4-(4-(1,5-diméthyl-1*H*-pyrazol-3-yl)cyclohexyl)morpholin-2-yl)méthyl)-6,6-diméthyl-3-azabicyclo[3.1.0]hexane-2,4-dione ;
3-(((2*R*,5*S*)-5-(4-chlorobenzyl)-4-(4-(4,5-diméthylthiazol-2-yl)cyclohexyl)morpholin-2-yl)méthyl)-6,6-diméthyl-3-azabicyclo[3.1.0]hexane-2,4-dione ;
3-((((2*S*,5*S*)-5-(4-chlorobenzyl)-4-(4-(1,5-diméthyl-1*H*-pyrazol-3-yl)cyclohexyl)morpholin-2-yl)méthyl)amino)-4-hydroxycyclobut-3-ène-1,2-dione ;
3-amino-4-((((2*S*,5*S*)-5-(4-chlorobenzyl)-4-(4-(1,5-diméthyl-1*H*-pyrazol-3-yl)cyclohexyl)morpholin-2-yl)méthyl) amino)cyclobut-3-ène-1,2-dione ;
(2*R*,5*S*)-5-(4-chlorobenzyl)-4-(4-(4,5-diméthylthiazol-2-yl)cyclohexyl)-2-((éthylsulfonyl)méthyl)morpholine ;
(2*R*,5*S*)-5-(4-chlorobenzyl)-4-(4-(1,5-diméthyl-1*H*-pyrazol-3-yl)cyclohexyl)-2-((éthylsulfonyl)méthyl)morpholine ;
3-(((2*R*,5*S*)-5-(4-chlorobenzyl)-4-(4-(1,5-diméthyl-1*H*-pyrazol-3-yl)cyclohexyl)morpholin-2-yl)méthyl)-1-méthylimidazolidine-2,4-dione ;
(2*R*,5*S*)-5-(4-chlorobenzyl)-4-(4-(4,5-diméthylthiazol-2-yl)cyclohexyl)-*N*-(1-méthyl-1*H*-imidazol-4-yl)morpholine-2-carboxamide ;
acide 4-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)butanolque ;
(2*R*,5*S*)-5-(4-chlorobenzyl)-4-(4-(4,5-diméthylthiazol-2-yl)cyclohexyl)-*N*-hydroxymorpholine-2-carboxamide ;
(2*R*,5*S*)-5-(4-chlorobenzyl)-*N*-(1,5-diméthyl-1*H*-pyrazol-3-yl)-4-(4-(4,5-diméthylthiazol-2-yl)cyclohexyl)morpholine-2-carboxamide ;
acide 5-(4-((2*R*,5*S*)-5-(4-chlorobenzyl)-4-(4-(4,5-diméthylthiazol-2-yl)cyclohexyl)morpholin-2-yl)-1*H*-1,2,3-triazol-1-yl)pentanoïque ;
(2*R*,5*S*)-5-(4-chlorobenzyl)-*N*-(1,5-diméthyl-1*H*-pyrazol-3-yl)-4-(4-(1,5-diméthyl-1H-pyrazol-3-yl)cyclohexyl) morpholine-2-carboxamide ; et
*N*-((2*R*,5*S*)-5-(4-chlorobenzyl)-4-(4-(4,5-diméthylthiazol-2-yl)cyclohexyl)morpholine-2-carbonyl)-*N*-méthylglycine ;
ou un tautomère, un stéréo-isomère, un sel pharmaceutiquement acceptable, un solvate ou un polymorphe de celui-ci.

**22.** Composé selon la revendication 2 ou 21, choisi dans le groupe constitué des composés suivants :

(2*S*,5*S*)-5-(4-chlorobenzyl)-2-méthyl-4-(4-(5-méthyloxazol-2-yl)cyclohexyl)morpholine ;
(2*S*,5*S*)-5-(4-chlorobenzyl)-4-(4-(1,5-diméthyl-1*H*-pyrazol-3-yl)cyclohexyl)-2-méthylmorpholine ;
(2*R*,5*S*)-5-(4-chlorobenzyl)-4-(4-(5-méthyloxazol-2-yl)cyclohexyl)-2-((méthylsulfonyl)méthyl)morpholine ;
(2*R*,5*S*)-5-(4-chlorobenzyl)-4-((1*r*,4*S*)-4-(1,5-diméthyl-1*H*-pyrazol-3-yl)cyclohexyl)-2-((méthylsulfonyl)méthyl) morpholine ;
(2*R*,5*S*)-5-(4-chlorobenzyl)-4-(4-(1,5-diméthyl-1*H*-pyrazol-3-yl)cyclohexyl)-2-(3-méthylisoxazol-5-yl)morpholine ;
acide (2*R*,5*S*)-5-(4-chlorobenzyl)-4-(4-(5-méthyloxazol-2-yl)cyclohexyl)morpholine-2-carboxylique ;
acide (2*R*,5*S*)-5-(4-chlorobenzyl)-4-(4-(1,5-diméthyl-1*H*-pyrazol-3-yl)cyclohexyl)morpholine-2-carboxylique ;
(2*R*,5*S*)-5-(4-chlorobenzyl)-4-(4-(1,5-diméthyl-1*H*-1,2,4-triazol-3-yl)cyclohexyl)-2-((méthylsulfonyl)méthyl) morpholine ;
(2*R*,5*S*)-5-(4-chlorobenzyl)-4-(4-(1,5-diméthyl-1*H*-pyrazol-3-yl)cyclohexyl)-2-(fluorométhyl)morpholine ;
((2*R*,5*S*)-5-(4-chlorobenzyl)-4-(4-(1,5-diméthyl-1*H*-pyrazol-3-yl)cyclohexyl)morpholin-2-yl)methanol ;
(2*R*,5*S*)-5-(4-chlorobenzyl)-4-(4-(1,5-diméthyl-1*H*-pyrazol-3-yl)cyclohexyl)-*N*,*N*-diméthylmorpholine-2-carboxamide ;
(2*R*,5*S*)-5-(4-chlorobenzyl)-4-(4-(4,5-diméthyloxazol-2-yl)cyclohexyl)-2-((méthylsulfonyl)méthyl)morpholine ;
(2*R*,5*S*)-5-(4-chlorobenzyl)-4-(4-(1,5-diméthyl-1*H*-pyrazol-3-yl)cyclohexyl)-*N*-méthylmorpholine-2-carboxamide ;
(2*R*,5*S*)-5-(4-chlorobenzyl)-4-(4-(5-fluoro-1-méthyl-1*H*-pyrazol-3-yl)cyclohexyl)-2-((méthylsulfonyl)méthyl) morpholine ;
(2*R*,5*S*)-5-(4-chlorobenzyl)-4-(4-(4,5-diméthylthiazol-2-yl)cyclohexyl)-2-((méthylsulfonyl)méthyl)morpholine ;
acide (2*R*,5*S*)-5-(4-chlorobenzyl)-4-(4-(4,5-diméthyloxazol-2-yl)cyclohexyl)morpholine-2-carboxylique ;
acide (2*R*,5*S*)-5-(4-chlorobenzyl)-4-(4-(4,5-diméthylthiazol-2-yl)cyclohexyl)morpholine-2-carboxylique ;
(2*R*,5*S*)-5-(4-chlorobenzyl)-4-(4-(4,5-diméthylthiazol-2-yl)cyclohex-3-én-1-yl)-2-((méthylsulfonyl)méthyl)morpholine ;
acide 2-((2*S*,5*S*)-5-(4-chlorobenzyl)-4-(4-(1,5-diméthyl-1*H*-pyrazol-3-yl)cyclohexyl)morpholin-2-yl)acétique ;
acide (2*R*,5*S*)-5-(4-chlorobenzyl)-4-(4-(1,5-diméthyl-1*H*-1,2,4-triazol-3-yl)cyclohexyl)morpholine-2-carboxyliique ;

(2*R*,5*S*)-5-(4-bromobenzyl)-4-(4-(1,5-diméthyl-1*H*-pyrazol-3-yl)cyclohexyl)-2-((méthylsulfonyl)méthyl)morpholine ;
*N*-(((2*R*,5*S*)-5-(4-chlorobenzyl)-4-(4-(1,5-diméthyl-1*H*-pyrazol-3-yl)cyclohexyl)morpholin-2-yl)méthyl)methanesulfonamide ;
acide (2*R*,5*S*)-5-(4-bromobenzyl)-4-(4-(1,5-diméthyl-1*H*-pyrazol-3-yl)cyclohexyl)morpholine-2-carboxylique ;
acide (2*R*,5*S*)-5-(4-bromobenzyl)-4-(4-(5-méthyloxazol-2-yl)cyclohexyl)morpholine-2-carboxylique ;
(2*R*,5*S*)-5-(4-bromobenzyl)-4-(4-(5-méthyloxazol-2-yl)cyclohexyl)-2-((méthylsulfonyl)méthyl)morpholine ;
(2*R*,5*S*)-5-(4-bromobenzyl)-4-(4-(1,5-diméthyl-1*H*-1,2,4-triazol-3-yl)cyclohexyl)-2-((méthylsulfonyl)méthyl) morpholine ;
acide (2*R*,5*S*)-5-(4-bromobenzyl)-4-(4-(1,5-diméthyl-1*H*-1,2,4-triazol-3-yl)cyclohexyl)morpholine-2-carboxylique ;
(2*R*,5*R*)-2-(4-chlorobenzyl)-1-(4-(1,5-diméthyl-1*H*-pyrazol-3-yl)cyclohexyl)-5-(méthylsulfonyl)piperidine ;
(2*R*,5*S*)-5-(4-chlorobenzyl)-4-(4-(1,5-diméthyl-1*H*-pyrazol-3-yl)cyclohexyl)morpholine-2-carboxamide ;
2-(4-((2*R*,5*R*)-2-(4-chlorobenzyl)-5-(méthylsulfonyl)pipéridin-1-yl)cyclohexyl)-5-méthyloxazole ;
(2*R*,5*S*)-5-(4-chlorobenzyl)-4-(4-(1,5-diméthyl-1*H*-pyrazol-3-yl)cyclohexyl)-2-((méthylsulfinyl)méthyl)morpholine ;
(2*R*,5*S*)-5-(4-chlorobenzyl)-4-(4-(1,5-diméthyl-1*H*-pyrazol-3-yl)cyclohexyl)-2-((méthylthio)méthyl)morpholine ;
(2*R*,5*S*)-5-(4-chlorobenzyl)-4-(4-(1,5-diméthyl-1*H*-pyrazol-3-yl)cyclohex-3-én-1-yl)-2-((méthylsulfonyl)méthyl) morpholine ;
acide 2-((2*S*,5*S*)-5-(4-chlorobenzyl)-4-(4-(4,5-diméthylthiazol-2-yl)cyclohexyl)morpholin-2-yl)acétique ; et
(2*R*,5*S*)-5-(4-chlorobenzyl)-4-(4-(1,5-diméthyl-1*H*-pyrazol-3-yl)cyclohexyl)-2-((éthylsulfonyl)méthyl)morpholine ;
ou un tautomère, un stéréo-isomère, un sel pharmaceutiquement acceptable, un solvate ou un polymorphe de celui-ci.

**23.** Composition pharmaceutique comprenant une quantité thérapeutiquement efficace d'au moins un composé selon l'une des revendications 1 à 22, et un support pharmaceutiquement acceptable.

**24.** Composé selon l'une quelconque des revendications 1 à 22, ou composition pharmaceutique selon la revendication 23, pour le traitement ou la prévention d'une maladie, d'un trouble ou d'un état associé à une expression ou une activité aberrante de la protéine YKL-40 de type chitinase.

**25.** Composé à utiliser selon la revendication 24, dans lequel la maladie, le trouble ou l'état est choisi dans le groupe consistant en le cancer, les maladies allergiques, les maladies inflammatoires aiguës et chroniques, maladies auto-immunes, maladies dentaires, maladies neurologiques, maladies métaboliques, maladies du foie, maladies des reins, syndrome des ovaires polykystiques, endométriose, asthme et troubles fibrotiques.

**26.** Composé à utiliser selon la revendication 25, dans lequel la maladie, le trouble ou l'état est un cancer choisi dans le groupe constitué par le glioblastome, le cancer du sein, le cancer du côlon, le cancer du rein, le cancer de l'utérus, le cancer du poumon primaire et métastatique, le mésothéliome, l'ostéosarcome, le mélanome malin, le cancer de l'ovaire, le cancer du col de l'utérus, le cancer de la prostate, le cancer du foie, le cancer gastrique, le cancer du rein métastatique, la leucémie, le lymphome, l'oligodendrogliome, le glioblastome et les tumeurs des cellules germinales.

**27.** Composé à utiliser selon la revendication 25, dans lequel la maladie, le trouble ou l'état est une maladie allergique choisie dans le groupe constitué par l'asthme, la rhinite allergique, la rhinite allergique saisonnière, la rhinosinusite chronique avec ou sans polypes nasaux, la conjonctivite, la kératoconjonctivite, la conjonctivite allergique saisonnière, le syndrome de l'œil sec, l'œsophagite à éosinophiles, la maladie cœliaque, l'allergie alimentaire, le syndrome du côlon irritable, la maladie du côlon irritable, l'eczéma atopique, la dermatite atopique, la dermatite de contact allergique, l'otite moyenne à éosinophiles, la pneumonie à éosinophiles et les maladies médiées par IgG4.

**28.** Composé à utiliser selon la revendication 25, dans lequel la maladie, le trouble ou l'état est une maladie inflammatoire aiguë ou chronique choisie dans le groupe constitué des maladies fongiques, des infections parasitaires, de la maladie cœliaque, de la colite microscopique, de la bronchopneumopathie chronique obstructive (BPCO), de la fibrose pulmonaire idiopathique, des maladies pulmonaires interstitielles, de la fibrose kystique, du syndrome d'Hermansky-Pudlak et de la maladie d'Alzheimer.

**29.** Composé à utiliser selon la revendication 25, dans lequel la maladie, le trouble ou l'état est un trouble auto-immun choisi dans le groupe constitué par les maladies inflammatoires de l'intestin, la colite ulcéreuse, la maladie de Crohn,

la polyarthrite rhumatoïde, l'arthrose, le psoriasis, la sclérodermie, la sclérose en plaques, le syndrome de Sjögren, l'athérosclérose et la sarcoïdose.

**30.** Composé à utiliser selon la revendication 25, dans lequel la maladie, le trouble ou l'état est une maladie métabolique choisie dans le groupe constitué par le diabète sucré insulinodépendant et le diabète sucré non insulinodépendant.

**31.** Composé à utiliser selon la revendication 25, dans lequel la maladie, le trouble ou l'état est une maladie du foie choisie dans le groupe constitué de la stéatose hépatique non alcoolique, de la stéatohépatite non alcoolique, de la fibrose et de la cirrhose induites par le virus de l'hépatite C et de la fibrose alcoolique.

**32.** Composé à utiliser selon la revendication 25, dans lequel la maladie, le trouble ou l'état est une maladie rénale choisie dans le groupe constitué par la néphropathie (*par exemple,* la néphropathie diabétique), la glomérulosclérose segmentaire focale, la fibrose tubulointerstitielle, la fibrose post-transplantation et la fibrose rétropéritonéale (maladie d'Ormond).

**33.** Composé à utiliser selon la revendication 25, dans lequel la maladie, le trouble ou l'état est un trouble fibrotique, en particulier la fibrose pulmonaire idiopathique (FPI).

**34.** Composé à utiliser selon la revendication 25, dans lequel la maladie, le trouble ou l'état est une maladie de stockage choisie dans le groupe constitué par la maladie de Gaucher, la maladie de Fabry, les troubles du stockage lysosomal, la maladie de Niemann-Pick, la cystinose néphropathique et la globotriaosylcéramidose liée au chromosome X.

**35.** Méthode d'identification des composés qui interfèrent avec l'interaction entre la protéine YKL-40 de type chitinase et l'asialofétuine :

(1) dans un essai de compétition de liaison, en soumettant (i) un composé selon l'une quelconque des revendications 1 à 22 et (ii) l'asialofétuine biotinylée à YKL-40 ou à un dérivé de YKL-40 comprenant une étiquette de purification ; et
(2) en évaluant le signal de luminescence AlphaScreen résultant ; dans lequel une diminution du signal d'émission du niveau élevé à la plus faible concentration d'inhibiteur au niveau inférieur (diminué par la valeur d'au moins 3x l'écart-type du bruit affiché aux valeurs de plateau aux plus fortes concentrations d'inhibiteur dans les diagrammes de réponse pour les déterminations de la CI50) à la plus forte concentration d'inhibiteur indique que le composé interfère avec l'interaction entre la protéine YKL-40 de type chitinase et l'asialofétuine.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- PL P441066
- US 63336426
- US 5284656 A, Platz
- US 5451569 A, Wong
- PL P440558
- US 63316477 A
- US 18117193 B

### Non-patent literature cited in the description


- **FUNKHOUSER et al.** Chitinase family GH18: evolutionary insights from the genomic history of a diverse protein family. *BMC Evol Biol.*, June 2007, vol. 26, 7-96
- **LEE et al.** Role of chitin and chitinase/chitinase-like proteins in inflammation, tissue remodeling, and injury. *Annu Rev Physiol.*, 2011, vol. 73, 479-501
- **JOHANSEN et al.** Identification of proteins secreted by human osteoblastic cells in culture. *Journal of Bone & Mineral Research*, 1992, vol. 7, 501-512
- **REHLI et al.** Molecular characterization of the gene for human cartilage gp-39 (CHI3L1), a member of the chitinase protein family and marker for late stages of macrophage differentiation. *Genomics*, 1997, vol. 43 (2), 221-5
- **RENKEMA et al.** Chitotriosidase, a chitinase, and the 39-kDa human cartilage glycoprotein, a chitin-binding lectin, are homologues of family 18 glycosyl hydrolases secreted by human macrophages. *Eur J Biochem.*, 1998, vol. 251, 504-509
- **CHUANET**. Chitinase 3-like 1 Regulates Cellular and Tissue Responses via IL-13 Receptor $\alpha 2$. *Cell Rep.*, 2013, vol. 4 (4), 830-841
- **SHAO et al.** YKL-40, a secreted glycoprotein, promotes tumor angiogenesis. *Oncogene*, 2009, vol. 28, 4456-4468
- **GENG et al.** Chitinase 3-like 1-CD44 interaction promotes metastasis and epithelial-to-mesenchymal transition through $\beta$-catenin/Erk/Akt signaling in gastric cancer. *J Exp Clin Cancer Res*, 2018, vol. 37, 208
- **LOW et al.** Chitinase 3-like 1 induces survival and proliferation of intestinal epithelial cells during chronic inflammation and colitis-associated cancer by regulating S100A9. *Oncotarget*, 2015, vol. 6, 36535-50
- **BOUVET et al.** Peripheral blood mononuclear cell response to YKL-40 and Galectin-3 in cystic fibrosis. *Cytokine*, 2021, vol. 146, 155635
- **LEE et al.** Chitin, chitinases and chitinase-like proteins in allergic inflammation and tissue remodeling. *Yonsei Med J*, 2009, vol. 50, 22-30
- **LEE et al.** Role of chitin and chitinase/chitinase-like proteins in inflammation, tissue remodeling and injury. *Annu Rev Physiol*, 2011, vol. 73, 479-501
- **LEE et al.** Role of breast regression protein-39/YKL-40 in asthma and allergic responses. *Allergy Asthma Immunol Res*, 2010, vol. 2, 20-7
- **LEE et al.** Role of breast regression protein 39 (BRP-39)/chitinase 3-like-1 in Th2 and IL-13-induced tissue responses and apoptosis. *J Exp Med*, 2009, vol. 206, 1149-66
- **DELA CRUZ et al.** Chitinase 3-like-1 (Chi3l1) Regulation of Streptococcus pneumoniae Lung Infection. *Cell Host Microbe*, 2012, vol. 12, 34-4
- **JOHANSEN et al.** A new biochemical marker for joint injury. Analysis of YKL-40 in serum and synovial fluid. *Br J Rheumatol*, 1993, vol. 32, 949-55
- **MUSZYŃSKI et al.** YKL-40 as a Potential Biomarker and a Possible Target in Therapeutic Strategies of Alzheimer's Disease. *Curr Neuropharmacol.*, 2017, vol. 15 (6), 906-917
- **JOHANSEN et al.** Is YKL-40 a new therapeutic target in cancer?. *Expert Opin Ther Targets*, 2017, vol. 11 (2), 219-34
- **SHAO et al.** YKL-40, a secreted glycoprotein, promotes tumor angiogenesis. *Oncogene*, 2009, vol. 28 (50), 4456-4468
- **FAIBISH et al.** A YKL-40-neutralizing antibody blocks tumor angiogenesis and progression: a potential therapeutic agent in cancers. *Mol Cancer Ther.*, 2011, vol. 10 (5), 742-51
- **PARK et al.** G721-0282 inhibits cell growth and induces apoptosis in human osteosarcoma through down-regulation of the STAT3 pathway. *International journal of biological sciences*, 2020, vol. 16 (2), 330-341

- **LEE et al.** A small molecule targeting CHI3L1 inhibits lung metastasis by blocking IL-13Rα2-mediated JNK-AP-1 signals. *Mol Oncol.*, 2022, vol. 16 (2), 508-526
- **FRANCESCONE et al.** *J Biol Chem*, 2011, vol. 286, 15332-43
- **KU et al.** *Int J Cancer*, 2011, vol. 128, 1316-26
- **ZHANG et al.** *Cancer*, 2010, vol. 116, 2688
- **JOHANSEN et al.** *Breast Cancer Res Treat*, 2003, vol. 80, 15-21
- **FIJNEMAN et al.** *Clin Cancer Res*, 2012, vol. 18, 2613
- **CHEN et al.** *Am J Pathol.*, 2011, vol. 179, 1494
- **WANG et al.** *Tumour Biol*, 2015, vol. 36, 901-7
- **JOHANSEN et al.** *Lung Cancer*, 2004, vol. 46, 333-40
- **CORRADI et al.** *Anticancer Res.*, December 2013, vol. 33 (12), 5517
- **MA et al.** *Cancer Res*, 2015, vol. 75, 487-96
- **HOGDALL et al.** *BMC Cancer*, 2009, vol. 9, 8
- **DUPONT et al.** *J Clin Oncol.*, 2004, vol. 22, 3330
- **NGERNYUANG et al.** *Int J Biochem Cell Biol*, 2014, vol. 51, 45-52
- **JEET et al.** *Endocr Relat Cancer.*, 2014, vol. 21, 723
- **PAN et al.** *J Cancer Res Clin Oncol*, 2013, vol. 139, 1043-54
- **LI et al.** *Chin Med J*, 2012, vol. 125, 1777
- **ZHANGG et al.** *Tumour Biol*, 2014, vol. 35, 12131-7
- **MACTIER et al.** *J Proteome Res.*, 2011, vol. 10, 1030
- **MARCHESI et al.** *Vet Pathol.*, 2006, vol. 43, 773-6
- **MARCHESI et al.** *J Vet Med A Physiol Pathol Clin Med.*, 2003, vol. 50, 103
- **QUERSHI et al.** *Genes Cancer*, 2011, vol. 2, 74
- **EURICH et al.** *World J Gastroenterol.*, 2009, vol. 15, 5249
- **ROSLIND** ; **JOHANSEN**. *Methods of Mol Biol.*, 2009, vol. 511, 159
- **JOHANSEN et al.** *Cancer Epidemiol. Biomarkers Prev.*, 2006, vol. 15 (2), 194-202
- **VALEYRE et al.** Sarcoidosis. *Lancet*, 2014, vol. 383 (9923), 1155-67
- **LONG et al.** Serum YKL-40 as predictor of outcome in hypersensitivity pneumonitis. *Eur Respir J.*, 2017, vol. 23, 49-51
- **JOHANSEN et al.** Increased serum YKL-40 in patients with pulmonary sarcoidosis-a potential marker of disease activity?. *Respir Med.*, 2005, vol. 99 (4), 396-402
- **RICHELDI et al.** Idiopathic pulmonary fibrosis. *Lancet*, 2017, vol. 389 (10082), 1941-1952
- **FURUHASHI et al.** Increased expression of YKL-40, a chitinase-like protein, in serum and lung of patients with idiopathic pulmonary fibrosis. *Respir Med.*, 2010, vol. 104 (8), 1204-10
- **VIJ et al.** Peripheral blood biomarkers in idiopathic pulmonary fibrosis. *Transl Res.*, 2012, vol. 159 (4), 218-27
- **KORTHAGEN et al.** Serum and BALF YKL-40 levels are predictors of survival in idiopathic pulmonary fibrosis. *Respir Med.*, January 2011, vol. 105 (1), 106-13
- **PAPI et al.** Asthma. *Lancet*, 2018, vol. 391 (10122), 783-800
- **RABE et al.** Chronic obstructive pulmonary disease. *Lancet*, 2017, vol. 389 (10082), 1931-1940
- **XIANG et al.** The YKL-40 protein is a potential biomarker for COPD: a meta-analysis and systematic review. *Int J Chron Obstruct Pulmon Dis.*, 2018, vol. 13, 409-418
- **XIANG et al.** The serum YKL-40 is a useful biomarker for asthma. *European Respiratory Journal*, 2017, vol. 50, PA3566
- **GREENE, T.W.** ; **WUTS, P.G.M.** Protective Groups in Organic Synthesis. Wiley, 1991
- Periodic Chart of the Elements. The Merck Index. 1996
- The McGraw-Hill Dictionary of Chemical Terms. McGraw-Hill, 1985
- **ISSELBACHER et al.** Harrison's Principles of Internal Medicine. 1996, 1814-1882
- Soluble Polymer-Enzyme Adducts. **ABUCHOWSKI** ; **DAVIS**. Enzymes as Drugs. Wiley-Interscience, 1981, 367-383
- **NEWMARK et al.** *J Appl Biochem*, 1982, vol. 4, 185-9
- **ADJEI et al.** *Pharm Res*, 1990, vol. 7, 565-569
- **ADJEI et al.** *Int J Pharmaceutics*, 1990, vol. 63, 135-144
- **BRAQUET et al.** *J Cardiovasc Pharmacol*, 1989, vol. 13 (5), 143-146
- **HUBBARD et al.** *Annal Int Med*, 1989, vol. 3, 206-212
- **SMITH et al.** *J Clin Invest*, 1989, vol. 84, 1145-1146
- **OSWEIN et al.** Aerosolization of Proteins. *Proceedings of Symposium on Respiratory Drug Delivery II, Keystone, Colorado*, March 1990
- **DEBS et al.** *J Immunol*, 1988, vol. 140, 3482-3488
- **LANGER R**. *Science*, 1990, vol. 249, 1527-33
- **SAWHNEY H S et al.** *Macromolecules*, 1993, vol. 26, 581-7
- **SMITH** ; **MARCH**. March's Advanced Organic Chemistry: Reactions, Mechanisms, and Structure. Wiley-Interscience, 2001
- **VOGEL**. A Textbook of Practical Organic Chemistry, Including Qualitative Organic Analysis. 1978

- **J. F. W. MCOMIE**. Protective Groups in Organic Chemistry. Plenum Press, 1973
- **T. W. GREENE** ; **P. G. M. WUTS**. Protective Groups in Organic Synthesis. Wiley, 1999
- The Peptides. Academic Press, 1981, vol. 3
- **HOUBEN** ; **WEYL**. Methoden der organischen Chemie. Georg Thieme Verlag, 1974, vol. 15
- **H.-D. JAKUBKE** ; **H. JESCHEIT**. Aminosauren, Peptide, Proteine. Verlag Chemie, 1982
- **JOCHEN LEHMANN**. Chemie der Kohlenhydrate: Monosaccharide und Derivate. Georg Thieme Verlag, 1974